(19) Europäisches Patentamt
European Patent Office
Office européen des brevets

(11) EP 3 943 108 A1

(12) EUROPEAN PATENT APPLICATION
published in accordance with Art. 153(4) EPC

(43) Date of publication:
**26.01.2022 Bulletin 2022/04**

(21) Application number: **20773939.2**

(22) Date of filing: **19.03.2020**

(51) International Patent Classification (IPC):
***A61K 39/395*** (2006.01) ***A61P 35/00*** (2006.01)
***C07K 16/28*** (2006.01) ***C07K 16/44*** (2006.01)
***C40B 40/08*** (2006.01) ***C40B 40/10*** (2006.01)
***C12N 15/13*** (2006.01) ***G01N 33/15*** (2006.01)
***G01N 33/53*** (2006.01) ***C12P 21/08*** (2006.01)

(52) Cooperative Patent Classification (CPC):
**A61K 39/395; A61P 35/00; C07K 16/28; C07K 16/44; C40B 40/08; C40B 40/10; G01N 33/15; G01N 33/53**

(86) International application number:
**PCT/JP2020/012189**

(87) International publication number:
**WO 2020/189748 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2019 JP 2019051965**

(71) Applicant: **CHUGAI SEIYAKU KABUSHIKI KAISHA**
**Tokyo 115-8543 (JP)**

(72) Inventors:
- **MIZUNO, Hideaki**
  **Kamakura-shi, Kanagawa 247-8530 (JP)**
- **SAKA, Koichiro**
  **Kamakura-shi, Kanagawa 247-8530 (JP)**
- **KAWAUCHI, Hiroki**
  **Kamakura-shi, Kanagawa 247-8530 (JP)**
- **KATO, Kuniyasu**
  **Kamakura-shi, Kanagawa 247-8530 (JP)**
- **SAITO, Ryoichi**
  **Kamakura-shi, Kanagawa 247-8530 (JP)**
- **IGAWA, Tomoyuki**
  **Synapse, 138623 (SG)**
- **OHARA, Kazuhiro**
  **Kamakura-shi, Kanagawa 247-8530 (JP)**
- **OKUDE, Junya**
  **Kamakura-shi, Kanagawa 247-8530 (JP)**

(74) Representative: **Vossius & Partner**
**Patentanwälte Rechtsanwälte mbB**
**Siebertstraße 3**
**81675 München (DE)**

# (54) ANTIGEN-BINDING MOLECULE CONTAINING ANTIGEN-BINDING DOMAIN OF WHICH BINDING ACTIVITY TO ANTIGEN IS CHANGED DEPENDING ON MTA, AND LIBRARY FOR OBTAINING SAID ANTIGEN-BINDING DOMAIN

(57) An objective of the present invention is to provide antigen-binding molecules whose antigen-binding activity changes depending on the concentration of a small molecule compound specific to a target tissue, polynucleotides encoding the antigen-binding molecules, vectors containing the polynucleotides, cells carrying the vectors, libraries containing a plurality of the antigen-binding molecules that are different from one another, pharmaceutical compositions containing the antigen-binding molecules, methods of screening for the antigen-binding molecules, methods of producing the same, and the like. The present inventors discovered methylthioadenosine (MTA) as a small molecule compound specific to tumor tissue and created an antigen-binding domain whose antigen-binding activity changes depending on the concentration of MTA, or an antigen-binding molecule containing the antigen-binding domain, and also created a library containing a plurality of antigen-binding domains or antigen-binding molecules containing the antigen-binding domains that are different from one another and discovered that the above objective can be achieved by using the library. By using the antigen-binding molecules of the present disclosure, various diseases (for example, cancer) caused by target tissues

EP 3 943 108 A1

(for example, tumor tissues) can be treated in a target tissue-specific manner.

SEQUENCE LISTING

<110> CHUGAI SEIYAKU KABUSHIKI KAISHA

<120> ANTIGEN-BINDING MOLECULES COMPRISING ANTIGEN BINDING DOMAIN THAT BINDING ACTIVITIES FOR ANTIGEN CHANGE MTA-DEPENDENTLY AND LIBRARIES FOR OBTAINING SAID ANTIGEN-BINDING DOMAIN

<130> C1-A1826P

<150> JP 2019-051965
<151> 2019-03-19

<160> 89

<170> PatentIn version 3.5

<210> 1
<211> 468
<212> PRT
<213> Homo sapiens

<400> 1

```
Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160
```

```
Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Gln Asp Ser Ser Ser Val Pro Leu Pro Thr Phe Leu
        355                 360                 365

Val Ala Gly Gly Ser Leu Ala Phe Gly Thr Leu Leu Cys Ile Ala Ile
    370                 375                 380

Val Leu Arg Phe Lys Lys Thr Trp Lys Leu Arg Ala Leu Lys Glu Gly
385                 390                 395                 400

Lys Thr Ser Met His Pro Pro Tyr Ser Leu Gly Gln Leu Val Pro Glu
                405                 410                 415
```

Arg Pro Arg Pro Thr Pro Val Leu Val Pro Leu Ile Ser Pro Pro Val
420 425 430

Ser Pro Ser Ser Leu Gly Ser Asp Asn Thr Ser Ser His Asn Arg Pro
435 440 445

Asp Ala Arg Asp Pro Arg Ser Pro Tyr Asp Ile Ser Asn Thr Asp Tyr
450 455 460

Phe Phe Pro Arg
465

<210> 2
<211> 1407
<212> DNA
<213> Homo sapiens

<400> 2
atgctggccg tcggctgcgc gctgctggct gccctgctgg ccgcgccggg agcggcgctg 60

gccccaaggc gctgccctgc gcaggaggtg gcgagaggcg tgctgaccag tctgccagga 120

gacagcgtga ctctgacctg cccgggggta gagccggaag acaatgccac tgttcactgg 180

gtgctcagga agccggctgc aggctcccac cccagcagat gggctggcat gggaaggagg 240

ctgctgctga ggtcggtgca gctccacgac tctggaaact attcatgcta ccgggccggc 300

cgcccagctg ggactgtgca cttgctggtg gatgttcccc ccgaggagcc ccagctctcc 360

tgcttccgga agagcccccct cagcaatgtt gtttgtgagt ggggtcctcg gagcacccca 420

tccctgacga caaaggctgt gctcttggtg aggaagtttc agaacagtcc ggccgaagac 480

ttccaggagc cgtgccagta ttcccaggag tcccagaagt tctcctgcca gttagcagtc 540

ccggagggag acagctcttt ctacatagtg tccatgtgcg tcgccagtag tgtcgggagc 600

aagttcagca aaactcaaac ctttcagggt tgtggaatct tgcagcctga tccgcctgcc 660

aacatcacag tcactgccgt ggccagaaac ccccgctggc tcagtgtcac ctggcaagac 720

ccccactcct ggaactcatc tttctacaga ctacggtttg agctcagata tcgggctgaa 780

cggtcaaaga cattcacaac atggatggtc aaggacctcc agcatcactg tgtcatccac 840

gacgcctgga gcggcctgag gcacgtggtg cagcttcgtg cccaggagga gttcgggcaa 900

ggcgagtgga gcgagtggag cccggaggcc atgggcacgc cttggacaga atccaggagt 960

cctccagctg agaacgaggt gtccaccccc atgcaggcac ttactactaa taaagacgat 1020

gataatattc tcttcagaga ttctgcaaat gcgacaagcc tcccagtgca agattcttct 1080

tcagtaccac tgcccacatt cctggttgct ggagggagcc tggccttcgg aacgctcctc 1140

tgcattgcca ttgttctgag gttcaagaag acgtggaagc tgcgggctct gaaggaaggc 1200

aagacaagca tgcatccgcc gtactctttg gggcagctgg tcccggagag gcctcgaccc 1260

accccagtgc ttgttcctct catctcccca ccggtgtccc ccagcagcct ggggtctgac 1320

aatacctcga gccacaaccg accagatgcc agggacccac ggagccctta tgacatcagc 1380

aatacagact acttcttccc cagatag 1407

<210> 3
<211> 19
<212> PRT
<213> Artificial Sequence

<220>
<223> An artificially synthesized sequence

<400> 3

Met Gly Trp Ser Cys Ile Ile Leu Phe Leu Val Ala Thr Ala Thr Gly
1               5                   10                  15

Val His Ser

<210> 4
<211> 21
<212> PRT
<213> Artificial Sequence

<220>
<223> An artificially synthesized sequence

<400> 4

Phe Asn Asn Phe Thr Val Ser Phe Trp Leu Arg Val Pro Lys Val Ser
1               5                   10                  15

Ala Ser His Leu Glu
            20

<210> 5
<211> 330
<212> PRT
<213> Artificial Sequence

<220>
<223> An artificially synthesized sequence

<400> 5

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 6
<211> 326
<212> PRT
<213> Artificial Sequence

<220>
<223> An artificially synthesized sequence

<400> 6

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

Thr Val Glu Arg Lys Cys Cys Val Glu Cys Pro Pro Cys Pro Ala Pro
100 105 110

Pro Val Ala Gly Pro Ser Val Phe Leu Phe Pro Pro Lys Pro Lys Asp
115 120 125

Thr Leu Met Ile Ser Arg Thr Pro Glu Val Thr Cys Val Val Val Asp
130 135 140

Val Ser His Glu Asp Pro Glu Val Gln Phe Asn Trp Tyr Val Asp Gly
145 150 155 160

```
Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325


<210>  7
<211>  377
<212>  PRT
<213>  Artificial Sequence

<220>
<223>  An artificially synthesized sequence

<400>  7

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30
```

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285
```

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210> 8
<211> 327
<212> PRT
<213> Artificial Sequence

<220>
<223> An artificially synthesized sequence

<400> 8

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

```
Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325


<210>  9
<211>  1122
<212>  DNA
<213>  Homo sapiens

<400>  9
atgtggttct tgacaactct gctcctttgg gttccagttg atgggcaagt ggacaccaca        60
```

```
aaggcagtga tcactttgca gcctccatgg gtcagcgtgt tccaagagga aaccgtaacc      120
ttgcactgtg aggtgctcca tctgcctggg agcagctcta cacagtggtt tctcaatggc      180
acagccactc agacctcgac cccagctac agaatcacct ctgccagtgt caatgacagt       240
ggtgaataca ggtgccagag aggtctctca gggcgaagtg accccataca gctggaaatc      300
cacagaggct ggctactact gcaggtctcc agcagagtct tcacggaagg agaacctctg      360
gccttgaggt gtcatgcgtg gaaggataag ctggtgtaca atgtgcttta ctatcgaaat      420
ggcaaagcct ttaagttttt ccactggaat tctaacctca ccattctgaa aaccaacata      480
agtcacaatg gcacctacca ttgctcaggc atgggaaagc atcgctacac atcagcagga      540
atatctgtca ctgtgaaaga gctatttcca gctccagtgc tgaatgcatc tgtgacatcc      600
ccactcctgg aggggaatct ggtcaccctg agctgtgaaa caaagttgct cttgcagagg      660
cctggtttgc agctttactt ctccttctac atgggcagca agaccctgcg aggcaggaac      720
acatcctctg aataccaaat actaactgct agaagagaag actctgggtt atactggtgc      780
gaggctgcca cagaggatgg aaatgtcctt aagcgcagcc ctgagttgga gcttcaagtg      840
cttggcctcc agttaccaac tcctgtctgg tttcatgtcc ttttctatct ggcagtggga      900
ataatgtttt tagtgaacac tgttctctgg gtgacaatac gtaaagaact gaaaagaaag      960
aaaaagtggg atttagaaat ctctttggat tctggtcatg agaagaaggt aatttccagc     1020
cttcaagaag acagacattt agaagaagag ctgaaatgtc aggaacaaaa agaagaacag     1080
ctgcaggaag ggtgcaccg gaaggagccc caggggccca cg                         1122
```

```
<210>  10
<211>  374
<212>  PRT
<213>  Homo sapiens

<400>  10

Met Trp Phe Leu Thr Thr Leu Leu Leu Trp Val Pro Val Asp Gly Gln
1               5                   10                  15

Val Asp Thr Thr Lys Ala Val Ile Thr Leu Gln Pro Pro Trp Val Ser
            20                  25                  30

Val Phe Gln Glu Glu Thr Val Thr Leu His Cys Glu Val Leu His Leu
        35                  40                  45

Pro Gly Ser Ser Ser Thr Gln Trp Phe Leu Asn Gly Thr Ala Thr Gln
    50                  55                  60

Thr Ser Thr Pro Ser Tyr Arg Ile Thr Ser Ala Ser Val Asn Asp Ser
65                  70                  75                  80
```

```
Gly Glu Tyr Arg Cys Gln Arg Gly Leu Ser Gly Arg Ser Asp Pro Ile
                85                  90                  95

Gln Leu Glu Ile His Arg Gly Trp Leu Leu Leu Gln Val Ser Ser Arg
            100                 105                 110

Val Phe Thr Glu Gly Glu Pro Leu Ala Leu Arg Cys His Ala Trp Lys
        115                 120                 125

Asp Lys Leu Val Tyr Asn Val Leu Tyr Tyr Arg Asn Gly Lys Ala Phe
    130                 135                 140

Lys Phe Phe His Trp Asn Ser Asn Leu Thr Ile Leu Lys Thr Asn Ile
145                 150                 155                 160

Ser His Asn Gly Thr Tyr His Cys Ser Gly Met Gly Lys His Arg Tyr
                165                 170                 175

Thr Ser Ala Gly Ile Ser Val Thr Val Lys Glu Leu Phe Pro Ala Pro
            180                 185                 190

Val Leu Asn Ala Ser Val Thr Ser Pro Leu Leu Glu Gly Asn Leu Val
        195                 200                 205

Thr Leu Ser Cys Glu Thr Lys Leu Leu Leu Gln Arg Pro Gly Leu Gln
    210                 215                 220

Leu Tyr Phe Ser Phe Tyr Met Gly Ser Lys Thr Leu Arg Gly Arg Asn
225                 230                 235                 240

Thr Ser Ser Glu Tyr Gln Ile Leu Thr Ala Arg Arg Glu Asp Ser Gly
                245                 250                 255

Leu Tyr Trp Cys Glu Ala Ala Thr Glu Asp Gly Asn Val Leu Lys Arg
            260                 265                 270

Ser Pro Glu Leu Glu Leu Gln Val Leu Gly Leu Gln Leu Pro Thr Pro
        275                 280                 285

Val Trp Phe His Val Leu Phe Tyr Leu Ala Val Gly Ile Met Phe Leu
    290                 295                 300

Val Asn Thr Val Leu Trp Val Thr Ile Arg Lys Glu Leu Lys Arg Lys
305                 310                 315                 320

Lys Lys Trp Asp Leu Glu Ile Ser Leu Asp Ser Gly His Glu Lys Lys
```

325 330 335

Val Ile Ser Ser Leu Gln Glu Asp Arg His Leu Glu Glu Glu Leu Lys
340 345 350

Cys Gln Glu Gln Lys Glu Glu Gln Leu Gln Glu Gly Val His Arg Lys
355 360 365

Glu Pro Gln Gly Ala Thr
370

<210> 11
<211> 948
<212> DNA
<213> Homo sapiens

<400> 11
atgactatgg agacccaaat gtctcagaat gtatgtccca gaaacctgtg gctgcttcaa 60

ccattgacag ttttgctgct gctggcttct gcagacagtc aagctgctcc cccaaaggct 120

gtgctgaaac ttgagccccc gtggatcaac gtgctccagg aggactctgt gactctgaca 180

tgccaggggg ctcgcagccc tgagagcgac tccattcagt ggttccacaa tgggaatctc 240

attcccaccc acacgcagcc cagctacagg ttcaaggcca acaacaatga cagcggggag 300

tacacgtgcc agactggcca gaccagcctc agcgaccctg tgcatctgac tgtgctttcc 360

gaatggctgg tgctccagac ccctcacctg gagttccagg agggagaaac catcatgctg 420

aggtgccaca gctggaagga caagcctctg gtcaaggtca cattcttcca gaatggaaaa 480

tcccagaaat tctcccattt ggatcccacc ttctccatcc cacaagcaaa ccacagtcac 540

agtggtgatt accactgcac aggaaacata ggctacacgc tgttctcatc caagcctgtg 600

accatcactg tccaagtgcc cagcatgggc agctcttcac caatgggggt cattgtggct 660

gtggtcattg cgactgctgt agcagccatt gttgctgctg tagtggcctt gatctactgc 720

aggaaaaagc ggatttcagc caattccact gatcctgtga aggctgccca atttgagcca 780

cctggacgtc aaatgattgc catcagaaag agacaacttg aagaaaccaa caatgactat 840

gaaacagctg acggcggcta catgactctg aaccccaggg cacctactga cgatgataaa 900

aacatctacc tgactcttcc tcccaacgac catgtcaaca gtaataac 948

<210> 12
<211> 316
<212> PRT
<213> Homo sapiens

<400> 12

Met Thr Met Glu Thr Gln Met Ser Gln Asn Val Cys Pro Arg Asn Leu
1 5 10 15

```
Trp Leu Leu Gln Pro Leu Thr Val Leu Leu Leu Leu Ala Ser Ala Asp
            20                  25                  30

Ser Gln Ala Ala Pro Pro Lys Ala Val Leu Lys Leu Glu Pro Pro Trp
        35                  40                  45

Ile Asn Val Leu Gln Glu Asp Ser Val Thr Leu Thr Cys Gln Gly Ala
    50                  55                  60

Arg Ser Pro Glu Ser Asp Ser Ile Gln Trp Phe His Asn Gly Asn Leu
65                  70                  75                  80

Ile Pro Thr His Thr Gln Pro Ser Tyr Arg Phe Lys Ala Asn Asn Asn
                85                  90                  95

Asp Ser Gly Glu Tyr Thr Cys Gln Thr Gly Gln Thr Ser Leu Ser Asp
            100                 105                 110

Pro Val His Leu Thr Val Leu Ser Glu Trp Leu Val Leu Gln Thr Pro
        115                 120                 125

His Leu Glu Phe Gln Glu Gly Glu Thr Ile Met Leu Arg Cys His Ser
    130                 135                 140

Trp Lys Asp Lys Pro Leu Val Lys Val Thr Phe Phe Gln Asn Gly Lys
145                 150                 155                 160

Ser Gln Lys Phe Ser His Leu Asp Pro Thr Phe Ser Ile Pro Gln Ala
                165                 170                 175

Asn His Ser His Ser Gly Asp Tyr His Cys Thr Gly Asn Ile Gly Tyr
            180                 185                 190

Thr Leu Phe Ser Ser Lys Pro Val Thr Ile Thr Val Gln Val Pro Ser
        195                 200                 205

Met Gly Ser Ser Ser Pro Met Gly Val Ile Val Ala Val Val Ile Ala
    210                 215                 220

Thr Ala Val Ala Ala Ile Val Ala Ala Val Val Ala Leu Ile Tyr Cys
225                 230                 235                 240

Arg Lys Lys Arg Ile Ser Ala Asn Ser Thr Asp Pro Val Lys Ala Ala
                245                 250                 255

Gln Phe Glu Pro Pro Gly Arg Gln Met Ile Ala Ile Arg Lys Arg Gln
```

260 265 270

Leu Glu Glu Thr Asn Asn Asp Tyr Glu Thr Ala Asp Gly Gly Tyr Met
275 280 285

Thr Leu Asn Pro Arg Ala Pro Thr Asp Asp Asp Lys Asn Ile Tyr Leu
290 295 300

Thr Leu Pro Pro Asn Asp His Val Asn Ser Asn Asn
305 310 315

<210> 13
<211> 873
<212> DNA
<213> Homo sapiens

<400> 13
atgggaatcc tgtcattctt acctgtcctt gccactgaga gtgactgggc tgactgcaag 60

tccccccagc cttggggtca tatgcttctg tggacagctg tgctattcct ggctcctgtt 120

gctgggacac ctgcagctcc cccaaaggct gtgctgaaac tcgagcccca gtggatcaac 180

gtgctccagg aggactctgt gactctgaca tgccggggga ctcacagccc tgagagcgac 240

tccattcagt ggttccacaa tgggaatctc attcccaccc acacgcagcc cagctacagg 300

ttcaaggcca acaacaatga cagcggggag tacacgtgcc agactggcca gaccagcctc 360

agcgaccctg tgcatctgac tgtgctttct gagtggctgg tgctccagac ccctcacctg 420

gagttccagg agggagaaac catcgtgctg aggtgccaca gctggaagga caagcctctg 480

gtcaaggtca cattcttcca gaatggaaaa tccaagaaat tttcccgttc ggatcccaac 540

ttctccatcc cacaagcaaa ccacagtcac agtggtgatt accactgcac aggaaacata 600

ggctacacgc tgtactcatc caagcctgtg accatcactg tccaagctcc cagctcttca 660

ccgatgggga tcattgtggc tgtggtcact gggattgctg tagcggccat tgttgctgct 720

gtagtggcct tgatctactg caggaaaaag cggatttcag ccaatcccac taatcctgat 780

gaggctgaca aagttggggc tgagaacaca atcacctatt cacttctcat gcacccggat 840

gctctggaag agcctgatga ccagaaccgt att 873

<210> 14
<211> 291
<212> PRT
<213> Homo sapiens

<400> 14

Met Gly Ile Leu Ser Phe Leu Pro Val Leu Ala Thr Glu Ser Asp Trp
1 5 10 15

```
Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu
    50                  55                  60

Asp Ser Val Thr Leu Thr Cys Arg Gly Thr His Ser Pro Glu Ser Asp
65                  70                  75                  80

Ser Ile Gln Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Asn Pro
                245                 250                 255

Thr Asn Pro Asp Glu Ala Asp Lys Val Gly Ala Glu Asn Thr Ile Thr
            260                 265                 270
```

```
Tyr Ser Leu Leu Met His Pro Asp Ala Leu Glu Glu Pro Asp Asp Gln
        275                 280                 285

Asn Arg Ile
    290
```

<210> 15
<211> 762
<212> DNA
<213> Homo sapiens

<400> 15

```
atgtggcagc tgctcctccc aactgctctg ctacttctag tttcagctgg catgcggact      60

gaagatctcc caaaggctgt ggtgttcctg gagcctcaat ggtacagggt gctcgagaag     120

gacagtgtga ctctgaagtg ccagggagcc tactcccctg aggacaattc cacacagtgg     180

tttcacaatg agagcctcat ctcaagccag gcctcgagct acttcattga cgctgccaca     240

gttgacgaca gtggagagta caggtgccag acaaacctct ccaccctcag tgacccggtg     300

cagctagaag tccatatcgg ctggctgttg ctccaggccc ctcggtgggt gttcaaggag     360

gaagacccta ttcacctgag gtgtcacagc tggaagaaca ctgctctgca taaggtcaca     420

tatttacaga atggcaaagg caggaagtat tttcatcata attctgactt ctacattcca     480

aaagccacac tcaaagacag cggctcctac ttctgcaggg ggcttgttgg gagtaaaaat     540

gtgtcttcag agactgtgaa catcaccatc actcaaggtt tgtcagtgtc aaccatctca     600

tcattctttc cacctgggta ccaagtctct ttctgcttgg tgatggtact cctttttgca     660

gtggacacag gactatattt ctctgtgaag acaaacattc gaagctcaac aagagactgg     720

aaggaccata aatttaaatg gagaaaggac cctcaagaca aa                        762
```

<210> 16
<211> 254
<212> PRT
<213> Homo sapiens

<400> 16

```
Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60
```

Ser Leu Ile Ser Ser Gln Ala Ser Ser Tyr Phe Ile Asp Ala Ala Thr
65 70 75 80

Val Asp Asp Ser Gly Glu Tyr Arg Cys Gln Thr Asn Leu Ser Thr Leu
85 90 95

Ser Asp Pro Val Gln Leu Glu Val His Ile Gly Trp Leu Leu Leu Gln
100 105 110

Ala Pro Arg Trp Val Phe Lys Glu Glu Asp Pro Ile His Leu Arg Cys
115 120 125

His Ser Trp Lys Asn Thr Ala Leu His Lys Val Thr Tyr Leu Gln Asn
130 135 140

Gly Lys Gly Arg Lys Tyr Phe His His Asn Ser Asp Phe Tyr Ile Pro
145 150 155 160

Lys Ala Thr Leu Lys Asp Ser Gly Ser Tyr Phe Cys Arg Gly Leu Val
165 170 175

Gly Ser Lys Asn Val Ser Ser Glu Thr Val Asn Ile Thr Ile Thr Gln
180 185 190

Gly Leu Ser Val Ser Thr Ile Ser Ser Phe Phe Pro Pro Gly Tyr Gln
195 200 205

Val Ser Phe Cys Leu Val Met Val Leu Leu Phe Ala Val Asp Thr Gly
210 215 220

Leu Tyr Phe Ser Val Lys Thr Asn Ile Arg Ser Ser Thr Arg Asp Trp
225 230 235 240

Lys Asp His Lys Phe Lys Trp Arg Lys Asp Pro Gln Asp Lys
245 250

<210> 17
<211> 699
<212> DNA
<213> Homo sapiens

<400> 17
atgtggcagc tgctcctccc aactgctctg ctacttctag tttcagctgg catgcggact 60

gaagatctcc caaaggctgt ggtgttcctg gagcctcaat ggtacagcgt gcttgagaag 120

gacagtgtga ctctgaagtg ccagggagcc tactcccctg aggacaattc cacacagtgg 180

tttcacaatg agagcctcat ctcaagccag gcctcgagct acttcattga cgctgccaca 240

gtcaacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaaga caggaagtat tttcatcata attctgactt ccacattcca 480

aaagccacac tcaaagatag cggctcctac ttctgcaggg ggcttgttgg gagtaaaaat 540

gtgtcttcag agactgtgaa catcaccatc actcaaggtt tggcagtgtc aaccatctca 600

tcattctctc cacctgggta ccaagtctct ttctgcttgg tgatggtact cctttttgca 660

gtggacacag gactatattt ctctgtgaag acaaacatt 699

<210> 18
<211> 233
<212> PRT
<213> Homo sapiens

<400> 18

Met Trp Gln Leu Leu Leu Pro Thr Ala Leu Leu Leu Leu Val Ser Ala
1 5 10 15

Gly Met Arg Thr Glu Asp Leu Pro Lys Ala Val Val Phe Leu Glu Pro
20 25 30

Gln Trp Tyr Ser Val Leu Glu Lys Asp Ser Val Thr Leu Lys Cys Gln
35 40 45

Gly Ala Tyr Ser Pro Glu Asp Asn Ser Thr Gln Trp Phe His Asn Glu
50 55 60

Ser Leu Ile Ser Ser Gln Ala Ser Ser Tyr Phe Ile Asp Ala Ala Thr
65 70 75 80

Val Asn Asp Ser Gly Glu Tyr Arg Cys Gln Thr Asn Leu Ser Thr Leu
85 90 95

Ser Asp Pro Val Gln Leu Glu Val His Ile Gly Trp Leu Leu Leu Gln
100 105 110

Ala Pro Arg Trp Val Phe Lys Glu Glu Asp Pro Ile His Leu Arg Cys
115 120 125

His Ser Trp Lys Asn Thr Ala Leu His Lys Val Thr Tyr Leu Gln Asn
130 135 140

Gly Lys Asp Arg Lys Tyr Phe His His Asn Ser Asp Phe His Ile Pro
145 150 155 160

Lys Ala Thr Leu Lys Asp Ser Gly Ser Tyr Phe Cys Arg Gly Leu Val
                165                 170                 175

Gly Ser Lys Asn Val Ser Ser Glu Thr Val Asn Ile Thr Ile Thr Gln
            180                 185                 190

Gly Leu Ala Val Ser Thr Ile Ser Ser Phe Ser Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile
225                 230

<210> 19
<211> 4
<212> PRT
<213> Artificial Sequence

<220>
<223> An artificially synthesized sequence

<400> 19

Gly Gly Gly Ser
1

<210> 20
<211> 4
<212> PRT
<213> Artificial Sequence

<220>
<223> An artificially synthesized sequence

<400> 20

Ser Gly Gly Gly
1

<210> 21
<211> 5
<212> PRT
<213> Artificial Sequence

<220>
<223> An artificially synthesized sequence

<400> 21

Gly Gly Gly Gly Ser
1               5

<210> 22
<211> 5
<212> PRT
<213> Artificial Sequence

<220>
<223> An artificially synthesized sequence

<400> 22

Ser Gly Gly Gly Gly
1 5

<210> 23
<211> 6
<212> PRT
<213> Artificial Sequence

<220>
<223> An artificially synthesized sequence

<400> 23

Gly Gly Gly Gly Gly Ser
1 5

<210> 24
<211> 6
<212> PRT
<213> Artificial Sequence

<220>
<223> An artificially synthesized sequence

<400> 24

Ser Gly Gly Gly Gly Gly
1 5

<210> 25
<211> 7
<212> PRT
<213> Artificial Sequence

<220>
<223> An artificially synthesized sequence

<400> 25

Gly Gly Gly Gly Gly Gly Ser
1 5

<210> 26
<211> 7
<212> PRT
<213> Artificial Sequence

<220>

<223> An artificially synthesized sequence

<400> 26

```
Ser Gly Gly Gly Gly Gly Gly
1               5
```

<210> 27
<211> 107
<212> PRT
<213> Artificial Sequence

<220>
<223> An artificially synthesized sequence

<400> 27

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Ser Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Gly Asn Thr Leu Pro Tyr
                85                  90                  95

Thr Phe Gly Gln Gly Thr Lys Val Glu Ile Lys
            100                 105
```

<210> 28
<211> 365
<212> PRT
<213> Artificial Sequence

<220>
<223> An artificially synthesized sequence

<400> 28

```
Met Gly Val Pro Arg Pro Gln Pro Trp Ala Leu Gly Leu Leu Leu Phe
1               5                   10                  15

Leu Leu Pro Gly Ser Leu Gly Ala Glu Ser His Leu Ser Leu Leu Tyr
            20                  25                  30
```

```
His Leu Thr Ala Val Ser Ser Pro Ala Pro Gly Thr Pro Ala Phe Trp
        35                  40                  45

Val Ser Gly Trp Leu Gly Pro Gln Gln Tyr Leu Ser Tyr Asn Ser Leu
    50                  55                  60

Arg Gly Glu Ala Glu Pro Cys Gly Ala Trp Val Trp Glu Asn Gln Val
65                  70                  75                  80

Ser Trp Tyr Trp Glu Lys Glu Thr Thr Asp Leu Arg Ile Lys Glu Lys
                85                  90                  95

Leu Phe Leu Glu Ala Phe Lys Ala Leu Gly Gly Lys Gly Pro Tyr Thr
            100                 105                 110

Leu Gln Gly Leu Leu Gly Cys Glu Leu Gly Pro Asp Asn Thr Ser Val
        115                 120                 125

Pro Thr Ala Lys Phe Ala Leu Asn Gly Glu Glu Phe Met Asn Phe Asp
    130                 135                 140

Leu Lys Gln Gly Thr Trp Gly Gly Asp Trp Pro Glu Ala Leu Ala Ile
145                 150                 155                 160

Ser Gln Arg Trp Gln Gln Gln Asp Lys Ala Ala Asn Lys Glu Leu Thr
                165                 170                 175

Phe Leu Leu Phe Ser Cys Pro His Arg Leu Arg Glu His Leu Glu Arg
            180                 185                 190

Gly Arg Gly Asn Leu Glu Trp Lys Glu Pro Pro Ser Met Arg Leu Lys
        195                 200                 205

Ala Arg Pro Ser Ser Pro Gly Phe Ser Val Leu Thr Cys Ser Ala Phe
    210                 215                 220

Ser Phe Tyr Pro Pro Glu Leu Gln Leu Arg Phe Leu Arg Asn Gly Leu
225                 230                 235                 240

Ala Ala Gly Thr Gly Gln Gly Asp Phe Gly Pro Asn Ser Asp Gly Ser
                245                 250                 255

Phe His Ala Ser Ser Ser Leu Thr Val Lys Ser Gly Asp Glu His His
            260                 265                 270

Tyr Cys Cys Ile Val Gln His Ala Gly Leu Ala Gln Pro Leu Arg Val
        275                 280                 285
```

Glu Leu Glu Ser Pro Ala Lys Ser Ser Val Leu Val Val Gly Ile Val
    290                 295                 300

Ile Gly Val Leu Leu Leu Thr Ala Ala Ala Val Gly Gly Ala Leu Leu
305                 310                 315                 320

Trp Arg Arg Met Arg Ser Gly Leu Pro Ala Pro Trp Ile Ser Leu Arg
                325                 330                 335

Gly Asp Asp Thr Gly Val Leu Leu Pro Thr Pro Gly Glu Ala Gln Asp
            340                 345                 350

Ala Asp Leu Lys Asp Val Asn Val Ile Pro Ala Thr Ala
        355                 360                 365

<210>  29
<211>  119
<212>  PRT
<213>  Artificial Sequence

<220>
<223>  An artificially synthesized sequence

<400>  29

Met Ser Arg Ser Val Ala Leu Ala Val Leu Ala Leu Leu Ser Leu Ser
1               5                   10                  15

Gly Leu Glu Ala Ile Gln Arg Thr Pro Lys Ile Gln Val Tyr Ser Arg
            20                  25                  30

His Pro Ala Glu Asn Gly Lys Ser Asn Phe Leu Asn Cys Tyr Val Ser
        35                  40                  45

Gly Phe His Pro Ser Asp Ile Glu Val Asp Leu Leu Lys Asn Gly Glu
    50                  55                  60

Arg Ile Glu Lys Val Glu His Ser Asp Leu Ser Phe Ser Lys Asp Trp
65                  70                  75                  80

Ser Phe Tyr Leu Leu Tyr Tyr Thr Glu Phe Thr Pro Thr Glu Lys Asp
                85                  90                  95

Glu Tyr Ala Cys Arg Val Asn His Val Thr Leu Ser Gln Pro Lys Ile
            100                 105                 110

Val Lys Trp Asp Arg Asp Met
        115

<210> 30
<211> 107
<212> PRT
<213> Artificial Sequence

<220>
<223> An artificially synthesized sequence

<400> 30

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Ala
            100                 105

<210> 31
<211> 120
<212> PRT
<213> Artificial Sequence

<220>
<223> An artificially synthesized sequence

<400> 31

Gln Val Gln Leu Val Gln Ser Gly Ala Glu Val Lys Lys Pro Gly Ala
1               5                   10                  15

Ser Val Lys Val Ser Cys Lys Val Ser Gly Ile Asp Leu Thr Asn Tyr
            20                  25                  30

Ala Met Gly Trp Val Arg Gln Pro Pro Gly Lys Gly Leu Glu Trp Ile
        35                  40                  45

Gly Ile Ile Gly Ala Asp Ser Ser Thr Trp Tyr Pro Ser Trp Val Lys
    50                  55                  60

Gly Arg Phe Thr Ile Ser Lys Asp Thr Ser Lys Asn Gln Val Val Leu
65 70 75 80

Thr Met Thr Asn Met Asp Pro Val Asp Thr Ala Thr Tyr Tyr Cys Ala
85 90 95

Arg Gly Arg Phe Val Gly Tyr Thr Asn Ala Phe Asp Pro Trp Gly Gln
100 105 110

Gly Thr Leu Val Thr Val Ser Ser
115 120

<210> 32
<211> 113
<212> PRT
<213> Artificial Sequence

<220>
<223> An artificially synthesized sequence

<400> 32

Asp Ile Gln Leu Thr Gln Ser Pro Ser Ser Leu Ser Ala Ser Val Gly
1 5 10 15

Asp Arg Val Thr Ile Thr Cys Gln Ser Ser Gln Ser Val Trp Asn Asn
20 25 30

Asn Tyr Leu Ser Trp Tyr Gln Gln Lys Pro Gly Gln Pro Pro Lys Leu
35 40 45

Leu Ile Tyr Asp Ala Ser Thr Leu Ala Ser Gly Val Pro Ser Arg Phe
50 55 60

Ser Gly Ser Gly Ser Gly Thr Asp Phe Thr Leu Thr Ile Asn Ser Leu
65 70 75 80

Glu Ala Glu Asp Ala Ala Thr Tyr Tyr Cys His Gly Ser Tyr Ala Asn
85 90 95

Ser Gly Trp Tyr Asp Asn Ala Phe Gly Gly Gly Thr Glu Val Val Val
100 105 110

Lys

<210> 33
<211> 18
<212> DNA

<213> Artificial Sequence

<220>
<223> An artificially synthesized primer sequence

<400> 33
gcgtcacact ttgctatg 18

<210> 34
<211> 21
<212> DNA
<213> Artificial Sequence

<220>
<223> An artificially synthesized primer sequence

<400> 34
tgagttccac gacaccgtca c 21

<210> 35
<211> 17
<212> DNA
<213> Artificial Sequence

<220>
<223> An artificially synthesized primer sequence

<400> 35
cgcccgtcac aaagagc 17

<210> 36
<211> 360
<212> DNA
<213> Artificial Sequence

<220>
<223> An artificially synthesized sequence

<400> 36
caggtccagc tggtccagtc aggagccgag gtgaagaaac ctggggcatc agtgaaggtc 60

agctgcaaag tctccggaat cgacctgact aactacgcaa tgggatgggt gaggcagcca 120

cctggcaagg gactggagtg gattgggatc attggagctg acagctccac ctggtatccc 180

agctgggtga aaggccggtt caccatctct aaggatacaa gcaaaaacca ggtggtcctg 240

accatgacaa atatggaccc agtcgatact gccacctact attgtgctcg gggcagattc 300

gtggggtaca caaatgcctt tgatccctgg ggacagggca cactggtgac tgtctctagt 360

<210> 37
<211> 339
<212> DNA
<213> Artificial Sequence

<220>
<223> An artificially synthesized sequence

<400> 37
gacatccagc tgacacagtc tccttcatca ctgtctgcta gtgtgggaga ccgggtcacc 60

atcacatgcc agagctccca gagcgtgtgg aacaataact acctgtcctg gtatcagcag 120

aagccaggcc agccccctaa actgctgatc tacgatgcat ctacactggc cagtggggtc 180

ccctcaagat tctcaggcag cgggtccgga actgacttta ctctgaccat taattctctg 240

gaggccgaag atgccgctac ctactattgt cacggctcct atgccaatag cgggtggtat 300

gacaacgcct tcggaggagg aacagaggtg gtcgtgaaa 339

<210> 38
<211> 328
<212> PRT
<213> Artificial Sequence

<220>
<223> An artificially synthesized sequence

<400> 38

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Arg Gly Gly Pro Lys Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Ala Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Tyr Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Glu Ser Leu Ser Leu Ser Pro
325

<210> 39
<211> 107
<212> PRT
<213> Artificial Sequence

<220>
<223> An artificially synthesized sequence

<400> 39

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 40
<211> 128
<212> PRT
<213> Artificial Sequence

<220>
<223> An artificially synthesized sequence

<400> 40

Gln Val Gln Leu Val Glu Ser Gly Gly Gly Leu Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Asp Lys Ala Asn Gly Tyr Asn Ala Tyr Tyr Ala Glu
50 55 60

Ser Val Lys Gly Arg Phe Thr Ile Ser Arg Asp Asp Ser Lys Asn Ser
65 70 75 80

Ile Tyr Leu Gln Met Asn Ser Leu Lys Thr Glu Asp Thr Ala Val Tyr
85 90 95

Tyr Cys Arg Tyr Val His Tyr Thr Thr Tyr Ala Gly Phe Ser Tyr Lys
100 105 110

Phe Gly Val Asp Ala Trp Gly Gln Gly Thr Thr Val Thr Val Ser Ser
115 120 125

<210> 41

<211> 112
<212> PRT
<213> Artificial Sequence

<220>
<223> An artificially synthesized sequence

<400> 41

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Pro Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gly Gln Gly
                85                  90                  95

Thr Gln Val Pro Tyr Thr Phe Gly Gln Gly Thr Lys Leu Glu Ile Lys
            100                 105                 110

<210> 42
<211> 328
<212> PRT
<213> Artificial Sequence

<220>
<223> An artificially synthesized sequence

<400> 42

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Gly Gly Pro Lys Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Lys Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Tyr Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

```
Gln Lys Ser Leu Ser Leu Ser Pro
                325


<210>  43
<211>  107
<212>  PRT
<213>  Artificial Sequence

<220>
<223>  An artificially synthesized sequence

<400>  43

Arg Thr Val Ala Ala Pro Ser Val Phe Ile Phe Pro Pro Ser Asp Glu
1               5                   10                  15


Gln Leu Lys Ser Gly Thr Ala Ser Val Val Cys Leu Leu Asn Asn Phe
            20                  25                  30


Tyr Pro Arg Glu Ala Lys Val Gln Trp Lys Val Asp Asn Ala Leu Gln
        35                  40                  45


Ser Gly Asn Ser Gln Glu Ser Val Thr Glu Gln Asp Ser Lys Asp Ser
    50                  55                  60


Thr Tyr Ser Leu Ser Ser Thr Leu Thr Leu Ser Lys Ala Asp Tyr Glu
65                  70                  75                  80


Lys His Lys Val Tyr Ala Cys Glu Val Thr His Gln Gly Leu Ser Ser
                85                  90                  95


Pro Val Thr Lys Ser Phe Asn Arg Gly Glu Cys
            100                 105


<210>  44
<211>  20
<212>  DNA
<213>  Artificial Sequence

<220>
<223>  An artificially synthesized primer sequence

<400>  44
atgcgatgga gtttccccac                                                   20


<210>  45
<211>  22
<212>  DNA
<213>  Artificial Sequence

<220>
<223>  An artificially synthesized primer sequence
```

<400> 45
acaaataaag caatagcatc ac 22

<210> 46
<211> 126
<212> PRT
<213> Artificial Sequence

<220>
<223> An artificially synthesized sequence

<400> 46

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ala Tyr
20 25 30

Trp Met Cys Trp Val Arg Gln Ala Pro Gly Lys Gly Leu Glu Trp Thr
35 40 45

Ala Cys Ile Phe Ala Ser Ala Ile Tyr Ala Gly Ser Gly Gly Ser Thr
50 55 60

Tyr Tyr Ala Ser Trp Ala Lys Gly Arg Phe Thr Ile Ser Lys Thr Ser
65 70 75 80

Ser Thr Thr Val Thr Leu Gln Met Thr Ser Leu Thr Ala Ala Asp Thr
85 90 95

Ala Thr Tyr Phe Cys Ala Thr Gly Tyr Gly Ser Ser Gly Gly Gly Phe
100 105 110

Asp Glu Leu Trp Gly Pro Gly Thr Leu Val Thr Val Ser Ser
115 120 125

<210> 47
<211> 109
<212> PRT
<213> Artificial Sequence

<220>
<223> An artificially synthesized sequence

<400> 47

Ala Gln Val Leu Thr Gln Thr Ala Ser Ser Val Ser Ala Ala Val Gly
1 5 10 15

Gly Thr Val Thr Ile Ser Cys Gln Ser Ser Glu Ser Val Tyr Ala Asn
20 25 30

Arg Leu Ser Trp Tyr Gln Gln Lys Pro Gly Gln Pro Pro Lys Leu Leu
35 40 45

Ile Tyr Ser Ala Ser Thr Leu Pro Ser Gly Val Pro Ser Arg Phe Lys
50 55 60

Gly Ser Gly Ser Gly Thr Gln Phe Thr Leu Thr Ile Ser Asp Leu Glu
65 70 75 80

Cys Asp Asp Val Gly Thr Tyr Tyr Cys Ala Gly Leu Tyr Ser Gly Asn
85 90 95

Ile Pro Ala Phe Gly Gly Gly Thr Glu Val Val Val Lys
100 105

<210> 48
<211> 120
<212> PRT
<213> Artificial Sequence

<220>
<223> An artificially synthesized sequence

<400> 48

Gln Glu Gln Leu Val Glu Ser Gly Gly Asp Leu Val Thr Pro Gly Ala
1 5 10 15

Ser Leu Thr Leu Thr Cys Lys Ala Ser Gly Phe Ser Phe Ser Ser Ser
20 25 30

Tyr Trp Met Cys Trp Val Arg Gln Ala Pro Gly Lys Gly Leu Glu Trp
35 40 45

Ile Ala Cys Ile Tyr Ser Gly Asp Gly Ser Thr Tyr Tyr Ala Ser Trp
50 55 60

Val Asn Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Arg Glu Asn Ile Phe Gly Ser Gly Ala Leu Asn Leu Trp Gly Pro
100 105 110

Gly Thr Leu Val Thr Val Ser Ser
115 120

<210> 49
<211> 112
<212> PRT
<213> Artificial Sequence

<220>
<223> An artificially synthesized sequence

<400> 49

Gln Ala Val Val Thr Gln Thr Pro Ser Pro Val Ser Ala Ala Val Gly
1 5 10 15

Gly Thr Val Thr Ile Ser Cys His Ser Ser Lys Ser Val Tyr Ser Asn
20 25 30

Asn Arg Leu Ala Trp Tyr Gln Gln Lys Pro Gly Gln Pro Pro Lys Leu
35 40 45

Leu Ile Tyr Gln Ala Ser Thr Leu Ala Ser Gly Val Ala Ser Arg Phe
50 55 60

Ser Gly Ser Gly Ser Gly Thr Gln Phe Thr Leu Thr Ile Ser Gly Val
65 70 75 80

Gln Cys Asp Asp Ala Ala Thr Tyr Tyr Cys Gln Gly Thr Tyr Tyr Ser
85 90 95

Ser Gly Phe Tyr Phe Ala Phe Gly Gly Gly Thr Glu Val Val Val Lys
100 105 110

<210> 50
<211> 128
<212> PRT
<213> Artificial Sequence

<220>
<223> An artificially synthesized sequence

<400> 50

Gln Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Ala
20 25 30

Tyr Trp Met Cys Trp Val Arg Gln Ala Pro Gly Lys Gly Leu Glu Trp
35 40 45

Val Ala Cys Ile Phe Ala Ser Ala Ile Tyr Ala Gly Ser Gly Gly Ser
50 55 60

Thr Tyr Tyr Ala Ser Trp Ala Lys Gly Arg Leu Thr Ile Ser Lys Asp
65 70 75 80

Thr Ser Lys Asn Gln Val Val Leu Thr Met Thr Asn Met Asp Pro Val
85 90 95

Asp Thr Ala Thr Tyr Tyr Cys Ala Thr Gly Tyr Gly Ser Ser Gly Gly
100 105 110

Gly Phe Asp Glu Leu Trp Gly Gln Gly Thr Leu Val Thr Val Ser Ser
115 120 125

<210> 51
<211> 109
<212> PRT
<213> Artificial Sequence

<220>
<223> An artificially synthesized sequence

<400> 51

Glu Ile Val Leu Thr Gln Ser Pro Ala Thr Leu Ser Leu Ser Pro Gly
1 5 10 15

Glu Arg Ala Thr Leu Ser Cys Gln Ser Ser Glu Ser Val Tyr Ala Asn
20 25 30

Arg Leu Ser Trp Tyr Gln Gln Lys Pro Gly Gln Pro Pro Lys Leu Leu
35 40 45

Ile Tyr Ser Ala Ser Thr Leu Pro Ser Gly Ile Pro Ala Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Ala Gly Leu Tyr Ser Gly Asn
85 90 95

Ile Pro Ala Phe Gly Gln Gly Thr Lys Val Glu Ile Lys
100 105

<210> 52
<211> 121
<212> PRT
<213> Artificial Sequence

<220>
<223> An artificially synthesized sequence

<400> 52

Gln Val Gln Leu Val Glu Ser Gly Gly Gly Leu Val Gln Pro Gly Gly
1               5                   10                  15

Ser Leu Arg Leu Ser Cys Ser Ala Ser Gly Phe Thr Phe Ser Ser Ser
            20                  25                  30

Tyr Trp Met Cys Trp Val Arg Gln Ala Pro Gly Lys Gly Leu Glu Trp
        35                  40                  45

Val Gly Cys Ile Tyr Ser Gly Asp Gly Ser Thr Tyr Tyr Ala Ser Trp
    50                  55                  60

Val Asn Gly Arg Leu Thr Ile Ser Lys Asp Thr Ser Lys Ser Gln Val
65                  70                  75                  80

Val Leu Thr Met Thr Asn Met Asp Pro Val Asp Thr Ala Thr Tyr Tyr
                85                  90                  95

Cys Ala Arg Glu Asn Ile Phe Gly Ser Gly Ala Leu Asn Leu Trp Gly
            100                 105                 110

Gln Gly Thr Leu Val Thr Val Ser Ser
        115                 120

<210> 53
<211> 112
<212> PRT
<213> Artificial Sequence

<220>
<223> An artificially synthesized sequence

<400> 53

Asp Ile Gln Val Thr Gln Ser Pro Ser Ser Leu Ser Ala Ser Val Gly
1               5                   10                  15

Asp Arg Val Thr Ile Thr Cys His Ser Ser Lys Ser Val Tyr Ser Asn
            20                  25                  30

Asn Arg Leu Ala Trp Tyr Gln Gln Lys Pro Gly Gln Pro Pro Lys Leu
        35                  40                  45

Leu Ile Tyr Gln Ala Ser Thr Leu Ala Ser Gly Val Pro Ser Arg Phe
    50                  55                  60

Ser Gly Ser Gly Ser Gly Thr Glu Phe Thr Leu Thr Ile Ser Ser Leu
65                  70                  75                  80

Gln Pro Asp Asp Phe Ala Thr Tyr Tyr Cys Gln Gly Thr Tyr Tyr Ser
85 90 95

Ser Gly Phe Tyr Phe Ala Phe Gly Gln Gly Thr Lys Val Glu Ile Lys
100 105 110

<210> 54
<211> 118
<212> PRT
<213> Artificial Sequence

<220>
<223> An artificially synthesized sequence

<400> 54

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Phe His Val Met Ser Ser Phe Glu Ile Trp Gly Gln Gly Thr
100 105 110

Leu Val Thr Val Ser Ser
115

<210> 55
<211> 107
<212> PRT
<213> Artificial Sequence

<220>
<223> An artificially synthesized sequence

<400> 55

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Leu Gln Phe Asn Asn Asp Pro Leu
85 90 95

Thr Phe Gly Gly Gly Thr Lys Val Glu Ile Lys
100 105

<210> 56
<211> 328
<212> PRT
<213> Artificial Sequence

<220>
<223> An artificially synthesized sequence

<400> 56

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Glu Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Asp Ala Leu Pro Met Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Tyr Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Glu Ser Leu Ser Leu Ser Pro
325

<210> 57
<211> 120
<212> PRT
<213> Artificial Sequence

<220>

<223>  An artificially synthesized sequence

<400>  57

Gln Val Gln Leu Val Gln Ser Gly Ala Glu Val Lys Lys Pro Gly Ala
1               5                   10                  15

Ser Val Lys Val Ser Cys Lys Val Ser Asn Ile Val Leu Met Gln His
            20                  25                  30

Thr Tyr Gly Trp Val Arg Gln Pro Pro Gly Lys Gly Leu Glu Trp Ile
        35                  40                  45

Gly Ile Ile Gly Gly Asp Gly His Ala Trp Tyr Pro Arg Trp Val Lys
    50                  55                  60

Gly Arg Phe Thr Ile Ser Lys Asp Asn Ser Lys Asn Gln Val Val Leu
65                  70                  75                  80

Thr Met Thr Asn Met Asp Pro Val Asp Thr Ala Thr Tyr Tyr Cys Ala
                85                  90                  95

Arg Gly Gly Lys Asp Gly Phe Thr Asn Ala Trp Asp Pro Trp Gly Gln
            100                 105                 110

Gly Thr Leu Val Thr Val Ser Ser
        115                 120

<210>  58
<211>  113
<212>  PRT
<213>  Artificial Sequence

<220>
<223>  An artificially synthesized sequence

<400>  58

Asp Ile Gln Leu Thr Gln Ser Pro Ser Ser Leu Ser Ala Ser Val Gly
1               5                   10                  15

Asp Arg Val Thr Ile Thr Cys Gln Ser Ser Gln Ser Val Phe Asn Asn
            20                  25                  30

Asn Gly Leu Ser Trp Tyr Gln Gln Lys Pro Gly Gln Pro Pro Lys Leu
        35                  40                  45

Leu Ile Tyr Asp Ala Ser Thr Leu Ala Ser Gly Val Pro Ser Arg Phe
    50                  55                  60

Ser Gly Ser Gly Ser Gly Thr Asp Phe Thr Leu Thr Ile Asn Ser Leu

65 70 75 80

Glu Ala Glu Asp Ala Ala Thr Tyr Tyr Cys His Gly Ser Gln Ala Thr
85 90 95

Gln Ile Trp Trp Asp Asn Ala Phe Gly Gly Gly Thr Glu Val Val Val
100 105 110

Lys

<210> 59
<211> 120
<212> PRT
<213> Artificial Sequence

<220>
<223> An artificially synthesized sequence

<400> 59

Gln Val Gln Leu Val Gln Ser Gly Ala Glu Val Lys Lys Pro Gly Ala
1 5 10 15

Ser Val Lys Val Ser Cys Lys Val Ser Asp Ile Asp Ala Met Gln Tyr
20 25 30

Thr Tyr Gly Trp Val Arg Gln Pro Pro Gly Lys Gly Leu Glu Trp Ile
35 40 45

Gly Ile Ile Gly Glu Gly Gly Glu Val Trp Tyr Pro Tyr Trp Val Lys
50 55 60

Gly Arg Phe Thr Ile Ser Lys Asp Asn Ser Lys Asn Gln Val Val Leu
65 70 75 80

Thr Met Thr Asn Met Asp Pro Val Asp Thr Ala Thr Tyr Tyr Cys Ala
85 90 95

Arg Gly Tyr His Glu Gly Phe Val Asn Ala Trp Asp Pro Trp Gly Gln
100 105 110

Gly Thr Leu Val Thr Val Ser Ser
115 120

<210> 60
<211> 113
<212> PRT
<213> Artificial Sequence

<220>

<223> An artificially synthesized sequence

<400> 60

Asp Ile Lys Leu Thr Gln Ser Pro Ser Ser Leu Ser Ala Ser Val Gly
1 5 10 15

Asp Arg Val Thr Ile Thr Cys Gln Ser Ser Gln Ser Val Leu Trp Asn
20 25 30

Asn Tyr Leu Ser Trp Tyr Gln Gln Lys Pro Gly Gln Pro Pro Lys Leu
35 40 45

Leu Ile Tyr Asp Ala Ser Thr Leu Ala Ser Gly Val Pro Ser Arg Phe
50 55 60

Ser Gly Ser Gly Ser Gly Thr Asp Phe Thr Leu Thr Ile Asn Ser Leu
65 70 75 80

Glu Ala Glu Asp Ala Ala Thr Tyr Tyr Cys His Gly Ser Gln Phe Glu
85 90 95

Gln Trp Val Phe Asp Asn Ala Phe Gly Gly Gly Thr Glu Val Val Val
100 105 110

Lys

<210> 61
<211> 120
<212> PRT
<213> Artificial Sequence

<220>
<223> An artificially synthesized sequence

<400> 61

Gln Val Gln Leu Val Gln Ser Gly Ala Glu Val Lys Lys Pro Gly Ala
1 5 10 15

Ser Val Lys Val Ser Cys Lys Val Ser Asp Ile Gln Asp Glu Gln Tyr
20 25 30

Ala Phe Gly Trp Val Arg Gln Pro Pro Gly Lys Gly Leu Glu Trp Ile
35 40 45

Gly Ile Ile Gly Ala Thr Gly His Val Trp Tyr Pro Gln Trp Val Lys
50 55 60

Gly Arg Phe Thr Ile Ser Lys Asp Asn Ser Lys Asn Gln Val Val Leu

65 70 75 80

Thr Met Thr Asn Met Asp Pro Val Asp Thr Ala Thr Tyr Tyr Cys Ala
85 90 95

Arg Gly His Lys Gln His Phe Thr Asn Ala Phe Asp Pro Trp Gly Gln
100 105 110

Gly Thr Leu Val Thr Val Ser Ser
115 120

<210> 62
<211> 112
<212> PRT
<213> Artificial Sequence

<220>
<223> An artificially synthesized sequence

<400> 62

Asp Ile Gln Leu Thr Gln Ser Pro Ser Ser Leu Ser Ala Ser Val Gly
1 5 10 15

Asp Arg Val Thr Ile Thr Cys Gln Ser Ser Gln Ser Val Tyr Lys Asn
20 25 30

Asn Tyr Leu Ser Trp Tyr Gln Gln Lys Pro Gly Gln Pro Pro Lys Leu
35 40 45

Leu Ile Tyr Asp Ala Ser Thr Leu Ala Ser Gly Val Pro Ser Arg Phe
50 55 60

Ser Gly Ser Gly Ser Gly Thr Asp Phe Thr Leu Thr Ile Asn Ser Leu
65 70 75 80

Glu Ala Glu Asp Ala Ala Thr Tyr Tyr Cys His Gly Ala Tyr Leu Tyr
85 90 95

Leu Gly Val Asp Asn Ala Phe Gly Gly Gly Thr Glu Val Val Val Lys
100 105 110

<210> 63
<211> 328
<212> PRT
<213> Artificial Sequence

<220>
<223> An artificially synthesized sequence

<400> 63

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255
```

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro
325

<210> 64
<211> 120
<212> PRT
<213> Artificial Sequence

<220>
<223> An artificially synthesized sequence

<400> 64

Gln Val Gln Leu Val Gln Ser Gly Ala Glu Val Lys Lys Pro Gly Ala
1 5 10 15

Ser Val Lys Val Ser Cys Lys Val Ser Gly Ile Asp Leu Thr Asn Tyr
20 25 30

Ala Met Gly Trp Val Arg Gln Pro Pro Gly Lys Gly Leu Glu Trp Ile
35 40 45

Gly Ile Ile Gly Ala Asp Ser Ser Thr Trp Tyr Pro Ser Trp Val Lys
50 55 60

Gly Arg Phe Thr Ile Ser Lys Asp Asn Ser Lys Asn Gln Val Val Leu
65 70 75 80

Thr Met Thr Asn Met Asp Pro Val Asp Thr Ala Thr Tyr Tyr Cys Ala
85 90 95

Arg Gly Arg Phe Val Gly Tyr Thr Asn Ala Phe Asp Pro Trp Gly Gln
100 105 110

Gly Thr Leu Val Thr Val Ser Ser
115 120

<210> 65
<211> 6
<212> PRT
<213> Artificial Sequence

<220>
<223> An artificially synthesized sequence

<220>
<221> MISC_FEATURE
<222> (1)..(1)
<223> Xaa can be any amino acid

<220>
<221> MISC_FEATURE
<222> (3)..(3)
<223> Xaa can be any amino acid

<400> 65

Xaa Ala Xaa Trp Met Cys
1               5

<210> 66
<211> 23
<212> PRT
<213> Artificial Sequence

<220>
<223> An artificially synthesized sequence

<220>
<221> MISC_FEATURE
<222> (5)..(7)
<223> Xaa can be any amino acid

<220>
<221> MISC_FEATURE
<222> (9)..(10)
<223> Xaa can be any amino acid

<400> 66

Cys Ile Phe Ala Xaa Xaa Xaa Tyr Xaa Xaa Ser Gly Gly Ser Thr Tyr
1               5                   10                  15

Tyr Ala Ser Trp Ala Lys Gly
            20

<210> 67
<211> 12
<212> PRT
<213> Artificial Sequence

<220>
<223> An artificially synthesized sequence

<220>
<221> MISC_FEATURE
<222> (2)..(2)
<223> Xaa can be any amino acid

<220>
<221> MISC_FEATURE
<222> (4)..(6)
<223> Xaa can be any amino acid

<220>
<221> MISC_FEATURE
<222> (8)..(9)
<223> Xaa can be any amino acid

<400> 67

Gly Xaa Gly Xaa Xaa Xaa Gly Xaa Xaa Asp Glu Leu
1               5                   10

<210> 68
<211> 12
<212> PRT
<213> Artificial Sequence

<220>
<223> An artificially synthesized sequence

<220>
<221> MISC_FEATURE
<222> (5)..(5)
<223> Xaa can be any amino acid

<220>
<221> MISC_FEATURE
<222> (7)..(10)
<223> Xaa can be any amino acid

<400> 68

Gln Ser Ser Glu Xaa Val Xaa Xaa Xaa Xaa Leu Ser
1               5                   10

<210> 69
<211> 7
<212> PRT
<213> Artificial Sequence

<220>
<223> An artificially synthesized sequence

<220>
<221> MISC_FEATURE
<222> (1)..(1)
<223> Xaa can be any amino acid

<220>
<221> MISC_FEATURE
<222> (3)..(3)

```
<223>  Xaa can be any amino acid

<220>
<221>  MISC_FEATURE
<222>  (5)..(5)
<223>  Xaa can be any amino acid

<220>
<221>  MISC_FEATURE
<222>  (7)..(7)
<223>  Xaa can be any amino acid

<400>  69

Xaa Ala Xaa Thr Xaa Pro Xaa
1               5


<210>  70
<211>  10
<212>  PRT
<213>  Artificial Sequence

<220>
<223>  An artificially synthesized sequence


<220>
<221>  MISC_FEATURE
<222>  (5)..(5)
<223>  Xaa can be any amino acid

<400>  70

Ala Gly Leu Tyr Xaa Gly Asn Ile Pro Ala
1               5                   10


<210>  71
<211>  23
<212>  PRT
<213>  Artificial Sequence

<220>
<223>  An artificially synthesized sequence


<220>
<221>  MISC_FEATURE
<222>  (5)..(5)
<223>  Xaa is Alanine, Aspartic acid, Glutamic acid, Glycine, Histidine,
       Isoleucine, Lysine, Leucine, Methionine, Asparagine, Proline,
       Glutamine, Arginine, Serine, Threonine, or Valine

<220>
<221>  MISC_FEATURE
<222>  (6)..(7)
<223>  Xaa can be any amino acid

<220>
<221>  MISC_FEATURE
<222>  (9)..(10)
<223>  Xaa can be any amino acid
```

<400> 71

Cys Ile Phe Ala Xaa Xaa Xaa Tyr Xaa Xaa Ser Gly Gly Ser Thr Tyr
1               5                   10                  15

Tyr Ala Ser Trp Ala Lys Gly
            20

<210> 72
<211> 12
<212> PRT
<213> Artificial Sequence

<220>
<223> An artificially synthesized sequence

<220>
<221> MISC_FEATURE
<222> (5)..(5)
<223> Xaa can be any amino acid

<220>
<221> MISC_FEATURE
<222> (7)..(8)
<223> Xaa can be any amino acid

<220>
<221> MISC_FEATURE
<222> (9)..(9)
<223> Xaa is Alanine, Aspartic acid, Glutamic acid, Glycine, Histidine, Isoleucine, Lysine, Leucine, Methionine, Asparagine, Proline, Glutamine, Arginine, Serine, Threonine, or Valine

<220>
<221> MISC_FEATURE
<222> (10)..(10)
<223> Xaa can be any amino acid

<400> 72

Gln Ser Ser Glu Xaa Val Xaa Xaa Xaa Xaa Leu Ser
1               5                   10

<210> 73
<211> 7
<212> PRT
<213> Artificial Sequence

<220>
<223> An artificially synthesized sequence

<220>
<221> MISC_FEATURE
<222> (1)..(2)
<223> Xaa can be any amino acid

<220>

<221> MISC_FEATURE
<222> (4)..(4)
<223> Xaa can be any amino acid

<400> 73

Xaa Xaa Ala Xaa Trp Met Cys
1 5

<210> 74
<211> 6
<212> PRT
<213> Artificial Sequence

<220>
<223> An artificially synthesized sequence

<220>
<221> MISC_FEATURE
<222> (1)..(3)
<223> Xaa can be any amino acid

<400> 74

Xaa Xaa Xaa Trp Met Cys
1 5

<210> 75
<211> 17
<212> PRT
<213> Artificial Sequence

<220>
<223> An artificially synthesized sequence

<220>
<221> MISC_FEATURE
<222> (3)..(3)
<223> Xaa can be any amino acid

<220>
<221> MISC_FEATURE
<222> (5)..(8)
<223> Xaa can be any amino acid

<220>
<221> MISC_FEATURE
<222> (10)..(10)
<223> Xaa can be any amino acid

<400> 75

Cys Ile Xaa Ser Xaa Xaa Xaa Xaa Thr Xaa Tyr Ala Ser Trp Val Asn
1 5 10 15

Gly

<210> 76
<211> 11
<212> PRT
<213> Artificial Sequence

<220>
<223> An artificially synthesized sequence

<220>
<221> MISC_FEATURE
<222> (2)..(5)
<223> Xaa can be any amino acid

<400> 76

Glu Xaa Xaa Xaa Xaa Ser Gly Ala Leu Asn Leu
1 5 10

<210> 77
<211> 13
<212> PRT
<213> Artificial Sequence

<220>
<223> An artificially synthesized sequence

<220>
<221> MISC_FEATURE
<222> (5)..(5)
<223> Xaa can be any amino acid

<220>
<221> MISC_FEATURE
<222> (7)..(11)
<223> Xaa can be any amino acid

<400> 77

His Ser Ser Lys Xaa Val Xaa Xaa Xaa Xaa Xaa Leu Ala
1 5 10

<210> 78
<211> 7
<212> PRT
<213> Artificial Sequence

<220>
<223> An artificially synthesized sequence

<220>
<221> MISC_FEATURE
<222> (1)..(1)
<223> Xaa can be any amino acid

<220>
<221> MISC_FEATURE
<222> (3)..(4)

<223> Xaa can be any amino acid

<400> 78

Xaa Ala Xaa Xaa Leu Ala Ser
1 5

<210> 79
<211> 12
<212> PRT
<213> Artificial Sequence

<220>
<223> An artificially synthesized sequence

<220>
<221> MISC_FEATURE
<222> (5)..(8)
<223> Xaa can be any amino acid

<400> 79

Gln Gly Thr Tyr Xaa Xaa Xaa Xaa Phe Tyr Phe Ala
1 5 10

<210> 80
<211> 5
<212> PRT
<213> Artificial Sequence

<220>
<223> An artificially synthesized sequence

<220>
<221> MISC_FEATURE
<222> (1)..(1)
<223> Xaa is Alanine, Aspartic acid, Glutamic acid, Phenylalanine, Glycine, Histidine, Isoleucine, Lysine, Leucine, Asparagine, Glutamine, Arginine, Serine, Threonine, Valine, Tryptophan, or Tyrosine

<220>
<221> MISC_FEATURE
<222> (2)..(2)
<223> Xaa is Aspartic acid, Glutamic acid, Phenylalanine, Histidine, Lysine, Asparagine, Proline, Arginine, or Tyrosine

<220>
<221> MISC_FEATURE
<222> (3)..(3)
<223> Xaa is Alanine, Isoleucine, Proline, Threonine, or Valine

<220>
<221> MISC_FEATURE
<222> (4)..(4)
<223> Xaa is Alanine, Glutamic acid, Phenylalanine, Histidine, Isoleucine, Lysine, Leucine, Methionine, Asparagine, Glutamine, Serine, Threonine, Valine, Tryptophan, or Tyrosine

<400> 80

```
Xaa Xaa Xaa Xaa Gly
1               5
```

<210> 81
<211> 16
<212> PRT
<213> Artificial Sequence

<220>
<223> An artificially synthesized sequence

<220>
<221> MISC_FEATURE
<222> (1)..(1)
<223> Xaa is Aspartic acid, Isoleucine, or Valine

<220>
<221> MISC_FEATURE
<222> (4)..(4)
<223> Xaa is Alanine, Aspartic acid, Glutamic acid, Glycine, Isoleucine, Lysine, Glutamine, or Arginine

<220>
<221> MISC_FEATURE
<222> (5)..(5)
<223> Xaa is Aspartic acid, Glutamic acid, Phenylalanine, Glycine, Histidine, Isoleucine, Lysine, Leucine, Proline, Glutamine, Arginine, Serine, Threonine, Valine, Tryptophan, or Tyrosine

<220>
<221> MISC_FEATURE
<222> (6)..(6)
<223> Xaa is Alanine, Aspartic acid, Glutamic acid, Phenylalanine, Glycine, Histidine, or Serine

<220>
<221> MISC_FEATURE
<222> (7)..(7)
<223> Xaa is Alanine, Aspartic acid, Glutamic acid, Phenylalanine, Glycine, Histidine, Isoleucine, Lysine, Leucine, Asparagine, Glutamine, Arginine, Serine, Threonine, Valine, Tryptophan, or Tyrosine

<220>
<221> MISC_FEATURE
<222> (8)..(8)
<223> Xaa is Alanine, Aspartic acid, Glutamic acid, Glycine, Histidine, Isoleucine, Lysine, Leucine, Asparagine, Proline, Glutamine, Arginine, Serine, Threonine, or Valine

<220>
<221> MISC_FEATURE
<222> (10)..(10)
<223> Xaa is Alanine, Aspartic acid, Glutamic acid, Phenylalanine, Glycine, Histidine, Isoleucine, Lysine, Leucine, Glutamine, Arginine, Serine, Threonine, Valine, Tryptophan, or Tyrosine

<220>
<221> MISC_FEATURE

<222> (12)..(12)
<223> Xaa is Alanine, Phenylalanine, Glutamine, Arginine, Serine, Threonine, Valine, Tryptophan, or Tyrosine

<220>
<221> MISC_FEATURE
<222> (16)..(16)
<223> Xaa is Alanine, Phenylalanine, or Glycine

<400> 81

Xaa Ile Gly Xaa Xaa Xaa Xaa Xaa Trp Xaa Pro Xaa Trp Val Lys Xaa
1               5                   10                  15

<210> 82
<211> 12
<212> PRT
<213> Artificial Sequence

<220>
<223> An artificially synthesized sequence

<220>
<221> MISC_FEATURE
<222> (2)..(2)
<223> Xaa is Alanine, Glutamic acid, Phenylalanine, Glycine, Histidine, Lysine, Leucine, Glutamine, Arginine, Serine, Threonine, Tryptophan, or Tyrosine

<220>
<221> MISC_FEATURE
<222> (3)..(3)
<223> Xaa is Phenylalanine, Histidine, Lysine, Asparagine, Tryptophan, or Tyrosine

<220>
<221> MISC_FEATURE
<222> (4)..(4)
<223> Xaa is Alanine, Aspartic acid, Glutamic acid, Phenylalanine, Glycine, Histidine, Isoleucine, Lysine, Leucine, Asparagine, Proline, Glutamine, Arginine, Serine, Threonine, Valine, Tryptophan, or Tyrosine

<220>
<221> MISC_FEATURE
<222> (5)..(5)
<223> Xaa is Alanine, Aspartic acid, Glutamic acid, Glycine, Histidine, Glutamine, or Serine

<220>
<221> MISC_FEATURE
<222> (6)..(6)
<223> Xaa is Phenylalanine or Tyrosine

<220>
<221> MISC_FEATURE
<222> (7)..(7)
<223> Xaa is Asparagine, Threonine, or Valine

<220>
<221> MISC_FEATURE

<222> (10)..(10)
<223> Xaa is Phenylalanine or Tryptophan

<400> 82

Gly Xaa Xaa Xaa Xaa Xaa Xaa Asn Ala Xaa Asp Pro
1 5 10

<210> 83
<211> 13
<212> PRT
<213> Artificial Sequence

<220>
<223> An artificially synthesized sequence

<220>
<221> MISC_FEATURE
<222> (7)..(7)
<223> Xaa is Alanine, Glutamic acid, Phenylalanine, Histidine, Isoleucine, Lysine, Leucine, Asparagine, Arginine, Serine, Threonine, Valine, Tryptophan, or Tyrosine

<220>
<221> MISC_FEATURE
<222> (8)..(8)
<223> Xaa is Alanine, Aspartic acid, Glutamic acid, Phenylalanine, Glycine, Histidine, Isoleucine, Lysine, Leucine, Asparagine, Proline, Glutamine, Arginine, Serine, Threonine, Valine, Tryptophan, or Tyrosine

<220>
<221> MISC_FEATURE
<222> (11)..(11)
<223> Xaa is Alanine, Glutamic acid, Phenylalanine, Glycine, Histidine, Serine, or Tyrosine

<400> 83

Gln Ser Ser Gln Ser Val Xaa Xaa Asn Asn Xaa Leu Ser
1 5 10

<210> 84
<211> 7
<212> PRT
<213> Artificial Sequence

<220>
<223> An artificially synthesized sequence

<400> 84

Asp Ala Ser Thr Leu Ala Ser
1 5

<210> 85
<211> 13
<212> PRT
<213> Artificial Sequence

<220>
<223> An artificially synthesized sequence

<220>
<221> MISC_FEATURE
<222> (3)..(3)
<223> Xaa is Alanine, Serine, or Threonine

<220>
<221> MISC_FEATURE
<222> (4)..(4)
<223> Xaa is Alanine, Aspartic acid, Glutamic acid, Phenylalanine, Glycine, Histidine, Isoleucine, Lysine, Leucine, Methionine, Asparagine, Glutamine, Arginine, Serine, Threonine, Valine, Tryptophan, or Tyrosine

<220>
<221> MISC_FEATURE
<222> (5)..(5)
<223> Xaa is Alanine, Aspartic acid, Glutamic acid, Phenylalanine, Glycine, Histidine, Leucine, Asparagine, Glutamine, Arginine, Serine, Threonine, Valine, or Tyrosine

<220>
<221> MISC_FEATURE
<222> (6)..(6)
<223> Xaa is Alanine, Glutamic acid, Phenylalanine, Glycine, Histidine, Isoleucine, Lysine, Leucine, Asparagine, Proline, Glutamine, Arginine, Serine, Threonine, Valine, Tryptophan, or Tyrosine

<220>
<221> MISC_FEATURE
<222> (7)..(7)
<223> Xaa is Alanine, Aspartic acid, Glutamic acid, Phenylalanine, Glycine, Histidine, Isoleucine, Lysine, Leucine, Asparagine, Proline, Glutamine, Arginine, Serine, Threonine, Valine,Tryptophan, Tyrosine

<220>
<221> MISC_FEATURE
<222> (8)..(8)
<223> Xaa is Alanine, Aspartic acid, Glutamic acid, Phenylalanine, Glycine, Histidine, Isoleucine, Lysine, Leucine, Asparagine, Proline, Glutamine, Arginine, Valine, Tryptophan, or Tyrosine

<220>
<221> MISC_FEATURE
<222> (9)..(9)
<223> Xaa is Alanine, Aspartic acid, Glutamic acid, Phenylalanine, Glycine, Histidine, Isoleucine, Lysine, Leucine, Asparagine, Proline, Glutamine, Arginine, Serine, Threonine, Valine, Tryptophan, or Tyrosine

<220>
<221> MISC_FEATURE
<222> (10)..(10)
<223> Xaa is Alanine, Phenylalanine, Histidine, Isoleucine, Lysine, Leucine, Asparagine, Proline, Glutamine, Arginine, Serine, Threonine, Valine, Tryptophan, or Tyrosine

<220>

<221> MISC_FEATURE
<222> (13)..(13)
<223> Xaa is Alanine or Glycine

<400> 85

His Gly Xaa Xaa Xaa Xaa Xaa Xaa Xaa Xaa Asp Asn Xaa
1 5 10

<210> 86
<211> 328
<212> PRT
<213> Artificial Sequence

<220>
<223> An artificially synthesized sequence

<400> 86

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro
325

<210> 87
<211> 107
<212> PRT
<213> Artificial Sequence

<220>
<223> An artificially synthesized sequence

<400> 87

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

```
Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105


<210>  88
<211>  18
<212>  DNA
<213>  Artificial Sequence

<220>
<223>  An artificially synthesized primer sequence

<400>  88
gttttccgcc tcggcgct                                                      18


<210>  89
<211>  19
<212>  DNA
<213>  Artificial Sequence

<220>
<223>  An artificially synthesized primer sequence

<400>  89
gaataccgat gggcccttg                                                     19
```

## Description

[Technical Field]

The present disclosure relates to an antigen-binding molecule comprising an antigen-binding domain whose antigen-binding activity changes in a methylthioadenosine (MTA)-dependent manner, a method of producing or screening for the antigen-binding domain or the antigen-binding molecule, a library for obtaining the antigen-binding molecule or the antigen-binding domain and a method for designing the same, and a pharmaceutical composition comprising the antigen-binding molecule. The present disclosure also relates to a method of designing a library for efficiently acquiring an antigen-binding domain whose antigen-binding activity changes depending on a small molecule compound. Furthermore, the present disclosure relates to an antigen-binding molecule that specifically binds to MTA, and to a method for measuring MTA concentration and a method for diagnosing a disease that uses the antigen-binding molecule.

[Background Art]

Antibodies are drawing attention as pharmaceuticals as they are highly stable in plasma and have few side effects. In particular, a number of IgG-type antibody pharmaceuticals are available on the market, and many antibody pharmaceuticals are currently under development (NPL 1 and 2).

As cancer therapeutic agents using antibody pharmaceuticals, Rituxan against a CD20 antigen, cetuximab against an EGFR antigen, herceptin against a HER2 antigen, and such have been approved so far (NPL 3). These antibody molecules bind to antigens expressed on cancer cells, and exhibit cytotoxic activity against cancer cells by ADCC and such. Such cytotoxic activity by ADCC and etc. are known to depend on the number of antigens expressed on cells targeted by the therapeutic antibodies (NPL 4); therefore, high expression level of the target antigen is preferable from the stand point of the effects of the therapeutic antibodies. However, even if the antigen expression level is high, when antigens are expressed in normal tissues, cytotoxic activity mediated by ADCC etc. will be exerted against normal cells, and therefore side-effects will become a major problem. Therefore, antigens targeted by therapeutic antibodies used as therapeutic agents for cancer are preferably antigens specifically expressed in cancer cells.

Following the success of antibody pharmaceuticals that exert cytotoxic activity by ADCC activity, a second generation of improved antibody molecules that exert strong cytotoxic activity through enhancement of ADCC activity by removing fucose of *N*-type sugar chains in the native human IgG1 Fc region (NPL 5), enhancement of ADCC activity by enhancing the binding toward FcγRIIIa by substitution of amino acids in the native human IgG1 Fc region (NPL 6), and such have been reported. As antibody pharmaceuticals that exert cytotoxic activity against cancer cells through a mechanism other than the above-mentioned ADCC activity mediated by NK cells, improved antibody molecules that exert a stronger cytotoxic activity, such as an antibody-drug conjugate (ADC) in which an antibody is conjugated with a drug having potent cytotoxic activity (NPL 7), and a low molecular weight antibody that exerts toxic activity against cancer cells by recruiting T cells to cancer cells (NPL 8), have been reported as well.

Such antibody molecules exerting a stronger cytotoxic activity can exert cytotoxic activity against cancer cells that do not have much antigen expression, but on the other hand, they will exert similar cytotoxic activity against normal tissues with low antigen expression. In fact, in comparison to cetuximab which is a natural human IgG1 against an EGFR antigen, EGFR-BiTE, which is a bispecific antibody against CD3 and EGFR, can exert a potent cytotoxic activity against cancer cells by recruiting T cells to cancer cells and exert antitumor effects. On the other hand, since EGFR is expressed also in normal tissues, when EGFR-BiTE is administered to cynomolgus monkeys, serious side effects have appeared (NPL 9). Furthermore, bivatuzumab mertansine, an ADC formed by linking mertansine to an antibody against CD44v6 which is highly expressed in cancer cells, has been shown to cause severe skin toxicity and liver toxicity in clinical practice because CD44v6 is expressed also in normal tissues (NPL 10).

When antibodies that can exert a potent cytotoxic activity against cancer cells having low antigen expression are used as such, the target antigen needs to be expressed in a highly cancer-specific manner. However, since HER2 and EGFR, which are target antigens of herceptin and cetuximab, respectively, are also expressed in normal tissues, the number of cancer antigens expressed in a highly cancer-specific manner is thought to be limited. Therefore, while it is possible to strengthen the cytotoxic activity against cancer, the side effects occurring due to cytotoxic actions against normal tissues may become problematic.

Furthermore, recently, ipilimumab which enhances tumor immunity by inhibiting CTLA4 which contributes to immunosuppression in cancer was shown to prolong overall survival of metastatic melanoma (NPL 11). However, since ipilimumab inhibits CTLA4 systemically, while tumor immunity is enhanced, the emergence of autoimmune disease-like severe side effects due to systemic activation of the immune system is becoming a problem (NPL 12).

Various techniques have been developed as techniques that can be applied to second-generation antibody pharmaceuticals. While techniques for improving effector functions, antigen-binding ability, pharmacokinetics, and stability, or techniques for reducing immunogenic risks have been reported (NPL 13), there are hardly any reports on

techniques that enable disease tissue-specific action of antibody pharmaceuticals to overcome such side effects. For example, regarding lesions such as cancer tissues and inflammatory tissues, pH-dependent antibodies that make use of the acidic pH condition at these disease tissues have been reported (PTL 1 and 2). However, the decrease of pH (that is, increase in hydrogen ion concentration) in cancer tissues and inflammatory tissues as compared to normal tissues is slight, and since it is difficult to produce antibodies that act by detecting a slight increase in the concentration of hydrogen ions which have an extremely small molecular weight, and also because acidic pH conditions may be found in normal tissues such as osteoclastic bone resorption region or in tissues other than the lesion of interest, use of pH conditions as a lesion-specific environmental factor was considered to face many challenges. On the other hand, methods for producing antibodies that exert antigen-binding activity only after they are cleaved by a protease expressed at lesion sites such as cancer tissues and inflammatory tissues have been reported (PTL 3). However, since cleavage of antibodies by proteases is irreversible, when the antibodies that have been cleaved at the lesion site enter the blood stream and return to normal tissues, they can bind to the antigens in normal tissues as well, and this is considered to be a problem. Furthermore, cancer specificity of such proteases is also thought to have problems that need to be addressed. In order to overcome such problems, antigen-binding molecules have been reported whose binding activity against an antigen changes depending on the concentration of disease tissue-specific compounds (PTL 4 and 5).

[Citation List]

[Patent Literature]

[PTL 1] WO2003/105757
[PTL 2] WO2012/033953
[PTL 3] WO2010/081173
[PTL 4] WO2013/180200
[PTL 5] WO2015/083764

[Non-Patent Literature]

[NPL 1] Monoclonal antibody successes in the clinic. Janice M Reichert, Clark J Rosensweig, Laura B Faden & Matthew C Dewitz, Nat. Biotechnol. (2005) 23, 1073 - 1078

[NPL 2] The therapeutic antibodies market to 2008. Pavlou AK, Belsey MJ., Eur. J. Pharm. Biopharm. (2005) 59 (3), 389-396

[NPL 3] Monoclonal antibodies: versatile platforms for cancer immunotherapy. Weiner LM, Surana R, Wang S., Nat. Rev. Immunol. (2010) 10 (5), 317-327

[NPL 4] Differential responses of human tumor cell lines to anti-p185HER2 monoclonal antibodies. Lewis GD, Figari I, Fendly B, Wong WL, Carter P, Gorman C, Shepard HM, Cancer Immunol. Immunotherapy (1993) 37, 255-263

[NPL 5] Non-fucosylated therapeutic antibodies as next-generation therapeutic antibodies. Satoh M, Iida S, Shitara K., Expert Opin. Biol. Ther. (2006) 6 (11), 1161-1173

[NPL 6] Optimizing engagement of the immune system by anti-tumor antibodies: an engineer's perspective. Desjarlais JR, Lazar GA, Zhukovsky EA, Chu SY, Drug Discov. Today (2007) 12 (21-22), 898-910

[NPL 7] Antibody-drug conjugates: targeted drug delivery for cancer. Alley SC, Okeley NM, Senter PD., Curr. Opin. Chem. Biol. (2010) 14 (4), 529-537

[NPL 8] BiTE: Teaching antibodies to engage T-cells for cancer therapy. Baeuerle PA, Kufer P, Bargou R., Curr. Opin. Mol. Ther. (2009) 11 (1), 22-30

[NPL 9] T cell-engaging BiTE antibodies specific for EGFR potently eliminate KRAS- and BRAF-mutated colorectal cancer cells. Lutterbuese R, Raum T, Kischel R, Hoffmann P, Mangold S, Rattel B, Friedrich M, Thomas O, Lorenczewski G, Rau D, Schaller E, Herrmann I, Wolf A, Urbig T, Baeuerle PA, Kufer P., Proc. Natl. Acad. Sci. U.S.A. (2010) 107 (28), 12605-12610 [NPL 10] Phase I trial with the CD44v6-targeting immunoconjugate bivatuzumab mertansine in head and neck squamous cell carcinoma. Riechelmann H, Sauter A, Golze W, Hanft G, Schroen C, Hoermann K, Erhardt T, Gronau S., Oral Oncol. (2008) 44 (9), 823-829

[NPL 11] Ipilimumab in the treatment of melanoma. Trinh VA, Hwu WJ., Expert Opin. Biol. Ther., (2012) Apr 14 (doi:10.1517/14712598.2012.675325)

[NPL 12] IPILIMUMAB - A NOVEL IMMUNOMODULATING THERAPY CAUSING AUTOIMMUNE HYPOPHYSITIS: A CASE REPORT AND REVIEW. Juszczak A, Gupta A, Karavitaki N, Middleton MR, Grossman A., Eur. J. Endocrinol.

(2012) Apr 10 (doi: 10.1530/EJE-12-0167)
[NPL 13] Antibody engineering for the development of therapeutic antibodies. Kim SJ, Park Y, Hong HJ., Mol. Cells. (2005) 20 (1), 17-29

[Summary of Invention]

[Technical Problem]

An objective of the present disclosure is to newly discover a small molecule compound that is specifically present or produced in a diseased tissue, and further, to provide an antigen-binding molecule whose binding to a target antigen is controlled depending on the small molecule compound (small molecule compound switch antigen-binding molecule). A further objective is to provide a method for efficiently obtaining such an antigen-binding molecule in a short period of time.

[Solution to Problem]

As a result of extensive research to achieve the above objectives, the present inventors newly discovered methylthioadenosine (MTA) as a small molecule compound specific to cancer tissues, and further created antigen-binding molecules comprising an antigen-binding domain whose antigen-binding activity changes in an MTA-dependent manner. In addition, the present inventors discovered that the antigen-binding molecules or pharmaceutical compositions comprising the antigen-binding molecules are useful for cancer treatment, as well as for cancer treatment including administration of the antigen-binding molecules, and that the antigen-binding molecules are useful in the manufacture of medicaments for treating cancer.

The present inventors also created a method of producing and screening for an antigen-binding domain whose antigen-binding activity changes in an MTA-dependent manner. In addition, the present inventors succeeded in creating a library that allows efficient screening for an antigen-binding domain whose antigen-binding activity changes in an MTA-dependent manner. The present inventors also succeeded in creating a library that allows screening for an antigen-binding domain whose antigen-binding activity changes depending on MTA and/or a small molecule compound other than MTA, and also created a method of screening for and producing an antigen-binding domain whose antigen-binding activity changes depending on MTA and/or a small molecule compound other than MTA.

Further, the present inventors succeeded in obtaining an antigen-binding molecule which specifically binds to MTA itself.

The present disclosure is based on such findings, and specifically encompasses the embodiments exemplified below.

[A1] An antigen-binding molecule comprising an antigen-binding domain whose antigen-binding activity changes in a 5'-methylthioadenosine (MTA)-dependent manner.

[A2] An antigen-binding molecule in which the antigen-binding activity of the antigen-binding domain in the presence of MTA is different from the antigen-binding activity in the absence of MTA.

[A3] The antigen-binding molecule according to [A1] or [A2], wherein the antigen-binding activity of the antigen-binding domain comprised in the antigen-binding molecule is substantially unaffected by adenosine.

[A4] The antigen-binding molecule according to [A1] to [A3], wherein the antigen-binding activity of the antigen antigen-binding domain comprised in the antigen-binding molecule is substantially unaffected by S-(5'-adenosyl)-L-homocysteine (SAH).

[A5] The antigen-binding molecule according to [A1] to [A4], wherein the antigen-binding activity of the antigen-binding domain comprised in the antigen-binding molecule is substantially unaffected by AMP, ADP, or ATP.

[A6] The antigen-binding molecule according to [A1] or [A2], wherein the antigen-binding activity of the antigen-binding domain comprised in the antigen-binding molecule changes also in an adenosine-dependent manner.

[A7] The antigen-binding molecule according to [A1], [A2], or [A6], wherein the antigen-binding activity of the antigen-binding domain comprised in the antigen-binding molecule also changes in an S-(5'-adenosyl)-L-homocysteine (SAH)-dependent manner.

[A8] The antigen-binding molecule according to [A1], [A2], [A6], or [A7], wherein the antigen-binding activity of the antigen binding domain comprised in the antigen-binding molecule also changes in an AMP, ADP, and/or ATP-dependent manner.

[A9] The antigen-binding molecule according to any one of [A1] to [A8], wherein the antigen-binding domain comprises an antibody variable region and/or a single-domain antibody.

[A10] The antigen-binding molecule according to any one of [A1] to [A9], wherein the antigen-binding molecule is an antibody.

[A11] The antigen-binding molecule according to any one of [A1] to [A10], which comprises an antibody Fc region.
[A12] The antigen-binding molecule according to [A11], wherein the antibody Fc region is a native Fc region or a modified Fc region.
[A13] The antigen-binding molecule according to any one of [A1] to [A12], wherein the antigen-binding activity of the antigen-binding domain in the presence of MTA is stronger than the antigen-binding activity of the antigen-binding domain in the absence of MTA.
[A14] The antigen-binding molecule according to any one of [A1] to [A12], wherein the antigen-binding activity of the antigen-binding domain in the presence of MTA is weaker than the antigen-binding activity of the antigen-binding domain in the absence of MTA.
[A15] The antigen-binding molecule according to any one of [A1] to [A14], wherein the antigen-binding domain has an amino acid residue that interacts with MTA.
[A16] The antigen-binding molecule according to [A15], wherein the amino acid residue that interacts with MTA does so in a state where it is bound to the antigen of the antigen-binding domain.
[A17] The antigen-binding molecule according to[A15] or [A16], wherein the antigen-binding domain comprises an antibody variable region or a single-domain antibody, and the amino acid residue that interacts with MTA is located in the antibody variable region or in the CDR of the single-domain antibody.
[A18] The antigen-binding molecule according to any one of [A15] to [A17], wherein the antigen-binding domain is an antibody variable region, and the amino acid residue that interacts with MTA is an amino acid residue located at at least one or more amino acid sites selected from the group of amino acid sites specified by Kabat numbering of heavy chain positions 34, 35a, 47, 52, 52e, and 101, and light chain positions 32, 34, 36, 46, 49, 50, 89, 90, 91, and 96, in the amino acid sequence of the antibody variable region.
[A19] The antigen-binding molecule according to any one of [A15] to [A18], wherein the antigen-binding domain is an antibody variable region, and the antibody variable region comprises at least one or more amino acids selected from heavy chain W34, C35a, W47, F52, Y52e, and E101, and light chain R32, S34, Y36, L46, Y49, S50, A89, G90, L91, and P96 (Kabat numbering).
[A20] The antigen-binding molecule according to any one of [A1] to [A19], wherein the antigen-binding domain is an antibody variable region, and the antibody variable region comprises at least one or more amino acids selected from the group of amino acids below (Kabat numbering): any of A, C, D, E, F, G, H, I, K, L, M, N, P, Q, R, S, T, V, W, or Y located at position 30 of the heavy chain;

any of A, C, D, E, F, G, H, I, K, L, M, N, P, Q, R, S, T, V, W, or Y located at position 31 of the heavy chain;
A located at position 32 of the heavy chain;
any of A, C, D, E, F, G, H, I, K, L, M, N, P, Q, R, S, T, V, W, or Y located at position 33 of the heavy chain;
W located at position 34 of the heavy chain;
M located at position 35 of the heavy chain;
C located at position 35a of the heavy chain;
C located at position 50 of the heavy chain;
I located at position 51 of the heavy chain;
F located at position 52 of the heavy chain;
A located at position 52a of the heavy chain;
any of A, D, E, G, H, I, K, L, M, N, P, Q, R, S, T, or V located at position 52b of the heavy chain;
any of A, C, D, E, F, G, H, I, K, L, M, N, P, Q, R, S, T, V, W, or Y located at position 52c of the heavy chain;
any of A, C, D, E, F, G, H, I, K, L, M, N, P, Q, R, S, T, V, W, or Y located at position 52d of the heavy chain;
Y located at position 52e of the heavy chain;
any of A, C, D, E, F, G, H, I, K, L, M, N, P, Q, R, S, T, V, W, or Y located at position 52f of the heavy chain;
any of A, C, D, E, F, G, H, I, K, L, M, N, P, Q, R, S, T, V, W, or Y located at position 52g of the heavy chain;
S located at position 53 of the heavy chain;
G located at position 54 of the heavy chain;
G located at position 55 of the heavy chain;
S located at position 56 of the heavy chain;
T located at position 57 of the heavy chain;
Y located at position 58 of the heavy chain;
Y located at position 59 of the heavy chain;
A located at position 60 of the heavy chain;
S located at position 61 of the heavy chain;
W located at position 62 of the heavy chain;
A located at position 63 of the heavy chain;
K located at position 64 of the heavy chain;

G located at position 65 of the heavy chain;
G located at position 95 of the heavy chain;
any of A, C, D, E, F, G, H, I, K, L, M, N, P, Q, R, S, T, V, W, or Y located at position 96 of the heavy chain;
G located at position 97 of the heavy chain;
any of A, C, D, E, F, G, H, I, K, L, M, N, P, Q, R, S, T, V, W, or Y located at position 98 of the heavy chain;
any of A, C, D, E, F, G, H, I, K, L, M, N, P, Q, R, S, T, V, W, or Y located at position 99 of the heavy chain;
any of A, C, D, E, F, G, H, I, K, L, M, N, P, Q, R, S, T, V, W, or Y located at position 100 of the heavy chain;
G located at position 100a of the heavy chain;
any of A, C, D, E, F, G, H, I, K, L, M, N, P, Q, R, S, T, V, W, or Y located at position 100b of the heavy chain;
any of A, C, D, E, F, G, H, I, K, L, M, N, P, Q, R, S, T, V, W, or Y located at position 100c of the heavy chain;
E located at position 101 of the heavy chain;
L located at position 102 of the heavy chain;
Q located at position 24 of the light chain;
S located at position 25 of the light chain;
S located at position 26 of the light chain;
E located at position 27 of the light chain;
any of A, C, D, E, F, G, H, I, K, L, M, N, P, Q, R, S, T, V, W, or Y located at position 27a of the light chain;
V located at position 28 of the light chain;
any of A, C, D, E, F, G, H, I, K, L, M, N, P, Q, R, S, T, V, W, or Y located at position 29 of the light chain;
any of A, C, D, E, F, G, H, I, K, L, M, N, P, Q, R, S, T, V, W, or Y located at position 30 of the light chain;
any of A, D, E, G, H, I, K, L, M, N, P, Q, R, S, T, or V located at position 31 of the light chain; any of A, C, D, E, F, G, H, I, K, L, M, N, P, Q, R, S, T, V, W, or Y located at position 32 of the light chain;
L located at position 33 of the light chain;
S located at position 34 of the light chain;
any of A, C, D, E, F, G, H, I, K, L, M, N, P, Q, R, S, T, V, W, or Y located at position 49 of the light chain;
any of A, C, D, E, F, G, H, I, K, L, M, N, P, Q, R, S, T, V, W, or Y located at position 50 of the light chain;
A located at position 51 of the light chain;
any of A, C, D, E, F, G, H, I, K, L, M, N, P, Q, R, S, T, V, W, or Y located at position 52 of the light chain;
T located at position 53 of the light chain;
any of A, C, D, E, F, G, H, I, K, L, M, N, P, Q, R, S, T, V, W, or Y located at position 54 of the light chain;
P located at position 55 of the light chain;
any of A, C, D, E, F, G, H, I, K, L, M, N, P, Q, R, S, T, V, W, or Y located at position 56 of the light chain;
A located at position 89 of the light chain;
G located at position 90 of the light chain;
L located at position 91 of the light chain;
Y located at position 92 of the light chain;
any of A, C, D, E, F, G, H, I, K, L, M, N, P, Q, R, S, T, V, W, or Y located at position 93 of the light chain;
G located at position 94 of the light chain;
N located at position 95 of the light chain;
I located at position 95a of the light chain;
P located at position 96 of the light chain; and
A located at position 97 of the light chain.

[A21] The antigen-binding molecule according to any one of [A15] to [A17], wherein the antigen-binding domain is an antibody variable region, and the amino acid residue that interacts with MTA is an amino acid residue located at at least one or more amino acid sites selected from the group of amino acid sites specified by Kabat numbering of heavy chain positions 34, 47, 50, 58, 95, 98, 99, and 100a, and light chain positions 28, 91, 95b, 95c, and 96 in the amino acid sequence of the antibody variable region.
[A22] The antigen-binding molecule according to any one of [A15] to [A17] and [A21], wherein the antigen-binding molecule is an antibody variable region, and the antibody variable region comprises at least one or more amino acids selected from heavy chain W34, W47, C50, Y58, E95, F98, G99, and G100a, and light chain Y28, T91, F95b, Y95c, and F96 (Kabat numbering).
[A23] The antigen-binding molecule according to any one of [A1] to [A17], or [A21] to [A22], wherein the antigen-binding domain is an antibody variable region, and the antibody variable region comprises at least one or more amino acids selected from the group of amino acids below (Kabat numbering):

any of A, C, D, E, F, G, H, I, K, L, M, N, P, Q, R, S, T, V, W, or Y located at position 31 of the heavy chain;
any of A, C, D, E, F, G, H, I, K, L, M, N, P, Q, R, S, T, V, W, or Y located at position 32 of the heavy chain;

any of A, C, D, E, F, G, H, I, K, L, M, N, P, Q, R, S, T, V, W, or Y located at position 33 of the heavy chain;
W located at position 34 of the heavy chain;
M located at position 35 of the heavy chain;
C located at position 35a of the heavy chain;
C located at position 50 of the heavy chain;
I located at position 51 of the heavy chain;
any of A, C, D, E, F, G, H, I, K, L, M, N, P, Q, R, S, T, V, W, or Y located at position 52 of the heavy chain;
S located at position 52a of the heavy chain;
any of A, C, D, E, F, G, H, I, K, L, M, N, P, Q, R, S, T, V, W, or Y located at position 53 of the heavy chain;
any of A, C, D, E, F, G, H, I, K, L, M, N, P, Q, R, S, T, V, W, or Y located at position 54 of the heavy chain;
any of A, C, D, E, F, G, H, I, K, L, M, N, P, Q, R, S, T, V, W, or Y located at position 55 of the heavy chain;
any of A, C, D, E, F, G, H, I, K, L, M, N, P, Q, R, S, T, V, W, or Y located at position 56 of the heavy chain;
T located at position 57 of the heavy chain;
any of A, C, D, E, F, G, H, I, K, L, M, N, P, Q, R, S, T, V, W, or Y located at position 58 of the heavy chain;
Y located at position 59 of the heavy chain;
A located at position 60 of the heavy chain;
S located at position 61 of the heavy chain;
W located at position 62 of the heavy chain;
V located at position 63 of the heavy chain;
N located at position 64 of the heavy chain;
G located at position 65 of the heavy chain;
E located at position 95 of the heavy chain;
any of A, C, D, E, F, G, H, I, K, L, M, N, P, Q, R, S, T, V, W, or Y located at position 96 of the heavy chain;
any of A, C, D, E, F, G, H, I, K, L, M, N, P, Q, R, S, T, V, W, or Y located at position 97 of the heavy chain;
any of A, C, D, E, F, G, H, I, K, L, M, N, P, Q, R, S, T, V, W, or Y located at position 98 of the heavy chain;
any of A, C, D, E, F, G, H, I, K, L, M, N, P, Q, R, S, T, V, W, or Y located at position 99 of the heavy chain;
S located at position 100 of the heavy chain;
G located at position 100a of the heavy chain;
A located at position 100b of the heavy chain;
L located at position 100c of the heavy chain;
N located at position 101 of the heavy chain;
L located at position 102 of the heavy chain;
H located at position 24 of the light chain;
S located at position 25 of the light chain;
S located at position 26 of the light chain;
K located at position 27 of the light chain;
any of A, C, D, E, F, G, H, I, K, L, M, N, P, Q, R, S, T, V, W, or Y located at position 27a of the light chain;
V located at position 27b of the light chain;
any of A, C, D, E, F, G, H, I, K, L, M, N, P, Q, R, S, T, V, W, or Y located at position 28 of the light chain;
any of A, C, D, E, F, G, H, I, K, L, M, N, P, Q, R, S, T, V, W, or Y located at position 29 of the light chain;
any of A, C, D, E, F, G, H, I, K, L, M, N, P, Q, R, S, T, V, W, or Y located at position 30 of the light chain;
any of A, C, D, E, F, G, H, I, K, L, M, N, P, Q, R, S, T, V, W, or Y located at position 31 of the light chain;
any of A, C, D, E, F, G, H, I, K, L, M, N, P, Q, R, S, T, V, W, or Y located at position 32 of the light chain;
L located at position 33 of the light chain;
A located at position 34 of the light chain;
any of A, C, D, E, F, G, H, I, K, L, M, N, P, Q, R, S, T, V, W, or Y located at position 49 of the light chain;
any of A, C, D, E, F, G, H, I, K, L, M, N, P, Q, R, S, T, V, W, or Y located at position 50 of the light chain;
A located at position 51 of the light chain;
any of A, C, D, E, F, G, H, I, K, L, M, N, P, Q, R, S, T, V, W, or Y located at position 52 of the light chain;
any of A, C, D, E, F, G, H, I, K, L, M, N, P, Q, R, S, T, V, W, or Y located at position 53 of the light chain;
L located at position 54 of the light chain;
A located at position 55 of the light chain;
S located at position 56 of the light chain;
Q located at position 89 of the light chain;
G located at position 90 of the light chain;
T located at position 91 of the light chain;
Y located at position 92 of the light chain;
any of A, C, D, E, F, G, H, I, K, L, M, N, P, Q, R, S, T, V, W, or Y located at position 93 of the light chain;

any of A, C, D, E, F, G, H, I, K, L, M, N, P, Q, R, S, T, V, W, or Y located at position 94 of the light chain;
any of A, C, D, E, F, G, H, I, K, L, M, N, P, Q, R, S, T, V, W, or Y located at position 95 of the light chain;
any of A, C, D, E, F, G, H, I, K, L, M, N, P, Q, R, S, T, V, W, or Y located at position 95a of the light chain;
F located at position 95b of the light chain;
Y located at the 95c position of the light chain;
F located at position 96 of the light chain; and
A located at position 97 of the light chain.

[A24] The antigen-binding molecule according to any one of [A15] to [A17], wherein the antigen-binding domain is an antibody variable region, and the amino acid residue that interacts with MTA is an amino acid residue located at at least one or more amino acid sites selected from the group of amino acid sites specified by Kabat numbering of heavy chain positions 33, 50, 52, 54, 56, 57, 58, 99, 100, and 100a, and light chain positions 91, 95c, and 96 in the amino acid sequence of the antibody variable region.
[A25] The antigen-binding molecule according to any one of [A15] to [A17] and [A24], wherein the antigen-binding domain is an antibody variable region, and the antibody variable region comprises at least one or more amino acids selected from heavy chain A33, 150, G52, D54, S56, T57, W58, G99, Y100, T100a, and light chain S91, Y95c, and N96 (Kabat numbering).
[A26] The antigen-binding molecule according to any one of [A1] to [A17] or [A24] to[A25], wherein the antigen-binding domain is an antibody variable region, and the antibody variable region comprises at least one or more amino acids selected from the group of amino acids below (Kabat numbering):

any of A, D, E, F, G, H, I, K, L, M, N, P, Q, R, S, T, V, W, or Y located at position 26 of the heavy chain;
any of A, D, E, F, G, H, I, K, L, M, N, P, Q, R, S, T, V, W, or Y located at position 28 of the heavy chain;
either A or L located at position 29 of the heavy chain;
any of A, D, E, F, G, H, I, K, L, M, N, P, Q, R, S, T, V, W, or Y located at position 30 of the heavy chain;
any of A, D, E, F, G, H, I, K, L, N, Q, R, S, T, V, W, or Y located at position 31 of the heavy chain;
any of D, E, F, H, N, P, R, or Y located at position 32 of the heavy chain;
any of A, I, P, T, or V located at position 33 of the heavy chain;
any of A, E, F, H, I, K, L, M, N, Q, S, T, V, W, or Y located at position 34 of the heavy chain; G located at position 35 of the heavy chain;
any of D, I, or V located at position 50 of the heavy chain;
I located at position 51 of the heavy chain;
G located at position 52 of the heavy chain;
any of A, D, E, G, I, K, Q, or R located at position 53 of the heavy chain;
any of D, E, F, G, H, I, K, L, P, Q, R, S, T, V, W, or Y located at position 54 of the heavy chain; any of A, D, E, F, G, or H located at position 55 of the heavy chain;
any of A, D, E, F, G, H, I, K, L, N, Q, R, S, T, V, W, or Y located at position 56 of the heavy chain;
any of A, D, E, G, H, I, K, L, N, P, Q, R, S, T, or V located at position 57 of the heavy chain;
W located at position 58 of the heavy chain;
any of A, D, E, F, G, H, I, K, L, Q, R, S, T, V, W, or Y located at position 59 of the heavy chain;
P located at position 60 of the heavy chain;
any of A, F, Q, R, S, T, V, W, or Y located at position 61 of the heavy chain;
W located at position 62 of the heavy chain;
V located at position 63 of the heavy chain;
K located at position 64 of the heavy chain;
A, F, or G located at position 65 of the heavy chain;
G located at position 95 of the heavy chain;
any of A, E, F, G, H, K, L, Q, R, S, T, W, or Y located at position 96 of the heavy chain;
any of A, F, H, K, N, W, or Y located at position 97 of the heavy chain;
any of A, D, E, F, G, H, I, K, L, N, P, Q, R, S, T, V, W, or Y located at position 98 of the heavy chain;
any of A, D, E, G, H, Q, or S located at position 99 of the heavy chain;
F or Y located at position 100 of the heavy chain;
N, T, or V located at position 100a of the heavy chain
N located at position 100b of the heavy chain;
A located at position 100c of the heavy chain;
F or W located at position 100d of the heavy chain;
D located at position 101 of the heavy chain;
P located at position 102 of the heavy chain;

Q located at position 24 of the light chain;
S located at position 25 of the light chain;
S located at position 26 of the light chain;
Q located at position 27 of the light chain;
S located at position 27e of the light chain;
V located at position 27f of the light chain;
any of A, E, F, H, I, K, L, N, R, S, T, V, W, or Y located at position 28 of the light chain;
any of A, D, E, F, G, H, I, K, L, N, P, Q, R, S, T, V, W, or Y located at position 29 of the light chain;
N located at position 30 of the light chain;
N located at position 31 of the light chain;
any of A, E, F, G, H, S, or Y located at position 32 of the light chain;
L located at position 33 of the light chain;
S located at position 34 of the light chain;
D located at position 50 of the light chain;
A located at position 51 of the light chain;
S located at position 52 of the light chain;
T located at position 53 of the light chain;
L located at position 54 of the light chain;
A located at position 55 of the light chain;
S located at position 56 of the light chain;
H located at position 89 of the light chain;
G located at position 90 of the light chain;
any of A, S, or T located at position 91 of the light chain;
any of A, D, E, F, G, H, I, K, L, M, N, Q, R, S, T, V, W, or Y located at position 92 of the light chain;
any of A, D, E, F, G, H, L, N, Q, R, S, T, V, or Y located at position 93 of the light chain;
any of A, E, F, G, H, I, K, L, N, P, Q, R, S, T, V, W, or Y located at position 94 of the light chain;
any of A, D, E, F, G, H, I, K, L, N, P, Q, R, S, T, V, W, or Y located at position 95 of the light chain;
any of A, D, E, F, G, H, I, K, L, N, P, Q, R, V, W, or Y located at position 95a of the light chain;
any of A, D, E, F, G, H, I, K, L, N, P, Q, R, S, T, V, W, or Y located at position 95b of the light chain;
any of A, F, H, I, K, L, N, P, Q, R, S, T, V, W, or Y located at position 95c of the light chain;
D located at position 96d of the light chain;
N located at position 96 of the light chain;
A or G located at position 97 of the light chain; and
A, F, I, L, or V located at position 98 of the light chain.

[A27] The antigen binding molecule according to any one of [A1] to [A26], wherein the antigen is a molecule other than MTA, or a molecule other than MTA and having immunogenicity in the human body.
[A28] The antigen-binding molecule according to any one of [A1] to [A27], wherein the antigen is any of a peptide, a polypeptide, or a protein.
[A29] The antigen-binding molecule according to any one of [A1] to [A28], wherein the antigen-binding molecule further has a second antigen-binding domain, and the second antigen-binding domain has a binding activity to a second antigen different from the antigen to which the antigen-binding domain binds.
[A30] The antigen-binding molecule according to [A29], wherein the binding activity of the second antigen-binding domain to the second antigen is substantially unaffected by MTA.
[A31] The antigen-binding molecule according to [A29], wherein the binding activity of the second antigen-binding domain to the second antigen changes in an MTA-dependent manner.
[A32] The antigen-binding molecule according to [A31], wherein the binding activity of the second antigen-binding domain to the second antigen in the presence of MTA is different from the binding activity of the second antigen-binding domain to the second antigen in the absence of MTA.
[A33] The antigen-binding molecule according to any one of [A1] to [A32], wherein the antigen is a membrane-type molecule or a soluble-type molecule.
[A34] The antigen-binding molecule according to [A33], wherein the antigen is a membrane-type molecule and is expressed in a diseased tissue.
[A35] The antigen-binding molecule according to [A33], wherein the antigen is a soluble-type molecule and is expressed in cancer tissue.
[A36] The antigen-binding molecule according to [A35], wherein the antigen expressed in cancer tissue is an antigen expressed in a cancer cell, or an antigen expressed in a cancer stromal cell in a cancer tissue or in an immune tissue.
[A37] The antigen-binding molecule according to [A35] or [A36], wherein the cancer tissue is a cancer tissue in

which MTA is accumulated.

[A38] The antigen-binding molecule according to any one of [A35] to [A37], wherein the cancer tissue is a cancer tissue in which the gene encoding MTA phosphorylase (MTAP) is absent or underexpressed, or which has a mutation or splicing variant that reduces enzyme activity.

[A39] The antigen-binding molecule according to any one of [A35] to [A37], wherein the cancer tissue is a cancer tissue in which the activity of MTAP is lost or decreased.

[A40] The antigen-binding molecule according to any one of [A1] to [A39], wherein the antigen is a membrane-type molecule, and the antigen-binding molecule exhibits cytotoxic activity on a cell expressing the antigen.

[A41] The antigen-binding molecule according to [A40], which exhibits at least one or more cytotoxic activities selected from ADCC activity, ADCP activity, or CDC activity.

[A42] The antigen-binding molecule according to [A1] to [A39], which has an agonistic activity against the antigen.

[A43] The antigen-binding molecule according to any one of [A29] to [A32], wherein one of the antigen and the second antigen is an antigen expressed in a target cell, and the other is an antigen expressed in an effector cell.

[A44] The antigen-binding molecule according to [A43], wherein the target cell is a cancer cell.

[A45] The antigen-binding molecule according to [A44], wherein the cancer cell is a cancer cell in which the gene encoding MTAP is absent or underexpressed, or which has a mutation or splicing variant that reduces enzyme activity, or is a cancer cell that is present around a cancer cell in which the gene encoding MTAP is absent or underexpressed, or which has a mutation or splicing variant that reduces enzyme activity.

[A46] The antigen-binding molecule according to [A43], wherein the target cell is a cell other than a cancer cell that is present around a cancer cell in which the gene encoding MTAP is absent or underexpressed, or which has a mutation or splicing variant that reduces enzyme activity.

[A47] The antigen-binding molecule according to [A43], wherein the cell other than the cancer cell that is present around a cancer cell is cancer-associated fibroblast (CAF) or tumor associated macrophage (TAM).

[A48] The antigen-binding molecule according to any one of [A43] to [47], wherein the effector cell is a T cell.

[A49] The antigen-binding molecule according to [A48], wherein the antigen expressed in the effector cell is a T cell receptor (TCR) complex.

[A50] The antigen-binding molecule according to either [A48] or [A49], wherein the antigen expressed in the effector cell is CD3.

[A51] The antigen-binding molecule according to any one of [A48] to [A50], which elicits cytotoxic activity against a target cell by activating an effector cell.

[A52] The antigen-binding molecule according to any one of [A48] to [A51], which has TDCC activity.

[A53] The antigen-binding molecule according to any one of [A1] to [A39], wherein the antigen is a soluble-type molecule, and the antigen-binding molecule exhibits neutralizing activity against the antigen.

[A54] The antigen-binding molecule according to any one of [A1] to [A53], wherein KD value of the antigen-binding domain for the antigen in the absence of MTA and KD value of the antigen-binding domain for the antigen in the presence of MTA are different.

[A55] A pharmaceutical composition comprising the antigen-binding molecule according to any one of [A1] to [A54].

[A56] A pharmaceutical composition for treating cancer comprising the antigen-binding molecule according to any one of [A1] to [A55] as an active ingredient.

[A57] The pharmaceutical composition according to [A56], wherein the cancer is a cancer in which MTA is accumulated in a tissue.

[A58] The pharmaceutical composition according to any one of [A56] to [A57], wherein the cancer is a cancer in which the gene encoding MTAP is absent or underexpressed, or which has a mutation or splicing variant that reduces enzyme activity.

[A59] The pharmaceutical composition according to any one of [A56] to [A58], wherein the cancer is a cancer in which the activity of MTAP is absent or decreased.

[A60] A method of producing the antigen-binding molecule according to any one of [A1] to [A54].

[A61] A polynucleotide encoding the antigen-binding molecule according to any one of [A1] to [A54].

[A62] A vector comprising the polynucleotide according to [A61].

[A63] A cell carrying the vector according to [A62].

[A64] An antigen-binding molecule obtained by culturing the cell according [A63] and recovering it from the culture supernatant.

[G1] The antigen binding molecule according to any one of [A1] to [A54], which has a high plasma retention property and/or low plasma antigen-accumulating ability as compared to a control antigen-binding molecule that does not bind in an MTA concentration-dependent manner.

[G2] A pharmaceutical preparation comprising the antigen-binding molecule according to [G1] and a pharmaceutically acceptable carrier.

[G3] A method of producing an antigen-binding molecule having a high plasma retention property and/or a low

plasma antigen-accumulating ability as compared to a control antigen-binding molecule, wherein the method comprises: (a) producing an antigen-binding molecule whose antigen-binding activity increases with the increase of MTA concentration, and (b) measuring the plasma retention property and/or plasma antigen-accumulating ability of the antigen-binding molecule produced in (a).

The present disclosure also encompasses embodiments exemplified below.

[B1] A library consisting of antigen-binding molecules comprising a plurality of antigen-binding domains differing in sequence from one another, and/or nucleic acids encoding a plurality of antigen-binding molecules comprising antigen-binding domains differing in sequence from one another, wherein the library mainly consists of antigen-binding molecules comprising an antigen-binding domain having amino acid residues that interact with MTA, and/or nucleic acids encoding the antigen-binding molecules.
[B2] The library according to [B1], wherein the antigen-binding domains are antibody variable regions.
[B3] The library according to [B2], wherein the library comprises a plurality of antibody variable region variants differing in sequence from one another and having amino acids different to amino acids located at one or more amino acid sites in an unmodified antibody variable region having a binding activity towards MTA and/or nucleic acids encoding a plurality of antibody variable region variants differing in sequence from one another and having amino acids different to amino acids located in one or more amino acid sites in an unmodified antibody variable region having a binding activity to MTA.
[B4] The library according to [B3], wherein the amino acid sites in the antibody variable region variants having amino acids different to the unmodified antibody variable region are one or more amino acid sites selected from the following group of amino acid sites:

1) an amino acid site corresponding to an amino acid site in the unmodified antibody variable region that is not involved in the binding to MTA,
2) an amino acid sites that does not significantly weaken the binding of the antibody variable region variant to MTA as compared to the antibody variable region variant, and
3) an amino acid site that is likely to contribute to the MTA-dependent binding of the antibody variable region variant to the antigen.

[B5] The library according to [B3], wherein the amino acid sites in the antibody variable region variants having amino acids different to the unmodified antibody variable region are one or more amino acid sites selected from the following group of amino acid sites:

1) an amino acid site corresponding to an amino acid site in the unmodified antibody variable region that is not involved in the binding to MTA,
2) an amino acid site that does not significantly weaken the binding of the antibody variable region variant to MTA as compared to the antibody variable region variant,
3) an amino acid site corresponding to an amino acid site that is exposed on the surface of the unmodified antibody variable region, and
4) an amino acid site corresponding to an amino acid site located in a region where the rate of structural change is large at the time of MTA binding/non-binding in the unmodified antibody variable region.

[B6] The library according any one of [B3] to [B5], wherein the unmodified antibody variable region does not substantially bind to adenosine and/or S-(5'-adenosyl)-L-homocysteine (SAH).
[B7] The library according to any one of [B3] to [B6], wherein the unmodified antibody variable region is any of the following:

a) an antibody variable region comprising the heavy chain variable region set forth in SEQ ID NO: 46 and the light chain variable region set forth in SEQ ID NO: 47;
b) an antibody variable region comprising the heavy chain variable region set forth in SEQ ID NO: 50 and the light chain variable region set forth in SEQ ID NO: 51;
c) an antibody variable region comprising the heavy chain variable region set forth in SEQ ID NO: 48 and the light chain variable region set forth in SEQ ID NO: 49;
d) an antibody variable region comprising the heavy chain variable region set forth in SEQ ID NO: 52 and the light chain variable region set forth in SEQ ID NO: 53.

[B8] The library according to [B2], wherein the plurality of antibody variable regions are antibody variable regions

comprising the following:

H-CDR1 comprising XAXWMC (SEQ ID NO: 65);
H-CDR2 comprising CIFAXXXYXXSGGSTYYASWAKG (SEQ ID NO: 66);
H-CDR3 comprising GXGXXXGXXDEL (SEQ ID NO: 67);
L-CDR1 comprising QSSEXVXXXXLS (SEQ ID NO: 68);
L-CDR2 comprising XAXTXPX (SEQ ID NO: 69); and
L-CDR3 comprising AGLYXGNIPA (SEQ ID NO: 70);

wherein X refers to an arbitrary amino acid and X existing at different positions does not have to be the same type of amino acid.

[B9] The library according to [B2], wherein the plurality of antibody variable regions are antibody variable regions comprising the following:

H-CDR1 comprising XAXWMC (SEQ ID NO: 65);
H-CDR2 comprising CIFA$X_1$XXYXXSGGSTYYASWAKG (SEQ ID NO: 71);
H-CDR3 comprising GXGXXXGXXDEL (SEQ ID NO: 67);
L-CDR1 comprising QSSEXVXXX$X_1$XLS (SEQ ID NO: 72);
L-CDR2 comprising XAXTXPX (SEQ ID NO: 69); and
L-CDR3 comprising AGLYXGNIPA (SEQ ID NO: 70);

wherein X is an arbitrary amino acid,
$X_1$ is an amino acid selected from A, D, E, G, H, I, K, L, M, N, P, Q, R, S, T, and V, and
X or $X_1$ existing at different positions does not have to be the same type of amino acid.

[B10] The library according to [B2], wherein the plurality of antibody variable regions are antibody variable regions comprising the following:

H-CDR1 comprising XXAXWMC (SEQ ID NO: 73);
H-CDR2 comprising CIFA$X_1$XXYXXSGGSTYYASWAKG (SEQ ID NO: 71);
H-CDR3 comprising GXGXXXGXXDEL (SEQ ID NO: 67);
L-CDR1 comprising QSSEXVXXX$X_1$XLS (SEQ ID NO: 72);
L-CDR2 comprising XAXTXPX (SEQ ID NO: 69); and
L-CDR3 comprising AGLYXGNIPA (SEQ ID NO: 70);

wherein X is an arbitrary amino acid,
$X_1$ is an amino acid selected from A, D, E, G, H, I, K, L, M, N, P, Q, R, S, T, and V, and
X or $X_1$ existing at different positions does not have to be the same type of amino acid.

[B11] The library according to [B2], where in the plurality of antibody variable regions are antibody variable regions comprising the following:

H-CDR1 comprising XXXWMC (SEQ ID NO: 74);
H-CDR2 comprising CIXSXXXXTXYASWVNG (SEQ ID NO: 75);
H-CDR3 comprising EXXXXSGALNL (SEQ ID NO: 76);
L-CDR1 comprising HSSKXVXXXXXLA (SEQ ID NO: 77);
L-CDR2 comprising XAXXLAS (SEQ ID NO: 78); and
L-CDR3 comprising QGTYXXXXFYFA (SEQ ID NO: 79);

wherein X refers to an arbitrary amino acid, and
X existing at different positions does not have to be the same type of amino acid.

[B12] The library according to any one of [B3] to [B5], wherein the unmodified antibody variable region also has a binding activity to adenosine.

[B13] The library according to [B12], wherein the unmodified antibody variable region also has a binding activity to S-(5'-adenosyl)-L-homocysteine (SAH), AMP, ADP, and/or ATP.

[B14] The library according to any one of [B3] to [B5] and [B12], wherein the unmodified antibody variable region is an antibody variable region having the heavy chain variable region set forth in SEQ ID NO: 31 and the light chain

variable region set forth in SEQ ID NO: 32.
[B15] The library according to [B2], wherein the plurality of antibody variable regions are antibody variable regions comprising the following:

H-CDR1 comprising $X_2X_3X_4X_5G$ (SEQ ID NO: 80);
H-CDR2 comprising $X_6IGX_7X_8X_9X_{10}X_{11}WX_{12}PX_{13}WVKX_{14}$ (SEQ ID NO: 81);
H-CDR3 comprising $GX_{15}X_{16}X_{17}X_{18}X_{19}X_{20}NAX_{21}DP$ (SEQ ID NO: 82);
L-CDR1 comprising $QSSQSVX_{22}X_{23}NNX_{24}LS$ (SEQ ID NO: 83);
L-CDR2 comprising DASTLAS (SEQ ID NO: 84); and
L-CDR3 comprising $HGX_{25}X_{26}X_{27}X_{28}X_{29}X_{30}X_{31}X_{32}DNX_{33}$ (SEQ ID NO: 85); wherein,
$X_2$ is an amino acid selected from A, D, E, F, G, H, I, K, L, N, Q, R, S, T, V, W, and Y,
$X_3$ is an amino acid selected from D, E, F, H, K, N, P, R, and Y,
$X_4$ is an amino acid selected from A, I, P, T, and V,
$X_5$ is an amino acid selected from A, E, F, H, I, K, L, M, N, Q, S, T, V, W, and Y,
$X_6$ is an amino acid selected from D, I, and V,
$X_7$ is an amino acid selected from A, D, E, G, I, K, Q, and R,
$X_8$ is an amino acid selected from D, E, F, G, H, I, K, L, P, Q, R, S, T, V, W, and Y,
$X_9$ is an amino acid selected from A, D, E, F, G, H, and S,
$X_{10}$ is an amino acid selected from A, D, E, F, G, H, I, K, L, N, Q, R, S, T, V, W, and Y,
$X_{11}$ is an amino acid selected from A, D, E, G, H, I, K, L, N, P, Q, R, S, T, and V,
$X_{12}$ is an amino acid selected from A, D, E, F, G, H, I, K, L, Q, R, S, T, V, W, and Y,
$X_{13}$ is an amino acid selected from A, F, Q, R, S, T, V, W, and Y,
$X_{14}$ is an amino acid selected from A, F, and G,
$X_{15}$ is an amino acid selected from A, E, F, G, H, K, L, Q, R, S, T, W, and Y,
$X_{16}$ is an amino acid selected from F, H, K, N, W, and Y,
$X_{17}$ is an amino acid selected from A, D, E, F, G, H, I, K, L, N, P, Q, R, S, T, V, W, and Y,
$X_{18}$ is an amino acid selected from A, D, E, G, H, Q, and S,
$X_{19}$ is an amino acid selected from F and Y,
$X_{20}$ is an amino acid selected from N, T, and V,
$X_{21}$ is an amino acid selected from F and W,
$X_{22}$ is an amino acid selected from A, E, F, H, I, K, L, N, R, S, T, V, W, and Y,
$X_{23}$ is an amino acid selected from A, D, E, F, G, H, I, K, L, N, P, Q, R, S, T, V, W, and Y,
$X_{24}$ is an amino acid selected from A, E, F, G, H, S, and Y,
$X_{25}$ is an amino acid selected from A, S, and T,
$X_{26}$ is an amino acid selected from A, D, E, F, G, H, I, K, L, M, N, Q, R, S, T, V, W, and Y,
$X_{27}$ is an amino acid selected from A, D, E, F, G, H, L, N, Q, R, S, T, V, and Y,
$X_{28}$ is an amino acid selected from A, E, F, G, H, I, K, L, N, P, Q, R, S, T, V, W, and Y,
$X_{29}$ is an amino acid selected from A, D, E, F, G, H, I, K, L, N, P, Q, R, S, T, V, W, Y,
$X_{30}$ is an amino acid selected from A, D, E, F, G, H, I, K, L, N, P, Q, R, V, W, and Y,
$X_{31}$ is an amino acid selected from A, D, E, F, G, H, I, K, L, N, P, Q, R, S, T, V, W, and Y,
$X_{32}$ is an amino acid selected from A, F, H, I, K, L, N, P, Q, R, S, T, V, W, and Y, and
$X_{33}$ is an amino acid selected from A and G.

[B16] A library, wherein nucleic acids encoding antigen-binding molecules comprising an antigen-binding domain that binds to MTA are concentrated from the library according to [B15].
[B17] The library according to [B16], wherein the binding of the antigen-binding domain to MTA is a binding to MTA in the absence of an antigen.
[B18] The library according to [B16] or [B17], wherein the concentration comprises the following steps (1) to (2):

(1) contacting an antigen-binding domain displayed by the library according to [B15] with MTA, and
(2) selecting an antigen-binding domain that bound to MTA in the above step (1).

[B19] A library, wherein nucleic acids encoding antigen-binding domains that bind to adenosine are concentrated from the library according to [B15].
[B20] The library according to [B19], wherein the binding of the antigen-binding domain to adenosine is a binding to adenosine in the absence of an antigen.
[B21] The library according to [B19] or [B20], wherein the concentration comprises the following steps (1) to (2):

(1) contacting an antigen-binding domain displayed by the library according to [B15] with adenosine, and
(2) selecting an antigen-binding domain that bound to adenosine in the above step (1).

The present disclosure also encompasses embodiments exemplified below.

[C1] A method of producing a library comprising the following steps (a) and (b):

(a) identifying an amino acid site that satisfies at least one or more of the following (i) to (vi) in an antigen-binding domain having MTA-binding activity:

(i) an amino acid site exposed on the surface of the antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs; or
(vi) an amino acid site that is not important for the formation of a canonical structure; and,

(b) designing a library comprising a nucleic acid encoding an unmodified antigen-binding domain, and nucleic acids encoding a plurality of variants of the antigen-binding domain differing in sequence from one another and having an amino acid modification at one or more amino acid sites identified in step (a).

[C2] The production method according to [C1], wherein the amino acid modification in step (b) satisfies at least one or more of the following (1) to (3):

(1) when the structure is compared between when the antigen-binding domain variant having the amino acid modification is bound to MTA and when it is not bound to MTA, the rate of structural change of the amino acid site where the modified amino acid is located is large;
(2) when the structure is compared between when the antigen-binding domain variant having the amino acid modification is bound to MTA and when it is not bound to MTA, the structural change of the antigen-binding domain variant is not inhibited by the presence of the modified amino acid; or
(3) the MTA-binding activity of the antigen-binding domain variant having the amino acid modification is not significantly weakened as compared to that of the unmodified antigen-binding domain.

[C3] The production method according to any one of [C1] to [C2], wherein the antigen-binding domain having MTA-binding activity does not substantially bind to adenosine.
[C4] The production method according to [C3], wherein the antigen-binding domain having MTA-binding activity does not bind to S-(5'-adenosyl)-L-homocysteine (SAH), AMP, ADP, or/and ATP.
[C5] A library produced by the production method according to any one of [C1] to [C4].
[C6] A method of producing a library comprising the following steps (a) and (b):

(a) identifying an amino acid site that satisfies at least one of the following (i) to (ii) in an antigen-binding domain having binding activity towards a small molecule compound:

(i) an amino acid site exposed on the surface of the antigen-binding domain;
(ii) an amino acid site located in a region where the rate of structural change is large when the structure is compared between when the antigen-binding domain is bound to the small molecule compound and when it is not bound to the small molecule compound; and

(b) designing a library comprising a nucleic acid encoding an unmodified antigen-binding domain, and nucleic acids encoding a plurality of variants of the antigen-binding domain differing in sequence from one another and having an amino acid modification at one or more amino acid sites identified in step (a).

[C7] The production method according to [C5], wherein the amino acid modification in step (b) satisfies at least one or more of the following (1) to (3):

(1) when the structure is compared between when the antigen-binding domain variant having the amino acid

modification is bound to the small molecule compound and when it is not bound to the small molecule compound, the rate of structural change of the amino acid site where the modified amino acid is located is large;
(2) when the structure is compared between when the antigen-binding domain variant having the amino acid modification is bound to the small molecule compound and when it is not bound to the small molecule compound, the structural change of the antigen-binding domain variant is not inhibited by the presence of the modified amino acid; or
(3) the binding activity to the small molecule compound of the antigen-binding domain variant having the amino acid modification is not significantly weakened as compared to the unmodified antigen-binding domain.

[C8] A method of producing a library comprising the following steps (a) and (b):

(a) identifying an amino acid site that satisfies at least one of the following (i) to (iv) in an antigen-binding domain that interacts with a small molecule compound:

(i) an amino acid site exposed on the surface of the antigen-binding domain;
(ii) an amino acid site located in a region where the rate of structural change is large when the structure is compared between when the antigen-binding domain is bound to the small molecule compound and when it is not bound to the small molecule compound;
(iii) an amino acid site that is not involved in the binding to the small molecule compound;
(iv) an amino acid site that does not significantly weaken the binding to the small molecule compound;
(v) an amino acid site with diverse amino acid occurrence frequencies in the animal species to which the antigen-binding domain belongs; or
(vi) an amino acid site that is not important for canonical structure formation; and

(b) designing a library comprising a nucleic acid encoding an unmodified antigen-binding domain, and nucleic acids encoding a plurality of variants of the antigen-binding domain differing in sequence from one another and having an amino acid modification that satisfies at least one of the following (1) to (2) in the one or more amino acid sites identified in step (a):

(1) when the structure is compared between when the antigen-binding domain variant having the amino acid modification is bound to the small molecule compound and when it is not bound to the small molecule compound, the rate of structural change of the amino acid site where the modified amino acid is located is large; or
(2) when the structure is compared between when the antigen-binding domain variant having the amino acid modification is bound to the small molecule compound and when it is not bound to the small molecule compound, the structural change of the antigen-binding domain variant is not inhibited by the presence of the modified amino acid.

[C9] The production method according to any one of [C6] to [C8], wherein the small molecule compound is selected from at least one selected from adenosine, adenosine triphosphate, adenosine diphosphate, adenosine monophosphate, and S-(5'-adenosyl)-L-homocysteine (SAH).
[C10] A library produced by the production method according to any one of [C6] to [C8].

The present disclosure also encompasses embodiments exemplified below.

[D1] A method of screening for an antigen-binding molecule comprising an antigen-binding domain whose antigen-binding activity changes in an MTA-dependent manner.
[D2] The method according to [D1], wherein the method comprises comparing the antigen-binding activity of the antigen-binding domain in the presence of a first concentration of MTA with the antigen-binding activity in the presence of MTA at a concentration different from the first concentration (a second concentration).
[D3] The method according to [D1] or [D2], wherein the method comprises the step of selecting an antigen-binding molecule comprising an antigen-binding domain whose antigen-binding activity in the presence of the first concentration of MTA is different from the antigen-binding activity in the presence of the second concentration of MTA.
[D4] The method according to anyone of [D2] to [D3], wherein the method comprises the step of selecting an antigen-binding molecule comprising an antigen-binding domain whose antigen-binding activity in the presence of the first concentration of MTA is higher than the antigen-binding activity in the presence of the second concentration of MTA.
[D5] The method according to any one of [D2] to [D3], wherein the method comprises the step of selecting an antigen-binding molecule comprising an antigen-binding domain whose antigen-binding activity in the presence of the first

concentration of MTA is lower than the antigen-binding activity in the presence of the second concentration of MTA.
[D6] The method according to [D1], wherein the method comprises the following steps (a) to (c):

(a) contacting an antigen-binding molecule comprising an antigen-binding domain with an antigen in the presence of a first concentration of MTA,
(b) placing the antigen-binding molecule comprising the antigen-binding domain bound in step (a) in a condition where a second concentration of MTA is present, and
(c) isolating the antigen-binding molecule comprising the antigen-binding domain that dissociated in step (b).

[D7] The method according to [D1], wherein the method comprises the following steps (a) to (d):

(a) contacting an antigen-binding molecule comprising an antigen-binding domain with an antigen in the presence of a first concentration of MTA,
(b) confirming the binding of the antigen-binding molecule comprising the antigen-binding domain to the antigen in step (a),
(c) placing the antigen-binding molecule comprising the antigen-binding domain bound to the antigen in a condition where a second concentration of MTA is present, and
(d) isolating the antigen-binding molecule comprising the antigen-binding domain whose antigen-binding activity in step (c) is weaker than the standard used for confirming the binding to the antigen in step (b).

[D8] The method according to [D6] or [D7], wherein the method comprises confirming that the antigen-binding domain can bind to MTA before performing step (a).
[D9] The method according to [D1], wherein the method comprises the following steps (a) to (d):

(a) contacting an antigen-binding molecule comprising an antigen-binding domain with an antigen in the presence of a first concentration of MTA,
(b) confirming the non-binding of the antigen-binding molecule comprising the antigen-binding domain to the antigen in step (a),
(c) allowing the antigen-binding molecule comprising the antigen-binding domain not bound to the antigen to bind to the antigen in the presence of a second concentration of MTA, and
(d) isolating the antigen-binding molecule comprising the antigen-binding domain bound to the antigen in step (c).

[D10] The method according to [D1], wherein the method comprises the following steps (a) to (c):

(a) contacting a library displaying an antigen-binding molecule comprising an antigen-binding domain with an antigen in the presence of a first concentration of MTA,
(b) placing the antigen-binding molecule comprising the antigen-binding domain bound in step (a) in a condition where a second concentration of MTA is present, and
(c) isolating the antigen-binding molecule comprising the antigen-binding domain that dissociated in step (b).

[D11] The method according to [D1], wherein the method comprises the following steps (a) to (d):

(a) contacting a library displaying an antigen-binding molecule comprising an antigen-binding domain with an antigen in the presence of a first concentration of MTA,
(b) selecting the antigen-binding molecule comprising the antigen-binding domain bound to the antigen in step (a),
(c) placing the antigen-binding molecule comprising the antigen-binding domain selected in step (b) in a condition where a second concentration of MTA is present, and
(d) isolating the antigen-binding molecule comprising the antigen-binding domain whose antigen-binding activity in step (c) is weaker than the standard used for the selection in step (b).

[D12] The method according to [D10] or [D11], wherein the method comprises before step (a), the following steps (1) to (2):

(1) contacting a library displaying an antigen-binding molecule comprising an antigen-binding domain with MTA, and
(2) selecting the antigen-binding molecule comprising the antigen-binding domain bound to MTA in step (1),

wherein the library that is contacted with the antigen in the presence of a first concentration of MTA in step (a) is a library displaying the antigen-binding molecule comprising the antigen-binding domain selected in steps (1) and (2).
[D13] The method according to [D1], wherein the method comprises the following steps (a) to (d):

(a) contacting a library displaying an antigen-binding molecule comprising an antigen-binding domain with an antigen in the presence of a first concentration of MTA,
(b) selecting the antigen-binding molecule comprising the antigen-binding domain not bound to the antigen in step (a),
(c) allowing the antigen to bind to the antigen-binding molecule comprising the antigen-binding domain selected in step (b) in the presence of a second concentration of MTA, and
(d) isolating the antigen-binding molecule comprising the antigen-binding domain bound to the antigen in step (c).

[D14] The method according to any one of [D2] to [D8], wherein the first concentration is higher than the second concentration.
[D15] The method according to [D1], wherein the method comprises comparing the antigen-binding activity of the antigen-binding domain in the presence of MTA with the antigen-binding activity in the absence of MTA.
[D16] The method according to [D1] or [D15], wherein the method comprises the step of selecting an antigen-binding domain whose antigen-binding activity in the presence of MTA is different from the antigen-binding activity in the absence of MTA.
[D17] The method according to [D1] or [D15], wherein the method comprises the step of selecting an antigen-binding domain whose antigen-binding activity in the presence of MTA is higher than the antigen-binding activity in the absence of MTA.
[D18] The method according to [D1], wherein the method comprises the following steps (a) to (c):

(a) contacting an antigen-binding molecule comprising an antigen-binding domain with an antigen in the presence of MTA,
(b) placing the antigen-binding molecule comprising the antigen-binding domain bound in step (a) in a condition where MTA is absent, and
(c) isolating the antigen-binding molecule comprising the antigen-binding domain that dissociated in step (b).

[D19] The method according to [D1], wherein the method comprises the following steps (a) to (d);

(a) contacting an antigen-binding molecule comprising an antigen-binding domain with an antigen in the presence of MTA,
(b) confirming the binding of the antigen-binding molecule comprising the antigen-binding domain to the antigen in step (a),
(c) placing the antigen-binding molecule comprising the antigen-binding domain bound to the antigen in a condition where MTA is absent, and
(d) isolating the antigen-binding molecule comprising the antigen-binding domain whose antigen-binding activity in step (c) is weaker than the standard used for confirming the binding to the antigen in step (b).

[D20] The method according to [D18] or [D19], wherein the method comprises confirming that the antigen-binding molecule comprising the antigen-binding domain can bind to MTA, before performing step (a).
[D21] The method according to [D1], wherein the method comprises the following steps (a) to (d):

(a) contacting an antigen-binding molecule comprising an antigen-binding domain with an antigen in the presence of MTA,
(b) confirming the non-binding of the antigen-binding molecule comprising the antigen-binding domain to the antigen in step (a),
(c) allowing the antigen-binding molecule comprising the antigen-binding domain not bound to the antigen to bind to the antigen in the absence of MTA, and
(d) isolating the antigen-binding molecule comprising the antigen-binding domain bound to the antigen in step (c).

[D22] The method according to [D1], wherein the method comprises the following steps (a) to (c):

(a) contacting a library displaying an antigen-binding molecule comprising an antigen-binding domain with an antigen in the presence of MTA,
(b) placing the antigen-binding molecule comprising the antigen-binding domain bound in step (a) in a condition

where MTA is absent, and
(c) isolating the antigen-binding molecule comprising the antigen-binding domain that dissociated in step (b).

[D23] The method according to [D1], wherein the method comprises the following steps (a) to (d):

(a) contacting a library displaying an antigen-binding molecule comprising an antigen-binding domain with an antigen in the presence of MTA,
(b) selecting the antigen-binding molecule comprising the antigen-binding domain bound to the antigen in step (a),
(c) placing the antigen-binding molecule comprising the antigen-binding domain selected in step (b) in a condition where MTA is absent, and
(d) isolating the antigen-binding molecule comprising the antigen-binding domain whose antigen-binding activity in step (c) is weaker than the standard used for the selection in step (b).

[D24] The method according to [D22] or [D23], wherein the method comprises before step (a) the following steps (1) to (2):

(1) contacting a library displaying an antigen-binding molecule comprising an antigen-binding domain with MTA, and
(2) selecting the antigen-binding molecule comprising the antigen-binding domain bound to MTA in step (1),

wherein the library contacted with the antigen in the presence of MTA in step (a) is a library displaying the antigen-binding molecule comprising the antigen-binding domain selected in steps (1) and (2).
[D25] The method according to [D1], wherein the method comprises the following steps (a) to (d):

(a) contacting a library displaying an antigen-binding molecule comprising an antigen-binding domain with an antigen in the presence of MTA,
(b) selecting the antigen-binding molecule comprising the antigen-binding domain not bound to the antigen in step (a),
(c) allowing the antigen to bind in the absence of MTA to the antigen-binding molecule comprising the antigen-binding domain selected in step (b), and
(d) isolating the antigen-binding molecule comprising the antigen-binding domain bound to the antigen in step (c).

[D26] The method according to any one of [D10] to [D14] or [D22] to [D25], wherein the library displaying the antigen-binding molecule comprising the antigen-binding domain is a naive human antibody display library, or a synthetic human antibody display library, or the library according to any one of [B1] to [B14], or the library according to [C5].
[D27] The method according to any one of [D1] to [D26], wherein the antigen-binding domain is an antibody variable region or a single-domain antibody.

The present disclosure also encompasses embodiments exemplified below.

[F1] A method of producing an antigen-binding molecule comprising an antigen-binding domain whose antigen-binding activity changes in an MTA-dependent manner.
[F2] The method according to [F1], wherein the method comprises comparing the antigen-binding activity of the antigen-binding domain in the presence of a first concentration of MTA with the antigen-binding activity in the presence of MTA at a concentration different from the first concentration (a second concentration).
[F3] The method according to [F1] or [F2], wherein the method comprises the step of selecting an antigen-binding domain whose antigen-binding activity in the presence of a first concentration of MTA is different from the antigen-binding activity in the presence of the second concentration of MTA.
[F4] The method according to any one of [F2] to [F3], wherein the method comprises the step of selecting an antigen-binding domain whose antigen-binding activity in the presence of the first concentration of MTA is higher than the antigen-binding activity in the presence of the second concentration of MTA.
[F5] The method according to any one of [F2] to [F3], wherein the method comprises the step of selecting an antigen-binding domain whose antigen-binding activity in the presence of the first concentration of MTA is lower than the antigen-binding activity in the presence of the second concentration of MTA.
[F6] The method according to any one of [F3] to [F5], wherein the method further comprises the step of culturing a cell introduced with a vector in which a polynucleotide encoding the selected antigen-binding molecule comprising the antigen-binding domain is operably linked, and recovering the antigen-binding molecule comprising the antigen-binding domain from the cell culture medium.
[F7] The method according to [F1], wherein the method comprises the following steps (a) to (d):

(a) contacting an antigen-binding domain with an antigen in the presence of a first concentration of MTA,
(b) placing the antigen-binding domain bound in step (a) in a condition where a second concentration of MTA is present,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

[F8] The method according to [F1], wherein the method comprises the following steps (a) to (e);

(a) contacting an antigen-binding domain with an antigen in the presence of a first concentration of MTA,
(b) confirming the binding of the antigen-binding domain to the antigen in step (a),
(c) placing the antigen-binding domain bound to the antigen in a condition where a second concentration of MTA is present,
(d) isolating the antigen-binding domain whose antigen-binding activity in step (c) is weaker than the standard used for confirming the binding to the antigen in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F9] The method according to [F7] or [F8], wherein the method comprises confirming that the antigen-binding domain can bind to MTA before performing step (a).
[F10] The method according to [F1], wherein the method comprises the following steps (a) to (e):

(a) contacting an antigen-binding domain with an antigen in the presence of a first concentration of MTA,
(b) confirming the non-binding of the antigen-binding domain to the antigen in step (a),
(c) allowing the antigen-binding domain not bound to the antigen to bind to the antigen in the presence of a second concentration of MTA,
(d) isolating the antigen-binding domain bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F11] The method according to [F1], wherein the method comprises the following steps (a) to (d):

(a) contacting a library displaying an antigen-binding domain with an antigen in the presence of a first concentration of MTA,
(b) placing the antigen-binding domain bound in step (a) in a condition where a second concentration of MTA is present,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

[F12] The method according to [F1], wherein the method comprises the following steps (a) to (e):

(a) contacting a library displaying an antigen-binding domain with an antigen in the presence of a first concentration of MTA,
(b) selecting the antigen-binding domain that bound to the antigen in step (a),
(c) placing the antigen-binding domain selected in step (b) in a condition where a second concentration of MTA is present,
(d) isolating the antigen-binding domain whose antigen-binding activity in step (c) is weaker than the standard used for the selection in step (b), and
(e) culturing a cell introduced with a vector in which a polynucleotide encoding an antigen-binding molecule comprising the antigen-binding domain isolated in step (d) is operably linked, and recovering the antigen-binding

molecule comprising the antigen-binding domain from the cell culture medium.

[F13] The method according to [F11] or [F12], wherein the method comprises the following steps (1) to (2) before step (a):

(1) contacting a library displaying an antigen-binding domain with MTA, and
(2) selecting the antigen-binding domain that bound to MTA in step (1),

wherein the library contacted with the antigen in the presence of a first concentration of MTA in step (a) is a library displaying the antigen-binding domain selected in steps (1) and (2).
[F14] The method according to [F1], wherein the method comprises the following steps (a) to (e):

(a) contacting a library displaying an antigen-binding domain with an antigen in the presence of a first concentration of MTA,
(b) selecting the antigen-binding domain that does not bind to the antigen in step (a),
(c) allowing the antigen-binding domain selected in step (b) to bind to the antigen in the presence of a second concentration of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F15] The method according to any one of [F2] to [F14], wherein the first concentration is higher than the second concentration.
[F16] The method according to [F1], wherein the method comprises comparing the antigen-binding activity of the antigen-binding domain in the presence of MTA with the antigen-binding activity in the absence of MTA.
[F17] The method according to [F1] or [F16], wherein the method comprises the step of selecting an antigen-binding domain whose antigen-binding activity in the presence of MTA and the antigen-binding activity in the absence of MTA are different.
[F18] The method according to [F1] or [F16], wherein the method comprises the step of selecting an antigen-binding domain whose antigen-binding activity in the presence of MTA is higher compared to the antigen-binding activity in the absence of MTA.
[F19] The method according to any one of [F16] to [F18], wherein the method further comprises the step of culturing a cell introduced with a vector in which a polynucleotide encoding an antigen-binding molecule comprising the selected antigen-binding domain is operably linked, and recovering the antigen-binding molecule comprising the antigen-binding domain from the cell culture medium.
[F20] The method according to [F1], wherein the method comprises the following steps (a) to (d):

(a) contacting an antigen binding domain with an antigen in the presence of MTA,
(b) placing the antigen-binding domain bound in step (a) in a condition where MTA is absent,
(c) isolating the antigen-binding domain that dissociated in step (b), and
(d) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F21] The method according to [F1], wherein the method comprises the following steps (a) to (e):

(a) contacting an antigen-binding domain with an antigen in the presence of MTA,
(b) confirming the binding of the antigen-binding domain to the antigen in step (a),
(c) placing the antigen-binding domain bound to the antigen in a condition where MTA is absent,
(d) isolating the antigen-binding domain whose antigen-binding activity in step (c) is weaker than the standard used for confirming the binding to the antigen in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F22] The method according to [F20] or [F21], wherein the method confirms before performing step (a) that the antigen-binding domain can bind to MTA.

[F23] The method according to [F1], wherein the method comprises the following steps (a) to (e):

(a) contacting an antigen-binding domain with an antigen in the presence of MTA,
(b) confirming that the antigen-binding domain does not bind to the antigen in step (a),
(c) allowing the antigen-binding domain that does not bind to the antigen to bind to the antigen in the absence of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F24] The method according to [F1], wherein the method comprises the following steps (a) to (d):

(a) contacting a library displaying an antigen-binding domain with an antigen in the presence of MTA,
(b) placing the antigen-binding domain bound in step (a) in a condition where MTA is absent,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

[F25] The method according to [F1], wherein the method comprises the following steps (a) to (e):

(a) contacting a library displaying an antigen-binding domain with an antigen in the presence of MTA,
(b) selecting the antigen-binding domain bound to the antigen in step (a),
(c) placing the antigen-binding domain selected in step (b) in a condition where MTA is absent,
(d) isolating the antigen-binding domain whose antigen-binding activity in step (c) is weaker than the standard used for the selection in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F26] The method according to [F24] or [F25], wherein the method comprises before step (a), the following steps (1) to (2):

(1) contacting a library displaying an antigen-binding domain with MTA, and
(2) selecting the antigen-binding domain that bound to MTA in step (1),

wherein the library contacted with the antigen in the presence of MTA in step (a) is a library displaying the antigen-binding domain selected in steps (1) and (2).
[F27] The method according to [F1], wherein the method comprises the following steps (a) to (e):

(a) contacting a library displaying an antigen-binding domain with an antigen in the presence of MTA,
(b) selecting the antigen-binding domain that does not bind to the antigen in step (a),
(c) allowing the antigen-binding domain selected in step (b) to bind to an antigen in the absence of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

[F28] The method according to any one of [F11] to [F15] or [F24] to [F27], wherein the library displaying an antigen-binding domain is a naive human antibody display library, or a synthetic human antibody display library, or the library according to any one of [B1] to [B14], or the library according to [C5].
[F29] The method according to any one of [F1] to [F28], wherein the antigen-binding domain is an antibody variable region or a single-domain antibody.

The present disclosure also encompasses embodiments exemplified below.

[E1] An antigen-binding molecule that specifically binds to MTA.

[E2] The antigen-binding molecule according to [E1], which is an antigen-binding molecule that binds to MTA, wherein the antigen-binding molecule does not substantially bind to adenosine.
[E3] The antigen-binding molecule according to [E1] or [E2], which is an antigen-binding molecule that binds to MTA, wherein the antigen-binding molecule does not substantially bind to S-(5'-adenosyl)-L-homocysteine (SAH), AMP, ADP or/and ATP.
[E4] A method for measuring MTA concentration, which uses the antigen-binding molecule according to any one of [E1] to [E3].
[E5] The measuring method according to [E4], wherein the MTA concentration is MTA concentration in a tissue.
[E6] The measuring method according to any one of [E4] or [E5], which measures MTA concentration using an antigen-antibody reaction.
[E7] The measuring method according to any one of [E4] to [E5], which uses an immunohistological staining method.
[E8] The measuring method according to any one of [E4] to [E5], which uses an imaging method.
[E9] The measuring method according to any one of [E4] to [E5], which uses an *in vivo* imaging method.
[E10] A method of diagnosing a disease, which uses the antigen-binding molecule according to any one of [E1] to [E3].
[E11] The method according to [E10], wherein the diagnosis is to determine the presence or absence of a disease, or to predict a therapeutic effect on a disease.
[E12] The method according to any one of [E10] to [E11], wherein the disease is cancer.
[E13] The method according to [E12], wherein the cancer is a cancer in which MTA is accumulated in cancer tissue.
[E14] The method according to any one of [E12] or [E13], wherein the cancer is a cancer tissue in which the gene encoding MTAP is absent or underexpressed, or which has a mutation or splicing variant that reduces enzyme activity.
[E15] A kit for diagnosing a disease, which comprises the antigen-binding molecule according to any one of [E1] to [E3].
[E16] The kit according to [E15], wherein the diagnosis is to determine the presence or absence of a disease, or to predict a therapeutic effect on a disease.
[E17] The kit according to any one of [E15] to [E16], wherein the disease is cancer.
[E18] The kit according to [E17], wherein the cancer is a cancer in which MTA is accumulated in cancer tissue.

[Effects of the Invention]

The antigen-binding molecules comprising an antigen-binding domain whose antigen-binding activity changes in an MTA-dependent manner and pharmaceutical compositions comprising them of the present disclosure do not act systemically in normal tissues or blood, and by acting reversibly in cancer, they are able to exert a medicinal effect while avoiding side effects and treat cancer.

Furthermore, by using a library comprising a plurality of antigen-binding molecules comprising antigen-binding domains, differing in sequence from one another, whose antigen-binding activity changes in an MTA-dependent manner, it is possible to efficiently obtain in a short time various antigen-binding molecules whose antigen-binding activity changes in an MTA-dependent manner which are useful for treating diseases that are cancer tissue-specific, as discussed above.

[Brief Description of Drawings]

Fig. 1 shows the intracellular MTA concentration of each cell line. The vertical axis is the intracellular MTA concentration, and the horizontal axis is the cell line name and MTAP deficiency status. MTAP- means an MTAP-deficient cell line and MTAP+ means an MTAP-non-deficient cell line.
Fig. 2 shows the MTA concentration in the culture medium in which each cell line was cultured. The vertical axis is MTA concentration in the culture medium, and the horizontal axis is the culture time, cell line name, and MTAP deficiency status. MTAP- means an MTAP-deficient cell line and MTAP+ means an MTAP-non-deficient cell line.
Fig. 3 shows the MTA concentration in the culture medium when each cell line was cultured in a medium to which MTA was added in advance. The vertical axis is the MTA concentration in the culture medium, and the horizontal axis is the culture time. Each spot shows each measured data. The graph on the left shows the MTA concentration in the culture medium when MTAP-non-deficient cells HT-1376 were cultured, and the graph on the right shows the MTA concentration in the culture medium when MTAP-non-deficient cells SK-MES-1 were cultured.
Fig. 4 shows the MTA concentration in tumors of tumor-bearing mice. The vertical axis is MTA concentration in the tumor, and the horizontal axis is the name of the cell line transplanted into the mice. Each spot shows each measured data.
Fig. 5 shows the relationship between the amount of MTAP DNA in a human clinical sample and the concentration of MTA in tissues. The graph on the left shows the results of measuring clinical samples of bladder cancer, and the

graph on the right shows the results of measuring clinical samples of esophageal cancer. Each spot in the graph shows each clinical sample, and the light-colored spots show samples in which MTA concentration in the tissue was below the detection limit. The vertical axis is MTA concentration in the tissue, and the horizontal axis is $\Delta$Ct obtained by subtracting the Ct value of the $\Psi$X4 gene from the Ct value of MTAP gene.

Fig. 6 shows the extracellular MTA concentration in tissues of tumor-bearing mice. The graph on the left shows the extracellular MTA concentration in tumor, and the graph on the right shows the extracellular MTA concentration in liver, which is a normal tissue. The vertical axis is the extracellular MTA concentration in the tissue, and the horizontal axis is the name of the cell line transplanted into the mice. Each spot shows each measured data, and the hollow spots are data below the lower limit of MTA quantification.

Fig. 7 shows the amount of binding of C03H-BH076N17/C03L-KT0 to hIL-6R under different concentrations of MTA or adenosine. The vertical axis shows the amount of hIL-6R bound per immobilized antibody amount, and the horizontal axis shows the concentration of MTA or adenosine.

Fig. 8 shows a mode of binding between SMB0002hFab and adenosine. In the figure, the antibody heavy chain is shown in black, the light chain is shown in gray, and adenosine is shown in a ball-and-stick model. Amino acid residues that interact with adenosine are shown in a stick model. The dashed lines and the numbers indicate the distance between hydrogen bonds, CH-$\pi$ interactions, or $\pi$-$\pi$ interactions between each amino acid residue and adenosine. The unit is indicated by Å.

Fig. 9 is a diagram in which variable regions are extracted from the two molecules, SMB0002hFab_1 and SMB0002hFab_2, comprised in the asymmetric unit of the crystal structure of SMB0002hFab, and superimposed. In the figure, SMB0002hFab_1 is shown in gray and SMB0002hFab_2 is shown in black.

Fig. 10 is a diagram in which adenosine and variable regions are extracted from SMB0002hFab_1, one molecule among the two molecules in the asymmetric unit of the crystal structure of SMB0002hFab, and from a complex of SMB0002hFab and adenosine (SMB0002hFab-adenosine complex), and superimposed. In the figure, SMB0002hFab_1 is shown in gray and the SMB0002hFab-adenosine complex is shown in black.

Fig. 11 is a diagram in which adenosine and variable regions are extracted from SMB0002hFab_2, one molecule among the two molecules in the asymmetric unit of the crystal structure of SMB0002hFab, and from a complex of SMB0002hFab and adenosine (SMB0002hFab-adenosine complex), and superimposed. In the figure, SMB0002hFab_2 is shown in gray and the SMB0002hFab-adenosine complex is shown in black.

Fig. 12 shows the amount of MTA binding of clone groups acquired after panning of a heavy chain variable region phage display library against MTA. The vertical axis shows the absorbance by phage ELISA when MTA is not fixed, and the horizontal axis shows the absorbance by phage ELISA when MTA is fixed.

Fig. 13 shows the amount of MTA binding of clone groups acquired after panning of a light chain variable region phage display library against MTA. The vertical axis shows the absorbance by phage ELISA when MTA is not fixed, and the horizontal axis shows the absorbance by phage ELISA when MTA is fixed.

Fig. 14 shows the amount of MTA-dependent antigen-binding antibody bound to each of hIL-6R, hIL-6, and hIgA in the presence of MTA or adenosine identified using SPR. The vertical axis shows the binding amount (from -100 to 200 RU), and the horizontal axis shows the reaction time (from -100 to 1200 seconds, 0 seconds is the antigen reaction start time).

Fig. 15 is a diagram in which a variable region and MTA are extracted from the crystal structure of a complex of MTA0303Fab and MTA. In the figure, the antibody heavy chain is shown in black, the light chain is shown in gray, and MTA is shown in a ball-and-stick model.

Fig. 16 shows a mode of binding between MTA0303Fab and MTA. In the figure, the antibody heavy chain is shown in black, the light chain is shown in gray, and MTA is shown in a ball-and-stick model. Amino acid residues that interact with MTA are shown in a stick model. The dashed lines and their numbers indicate the distances between hydrogen bonds, CH-$\pi$ interactions, $\pi$-$\pi$ interactions, and sulfur-$\pi$ interactions between each amino acid residue and MTA. The unit is indicated by Å.

Fig. 17 shows a mode of binding between MTA0303Fab and MTA. In the figure, the antibody heavy chain is shown in black, the light chain is shown in gray, and MTA is shown in a ball-and-stick model. Amino acid residues that interact with MTA are shown in a stick model. The dashed line and its value indicate the hydrogen bond distance between the amino acid residue and MTA. The unit is indicated by Å.

Fig. 18 is a diagram in which a variable region and MTA are extracted from the crystal structure of a complex of MTA0330Fab and MTA. In the figure, the antibody heavy chain is shown in black, the light chain is shown in gray, and MTA is shown in a ball-and-stick model.

Fig. 19 shows a mode of binding between MTA0330Fab and MTA. In the figure, the antibody heavy chain is shown in black, the light chain is shown in gray, and MTA is shown in a ball-and-stick model. Amino acid residues that interact with MTA are shown in a stick model. The dashed lines and their numbers indicate the distance between hydrogen bonds, CH-$\pi$ interactions, or $\pi$-$\pi$ interactions between each amino acid residue and MTA. The unit is indicated by Å.

Fig. 20 is a diagram of the crystal structure of a complex of MTA0303Fab and MTA. In the figure, the antibody heavy chain is shown in black, the light chain is shown in light gray, and MTA is shown in a stick model. The Cα atoms of isoleucine, leucine, valine, and alanine contained in the heavy chain of the antibody are indicated by dark gray spheres. The Cα atoms of isoleucine, leucine, valine, and alanine contained in the light chain are indicated by white spheres.

Fig. 21 is a diagram of the crystal structure of a complex of MTA0330Fab and MTA. In the figure, the antibody heavy chain is shown in black, the light chain is shown in light gray, and MTA is shown in a stick model. The Cα atoms of isoleucine, leucine, valine, and alanine contained in the heavy chain of the antibody are indicated by dark gray spheres. The Cα atoms of isoleucine, leucine, valine, and alanine contained in the light chain are indicated by white spheres.

Fig. 22 shows the superposition of 1H-15N TROSY spectra of MTA0303Fab in MTA-bound state and MTA-unbound state. The spectra of the bound state are shown in black, and the unbound state are shown in gray.

Fig. 23 shows the superposition of 1H-13C SOFAST-HMQC spectra of MTA0303Fab in MTA-bound state and MTA-unbound state. The spectra of the bound state are shown in black, and the unbound state are shown in gray.

Fig. 24 shows the superposition of 1H-15N TROSY spectra of MTA0330Fab in MTA-bound state and MTA-unbound state. The spectra of the bound state are shown in black, and the unbound state are shown in gray.

Fig. 25 shows the superposition of 1H-13C SOFAST-HMQC spectra of MTA0330Fab in MTA-bound state and MTA-unbound state. The spectra of the bound state are shown in black, and the unbound state are shown in gray.

Fig. 26 shows that a small molecule switch antibody does not bind to an antigen in a normal environment where small molecules do not exist, and binds to an antigen in a target tissue in which a high concentration of small molecules is present.

Fig. 27 shows that a small molecule achieves a switch function by being sandwiched between a complex of a low molecular weight antibody and an antigen. In the absence of a small molecule, the interaction between the antibody and the antigen is insufficient and the antibody cannot bind to the antigen, but in the presence of a small molecule, the antibody can bind to the antigen due to the small molecule being sandwiched between the antibody and the antigen.

Fig. 28 shows the T cell activation ability of a bispecific antibody having an antigen-binding domain that binds to IL-6R and an antigen-binding domain that binds to CD3 in an MTA-dependent manner, tested using NFAT-RE-luc2-Jurkat cells in the presence or absence of MTA or ADO. The X-axis shows the antibody concentration (μg/mL), and the Y-axis shows the Relative Light Unit (RLU).

Fig. 29 shows the binding amount to hIL-6R of the anti-IL-6R antibody which binds to IL-6R in an MTA-dependent manner under different MTA concentrations measured using Biacore T200. The vertical axis shows the amount of hIL-6R bound per immobilized antibody amount, and the horizontal axis shows the concentration of MTA.

Fig. 30 is a sensorgram showing the change over time in the amount of binding to the antigen (hIL-6R) of the anti-IL-6R antibody that binds to IL-6R in an MTA-dependent manner, as measured using the Octet RED384 system. The upper and lower diagrams show the measurement results with MTA of 100 μM and 10 μM, respectively.

[Description of Embodiments]

The definitions and detailed description below are provided to facilitate understanding of the present disclosure illustrated herein.

Amino acids

Herein, amino acids are described by one- or three-letter codes or both, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

Alteration of amino acids

For amino acid alteration in the amino acid sequence of an antigen-binding molecule, known methods such as site-directed mutagenesis methods (Kunkel et al. (Proc. Natl. Acad. Sci. USA (1985) 82, 488-492)) and overlap extension PCR may be appropriately employed. Furthermore, several known methods may also be employed as amino acid alteration methods for substitution to unnatural amino acids (Annu. Rev. Biophys. Biomol. Struct. (2006) 35, 225-249; and Proc. Natl. Acad. Sci. U.S.A. (2003) 100 (11), 6353-6357). For example, it is suitable to use a cell-free translation system (Clover Direct (Protein Express)) containing a tRNA which has an unnatural amino acid bound to a complementary amber suppressor tRNA of one of the stop codons, the UAG codon (amber codon).

In the present specification, the meaning of the term "and/or" when describing the site of amino acid alteration includes every combination where "and" and "or" are suitably combined. Specifically, for example, "the amino acids at

positions 33, 55, and/or 96 are substituted" includes the following variation of amino acid alterations: amino acid(s) at (a) position 33, (b) position 55, (c) position 96, (d) positions 33 and 55, (e) positions 33 and 96, (f) positions 55 and 96, and (g) positions 33, 55, and 96.

Furthermore, herein, as an expression showing alteration of amino acids, an expression that shows before and after a number indicating a specific position, one-letter or three-letter codes for amino acids before and after alteration, respectively, may be used appropriately. For example, the alteration N100bL or Asn100bLeu used when substituting an amino acid contained in an antibody variable region indicates substitution of Asn at position 100b (according to Kabat numbering) with Leu. That is, the number shows the amino acid position according to Kabat numbering, the one-letter or three-letter amino-acid code written before the number shows the amino acid before substitution, and the one-letter or three-letter amino-acid code written after the number shows the amino acid after substitution. Similarly the alteration P238D or Pro238Asp used when substituting an amino acid of the Fc region contained in an antibody constant region indicates substitution of Pro at position 238 (according to EU numbering) with Asp. That is, the number shows the amino acid position according to EU numbering, the one-letter or three-letter amino-acid code written before the number shows the amino acid before substitution, and the one-letter or three-letter amino-acid code written after the number shows the amino acid after substitution.

Antigens

Herein, "antigens" are not particularly limited in their structure, as long as they comprise epitopes to which antigen-binding domains bind. In one embodiment, antigens are peptides of 4 amino acids or more, polypeptides, or proteins. Antigens include, for example, the molecules below: 17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7,alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic peptide, av/b3 integrin, Axl, b2M, B7-1, B7-2, B7-H, B-lymphocyte stimulating factor (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 Osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer associated antigen, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, Botulinum toxin, Clostridium perfringens toxin, CKb8-1, CLC, CMV, CMV UL, CNTF, CNTN-1, COX, C-Ret, CRG-2, CT-1, CTACK, CTGF, CTLA-4, PD1, PDL1, LAG3, TIM3, galectin-9, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6,cytokeratin tumor associated antigen, DAN, DCC, DcR3, DC-SIGN, complement regulatory factor (Decay accelerating factor), des (1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, ephrin B2/EphB4, EPO, ERCC, E-selectin, ET-1, factor IIa, factor VII, factor VIIIc, factor IX, fibroblast activation protein (FAP), Fas, FcR1, FEN-1, ferritin, FGF, FGF-19, FGF-2, FGF3, FGF-8, FGFR, FGFR-3, fibrin, FL, FLIP, Flt-3, Flt-4, follicle stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha1, GFR-alpha2, GFR-alpha3, GITR, glucagon, Glut4, glycoprotein IIb/IIIa (GPIIb/IIIa), GM-CSF, gp130, gp72, GRO, growth hormone releasing hormone, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high molecular weight melanoma-associated antigen (HMW-MAA), HIV gp120, HIV IIIB gp 120 V3 loop, HLA, HLA-DR, HM1.24, HMFG PEM, HRG, Hrk, human cardiac myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF binding protein, IGF-1R, IGFBP, IGF-I, IGF-II, IL, IL-1, IL-1R, IL-2, IL-2R,

IL-4, IL-4R, IL-5, IL-5R, IL-6, IL-6R, IL-8, IL-9, IL-10, IL-12, IL-13, IL-15, IL-18, IL-18R, IL-21, IL-23, IL-27, interferon (INF)-alpha, INF-beta, INF-gamma, inhibin, iNOS, insulin A chain, insulin B chain, insulin-like growth factor1, integrin alpha2, integrin alpha3, integrin alpha4, integrin alpha4/beta1, integrin alpha4/beta7, integrin alpha5 (alpha V), integrin alpha5/beta1, integrin alpha5/beta3, integrin alpha6, integrin beta1, integrin beta2,interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y associated antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Mucl), MUC18, Mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), P1GF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, Rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T-cell receptor (for example, T-cell receptor alpha/beta), TdT, TECK, TEM1, TEM5, TEM7, TEM8, TERT, testis PLAP-like alkaline phosphatase, TfR, TGF, TGF-alpha, TGF-beta, TGF-beta Pan Specific, TGF-betaRI (ALK-5), TGF-betaRII, TGF-betaRIIb, TGF-betaRIII, TGF-beta1, TGF-beta2, TGF-beta3, TGF-beta4, TGF-beta5, thrombin, thymus Ck-1, thyroid-stimulating hormone, Tie, TIMP, TIQ, tissue factor, TMEFF2, Tmpo, TMPRSS2, TNF, TNF-alpha, TNF-alphabeta, TNF-beta2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF 14 (HVEM ATAR, HveA, LIGHT R, TR2), TNFRSF 16 (NGFR p75NTR), TNFRSF 17 (BCMA), TNFRSF 18 (GITR AITR), TNFRSF 19 (TROY TAJ, TRADE), TNFRSF 19L (RELT), TNFRSF1A (TNF RI CD120a, p55-60), TNFRSF1B (TNF RII CD120b, p75-80), TNFRSF26 (TNFRH3) , TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAIL-R2, TRANCE, transferrin receptor, TRF, Trk, TROP-2, TLR1 (Toll-like receptor 1), TLR2, TLR3, TLR4, TLR5, TLR6, TLR7, TLR8, TLR9, TLR10, TSG, TSLP, tumor associated antigen CA125, tumor associated antigen expressing Lewis-Y associated carbohydrates, TWEAK, TXB2, Ung, uPAR, uPAR-1, urokinase, VCAM, VCAM-1, VECAD, VE-Cadherin, VE-cadherin-2, VEFGR-1 (flt-1), VEGF, VEGFR, VEGFR-3 (flt-4), VEGI, VIM, virus antigen, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, Aβ, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, Chromogranin A, Chromogranin B, tau, VAP1, high molecular weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, Syndecan-1, Syndecan-2, Syndecan-3, Syndecan-4, LPA, and SIP; and receptors for hormone and growth factors. Preferred antigens are antigens that are expressed in cancer cells, immune cells, stromal cells, or such present in cancer tissues.

While receptors are recited as examples of the above-mentioned antigens, when these receptors exist in soluble forms in biological fluids, they may also be used as antigens that bind to the antigen-binding molecule of the present disclosure, which contains an antigen-binding domain whose antigen-binding activity varies in an MTA dependent manner. An example of a non-limiting embodiment of such a soluble receptor is the soluble IL-6R, which is a protein consisting of the amino acids at positions 1 to 357 in the IL-6R polypeptide sequence of SEQ ID NO: 1 as described in Mullberg et al. (J. Immunol. (1994) 152 (10), 4958-4968).

Membrane-type molecules expressed on cell membranes and soluble molecules secreted from cells to the outside of the cells are included in the examples of the above-mentioned antigens. When the antigen-binding molecule of the present disclosure, which contains an antigen-binding domain whose antigen-binding activity varies in an MTA dependent manner, binds to a soluble molecule secreted from cells, it is preferable that the antigen-binding molecule has neutralizing activity as described later.

The fluids in which the soluble molecules exist are not limited, and the soluble molecules may exist in biological fluids, or more specifically in all fluids filling the space between tissues and cells or vessels in organisms. In a non-limiting embodiment, the soluble molecules to which antigen-binding molecules of the present disclosure bind may be present in the extracellular fluid. In vertebrates, extracellular fluid is a general term for plasma, interstitial fluid, lymph, compact connective tissue, cerebrospinal fluid, spinal fluid, puncture fluid, synovial fluid, or such components in the bone and cartilage, alveolar fluid (bronchoalveolar lavage fluid), peritoneal fluid, pleural fluid, pericardial effusion, cyst fluid, aqueous humor (hydatoid), or such transcellular fluids (various fluids in the glandular cavities and fluids in the digestive tract cavity and other body cavity fluids produced as a result of active transport / secretory activities of cells).

When an antigen-binding molecule of the present disclosure comprising an antigen-binding domain whose antigen-binding activity varies in an MTA dependent manner binds to a membrane-type molecule expressed on a cell membrane, suitable examples of the antigen-binding molecule include antigen-binding molecules which have cytotoxic activity, bind to a cytotoxic substance, or have the ability to bind to a cytotoxic substance, as described later. Furthermore, antigen-binding molecules having a neutralizing activity instead of the properties of having a cytotoxic activity, binding to a cytotoxic substance, or having the ability to bind to a cytotoxic substance; or in addition to these properties are also suitable examples of a non-limiting embodiment.

Antigen-binding domain

Herein, an "antigen-binding domain" may be of any structure as long as it binds to an antigen of interest. Such domains preferably include, for example:

antibody heavy-chain and light-chain variable regions;
a module of about 35 amino acids called A domain which is contained in the *in vivo* cell membrane protein Avimer (International Publication No. WO 2004/044011, International Publication No. WO 2005/040229);
Adnectin containing the 10Fn3 domain which binds to the protein moiety of fibronectin, a glycoprotein expressed on cell membrane (International Publication No. WO 2002/032925); Affibody which is composed of a 58-amino acid three-helix bundle based on the scaffold of the IgG-binding domain of Protein A (International Publication No. WO 1995/001937);
Designed Ankyrin Repeat proteins (DARPins) which are a region exposed on the molecular surface of ankyrin repeats (AR) having a structure in which a subunit consisting of a turn comprising 33 amino acid residues, two antiparallel helices, and a loop is repeatedly stacked (International Publication No. WO 2002/020565);
Anticalins and such, which are domains consisting of four loops that support one side of a barrel structure composed of eight circularly arranged antiparallel strands that are highly conserved among lipocalin molecules such as neutrophil gelatinase-associated lipocalin (NGAL) (International Publication No. WO 2003/029462); and
the concave region formed by the parallel-sheet structure inside the horseshoe-shaped structure constituted by stacked repeats of the leucine-rich-repeat (LRR) module of the variable lymphocyte receptor (VLR) which does not have the immunoglobulin structure and is used in the system of acquired immunity in jawless vertebrate such as lamprey and hagfish (International Publication No. WO 2008/016854).

Suitable examples of the antigen-binding domains of the present disclosure include antigen-binding domains comprising antibody heavy-chain and light-chain variable regions. Examples of such antigen-binding domains are suitably "single chain Fv (scFv)", "single chain antibody", "Fv", "single chain Fv 2 (scFv2)", "Fab", or "F(ab')2".

Antigen-binding molecule

In the present disclosure, "an antigen-binding molecule comprising an antigen-binding domain" is used in the broadest sense; and specifically, it includes various types of molecules as long as they comprise an antigen-binding domain. An antigen-binding molecule may be a molecule consisting only of an antigen-binding domain, and may also be a molecule comprising an antigen -binding domain and other domains. For example, when the antigen-binding molecule is a molecule in which an antigen-binding domain is linked to an Fc region, examples include complete antibodies

and antibody fragments. Antibodies may include single monoclonal antibodies (including agonistic antibodies and antagonistic antibodies), human antibodies, humanized antibodies, chimeric antibodies, and such. Scaffold molecules where three dimensional structures, such as already-known stable α/β barrel protein structure, are used as a scaffold (base) and only some portions of the structures are made into libraries to construct antigen-binding domains are also included in antigen-binding molecules of the present disclosure.

Antibody

Herein, "antibody" refers to a natural immunoglobulin or an immunoglobulin produced by partial or complete synthesis. Antibodies can be isolated from natural sources such as naturally-occurring plasma and serum, or culture supernatants of antibody-producing hybridomas. Alternatively, antibodies can be partially or completely synthesized using techniques such as genetic recombination. Preferred antibodies include, for example, antibodies of an immunoglobulin isotype or subclass belonging thereto. Known human immunoglobulins include antibodies of the following nine classes (isotypes): IgG1, IgG2, IgG3, IgG4, IgA1, IgA2, IgD, IgE, and IgM. Of these isotypes, antibodies of the present disclosure include IgG1, IgG2, IgG3, and IgG4. A number of allotype sequences of human IgG1, human IgG2, human IgG3, and human IgG4 constant regions due to gene polymorphisms are described in "Sequences of proteins of immunological interest", NIH Publication No. 91-3242. Any of such sequences may be used in the present disclosure. In particular, for the human IgG1sequence, the amino acid sequence at positions 356 to 358 as indicated by EU numbering may be DEL or EEM. Several allotype sequences due to genetic polymorphisms have been described in "Sequences of proteins of immunological interest", NIH Publication No. 91-3242 for the human Igκ (Kappa) constant region and human Igλ (Lambda) constant region, and any of the sequences may be used in the present disclosure.

EU numbering and Kabat numbering

According to the methods used in the present disclosure, amino acid positions assigned to antibody CDR and FR are specified according to Kabat's numbering (Sequences of Proteins of Immunological Interest (National Institute of Health, Bethesda, Md., 1987 and 1991)). Herein, when an antigen-binding molecule is an antibody or antigen-binding fragment, variable region amino acids are indicated by Kabat numbering, while constant region amino acids are indicated by EU numbering based on Kabat's amino acid positions.

Variable region

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

Hypervariable region

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al., *supra*.

Framework

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

Fc region

An Fc region contains an amino acid sequence derived from the heavy chain constant region of an antibody. An Fc region is a portion of the antibody heavy chain constant region that includes the N terminal end of the hinge region, which is the papain cleavage site, at an amino acid around position 216 (indicated by EU numbering), and the hinge, CH2, and CH3 domains. Fc regions can be obtained from human IgG1; however, they are not limited to any specific IgG subclass. Preferred examples of the Fc regions include Fc regions having FcRn-binding activity in an acidic pH range as described below. Preferred examples of the Fc regions include Fc regions having Fcγ receptor-binding activity as described below. In a non-limiting embodiment, examples of such Fc regions include the Fc regions of human IgG1 (SEQ ID NO: 5), IgG2 (SEQ ID NO: 6), IgG3 (SEQ ID NO: 7), or IgG4 (SEQ ID NO: 8).

Low-molecular-weight antibody

The antibodies used in the present disclosure are not limited to full-length antibody molecules, and can be low-molecular-weight antibodies (minibodies) and modified products thereof. A low-molecular-weight antibody includes an antibody fragment that lacks a portion of a full-length antibody (for example, whole antibody such as whole IgG); and is not particularly limited as long as it has an antigen-binding activity. The low-molecular-weight antibody of the present disclosure is not particularly limited as long as it is a portion of a full-length antibody, but preferably comprises a heavy-chain variable region (VH) and/or a light-chain variable region (VL). The amino acid sequence of VH or VL may have substitution(s), deletion(s), addition(s), and/or insertion(s). Furthermore, as long as it has an antigen-binding activity, VH and/or VL can be partially deleted. The variable region may be chimerized or humanized. Specific examples of antibody fragments include Fab, Fab', F(ab')2, and Fv. Specific examples of low-molecular-weight antibodies include Fab, Fab', F(ab')2, Fv, scFv (single chain Fv), diabody, and sc(Fv)2 (single chain (Fv)2). Multimers of these antibodies (for example, dimers, trimers, tetramers, and polymers) are also included in the low-molecular-weight antibodies of the present disclosure.

Antibody fragments can be produced by treating an antibody with an enzyme such as papain and pepsin. Alternatively, genes encoding these antibody fragments can be constructed, inserted into expression vectors, and then expressed in appropriate host cells (see, for example, Co et al., (J. Immunol. (1994) 152, 2968-2976); Better and Horwitz (Methods in Enzymology (1989) 178, 476-496), Plueckthun and Skerra (Methods in Enzymology (1989) 178, 476-496); Lamoyi (Methods in Enzymology (1989) 121, 652-663); Rousseaux (Methods in Enzymology (1989) 121, 663-669); and Bird, et al., TIBTECH (1991) 9, 132-137).

A diabody refers to a bivalent low-molecular-weight antibody constructed by gene fusion (Hollinger et al., (Proc. Natl. Acad. Sci. USA 90, 6444-6448 (1993)); EP 404,097; WO 1993/11161; and such). A diabody is a dimer composed of two polypeptide chains. Generally, in each polypeptide chain constituting the dimer, VL and VH are linked by a linker within the same chain. The linker in a diabody is generally short enough to prevent binding between VL and VH Specifically, the amino acid residues constituting the linker are, for example, about five residues. A linker between VL and VH that are encoded by the same polypeptide chain is too short to form a single-chain variable region fragment, and a dimer is formed between the polypeptide chains. As a result, diabodies have two antigen binding sites.

scFv can be obtained by linking the H-chain variable region and L-chain variable region of an antibody. In scFv, the H-chain variable region and L-chain variable region are ligated *via* a linker, preferably a peptide linker (Huston, et al., Proc. Natl. Acad. Sci. U.S.A. (1988) 85, 5879-5883). The H-chain variable region and L-chain variable region of scFv may be derived from any of the antibodies described herein. The peptide linker for ligating the variable regions is not particularly limited; and for example, any single-chain peptide consisting of 3 to 25 residues or so, or peptide linkers described later or such can be used as the linker. PCR methods such as those described above can be used for ligating the variable regions. DNA encoding scFv can be amplified by a PCR method using as a template either whole DNA or a partial DNA encoding a desired amino acid sequence, which is selected from a DNA sequence encoding the H chain or the H chain variable region of the above-mentioned antibody, and a DNA encoding the L chain or the L chain variable region of the above-mentioned antibody; and using a pair of primers having sequences corresponding to the sequences of the two ends. Next, a DNA having the desired sequence can be obtained by performing a PCR reaction using a

combination of a DNA encoding the peptide linker portion, and a pair of primers having sequences designed so that both ends of the DNA will be ligated to the H chain and the L chain, respectively. Once the scFv-encoding DNA is constructed, expression vectors having the DNA, and recombinant cells transformed with the expression vector can be obtained according to conventional methods. Furthermore, the scFvs can be obtained by culturing the resulting recombinant cells to express the scFv-encoding DNA.

sc(Fv)2 is a low-molecular-weight antibody prepared by linking two VHs and two VLs with linkers or such to form a single chain (Hudson et al. (J. Immunol. Methods 1999; 231: 177-189)). sc(Fv)2 can be produced, for example, by linking scFvs with a linker.

Moreover, antibodies in which two VHs and two VLs are arranged in the order of VH, VL, VH, and VL starting from the N-terminal side of a single chain polypeptide ([VH]-linker-[VL]-linker-[VH]-linker-[VL]) are preferred. The order of the two VHs and the two VLs is not particularly limited to the above-mentioned arrangement, and they may be arranged in any order. Examples include the following arrangements:

[VL]-linker-[VH]-linker-[VH]-linker-[VL]

[VH]-linker-[VL]-linker-[VL]-linker-[VH]

[VH]-linker-[VH]-linker-[VL]-linker-[VL]

[VL]-linker-[VL]-linker-[VH]-linker-[VH]

[VL]-linker-[VH]-linker-[VL]-linker-[VH]

A linker similar to the linker described in the section "Antigen-binding molecules" above may be used as the linker for linking the antibody variable regions. A particularly preferred embodiment of sc(Fv)2 in the present disclosure includes, for example, the following sc(Fv)2:
[VH]-peptide linker (15 amino acids)-[VL]-peptide linker (15 amino acids)-[VH]-peptide linker (15 amino acids)-[VL]

Typically, three linkers are required to link four antibody variable regions. The linkers to be used may be the same or different linkers may be used.

Such low-molecular-weight antibody can be obtained by treating an antibody with an enzyme such as papain or pepsin to generate antibody fragments, or by constructing DNAs that encode these antibody fragments or low-molecular-weight antibodies, inserting them into expression vectors, and then expressing them in appropriate host cells (see, for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

A non-limiting embodiment of antibodies in the present disclosure includes but is not limited to chimeric antigen receptors that are incorporated into T-cells, which are fusions of an antibody or fragments thereof that recognize antigens instead of a T-cell receptor and T-cell signal domains, as well as T-cells into which the chimeric antigen receptor has been incorporated.

Single-domain antibody

A suitable example of the antigen-binding domain of the present invention includes a single-domain antibody (sdAb).

The structure implied by the term "single-domain antibody" in the present specification is not limited as long as the domain can exert antigen-binding activity by itself. Conventional antibodies exemplified by IgG antibodies and such show antigen-binding activity when a variable region is formed by pairing VH and VL, whereas single-domain antibodies do not pair with other domains, and are known to exert antigen-binding activity by just the domain structure of the single-domain antibody itself. Single-domain antibodies usually have a relatively low molecular weight and are present in monomeric form.

Examples of single-domain antibodies include, but are not limited to, antigen-binding molecules that are congenitally lacking a light chain, such as VHH of camelids, VNAR of sharks, or antibody fragments comprising the whole or part of an antibody VH domain or the whole or part of an antibody VL domain. Examples of single-domain antibodies that are antibody fragments comprising the whole or part of an antibody VH domain or the whole or part of the antibody VL domain include, but are not limited to, single-domain antibodies that are artificially produced starting from human antibody VH or human antibody VL as described in, for example, US Patent No. 6,248,516 B1. In some embodiments of the invention, one single-domain antibody has three CDRs (CDR1, CDR2 and CDR3).

The single-domain antibody can be obtained from an animal capable of producing a single-domain antibody or by immunizing an animal capable of producing a single-domain antibody. Examples of animals capable of producing a single-domain antibody include, but are not limited to, camelids and transgenic animals into which a gene capable of producing a single-domain antibody has been introduced. Camelids include camels, llamas, alpacas, dromedaries, and guanacos. Examples of transgenic animals into which a gene capable of producing a single domain antibody has been introduced include, but are not limited to, the transgenic animals described in International Publication WO2015/143414 and US Patent Publication US2011/0123527 A1. A humanized single-domain antibody can be obtained by converting the framework sequence of a single-domain antibody obtained from an animal to a human germline sequence or a sequence similar thereto. A humanized single-domain antibody (e.g., humanized VHH) is also an embodiment of the single-domain antibody of the present invention. "Humanized single-domain antibody" refers to a chimeric single-domain antibody that comprises amino acid residues from non-human CDR and amino acid residues from human FR. In some embodiments, in the humanized single-domain antibody, all or substantially all CDRs correspond to those of a non-human antibody, and all or substantially all FRs correspond to those of a human antibody. In a humanized antibody, even if some of the residues in the FR do not correspond to those of a human antibody, it is considered as an example where substantially all FRs correspond to those of the human antibody. For example, when humanizing VHH, which is an embodiment of a single-domain antibody, it is necessary to make some of the residues within the FR as residues not corresponding to those of the human antibody (C Vincke et al., The Journal of Biological Chemistry) 284, 3273-3284.).

In addition, the single-domain antibody can be obtained from a polypeptide library containing single-domain antibodies by ELISA, panning, or the like. Examples of polypeptide libraries containing single-domain antibodies include, but are not limited to, naive antibody libraries obtained from various animals or humans (e.g., Methods in Molecular Biology 2012 911 (65-78), Biochimica et Biophysica Acta-Proteins and Proteomics 2006 1764: 8 (1307-1319)), antibody libraries obtained by immunizing various animals (e.g., Journal of Applied Microbiology 2014 117: 2 (528-536)), or synthetic antibody libraries prepared from antibody genes of various animals or humans (e.g., Journal of Biomolecular Screening 2016 21: 1 (35-43), Journal of Biological Chemistry 2016 291: 24 (12641-12657), AIDS 2016 30:11 (1691-1701)).

Methods of producing an antibody having a desired binding activity to an antigen

Methods of producing an antibody that is not dependent on a small molecule compound including MTA and has a desired binding activity against an antigen that is a molecule different from the small molecule compound are known to those skilled in the art. A method of producing an antibody that binds to IL-6R (anti-IL-6R antibody) is exemplified below. Antibodies that bind to antigens other than IL-6R can also be appropriately prepared according to the following example.

Anti-IL-6R antibodies can be obtained as polyclonal or monoclonal antibodies using known methods. The anti-IL-6R antibodies preferably produced are monoclonal antibodies derived from mammals. Such mammal-derived monoclonal antibodies include antibodies produced by hybridomas or host cells transformed with an expression vector carrying an antibody gene by genetic engineering techniques. "Humanized antibodies" or "chimeric antibodies" are included in the monoclonal antibodies of the present invention.

Monoclonal antibody-producing hybridomas can be produced using known techniques, for example, as described below. Specifically, mammals are immunized by conventional immunization methods using an IL-6R protein as a sensitizing antigen. Resulting immune cells are fused with known parental cells by conventional cell fusion methods. Then, hybridomas producing an anti-IL-6R antibody can be selected by screening for monoclonal antibody-producing cells using conventional screening methods.

Specifically, monoclonal antibodies are prepared as mentioned below. First, the IL-6R gene whose nucleotide sequence is disclosed in SEQ ID NO: 2 can be expressed to produce an IL-6R protein shown in SEQ ID NO: 1, which will be used as a sensitizing antigen for antibody preparation. That is, a gene sequence encoding IL-6R is inserted into a known expression vector, and appropriate host cells are transformed with this vector. The desired human IL-6R protein is purified from the host cells or their culture supernatants by known methods. In order to obtain soluble IL-6R from culture supernatants, for example, a protein consisting of the amino acids at positions 1 to 357 in the IL-6R polypeptide sequence of SEQ ID NO: 1, such as described in Mullberg et al. (J. Immunol. (1994) 152 (10), 4958-4968), is expressed as a soluble IL-6R, instead of the IL-6R protein of SEQ ID NO: 1. Purified natural IL-6R protein can also be used as a sensitizing antigen.

The purified IL-6R protein can be used as a sensitizing antigen for immunization of mammals. A partial IL-6R peptide may also be used as a sensitizing antigen. In this case, a partial peptide can be prepared by chemical synthesis based on the amino acid sequence of human IL-6R, or by inserting a partial IL-6R gene into an expression vector for expression. Alternatively, a partial peptide can be produced by degrading an IL-6R protein with a protease. The length and region of the partial IL-6R peptide are not limited to particular embodiments. A preferred region can be arbitrarily selected from the amino acid sequence at amino acid positions 20 to 357 in the amino acid sequence of SEQ ID NO:

1. The number of amino acids forming a peptide to be used as a sensitizing antigen is preferably at least five or more, six or more, or seven or more. More specifically, a peptide of 8 to 50 residues, more preferably 10 to 30 residues can be used as a sensitizing antigen.

For sensitizing antigen, alternatively it is possible to use a fusion protein prepared by fusing a desired partial polypeptide or peptide of the IL-6R protein with a different polypeptide. For example, antibody Fc fragments and peptide tags are preferably used to produce fusion proteins to be used as sensitizing antigens. Vectors for expression of such fusion proteins can be constructed by fusing in frame genes encoding two or more desired polypeptide fragments and inserting the fusion gene into an expression vector as described above. Methods for producing fusion proteins are described in Molecular Cloning 2nd ed. (Sambrook, J et al., Molecular Cloning 2nd ed., 9.47-9.58 (1989) Cold Spring Harbor Lab. Press). Methods for preparing IL-6R to be used as a sensitizing antigen, and immunization methods using IL-6R are specifically described in WO 2003/000883, WO 2004/022754, WO 2006/006693, and such.

There is no particular limitation on the mammals to be immunized with the sensitizing antigen. However, it is preferable to select the mammals by considering their compatibility with the parent cells to be used for cell fusion. In general, rodents such as mice, rats, and hamsters, rabbits, and monkeys are preferably used.

The above animals are immunized with a sensitizing antigen by known methods. Generally performed immunization methods include, for example, intraperitoneal or subcutaneous injection administration of a sensitizing antigen into mammals. Specifically, a sensitizing antigen is appropriately diluted with PBS (Phosphate-Buffered Saline), physiological saline, or the like. If desired, a conventional adjuvant such as Freund's complete adjuvant is mixed with the antigen, and the mixture is emulsified. Then, the sensitizing antigen is administered to a mammal several times at 4- to 21-day intervals. Appropriate carriers may be used in immunization with the sensitizing antigen. In particular, when a low-molecular-weight partial peptide is used as the sensitizing antigen, it is sometimes desirable to couple the sensitizing antigen peptide to a carrier protein such as albumin or keyhole limpet hemocyanin for immunization.

Alternatively, hybridomas producing a desired antibody can be prepared using DNA immunization as mentioned below. DNA immunization is an immunization method that confers immunostimulation by expressing a sensitizing antigen in an animal immunized as a result of administering a vector DNA constructed to allow expression of an antigen proteinencoding gene in the animal. As compared to conventional immunization methods in which a protein antigen is administered to animals to be immunized, DNA immunization is expected to be superior in that:

- immunostimulation can be provided while retaining the structure of a membrane protein such as IL-6R; and
- there is no need to purify the antigen for immunization.

In order to prepare a monoclonal antibody of the present invention using DNA immunization, first, a DNA expressing an IL-6R protein is administered to an animal to be immunized. The IL-6R-encoding DNA can be synthesized by known methods such as PCR.

The obtained DNA is inserted into an appropriate expression vector, and then this is administered to an animal to be immunized. Preferably used expression vectors include, for example, commercially-available expression vectors such as pcDNA3.1. Vectors can be administered to an organism using conventional methods. For example, DNA immunization is performed by using a gene gun to introduce expression vector-coated gold particles into cells in the body of an animal to be immunized. Antibodies that recognized IL-6R can also be produced by the methods described in WO 2003/104453.

After immunizing a mammal as described above, an increase in the titer of an IL-6R-binding antibody is confirmed in the serum. Then, immune cells are collected from the mammal, and then subjected to cell fusion. In particular, splenocytes are preferably used as immune cells.

A mammalian myeloma cell is used as a cell to be fused with the above-mentioned immune cells. The myeloma cells preferably comprise a suitable selection marker for screening. A selection marker confers characteristics to cells for their survival (or death) under a specific culture condition. Hypoxanthine-guanine phosphoribosyltransferase deficiency (hereinafter abbreviated as HGPRT deficiency) and thymidine kinase deficiency (hereinafter abbreviated as TK deficiency) are known as selection markers. Cells with HGPRT or TK deficiency have hypoxanthine-aminopterin-thymidine sensitivity (hereinafter abbreviated as HAT sensitivity). HAT-sensitive cells cannot synthesize DNA in a HAT selection medium, and are thus killed. However, when the cells are fused with normal cells, they can continue DNA synthesis using the salvage pathway of the normal cells, and therefore they can grow even in the HAT selection medium.

HGPRT-deficient and TK-deficient cells can be selected in a medium containing 6-thioguanine, 8-azaguanine (hereinafter abbreviated as 8AG), or 5'-bromodeoxyuridine, respectively. Normal cells are killed because they incorporate these pyrimidine analogs into their DNA. Meanwhile, cells that are deficient in these enzymes can survive in the selection medium, since they cannot incorporate these pyrimidine analogs. In addition, a selection marker referred to as G418 resistance provided by the neomycin-resistant gene confers resistance to 2-deoxystreptamine antibiotics (gentamycin analogs). Various types of myeloma cells that are suitable for cell fusion are known.

For example, myeloma cells including the following cells can be preferably used: P3(P3x63Ag8.653) (J. Immunol. (1979) 123 (4), 1548-1550);

P3x63Ag8U.1 (Current Topics in Microbiology and Immunology (1978)81, 1-7);
NS-1 (C. Eur. J. Immunol. (1976)6 (7), 511-519);
MPC-11 (Cell (1976) 8 (3), 405-415);
SP2/0 (Nature (1978) 276 (5685), 269-270);
FO (J. Immunol. Methods (1980) 35 (1-2), 1-21);
S194/5.XX0.BU.1 (J. Exp. Med. (1978) 148 (1), 313-323);
R210 (Nature (1979) 277 (5692), 131-133), etc.

Cell fusions between the immunocytes and myeloma cells are essentially carried out using known methods, for example, a method by Kohler and Milstein et al. (Methods Enzymol. (1981) 73: 3-46).

More specifically, cell fusion can be carried out, for example, in a conventional culture medium in the presence of a cell fusion-promoting agent. The fusion-promoting agents include, for example, polyethylene glycol (PEG) and Sendai virus (HVJ). If required, an auxiliary substance such as dimethyl sulfoxide is also added to improve fusion efficiency.

The ratio of immune cells to myeloma cells may be determined at one's own discretion, preferably, for example, one myeloma cell for every one to ten immunocytes. Culture media to be used for cell fusions include, for example, media that are suitable for the growth of myeloma cell lines, such as RPMI1640 medium and MEM medium, and other conventional culture medium used for this type of cell culture. In addition, serum supplements such as fetal calf serum (FCS) may be preferably added to the culture medium.

For cell fusion, predetermined amounts of the above immune cells and myeloma cells are mixed well in the above culture medium. Then, a PEG solution (for example, the average molecular weight is about 1,000 to 6,000) prewarmed to about 37°C is added thereto at a concentration of generally 30% to 60% (w/v). This is gently mixed to produce desired fusion cells (hybridomas). Then, an appropriate culture medium mentioned above is gradually added to the cells, and this is repeatedly centrifuged to remove the supernatant. Thus, cell fusion agents and such which are unfavorable to hybridoma growth can be removed.

The hybridomas thus obtained can be selected by culture using a conventional selective medium, for example, HAT medium (a culture medium containing hypoxanthine, aminopterin, and thymidine). Cells other than the desired hybridomas (non-fused cells) can be killed by continuing culture in the above HAT medium for a sufficient period of time.

Typically, the period is several days to several weeks. Then, hybridomas producing the desired antibody are screened and singly cloned by conventional limiting dilution methods.

The hybridomas thus obtained can be selected using a selection medium based on the selection marker possessed by the myeloma used for cell fusion. For example, HGPRT- or TK-deficient cells can be selected by culture using the HAT medium (a culture medium containing hypoxanthine, aminopterin, and thymidine). Specifically, when HAT-sensitive myeloma cells are used for cell fusion, cells successfully fused with normal cells can selectively proliferate in the HAT medium. Cells other than the desired hybridomas (non-fused cells) can be killed by continuing culture in the above HAT medium for a sufficient period of time. Specifically, desired hybridomas can be selected by culture for generally several days to several weeks. Then, hybridomas producing the desired antibody are screened and singly cloned by conventional limiting dilution methods.

Desired antibodies can be preferably selected and singly cloned by screening methods based on known antigen/antibody reaction. For example, an IL-6R-binding monoclonal antibody can bind to IL-6R expressed on the cell surface. Such a monoclonal antibody can be screened by fluorescence activated cell sorting (FACS). FACS is a system that assesses the binding of an antibody to cell surface by analyzing cells contacted with a fluorescent antibody using laser beam, and measuring the fluorescence emitted from individual cells.

To screen for hybridomas that produce a monoclonal antibody of the present invention by FACS, IL-6R-expressing cells are first prepared. Cells preferably used for screening are mammalian cells in which IL-6R is forcedly expressed. As control, the activity of an antibody to bind to cell-surface IL-6R can be selectively detected using non-transformed mammalian cells as host cells. Specifically, hybridomas producing an anti-IL-6R monoclonal antibody can be isolated by selecting hybridomas that produce an antibody which binds to cells forced to express IL-6R, but not to host cells.

Alternatively, the activity of an antibody to bind to immobilized IL-6R-expressing cells can be assessed based on the principle of ELISA. For example, IL-6R-expressing cells are immobilized to the wells of an ELISA plate. Culture supernatants of hybridomas are contacted with the immobilized cells in the wells, and antibodies that bind to the immobilized cells are detected. When the monoclonal antibodies are derived from mouse, antibodies bound to the cells can be detected using an anti-mouse immunoglobulin antibody. Hybridomas producing a desired antibody having the antigen-binding ability are selected by the above screening, and they can be cloned by a limiting dilution method or the like.

Monoclonal antibody-producing hybridomas thus prepared can be passaged in a conventional culture medium,

and stored in liquid nitrogen for a long period.

The above hybridomas are cultured by a conventional method, and desired monoclonal antibodies can be prepared from the culture supernatants. Alternatively, the hybridomas are administered to and grown in compatible mammals, and monoclonal antibodies are prepared from the ascites. The former method is suitable for preparing antibodies with high purity.

Antibodies encoded by antibody genes that are cloned from antibody-producing cells such as the above hybridomas can also be preferably used. A cloned antibody gene is inserted into an appropriate vector, and this is introduced into a host to express the antibody encoded by the gene. Methods for isolating antibody genes, inserting the genes into vectors, and transforming host cells have already been established, for example, by Vandamme et al. (Eur. J. Biochem. (1990) 192(3), 767-775). Methods for producing recombinant antibodies are also known as described below.

For example, a cDNA encoding the variable region (V region) of an anti-IL-6R antibody is prepared from hybridoma cells expressing the anti-IL-6R antibody. For this purpose, total RNA is first extracted from hybridomas. Methods used for extracting mRNAs from cells include, for example:

- the guanidine ultracentrifugation method (Biochemistry (1979) 18(24), 5294-5299), and
- the AGPC method (Anal. Biochem. (1987) 162(1), 156-159)

Extracted mRNAs can be purified using the mRNA Purification Kit (GE Healthcare Bioscience) or such. Alternatively, kits for extracting total mRNA directly from cells, such as the QuickPrep mRNA Purification Kit (GE Healthcare Bioscience), are also commercially available. mRNAs can be prepared from hybridomas using such kits. cDNAs encoding the antibody V region can be synthesized from the prepared mRNAs using a reverse transcriptase. cDNAs can be synthesized using the AMV Reverse Transcriptase First-strand cDNA Synthesis Kit (Seikagaku Co.) or such. Furthermore, the SMART RACE cDNA amplification kit (Clontech) and the PCR-based 5'-RACE method (Proc. Natl. Acad. Sci. U.S.A. (1988) 85(23), 8998-9002; Nucleic Acids Res. (1989) 17(8), 2919-2932) can be appropriately used to synthesize and amplify cDNAs. In such a cDNA synthesis process, appropriate restriction enzyme sites described below may be introduced into both ends of a cDNA.

The cDNA fragment of interest is purified from the resulting PCR product, and then this is ligated to a vector DNA. A recombinant vector is thus constructed, and introduced into *E. coli* or such. After colony selection, the desired recombinant vector can be prepared from the colony-forming *E. coli.* Then, whether the recombinant vector has the cDNA nucleotide sequence of interest is tested by a known method such as the dideoxy nucleotide chain termination method.

The 5'-RACE method which uses primers to amplify the variable region gene is conveniently used for isolating the gene encoding the variable region. First, a 5'-RACE cDNA library is constructed by cDNA synthesis using RNAs extracted from hybridoma cells as a template. A commercially available kit such as the SMART RACE cDNA amplification kit is appropriately used to synthesize the 5'-RACE cDNA library.

The antibody gene is amplified by PCR using the prepared 5'-RACE cDNA library as a template. Primers for amplifying the mouse antibody gene can be designed based on known antibody gene sequences. The nucleotide sequences of the primers vary depending on the immunoglobulin subclass. Therefore, it is preferable that the subclass is determined in advance using a commercially available kit such as the Iso Strip mouse monoclonal antibody isotyping kit (Roche Diagnostics).

Specifically, for example, primers that allow amplification of genes encoding $\gamma$1, $\gamma$2a, $\gamma$2b, and $\gamma$3 heavy chains and $\kappa$ and $\lambda$ light chains are used to isolate mouse IgG-encoding genes. In general, a primer that anneals to a constant region site close to the variable region is used as a 3'-side primer to amplify an IgG variable region gene. Meanwhile, a primer attached to a 5' RACE cDNA library construction kit is used as a 5'-side primer.

PCR products thus amplified are used to reshape immunoglobulins composed of a combination of heavy and light chains. A desired antibody can be selected using the IL-6R-binding activity of a reshaped immunoglobulin as an indicator. For example, when the objective is to isolate an antibody against IL-6R, it is more preferred that the binding of the antibody to IL-6R is specific. An IL-6R-binding antibody can be screened, for example, by the following steps:

(1) contacting an IL-6R-expressing cell with an antibody comprising the V region encoded by a cDNA isolated from a hybridoma;
(2) detecting the binding of the antibody to the IL-6R-expressing cell; and
(3) selecting an antibody that binds to the IL-6R-expressing cell.

Methods for detecting the binding of an antibody to IL-6R-expressing cells are known. Specifically, the binding of an antibody to IL-6R-expressing cells can be detected by the above-described techniques such as FACS. Immobilized samples of IL-6R-expressing cells are appropriately used to assess the binding activity of an antibody.

Preferred antibody screening methods that use the binding activity as an indicator also include panning methods

using phage vectors. Screening methods using phage vectors are advantageous when the antibody genes are isolated from heavy-chain and light-chain subclass libraries from a polyclonal antibody-expressing cell population. Genes encoding the heavy-chain and light-chain variable regions can be linked by an appropriate linker sequence to form a single-chain Fv (scFv). Phages presenting scFv on their surface can be produced by inserting a gene encoding scFv into a phage vector. The phages are contacted with an antigen of interest. Then, a DNA encoding scFv having the binding activity of interest can be isolated by collecting phages bound to the antigen. This process can be repeated as necessary to enrich scFv having the binding activity of interest.

After isolation of the cDNA encoding the V region of the anti-IL-6R antibody of interest, the cDNA is digested with restriction enzymes that recognize the restriction sites introduced into both ends of the cDNA. Preferred restriction enzymes recognize and cleave a nucleotide sequence that occurs in the nucleotide sequence of the antibody gene at a low frequency. Furthermore, a restriction site for an enzyme that produces a sticky end is preferably introduced into a vector to insert a single-copy digested fragment in the correct orientation. The cDNA encoding the V region of the anti-IL-6R antibody is digested as described above, and this is inserted into an appropriate expression vector to construct an antibody expression vector. In this case, if a gene encoding the antibody constant region (C region) and a gene encoding the above V region are fused in-frame, a chimeric antibody is obtained. Herein, "chimeric antibody" means that the origin of the constant region is different from that of the variable region. Thus, in addition to mouse/human heterochimeric antibodies, human/human allochimeric antibodies are included in the chimeric antibodies of the present invention. A chimeric antibody expression vector can be constructed by inserting the above V region gene into an expression vector that already has the constant region. Specifically, for example, a recognition sequence for a restriction enzyme that excises the above V region gene can be appropriately placed on the 5' side of an expression vector carrying a DNA encoding a desired antibody constant region. A chimeric antibody expression vector is constructed by fusing in frame the two genes digested with the same combination of restriction enzymes.

To produce an anti-IL-6R monoclonal antibody, antibody genes are inserted into an expression vector so that the genes are expressed under the control of an expression regulatory region. The expression regulatory region for antibody expression includes, for example, enhancers and promoters. Furthermore, an appropriate signal sequence may be attached to the amino terminus so that the expressed antibody is secreted to the outside of cells. In the Examples below, a peptide having the amino acid sequence MGWSCIILFLVATATGVHS (SEQ ID NO: 3) is used as a signal sequence. Meanwhile, other appropriate signal sequences may be attached. The expressed polypeptide is cleaved at the carboxyl terminus of the above sequence, and the resulting polypeptide is secreted to the outside of cells as a mature polypeptide. Then, appropriate host cells are transformed with the expression vector, and recombinant cells expressing the anti-IL-6R antibody-encoding DNA are obtained.

DNAs encoding the antibody heavy chain (H chain) and light chain (L chain) are separately inserted into different expression vectors to express the antibody gene. An antibody molecule having the H and L chains can be expressed by co-transfecting the same host cell with vectors into which the H-chain and L-chain genes are respectively inserted. Alternatively, host cells can be transformed with a single expression vector into which DNAs encoding the H and L chains are inserted (see WO 1994/011523).

There are various known host cell/expression vector combinations for antibody preparation by introducing isolated antibody genes into appropriate hosts. All of these expression systems are applicable to isolation of the antigen-binding domains of the present invention. Appropriate eukaryotic cells used as host cells include animal cells, plant cells, and fungal cells. Specifically, the animal cells include, for example, the following cells.

(1) mammalian cells: CHO (Chinese hamster ovary cell line), COS (Monkey kidney cell line), myeloma (Sp2/0, NS0, etc.), BHK (baby hamster kidney cell line), HeLa, Vero, HEK293 (human embryonic kidney cell line with sheared adenovirus (Ad)5 DNA), PER.C6 cell (human embryonic retinal cell line transformed with the Adenovirus Type 5 (Ad5) E1A and E1B genes) and such (Current Protocols in Protein Science (May, 2001, Unit 5.9, Table 5.9.1));
(2) amphibian cells: Xenopus oocytes, or such; and
(3) insect cells: sf9, sf21, Tn5, or such.

In addition, as a plant cell, an antibody gene expression system using cells derived from the *Nicotiana* genus such as *Nicotiana tabacum* is known. Callus cultured cells can be appropriately used to transform plant cells.

Furthermore, the following cells can be used as fungal cells:

yeasts: the *Saccharomyces* genus such as *Saccharomyces cerevisiae,* and the *Pichia* genus such as *Pichia pastoris;* and
filamentous fungi: the *Aspergillus* genus such as *Aspergillus niger.*

Furthermore, antibody gene expression systems that utilize prokaryotic cells are also known. For example, when using bacterial cells, *E. coli* cells, *Bacillus subtilis* cells, and such can suitably be utilized in the present invention.

Expression vectors carrying the antibody genes of interest are introduced into these cells by transfection. The transfected cells are cultured *in vitro,* and the desired antibody can be prepared from the culture of transformed cells.

In addition to the above-described host cells, transgenic animals can also be used to produce a recombinant antibody. That is, the antibody can be obtained from an animal into which the gene encoding the antibody of interest is introduced. For example, the antibody gene can be constructed as a fusion gene by inserting in frame into a gene that encodes a protein produced specifically in milk. Goat β-casein or such can be used, for example, as the protein secreted in milk. DNA fragments containing the fused gene inserted with the antibody gene is injected into a goat embryo, and then this embryo is introduced into a female goat. Desired antibodies can be obtained as a protein fused with the milk protein from milk produced by the transgenic goat born from the embryo-recipient goat (or progeny thereof). In addition, to increase the volume of milk containing the desired antibody produced by the transgenic goat, hormones can be administered to the transgenic goat as necessary (Bio/Technology (1994) 12 (7), 699-702).

When an antigen-binding molecule described herein is administered to human, an antigen-binding domain derived from a genetically recombinant antibody that has been artificially altered to reduce the heterologous antigenicity against human and such, can be appropriately used as the antigen-binding domain of the antigen-binding molecule. Such genetically recombinant antibodies include, for example, humanized antibodies. These altered antibodies are appropriately produced by known methods.

As a method of producing an antibody having a desired binding activity for a specific small molecule compound, it is possible to obtain an antibody that has a desired binding activity for a small molecule compound by a method similar to a method of producing an antibody that binds to an ordinary protein antigen. One example of an embodiment of a method of producing a sensitizing antigen used for obtaining an antibody against a small molecule compound is the method of linking Mariculture Keyhole Limpet Hemocyanin (KLH) with a small molecule compound. A non-limiting example of a sensitizing antigen created to obtain an antibody against MTA is 6'-MTA-Keyhole Limpet Hemocyanin (6'-MTA-KLH). Mariculture Keyhole Limpet Hemocyanin (KLH) is a highly antigenic protein that can be recognized by T cell receptors expressed on helper T cells and is known to activate antibody production, and therefore, by linking with MTA, it is expected to enhance the production of antibodies against MTA. The small molecule compound to be linked to KLH is not limited to MTA, and sensitizing antigens can be prepared by similar methods for various small molecule compounds that can be synthesized, and non-limiting examples include AMP, ADP, ATP, adenosine, or SAH The international publication WO2013/180200 also discloses the design of small molecule compound immunogens.

Further, antigens to be linked to small molecule compounds are not limited to KLH, and for example, an antigen linked to biotin or the like may be used as a sensitizing antigen, and further, those other than KLH and biotin can be used if they can be linked to small molecule compounds.

The present disclosure also encompasses the embodiments exemplified below.

[1] Biotinylated MTA.

[2] Biotin-2'-MTA.

[3] 6'-MTA-biotin.

[4] Use of the biotinylated MTA according to [1] to [3] for the screening of an antigen-binding molecule that binds to MTA.

[5] Use of the biotinylated MTA according to [1] to [3] as an immunogen for obtaining an antigen-binding molecule that binds to MTA.

[6] A method of screening for an antigen-binding molecule that binds to MTA, which comprises using the biotinylated MTA according to [1] to [3].

Multispecific antigen-binding molecules or multiparatopic antigen-binding molecules

An antigen-binding molecule comprising at least two antigen-binding domains in which at least one of the antigen-binding domains binds to a first epitope in an antigen molecule, and at least another one of the antigen-binding domains binds to a second epitope in the antigen molecule, is called "multispecific antigen-binding molecule" from the viewpoint of its reaction specificity. When two types of antigen-binding domains contained in a single antigen-binding molecule allow binding to two different epitopes by the antigen-binding molecule, this molecule is called "bispecific antigen-binding molecule". When three types of antigen-binding domains contained in a single antigen-binding molecule allow binding to three different epitopes by the antigen-binding molecule, this antigen-binding molecule is called "trispecific antigen-binding molecule".

A paratope in the antigen-binding domain that binds to the first epitope in the antigen molecule and a paratope in the antigen-binding domain that binds to the second epitope which is structurally different from the first epitope have different structures. Therefore, an antigen-binding molecule comprising at least two antigen-binding domains in which at least one of the antigen-binding domains binds to a first epitope in an antigen molecule, and at least another one of the antigen-binding domains binds to a second epitope in the antigen molecule, is called "multiparatopic antigen-binding

molecule" from the viewpoint of the specificity of its structure. When two types of antigen-binding domains contained in a single antigen-binding molecule allow binding to two different epitopes by the antigen-binding molecule, this molecule is called "biparatopic antigen-binding molecule". When three types of antigen-binding domains contained in a single antigen-binding molecule allow binding to three different epitopes by the antigen-binding molecule, this molecule is called "triparatopic antigen-binding molecule".

Bispecific antibodies and methods for producing them

Multivalent multispecific or multiparatopic antigen-binding molecules comprising one or more antigen-binding domains and methods for preparing them are described in non-patent literatures such as Conrath et al., (J. Biol. Chem. (2001) 276 (10) 7346-7350), Muyldermans (Rev. Mol. Biotech. (2001) 74, 277-302), and Kontermann R.E. (2011) Bispecific Antibodies (Springer-Verlag), and in patent literatures such as WO1996/034103 and WO1999/023221. Antigen-binding molecules of the present disclosure can be produced using multispecific or multiparatopic antigen-binding molecules, and their preparation methods described in these documents.

In an embodiment, bispecific antibodies and methods for producing them are mentioned below as examples of one embodiment of the aforementioned multispecific or multiparatopic antigen-binding molecules and methods for preparing them. Bispecific antibodies are antibodies comprising two types of variable regions that bind specifically to different epitopes. IgG-type bispecific antibodies can be secreted from a hybrid hybridoma (quadroma) produced by fusing two types of hybridomas that produce IgG antibodies (Milstein et al., Nature (1983) 305, 537-540).

When a bispecific antibody is produced by using recombination techniques such as those described in the above-mentioned section on antibodies, one may adopt a method that introduces genes encoding heavy chains containing the two types of variable regions of interest into cells to co-express them. However, even when only the heavy-chain combination is considered, such a co-expression method will produce a mixture of (i) a combination of a pair of heavy chains in which one of the heavy chains contains a variable region that binds to a first epitope and the other heavy chain contains a variable region that binds to a second epitope, (ii) a combination of a pair of heavy chains which include only heavy chains containing a variable region that binds to the first epitope, and (iii) a combination of a pair of heavy chains which include only heavy chains containing a variable region that binds to the second epitope, which are present at a molecular ratio of 2:1:1. It is difficult to purify antigen-binding molecules containing the desired combination of heavy chains from the mixture of three types of heavy chain combinations.

When producing bispecific antibodies using such recombination techniques, bispecific antibodies containing a heteromeric combination of heavy chains can be preferentially secreted by adding appropriate amino acid substitutions in the CH3 domains constituting the heavy chains. Specifically, this method is conducted by substituting an amino acid having a larger side chain (knob (which means "bulge")) for an amino acid in the CH3 domain of one of the heavy chains, and substituting an amino acid having a smaller side chain (hole (which means "void")) for an amino acid in the CH3 domain of the other heavy chain so that the knob is placed in the hole. This promotes heteromeric heavy chain formation and simultaneously inhibits homomeric heavy chain formation (International Publication No. WO 1996027011; Ridgway et al., Protein Engineering (1996) 9, 617-621; Merchant et al., Nature Biotechnology (1998) 16, 677-681).

Furthermore, there are also known techniques for producing a bispecific antibody by applying methods for controlling polypeptide association, or association of polypeptide-formed heteromeric multimers to the association between heavy chains. Specifically, methods for controlling heavy chain formation may be employed to produce a bispecific antibody (International Publication No. WO 2006/106905), in which amino acid residues forming the interface between the heavy chains are altered to inhibit the association between the heavy chains having the same sequence and to allow the formation of heavy chains of different sequences. Such methods can be used for generating bispecific antibodies.

Cancer

Herein, the term "cancer" is generally used to describe malignant neoplasms, which may be metastatic or non-metastatic. Non-limiting examples of carcinomas developed from epithelial tissues such as skin or digestive tract include brain tumor, skin cancer, head and neck cancer, esophageal cancer, lung cancer, stomach cancer, duodenal cancer, breast cancer, prostate cancer, cervical cancer, endometrial cancer, pancreatic cancer, liver cancer, colorectal cancer, colon cancer, bladder cancer, and ovarian cancer. Non-limiting examples of sarcomas developed from non-epithelial (interstitial) tissues such as muscles include osteosarcoma, chondrosarcoma, rhabdomyosarcoma, leiomyosarcoma, liposarcoma, and angiosarcoma. Non-limiting examples of hematological cancer derived from hematopoietic organs include malignant lymphomas including Hodgkin’s lymphoma and non Hodgkin’s lymphoma; leukemia including acute myelocytic leukemia or chronic myelocytic leukemia, and acute lymphatic leukemia or chronic lymphatic leukemia; and multiple myeloma. The term "neoplasm" widely used herein refers to any newly formed diseased tissue tumor. In the present disclosure, neoplasms cause formation of tumors, which are partly characterized by angiogenesis. Neoplasms may be benign such as hemangioma, glioma, or teratoma, or malignant such as carcinoma, sarcoma, glioma, astrocytoma, neuroblastoma, or retinoblastoma.

The term "cancer tissue" refers to a tissue containing at least one cancer cell. Therefore, as cancer tissues contain cancer cells and blood vessels, it refers to all cell types contributing to the formation of a tumor mass containing cancer cells and endothelial cells. Herein, "tumor mass" refers to a foci of tumor tissue. The term "tumor" is generally used to mean a benign neoplasm or a malignant neoplasm.

In the present disclosure, "cancer tissue in which MTA is accumulated" means a cancer tissue among cancer tissues mentioned above in which a large amount of MTA is detected as compared with a normal tissue, and one non-limiting example of a normal tissue to be compared is a normal tissue adjacent to a cancer tissue or a tissue of a healthy individual.

In addition, cancer tissues lacking methylthioadenosine phosphorylase (MTAP), which metabolizes MTA, and cancer tissues with reduced MTAP function are also embodiments of cancer tissues in which MTA is accumulated. Specifically, cancer tissues in which the gene encoding MTAP is deficient or underexpressed, cancer tissues expressing mutations or splicing variants that reduce the activity of MTAP, or cancer tissues in which the enzyme activity of MTAP is reduced, are one embodiment of cancer tissues in which MTA is accumulated. Decreased expression or decreased function of MTAP can be determined by comparing with normal tissue, and non-limiting examples of normal tissue to be compared include normal tissue adjacent to a cancer tissue or a tissue of a healthy individual.

Cancer-associated fibroblasts (CAFs)

In the present disclosure, "Cancer-Associated Fibroblasts (CAFs)" are a heterogeneous population of various origin such as endothelial cells existing around a cancer tissue. Non-limiting characteristics of CAFs include promoting the growth of cancer cells, promoting angiogenesis, promoting vascular infiltration of cancer cells, and constructing a microenvironment favorable for cancer progression by controlling the immune response, etc. Furthermore, one example of a non-limiting embodiment of CAF is cells expressing markers selected from $\alpha$-smooth muscle actin (a-SMA), fibroblast activation protein (FAP), tenascin-C (TN-C), periostin (POSTN), NG2 chondroitin sulfate proteoglycan (NG2), platelet derived growth factor receptor (PDGFR), vimentin, desmin, fibroblast specific protein-1 (FSP1), and fibronectin.

Tumor-associated macrophages (TAMs)

In the present disclosure, "Tumor-associated macrophage (TAM)" refers to a macrophage present in a cancer tissue and its surroundings. Examples include macrophage populations that form the cancer microenvironment together with fibroblasts, vascular endothelial cells, and the like. Non-limiting examples of the characteristics of TAMs include the suppression of anti-tumor immunity by producing anti-inflammatory factors and promoting the infiltration of regulatory T cells, and the induction of new blood vessels by producing various angiogenic factors. Furthermore, examples of a non-limiting embodiment of TAMs include cells expressing a marker selected from CD163, CD204, IL-10, TGF-$\beta$, and prostaglandin E2.

Effector cells

In the present disclosure, the term "effector cells" may be used in the broadest sense including T cells ($CD4^+$ (helper lymphocyte) T cells and/or $CD8^+$ (cytotoxic) T cells), multinuclear leucocytes (neutrophils, eosinophils, basophils, mast cells), monocytes, macrophages, histiocytes, or leukocytes such as natural killer cells (NK cells), NK-like T cells, Kupffer cells, Langerhans cells, or lymphokine-activated killer cells (LAK cells), B-lymphocytes, or antigen-presenting cells such as dendritic cells or macrophages. Preferred examples of effector cells include $CD8^+$ (cytotoxic) T cells, NK cells, or macrophages. Membrane-type molecules expressed on the cell membrane of effector cells may be used as antigens to which at least one antigen-binding domain contained in the antigen-binding molecule of the present disclosure binds. Non-limiting examples of a preferred membrane-type molecule may be CD3, CD2, CD28, CD44, CD16, CD32, CD64, or NKG2D, NK cell-activating ligands, or polypeptides constituting TCR.

Methylthioadenosine (MTA)

The term "MTA" used in the present specification refers to methylthioadenosine, and specifically refers to a compound represented by the following chemical formula.

[Compound 1]

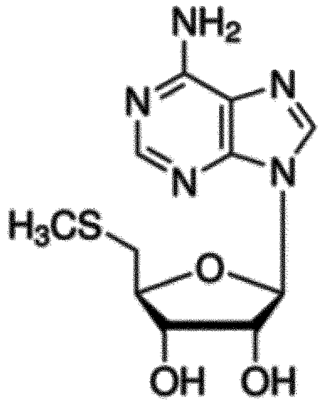

MTA (CAS No: 2457-80-9)

MTA analogs

The term "MTA analog" used in the present specification refers to a small molecule compound other than MTA, which has a structure partially common with MTA. Examples of MTA analogs include small molecule compounds having adenosine as a common skeleton in the molecules, as well as small molecule compounds having a side chain containing a sulfur atom or an oxygen atom in the carbon at the 5$^{th}$ position in adenosine. Further examples of MTA analogs suitably include, but are not limited to, metabolites of the polyamine biosynthetic pathway such as S-adenosylmethionine (SAM: S-adenosylmethionine), S-adenosylhomocysteine (SAH: S-adenosylhomocysteine, S-(5'-adenosyl)-L-homocysteine) (Stevens et al. (J Chromatogr A. 2010 May 7; 1217 (19): 3282-8)).

[Compound 2]

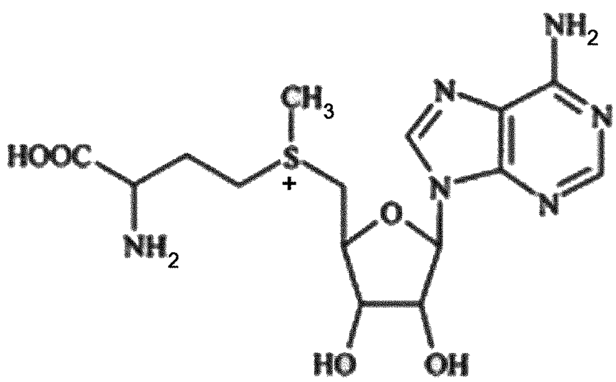

SAM

[Compound 3]

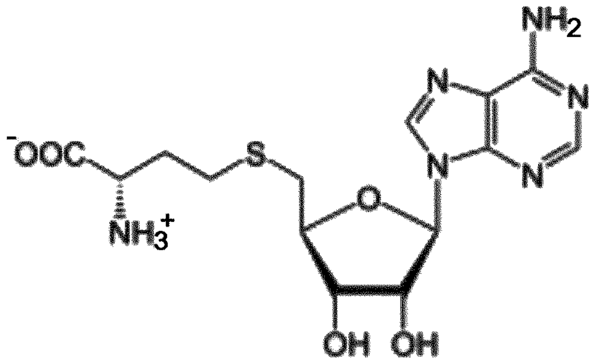

SAH

Further, examples of non-limiting embodiments of MTA analogs include, adenosine, adenosine triphosphate (ATP), adenosine diphosphate (ADP), and adenosine monophosphate

(AMP).

[Compound 4]

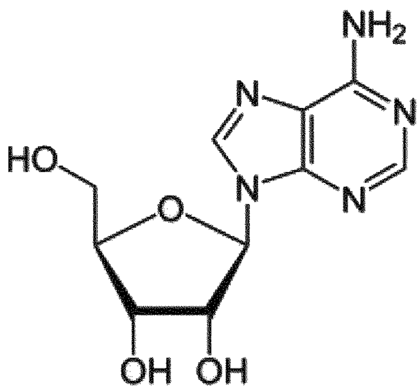

Adenosine

[Compound 5]

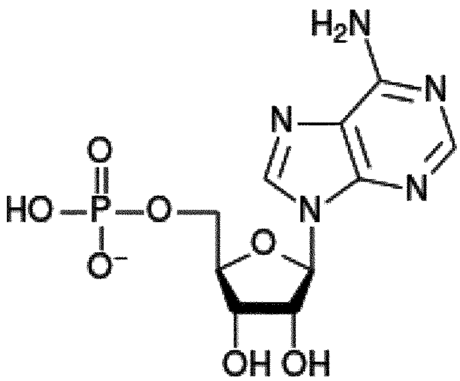

Adenosine monophosphate (AMP)

[Compound 6]

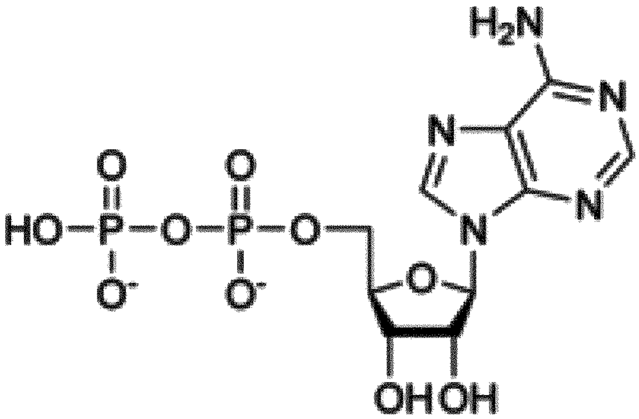

Adenosine diphosphate (ADP)

[Compound 7]

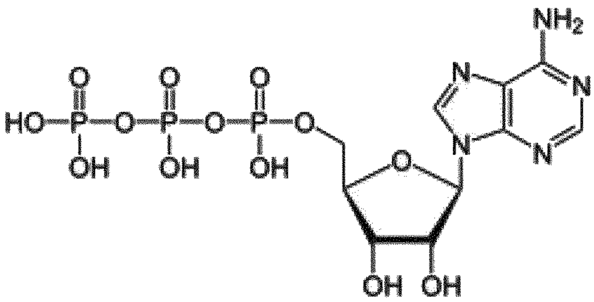

Adenosine triphosphate (ATP)

Small molecule compounds

The term "small molecule compound" used in the present disclosure refers to a naturally-occurring chemical substance or a non-naturally-occurring chemical substance other than a "biopolymer" existing in the living body. Examples of small molecule compounds include, but are not limited to, naturally-occurring or artificially-synthesized compounds having a molecular weight of 10,000 or less, preferably compounds having a molecular weight of 1000 or less. Non-limiting embodiments of small molecule compounds include, for example, cancer tissue-specific compounds, inflammatory tissue-specific compounds, and non-natural compounds.

Cancer tissue-specific compounds

As used herein, the term "compound specific to cancer tissue (cancer tissue-specific compound)" refers to a compound that is differentially present in a cancer tissue as compared to a non-cancer tissue.

For example, in several embodiments, cancer tissue-specific compounds may be compounds defined by qualitative properties of cancer tissues such as being present in cancer tissues but absent in non-cancer tissues, or being absent in cancer tissues but present in non-cancer tissues. In other embodiments, cancer tissue-specific compounds may be compounds defined by quantitative properties of cancer tissues such as being present in cancer tissues at a concentration different (for example, higher concentration or lower concentration) from that in non-cancer tissues. For

example, cancer tissue-specific compounds are present differentially at arbitrary concentrations. Generally, cancer tissue-specific compounds can be present at a concentration increased by at least 5%, at least 10%, at least 15%, at least 20%, at least 25%, at least 30%, at least 35%, at least40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 2-fold, at least 5-fold, at least 10-fold, at least 50-fold, at least 100-fold, at least $10^3$-fold, at least $10^4$-fold, at least $10^5$-fold, at least $10^6$-fold, or more, or up to infinity (i.e., when the compound is absent in non-cancerous tissues). Alternatively, they can generally be present at a concentration decreased by at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, or at least 100% (i.e., absent). Preferably, cancer tissue-specific compounds are differentially present at statistically significant concentrations (that is, as determined using either Welch's t-test or Wilcoxon rank sum test, the p value is less than 0.05 and/or the q value is less than 0.10). Examples of a non-limiting embodiment of a cancer tissue-specific compound include compounds which are cancer tissue-specific metabolites produced by metabolic activities characteristic of cancer cells, immune cells, or stromal cells contained in cancer tissues, such as those described below (cancer tissue-specific metabolites, cancer cell-specific metabolites, metabolites specific to immune cells that infiltrated into cancer tissues, and cancer stromal cell-specific metabolites).

Cancer tissue-specific metabolites

The term "metabolism" refers to chemical changes that take place in biological tissues and includes "anabolism" and "catabolism". Anabolism refers to biosynthesis or accumulation of molecules, and catabolism refers to degradation of molecules. "Metabolites" are intermediates or products that arise from metabolism. "Primary metabolites" refers to metabolites directly involved in the process of growth or proliferation of cells or organisms. "Secondary metabolites" refer to products that are not directly involved in such process of growth or proliferation, and are products such as pigments or antibiotics that are produced as a result of metabolism which biosynthesizes substances that are not directly involved in biological phenomena common to cells and organisms. The metabolites may be metabolites of "biopolymers", or they may be metabolites of "small molecules". "Biopolymers" are polymers comprising one or more types of repeating units. Biopolymers are generally found in biological systems, and examples include cells forming the organism and intercellular matrices that adhere to them, molecules having a molecular weight of approximately 5000 or more which form structures such as interstitial matrices, particularly polysaccharides (carbohydrates and such), peptides (this term is used so as to include polypeptides and proteins), and polynucleotides, and similarly their analogs such as compounds composed of or including amino acid analogs or nonamino acid groups.

Suitable examples of a non-limiting embodiment of a cancer tissue-specific metabolite described herein include cancer cell-specific small-molecule metabolites (Eva Gottfried, Katrin Peter and Marina P. Kreutz, From Molecular to Modular Tumor Therapy (2010) 3 (2), 111-132). In addition, metabolites that are highly produced by immune cells that infiltrate into cancer tissues, and metabolites that are highly produced by stromal cells that support the survival and/or growth of cancer cells (cancer stromal cells or cancer associated stromal fibroblasts (CAF)) are also included. Infiltrating immune cells are, for example, dendritic cells, inhibitory dendritic cells, inhibitory T cells, exhausted T cells, and myeloma derived suppressor cells (MDSC). Furthermore, metabolites of the present invention include compounds released from inside the cells to outside the cells when cells present in cancer tissues (cancer cells, immune cells, or stromal cells) die due to apoptosis, necrosis, or such.

To identify cancer cell-specific metabolites, metabolomic analyses focused on metabolic profiling can be suitably used, in addition to transcriptome-level analyses (for example, Dhanasekaran et al. (Nature (2001) 412, 822-826), Lapointe et al. (Proc. Natl. Acad. Sci. U.S.A. (2004) 101, 811-816) or Perou et al. (Nature (2000) 406, 747-752)) and proteomelevel analyses (for example, Ahram et al. (Mol. Carcinog. (2002) 33, 9-15), Hood et al. (Mol. Cell. Proteomics (2005) 4, 1741-1753)). More specifically, to identify metabolites in test samples, metabolic profiling that uses high-pressure liquid chromatography (HPLC), nuclear magnetic resonance (NMR) (Brindle et al. (J. Mol. Recognit. (1997) 10, 182-187), mass spectrometry (Gates and Sweeley (Clin. Chem. (1978) 24, 1663-1673) (GC/MS and LC/MS)), and ELISA or such individually and/or in combination may be used appropriately.

These studies elucidated heterogeneity within the constituted tumors which results from changing the concentration gradient of growth factors and metabolites (glucose, oxygen, or such) that enable cancer cell growth under low oxygen pressure conditions (Dang and Semenza (Trends Biochem. Sci. (1999) 24, 68-72)). In these studies, cell line models are also used to understand the change in energy utilization pathway depending on the different malignancy levels of tumors (Vizan et al. (Cancer Res. (2005) 65, 5512-5515)). Examples of a non-limiting embodiment of the technical components of the metabolomics platform include sample extraction, separation, detection, spectroscopic analysis, data normalization, description of classspecific metabolites, pathway mapping, confirmation, and functional characterization of candidate metabolites described by Lawton et al. (Pharmacogenomics (2008) 9, 383). These methods enable identification of cancer cell-specific metabolites in desired cancer tissues.

Inflammatory tissue-specific compounds

The term "compound specific to inflammatory tissue (inflammatory tissue-specific compound)" as used herein refers to a compound that is present differentially in inflammatory tissues as compared to non-inflammatory tissues. Herein, examples of "inflammatory tissues" include:

- joints with rheumatoid arthritis or osteoarthritis;
- lungs (alveoli) with bronchial asthma or COPD;
- digestive organs of inflammatory bowel disease, Crohn's disease, or ulcerative colitis;
- fibrotic tissues of fibrosis of the liver, kidney, or lung;
- tissues undergoing rejection reaction in organ transplantation;
- blood vessels and heart (myocardium) in arteriosclerosis or heart failure;
- visceral fat in metabolic syndrome;
- skin tissues in atopic dermatitis or other dermatitis; and
- spinal nerves in disk herniation or chronic low back pain.

Inflammatory tissue-specific metabolites

"Inflammatory tissue-specific metabolite" refers to metabolites highly produced by immune cells that have infiltrated into inflammatory tissues, and metabolites highly produced by specifically normal cells that have been damaged in inflammatory tissues. Examples of infiltrating immune cells include effector T cells, mature dendritic cells, neutrophils, granule cells (mast cells), and basophils. Furthermore, metabolites in the present invention include compounds that are released from inside the cells to the outside of the cells when the cells that are present in inflammatory tissues (immune cells and normal cells) die by apoptosis, necrosis, or such.

The term "unnatural compound" as used herein refers to an unnaturally derived chemical substance and its metabolites. An embodiment of the invention is an unnaturally derived chemical substance that has the property of accumulating at the target tissue after being administered to a living body from outside the body, and metabolites thereof. Examples of an unnatural compound include (1) Capecitabine (Xeloda) and its metabolite 5-FU (fluorouracil), and (2) TH-302 and bromo-isophosphoramide mustard (Br-IPM). 5-FU is a metabolite of Capecitabine (Xeloda), and is known to be metabolized by cytidine deaminase and thymidine phosphorylase which are metabolic enzymes specific in cancer tissues (Desmoulin F. et al. Drug Metab Dispos. 2002). TH-302 is known to be converted to Br-IPM by reduction under a low-oxygen condition as in the periphery of cancer tissues (Duan JX, et al. J Med Chem. 2008). For example, when Capecitabine (Xeloda) is administered, it is metabolized into 5-FU by cancerspecific metabolic enzymes, and therefore, the concentration of 5-FU becomes high at the cancer site (Desmoulin F. et al. Drug Metab Dispos. 2002). Accordingly, antibodies that use 5-FU as their switch may be able to bind selectively to the target antigen only at the cancer site. Furthermore, besides metabolic enzymes, molecules formed in a low-oxygen environment or an acidic environment specific to cancers may also be used as the switch. For example, TH-302 (Duan JX, et al. J Med Chem. 2008) is metabolized into Br-IPM under a low-oxygen condition, and therefore, antibodies that use Br-IPM as their switch may be able to bind selectively to the target antigen only at the cancer site. Examples of administration methods of the unnatural compound to a living body include known administration methods such as oral administration, administration through instillation, transdermal administration, transnasal administration, intravenous administration, and transpulmonary administration, but are not limited thereto.

Non-limiting embodiments of small molecule compounds of the present disclosure include, for example, MTA, SAM, SAH, adenosine, adenosine triphosphate (ATP), adenosine diphosphate (ADP), and adenosine monophosphate (AMP).

The following compounds are further exemplified as non-limiting embodiments of the small molecule compounds of the present disclosure:

(1) Primary metabolites of glycolysis or the Krebs cycle such as lactic acid, succinic acid, and citric acid

As non-limiting embodiments of the small molecule compounds or cancer tissue-specific compounds used in the present invention, particularly cancer cell-specific metabolites, preferred examples are primary metabolites such as lactic acid, succinic acid, and citric acid that are produced as a result of the glucose metabolism, which are present in higher concentrations in cancer tissues than in the surrounding, non-cancer tissues. The glycolytic phenotype, characterized as an upregulation of glycolytic (Embden-Meyerhof pathway) enzymes such as pyruvate kinase, hexokinase, and lactate dehydrogenase (LDH), has been conventionally known as the Warburg effect, a feature of solid tumors.

That is, in tumor cells, high expression of the pyruvate kinase isoform M2 which is necessary for anaerobic glycolysis, and not isoform M1, is considered to be working advantageously for the growth of tumor cells *in vivo* (Christofk

et al. (Nature (2008) 452, 230-233). Pyruvic acid produced by pyruvate kinase is subjected to feedback inhibition by lactic acid produced as a result of equilibrium reaction by lactic acid dehydrogenase (LDH) under anaerobic conditions. Since the feedback inhibition causes promotion of respiration in mitochondria (Krebs cycle) and cell growth inhibition, up regulation of LDH, hexokinase, and glucose transporter (GLUT) is said to play an important role in the proliferation of cancer cells (Fantin et al. (Cancer Cell (2006) 9, 425-434)). Glucose is metabolized by the glycolytic system, and the final metabolite lactic acid is transported together with protons to the tumor surrounding, and as a result, the pH of the tissues surrounding the tumor is said to become acidic. Lactic acid, which is the final product of the glycolytic pathway, as well as succinic acid and citric acid produced by promotion of respiration in mitochondria are known to be accumulated in cancer tissues (Teresa et al. (Mol. Cancer (2009) 8, 41-59)). Examples of a non-limiting embodiment of small molecule compounds and cancer tissue-specific compounds, particularly cancer cell-specific metabolites, used in the present invention preferably include such primary metabolites such as lactic acid, succinic acid, and citric acid produced by metabolism by the glycolytic pathway. Furthermore, succinic acid which is present at high concentration in cells is known to leak out to the outside of the cells upon cell death (Nature Immunology, (2008) 9, 1261-1269). Therefore, succinic acid concentration is thought to be increased in cancer tissues in which cell death occurs frequently.

(2) Amino acids such as alanine, glutamic acid, and aspartic acid

Besides the above-mentioned glucose metabolism, the amino acid metabolism is also known to be altered in tumor cells which require continuous supply of essential amino acids and non-essential amino acids that are necessary for the biosynthesis of biopolymers under anaerobic conditions. Glutamine which contains two nitrogens in its side chain acts as a nitrogen transporter, and is an amino acid that is most widely distributed in an organism. Tumor cells, in which the rate of glutamine uptake into cells is increased, is said to be functioning as a glutamine trap. Such increase in the uptake of glutamine and activity of converting into glutamic acid and lactic acid is called "glutaminolysis", and is considered to be a characteristic of transformed (tumor) cells (Mazurek and Eigenbrodt (Anticancer Res. (2003) 23, 1149-1154); and Mazurek et al. (J. Cell. Physiol. (1999) 181, 136-146)). As a result, cancer patients show an increase in glutamic acid concentration while showing a decrease in plasma glutamine level (Droge et al. (Immunobiology (1987) 174, 473-479)). Furthermore, correlation was observed between concentrations of $^{13}C$-labeled succinic acid, $^{13}C$-labeled alanine, $^{13}C$-labeled glutamic acid, and $^{13}C$-labeled citric acid in studies on $^{13}C$-radiolabeled glucose metabolism in lung cancer tissues. Suitable examples of a non-limiting embodiment of small molecule compounds and cancer tissue-specific compounds used in this invention include alanine, glutamic acid, and aspartic acid which accumulate at high concentrations in cancer tissues through such glutaminolysis and the like.

(3) Amino acid metabolite such as kynurenine

Indolamine 2,3-dioxygenase (IDO) is a tryptophan-metabolizing enzyme which is highly expressed in many cancers such as melanoma, colon cancer, and kidney cancer (Uyttenhove et al. (Nat. Med. (2003) 9, 1269-127)); and it is known to have two isoforms (Lob et al. (Cancer Immunol. Immunother. (2009) 58, 153-157)). IDO catalyzes the conversion of tryptophan to kynurenine (represented by the formula indicated below), and is the first enzyme in the nicotinamide nucleotide (NAD) *de novo* pathway. Furthermore, in glioma which does not express IDO, kynurenine is produced from tryptophan by tryptophan 2,3-dioxygenase (TDO) in the liver (Opitz et al. (Nature (2011) 478, 7368, 197-203)). IDO is also expressed in dendritic cells infiltrated into cancer tissues, and dendritic cells also produce kynurenine (J. Immunol. (2008) 181, 5396-5404). IDO is also expressed in myeloid-derived suppressor cells (MDSC) in cancer tissues, and MDSC also produces kynurenine (Yu et al. (J. Immunol. (2013) 190, 3783-3797)).

[Compound 8]

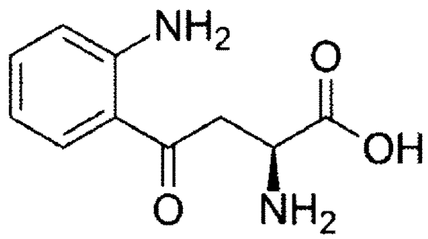

Kynurenine

Kynurenine is known to suppress the same type of T cell response (Frumento et al. (J. Exp. Med. (2002) 196,

459-468); and a mechanism has been suggested, in which tumor cells evade antitumor immune responses through such inhibition, and proliferation of glioma cells is promoted through an autocrine proliferation mechanism in which kynurenine acts as an endogenous ligand for the aryl hydrocarbon receptor expressed on gliomas (Optiz *et al.* (mentioned above)). Kynurenine is converted to anthranilic acid (represented by the formula indicated below) by kynurenidase, and to 3-hydroxykynurenine (represented by the formula indicated below) by kynurenine 3-hydroxylase. Anthranilic acid and 3-hydroxykynurenine are both converted to 3-hydroxyanthranilic acid, the precursor of NAD.

[Compound 9]

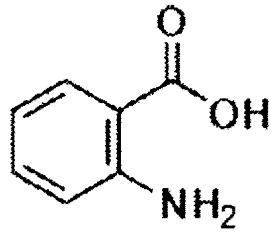

Anthranilic acid

[Compound 10]

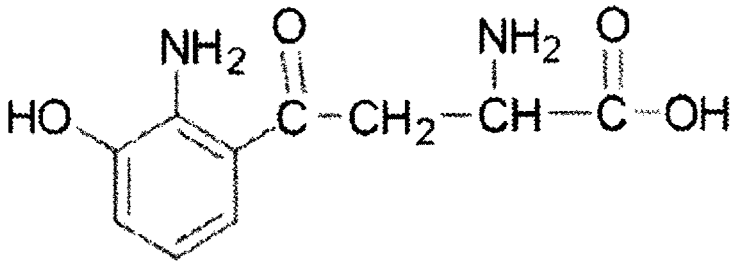

3-hydroxykynurenine

Kynurenine is converted to kynurenic acid (represented by the formula indicated below) by kynurenine aminotransferase. Examples of a non-limiting embodiment of small molecule compounds and cancer tissue-specific compounds, particularly cancer cell-specific metabolites, used in the present invention preferably include such amino acid metabolites such as kynurenine and its metabolites such as anthranilic acid, 3-hydroxykynurenine, and kynurenic acid.

[Compound 11]

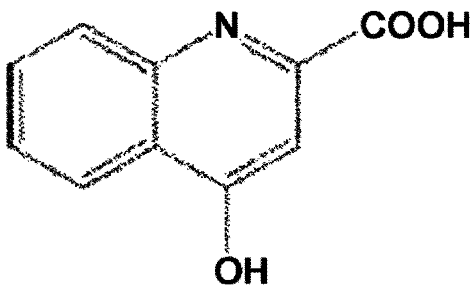

Kynurenic acid

(4) Arachidonic acid metabolites such as prostaglandin E2

Prostaglandin E2 (PGE2) (represented by the formula indicated below) is an arachidonic acid metabolite called a prostanoid, which includes thromboxane and prostaglandin synthesized by cyclooxygenase (COX)-1/2 (Warner and

Mitchell (FASEB J. (2004) 18, 790-804)). PGE2 promotes the proliferation of colon cancer cells and suppresses their apoptosis (Sheng et al. (Cancer Res. (1998) 58, 362-366)). Cyclooxygenase expression is known to be altered in many cancer cells. More specifically, while COX-1 is expressed constitutively in almost all tissues, COX-2 has been found to be mainly induced by certain types of inflammatory cytokines and cancer genes in tumors (Warner and Mitchell (mentioned above)). In addition, COX-2 overexpression has been reported to be related to bad prognosis for breast cancer (Denkert et al. (Clin. Breast Cancer (2004) 4, 428-433)), and rapid disease progression for ovarian cancer (Denker et al. (Mod. Pathol. (2006) 19, 1261-1269)). Inhibitory T cells that have infiltrated into cancer tissues also produce prostaglandin E2 (Curr. Med. Chem. (2011) 18, 5217-5223). Small molecule compounds such as the arachidonic acid metabolites prostaglandin and leukotriene are known to act as a stimulating factor that regulates autocrine and/or paracrine growth of cancer (Nat. Rev. Cancer (2012) 12 (11) 782-792). Examples of a non-limiting embodiment of small molecule compounds and cancer tissue-specific compounds used in the present invention, particularly cancer cell-specific metabolites and immune cell-specific metabolites that have infiltrated into cancer tissues, preferably include such arachidonic acid metabolites such as prostaglandin E2. Besides prostaglandin E2, production of thromboxane A2 (TXA2) is enhanced in cancer tissues such as colorectal cancer tissues (J. Lab. Clin. Med. (1993) 122, 518-523), and thromboxane A2 can be suitably presented as a non-limiting embodiment of an arachidonic acid metabolite of the present invention.

[Compound 12]

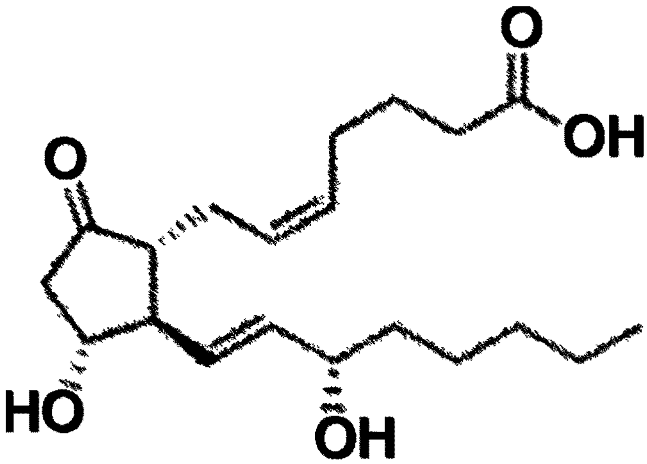

Prostaglandin E2 (PGE2)

It is also known that PGE2 concentration is high in rheumatoid arthritis and osteoarthritis (Eur. J. Clin. Pharmacol. (1994) 46, 3-7., Clin. Exp. Rheumatol. (1999) 17, 151-160, Am. J. Vet. Res. (2004) 65, 1269-1275). As non-limiting embodiments of small molecule compounds and inflammatory tissue-specific compounds used in the present invention, particularly inflammatory cell-specific metabolites and immune cell-specific metabolites that infiltrate inflammatory tissues, prostaglandin E2 and such metabolites of arachidonic acid can be preferably mentioned.

(5) Nucleosides carrying a purine ring structure such as adenosine, adenosine triphosphate (ATP), adenosine diphosphate (ADP), and adenosine monophosphate (AMP)

When cancer cells undergo cell death, a large amount of ATP in the cell is known to leak out to the outside of the cells. Therefore, the ATP concentration is remarkably higher in cancer tissues than in normal tissues (PLoS One. (2008) 3, e2599). Multiple types of cells release adenine nucleotides in the form of ATP, ADP, and AMP. Metabolism takes place through an extracellular enzyme on the cell surface such as extracellular 5'-nucleotidase (ecto-5'nucleotidase) (CD73) (Resta and Thompson (Immunol. Rev. (1998) 161, 95-109) and Sadej et al. (Melanoma Res. (2006) 16, 213-222)). Adenosine is a purine nucleoside that exists constitutively at low concentration in the extracellular environment, but in hypoxic tissues found in solid cancers, a remarkable increase in the extracellular adenosine concentration has been reported (Blay and Hoskin (Cancer Res. (1997) 57, 2602-2605). CD73 is expressed on the surface of immune cells and tumors (Kobie et al. (J. Immunol. (2006) 177, 6780-6786)), and its activity has been found to be increased in breast cancer (Canbolat et al. (Breast Cancer Res. Treat. (1996) 37, 189-193)), stomach cancer (Durak et al. (Cancer Lett. (1994) 84, 199-202)), pancreatic cancer (Flocke and Mannherz (Biochim. Biophys. Acta (1991) 1076, 273-281), and glioblastoma (Bardot et al. (Br. J. Cancer (1994) 70, 212-218)). It has been proposed that the accumulation of adenosine in cancer tissues may be caused by an increase in the intracellular adenosine production through dephosphorylation of

AMP by 5'-nucleotidase in the cytoplasm (Headrick and Willis (Biochem. J. (1989) 261, 541-550)). Furthermore, inhibitory T cells and such that have infiltrated into cancer tissues also express ATPase and produce adenosine (Proc. Natl. Acad. Sci. (2006) 103 (35), 13132-13137; Curr. Med. Chem. (2011) 18, 5217-5223). The produced adenosine is considered to be rendering the cancer tissue an immunosuppressive environment through adenosine receptors such as the A2A receptor (Curr. Med. Chem. (2011),18, 5217-23). Examples of a non-limiting embodiment of the small molecule compound and cancer tissue-specific compound used in the present invention preferably include ATP, ADP, AMP, and adenosine which accumulate at high concentration in cancer tissues through such metabolism of purine nucleotides such as ATP. Furthermore, since adenosine is degraded to inosine by adenosine deaminase, inosine accumulates at high concentration.

It is also known that ATP concentration is high in alveoli inflamed due to bronchial asthma (Nat. Med. (2007) 13, 913-919). It is also known that ATP concentration is high in alveoli inflamed due to COPD (Am. J. Respir. Crit. Care Med. (2010) 181, 928-934). In addition, high adenosine levels have been observed in the synovial fluid of patients with rheumatoid arthritis (Journal of Pharmaceutical and Biomedical Analysis (2004) 36 877-882). Furthermore, it is known that ATP concentration is high in tissues wherein rejection reactions are taking place due to GVHD (Nat. Med. (2010) 16, 1434-1438). It is also known that adenosine levels are increased in fibrotic tissues in the lungs, liver, and kidneys (FASEB J. (2008) 22, 2263-2272, J. Immunol. (2006) 176, 4449-4458., J. Am. Soc. Nephrol. (2011) 22 (5), 890-901, PLoS ONE J. (2010) 5 (2), e9242). It has also been observed that ATP levels are elevated in fibrotic tissues of patients with pulmonary fibrosis (Am. J. Respir. Crit. Care Med. (2010) 182, 774-783). Preferable examples of non-limiting embodiments of small molecule compounds and inflammatory tissue-specific compounds used in the present invention are ATP, ADP, AMP, adenosine, etc., which accumulate in high concentrations in inflammatory tissues by metabolism of purine nucleotides such as ATP. Furthermore, as adenosine is decomposed into inosine by adenosine deaminase, inosine accumulates at a high concentration.

(6) Uric acid

Uric acid is a product of the metabolic pathway of purine nucleosides *in vivo,* and is released to the outside of cells such as the interstitial space and blood. In recent years, it has been found to be released from dead cells that are present at sites of lesions such as cancer tissues (Nat. Med. (2007) 13, 851-856). Examples of a non-limiting embodiment of small molecule compounds and cancer tissue-specific compounds used in the present invention preferably include such uric acid which accumulates at high concentration in cancer tissues due to metabolism of purine nucleotides such as ATP.

In recent years, uric acid released from cells undergoing necrosis has been found to promote inflammatory response (J. Clin. Invest. (2010) 120 (6), 1939-1949). Examples of a non-limiting embodiment of small molecule compounds and inflammatory tissue-specific compounds to be used in the present invention suitably include such uric acid which accumulates at high concentration in inflammatory tissues due to metabolism of purine nucleotides such as

ATP.

(7) 1-Methyl nicotinamide

The enzyme nicotinamide *N*-methyl transferase is known to be highly expressed in several human cancer tissues. When this enzyme produces the stable metabolite 1-methylnicotinamide from nicotinamide, the methyl group of *S*-adenosylmethionine (SAM) which serves as a methyl donor is consumed; therefore, the high expression of nicotinamide N-methyltransferase has been suggested to contribute to tumorigenesis through a mechanism that impairs the DNA methylation ability accompanying a decrease in the SAM concentration in cancer cells (Ulanovskaya et al. (Nat. Chem. Biol. (2013) 9 (5) 300-306)). The stable metabolite of this enzyme, 1-methylnicotinamide is known to be secreted to the outside of cancer cells (Yamada et al. (J. Nutr. Sci. Vitaminol. (2010) 56, 83-86)), and preferable examples of a non-limiting embodiment of small molecule compounds and cancer tissue-specific compounds used in the present invention include 1-methylnicotinamide and such which accumulate at high concentration in cancer tissues through nicotinamide metabolism.

The small molecule compounds of the present disclosure can interact with antigen-binding molecules. Amino acid residues in an antigen-binding molecule capable of interacting with a small molecule compound may be present in the antigen-binding domain in the antigen-binding molecule, or may be present at a site other than the antigen-binding domain. An example of the site in an antigen-binding molecule that interacts with a small molecule compound is an antigen-binding domain, but is not limited there to.

Antigen-binding domains that specifically bind to an antigen

In the present specification, the expression "an antigen-binding domain that specifically binds to an antigen" is used when the antigen-binding domain is specific to a specific epitope among a plurality of epitopes contained in an antigen. When the epitope to which the antigen-binding domain binds is contained in a plurality of different antigens, an antigen-binding molecule having the antigen-binding domain can bind to various antigens containing the epitope. Here, "not substantially binding" is determined according to the method mentioned in the section of binding activity, and means that the binding activity of a specific binding molecule to a molecule other than the partner is 80% or less, usually 50% or less, preferably 30% or less, and particularly preferably 15% or less of the binding activity to the above-mentioned partner molecule. When the antigen-binding domain is an antigen-binding domain whose antigen-binding activity changes depending on MTA or on a small molecule compound other than MTA, the binding of the antigen-binding domain to the antigen is measured under conditions in which the antigen-binding activity of the antigen-binding domain to the antigen is high (for example, under a specific concentration of MTA, or in the absence of MTA, under a specific concentration of a small molecule compound other than MTA, or in the absence of a small molecule compound other than MTA).

Antigen-binding activity and methods for confirming antigen-binding activity

The term "binding activity" refers to the total strength of a non-covalent interaction between one or more binding sites of a molecule (e.g., an antibody) and the molecule's binding partner (e.g., an antigen). Here, "binding activity" is not strictly limited to a 1:1 interaction between members of a binding pair (e.g., antibody and antigen). For example, when the members of a binding pair reflect a monovalent 1:1 interaction, the binding activity refers to the inherent binding affinity ("affinity"). If the members of the binding pair are capable of both monovalent and multivalent binding, the binding activity is the sum of these binding forces. The binding activity of molecule X to its partner Y can generally be expressed by the dissociation constant (KD) or the "analyte binding amount per unit ligand amount". Binding activity can be measured by conventional methods known in the art, including those described herein. Conditions other than the concentration of the target tissue-specific compound can be appropriately determined by those skilled in the art.

Specific examples and exemplary embodiments for measuring binding activity will be described below.

Binding activity of antigen-binding molecules

In certain embodiments, the antigen-binding molecule provided herein is an antibody and the binding activity of the antibody is a dissociation constant (KD) of ≦1 μM, ≦100 nM, ≦10 nM, ≦1 nM, ≦0.1 nM, ≦0.01 nM, or ≦ 0.001 nM (e.g., $10^{-8}$ M or less, for example, $10^{-8}$ M to $10^{-13}$ M, e.g., $10^{-9}$ M to $10^{-13}$ M).

In one embodiment, the binding activity of the antibody is measured by, for example, a ligand capture method using BIACORE (registered trademark) T200 or BIACORE (registered trademark) 4000 (GE Healthcare, Uppsala, Sweden), which uses surface plasmon resonance analysis as the principle of measurement. BIACORE (registered trademark) Control Software is used to operate the equipment. In one embodiment, an amine coupling kit (GE Healthcare, Uppsala, Sweden) is used according to the supplier's instructions, and a molecule for ligand capture, e.g., an anti-tag antibody, anti-IgG antibody, protein A, or such, is immobilized onto a carboxymethyl dextran-coated sensor chip (GE Healthcare, Uppsala, Sweden). The ligand-capturing molecule is diluted with a 10 mM sodium acetate solution at the appropriate pH and injected at the appropriate flow rate and injection time. To measure binding activity, a buffer solution containing 0.05% polysorbate 20 (also called Tween (registered trademark)-20) is used as the buffer solution for measurement, the flow rate is 10-30 μL/min, and the measurement temperature is preferably 25°C or 37°C. When the measurement is done by making the ligand-capturing molecule capture an antibody as ligand, the antibody is injected to allow capturing of a target amount, and then the antigen and/or a serial dilution of an Fc receptor (analyte) prepared using the measurement buffer solution is/are injected. When conducting the measurement after making the ligand-capturing molecule capture the antigen and/or an Fc receptor as ligand, the antigen and/or Fc receptor is injected to allow capturing of a target amount, and a serial dilution of the antibody (analyte) prepared using the measurement buffer is injected.

In one embodiment, the measurement results are analyzed using the BIACORE (registered trademark) Evaluation Software. Kinetic parameter calculations are performed by simultaneously fitting sensorgrams for binding and dissociation using a 1:1 binding model, and the binding rate (kon or ka), the dissociation rate (koff or kd), and the equilibrium dissociation constant (KD) can be calculated. The equilibrium dissociation constant (KD) may be calculated using the steady state model when the binding activity is weak, especially when the dissociation is fast and the kinetic parameters are difficult to calculate. As another parameter of the binding activity, the "analyte binding amount per unit ligand amount" can also be calculated by dividing the binding amount (RU) of a specific concentration of analyte by the ligand capture amount (RU).

In one embodiment, the antibody's binding activity can be measured by, for example, the Octet RED 96e system or the Octet RED 384 system (Pall Forte Bio), which uses the Bio-Layer Interferometry (BLI) as the measurement

principle. Qualitative binding characteristic analysis and kinetic analysis of antigen-antibody reaction can be performed according to the supplier's instructions. As a non-limiting embodiment of a specific measurement method, after immobilizing the antibody on a Protein A (ProA) biosensor (Pall Forte Bio), the antigen is allowed to interact as an analyte to measure the change in the amount of binding between antibody and antigen. For example, when measuring the amount of binding between the antigen and an antibody that binds to the antigen in the presence of MTA, it is possible to measure the binding reaction between the antibody and analyte in a buffer containing 3000 nM of analyte diluted with 20 mM ACES added with MTA at a final concentration of 0, 10, 100 μM, 150 mM NaCl, 0.05% (w/v) Tween20, pH 7.4, as binding phase. It is possible to measure the dissociation reaction between the antibody and analyte by using the same buffer as the binding phase but without the analyte, as dissociation phase. Furthermore, by using as the dissociation phase a buffer that contains the same concentration of analyte as the binding phase but does not contain MTA, it is possible to observe over time that the binding between the antibody and the antigen in the presence of MTA is reversibly dissociated in the absence of MTA.

For the value of antigen-binding activity, if the antigen is a soluble molecule, dissociation constant (kd) can be used; and if the antigen is a membrane-type molecule, apparent dissociation constant (apparent kd) can be used. The dissociation constant (kd) and apparent dissociation constant (apparent kd) can be determined by methods known to those skilled in the art, for example, using Biacore (GE Healthcare), a flow cytometer, or such.

When measuring the antigen-binding activity of a test antigen-binding molecule comprising an antigen-binding domain whose antigen-binding activity changes in a small molecule compound-dependent manner, the interaction between the test antigen-binding molecule and the antigen can be carried out under a specific concentration of the small molecule compound, or in the absence thereof.

When an antigen-binding molecule that does not exhibit an antigen-binding activity in the absence of a specific small molecule compound exhibits binding activity to the antigen in the presence of the small molecule compound, the binding activity can be evaluated in the presence of the small molecule compound. Similarly, when the antigen-binding activity is not exhibited in the presence of a specific small molecule compound, but binding activity to the antigen is exhibited in the absence of the small molecule compound, the binding activity can be evaluated in the absence of the small molecule compound. When the antigen-binding activity under the condition of the specific small molecule compound being present at a low concentration is higher than the antigen-binding activity under the condition of the small molecule compound being present at a high concentration, the binding activity can be evaluated under the condition where the small molecule compound is present at a high concentration. Similarly, when the antigen-binding activity under the condition of the specific small molecule compound being present at a high concentration is lower than the antigen-binding activity under the condition of the small molecule compound being present at a low concentration, the binding activity can be evaluated under the condition where the small molecule compound is present at a low concentration. The conditions that can affect the binding activity of the antigen and the antigen-binding molecule are not limited to the presence/absence of the small molecule compound and the concentration of the small molecule compound, and examples of such conditions include ion concentration, ion composition, and temperature, without limitation. Furthermore, it is of course possible to evaluate the binding activity under the condition where the above-mentioned different plurality of factors are combined.

## Epitopes

"Epitope" means an antigenic determinant in an antigen, and refers to an antigen site to which the antigen-binding domain of an antigen-binding molecule disclosed herein binds. The antigen site to which the antigen-binding molecule disclosed herein binds can be defined by evaluating the presence or absence of binding of the antigen-binding molecule.

The epitope can be defined according to its structure. Alternatively, the epitope may be defined according to the antigen-binding activity of an antigen-binding molecule that recognizes the epitope. When the antigen is a peptide or polypeptide, the epitope can be specified by the amino acid residues forming the epitope. Alternatively, when the epitope is a sugar chain, the epitope can be specified by its specific sugar chain structure.

A linear epitope is an epitope that contains an epitope whose primary amino acid sequence has been recognized. Such a linear epitope typically contains at least three and most commonly at least five, for example, about 8 to about 10 or 6 to 20 amino acids in a specific sequence.

In contrast to the linear epitope, a "conformational epitope" is an epitope in which the primary amino acid sequence containing the epitope is not the only determinant of the recognized epitope (for example, the primary amino acid sequence of a conformational epitope is not necessarily recognized by an epitope-defining antibody). Conformational epitopes may contain a greater number of amino acids compared to linear epitopes. A conformational epitope-recognizing antibody recognizes the three-dimensional structure of a peptide or protein. For example, when a protein molecule folds and forms a three-dimensional structure, amino acids and/or polypeptide main chains that form a conformational epitope become aligned, and the epitope is made recognizable by the antibody. Methods for determining epitope conformations

include, for example, X ray crystallography, two-dimensional nuclear magnetic resonance, site-specific spin labeling, and electron paramagnetic resonance, but are not limited thereto. See, for example, Epitope Mapping Protocols in Methods in Molecular Biology (1996), Vol. 66, Morris (ed.).

The structure of the antigen-binding domain which binds to an epitope is called a paratope. An epitope and a paratope bind with stability through the action of hydrogen bonds, electrostatic force, van der Waals force, hydrophobic bonds, and such between the epitope and the paratope. This strength of binding between the epitope and paratope is called affinity. The total sum of binding strength when a plurality of antigens and a plurality of antigen-binding molecules bind is referred to as avidity. When an antibody comprising a plurality of antigen-binding domains (i.e., multivalent antibody) or such binds to a plurality of epitopes, the affinity acts synergistically, and therefore avidity becomes higher than affinity.

Examples of a method for assessing the epitope binding by a test antigen-binding molecule containing an IL-6R antigen-binding domain are described below. According to the examples below, methods for assessing the epitope binding by a test antigen-binding molecule containing an antigen-binding domain for an antigen other than IL-6R, can also be appropriately conducted.

For example, whether a test antigen-binding molecule containing an IL-6R antigen-binding domain recognizes a linear epitope in the IL-6R molecule can be confirmed for example as mentioned below. A linear peptide comprising an amino acid sequence forming the extracellular domain of IL-6R is synthesized for the above purpose. The peptide can be synthesized chemically, or obtained by genetic engineering techniques using a region encoding the amino acid sequence corresponding to the extracellular domain in an IL-6R cDNA. Then, a test antigen-binding molecule containing an IL-6R antigen-binding domain is assessed for its binding activity towards a linear peptide comprising the amino acid sequence forming the extracellular domain. For example, an immobilized linear peptide can be used as an antigen by ELISA to evaluate the binding activity of the antigen-binding molecule towards the peptide. Alternatively, the binding activity towards a linear peptide can be assessed based on the level that the linear peptide inhibits the binding of the antigen-binding molecule to IL-6R-expressing cells. These tests can demonstrate the binding activity of the antigen-binding molecule towards the linear peptide.

Whether a test antigen-binding molecule containing an IL-6R antigen-binding domain recognizes a conformational epitope can be assessed as follows. IL-6R-expressing cells are prepared for the above purpose. A test antigen-binding molecule containing an IL-6R antigen-binding domain can be determined to recognize a conformational epitope when it strongly binds to IL-6R-expressing cells upon contact, but does not substantially bind to an immobilized linear peptide comprising an amino acid sequence forming the extracellular domain of IL-6R. Herein, "not substantially bind" means that the binding activity is 80% or less, generally 50% or less, preferably 30% or less, and particularly preferably 15% or less compared to the binding activity towards cells expressing human IL-6R.

Methods for assaying the binding activity of a test antigen-binding molecule containing an IL-6R antigen-binding domain towards IL-6R-expressing cells include, for example, the methods described in Antibodies: A Laboratory Manual (Ed Harlow, David Lane, Cold Spring Harbor Laboratory (1988) 359-420). Specifically, the assessment can be performed based on the principle of ELISA or fluorescence activated cell sorting (FACS) using IL-6R-expressing cells as antigen.

In the ELISA format, the binding activity of a test antigen-binding molecule containing an IL-6R antigen-binding domain towards IL-6R-expressing cells can be assessed quantitatively by comparing the levels of signal generated by enzymatic reaction. Specifically, a test polypeptide complex is added to an ELISA plate onto which IL-6R-expressing cells are immobilized. Then, the test antigen-binding molecule bound to the cells is detected using an enzyme-labeled antibody that recognizes the test antigen-binding molecule. Alternatively, when FACS is used, a dilution series of a test antigen-binding molecule is prepared, and the antibody binding titer for IL-6R-expressing cells can be determined to compare the binding activity of the test antigen-binding molecule towards IL-6R-expressing cells.

The binding of a test antigen-binding molecule towards an antigen expressed on the surface of cells suspended in buffer or the like can be detected using a flow cytometer. Known flow cytometers include, for example, the following devices:

FACSCanto™ II
FACSAria™
FACSArray™
FACSVantage™ SE
FACSCalibur™ (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter).

Preferable methods for assaying the binding activity of a test antigen-binding molecule containing an IL-6R

antigen-binding domain towards an antigen include, for example, the following method. First, IL-6R-expressing cells are reacted with a test antigen-binding molecule, and then this is stained with an FITC-labeled secondary antibody that recognizes the antigen-binding molecule. The test antigen-binding molecule is appropriately diluted with a suitable buffer to prepare the molecule at a desired concentration. For example, the molecule can be used at a concentration within the range of 10 μg/ml to 10 ng/ml. Then, the fluorescence intensity and cell count are determined using FACSCalibur (BD). The fluorescence intensity obtained by analysis using the CELL QUEST Software (BD), *i.e.,* the Geometric Mean value, reflects the quantity of antibody bound to cells. That is, the binding activity of a test antigen-binding molecule, which is represented by the quantity of the test antigen-binding molecule bound, can be determined by measuring the Geometric Mean value.

Antibodies that bind the same epitope

Whether a test antigen-binding molecule containing an IL-6R antigen-binding domain shares a common epitope with another antigen-binding molecule can be assessed based on the competition between the two molecules for the same epitope. The competition between antigen-binding molecules can be detected by cross-blocking assay or the like. For example, the competitive ELISA assay is a preferred cross-blocking assay.

Specifically, in cross-blocking assay, the IL-6R protein immobilized to the wells of a microtiter plate is pre-incubated in the presence or absence of a candidate competitor antigen-binding molecule, and then a test antigen-binding molecule is added thereto. The quantity of test antigen-binding molecule bound to the IL-6R protein in the wells is indirectly correlated with the binding ability of a candidate competitor antigen-binding molecule that competes for the binding to the same epitope. That is, the greater the affinity of the competitor antigen-binding molecule for the same epitope, the lower the binding activity of the test antigen-binding molecule towards the IL-6R protein-coated wells.

The quantity of the test antigen-binding molecule bound to the wells *via* the IL-6R protein can be readily determined by labeling the antigen-binding molecule in advance. For example, a biotin-labeled antigen-binding molecule is measured using an avidin/peroxidase conjugate and appropriate substrate. In particular, cross-blocking assay that uses enzyme labels such as peroxidase is called "competitive ELISA assay". The antigen-binding molecule can also be labeled with other labeling substances that enable detection or measurement. Specifically, radiolabels, fluorescent labels, and such are known.

When the candidate competitor antigen-binding molecule can block the binding by a test antigen-binding molecule containing an IL-6R antigen-binding domain by at least 20%, preferably at least 20 to 50%, and more preferably at least 50% compared to the binding activity in a control experiment conducted in the absence of the competitor antigen-binding molecule, the test antigen-binding molecule is determined to substantially bind to the same epitope bound by the competitor antigen-binding molecule, or compete for the binding to the same epitope.

When the structure of an epitope bound by a test antigen-binding molecule containing an IL-6R antigen-binding domain has already been identified, whether the test and control antigen-binding molecules share a common epitope can be assessed by comparing the binding activities of the two antigen-binding molecules towards a peptide prepared by introducing amino acid mutations into the peptide forming the epitope.

To measure the above binding activities, for example, the binding activities of test and control antigen-binding molecules towards a linear peptide into which a mutation is introduced are compared in the above ELISA format. Besides the ELISA methods, the binding activity towards the mutant peptide bound to a column can be determined by flowing test and control antigen-binding molecules in the column, and then quantifying the antigen-binding molecule eluted in the elution solution. Methods for adsorbing a mutant peptide to a column, for example, in the form of a GST fusion peptide, are known.

Alternatively, when the identified epitope is a conformational epitope, whether test and control antigen-binding molecules share a common epitope can be assessed by the following method. First, IL-6R-expressing cells and cells expressing IL-6R with a mutation introduced into the epitope are prepared. The test and control antigen-binding molecules are added to a cell suspension prepared by suspending these cells in an appropriate buffer such as PBS. Then, the cell suspensions are appropriately washed with a buffer, and an FITC-labeled antibody that recognizes the test and control antigen-binding molecules is added thereto. The fluorescence intensity and number of cells stained with the labeled antibody are determined using FACSCalibur (BD). The test and control antigen-binding molecules are appropriately diluted using a suitable buffer, and used at desired concentrations. For example, they may be used at a concentration within the range of 10 μg/ml to 10 ng/ml. The fluorescence intensity determined by analysis using the CELL QUEST Software (BD), *i.e.,* the Geometric Mean value, reflects the quantity of labeled antibody bound to cells. That is, the binding activities of the test and control antigen-binding molecules, which are represented by the quantity of labeled antibody bound, can be determined by measuring the Geometric Mean value.

In the above method, whether an antigen-binding molecule does "not substantially bind to cells expressing mutant IL-6R" can be assessed, for example, by the following method. First, the test and control antigen-binding molecules bound to cells expressing mutant IL-6R are stained with a labeled antibody. Then, the fluorescence intensity of the cells

is determined. When FACSCalibur is used for fluorescence detection by flow cytometry, the determined fluorescence intensity can be analyzed using the CELL QUEST Software. From the Geometric Mean values in the presence and absence of the polypeptide complex, the comparison value (ΔGeo-Mean) can be calculated according to Formula 1 below to determine the ratio of increase in fluorescence intensity as a result of the binding by the antigen-binding molecule.

Formula 1:

ΔGeo-Mean = Geo-Mean (in the presence of the polypeptide complex)/Geo-Mean (in the absence of the polypeptide complex)

The Geometric Mean comparison value (ΔGeo-Mean value for the mutant IL-6R molecule) determined by the above analysis, which reflects the quantity of a test antigen-binding molecule bound to cells expressing mutant IL-6R, is compared to the ΔGeo-Mean comparison value that reflects the quantity of the test antigen-binding molecule bound to IL-6R-expressing cells. In this case, the concentrations of the test antigen-binding molecule used to determine the ΔGeo-Mean comparison values for IL-6R-expressing cells and cells expressing mutant IL-6R are particularly preferably adjusted to be equal or substantially equal. An antigen-binding molecule that has been confirmed to recognize an epitope in IL-6R is used as a control antigen-binding molecule.

If the ΔGeo-Mean comparison value of a test antigen-binding molecule for cells expressing mutant IL-6R is smaller than the ΔGeo-Mean comparison value of the test antigen-binding molecule for IL-6R-expressing cells by at least 80%, preferably 50%, more preferably 30%, and particularly preferably 15%, then the test antigen-binding molecule "does not substantially bind to cells expressing mutant IL-6R". The formula for determining the Geo-Mean (Geometric Mean) value is described in the CELL QUEST Software User's Guide (BD biosciences). When the comparison shows that the comparison values are substantially equivalent, the epitope for the test and control antigen-binding molecules can be determined to be the same.

When assessing competition/binding to the same epitope between test antigen-binding molecules comprising an antigen-binding domain whose antigen-binding activity changes in a small molecule compound-dependent manner, it is possible to carry out the interaction between test antigen-binding molecules and antigens under a specific concentration of the small molecule compound, or in the absence thereof, and it is preferable to keep the concentration condition of the small molecule compound the same between the test antigen-binding molecules.

Antigen-binding domains whose antigen-binding activity changes in a small molecule compound-dependent manner

In the present specification, "an antigen-binding domain whose antigen-binding activity changes in a small molecule compound-dependent manner" means an antigen-binding domain whose binding activity to an antigen, which is a molecule different from the small molecule compound, changes in the presence of different concentrations of the small molecule compound.

Antigen-binding domains whose antigen-binding activity changes in an MTA-dependent manner

The antigen-binding domains of the present disclosure whose antigen-binding activity changes in an MTA-dependent manner are antigen-binding domains having different binding activities towards an antigen that is a molecule different from MTA, under the condition of different MTA concentrations. The antigens to which these antigen-binding domains bind may be membrane-type molecules or soluble-type molecules. The antigens to which these antigen-binding domains bind are antigens expressed in a diseased tissue, more preferably, are antigens expressed in a cancer tissue, and further preferably, antigens expressed in a cancer tissue in which MTA is accumulated. Antigens expressed in a cancer tissue may be antigens expressed in cancer cells, or antigens expressed in cancer stromal cells in a cancer tissue or in immune tissues.

Non-limiting examples of the antigen-binding activity changing in an MTA-dependent manner include an antigen-binding domain whose antigen-binding activity in the presence of MTA is stronger than the antigen-binding activity in the absence of MTA, and an antigen-binding domain whose antigen-binding activity in the presence of MTA is weaker than the antigen-binding activity of the antigen-binding domain in the absence of MTA.

As long as the antigen-binding activity in the absence of MTA of an antigen-binding domain of the present disclosure, whose antigen-binding activity in the presence of MTA is stronger than the antigen-binding activity in the absence of MTA, is weaker than the antigen-binding activity in the presence of MTA, the ratio of the antigen-binding activity in the absence of MTA and the antigen-binding activity in the presence of MTA is not particularly limited. Preferably, the value of the ratio of KD (dissociation constant) for the antigen in the absence of MTA and the KD in the presence of MTA (KD (without MTA)/KD (with MTA)) is 2 or more, more preferably, the value of KD (without MTA)/KD (with MTA) is

10 or more, and even more preferably, the value of KD (without MTA)/KD (with MTA) is 40 or more. The upper limit of the value of KD (without MTA)/KD (with MTA) is not particularly limited, and may be any value such as 400, 1000, 10000, and the like, as long as it can be produced with the skill of a person skilled in the art. If no antigen-binding activity is observed in the absence of MTA, this upper limit is an infinite number.

An antigen-binding domain whose antigen-binding activity in the presence of MTA is stronger than the antigen-binding activity in the absence of MTA includes an antigen-binding domain that does not substantially bind to an antigen in the absence of MTA.

As long as the antigen-binding activity in the absence of MTA of an antigen-binding domain of the present disclosure, whose antigen-binding activity in the presence of MTA is weaker than the antigen-binding activity in the absence of MTA, is stronger than the antigen-binding activity in the presence of MTA, the ratio of the antigen-binding activity in the absence of MTA and the antigen-binding activity in the presence of MTA is not particularly limited. Preferably, the value of the ratio of KD (dissociation constant) for the antigen in the presence of MTA and the KD in the absence of MTA (KD (with MTA)/KD (without MTA)) is 2 or more, more preferably 10 or more, and even more preferably 40 or more. The upper limit of the value of KD (with MTA)/KD (without MTA) is not particularly limited, and may be any value such as 400, 1000, 10000, and the like, as long as it can be produced with the skill of a person skilled in the art. If no antigen-binding activity is observed in the presence of MTA, this upper limit is an infinite number.

An antigen-binding domain whose antigen-binding activity in the presence of MTA is weaker than the antigen-binding activity of the antigen-binding domain in the absence of MTA includes an antigen-binding domain that does not substantially bind to the antigen in the presence of MTA.

As another indicator that shows the ratio between the antigen-binding activity of an antigen-binding domain (or an antigen-binding molecule containing the domain) of the present disclosure in the absence of MTA and the antigen-binding activity in the presence of MTA, for example, dissociation rate constant kd can be suitably used. When the dissociation rate constant (kd) is used instead of the dissociation constant (KD) as an indicator that shows the binding activity ratio, the value of kd (in the absence of MTA) / kd (in the presence of MTA), which is a ratio between kd (dissociation rate constant) for an antigen in the absence of MTA and kd in the presence of MTA, is preferably 2 or greater, more preferably 5 or greater, even more preferably 10 or greater, and still more preferably 30 or greater. The upper limit of the value of kd (in the absence of MTA) / kd (in the presence of MTA) is not particularly limited, and may be any value, for example, 50, 100, or 200, as long as it can be provided by the common technical knowledge of those skilled in the art. When antigen-binding activity is not observed in the absence of MTA, there is no dissociation and the value of the upper limit becomes infinity.

As a condition where MTA is present, an appropriate MTA concentration can be set, and the condition where 100 μM MTA is present can be regarded as a non-limiting example of a condition where MTA is present. Further, as a non-limiting embodiment of MTA concentration described as "in the presence of MTA" in the present disclosure, the laterdescribed concentration exemplified as the threshold value for distinguishing between a low concentration and a high concentration of MTA can be applied.

Non-limiting examples of antigen-binding domains whose antigen-binding activity changes in an MTA-dependent manner include antigen-binding domains whose antigen-binding activity in the presence of a high concentration of MTA is stronger than the antigen-binding activity in the presence of a low concentration of MTA, and antigen-binding domains whose antigen-binding activity in the presence of a high concentration of MTA is weaker than the antigen-binding activity in the presence of a low concentration of MTA.

As long as the antigen-binding activity of an antigen-binding domain of the present disclosure, whose antigen binding activity in the presence of a high concentration of MTA is stronger than the antigen binding activity in the presence of a low concentration of MTA, in the presence of a low concentration of MTA is weaker than the antigen-binding activity in the presence of a high concentration of MTA, the ratio between the antigen-binding activity in the presence of a low concentration of MTA and the antigen-binding activity in the presence of a high concentration of MTA is not particularly limited. However, the value of KD (in the presence of a low concentration of MTA) / KD (in the presence of a high concentration of MTA), which is a ratio of dissociation constant (KD) against an antigen in the presence of a low concentration of MTA to KD in the presence of a high concentration of MTA, is preferably 2 or greater, more preferably the value of KD (in the presence of a low concentration of MTA) / KD (in the presence of a high concentration of MTA) is 10 or greater, and still more preferably the value of KD (in the presence of a low concentration of MTA) / KD (in the presence of a high concentration of MTA) is 40 or greater. The upper limit of the value of KD (in the presence of a low concentration of MTA) / KD (in the presence of a high concentration of MTA) is not particularly limited, and may be any value, for example, 400, 1,000, or 10,000, as long as it can be provided by the technologies of those skilled in the art. When antigen-binding activity is not observed in the presence of a low concentration of MTA, the value of the upper limit is infinity.

The antigen-binding domains whose antigen-binding activity in the presence of a high concentration of MTA is stronger than the antigen-binding activity in the presence of a low concentration of MTA include antigen-binding domains that do not substantially bind to an antigen in the presence of a low concentration of MTA.

As long as the antigen-binding activity in the presence of a low concentration of MTA of an antigen-binding domain of the present disclosure, whose antigen-binding activity in the presence of a high concentration of MTA is weaker than the antigen-binding activity in the presence of a low concentration of MTA, is stronger than the antigen-binding activity in the presence of a high concentration of MTA, the ratio of the antigen-binding activity in the presence of a low concentration of MTA and the antigen-binding activity in the presence of a high concentration of MTA is not particularly limited. Preferably, the value of the ratio of KD (dissociation constant) for the antigen in the presence of a high concentration of MTA and the KD in the presence of a low concentration of MTA (KD (with a high concentration of MTA)/KD (with a low concentration of MTA)) is 2 or more, and more preferably 10 or more, and even more preferably 40 or more. The upper limit of the value of KD (with a high concentration of MTA)/KD (with a low concentration of MTA) is not particularly limited, and may be any value such as 400, 1000, 10000, and the like, as long as it can be produced with the skill of a person skilled in the art. If no antigen-binding activity is observed in the presence of a high concentration of MTA, this upper limit is an infinite number.

An antigen-binding domain whose antigen-binding activity in the presence of a high concentration of MTA is weaker than the antigen-binding activity in the presence of a low concentration of MTA includes an antigen-binding domain that does not substantially bind to the antigen in the presence of a high concentration of MTA.

As another indicator that shows the ratio between the antigen-binding activity of an antigen-binding domain (or an antigen-binding molecule containing the domain) of the present disclosure in the presence of a low concentration of MTA and the antigen-binding activity in the presence of a high concentration of MTA, for example, dissociation rate constant kd can be suitably used. When the dissociation rate constant (kd) is used instead of the dissociation constant (KD) as an indicator showing the binding activity ratio, the value of kd (in the presence of a low concentration of MTA) / kd (in the presence of a high concentration of MTA), which is a ratio between kd (dissociation rate constant) for an antigen in the presence of a low concentration of MTA and kd in the presence of a high concentration of MTA, is preferably 2 or greater, more preferably 5 or greater, even more preferably 10 or greater, and still more preferably 30 or greater. The upper limit of the value of kd (in the presence of a low concentration of MTA) / kd (in the presence of a high concentration of MTA) is not particularly limited, and may be any value, for example, 50, 100, or 200, as long as it can be provided by the common technical knowledge of those skilled in the art. When antigen-binding activity is not observed in the presence of a low concentration of MTA, there is no dissociation and the value of the upper limit becomes infinity.

As a method for evaluating the binding activity, a method for evaluating the relative binding activity can also be used in addition to the method for measuring the KD value described above. As non-limiting examples of such a method, methods using a flow cytometer, ELISA, capillary electrophoresis, liquid chromatography, or the like are generally known, but the method is not limited thereto. Further, even when the KD value cannot be calculated directly, it is possible to evaluate the relative binding activity, for example, by comparing with an antigen-binding binding molecule whose KD value is calculated by Biacore as a reference molecule, and evaluating if the test molecule has a stronger binding activity or weaker binding activity than the reference molecule.

For example, in a non-limiting embodiment of the threshold differentiating low and high concentrations of MTA, the threshold for a low-concentration condition may be selected appropriately from the values of 10 nM, 1 nM, 100 pM, 10 pM, 1 pM, and 0 M. Depending on the predetermined threshold, the high-concentration condition may be set appropriately at a value selected from at least 110%, at least 120%, at least 130%, at least 140%, at least 150%, at least twice, at least five-fold, at least 10-fold, at least 50-fold, at least 100-fold, at least $10^3$-fold, at least $10^4$-fold, at least $10^5$-fold, and at least $10^6$-fold the value of each threshold.

An example of a non-limiting embodiment of an antigen-binding domain of the present disclosure whose antigen-binding activity changes in an MTA-dependent manner includes an antigen-binding domain whose binding activity towards an antigen is substantially unaffected by one or more small molecule compounds selected from adenosine, S-(5'-adenosyl)-L-homocysteine (SAH), SAM, AMP, ADP, and ATP. Here, adenosine, S-(5'-adenosyl)-L-homocysteine (SAH), SAM, AMP, ADP, and ATP have adenosine as a common skeleton in the molecule and are compounds similar to MTA. It is generally considered difficult to obtain antigen-binding molecules in which the antigen-binding activity of the antigen-binding domain is not substantially affected by the presence of these similar molecules, and the binding activity is changed solely depending on MTA, as in the present disclosure. However, in the present disclosure as exemplified in various examples described later, a library for obtaining an antibody that binds to an antibody in an MTA-dependent manner was constructed, and by screening such a library, the present inventors succeeded in obtaining antigen-binding domains with excellent MTA-dependent specificity, which bind to an antigen in an MTA-specific and dependent manner and do not bind to an antigen depending on small molecule compounds having a structure similar to MTA.

In the present specification, "the antigen-binding activity is substantially unaffected by a small molecule compound" means that the antigen-binding activity of the antigen-binding domain in the presence of the small molecule compound and the antigen-binding activity of the antigen-binding domain in the absence of the small molecule compound is at least 0.5 to 2 times that of each other.

In one non-limiting embodiment, the antigen-binding activity of an antigen-binding domain according to the present disclosure whose antigen-binding activity changes in an MTA-dependent manner is substantially unaffected by

adenosine.

In another non-limiting embodiment, the antigen-binding activity of an antigen-binding domain according to the present disclosure whose antigen-binding activity changes in an MTA-dependent manner is substantially unaffected by S-(5'-adenosyl)-L-homocysteine (SAH).

In another non-limiting embodiment, the antigen-binding activity of an antigen-binding domain according to the present disclosure whose antigen-binding activity changes in an MTA-dependent manner is substantially unaffected by SAM

In another non-limiting embodiment, the antigen-binding activity of an antigen-binding domain according to the present disclosure whose antigen-binding activity changes in an MTA-dependent manner is substantially unaffected by any of adenosine, AMP, ADP, or ATP.

In another non-limiting embodiment, the antigen-binding activity of an antigen-binding domain according to the present disclosure whose antigen-binding activity changes in an MTA-dependent manner is substantially unaffected by any of adenosine or S-(5'-adenosyl)-L-homocysteine (SAH).

In another non-limiting embodiment, the antigen-binding activity of an antigen-binding domain according to the present disclosure whose antigen-binding activity changes in an MTA-dependent manner is substantially unaffected by any of adenosine, S-(5'-adenosyl)-L-homocysteine (SAH), or SAM

In another non-limiting embodiment, the antigen-binding activity of an antigen-binding domain according to the present disclosure whose antigen-binding activity changes in an MTA-dependent manner is substantially unaffected by any of adenosine, S-(5'-adenosyl)-L-homocysteine (SAH), SAM, AMP, ADP, or ATP.

A non-limiting example of an embodiment of an antigen-binding domain of the present disclosure whose antigen-binding activity changes in an MTA-dependent manner includes an antigen-binding domain whose antigen-binding activity changes also depending on one or more small molecule compounds selected from adenosine, S-(5'-adenosyl)-L-homocysteine (SAH), SAM, AMP, ADP, and ATP.

In one non-limiting embodiment, the antigen-binding activity of an antigen-binding domain according to the present disclosure whose antigen-binding activity changes in an MTA-dependent manner changes also in an adenosine-dependent manner. While not being bound by any particular theory, the antigen-binding domain whose binding activity changes depending on both MTA and adenosine molecules can be understood as that in which the antigen-binding activity changes by interacting with a common structure in both MTA and adenosine molecules. In that case, even if the concentration of MTA is decreased in the solvent for evaluating the binding activity, the binding activity is maintained, or may be detected strongly, if adenosine is present at a concentration exceeding the degree of decrease in binding activity due to the decrease in the concentration of MTA. Furthermore, even if MTA concentration is increased in the solvent for evaluating the binding activity, the binding activity is maintained, or maybe detected weakly, if the adenosine concentration decreases beyond the degree of increase in the binding activity due to the increase in MTA concentration.

In another non-limiting embodiment, the antigen-binding activity of an antigen-binding domain according to the present disclosure whose antigen-binding activity changes in an MTA-dependent manner changes also in an S-(5'-adenosyl)-L-homocysteine (SAH)-dependent manner.

In another non-limiting embodiment, the antigen-binding activity of an antigen-binding domain according to the present disclosure whose antigen-binding activity changes in an MTA-dependent manner changes also in a SAM-dependent manner.

In another non-limiting embodiment, the antigen-binding activity of an antigen-binding domain according to the present disclosure whose antigen-binding activity changes in an MTA-dependent manner also changes depending on any of adenosine, AMP, ADP, or ATP.

In another non-limiting embodiment, the antigen-binding activity of an antigen-binding domain according to the present disclosure whose antigen-binding activity changes in an MTA-dependent manner also changes depending on any of adenosine or S-(5'-adenosyl)-L-homocysteine (SAH).

In another non-limiting embodiment, the antigen-binding activity of an antigen-binding domain according to the present disclosure whose antigen-binding activity changes in an MTA-dependent manner also changes depending on any of adenosine, S-(5'-adenosyl)-L-homocysteine (SAH), or SAM

In another non-limiting embodiment, the antigen-binding activity of an antigen-binding domain according to the present disclosure whose antigen-binding activity changes in an MTA-dependent manner also changes depending on any of adenosine, S-(5'-adenosyl)-L-homocysteine (SAH), SAM, AMP, ADP, or ATP.

An example of a non-limiting embodiment of the antigen-binding domain of the present disclosure whose antigen-binding activity changes in an MTA-dependent manner includes an antigen-binding domain comprising an antibody variable region and/or a single-domain antibody.

As a non-limiting embodiment, the antigen-binding domain according to the present disclosure whose antigen-binding activity changes in an MTA-dependent manner is an antibody variable region, and an example of the antibody variable region includes an antigen-binding molecule comprising at least one or more amino acids selected from the group of amino acids below (Kabat numbering):

any of A, C, D, E, F, G, H, I, K, L, M, N, P, Q, R, S, T, V, W, or Y located at position 30 of the heavy chain;
any of A, C, D, E, F, G, H, I, K, L, M, N, P, Q, R, S, T, V, W, or Y located at position 31 of the heavy chain;
A located at position 32 of the heavy chain;
any of A, C, D, E, F, G, H, I, K, L, M, N, P, Q, R, S, T, V, W, or Y located at position 33 of the heavy chain;
W located at position 34 of the heavy chain;
M located at position 35 of the heavy chain;
C located at position 35a of the heavy chain;
C located at position 50 of the heavy chain;
I located at position 51 of the heavy chain;
F located at position 52 of the heavy chain;
A located at position 52a of the heavy chain;
any of A, D, E, G, H, I, K, L, M, N, P, Q, R, S, T, or V located at position 52b of the heavy chain;
any of A, C, D, E, F, G, H, I, K, L, M, N, P, Q, R, S, T, V, W, or Y located at position 52c of the heavy chain;
any of A, C, D, E, F, G, H, I, K, L, M, N, P, Q, R, S, T, V, W, or Y located at position 52d of the heavy chain;
Y located at position 52e of the heavy chain;
any of A, C, D, E, F, G, H, I, K, L, M, N, P, Q, R, S, T, V, W, or Y located at position 52f of the heavy chain;
any of A, C, D, E, F, G, H, I, K, L, M, N, P, Q, R, S, T, V, W, or Y located at position 52g of the heavy chain;
S located at position 53 of the heavy chain;
G located at position 54 of the heavy chain;
G located at position 55 of the heavy chain;
S located at position 56 of the heavy chain;
T located at position 57 of the heavy chain;
Y located at position 58 of the heavy chain;
Y located at position 59 of the heavy chain;
A located at position 60 of the heavy chain;
S located at position 61 of the heavy chain;
W located at position 62 of the heavy chain;
A located at position 63 of the heavy chain;
K located at position 64 of the heavy chain;
G located at position 65 of the heavy chain;
G located at position 95 of the heavy chain;
any of A, C, D, E, F, G, H, I, K, L, M, N, P, Q, R, S, T, V, W, or Y located at position 96 of the heavy chain;
G located at position 97 of the heavy chain;
any of A, C, D, E, F, G, H, I, K, L, M, N, P, Q, R, S, T, V, W, or Y located at position 98 of the heavy chain;
any of A, C, D, E, F, G, H, I, K, L, M, N, P, Q, R, S, T, V, W, or Y located at position 99 of the heavy chain;
any of A, C, D, E, F, G, H, I, K, L, M, N, P, Q, R, S, T, V, W, or Y located at position 100 of the heavy chain;
G located at position 100a of the heavy chain;
any of A, C, D, E, F, G, H, I, K, L, M, N, P, Q, R, S, T, V, W, or Y located at position 100b of the heavy chain;
any of A, C, D, E, F, G, H, I, K, L, M, N, P, Q, R, S, T, V, W, or Y located at position 100c of the heavy chain;
E located at position 101 of the heavy chain;
L located at position 102 of the heavy chain;
Q located at position 24 of the light chain;
S located at position 25 of the light chain;
S located at position 26 of the light chain;
E located at position 27 of the light chain;
any of A, C, D, E, F, G, H, I, K, L, M, N, P, Q, R, S, T, V, W, or Y located at position 27a of the light chain;
V located at position 28 of the light chain;
any of A, C, D, E, F, G, H, I, K, L, M, N, P, Q, R, S, T, V, W, or Y located at position 29 of the light chain;
any of A, C, D, E, F, G, H, I, K, L, M, N, P, Q, R, S, T, V, W, or Y located at position 30 of the light chain;
any of A, D, E, G, H, I, K, L, M, N, P, Q, R, S, T, or V located at position 31 of the light chain;
any of A, C, D, E, F, G, H, I, K, L, M, N, P, Q, R, S, T, V, W, or Y located at position 32 of the light chain;
L located at position 33 of the light chain;
S located at position 34 of the light chain;
any of A, C, D, E, F, G, H, I, K, L, M, N, P, Q, R, S, T, V, W, or Y located at position 49 of the light chain;
any of A, C, D, E, F, G, H, I, K, L, M, N, P, Q, R, S, T, V, W, or Y located at position 50 of the light chain;
A located at position 51 of the light chain;
any of A, C, D, E, F, G, H, I, K, L, M, N, P, Q, R, S, T, V, W, or Y located at position 52 of the light chain;
T located at position 53 of the light chain;

any of A, C, D, E, F, G, H, I, K, L, M, N, P, Q, R, S, T, V, W, or Y located at position 54 of the light chain;
P located at position 55 of the light chain;
any of A, C, D, E, F, G, H, I, K, L, M, N, P, Q, R, S, T, V, W, or Y located at position 56 of the light chain;
A located at position 89 of the light chain;
G located at position 90 of the light chain;
L located at position 91 of the light chain;
Y located at position 92 of the light chain;
any of A, C, D, E, F, G, H, I, K, L, M, N, P, Q, R, S, T, V, W, or Y located at position 93 of the light chain;
G located at position 94 of the light chain;
N located at position 95 of the light chain;
I located at position 95a of the light chain;
P located at position 96 of the light chain; and
A located at position 97 of the light chain.

In another non-limiting embodiment, the antigen-binding domain according to the present disclosure whose antigen-binding activity changes in an MTA-dependent manner is an antibody variable region, and an example of the antibody variable region includes an antigen-binding molecule comprising at least one or more amino acids selected from the group of amino acids below (Kabat numbering):

any of A, C, D, E, F, G, H, I, K, L, M, N, P, Q, R, S, T, V, W, or Y located at position 31 of the heavy chain;
any of A, C, D, E, F, G, H, I, K, L, M, N, P, Q, R, S, T, V, W, or Y located at position 32 of the heavy chain;
any of A, C, D, E, F, G, H, I, K, L, M, N, P, Q, R, S, T, V, W, or Y located at position 33 of the heavy chain;
W located at position 34 of the heavy chain;
M located at position 35 of the heavy chain;
C located at position 35a of the heavy chain;
C located at position 50 of the heavy chain;
I located at position 51 of the heavy chain;
any of A, C, D, E, F, G, H, I, K, L, M, N, P, Q, R, S, T, V, W, or Y located at position 52 of the heavy chain;
S located at position 52a of the heavy chain;
any of A, C, D, E, F, G, H, I, K, L, M, N, P, Q, R, S, T, V, W, or Y located at position 53 of the heavy chain;
any of A, C, D, E, F, G, H, I, K, L, M, N, P, Q, R, S, T, V, W, or Y located at position 54 of the heavy chain;
any of A, C, D, E, F, G, H, I, K, L, M, N, P, Q, R, S, T, V, W, or Y located at position 55 of the heavy chain;
any of A, C, D, E, F, G, H, I, K, L, M, N, P, Q, R, S, T, V, W, or Y located at position 56 of the heavy chain;
T located at position 57 of the heavy chain;
any of A, C, D, E, F, G, H, I, K, L, M, N, P, Q, R, S, T, V, W, or Y located at position 58 of the heavy chain;
Y located at position 59 of the heavy chain;
A located at position 60 of the heavy chain;
S located at position 61 of the heavy chain;
W located at position 62 of the heavy chain;
V located at position 63 of the heavy chain;
N located at position 64 of the heavy chain;
G located at position 65 of the heavy chain;
E located at position 95 of the heavy chain;
any of A, C, D, E, F, G, H, I, K, L, M, N, P, Q, R, S, T, V, W, or Y located at position 96 of the heavy chain;
any of A, C, D, E, F, G, H, I, K, L, M, N, P, Q, R, S, T, V, W, or Y located at position 97 of the heavy chain;
any of A, C, D, E, F, G, H, I, K, L, M, N, P, Q, R, S, T, V, W, or Y located at position 98 of the heavy chain;
any of A, C, D, E, F, G, H, I, K, L, M, N, P, Q, R, S, T, V, W, or Y located at position 99 of the heavy chain;
S located at position 100 of the heavy chain;
G located at position 100a of the heavy chain;
A located at position 100b of the heavy chain;
L located at position 100c of the heavy chain;
N located at position 101 of the heavy chain;
L located at position 102 of the heavy chain;
H located at position 24 of the light chain;
S located at position 25 of the light chain;
S located at position 26 of the light chain;
K located at position 27 of the light chain;
any of A, C, D, E, F, G, H, I, K, L, M, N, P, Q, R, S, T, V, W, or Y located at position 27a of the light chain;

V located at position 27b of the light chain;
any of A, C, D, E, F, G, H, I, K, L, M, N, P, Q, R, S, T, V, W, or Y located at position 28 of the light chain;
any of A, C, D, E, F, G, H, I, K, L, M, N, P, Q, R, S, T, V, W, or Y located at position 29 of the light chain;
any of A, C, D, E, F, G, H, I, K, L, M, N, P, Q, R, S, T, V, W, or Y located at position 30 of the light chain;
any of A, C, D, E, F, G, H, I, K, L, M, N, P, Q, R, S, T, V, W, or Y located at position 31 of the light chain;
any of A, C, D, E, F, G, H, I, K, L, M, N, P, Q, R, S, T, V, W, or Y located at position 32 of the light chain;
L located at position 33 of the light chain;
A located at position 34 of the light chain;
any of A, C, D, E, F, G, H, I, K, L, M, N, P, Q, R, S, T, V, W, or Y located at position 49 of the light chain;
any of A, C, D, E, F, G, H, I, K, L, M, N, P, Q, R, S, T, V, W, or Y located at position 50 of the light chain;
A located at position 51 of the light chain;
any of A, C, D, E, F, G, H, I, K, L, M, N, P, Q, R, S, T, V, W, or Y located at position 52 of the light chain;
any of A, C, D, E, F, G, H, I, K, L, M, N, P, Q, R, S, T, V, W, or Y located at position 53 of the light chain;
L located at position 54 of the light chain;
A located at position 55 of the light chain;
S located at position 56 of the light chain;
Q located at position 89 of the light chain;
G located at position 90 of the light chain;
T located at position 91 of the light chain;
Y located at position 92 of the light chain;
any of A, C, D, E, F, G, H, I, K, L, M, N, P, Q, R, S, T, V, W, or Y located at position 93 of the light chain;
any of A, C, D, E, F, G, H, I, K, L, M, N, P, Q, R, S, T, V, W, or Y located at position 94 of the light chain;
any of A, C, D, E, F, G, H, I, K, L, M, N, P, Q, R, S, T, V, W, or Y located at position 95 of the light chain;
any of A, C, D, E, F, G, H, I, K, L, M, N, P, Q, R, S, T, V, W, or Y located at position 95a of the light chain;
F located at position 95b of the light chain;
Y located at position 95c of the light chain;
F located at position 96 of the light chain; and
A located at position 97 of the light chain.

As a further non-limiting embodiment, the antigen-binding domain according to the present disclosure whose antigen-binding activity changes in an MTA-dependent manner is an antibody variable region, and an example of the antibody variable region includes an antigen-binding molecule comprising at least one or more amino acids selected from the group of amino acids below (Kabat numbering):

any of A, D, E, F, G, H, I, K, L, M, N, P, Q, R, S, T, V, W, or Y located at position 26 of the heavy chain;
any of A, D, E, F, G, H, I, K, L, M, N, P, Q, R, S, T, V, W, or Y located at position 28 of the heavy chain;
either A or L located at position 29 of the heavy chain;
any of A, D, E, F, G, H, I, K, L, M, N, P, Q, R, S, T, V, W, or Y located at position 30 of the heavy chain;
any of A, D, E, F, G, H, I, K, L, N, Q, R, S, T, V, W, or Y located at position 31 of the heavy chain;
any of D, E, F, H, N, P, R, or Y located at position 32 of the heavy chain;
any of A, I, P, T, or V located at position 33 of the heavy chain;
any of A, E, F, H, I, K, L, M, N, Q, S, T, V, W, or Y located at position 34 of the heavy chain;
G located at position 35 of the heavy chain;
any of D, I, or V located at position 50 of the heavy chain;
I located at position 51 of the heavy chain;
G located at position 52 of the heavy chain;
any of A, D, E, G, I, K, Q, or R located at position 53 of the heavy chain;
any of D, E, F, G, H, I, K, L, P, Q, R, S, T, V, W, or Y located at position 54 of the heavy chain;
any of A, D, E, F, G, or H located at position 55 of the heavy chain;
any of A, D, E, F, G, H, I, K, L, N, Q, R, S, T, V, W, or Y located at position 56 of the heavy chain;
any of A, D, E, G, H, I, K, L, N, P, Q, R, S, T, or V located at position 57 of the heavy chain;
W located at position 58 of the heavy chain;
any of A, D, E, F, G, H, I, K, L, Q, R, S, T, V, W, or Y located at position 59 of the heavy chain;
P located at position 60 of the heavy chain;
any of A, F, Q, R, S, T, V, W, or Y located at position 61 of the heavy chain;
W located at position 62 of the heavy chain;
V located at position 63 of the heavy chain;
K located at position 64 of the heavy chain;

A, F, or G located at position 65 of the heavy chain;
G located at position 95 of the heavy chain;
any of A, E, F, G, H, K, L, Q, R, S, T, W, or Y located at position 96 of the heavy chain;
any of A, F, H, K, N, W, or Y located at position 97 of the heavy chain;
any of A, D, E, F, G, H, I, K, L, N, P, Q, R, S, T, V, W, or Y located at position 98 of the heavy chain;
any of A, D, E, G, H, Q, or S located at position 99 of the heavy chain;
F or Y located at position 100 of the heavy chain;
N, T, or V located at position 100a of the heavy chain;
N located at position 100b of the heavy chain;
A located at position 100c of the heavy chain;
F or W located at position 100d of the heavy chain;
D located at position 101 of the heavy chain;
P located at position 102 of the heavy chain;
Q located at position 24 of the light chain;
S located at position 25 of the light chain;
S located at position 26 of the light chain;
Q located at position 27 of the light chain;
S located at position 27e of the light chain;
V located at position 27f of the light chain;
any of A, E, F, H, I, K, L, N, R, S, T, V, W, or Y located at position 28 of the light chain;
any of A, D, E, F, G, H, I, K, L, N, P, Q, R, S, T, V, W, or Y located at position 29 of the light chain;
N located at position 30 of the light chain;
N located at position 31 of the light chain;
any of A, E, F, G, H, S, or Y located at position 32 of the light chain;
L located at position 33 of the light chain;
S located at position 34 of the light chain;
D located at position 50 of the light chain;
A located at position 51 of the light chain;
S located at position 52 of the light chain;
T located at position 53 of the light chain;
L located at position 54 of the light chain;
A located at position 55 of the light chain;
S located at position 56 of the light chain;
H located at position 89 of the light chain;
G located at position 90 of the light chain;
any of A, S, or T located at position 91 of the light chain;
any of A, D, E, F, G, H, I, K, L, M, N, Q, R, S, T, V, W, or Y located at position 92 of the light chain;
any of A, D, E, F, G, H, L, N, Q, R, S, T, V, or Y located at position 93 of the light chain;
any of A, E, F, G, H, I, K, L, N, P, Q, R, S, T, V, W, or Y located at position 94 of the light chain;
any of A, D, E, F, G, H, I, K, L, N, P, Q, R, S, T, V, W, or Y located at position 95 of the light chain;
any of A, D, E, F, G, H, I, K, L, N, P, Q, R, V, W, or Y located at position 95a of the light chain;
any of A, D, E, F, G, H, I, K, L, N, P, Q, R, S, T, V, W, or Y located at position 95b of the light chain;
any of A, F, H, I, K, L, N, P, Q, R, S, T, V, W, or Y located at position 95c of the light chain;
D located at position 96d of the light chain;
N located at position 96 of the light chain;
A or G located at position 97 of the light chain; and
A, F, I, L, or V located at position 98 of the light chain.

The antigen-binding domain of the present disclosure may have amino acid residues that interact with MTA. The amino acid residues that interact with MTA may be present at the interface that directly interacts with the antigen in the antigen-binding domain, or may be present at another site. "Interface that directly interacts with an antigen" refers to a site in which an antigen and an antigen-binding molecule are in close proximity to each other in the structure of a complex between an antigen and an antigen-binding domain when analyzed by a method such as crystal structure analysis. A distance of 4 to 6 Å can be exemplified as the distance in which the antigen and the antigen-binding molecule are in close proximity to each other, but in the structure of the complex of the antigen and the antigen-binding molecule, "the antigen and the antigen-binding molecule are in close proximity to each other" refers to sites that are relatively close to each other in the complex, and is not limited to the distance illustrated above. An amino acid residue that interacts with MTA may be a residue that interacts with MTA in a state where the antigen-binding molecule is bound to the antigen,

or a residue that interacts with MTA in the absence of the antigen. Furthermore, the amino acid residue that interacts with MTA can be a residue that interacts with MTA both in a state where the antigen-binding molecule is bound to the antigen and in the absence of the antigen. Further, the amino acid residue that interacts with MTA may be only one residue or a plurality of amino acid residues in the antigen-binding domain.

In an embodiment in which the antigen-binding domain comprises an antibody heavy chain variable region and an antibody light chain variable region, the amino acid that interacts with MTA may be present in the CDR or in the FR in the antibody variable region. In embodiments where the antigen-binding domain comprises a single-domain antibody, the amino acid that interacts with MTA may be present in the CDR or FR in the single-domain antibody.

An amino acid residue in the antigen-binding domain that interacts with MTA can be identified by analyzing a two-molecule complex of MTA and an antigen-binding molecule comprising an antigen-binding domain, or a three-molecule complex of MTA, an antigen, and an antigen-binding molecule comprising an antigen-binding domain, using a method such as crystal structure analysis, three-dimensional structure analysis using NMR, or introduction of an amino acid mutation.

As a non-limiting embodiment of the present disclosure, an amino acid residue of the antigen-binding molecule that interacts with MTA can be identified from a crystal structure analysis of a two-molecule complex of MTA and antigen-binding molecule. Here, "interacts with MTA" means, the condition where an interaction between an antigen-binding molecule and MTA is taking place between the atoms of the main chain or side chains of the amino acids forming the antigen-binding molecule and the atoms of the small molecule compound at a distance that may have an effect on the MTA-binding activity; the condition where certain amino acid residues are involved in the MTA binding, including an indirect effect of stabilizing the three-dimensional structure of the CDR loop and such to the conformation when bound to MTA in an embodiment where the antigen-binding domain within the antigen-binding molecule includes an antibody heavy chain variable region and an antibody light chain variable region; and a condition that satisfies both of these conditions.

The "condition where intermolecular interactions are taking place" in the present specification can be determined, for example, based on the interatomic distances between non-hydrogen atoms constituting the main chain or side chains of the amino acids that form the antigen-binding molecule and the non-hydrogen atoms constituting MTA obtained from a crystal structure analysis of the two-molecule complex formed by MTA and the antigen-binding molecule. For example, the above-mentioned interatomic distances are preferably within 3.0 Å, 3.2 Å, 3.4 Å, 3.6 Å, 3.8 Å, 4.0 Å, 4.2 Å, 4.4 Å, 4.6 Å, 4.8 Å, or 5.0 Å, but are not limited thereto. More preferably, examples of the interatomic distance are within 3.6 Å, 3.8 Å, 4.0 Å, or 4.2 Å.

More specifically, the possibility of a direct interaction can be determined based on information on the interatomic distances in the three-dimensional structure, the types of intermolecular interactions that take place, and the types of atoms. The determination can be done more accurately by, without limitation, observing the effect of introducing amino acid residue mutations, such as modifications to Ala or Gly, on the activity of the small molecule compound.

Also with respect to the "indirectly influencing condition" in the present specification, whether there is an indirect influence on MTA binding can be estimated, for example, by analyzing in detail the state of the conformation of amino acid residues and intermolecular interactions with surrounding residues from the three-dimensional structure of the two-molecule complex of MTA and an antigen-binding molecule. The determination can be done more accurately by observing the effect of introducing amino acid residue mutations such as modifications to Ala or Gly on the activity of MTA.

As one non-limiting embodiment of the present disclosure, an amino acid residue of the antigen-binding molecule that interacts with MTA can be identified from a crystal structure analysis of the three-molecule complex of MTA, antigen, and antigen-binding molecule. Here, "interacts with MTA" means the condition where an interaction between an antigen-binding molecule and MTA is taking place between the atoms of the main chain or side chains of the amino acids forming the antigen-binding molecule and the atoms of the small molecule compound at a distance that may have an effect on the MTA-binding activity in the presence of an antigen; the condition where certain amino acid residues are involved in the MTA binding, including an indirect effect of stabilizing the three-dimensional structure of the CDR loop and such to the conformation when bound to MTA in the presence of an antigen in an embodiment where the antigen-binding domain within the antigen-binding molecule includes an antibody heavy chain variable region and an antibody light chain variable region; and a condition that satisfies both of these conditions.

The "condition where intermolecular interactions are taking place" in the present specification can be determined, for example, based on the interatomic distances, in the presence of the antigen, between non-hydrogen atoms constituting the main chain or side chains of the amino acids that form the antigen-binding domain and the non-hydrogen atoms constituting MTA obtained from a crystal structure analysis of the three-molecule complex formed by MTA, antigen, and the antigen-binding molecule. For example, the above-mentioned interatomic distances are preferably within 3.0 Å, 3.2 Å, 3.4 Å, 3.6 Å, 3.8 Å, 4.0 Å, 4.2 Å, 4.4 Å, 4.6 Å, 4.8 Å, or 5.0 Å, but are not limited thereto. More preferably, examples of the interatomic distance are within 3.6 Å, 3.8 Å, 4.0 Å, or 4.2 Å.

More specifically, the possibility of a direct interaction can be determined based on information on the interatomic distances in the three-dimensional structure, the types of intermolecular interactions that take place, and the types of

atoms. The determination can be done more accurately by, without limitation, observing the effect of introducing amino acid residue mutations such as modifications to Ala or Gly on the activity of a small molecule compound.

Also with respect to the "indirectly influencing condition" in the present specification, whether there is an indirect influence on MTA-binding in the presence of an antigen can be estimated, for example, by analyzing in detail the state of the conformation of amino acid residues and intermolecular interactions with the surrounding residues from the three-dimensional structure of the three-molecule complex of MTA, antigen, and antigen-binding molecule. The determination can be done more accurately by observing the effect of introducing amino acid residue mutations such as modifications to Ala or Gly on the activity of MTA.

In one non-limiting embodiment, the antigen-binding domain is an antibody variable region, and an example may include an antigen-binding domain where an amino acid residue that interacts with MTA is an amino acid residue located in at least one or more amino acid sites selected from the group of amino acid sites of heavy chain positions 34, 35a, 47, 52, 52e, and 101, and light chain positions 32, 34, 36, 46, 49, 50, 89, 90, 91, and 96, specified by Kabat numbering, in the amino acid sequence of the antibody variable region.

In one non-limiting embodiment, the antigen-binding domain is an antibody variable region, and an example is an antigen-binding domain wherein the antibody variable region comprises at least one or more amino acids selected from heavy chain W34, C35a, W47, F52, Y52e, and E101, and light chain R32, S34, Y36, L46, Y49, S50, A89, G90, L91, and P96 (Kabat numbering).

Further, as a non-limiting embodiment, the antigen-binding domain is an antibody variable region, and an example is an antigen-binding domain wherein an amino acid residue that interacts with MTA is an amino acid residue located in at least one or more amino acid sites selected from the group of amino acid sites of heavy chain positions 34, 47, 50, 58, 95, 98, 99, and 100a, and light chain positions 28, 91, 95b, 95c, and 96 specified by Kabat numbering in the amino acid sequence of the antibody variable region.

As a non-limiting embodiment, the antigen-binding domain is an antibody variable region, and an example is an antigen-binding domain wherein the antibody variable region comprises at least one or more amino acids selected from heavy chain W34, W47, C50, Y58, E95, F98, G99, and G100a, and light chain Y28, T91, F95b, Y95c, and F96 (Kabat numbering).

Further, as a non-limiting embodiment of an antigen-binding domain that interacts with MTA, the antigen-binding domain is an antibody variable region, and an example is an antigen-binding domain wherein the amino acid residue that interacts with MTA is an amino acid residue located in at least one or more amino acid sites selected from the group of amino acid sites of heavy chain positions 33, 50, 52, 54, 56, 57, 58, 99, 100, 100a, 91, 95c, and 96 specified by Kabat numbering in the amino acid sequence of the antibody variable region.

Furthermore, the antigen-binding domain is an antibody variable region, and an example is an antigen-binding domain wherein the antibody variable region comprises at least one or more amino acids selected from heavy chain A33, I50, G52, D54, S56, T57, W58, G99, Y100, and T100a, and light chain S91, Y95c, and N96 (Kabat numbering).

## Antigen-binding molecules comprising an antigen-binding domain whose antigen-binding activity changes in an MTA-dependent manner

In one non-limiting embodiment, the antigen-binding molecules comprising an antigen-binding domain whose antigen-binding activity changes in an MTA-dependent manner in the present disclosure are molecules comprising an antibody Fc region. The antibody Fc region contained in the antigen-binding molecule of the present disclosure may be a native Fc region or a modified Fc region. Examples of a native Fc region include an Fc region represented by human IgG1 (SEQ ID NO: 5), IgG2 (SEQ ID NO: 6), IgG3 (SEQ ID NO: 7), or IgG4 (SEQ ID NO: 8).

An antigen-binding molecule of the present disclosure may contain at least some portions of an Fc region that mediates the binding to Fcγ receptor and/or FcRn. In a non-limiting embodiment, the antigen-binding molecule includes, for example, antibodies and Fc fusion proteins. A fusion protein refers to a chimeric polypeptide comprising a polypeptide having a first amino acid sequence that is linked to a polypeptide having a second amino acid sequence that would not naturally link in nature. For example, a fusion protein may comprise a polypeptide comprising the amino acid sequence of at least a portion of an Fc region (for example, a portion of an Fc region responsible for the binding to Fcγ receptor, and/or a portion of an Fc region responsible for the binding to FcRn). The amino acid sequences may be present in separate proteins that are transported together to a fusion protein, or generally may be present in a single protein; however, they are included in a new rearrangement in a fusion polypeptide. Fusion proteins can be produced, for example, by chemical synthesis, or by genetic recombination techniques to express a polynucleotide encoding peptide regions in a desired arrangement.

Respective domains in the antigen-binding molecules of the present disclosure can be linked together via linkers or directly via polypeptide binding. The linkers comprise arbitrary peptide linkers that can be introduced by genetic engineering, synthetic linkers, and linkers disclosed in, for example, Holliger et al., Protein Engineering (1996) 9(3), 299-305. However, peptide linkers are preferred in the present disclosure. The length of the peptide linkers is not

particularly limited, and can be suitably selected by those skilled in the art according to the purpose. The length is preferably five amino acids or more (without particular limitation, the upper limit is generally 30 amino acids or less, preferably 20 amino acids or less), and particularly preferably 15 amino acids.

For example, such peptide linkers preferably include:

Ser
Gly·Ser
Gly·Gly·Ser
Ser·Gly·Gly
Gly·Gly·Gly·Ser (SEQ ID NO: 19)
Ser·Gly·Gly·Gly (SEQ ID NO: 20)
Gly·Gly·Gly·Gly·Ser (SEQ ID NO: 21)
Ser·Gly·Gly·Gly·Gly (SEQ ID NO: 22)
Gly·Gly·Gly·Gly·Gly·Ser (SEQ ID NO: 23)
Ser·Gly·Gly·Gly·Gly·Gly (SEQ ID NO: 24)
Gly·Gly·Gly·Gly·Gly·Gly·Ser (SEQ ID NO: 25)
Ser·Gly·Gly·Gly·Gly·Gly·Gly (SEQ ID NO: 26)
(Gly·Gly·Gly·Gly·Ser (SEQ ID NO: 21))n
(Ser·Gly·Gly·Gly·Gly (SEQ ID NO: 22))n

where n is an integer of 1 or larger. The length or sequences of peptide linkers can be selected accordingly by those skilled in the art depending on the purpose.

Synthetic linkers (chemical crosslinking agents) is routinely used to crosslink peptides, and for example:

N-hydroxy succinimide (NHS),
disuccinimidyl suberate (DSS),
bis(sulfosuccinimidyl) suberate ($BS^3$),
dithiobis(succinimidyl propionate) (DSP),
dithiobis(sulfosuccinimidyl propionate) (DTSSP),
ethylene glycol bis(succinimidyl succinate) (EGS),
ethylene glycol bis(sulfosuccinimidyl succinate) (sulfo-EGS),
disuccinimidyl tartrate (DST), disulfosuccinimidyl tartrate (sulfo-DST),
bis[2-(succinimidoxycarbonyloxy)ethyl] sulfone (BSOCOES),

and bis[2-(sulfosuccinimidoxycarbonyloxy)ethyl] sulfone (sulfo-BSOCOES). These crosslinking agents are commercially available.

When multiple linkers for linking the respective domains are used, they may all be of the same type, or may be of different types. In addition to the linkers exemplified above, linkers with peptide tags such as His tag, HA tag, myc tag, and FLAG tag may also be suitably used. Furthermore, hydrogen bonding, disulfide bonding, covalent bonding, ionic interaction, and properties of binding with each other as a result of combination thereof may be suitably used. For example, the affinity between CH1 and CL of antibody may be used, and Fc regions originating from the above-described bispecific antibodies may also be used for hetero Fc region association. Moreover, disulfide bonds formed between domains may also be suitably used.

In order to link respective domains via peptide linkage, polynucleotides encoding the domains are linked together in frame. Known methods for linking polynucleotides in frame include techniques such as ligation of restriction fragments, fusion PCR, and overlapping PCR. Such methods can be appropriately used alone or in combination to construct antigen-binding molecules of the present disclosure. In the present disclosure, the terms "linked" and "fused", or "linkage" and "fusion" are used interchangeably. These terms mean that two or more elements or components such as polypeptides are linked together to form a single structure by any means including the above-described chemical linking means and genetic recombination techniques. Fusing in frame means, when two or more elements or components are polypeptides, linking two or more units of reading frames to form a continuous longer reading frame while maintaining the correct reading frames of the polypeptides. When two molecules of Fab are used as an antigen-binding domain, an antibody, which is an antigen-binding molecule of the present disclosure where the antigen-binding domain is linked in frame to a constant region including an Fc region via peptide bond without linker, can be used as a preferred antigen-binding molecule of the present disclosure.

In another aspect, the present disclosure provides antigen-binding molecules with high plasma retention ability. In one aspect, the antigen-binding activity of the antigen-binding molecule increases as the concentration of MTA increases. In some embodiments, the antigen-binding molecule has a higher antigen-binding activity in a target tissue

than the antigen-binding activity in a non-target tissue. In some aspects, the antigen-binding molecule is an antibody. Although not bound by any particular theory, the above-mentioned changes in plasma kinetics can be interpreted as follows. As the antigen-binding activity of the antigen-binding molecule depending on MTA concentration increases, the antigen-binding ability of the antigen-binding molecule in tissues other than the target tissue decreases. As a result, the antigen-dependent elimination (clearance) of the antigen-binding molecule in tissues other than the target tissue is reduced. Overall, the reduction in antigen-dependent elimination (clearance) in most tissues in the body (tissues other than the target tissue) leads to a high plasma retention of the antigen-binding molecule.

Whether or not an antigen-binding molecule in the present invention has high plasma retention can be determined by a relative comparison with a control antigen-binding molecule. In some embodiments, the antigen-binding molecule whose antigen-binding activity increases with the increase of MTA concentration has a higher plasma retention as compared to the control antigen-binding molecule. In one embodiment, the control antigen-binding molecule is an antigen-binding molecule that does not have an MTA concentration-dependent antigen-binding activity. In a particular embodiment, an antigen-binding molecule that does not have antigen-binding activity dependent on a compound's concentration means that the difference in antigen-binding activity between the presence and absence of MTA is, for example, less than 2-times, less than 1.8 times, less than 1.5 times, less than 1.3 times, less than 1.2 times, or less than 1.1 times. From a comparative point of view, it is desirable that the antigen-binding molecule of the present disclosure and the control antigen-binding molecule have substantially the same antigen-binding activity in the presence of a sufficient amount of MTA.

Here, it is thought that the magnitude of the antigen-dependent elimination of the antigen-binding molecule detected in the living body changes according to a quantitative balance between the antigen and antigen-binding molecule present in plasma. In general, the more antigens/the less antigen-binding molecules present in plasma, the easier it is to detect antigen-dependent elimination of antigen-binding molecules, and conversely, the less antigens/antigen-binding molecules present in plasma, the more difficult it is to detect the antigen-dependent elimination of antigen-binding molecules. The antigen-binding molecule of the present disclosure need not exhibit a high plasma retention under all conditions, but it is sufficient to show a high plasma retention under conditions appropriate for detection of a sufficient antigen-dependent elimination. When the amount of antigen in plasma is small, the amount of antigen may be increased by some artificial means and then the plasma retention may be evaluated.

In another aspect, the present invention provides an antigen-binding molecule having a low plasma antigen-accumulating capacity. In a further embodiment, the antigen-binding activity of the antigen-binding molecule increases with the increase in MTA concentration. In a certain embodiment, MTA is a target tissue-specific compound. In a further embodiment, the antigen-binding molecule has higher antigen-binding activity in a target tissue than the antigen-binding activity in a non-target tissue. In some aspects, the antigen-binding molecule is an antibody. Although not bound by any particular theory, the above-mentioned changes in plasma kinetics can be interpreted as follows. As the antigen-binding activity of the antigen-binding molecule depending on MTA concentration increases, the antigen-binding ability of the antigen-binding molecule in tissues other than the target tissue decreases. As a result, the ability of the antigen-binding molecule to form an antigen-antibody complex in a tissue other than the target tissue is reduced. In general, it is known that when an antigen-binding molecule such as an antibody binds to an antigen, the clearance of the antigen decreases and the concentration of the antigen in plasma increases (antigen accumulates). Overall, the decrease in the ability to form an antigen-antibody complex in most tissues in the body (tissues other than the target tissue) leads to low antigen accumulation (in other words, low antigen-accumulating capacity of the antigen-binding molecule). Whether or not the antigen-binding molecule in the present invention has a low plasma antigen-accumulating ability can be determined by a relative comparison with a control antigen-binding molecule. In some embodiments, the antigen-binding molecule whose antigen-binding activity increases as the concentration of MTA increases has a lower plasma antigen-accumulating capacity as compared to a control antigen-binding molecule. In one embodiment, the control antigen-binding molecule is an antigen-binding molecule that does not have antigen-binding activity depending on the concentration of MTA. In a particular embodiment, an antigen-binding molecule that does not have MTA concentration-dependent antigen-binding activity means an antigen-binding molecule wherein the difference in antigen-binding activity between the presence or absence of the compound is, for example, less than 2-times, less than 1.8 times, less than 1.5 times, less than 1.3 times, less than 1.2 times, or less than 1.1 times. From a comparative viewpoint, it is desirable that the antigen-binding molecule of the present invention and the control antigen-binding molecule have substantially the same antigen-binding activity in the presence of a sufficient amount of the compound.

Here, the amount of the antigen-antibody complex formed in the living body is considered to depend on the amount of antigen and antibody present in plasma. In general, it is thought that as the amount of antigen/antibody in plasma increases, the amount of antigen-antibody complex formed also increases, and conversely, as the amount of antigen/antibody in plasma decreases, the amount of antigen-antibody complex formed decreases. The antigen-binding molecules of the present invention do not have to exhibit a low plasma antigen-accumulating capacity under all conditions, and it is sufficient to show a low plasma antigen-accumulating capacity under conditions appropriate for forming a sufficient amount of antigen-antibody complex. If the amount of antigen in plasma is low, the amount of antigen may be

increased by some artificial means and then antigen-accumulating capacity can be evaluated.

Fcγ receptor (FcγR)

"Fcγ receptor" (also called "FcγR") refers to a receptor capable of binding to the Fc region of monoclonal IgG1, IgG2, IgG3, or IgG4 antibodies; and means all members belonging to the family of proteins substantially encoded by Fcγ receptor genes. In humans, the family includes FcγRI (CD64) including isoforms FcγRIa, FcγRIb, and FcγRIc; FcγRII (CD32) including isoforms FcγRIIa (including allotype H131 and R131, i.e., FcγRIIa(H) and FcγRIIa(R)), FcγRIIb (including FcγRIIb-1 and FcγRIIb-2), and FcγRIIc; and FcγRIII (CD16) including isoform FcγRIIIa (including allotype V158 and F158, i.e., FcγRIIIa(V) and FcγRIIIa(F)) and FcγRIIIb (including allotype FcγRIIIb-NA1 and FcγRIIIb-NA2); as well as all unidentified human FcγRs, FcγR isoforms, and allotypes thereof; but the family is not limited to these examples. Without being limited thereto, FcγRs include those derived from humans, mice, rats, rabbits, and monkeys. FcγRs may be derived from any organism. Mouse FcγRs include FcγRI (CD64), FcγRII (CD32), FcγRIII (CD16), and FcγRIII-2 (FcγRIV, CD16-2), as well as all unidentified mouse FcγRs, FcγR isoforms, and allotypes thereof, but they are not limited to these examples. Preferred examples of such Fcγ receptors include, human FcγRI (CD64), FcγRIIa (CD32), FcγRIIb (CD32), FcγRIIIa (CD16), and/or FcγRIIIb (CD16). The polynucleotide sequence and amino acid sequence of human FcγRI are shown in SEQ ID NOs: 9 (NM_000566.3) and 10 (NP_000557.1), respectively; the polynucleotide sequence and amino acid sequence of human FcγRIIa (allotype H131) are shown in SEQ ID NOs: 11 (BC020823.1) and 12 (AAH20823.1), respectively (allotype R131 is a sequence in which the amino acid at position 166 of SEQ ID NO: 12 is substituted with Arg); the polynucleotide sequence and amino acid sequence of FcγIIb are shown in SEQ ID NOs: 13 (BC146678.1) and 14 (AAI46679.1), respectively; the polynucleotide sequence and amino acid sequence of FcγRIIIa are shown in SEQ ID NOs: 15 (BC033678.1) and 16 (AAH33678.1), respectively; and the polynucleotide sequence and amino acid sequence of FcγRIIIb are shown in SEQ ID NOs: 17 (BC128562.1) and 18 (AAI28563.1), respectively (RefSeq accession number or such is shown in parentheses). Whether an Fcγ receptor has binding activity to the Fc region of a monoclonal IgG1, IgG2, IgG3, or IgG4 antibody can be assessed by ALPHA (Amplified Luminescent Proximity Homogeneous Assay) screen, surface plasmon resonance (SPR)-based BIACORE methods, and others (Proc. Natl. Acad. Sci. USA (2006) 103(11), 4005-4010), in addition to the above-described FACS and ELISA formats.

In FcγRI (CD64) including FcγRIa, FcγRIb, and FcγRIc, and FcγRIII (CD16) including isoforms FcγRIIIa (including allotypes V158 and F158) and FcγRIIIb (including allotypes FcγRIIIb-NA1 and FcγRIIIb-NA2), α chain that binds to the Fc region of IgG is associated with common γ chain having ITAM responsible for transduction of intracellular activation signal. Meanwhile, the cytoplasmic domain of FcγRII (CD32) including isoforms FcγRIIa (including allotypes H131 and R131) and FcγRIIc contains ITAM These receptors are expressed on many immune cells such as macrophages, mast cells, and antigen-presenting cells. The activation signal transduced upon binding of these receptors to the Fc region of IgG results in enhancement of the phagocytic activity of macrophages, inflammatory cytokine production, mast cell degranulation, and the enhanced function of antigen-presenting cells. Fcγ receptors having the ability to transduce the activation signal as described above are herein referred to as activating Fcγ receptors.

Meanwhile, the intracytoplasmic domain of FcγRIIb (including FcγRIIb-1 and FcγRIIb-2) contains ITIM responsible for transduction of inhibitory signals. The crosslinking between FcγRIIb and B cell receptor (BCR) on B cells suppresses the activation signal from BCR, which results in suppression of antibody production via BCR. The crosslinking of FcγRIII and FcγRIIb on macrophages suppresses the phagocytic activity and inflammatory cytokine production. Fcγ receptors having the ability to transduce the inhibitory signal as described above are herein referred to as inhibitory Fcγ receptor.

FcγR-binding activity of Fc region

As mentioned above, Fc regions having an Fcγ receptor-binding activity are examples of Fc regions comprised in the antigen-binding molecules of the present disclosure. A non-limiting embodiment of such an Fc region includes the Fc region of human IgG1 (SEQ ID NO: 5), IgG2 (SEQ ID NO: 6), IgG3 (SEQ ID NO: 7), or IgG4 (SEQ ID NO: 8). Whether an Fcγ receptor has binding activity to the Fc region of a monoclonal IgG1, IgG2, IgG3, or IgG4 antibody can be assessed by ALPHA screen (Amplified Luminescent Proximity Homogeneous Assay), surface plasmon resonance (SPR)-based BIACORE method, and others (Proc. Natl. Acad. Sci. U.S.A. (2006) 103(11), 4005-4010), in addition to the above-described FACS and ELISA formats.

ALPHA screen is performed by the ALPHA technology based on the principle described below using two types of beads: donor and acceptor beads. A luminescent signal is detected only when molecules linked to the donor beads interact biologically with molecules linked to the acceptor beads and when the two beads are located in close proximity. Excited by laser beam, the photosensitizer in a donor bead converts oxygen around the bead into excited singlet oxygen. When the singlet oxygen diffuses around the donor beads and reaches the acceptor beads located in close proximity, a chemiluminescent reaction within the acceptor beads is induced. This reaction ultimately results in light emission. If

molecules linked to the donor beads do not interact with molecules linked to the acceptor beads, the singlet oxygen produced by donor beads do not reach the acceptor beads and chemiluminescent reaction does not occur.

For example, a biotin-labeled antigen-binding molecule comprising Fc region is immobilized to the donor beads and glutathione S-transferase (GST)-tagged Fcγ receptor is immobilized to the acceptor beads. In the absence of an antigen-binding molecule comprising a competitive Fc region variant, Fcγ receptor interacts with an antigen-binding molecule comprising a native Fc region, inducing a signal of 520 to 620 nm as a result. The antigen-binding molecule having a non-tagged Fc region variant competes with the antigen-binding molecule comprising a native Fc region for the interaction with Fcγ receptor. The relative binding affinity can be determined by quantifying the reduction of fluorescence as a result of competition. Methods for biotinylating the antigen-binding molecules such as antibodies using Sulfo-NHS-biotin or the like are known. Appropriate methods for adding the GST tag to an Fcγ receptor include methods that involve fusing polypeptides encoding Fcγ and GST in-frame, expressing the fused gene using cells introduced with a vector to which the gene is operably linked, and then purifying using a glutathione column. The induced signal can be preferably analyzed, for example, by fitting to a one-site competition model based on nonlinear regression analysis using software such as GRAPHPAD PRISM (GraphPad; San Diego).

One of the substances for observing their interaction is immobilized as a ligand onto the gold thin layer of a sensor chip. When light is shed on the rear surface of the sensor chip so that total reflection occurs at the interface between the gold thin layer and glass, the intensity of reflected light is partially reduced at a certain site (SPR signal). The other substance for observing their interaction is injected as an analyte onto the surface of the sensor chip. The mass of immobilized ligand molecule increases when the analyte binds to the ligand. This alters the refraction index of solvent on the surface of the sensor chip. The change in refraction index causes a positional shift of SPR signal (conversely, the dissociation shifts the signal back to the original position). In the Biacore system, the amount of shift described above (*i.e.,* the change of mass on the sensor chip surface) is plotted on the vertical axis, and thus the change of mass over time is shown as measured data (sensorgram). Kinetic parameters (association rate constant (ka) and dissociation rate constant (kd)) are determined from the curve of sensorgram, and affinity (KD) is determined from the ratio between these constants. Inhibition assay is preferably used in the BIACORE methods. Examples of such inhibition assay are described in Proc. Natl. Acad. Sci. U.S.A. (2006) 103(11), 4005-4010.

Fc regions with altered Fcγ receptor (FcγR) binding

In addition to the Fc region of human IgG1 (SEQ ID NO: 5), IgG2 (SEQ ID NO: 6), IgG3 (SEQ ID NO: 7), or IgG4 (SEQ ID NO: 8), an Fc region with altered FcγR binding, which has a higher Fcγ receptor-binding activity than an Fc region of a native human IgG may be appropriately used as an Fc region included in the present disclosure. Herein, "Fc region of a native human IgG" refers to an Fc region in which the sugar chain bonded to position 297 (EU numbering) of the Fc region of human IgG1, IgG2, IgG3, or IgG4 shown in SEQ ID NOs: 5, 6, 7, or 8 is a fucose-containing sugar chain. Such Fc regions with altered FcγR binding may be produced by altering amino acids of the Fc region of a native human IgG. Whether the FcγR-binding activity of an Fc region with altered FcγR binding is higher than that of an Fc region of a native human IgG can be determined appropriately using methods described in the abovementioned section on binding activity.

In the present disclosure, "alteration of amino acids" or "amino acid alteration" of an Fc region includes alteration into an amino acid sequence which is different from that of the starting Fc region. The starting Fc region may be any Fc region, as long as a variant modified from the starting Fc region can bind to human Fcγ receptor in a neutral pH range. Furthermore, an Fc region altered from a starting Fc region which had been already altered can also be used preferably as an Fc region of the present disclosure. The "starting Fc region" can refer to the polypeptide itself, a composition comprising the starting Fc region, or an amino acid sequence encoding the starting Fc region. Starting Fc regions can comprise known Fc regions produced *via* recombination described briefly in the section "Antibodies". The origin of starting Fc regions is not limited, and they may be obtained from human or any nonhuman organisms. Such organisms preferably include mice, rats, guinea pigs, hamsters, gerbils, cats, rabbits, dogs, goats, sheep, bovines, horses, camels and organisms selected from nonhuman primates. In another embodiment, starting Fc regions can also be obtained from cynomolgus monkeys, marmosets, rhesus monkeys, chimpanzees, or humans. Starting Fc regions can be obtained preferably from human IgG1; however, they are not limited to any particular IgG class. This means that an Fc region of human IgG1, IgG2, IgG3, or IgG4 can be used appropriately as a starting Fc region, and herein also means that an Fc region of an arbitrary IgG class or subclass derived from any organisms described above can be preferably used as a starting Fc region. Examples of native IgG variants or altered forms are described in published documents (Curr. Opin. Biotechnol. (2009) 20 (6): 685-91; Curr. Opin. Immunol. (2008) 20 (4), 460-470; Protein Eng. Des. Sel. (2010) 23 (4): 195-202; International Publication Nos. WO 2009/086320, WO 2008/092117, WO 2007/041635, and WO 2006/105338); however, they are not limited to the examples.

Examples of alterations include those with one or more mutations, for example, mutations by substitution of different amino acid residues for amino acids of starting Fc regions, by insertion of one or more amino acid residues into

starting Fc regions, or by deletion of one or more amino acids from starting Fc region. Preferably, the amino acid sequences of altered Fc regions comprise at least a part of the amino acid sequence of a non-native Fc region. Such variants necessarily have sequence identity or similarity less than 100% to their starting Fc region. In a preferred embodiment, the variants have amino acid sequence identity or similarity about 75% to less than 100%, more preferably about 80% to less than 100%, even more preferably about 85% to less than 100%, still more preferably about 90% to less than 100%, and yet more preferably about 95% to less than 100% to the amino acid sequence of their starting Fc region. In a non-limiting embodiment of the present disclosure, at least one amino acid is different between an FcyR-binding altered Fc region of the present disclosure and its starting Fc region. Amino acid difference between an FcyR-binding altered Fc region of the present disclosure and its starting Fc region can also be preferably specified based on the specific amino acid differences at the above-described specific amino acid positions by EU numbering. Examples of methods of preparing such variants are shown in the section "Alteration of amino acids".

Included in the antigen-binding molecules of the present disclosure, an Fc region with altered FcγR binding, which has a higher Fcγ receptor-binding activity than that of an Fc region of a native human IgG, (an FcγR binding-altered Fc region) may be obtained by any method. Specifically, the Fc region with altered FcγR binding may be obtained by altering amino acids of an IgG-type human immunoglobulin used as a starting Fc region. Preferred Fc regions of the IgG-type immunoglobulins for alteration include, for example, those of human IgGs shown in SEQ ID NOs: 5, 6, 7, or 8 (IgG1, IgG2, IgG3, or IgG4, respectively, and variants thereof).

Amino acids of any positions may be altered into other amino acids, as long as the binding activity toward the Fcγ receptor is higher than that of the Fc region of a native human IgG. When the antigen-binding molecule contains a human IgG1 Fc region as the human Fc region, it preferably contains an alteration that yields the effect of a higher Fcγ receptor-binding activity than that of the Fc region of a native human IgG, in which the sugar chain bound at position 297 (EU numbering) is a fucose-containing sugar chain. Such amino acid alterations have been reported, for example, in international publications such as WO2007/024249, WO2007/021841, WO2006/031370, WO2000/042072, WO2004/029207, WO2004/099249, WO2006/105338, WO2007/041635, WO2008/092117, WO2005/070963, WO2006/020114, WO2006/116260, and WO2006/023403.

For the pH conditions to measure the binding activity of the Fcγ receptor binding domain and the Fcγ receptor contained in the antigen-binding molecule of the present disclosure, conditions in an acidic pH range or in a neutral pH range may be suitably used. The acidic pH range or neutral pH range, as a condition to measure the binding activity of the Fcγ receptor binding domain and the Fcγ receptor contained in the antigen-binding molecule of the present disclosure, generally indicates pH 5.8 to pH 8.0. Preferably, it is a range indicated with arbitrary pH values between pH 6.0 and pH 7.4; and preferably, it is selected from pH 6.0, pH 6.1, pH 6.2, pH 6.3, pH 6.4, pH 6.5, pH 6.6, pH 6.7, pH 6.8, pH 6.9, pH 7.0, pH 7.1, pH 7.2, pH 7.3, and pH 7.4; and particularly preferably, it is pH 6.15 to 7.4, which is close to the pH of cancer tissues (Vaupel et al., Cancer Res. (1989) 49, 6449-6665). With regard to the temperature used as a measurement condition, the binding affinity between an Fcγ receptor binding domain and a human Fcγ receptor can be evaluated at any temperature between 10°C and 50°C. Preferably, a temperature between 15°C and 40°C is used to determine the binding affinity between a human Fcγ receptor binding domain and Fcγ receptor. More preferably, any temperature between 20°C and 35°C, such as any single temperature from 20°C, 21°C, 22°C, 23°C, 24°C, 25°C, 26°C, 27°C, 28°C, 29°C, 30°C, 31°C, 32°C, 33°C, 34°C, and 35°C, can be similarly used to determine the binding affinity between an Fcγ receptor binding domain and an Fcγ receptor. A temperature of 25°C is a non-limiting example in an embodiment of the present disclosure.

Herein, "Fc region with altered FcγR binding has a higher Fcγ receptor-binding activity than the native Fc region" means that the human Fcγ receptor-binding activity of the Fc region with altered FcγR binding toward any of the human Fcγ receptors of FcγRI, FcγRIIa, FcγRIIb, FcγRIIIa, and/or FcγRIIIb is higher than the binding activity of the native Fc region toward these human Fcγ receptors. For example, it means that based on an above-described analytical method, in comparison to the binding activity of an antigen-binding molecule containing a native human IgG Fc region as a control, the binding activity of the antigen-binding molecule comprising an Fc region with altered FcγR binding is 105% or more, preferably 110% or more, 115% or more, 120% or more, 125% or more, particularly preferably 130% or more, 135% or more, 140% or more, 145% or more, 150% or more, 155% or more, 160% or more, 165% or more, 170% or more, 175% or more, 180% or more, 185% or more, 190% or more, 195% or more, 2-fold or more, 2.5-fold or more, 3-fold or more, 3.5-fold or more, 4-fold or more, 4.5-fold or more, 5-fold or more, 7.5-fold or more, 10-fold or more, 20-fold or more, 30-fold or more, 40-fold or more, 50-fold or more, 60-fold or more, 70-fold or more, 80-fold or more, 90-fold or more, or 100-fold or more. The starting Fc region may be used as a native Fc region, and native Fc regions of antibodies of the same subclass may also be used.

In the present disclosure, an Fc region of a native human IgG in which the sugar chain bonded to the amino acid at position 297 (EU numbering) is a fucose-containing sugar chain, is suitably used as a native Fc region of human IgG to be used as a control. Whether or not the sugar chain bonded to the amino acid at position 297 (EU numbering) is a fucose-containing sugar chain can be determined using a known technique (Non-fucosylated therapeutic antibodies as next-generation therapeutic antibodies. Satoh M, Iida S, Shitara K., Expert Opin. Biol. Ther. (2006) 6 (11), 1161-1173).

For example, it is possible to determine whether or not the sugar chain bonded to the native human IgG Fc region is a fucose-containing sugar chain by a method such as the one below. Sugar chain is dissociated from a native human IgG to be tested, by reacting the test native human IgG with *N*-Glycosidase F (Roche diagnostics) (Weitzhandler et al. (J. Pharma. Sciences (1994) 83, 12, 1670-1675)). Next, a dried concentrate of a reaction solution from which protein has been removed by reaction with ethanol (Schenk et al. (J. Clin. Investigation (2001) 108 (11) 1687-1695)) is fluorescently labeled with 2-aminopyridine (Bigge et al. (Anal. Biochem. (1995) 230 (2) 229-238)). Reagents are removed by solid extraction using a cellulose cartridge, and the fluorescently labeled 2-AB-modified sugar chain is analyzed by normal-phase chromatography. It is possible to determine whether or not the sugar chain bonded to the native Fc region of a human IgG is a fucose-containing sugar chain by observing the detected chromatogram peaks.

As an antigen-binding molecule containing a native Fc region of an antibody of the same subclass, which is to be used as a control, an antigen-binding molecule having an Fc region of a monoclonal IgG antibody may be suitably used. The structures of the Fc regions are described in SEQ ID NO: 5 (A is added to the N terminus of Database Accession No. AAC82527.1), SEQ ID NO: 6 (A is added to the N terminus of Database Accession No. AAB59393.1), SEQ ID NO: 7 (Database Accession No. CAA27268.1), and SEQ ID NO: 8 (A is added to the N terminus of Database Accession No. AAB59394.1). Further, when an antigen-binding molecule containing an Fc region of a particular antibody isotype is used as the test substance, the effect of the antigen-binding molecule containing the test Fc region on Fcγ receptor-binding activity is tested by using as a control an antigen-binding molecule having an Fc region of a monoclonal IgG antibody of that particular isotype. In this way, antigen-binding molecules containing an Fc region of which Fcγ receptor-binding activity is demonstrated to be high are suitably selected.

Fc regions having a selective binding activity toward an Fcγ receptor

Examples of Fcγ receptor binding domains suitable for use in the present disclosure include Fcγ receptor binding domains having a higher binding activity to a particular Fcγ receptor than to other Fcγ receptors (Fcγ receptor binding domains having a selective binding activity to an Fcγ receptor). When an antibody is used as the antigen-binding molecule (when an Fc region is used as the Fcγ receptor binding domain), a single antibody molecule can only bind to a single Fcγ receptor molecule. Therefore, a single antigen-binding molecule cannot bind to other activating FcγRs in an inhibitory Fcγ receptor-bound state, and cannot bind to other activating Fcγ receptors or inhibitory Fcγ receptors in an activating Fcγ receptor-bound state.

Fc regions with a higher binding activity toward an activating Fcγ receptor than the binding activity toward an inhibitory Fcγ receptor

As described above, preferable activating Fcγ receptors include FcγRI (CD64) including FcγRIa, FcγRIb, and FcγRIc; FcγRIIa; and FcγRIII (CD16) including FcγRIIIa (including allotypes V158 and F158) and FcγRIIIb (including allotypes FcγRIIIb-NA1 and FcγRIIIb-NA2). Meanwhile, preferred examples of inhibitory Fcγ receptors include FcγRIIb (including FcγRIIb-1 and FcγRIIb-2).

Herein, an example of a case where the binding activity toward a certain Fcγ receptor is higher than the binding activity toward another Fcγ receptor is the case where the binding activity toward an activating Fcγ receptor is higher than the binding activity toward an inhibitory Fcγ receptor. In this case, the binding activity of the Fc region toward any of the human Fcγ receptors of FcγRIa, FcγRIIa, FcγRIIIa, and/or FcγRIIIb is said to be higher than the binding activity toward FcγRIIb. For example, this means that, based on an above-described analytical method, the binding activity of an antigen-binding molecule containing the Fc region toward any of the human Fcγ receptors, FcγRIa, FcγRIIa, FcγRIIIa, and/or FcγRIIIb, is 105% or more, preferably 110% or more, 120% or more, 130% or more, 140% or more, particularly preferably 150% or more, 160% or more, 170% or more, 180% or more, 190% or more, 200% or more, 250% or more, 300% or more, 350% or more, 400% or more, 450% or more, 500% or more, 750% or more, 10-fold or more, 20-fold or more, 30-fold or more, 40-fold or more, 50-fold or more, 60-fold, 70-fold, 80-fold, 90-fold, or 100-fold or more as compared with the binding activity toward FcγRIIb. The Fc region with a higher binding activity toward activating Fcγ receptors than to inhibitory Fcγ receptors may be favorably included in antigen-binding molecules of the present disclosure whose antigen-binding domain binds to a membrane-type molecule. IgG1 antibodies containing such Fc regions are known to enhance the ADCC activity mentioned below. Therefore, antigen-binding molecules containing the Fc-region are also useful as antigen-binding molecules to be included in the pharmaceutical compositions of the present disclosure.

In a non-limiting embodiment of the present disclosure, examples of the Fc region with a higher binding activity toward activating Fcγ receptors than to inhibitory Fcγ receptors (or having a selective binding activity toward inhibitory Fcγ receptors) preferably include Fc regions in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298,

299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 indicated by EU numbering mentioned above, have been altered to amino acids different from those of the native Fc region.

Fc regions whose binding activity toward an inhibitory Fcγ receptor is higher than the binding activity toward an activating Fcγ receptor

Herein, an example of a case where the binding activity toward a certain Fcγ receptor is higher than the binding activity toward another Fcγ receptor is the case where the binding activity toward an inhibitory Fcγ receptor is higher than the binding activity toward an activating Fcγ receptor. In this case, the binding activity of the Fc region toward FcγRIIb is said to be higher than the binding activity toward any of the human Fcγ receptors of FcγRIa, FcγRIIa, FcγRIIIa, and/or FcγRIIIb. For example, this means that, based on an above-described analytical method, the binding activity of an antigen-binding molecule containing the Fc region toward FcγRIIb is 105% or more, preferably 110% or more, 120% or more, 130% or more, 140% or more, particularly preferably 150% or more, 160% or more, 170% or more, 180% or more, 190% or more, 200% or more, 250% or more, 300% or more, 350% or more, 400% or more, 450% or more, 500% or more, 750% or more, 10-fold or more, 20-fold or more, 30-fold or more, 40-fold or more, 50-fold, 60-fold, 70-fold, 80-fold, 90-fold, or 100-fold or more as compared with the binding activity toward any of the human Fcγ receptors of FcγRIa, FcγRIIa, FcγRIIIa, and/or FcγRIIIb. The Fc region with a higher binding activity toward inhibitory Fcγ receptors than to activating Fcγ receptors may be favorably included in antigen-binding molecules of the present disclosure whose antigen-binding domain binds to a soluble molecule.

In a non-limiting embodiment of the present disclosure, examples of the Fc region with a higher binding activity toward inhibitory Fcγ receptors than to activating Fcγ receptors (or having a selective binding activity toward inhibitory Fcγ receptors) preferably include Fc regions in which, of the amino acids of the above Fc region, the amino acids at 238 and 328 indicated by EU numbering are altered to amino acids different from those of the native Fc region.

In a non-limiting embodiment of the present disclosure, examples of the Fc region with a higher binding activity toward inhibitory Fcγ receptors than to activating Fcγ receptors (or having a selective binding activity toward inhibitory Fcγ receptors) preferably include Fc regions altered at any one or more of the amino acids in the above Fc region as indicated by EU numbering: the amino acid at position 238 (indicated by EU numbering) is altered into Asp; and the amino acid at position 328 (indicated by EU numbering) is altered into Glu. Furthermore, as the Fc regions having a selective binding activity toward inhibitory Fcγ receptors, the Fc regions or alterations described in US 2009/0136485 can be suitably selected.

In another non-limiting embodiment of the present disclosure, preferred examples include Fc regions altered at any one or more of the amino acids in the above Fc region as indicated by EU numbering: the amino acid at position 238 (indicated by EU numbering) to Asp; and the amino acid at position 328 (indicated by EU numbering) to Glu.

In still another non-limiting embodiment of the present disclosure, preferred examples include Fc regions that have one or more of the alterations exemplified in PCT/JP2012/054624: substitution of Pro at position 238 (indicated by EU numbering) with Asp; alteration of the amino acid at position 237 (indicated by EU numbering) to Trp; alteration of the amino acid at position 237 (indicated by EU numbering) to Phe; alteration of the amino acid at position 267 (indicated by EU numbering) to Val; alteration of the amino acid at position 267 (indicated by EU numbering) to Gln; alteration of the amino acid at position 268 (indicated by EU numbering) to Asn; alteration of the amino acid at position 271 (indicated by EU numbering) to Gly; alteration of the amino acid at position 326 (indicated by EU numbering) to Leu; alteration of the amino acid at position 326 (indicated by EU numbering) to Gln; alteration of the amino acid at position 326 (indicated by EU numbering) to Glu; alteration of the amino acid at position 326 (indicated by EU numbering) to Met; alteration of the amino acid at position 239 (indicated by EU numbering) to Asp; alteration of the amino acid at position 267 (indicated by EU numbering) to Ala; alteration of the amino acid at position 234 (indicated by EU numbering) to Trp; alteration of the amino acid at position 234 (indicated by EU numbering) to Tyr; alteration of the amino acid at position 237 (indicated by EU numbering) to Ala; alteration of the amino acid at position 237 (indicated by EU numbering) to Asp; alteration of the amino acid at position 237 (indicated by EU numbering) to Glu; alteration of the amino acid at position 237 (indicated by EU numbering) to Leu; alteration of the amino acid at position 237 (indicated by EU numbering) to Met; alteration of the amino acid at position 237 (indicated by EU numbering) to Tyr; alteration of the amino acid at position 330 (indicated by EU numbering) to Lys; alteration of the amino acid at position 330 (indicated by EU numbering) to Arg, alteration of the amino acid at position 233 (indicated by EU numbering) to Asp, alteration of the amino acid at position 268 (indicated by EU numbering) to Asp, alteration of the amino acid at position 268 (indicated by EU numbering) to Glu, alteration of the amino acid at position 326 (indicated by EU numbering) to Asp, alteration of the amino acid at position 326 (indicated by EU numbering) to Ser, alteration of the amino acid at position 326 (indicated by EU numbering) to Thr, alteration of the amino acid at position 323 (indicated by EU numbering) to Ile, alteration of the amino acid at position 323 (indicated by EU numbering) to Leu, alteration of the amino acid at position 323 (indicated by EU numbering) to Met, alteration of the amino acid at position 296 (indicated by EU numbering) to Asp, alteration of the amino acid at

position 326 (indicated by EU numbering) to Ala, alteration of the amino acid at position 326 (indicated by EU numbering) to Asn, and alteration of the amino acid at position 330 (indicated by EU numbering) to Met.

Fc regions with modified sugar chains

Fc regions contained in the antigen-binding molecules provided by the present disclosure may include Fc regions that have been modified so that the composition of the sugar-chain-attached Fc regions has a high percentage of fucose-deficient sugar-chain-attached Fc regions, or a high percentage of bisecting *N*-acetylglucosamine-added Fc regions. Removal of fucose residue from *N*-acetylglucosamine at the reducing end of *N*-glycoside linkage complex sugar chains bonded to the antibody Fc region is known to enhance the affinity to FcγRIIIa (NPL 6). It is known that for IgG1 antibodies containing such Fc regions, the ADCC activity mentioned below is enhanced; therefore, antigen-binding molecules containing such Fc regions are also useful as antigen-binding molecules to be contained in pharmaceutical compositions of the present disclosure. Examples of antibodies with fucose residue removed from *N*-acetylglucosamine at the reducing end of *N*-glycoside linkage complex sugar chains bonded to the antibody Fc regions are antibodies such as:

antibodies modified by glycosylation (for example, WO 1999/054342); and
antibodies deficient in fucose attached to sugar chains (for example, WO 2000/061739, WO 2002/031140, and WO 2006/067913).

More specifically, to produce antibodies deficient in fucose attached to sugar chains (for example, WO 2000/061739, WO 2002/031140, and WO 2006/067913) as another non-limiting embodiment of antibodies with fucose residue removed from *N*-acetylglucosamine at the reducing end of *N*-glycoside linkage complex sugar chains bonded to the antibody Fc regions, host cells having a low ability to add fucose to sugar chains are produced by altering the activity of forming the sugar chain structure of the polypeptide to be glycosylated. Antibodies that lack fucose in their sugar chains can be collected from culture of the host cells by expressing a desired antibody gene in the host cells. Non-limiting suitable examples of the activity to form the sugar chain structure of a polypeptide include the activity of a transporter or an enzyme selected from the group consisting of fucosyltransferase (EC 2.4.1.152), fucose transporter (SLC35C1), GMD (GDP-mannose-4,6-dehydratase) (EC 4.2.1.47), Fx (GDP-keto-6-deoxymannose-3,5-epimerase, 4-reductase) (EC 1.1.1.271), and GFPP (GDP-β-L-fucose pyrophosphorylase (EC 2.7.7.30). As long as these enzymes or transporters can exhibit their activities, their structures are not necessarily specified. Herein, proteins that can exhibit these activities are referred to as "functional proteins". In a non-limiting embodiment, methods for altering these activities include deletion of these activities. To produce host cells deficient in these activities, known methods such as a method for destroying the genes of these functional proteins to make them unable to function may be appropriately employed (for example, WO2000/061739, WO2002/031140, and WO2006/067913). Host cells deficient in such activities can be produced, for example, by a method that destroys the genes of these functional proteins endogenous to CHO cells, BHK cells, NS0 cells, SP2/0 cells, YO myeloma cells, P3X63 mouse myeloma cells, PER cells, PER.C6 cells, HEK293 cells, hybridoma cells, or such, so that the genes are unable to function.

Antibodies that have a sugar chain containing bisecting GlcNAc (WO2002/079255, etc.) are known. In a non-limiting embodiment, host cells for expressing a gene that encodes a functional protein having GnTIII (β-1,4-mannosyl-glycoprotein 4-β-N-acetylglucosaminyltransferase) (EC 2.4.1.144) activity or GalT (β-1,4-galactosyltransferase) (EC 2.4.1.38) activity are produced to prepare antibodies that have bisecting GlcNAc-containing sugar chains. In another suitable non-limiting embodiment, host cells that co-express, in addition to the aforementioned functional proteins, a gene encoding a functional protein having human ManII (mannosidase II) (3.2.1.114) activity, a gene encoding a functional protein having GnTI (β-1,2-acetylglucosaminyltransferase I) (EC 2.4.1.94) activity, a gene encoding a functional protein having GnTII (β-1,2-acetylglucosaminyltransferase II) (EC 2.4.1.143) activity, a gene encoding a functional protein having ManI (mannosidase) (EC 3.2.1.113) activity, and α-1,6-fucosyl transferase (EC 2.4.1.68), are produced (WO2004/065540).

Antibodies with fucose residue removed from *N*-acetylglucosamine at the reducing end of *N*-glycoside linkage complex sugar chains bonded to the antibody Fc regions and antibodies having sugar chains containing bisecting GlcNAc can be produced, respectively, by transfecting an expression vector containing the antibody gene into host cells with a low ability to add fucose to sugar chains, and into host cells having the activity to form bisecting GlcNAc structure-containing sugar chains. Methods for producing these antibodies can be applied to methods for producing antigen-binding molecules containing altered Fc regions that have been modified so that the composition of the sugar-chain-attached Fc regions of the present disclosure has a high percentage of fucose-deficient sugar chain-attached Fc regions or a high percentage of bisecting *N*-acetylglucosamine-added Fc regions. The composition of the sugar-chain-attached Fc regions contained in the antigen-binding molecules of the present disclosure produced by such production methods can be assessed by the method described in "Fc regions with altered Fcγ receptor (FcγR) binding" above.

Heterocomplex comprising the four molecules including two molecules of FcRn and one molecule of activating Fcγ receptor

Crystallographic studies on FcRn with IgG antibodies demonstrated that an FcRn-IgG complex is composed of one molecule of IgG for two molecules of FcRn, and the two molecules are thought to bind around the interface of the CH2 and CH3 domains located on both sides of the IgG Fc region (Burmeister et al. (Nature (1994) 372, 336-343)). Meanwhile, as demonstrated in Example 3 of PCT/JP2012/058603, the antibody Fc region was demonstrated to be able to form a complex comprising the four molecules including two molecules of FcRn and one molecule of activating Fcγ receptor (PCT/JP2012/058603). This heterocomplex formation is a phenomenon which was revealed as a result of analyzing the properties of antigen-binding molecules containing an Fc region having an FcRn-binding activity under a neutral pH range condition.

While the present disclosure is not bound to a particular principle, it can be considered that antigen-binding molecules administered *in vivo* produce the effects described below on the *in vivo* pharmacokinetics (plasma retention) of the antigen-binding molecules and an immune response (immunogenicity) to the administered antigen-binding molecules, as a result of the formation of heterocomplexes containing the four molecules including the Fc region contained in the antigen-binding molecules, two molecules of FcRn, and one molecule of activating Fcγ receptor. In addition to the various types of activating Fcγ receptors, FcRn is expressed on immune cells. It is suggested that the formation of such tetrameric complexes on immune cells by antigen-binding molecules promotes incorporation of antigen-binding molecules into immune cells by increasing affinity toward immune cells and by causing association of intracellular domains to enhance the internalization signal. The same also applies to antigen-presenting cells and the possibility that antigen binding-molecules are likely to be incorporated into antigen-presenting cells by formation of tetrameric complexes on the cell membrane of antigen-presenting cells. In general, antigen-binding molecules incorporated into antigen-presenting cells are degraded in the lysosomes of the antigen-presenting cells and are presented to T cells. As a result, plasma retention of antigen-binding molecules may be worsened because incorporation of antigen-binding molecules into antigen-presenting cells is promoted by the formation of the above-described tetrameric complexes on the cell membrane of the antigen-presenting cells. Similarly, an immune response may be induced (aggravated).

For this reason, it is conceivable that when an antigen-binding molecule having lowered ability to form such tetrameric complexes is administered *in vivo,* plasma retention of the antigen-binding molecules would improve, and induction of *in vivo* immune response would be suppressed. Preferred embodiments of such antigen-binding molecules which inhibit the formation of these complexes on immune cells including antigen-presenting cells are, for example, the three embodiments described below.

Antigen-binding molecules which inhibit the formation of heterocomplexes

(Embodiment 1) An antigen-binding molecule containing an Fc region having FcRn-binding activity under a neutral pH range condition and whose binding activity toward activating FcγR is lower than the binding activity of a native Fc region toward activating FcγR

The antigen-binding molecule of Embodiment 1 forms a trimeric complex by binding to two molecules of FcRn; however, it does not form any complex containing activating FcγR. An Fc region whose binding activity toward activating FcγR is lower than the binding activity of a native Fc region toward activating FcγR can be prepared by altering the amino acids of the native Fc region as described above. Whether the binding activity toward activating FcγR of the altered Fc region is lower than the binding activity toward activating FcγR of the native Fc region can be appropriately tested using the methods described in the section "Binding Activity" above.

Preferred activating Fcγ receptors include FcγRI (CD64) which includes FcγRIa, FcγRIb, and FcγRIc; FcγRIIa (including allotypes R131 and H131); and FcγRIII (CD16) which includes isoforms FcγRIIIa (including allotypes V158 and F158) and FcγRIIIb (including allotypes FcγRIIIb-NA1 and FcγRIIIb-NA2).

Herein, "a binding activity of the Fc region variant toward an activating Fcγ receptor is lower than the binding activity of the native Fc region toward an activating Fcγ receptor" means that the binding activity of the Fc region variant toward any of the human Fcγ receptors (FcγRI, FcγRIIa, FcγRIIIa, and/or FcγRIIIb) is lower than the binding activity of the native Fc region toward these human Fcγ receptors. For example, it means that based on an above-described analytical method, the binding activity of the antigen-binding molecule containing an Fc region variant as compared to the binding activity of an antigen-binding molecule containing a native Fc region as a control is 95% or less, preferably 90% or less, 85% or less, 80% or less, 75% or less, and particularly preferably 70% or less, 65% or less, 60% or less, 55% or less, 50% or less, 45% or less, 40% or less, 35% or less, 30% or less, 25% or less, 20% or less, 15% or less, 10% or less, 9% or less, 8% or less, 7% or less, 6% or less, 5% or less, 4% or less, 3% or less, 2% or less, or 1% or less. As a native Fc region, a starting Fc region may be used, and Fc regions of wild-type antibodies of different isotypes may also be used.

Meanwhile, the binding activity of the native form toward an activating FcγR is preferably a binding activity toward the Fcγ receptor for human IgG1. Other than performing the above-described alterations, binding activity toward the Fcγ receptor can be lowered by changing the isotype to human IgG2, human IgG3, or human IgG4. Alternatively, besides by performing the above-described alterations, the binding activity toward an Fcγ receptor can also be lowered by expressing the antigen-binding molecule containing an Fc region having a binding activity toward the Fcγ receptor in hosts that do not add sugar chains such as *Escherichia coli*.

For the antigen-binding molecule containing a control Fc region, an antigen-binding molecule having an Fc region of a monoclonal IgG antibody may be appropriately used. The structures of such Fc regions are shown in SEQ ID NO: 5 (A is added to the N terminus of RefSeq Accession No. AAC82527.1), SEQ ID NO: 6 (A is added to the N terminus of RefSeq Accession No. AAB59393.1), SEQ ID NO: 7 (RefSeq Accession No. CAA27268.1), and SEQ ID NO: 8 (A is added to the N terminus of RefSeq Accession No. AAB59394.1). Further, when an antigen-binding molecule containing an Fc region of a particular antibody isotype is used as the test substance, effect on the binding activity of the antigen-binding molecule containing the Fc region toward an Fcγ receptor is tested by using the antigen-binding molecule having an Fc region of a monoclonal IgG antibody of a particular isotype as a control. In this way, antigen-binding molecules containing an Fc region whose binding activity toward the Fcγ receptor was demonstrated to be high are suitably selected.

In a non-limiting embodiment of the present disclosure, preferred examples of Fc regions whose binding activity toward an activating FcγR is lower than the binding activity of the native Fc region toward an activating FcγR include Fc regions with alteration of one or more amino acids at any of positions 234, 235, 236, 237, 238, 239, 270, 297, 298, 325, 328, and 329 as indicated by EU numbering in the amino acids of an above-described Fc region to be different from those of the native Fc region. The alterations in the Fc region are not limited to the above example, and they may be, for example, modifications such as deglycosylation (N297A and N297Q), IgG1-L234A/L235A, IgG1-A325A/A330S/P331S, IgG1-C226S/C229S, IgG1-C226S/C229S/E233P/L234V/L235A, IgG1-L234F/L235E/P331S, IgG1-S267E/L328F, IgG2-V234A/G237A, IgG2-H268Q/V309L/A330S/A331S, IgG4-L235A/G237A/E318A, and IgG4-L236E described in Cur. Opin. in Biotech. (2009) 20 (6), 685-691; alterations such as G236R/L328R, L235G/G236R, N325A/L328R, and N325L/L328R described in WO 2008/092117; amino acid insertions at positions 233, 234, 235, and 237 according to EU numbering; and alterations at the positions described in WO 2000/042072.

In a non-limiting embodiment of the present disclosure, examples of a preferred Fc region include Fc regions having one or more of the following alterations as indicated by EU numbering in an aforementioned Fc region:

Ala, Arg, Asn, Asp, Gln, Glu, Gly, His, Lys, Met, Phe, Pro, Ser, Thr, or Trp for the amino acid at position 234;
Ala, Asn, Asp, Gln, Glu, Gly, His, Ile, Lys, Met, Pro, Ser, Thr, Val, or Arg for the amino acid at position 235;
Arg, Asn, Gln, His, Leu, Lys, Met, Phe, Pro, or Tyr for the amino acid at position 236;
Ala, Asn, Asp, Gln, Glu, His, Ile, Leu, Lys, Met, Pro, Ser, Thr, Val, Tyr, or Arg for the amino acid at position 237;
Ala, Asn, Gln, Glu, Gly, His, Ile, Lys, Thr, Trp, or Arg for the amino acid at position 238;
Gln, His, Lys, Phe, Pro, Trp, Tyr, or Arg for the amino acid at position 239;
Ala, Arg, Asn, Gln, Gly, His, Ile, Leu, Lys, Met, Phe, Ser, Thr, Trp, Tyr, or Val for the amino acid at position 265;
Ala, Arg, Asn, Asp, Gln, Glu, Gly, His, Lys, Phe, Pro, Ser, Thr, Trp, or Tyr for the amino acid at position 266;
Arg, His, Lys, Phe, Pro, Trp, or Tyr for the amino acid at position 267;
Ala, Arg, Asn, Gln, Gly, His, Ile, Leu, Lys, Met, Phe, Pro, Ser, Thr, Trp, Tyr, or Val for the amino acid at position 269;
Ala, Arg, Asn, Gln, Gly, His, Ile, Leu, Lys, Met, Phe, Pro, Ser, Thr, Trp, Tyr, or Val for the amino acid at position 270;
Arg, His, Phe, Ser, Thr, Trp, or Tyr for the amino acid at position 271;
Arg, Asn, Asp, Gly, His, Phe, Ser, Trp, or Tyr for the amino acid at position 295;
Arg, Gly, Lys, or Pro for the amino acid at position 296;
Ala for the amino acid at position 297;
Arg, Gly, Lys, Pro, Trp, or Tyr for the amino acid at position 298;
Arg, Lys, or Pro for the amino acid at position 300;
Lys or Pro for the amino acid at position 324;
Ala, Arg, Gly, His, Ile, Lys, Phe, Pro, Thr, Trp, Tyr, or Val for the amino acid at position 325;
Arg, Gln, His, Ile, Leu, Lys, Met, Phe, Pro, Ser, Thr, Trp, Tyr, or Val for the amino acid at position 327;
Arg, Asn, Gly, His, Lys, or Pro for the amino acid at position 328;
Asn, Asp, Gln, Glu, Gly, His, Ile, Leu, Lys, Met, Phe, Ser, Thr, Trp, Tyr, Val, or Arg for the amino acid at position 329;
Pro or Ser for the amino acid at position 330;
Arg, Gly, or Lys for the amino acid at position 331; or
Arg, Lys, or Pro for the amino acid at position 332.

(Embodiment 2) An antigen-binding molecule containing an Fc region having FcRn-binding activity under a neutral pH range condition and whose binding activity toward an inhibitory FcγR is higher than the binding activity toward an activating Fcγ receptor

By binding to two molecules of FcRn and one molecule of inhibitory FcγR, the antigen-binding molecule of Embodiment 2 can form a complex comprising these four molecules. However, since a single antigen-binding molecule can bind with only one molecule of FcγR, the single antigen-binding molecule in a state bound to an inhibitory FcγR cannot bind to other activating FcγRs. Furthermore, it has been reported that an antigen-binding molecule that is incorporated into cells in a state bound to an inhibitory FcγR is recycled onto the cell membrane, and thus escapes from degradation inside the cells (Immunity (2005) 23, 503-514). More specifically, it is considered that antigen-binding molecules having selective binding activity toward an inhibitory FcγR cannot form heterocomplexes containing an activating FcγR and two molecules of FcRn, which cause an immune response.

Preferred activating Fcγ receptors include FcγRI (CD64) which includes FcγRIa, FcγRIb, and FcγRIc; FcγRIIa (including allotypes R131 and H131); and FcγRIII (CD16) which includes isoforms FcγRIIIa (including allotypes V158 and F158) and FcγRIIIb (including allotypes FcγRIIIb-NA1 and FcγRIIIb-NA2). Meanwhile, examples of preferred inhibitory Fcγ receptors include FcγRIIb (including FcγRIIb-1 and FcγRIIb-2).

Herein, "a binding activity toward an inhibitory FcγR is higher than the binding activity toward an activating Fcγ receptor" means that the binding activity of the Fc region variant toward FcγRIIb is higher than the binding activity toward any of the human Fcγ receptors, FcγRI, FcγRIIa, FcγRIIIa, and/or FcγRIIIb. For example, it means that based on an above-described analytical method, the binding activity toward FcγRIIb of the antigen-binding molecule containing an Fc region variant as compared with the binding activity toward any of the human Fcγ receptors, FcγRI, FcγRIIa, FcγRIIIa, and/or FcγRIIIb is 105% or more, preferably 110% or more, 120% or more, 130% or more, 140% or more, and particularly preferably 150% or more, 160% or more, 170% or more, 180% or more, 190% or more, 200% or more, 250% or more, 300% or more, 350% or more, 400% or more, 450% or more, 500% or more, 750% or more, 10 times or more, 20 times or more, 30 times or more, 40 times or more, 50 times or more.

Most preferably, the binding activity toward FcγRIIb is higher than each of the binding activities toward FcγRIa, FcγRIIa (including allotypes R131 and H131), and FcγRIIIa (including allotypes V158 and F158). FcγRIa shows a markedly high affinity toward native IgG1; thus, the binding is thought to be saturated *in vivo* due to the presence of a large amount of endogenous IgG1. For this reason, inhibition of complex formation may be possible even if the binding activity toward FcγRIIb is greater than the binding activities toward FcγRIIa and FcγRIIIa, and lower than the binding activity toward FcγRIa.

As a control antigen-binding molecule containing an Fc region, antigen-binding molecules having an Fc region of a monoclonal IgG antibody may be appropriately used. The structures of such Fc regions are shown in SEQ ID NO: 5 (A is added to the N terminus of RefSeq Accession No. AAC82527.1), SEQ ID NO: 6 (A is added to the N terminus of RefSeq Accession No. AAB59393.1), SEQ ID NO: 7 (RefSeq Accession No. CAA27268.1), and SEQ ID NO: 8 (A is added to the N terminus of RefSeq Accession No. AAB59394.1). Further, when an antigen-binding molecule containing an Fc region of a particular antibody isotype is used as the test substance, effect on the binding activity of the Fc region-containing antigen-binding molecule toward an Fcγ receptor is tested by using an antigen-binding molecule having the Fc region of a monoclonal IgG antibody of a particular isotype as a control. In this way, antigen-binding molecules containing an Fc region whose binding activity toward the Fcγ receptor was demonstrated to be high are appropriately selected.

In a non-limiting embodiment of the present disclosure, preferred examples of Fc regions having a selective binding activity toward an inhibitory FcγR include Fc regions in which among the amino acids of an above-described Fc region, the amino acid at 238 or 328 as indicated by EU numbering is altered to an amino acid different from that of the native Fc region. Furthermore, as an Fc region having a selective binding activity toward an inhibitory Fcγ receptor, the Fc regions or alterations described in US 2009/0136485 can be appropriately selected.

In a non-limiting embodiment of the present disclosure, a preferred example is an Fc region having one or more of the following alterations as indicated by EU numbering in an aforementioned Fc region: the amino acid at position 238 is Asp; or the amino acid at position 328 is Glu.

In still another non-limiting embodiment of the present disclosure, examples of a preferred Fc region include Fc regions having a substitution of Pro at position 238 according to EU numbering with Asp and having one or more of the alterations:

alteration of the amino acid at position 237 according to EU numbering to Trp, the amino acid at position 237 according to EU numbering is Phe, the amino acid at position 267 according to EU numbering is Val, the amino acid at position 267 according to EU numbering is Gln, the amino acid at position 268 according to EU numbering is Asn, the amino acid at position 271 according to EU numbering is Gly, the amino acid at position 326 according to EU numbering is Leu, the amino acid at position 326 according to EU numbering is Gln, the amino acid at position 326 according to EU numbering is Glu, the amino acid at position 326 according to EU numbering is Met, the amino acid at position 239

according to EU numbering is Asp, the amino acid at position 267 according to EU numbering is Ala, the amino acid at position 234 according to EU numbering is Trp, the amino acid at position 234 according to EU numbering is Tyr, the amino acid at position 237 according to EU numbering is Ala, the amino acid at position 237 according to EU numbering is Asp, the amino acid at position 237 according to EU numbering is Glu, the amino acid at position 237 according to EU numbering is Leu, the amino acid at position 237 according to EU numbering is Met, the amino acid at position 237 according to EU numbering is Tyr, the amino acid at position 330 according to EU numbering is Lys, the amino acid at position 330 according to EU numbering is Arg, the amino acid at position 233 according to EU numbering is Asp, the amino acid at position 268 according to EU numbering is Asp, the amino acid at position 268 according to EU numbering is Glu, the amino acid at position 326 according to EU numbering is Asp, the amino acid at position 326 according to EU numbering is Ser, the amino acid at position 326 according to EU numbering is Thr, the amino acid at position 323 according to EU numbering is Ile, the amino acid at position 323 according to EU numbering is Leu, the amino acid at position 323 according to EU numbering is Met, the amino acid at position 296 according to EU numbering is Asp, the amino acid at position 326 according to EU numbering is Ala, the amino acid at position 326 according to EU numbering is Asn, and the amino acid at position 330 according to EU numbering is Met.

(Embodiment 3) An antigen-binding molecule containing an Fc region, in which one of the two polypeptides constituting the Fc region has an FcRn-binding activity under a neutral pH range condition and the other polypeptide does not have FcRn-binding activity under a neutral pH range condition

By binding to one molecule of FcRn and one molecule of FcγR, the antigen-binding molecule of Embodiment 3 can form a trimeric complex; however, it does not form any heterocomplex comprising four molecules including two molecules of FcRn and one molecule of FcγR. As an Fc region in which one of the two polypeptides constituting the Fc region has an FcRn-binding activity under a neutral pH range condition and the other does not have any FcRn-binding activity under a neutral pH range condition contained in the antigen-binding molecule of Embodiment 3, Fc regions derived from bispecific antibodies may be suitably used. Bispecific antibodies are two types of antibodies having specificities toward different antigens. Bispecific antibodies of an IgG type can be secreted from hybrid hybridomas (quadromas) resulting from fusion of two types of hybridomas producing IgG antibodies (Milstein et al. (Nature (1983) 305, 537-540).

When an antigen-binding molecule of Embodiment 3 described above is produced by using recombination techniques such as those described in the section "Antibodies" above, one can use a method in which genes encoding the polypeptides that constitute the two types of Fc regions of interest are transfected into cells to co-express them. However, the produced Fc regions will be a mixture in which the following will exist at a molecular ratio of 2:1:1: an Fc region in which one of the two polypeptides constituting the Fc region has an FcRn-binding activity under a neutral pH range condition and the other polypeptide does not have any FcRn-binding activity under a neutral pH range condition; an Fc region in which the two polypeptides constituting the Fc region both have an FcRn-binding activity under a neutral pH range condition; and an Fc region in which both of the two polypeptides constituting the Fc region do not have FcRn-binding activity under a neutral pH range condition. It is difficult to purify antigen-binding molecules containing the desired combination of Fc regions from the three types of IgGs.

When producing the antigen-binding molecules of Embodiment 3 using such recombination techniques, antigen-binding molecules comprising a heteromeric combination of Fc regions can be preferentially secreted by adding appropriate amino acid substitutions to the CH3 domains constituting the Fc regions. Specifically, this method is conducted by substituting an amino acid having a larger side chain (knob (which means "bulge")) for an amino acid in the CH3 domain of one of the heavy chains, and substituting an amino acid having a smaller side chain (hole (which means "void")) for an amino acid in the CH3 domain of the other heavy chain so that the knob is arranged in the hole. This promotes heteromeric H chain formation and simultaneously inhibits homomeric H chain formation (WO 1996027011; Ridgway et al., (Protein Engineering (1996) 9, 617-621); Merchant et al., (Nature Biotechnology (1998) 16, 677-681)).

Furthermore, there are also known techniques for producing a bispecific antibody by applying methods for controlling polypeptide association or association of polypeptide-formed heteromeric multimers to the association between two polypeptides that constitute an Fc region. Specifically, methods for controlling polypeptide association may be employed to produce a bispecific antibody (WO 2006/106905), in which amino acid residues forming the interface between two polypeptides that constitute the Fc region are altered to inhibit the association between Fc regions having the same sequence, and to allow the formation of polypeptide complexes formed by two Fc regions of different sequences. Specifically, the methods in the above-described section on bispecific antibodies and methods for producing them can be used as a non-limiting embodiment for preparing the antigen-binding molecule of Embodiment 3 of the present disclosure.

These antigen-binding molecules of Embodiments 1 to 3 are all expected to be able to reduce immunogenicity and improve plasma retention as compared to antigen-binding molecules capable of forming tetrameric complexes.

FcRn

Unlike Fcγ receptor belonging to the immunoglobulin superfamily, human FcRn is structurally similar to polypeptides of major histocompatibility complex (MHC) class I, exhibiting 22% to 29% sequence identity to class I MHC molecules (Ghetie et al., Immunol. Today (1997) 18 (12): 592-598). FcRn is expressed as a heterodimer consisting of soluble β or light chain (β2 microglobulin) complexed with transmembrane α or heavy chain. Like MHC, FcRn α chain comprises three extracellular domains (α1, α2, and α3) and its short cytoplasmic domain anchors the protein onto the cell surface, α1 and α2 domains interact with the FcRn-binding domain of the antibody Fc region (Raghavan et al., Immunity (1994) 1: 303-315).

FcRn is expressed in maternal placenta and yolk sac of mammals, and is involved in mother-to-fetus IgG transfer. In addition, in neonatal small intestine of rodents, where FcRn is expressed, FcRn is involved in transfer of maternal IgG across brush border epithelium from ingested colostrum or milk. FcRn is expressed in a variety of other tissues and endothelial cell systems of various species. FcRn is also expressed in adult human endothelia, muscular blood vessels, and hepatic sinusoidal capillaries. FcRn is believed to play a role in maintaining the plasma IgG concentration by mediating recycling of IgG to serum upon binding to IgG. Typically, binding of FcRn to IgG molecules is strictly pH dependent. The optimal binding is observed in an acidic pH range below 7.0.

Human FcRn whose precursor is a polypeptide having the signal sequence of SEQ ID NO: 28 (the polypeptide with the signal sequence is shown in SEQ ID NO: 29) forms a complex with human β2-microglobulin *in vivo.* Soluble human FcRn complexed with β2-microglobulin is produced by using conventional recombinant expression techniques. Fc regions of the present disclosure can be assessed for their binding activity to such a soluble human FcRn complexed with β2-microglobulin. Herein, unless otherwise specified, human FcRn refers to a form capable of binding to an Fc region of the present disclosure. Examples include a complex between human FcRn and human β2-microglobulin.

Embodiments of combining the present disclosure with techniques for modifying the constant region are, for example, combinations with antibody modification techniques such as Fc-modifying techniques to enhance FcRn binding at acidic pH (WO2002060919, WO2004035752, and WO2000042072), Fc-modifying techniques to enhance FcRn binding at neutral pH (WO2011122011 and WO2012133782), techniques for enhancing inhibitory Fcγ receptor-selective binding (WO2012115241 and WO2013125667), techniques for enhancing activating Fcγ receptor-selective binding (techniques for enhancing ADCC activity) (WO2013002362), and techniques for lowering the binding activity to a Rheumatoid factor (WO2013046704).

A non-limiting embodiment of a combination of the present disclosure with techniques for modifying the variable region includes, for example, combinations with techniques for modifying pH-dependent antibodies (WO2009125825), calcium-dependent antibodies (WO2012073992), and such.

The antigen-binding molecule in the present disclosure may have an amino acid residue that interacts with MTA. The amino acid residue that interacts with MTA may be present in the antigen-binding domain in the antigen-binding molecule, or may be present at a site other than the antigen-binding domain. Further, the amino acid residue that interacts with MTA may be only one residue or a plurality of amino acid residues in the antigen-binding molecule.

An antigen-binding domain is exemplified as a site where an amino acid residue that interacts with MTA is located. Further, an antibody is exemplified as a non-limiting embodiment of an antigen-binding molecule comprising an amino acid residue that interacts with MTA. Amino acid residues that interact with MTA can be present in the constant and/or variable regions of the antibody, and can also be present in CDRs and FRs in the antibody variable regions, but are not limited to any of these.

An example of a site where an amino acid residue that interacts with MTA is located includes a site different from the antigen-binding domain of the antigen-binding molecule. Interaction of MTA and an amino acid residue in a site different from the antigen-binding domain of the antigen-binding molecule may change the structure of the antigen-binding domain, and indirectly change the antigen-binding activity in an MTA-dependent manner.

Amino acid residues that interact with MTA in the antigen-binding molecule can be identified by analyzing a two-molecule complex between MTA and an antigen-binding molecule, or a three-molecule complex between MTA, antigen, and antigen-binding molecule, by a method such as crystal structure analysis, three-dimensional structure analysis using NMR, or introduction of amino acid mutations.

An antibody is an example of a non-limiting embodiment of an antigen-binding molecule comprising an antigen-binding domain whose antigen-binding activity to an antigen changes in an MTA-dependent manner in the present disclosure.

In the present disclosure, an antigen-binding molecule can have two or more antigen-binding domains and bind to a plurality of different antigens at the same time.

As a non-limiting embodiment, an antigen-binding molecule of the present disclosure further has a second antigen-binding domain, and the second antigen-binding domain has a binding activity for a second antigen different from the antigen to which the antigen-binding domain binds.

When the antigen-binding molecule has a plurality of different antigen-binding domains, at least one of the

antigen-binding domains may be an antigen-binding domain whose antigen-binding activity changes depending on MTA or a small molecule compound other than MTA. Alternatively, all the antigen-binding domains contained in the antigen-binding molecule may be antigen-binding domains whose antigen-binding activity changes depending on MTA or a small molecule compound other than MTA. Further, there may be a plurality of antigen-binding domains whose antigen-binding activity changes depending on different small molecule compounds, or one antigen-binding molecule may have, co-existing therein, an antigen-binding domain whose antigen-binding activity changes in a small molecule compound-dependent manner and an antigen-binding domain whose antigen-binding activity is not affected by the concentration of a small molecule compound.

In a further embodiment, the binding activity of the second antigen-binding domain to the second antigen is substantially unaffected by MTA.

In a further embodiment, the binding activity of the second antigen-binding domain to the second antigen changes in an MTA-dependent manner. In a more detailed embodiment, the binding activity of the second antigen-binding domain to the second antigen in the presence of MTA is different from the binding activity of the second antigen-binding domain to the second antigen in the absence of MTA.

A non-limiting example of antigen-binding molecules having two or more antigen-binding domains and capable of simultaneously binding to a plurality of different antigens is, without restriction, a bispecific antibody.

An example of a non-limiting embodiment of a biparatopic antigen-binding molecule of the present disclosure is a diabody or sc(Fv)2, which are paratopes different from each other, where one paratope binds to an epitope present in a membrane-type molecule that binds to the cell membrane of cancer cells, cells infiltrating cancer tissue, etc., and the other paratope binds to an epitope present in a membrane-type molecule expressed on the cell membrane of effector cells. In the above diabody or sc(Fv)2, the binding activity of one paratope to an epitope present in a membrane-type molecule that binds to the cell membrane of cancer cells or cells infiltrating cancer tissue can change in an MTA-dependent manner, and the binding activity of one paratope to an epitope present in a membrane-type molecule that binds to the cell membrane of an effector cell can change in an MTA-dependent manner, and also the binding activity of both paratopes can change in an MTA-dependent manner. When only one of the binding activities of one paratope to an epitope present in a membrane-type molecule that binds to the cell membrane of cancer cells, cells infiltrating cancer tissue, etc., or of one paratope to an epitope present in a membrane-type molecule that binds to the cell membrane of effector cells, changes depending on MTA, an antigen-binding domain whose antigen-binding activity does not change in an MTA-dependent manner may be an antigen-binding domain whose antigen-binding activity changes depending on a small molecule compound other than MTA (for example, a cancer tissue-specific compound or a non-natural small molecule compound).

Examples of membrane-type molecules that bind to the cell membrane of effector cells include, but are not limited to, TCR complex, CD3, and CD3e.

In one embodiment of a biparatopic antigen-binding molecule, where one paratope binds to an epitope present in a membrane-type molecule that binds to the cell membrane of cancer cells, cells infiltrating cancer tissue, and such (also called target cells), and the other paratope binds to an epitope present in a membrane-type molecule expressed on the cell membrane of effector cells, the effector cells are activated by the binding of the biparatopic antigen-binding molecule to both the target cell and the effector cell, and can elicit cytotoxic activity against target cells. When the effector cells are T cells, the cytotoxic activity evoked is T-cell-dependent cytotoxicity (TDCC) activity.

## Cytotoxic substances

In order for antigen-binding molecules of the present disclosure to bind to cancer cells and exhibit cytotoxic activity, cytotoxic substances may be linked to antigen-binding molecules. The cytotoxic substances may be chemotherapeutic agents exemplified below, or compounds disclosed in Curr Opin Chem Biol (2010) 14, 529-37 and WO 2009/140242; and these compounds are linked to antigen-binding molecules by appropriate linkers and such. When antigen-binding molecules of the present disclosure are used as pharmaceutical compositions, these cytotoxic substances may be linked to the antigen-binding molecules prior to administration, or they may be administered before, after, or at the same time when the antigen-binding molecules are administered to subjects (test individuals, patients, and such).

The later-described modified antigen-binding molecules to which cytotoxic substances such as chemotherapeutic agents, toxic peptides, or radioactive chemical substances have been linked may also be used preferably as antigen-binding molecules of the present disclosure having cytotoxic activity. Such modified antigen-binding molecules (hereinafter referred to as antigen-binding molecule-drug conjugate) can be obtained by chemically modifying the obtained antigen-binding molecules. Methods that have been already established in the field of antibody-drug conjugates and such may be used appropriately as methods for modifying antigen-binding molecules. Furthermore, a modified antigen-binding molecule to which a toxic peptide is linked can be obtained by expressing in appropriate host cells a fused gene produced by linking a gene encoding the toxic peptide in frame with a gene encoding an antigen-binding

molecule of the present disclosure, and then isolating it from the cell culture.

Examples of chemotherapeutic agents linked to the antigen-binding molecules of the present disclosure may include:

azaribine, anastrozole, azacytidine, bleomycin, bortezomib, bryostatin-1, busulfan, camptothecin, 10-hydroxycamptothecin, carmustine, celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxyurea, idarubicin, ifosfamide, leucovorin, lomustine, maytansinoid, mechlorethamine, medroxyprogesterone acetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine, streptozocin, tamoxifen, taxanes, taxol, testosterone propionate, thalidomide, thioguanine, thiotepa, teniposide, topotecan, uracil mustard, vinblastine, vinorelbine, and vincristine.

In the present disclosure, preferred chemotherapeutic agents are low-molecular-weight chemotherapeutic agents. Low-molecular-weight chemotherapeutic agents are unlikely to interfere with the function of antigen-binding molecules even after they bind to antigen-binding molecules of the present disclosure. In the present disclosure, low-molecular-weight chemotherapeutic agents usually have a molecular weight of 100 to 2000, preferably 200 to 1000. The chemotherapeutic agents exemplified herein are all low-molecular-weight chemotherapeutic agents. The chemotherapeutic agents of the present disclosure include prodrugs that are converted into active chemotherapeutic agents *in vivo.* Prodrug activation may be enzymatic conversion or non-enzymatic conversion.

Moreover, cytotoxic substances that are linked to antigen-binding molecules of the present disclosure include, for example, toxic peptides (toxins) such as Pseudomonas exotoxin A, Saporin-s6, Diphtheria toxin, Cnidarian toxin; radioiodine; and photosensitizers. Suitable examples of the toxic peptides include the following:

Diphtheria toxin A Chain (Langone et al. (Methods in Enzymology (1983) 93, 307-308));
Pseudomonas Exotoxin (Nature Medicine (1996) 2, 350-353);
Ricin Chain (Ricin A Chain) (Fulton et al. (J. Biol. Chem. (1986) 261, 5314-5319), Sivam et al. (Cancer Res. (1987) 47, 3169-3173), Cumber et al. (J. Immunol. Methods (1990) 135, 15-24), Wawrzynczak et al. (Cancer Res. (1990) 50, 7519-7562), and Gheeite et al. (J. Immunol. Methods (1991) 142, 223-230));
Deglicosylated Ricin A Chain (Thorpe et al. (Cancer Res. (1987) 47, 5924-5931));
Abrin A Chain (Wawrzynczak et al. (Br. J. Cancer (1992) 66, 361-366), Wawrzynczak et al. (Cancer Res. (1990) 50, 7519-7562), Sivam et al. (Cancer Res. (1987) 47, 3169-3173), and Thorpe et al. (Cancer Res. (1987) 47, 5924-5931));
Gelonin (Sivam et al. (Cancer Res. (1987) 47, 3169-3173), Cumber et al. (J. Immunol. Methods (1990) 135, 15-24), Wawrzynczak et al. (Cancer Res., (1990) 50, 7519-7562), and Bolognesi et al. (Clin. exp. Immunol. (1992) 89, 341-346));
PAP-s; Pokeweed anti-viral protein from seeds (Bolognesi et al. (Clin. exp. Immunol. (1992) 89, 341-346));
Briodin (Bolognesi et al. (Clin. exp. Immunol. (1992) 89, 341-346));
Saporin (Bolognesi et al. (Clin. exp. Immunol. (1992) 89, 341-346));
Momordin (Cumber et al. (J. Immunol. Methods (1990) 135, 15-24); Wawrzynczak et al. (Cancer Res. (1990) 50, 7519-7562); and Bolognesi et al. (Clin. exp. Immunol. (1992) 89, 341-346));
Momorcochin (Bolognesi et al. (Clin. exp. Immunol. (1992) 89, 341-346));
Dianthin 32 (Bolognesi et al. (Clin. exp. Immunol. (1992) 89, 341-346));
Dianthin 30 (Stirpe F., Barbieri L. (FEBS letter (1986) 195, 1-8));
Modeccin (Stirpe F., Barbieri L. (FEBS letter (1986) 195, 1-8));
Viscumin (Stirpe F., Barbieri L. (FEBS letter (1986) 195, 1-8));
Volkesin (Stirpe F., Barbieri L. (FEBS letter (1986) 195, 1-8));
Dodecandrin (Stirpe F., Barbieri L. (FEBS letter (1986) 195, 1-8));
Tritin (Stirpe F., Barbieri L. (FEBS letter (1986) 195, 1-8));
Luffin (Stirpe F., Barbieri L. (FEBS letter (1986) 195, 1-8)); and
Trichokirin (Casellas et al. (Eur. J. Biochem. (1988) 176, 581-588), and Bolognesi et al. (Clin. exp. Immunol., (1992) 89, 341-346)).

Cytotoxic activity and method for measuring cytotoxic activity

One non-limiting embodiment of the present disclosure provides an antigen-binding molecule comprising an antigen-binding domain whose antigen-binding activity changes in an MTA-dependent manner and has cytotoxic activity against cells expressing a membrane-type molecule on the cell membrane, and a pharmaceutical composition comprising

the antigen-binding molecule as an active ingredient. In the present disclosure, cytotoxic activity refers to, for example, antibody-dependent cell-mediated cytotoxicity (ADCC) activity, complement-dependent cytotoxicity (CDC) activity, T-cell-dependent cytotoxicity (TDCC) activity, and antibody-dependent cellular phagocytosis (ADCP) activity. In the present disclosure, CDC activity means cytotoxic activity by the complement system. ADCC activity means the activity of an immune cell to damage the target cell when the immune cell or the like binds to the Fc region of an antigen-binding molecule comprising an antigen-binding domain that binds to a membrane-type molecule expressed on the cell membrane of the target cell via an Fcγ receptor expressed on the immune cell. TDCC activity means the activity of T cells to damage the target cell by bringing the target cell close to the T cell by using a bispecific antibody that comprises an antigen-binding domain that binds to a membrane-type molecule expressed on the cell membrane of the target cell, or an antigen-binding domain to any of the subunits constituting the T cell receptor (TCR) complex on the T cell, particularly an antigen-binding domain that binds to the CD3 epsilon chain. Whether or not an antigen-binding molecule of interest has ADCC activity, CDC activity, TDCC activity, or ADCP activity can be measured by a known method.

Specifically, effector cells, complement solution, and target cells are first prepared.

(1) Preparation of effector cells

Spleen is removed from a CBA/N mouse or the like, and spleen cells are dispersed in an RPMI1640 medium (Invitrogen). After the cells are washed in the same medium containing 10% fetal bovine serum (FBS, HyClone), effector cells are prepared by adjusting the spleen cell concentration to $5 \times 10^6$ /mL.

(2) Preparation of complement solution

Baby Rabbit Complement (CEDARLANE) is diluted 10-fold in a culture medium (Invitrogen) containing 10% FBS to prepare a complement solution.

(3) Preparation of target cells

The target cells can be radioactively labeled by culturing cells expressing the antigen with 0.2 mCi of $^{51}$Cr-sodium chromate- (GE Healthcare Bio-Sciences) in a DMEM medium containing 10% FBS for one hour at 37°C. After radioactive labeling, cells are washed three times in an RPMI1640 medium containing 10% FBS, and the target cells can be prepared by adjusting the cell concentration to $2 \times 10^5$ /mL.

ADCC activity or CDC activity can be measured by the method described below. In the case of ADCC activity measurement, 50 μL each of the target cell and antigen-binding molecule are added to a 96-well U-bottom plate (Becton Dickinson), and allowed to react for 15 minutes at room temperature. Then, 100 μL of effector cells are added to the plate and this plate is placed in a carbon dioxide incubator for four hours. The final concentration of the antigen-binding molecule may be set, for example, to 0 μg/mL or 10 μg/mL. After incubation, 100 μL of the supernatant is collected from each well, and the radioactivity is measured with a gamma counter (COBRAII AUTO-GAMMA, MODEL D5005, Packard Instrument Company). The cytotoxic activity (%) can be calculated using the measured values according to the equation: $(A - C) / (B - C) \times 100$. A represents the radioactivity (cpm) in each sample, B represents the radioactivity (cpm) in a sample to which 1% NP-40 (Nacalai Tesque) has been added, and C represents the radioactivity (cpm) of a sample containing the target cells alone.

Meanwhile, in the case of CDC activity measurement, 50 μL of target cell and 50 μL of an antigen-binding molecule are added to a 96-well flat-bottomed plate (Becton Dickinson), and allowed to react for 15 minutes on ice. Then, 100 μL of a complement solution is added to the plate, and this plate is placed in a carbon dioxide incubator for four hours. The final concentration of the antigen-binding molecule may be set, for example, to 0 μg/mL or 3 μg/mL. After incubation, 100 μL of supernatant is collected from each well, and the radioactivity is measured with a gamma counter. The cytotoxic activity can be calculated in the same way as in the determination of ADCC activity.

ADCP activity can be measured by the method described below. Labeling of target cells expressing the antigen is performed using a PKH26 dye labeling kit (Sigma). The target cells are detached with a trypsin solution and washed with PBS. The detached cells are suspended in Diluent C to $1 \times 10^7$ cells/ml, the PKH26 dye stock (1 mM) is diluted to 8 μM with Diluent C, and diluted dye at an equal amount to the cell suspension is immediately added. After allowing to stand at room temperature for 5 minutes, 10% FBS-containing RPMI1640 is added and washed twice to prepare the target cells. The antigen-binding molecule, which is the test substance, is diluted to 20 μg/ml using the target cell medium, and the target cells are dispensed and mixed so as to be $2 \times 10^6$ cells/100 μl/tube. The final concentration of the antigen-binding molecule can be set to, for example, 0 or 10 μg/ml. After allowing to stand on ice for 30 minutes, the supernatant is discarded, the cells are washed twice with the culture solution, and suspended in 500 μL of the culture solution. The supernatant is removed from the effector cells, and the effector cells are added to the target cell suspension mixed with the antigen-binding molecule and mixed. After culturing in a $CO_2$ incubator for 3 hours, the cells are detached with

Trypsin-EDTA and collected. To the collected cells, for example, FITC-labeled anti-CD11b antibody is added, and the cells are allowed to stand on ice for 30 minutes. After allowing the cells to stand, the supernatant is discarded, and after washing twice with the culture solution, the collected cells are suspended in 300 μl of the culture solution, and measurement is performed with, for example, FACSCalibur (Becton Dickinson). ADCP activity can be measured in CD11b-positive macrophage cells by evaluating the PKH26-positive fraction as phagocytosis-positive cells.
TDCC activity can be measured by the methods described below. The TDCC activity of a test antibody can be measured using human peripheral blood mononuclear cells (hereinafter referred to as human PBMC) as effector cells.

(1) Preparation of human PBMC solution

50 ml of peripheral blood is collected from a healthy person using a syringe in which 500 μl of 5000 units/5 ml of heparin solution has been filled in advance. The peripheral blood is diluted 2-fold with PBS and divided into 4 equal parts and added to Leucosep lymphocyte separation tube (GE Healthcare) which had been pre-filled with 15 ml Ficoll-Paque PLUS and centrifuged. The separation tube into which the peripheral blood has been dispensed is centrifuged for 10 minutes at room temperature at a rate of 1000g to separate the mononuclear cell fraction. After washing the cells contained in each fraction once with RPMI-1640 containing 10% FBS (10% FBS/RPMI-1640), the cells are suspended in the culture medium of each target cell at a cell density of $2 \times 10^6$ cells/ml. The cell suspension is used as an effector cell for subsequent experiments.

(2) LDH release test (TDCC activity)

TDCC activity can be evaluated by the LDH release method (LDH Cytotoxicity Detection Kit: TAKARA). First, 50 μl each of the concentrations (0.000004, 0.00004, 0.0004, 0.004, 0.04, 0.4, 4, 40 μg/ml) of antibody solution that have been diluted with the culture medium of each target cell to 4 times the final concentration is added to each well of a 96-well U-bottom plate. Next, 50 μl each of target cells prepared to $2 \times 10^5$ cells/ml with the culture medium of each target cell is seeded ($1 \times 10^4$ cells/well) and allowed to stand at room temperature for 15 minutes. A plate containing in each well 100 μl ($2 \times 10^5$ cells/well) of the human PBMC solution prepared in (1) with the culture medium of each target cell is left to stand for about 24 hours at 37 °C in a 5% $CO_2$ incubator and then centrifuged. 100 μl of culture supernatant in each well of the plate is transferred to a 96-well flat bottom plate. The catalyst solution of the LDH Cytotoxicity Detection Kit is dissolved in 1 ml of H2O and mixed with the dye solution at a ratio of 1:45. The mixed solution of the catalyst solution and the dye solution is dispensed into a 96-well flat bottom plate to which the culture supernatant is transferred at 100 μl/well, and allowed to stand at room temperature for 15-30 minutes. The absorbance is measured at 490-492 nm with a plate reader. The control wavelength was set as 600-620 nm and subtracted from absorbance at 490-492 nm. The value obtained by subtracting the average value of the wells containing only the culture solution (blanks) is applied to the formula below. The cytotoxic activity can be determined based on the following formula:

$$\text{Cytotoxicity (TDCC) (\%)} = ((\text{A-B}) - \text{C})) \times 100/(\text{D-C})$$

Here, A is the absorbance of a mixture of target cells, effector cells, and antibodies, B is the absorbance of effector cells, C is the absorbance of target cells, and D is the absorbance of target cells added with Triton X-100.

<u>T cell activation and method for measuring T cell activation potency</u>

One non-limiting embodiment of the present disclosure provides an antigen-binding molecule comprising an antigen-binding domain whose antigen-binding activity changes in an MTA-dependent manner, and is capable of activating T cells through binding to any of the subunits constituting the T cell receptor (TCR) complex on T cells, and a pharmaceutical composition comprising the antigen-binding molecule as an active ingredient. T cell activation can be induced by using an antigen-binding domain that binds to a membrane-type molecule expressed on the cell membrane of the target cell, and an antigen-binding domain for any of the subunits constituting the T cell receptor (TCR) complex on T cells, in particular, by using a bispecific antibody comprising an antigen-binding domain that binds to the CD3 epsilon chain and bringing the target cell close to the T cell. The T cell activation potency can be measured using, for example, GloResponse NFAT-RE-Luc2 Jurkat cells (Promega). The cells are incorporated with a luciferase reporter whose expression is induced by the NFAT response element (NFAT-RE), which is a Nuclear Factor of Activated T cell (NFAT) response sequence, and activation of a TCR complex expressed on the Jurkat cell membrane can be detected by measuring the expression of the reporter protein. By using these cells, it is possible to measure the T cell activation potency by TCR ligands, anti-TCR antibodies, and anti-CD3 antibodies that can activate the TCR complex. The measurement of the T cell activation potency is not limited to the method using the above cells, and can be carried out by

constructing and using cells of the T cell lineage into which the same NFAT response element (NFAT-RE) and reporter protein have been introduced. Further, the measurement of the T cell activation potency is not limited to the above method, and for example, the expression of T cell activation markers such as CD69 and CD25 may be confirmed by a flow cytometry method or a gene expression analysis method, by measuring cytokines secreted by activated T cells such as TNF-$\alpha$, IFN-$\gamma$, IL-6, and IL-2, but it is not limited to these methods, and any method can be used regardless of the cell line or method of measurement as long as T cell activation can be measured.

As a specific non-limiting embodiment of the method for measuring the T cell activation potency, the method for measuring the T cell activation potency of a bispecific antibody having an antigen-binding domain that binds to the target antigen hIL-6R and an antigen-binding domain that binds to CD3 is illustrated below. NFAT-RE-luc2-Jurkat cells are used as the human T cell line, and CT26/hIL-6R in which hIL-6R is forcibly expressed in CT26 is used as the hIL-6R expression cell line. NFAT-RE-luc2-Jurkat cells and CT26/hIL-6R cells can be suspended in assay buffer to achieve cell densities of $3 \times 10^6$ cells/mL and $1 \times 10^6$ cells/mL, respectively. The cell density and the mixing ratio of each cell may be increased or decreased depending on the cell type used for evaluation. To each well of the 384-well plate is added 10 $\mu$l of the prepared suspensions of human T cell line and hIL-6R-expressing cell line, and 10 $\mu$l of the prepared antibody solution is further added. The ability to activate the human T cell line can be evaluated by allowing the plate to stand at 37°C for 6 hours in a 5% $CO_2$ incubator, and then measuring the luciferase activity of the sample. Luciferase activity can be measured using a commercially available reagent for measuring luciferase activity (Bio-Glo luciferase assay system, Promega), but the reagent is not limited as long as luciferase activity can be measured.

Neutralizing activity and method of measuring neutralizing activity

The present disclosure provides in a non-limiting embodiment a pharmaceutical composition comprising as an active ingredient an antigen-binding molecule that contains an antigen-binding domain whose antigen-binding activity varies in an MTA-dependent manner and has a neutralizing activity against a membrane-type molecule. In another non-limiting embodiment, the present disclosure provides a pharmaceutical composition comprising as an active ingredient an antigen-binding molecule that contains an antigen-binding domain whose antigen-binding activity varies in a small molecule compound-dependent manner and has a neutralizing activity against a membrane-type molecule in addition to a cytotoxic activity against cells expressing the membrane-type molecule on their cell membrane. Generally, a neutralizing activity refers to an activity of inhibiting the biological activity of a ligand which has a biological activity towards cells, such as viruses and toxins. Thus, a substance having a neutralizing activity refers to a substance that binds to a ligand or a receptor to which the ligand binds and inhibits the binding between the ligand and the receptor. A receptor whose binding to the ligand has been blocked by the neutralizing activity will not be able to exhibit the biological activity through the receptor. When the antigen-binding molecule is an antibody, the antibody having such a neutralizing activity is generally called a neutralizing antibody. The neutralizing activity of a test substance may be measured by comparing the biological activities in the presence of a ligand between conditions when the test substance is present or absent.

A suitable example of a major ligand for the IL-6 receptor is IL-6, which is shown in SEQ ID NO: 27. The IL-6 receptor, which is an I-type membrane protein whose amino terminus forms the extracellular domain, forms a heterotetramer with the gp130 receptor which was induced by IL-6 to dimerize (Heinrich et al. (Biochem. J. (1998) 334, 297-314)). Formation of the heterotetramer activates Jak associated with the gp130 receptor. Jak carries out autophosphorylation and receptor phosphorylation. The phosphorylation sites of the receptor and of Jak serve as binding sites for molecules belonging to the Stat family having SH2 such as Stat3, and for the MAP kinases, PI3/Akt, and other proteins and adapters having SH2. Next, Stat that bound to the gp130 receptor is phosphorylated by Jak. The phosphorylated Stat dimerizes and translocates to the nucleus, and regulates transcription of target genes. Jak and Stat can also be involved in the signaling cascade through receptors of other classes. A deregulated IL-6 signaling cascade is observed in inflammation and pathological conditions of autoimmune diseases, and cancers such as prostate cancer and multiple myeloma. Stat3 which may act as an oncogene is constitutively activated in many cancers. In prostate cancer and multiple myeloma, there is a crosstalk between the signaling cascade from the IL-6 receptor and the signaling cascade from members of the epidermal growth factor receptor (EGFR) family (Ishikawa et al. (J. Clin. Exp. Hematopathol. (2006) 46 (2), 55-66)).

Such intracellular signaling cascades are different for each cell type; therefore, an appropriate target molecule can be set according to each of the target cells of interest, and the target molecule is not limited to the above-mentioned factors. The neutralization activity can be evaluated by measuring the *in vivo* signal activation. Furthermore, activation of *in vivo* signals can also be detected by using as an indicator the transcription-inducing action on a target gene that exists downstream of the *in vivo* signaling cascade. A change in the transcription activity of a target gene can be detected by the principle of a reporter assay. Specifically, a reporter gene such as the green fluorescence protein (GFP) or luciferase is placed downstream of a transcription factor or a promoter region of the target gene; and a change in transcription activity can be measured in terms of reporter activity by measuring the reporter activity. Commercially available kits for measuring *in vivo* signal activation can be suitably used (for example, the Mercury Pathway Profiling Luciferase System (Clontech)).

Furthermore, as a method for measuring the neutralization activity on a receptor ligand in the EGF receptor family and such which acts on a signaling cascade that typically works toward enhancing cell proliferation, neutralization activity of an antigen-binding molecule can be evaluated by measuring the proliferation activity of the target cells. For example, the following method is suitably used as a method for measuring or evaluating inhibitory effects based on the neutralization activity of an anti-HB-EGF antibody against the proliferation of cells whose proliferation is promoted by EGF family growth factors such as HB-EGF. As a method for evaluating or measuring the activity of inhibiting cell proliferation in a test tube, a method that measures the incorporation by living cells of [$^3$H]-labeled thymidine added to the culture medium as an index of the DNA replication ability is used. As a more convenient method, a dye exclusion method that measures under a microscope the ability of a cell to release a dye such as trypan blue to the outside of the cell, or the MTT method is used. The latter makes use of the ability of living cells to convert 3-(4,5-dimethylthiazol-2-yl)-2,5-diphenyl tetrazolium bromide (MTT), which is a tetrazolium salt, to a blue formazan product.

More specifically, a test antibody is added along with a ligand to the culture solution of a test cell; and after a certain period of time has elapsed, an MTT solution is added to the culture, and this is left to stand for a certain amount of time to let the cell incorporate MTT. As a result, MTT which is a yellow compound is converted to a blue compound by succinate dehydrogenase in the mitochondria of the cell. After this blue product is dissolved for coloration, its absorbance is measured and used as an indicator of the number of viable cells. Besides MTT, reagents such as MTS, XTT, WST-1, and WST-8 are also commercially available (Nacalai Tesque, and such), and can be suitably used. For measurement of the activity, a binding antibody that has the same isotype as the anti-HB-EGF antibody but does not have the cell proliferation-inhibiting activity can be used as a control antibody in the same manner as the anti-HB-EGF antibody, and the anti-HB-EGF antibody is judged to have the activity when it shows a stronger cell proliferation-inhibiting activity than the control antibody.

As cells for evaluating activity, for example, cells showing HB-EGF-promoted proliferation such as the RMG-1 cell line which is an ovarian cancer cell line may be suitably used; and mouse Ba/F3 cells transformed with a vector in which a gene encoding hEGFR/mG-CSFR, which is a fusion protein of the extracellular domain of human EGFR fused in frame with the intracellular domain of the mouse G-CSF receptor, is linked so as to allow expression, may also be suitably used. This way, those skilled in the art may appropriately select cells for evaluating activity to measure the cell proliferation activity mentioned above.

The "antigen-binding molecules comprising an antigen-binding domain whose antigen-binding activity varies in an MTA-dependent manner" of the present disclosure may yield positive pharmacological effects when administered to a living body.

Method of producing humanized antibodies

When an antigen-binding molecule described herein is administered to human, an antigen-binding domain derived from a genetically recombinant antibody that has been artificially altered to reduce the heterologous antigenicity against human and such, can be appropriately used as the antigen-binding domain of the antigen-binding molecule. Such genetically recombinant antibodies include, for example, humanized antibodies. These altered antibodies are appropriately produced by known methods.

An antibody variable region used to produce the antigen-binding domain of an antigen-binding molecule described herein is generally formed by three complementarity-determining regions (CDRs) that are separated by four framework regions (FRs). CDR is a region that substantially determines the binding specificity of an antibody. The amino acid sequences of CDRs are highly diverse. On the other hand, the FR-forming amino acid sequences often have high identity even among antibodies with different binding specificities. Therefore, generally, the binding specificity of a certain antibody can be introduced to another antibody by CDR grafting.

A humanized antibody is also called a reshaped human antibody. Specifically, humanized antibodies prepared by grafting the CDR of a non-human animal antibody such as a mouse antibody or a rabbit antibody to a human antibody and such are known. Common genetic engineering techniques for obtaining humanized antibodies are also known. Specifically, for example, overlap extension PCR is known as a method for grafting a mouse antibody CDR to a human FR, but is not limited thereto. In overlap extension PCR, a nucleotide sequence encoding a mouse antibody CDR to be grafted is added to primers for synthesizing a human antibody FR. Primers are prepared for each of the four FRs. It is generally considered that when grafting a mouse CDR to a human FR, selecting a human FR that has high identity to a mouse FR is advantageous for maintaining the CDR function. That is, it is generally preferable to use a human FR comprising an amino acid sequence which has high identity to the amino acid sequence of the FR adjacent to the mouse CDR to be grafted. The origin of the CDR is not limited to mouse or rabbit and may be selected from any non-human animal antibodies.

Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of

each product are designed so that they overlap with each other. Then, complementary strand synthesis reaction is conducted to anneal the overlapping CDR regions of the products synthesized using a human antibody gene as template. Human FRs are ligated *via* the mouse CDR sequences by this reaction.

The full length variable region gene, in which three CDRs and four FRs are ultimately ligated, is amplified using primers that anneal to its 5' - or 3'-end, which are added with suitable restriction enzyme recognition sequences. An expression vector for humanized antibody can be produced by inserting the DNA obtained as described above and a DNA that encodes a human antibody C region into an expression vector so that they will ligate in frame. After the recombinant vector is transfected into a host to establish recombinant cells, the recombinant cells are cultured, and the DNA encoding the humanized antibody is expressed to produce the humanized antibody in the cell culture (see, European Patent Publication No. EP 239400 and International Patent Publication No. WO 1996/002576).

By qualitatively or quantitatively measuring and evaluating the antigen-binding activity of the humanized antibody produced as described above, one can suitably select human antibody FRs that allow CDRs to form a favorable antigen-binding site when ligated through the CDRs. Amino acid residues in FRs may be substituted as necessary, so that the CDRs of a reshaped human antibody form an appropriate antigen-binding site. For example, amino acid sequence mutations can be introduced into FRs by applying the PCR method used for grafting a mouse CDR into a human FR. More specifically, partial nucleotide sequence mutations can be introduced into primers that anneal to the FR. Nucleotide sequence mutations are introduced into the FRs synthesized by using such primers. Mutant FR sequences having the desired characteristics can be selected by measuring and evaluating the activity of the amino acid-substituted mutant antibody to bind to the antigen by the above-mentioned method (Cancer Res. (1993) 53: 851-856).

Pharmaceutical compositions

The present disclosure provides pharmaceutical compositions comprising an antigen-binding molecule that does not act systemically in normal tissues or blood, but acts in cancer in order to exert a drug effect while avoiding side effects. Since the antigen-binding molecule comprised in the pharmaceutical composition of the present disclosure is regulated in its binding to a target antigen in an MTA-dependent manner, for example, when the antigen-binding molecule targets an antigen in cancer tissue, it cannot bind to antigens expressed in cancer cells, immune cells, stromal cells, etc. in cancer tissues or antigens secreted in cancer tissues, and therefore, while avoiding side effects due to cytotoxic effects and neutralizing effects on normal tissues, it exerts a strong cytotoxic effect, growth inhibitory effect, immune enhancing effect, and such on cancer. For example, a bispecific antigen-binding molecule or biparatopic antigen-binding molecule comprising an antigen-binding domain whose binding activity to CD3 expressed in T cells changes in an MTA-dependent manner and an antigen-binding domain that binds to EGFR expressed in cancer cells, does not bind to EGFR expressed in normal tissues, but binds to EGFR expressed in cancer cells, and thus, it exerts a strong antitumor effect while avoiding side effects. That is, it binds to CD3 expressed on T cells adjacent to the cancer cells in an MTA-dependent manner, but does not bind to CD3 expressed on T cells outside the vicinity of the cancer cells, and thus activates T cells in the vicinity of cancer cells and exerts a strong antitumor effect while avoiding side effects.

In this way, antigen-binding molecules that bind to antigens in cancer tissues and do not bind to antigens in other normal tissues or in blood exert their drug effects while avoiding side effects. The antigen-binding molecules comprising an antigen-binding domain whose antigen-binding activity changes in an MTA-dependent manner (also referred to as MTA switch antigen-binding molecules, antigen-binding molecules that bind to an antigen using MTA as a switch) provided by the present disclosure, do not bind to the antigen in a normal environment where MTA is absent, and can bind to the antigen in cancer tissues where MTA is present at a high concentration.

An antigen-binding molecule whose antigen-binding activity changes depending on MTA, which functions as a switch by being sandwiched between the antigen-binding molecule of the present disclosure (the paratope contained therein) and the antigen (the epitope contained therein), or by binding with the antigen-binding molecule of the present disclosure to thereby change the structure of the paratope of the antigen-binding molecule for the antigen, is exemplified. In the absence of MTA, the interaction between the paratope comprised in the antigen-binding molecule of the present disclosure and the epitope comprised in the antigen is not sufficient and the antigen-binding molecule of the present disclosure cannot bind to the antigen. In the presence of MTA, it interposes between the paratope contained in the antigen-binding molecule of the present disclosure and the epitope contained in the antigen, or changes the structure of the paratope, and thereby, the antigen-binding molecule that has bound to the antigen in a cancer tissue, where MTA is present at a high concentration, can exhibit a drug effect on cells expressing the antigen (Figs. 26 and 27). When an amino acid residue that interacts with MTA is present in the antigen-binding domain of the present disclosure whose antigen-binding activity changes in an MTA-dependent manner, it becomes easier for MTA to be interposed between the paratope contained in the antigen-binding molecule and the epitope contained in the antigen, or it becomes easier for MTA to change the structure of the paratope. Moreover, since this binding to MTA which serves as the switch is reversible, it is thought that the binding of an antigen-binding molecule of the present disclosure to an antigen by means of this MTA switch may be controlled in a reversible manner. Thus, antigen-binding molecules of the present disclosure

capable of binding to pathological cells such as cancer cells and immune cells in cancer tissue, or binding to antigens secreted in cancer tissue to exert a drug effect are useful as pharmaceutical compositions. The pharmaceutical compositions of the present disclosure may include a pharmaceutically-acceptable carrier.

In the present disclosure, "pharmaceutical composition" usually refers to agents for treating or preventing, or testing and diagnosing diseases. Further, in the present disclosure, the term "pharmaceutical composition comprising an antigen-binding molecule comprising an antigen-binding domain whose antigen-binding activity changes in an MTA-dependent manner" can be rephrased as "a method of treating a disease which comprises administering to a subject to be treated an antigen-binding molecule comprising an antigen-binding domain whose antigen-binding activity changes in an MTA-dependent manner", or as the "use of an antigen-binding molecule comprising an antigen-binding domain whose antigen-binding activity changes in an MTA-dependent manner in the production of a pharmaceutical for treating a disease". Furthermore, the phrase "pharmaceutical composition comprising an antigen-binding molecule comprising an antigen-binding domain whose antigen-binding activity changes in an MTA-dependent manner" can be rephrased as the "use of an antigen-binding molecule comprising an antigen-binding domain whose antigen-binding activity changes in an MTA-dependent manner, for treating a disease".

The pharmaceutical compositions of the present disclosure can be formulated by methods known to those skilled in the art. For example, they can be used parenterally, in the form of injections of sterile solutions or suspensions including water or other pharmaceutically acceptable liquid. For example, such compositions can be formulated by mixing in the form of unit dose required in the generally approved medicine manufacturing practice, by appropriately combining with pharmacologically acceptable carriers or media, specifically with sterile water, physiological saline, vegetable oil, emulsifier, suspension, surfactant, stabilizer, flavoring agent, excipient, vehicle, preservative, binder, or such. In such formulations, the amount of active ingredient is adjusted to obtain an appropriate amount in a pre-determined range.

Sterile compositions for injection can be formulated using vehicles such as distilled water for injection, according to standard formulation practice. Aqueous solutions for injection include, for example, physiological saline and isotonic solutions containing dextrose or other adjuvants (for example, D-sorbitol, D-mannose, D-mannitol, and sodium chloride). It is also possible to use in combination appropriate solubilizers, for example, alcohols (ethanol and such), polyalcohols (propylene glycol, polyethylene glycol, and such), non-ionic surfactants (polysorbate 80(TM), HCO-50, and such).

Oils include sesame oil and soybean oils. Benzyl benzoate and/or benzyl alcohol can be used in combination as solubilizers. It is also possible to combine buffers (for example, phosphate buffer and sodium acetate buffer), soothing agents (for example, procaine hydrochloride), stabilizers (for example, benzyl alcohol and phenol), and/or antioxidants. Appropriate ampules are filled with the prepared injections.

The pharmaceutical compositions of the present disclosure are preferably administered parenterally. For example, the compositions in the dosage form for injections, transnasal administration, transpulmonary administration, or transdermal administration are administered. For example, they can be administered systemically or locally by intravenous injection, intramuscular injection, intraperitoneal injection, subcutaneous injection, or such.

Administration methods can be appropriately selected in consideration of the patient's age and symptoms. The dose of a pharmaceutical composition containing an antigen-binding molecule can be, for example, from 0.0001 to 1,000 mg/kg for each administration. Alternatively, the dose can be, for example, from 0.001 to 100,000 mg per patient. However, the present disclosure is not limited by the numeric values described above. The doses and administration methods vary depending on the patient's weight, age, symptoms, and such. Those skilled in the art can set appropriate doses and administration methods in consideration of the factors described above.

## Method of using antigen-binding molecules that bind in an MTA-dependent manner

Antigen-binding molecules that bind in an MTA-dependent manner in the present disclosure can be combined with various existing pharmaceutical use technologies. An example of a non-limiting embodiment of such a combination of techniques is the production of chimeric antigen receptor T cells (CAR-T cells) utilizing an antigen-binding molecule that binds in an MTA-dependent manner. One of the non-limiting methods for producing CAR-T includes introducing a chimeric receptor into effector cells, mainly T cells, by gene modification technology, wherein the chimeric receptor comprises an antigen-binding molecule that specifically binds to a tumor-related antigen linked to the extracellular domain of TCR and a signal domain of a co-stimulatory molecule such as CD28 linked intracellularly with the aim of enhancing T cell activation. By using an MTA-dependently-binding antigen-binding molecule in the present disclosure as the antigen-binding molecule that specifically binds to a tumor-related antigen, it becomes possible for CAR-T cells to bind to an antigen specifically in tumor tissue with MTA accumulation.

In addition, a non-limiting example of a technique that can be combined with an antigen-binding molecule of the present disclosure that binds in an MTA-dependent manner includes the method of directly expressing the antigen-binding molecule by incorporating into a living body a nucleic acid encoding an antigen-binding molecule that binds in an MTA-dependent manner using a viral vector or the like. An example of a viral vector is Adenovirus, but it is not limited thereto. Without using a viral vector, it is also possible to directly incorporate into a living body a nucleic acid encoding

an antigen-binding molecule that binds in an MTA-dependent manner by an electroporation method or a method of directly administering a nucleic acid, and it is also possible to achieve continuous secretion of the antigen-binding molecule in the living body by administering to the living body a cell genetically modified so as to secrete and express the antigen-binding molecule.

Oligonucleotides

As used herein, "oligonucleotide" refers to, but is not limited to, a synthetic polynucleotide that is usually single-stranded, usually less than about 200 nucleotides in length. The terms "oligonucleotide" and "polynucleotide" are not mutually exclusive. The above description of polynucleotides is equally and fully applicable to oligonucleotides. In the present specification, "oligonucleotide" and "nucleic acid" are used interchangeably.

"An isolated polynucleotide encoding an antigen-binding molecule comprising an antigen-binding domain whose antigen-binding activity changes in an MTA-dependent manner" is one or more polynucleotide molecules encoding an antigen-binding molecule comprising an antigen-binding domain whose antigen-binding activity changes in an MTA-dependent manner, and includes polynucleotide molecules carried by one vector or separate vectors, and polynucleotide molecules present at one or more positions in a host cell. When the antigen-binding molecule is an antibody, the polynucleotide refers to one or more polynucleotide molecules encoding the heavy and light chains (or fragments thereof) of the antibody, and includes polynucleotide molecules carried by one vector or separate vectors, and polynucleotide molecules present at one or more positions in a host cell.

Vector

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Expression vectors may be introduced into host cells by methods using viruses, by electroporation, and such. Introduction of expression vectors is not limited to *ex vivo* introductions, and vectors may be directly introduced into living bodies.

Host cell

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

Recombinant Methods and Compositions

Antigen-binding molecules may be produced using recombinant methods and compositions, e.g., as described in U.S. Patent No. 4,816,567. In one embodiment, isolated nucleic acid encoding an antigen-binding molecule comprising an antigen-binding domain whose antigen-binding activity varies in an MTA-dependent manner described herein is provided. In an embodiment where the antigen-binding domain whose antigen-binding activity varies in an MTA-dependent manner comprises an antibody variable region, such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antigen-binding molecule and an amino acid sequence comprising the VH of the antigen-binding molecule, or (2) a first vector comprising a nucleic acid that encodes an amino acid sequence comprising the VL of the antigen-binding molecule and a second vector comprising a nucleic acid that encodes an amino acid sequence comprising the VH of the antigen-binding molecule. In one embodiment, the host cell is eukaryotic, e.g. a Chinese Hamster Ovary (CHO) cell or lymphoid cell (e.g., Y0, NS0, Sp2/0 cell). In one embodiment, a method of making an antigen-binding molecule comprising an antigen-binding domain whose antigen-binding activity varies in an MTA-dependent manner is provided, wherein the method comprises culturing a host cell comprising a nucleic acid encoding the antigen-binding molecule, as provided above, under conditions suitable for expression of the antigen-binding molecule

comprising an antigen-binding domain whose antigen-binding activity varies in an MTA-dependent manner, and optionally recovering the antibody from the host cell (or host cell culture medium).

For recombinant production of an antigen-binding molecule comprising an antigen-binding domain whose antigen-binding activity varies in an MTA-dependent manner, nucleic acid encoding an antigen-binding molecule, e.g., as described above, is isolated and inserted into one or more vectors for further cloning and/or expression in a host cell. Such nucleic acid may be readily isolated and sequenced using conventional procedures (e.g., in the case of an antibody, by using oligonucleotide probes that are capable of binding specifically to genes encoding the heavy and light chains of the antibody).

Suitable host cells for cloning or expression of vectors encoding an antigen-binding molecule comprising an antigen-binding domain whose antigen-binding activity varies in an MTA-dependent manner include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antigen-binding molecule comprising an antigen-binding domain whose antigen-binding activity varies in an MTA-dependent manner may be isolated from the bacterial cell paste in a soluble fraction and can be further purified.

In addition to prokaryotes, eukaryotic microbes such as filamentous fungi or yeast are suitable cloning or expression hosts for vectors encoding antigen-binding molecules, including fungi and yeast strains whose glycosylation pathways have been "humanized," resulting in the production of an antigen-binding molecule with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antigen-binding molecule are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antigen-binding molecules in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR" CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antigen-binding molecule production, see, e.g., Yazaki and Wu, Methods in Molecular Biology, Vol. 248 (B.K.C. Lo, ed., Humana Press, Totowa, NJ), pp. 255-268 (2003). Further, examples of host cells are not limited to animal cell lines. Effector cells extracted from living bodies and various cells including mesenchymal stem cell (MSC) can naturally be used as host cells.

A method of screening for antigen-binding molecules comprising an antigen-binding domain whose antigen-binding activity changes in an MTA-dependent manner

The present disclosure provides a method of screening for antigen-binding molecules comprising an antigen-binding domain whose antigen-binding activity changes in an MTA-dependent manner. An antigen-binding domain (or an antigen-binding molecule containing the domain) whose antigen-binding activity changes in an MTA-dependent manner can be acquired according to the teachings of the present disclosure. Some non-limiting examples of an embodiment of the method of acquisition are illustrated below.

For example, in order to confirm that the antigen-binding activity of an antigen-binding domain (or an antigen-binding molecule containing the domain) in the presence of a first concentration of MTA is different from the antigen-binding activity of the antigen-binding domain (or an antigen-binding molecule containing the domain) in the presence of a second concentration of MTA, the antigen-binding activities of the antigen-binding domain (or the antigen-binding molecule containing the domain) in the presence of a first concentration of MTA and a second concentration of MTA are compared. To select an antigen-binding domain whose antigen-binding activity changes in an MTA-dependent manner, an antigen-binding domain (or an antigen-binding molecule containing the domain) whose antigen-binding

activity in the presence of the first concentration of MTA is different from that in the presence of the second concentration of MTA is selected. The first concentration and the second concentration are different concentrations, and either one of these concentrations can be set to 0 (that is, MTA is absent).

When the first concentration is set to a concentration higher than the second concentration, the following acquisition method can be exemplified.

In order to obtain an antigen-binding domain (or an antigen-binding molecule containing the domain) whose antigen-binding activity in the presence of a high concentration of MTA is higher than that in the presence of a low concentration of MTA, an antigen-binding domain (or an antigen-binding molecule containing the domain) whose antigen-binding activity in a first concentration is higher than that in a second concentration is selected.

In order to obtain an antigen-binding domain (or an antigen-binding molecule containing the domain) whose antigen-binding activity in the presence of MTA is higher than that in the absence of MTA, the second concentration is set to 0, and an antigen-binding domain (or an antigen-binding molecule containing the domain) whose antigen-binding activity in the first concentration is higher than that in the second concentration is selected.

In order to select an antigen-binding domain whose antigen-binding activity in the presence of a first concentration of MTA is higher than that in the presence of a second concentration of MTA, as long as the antigen-binding activity in the presence of the first concentration of MTA is higher than the antigen-binding activity in presence of the second concentration of MTA, the difference between the antigen-binding activity in the presence of the first concentration of MTA and the antigen-binding activity in the presence of the second concentration of MTA is not particularly limited, but preferably, the antigen-binding activity in the presence of the first concentration of MTA as compared to that in the presence of the second concentration of MTA is 2 times or more, more preferably 10 times or more, and even more preferably 40 times or more. The upper limit of the difference in the antigen-binding activity is not particularly limited, and may be any value such as 400 times, 1000 times, 10000 times, etc. as long as it can be produced within the skill of a person skilled in the art. If no antigen-binding activity is observed in the presence the a second concentration of MTA, this upper limit is an infinite number.

In order to obtain an antigen-binding domain (or an antigen-binding molecule containing the domain) whose antigen-binding activity in the presence of a high concentration of MTA is lower than that in the presence of a low concentration of MTA, an antigen-binding domain (or an antigen-binding molecule containing the domain) whose antigen-binding activity in the first concentration is lower than that in the second concentration is selected.

In order to obtain an antigen-binding domain (or an antigen-binding molecule containing the domain) whose antigen-binding activity in the presence of MTA is lower than that in the absence MTA, the second concentration is set to 0, and an antigen-binding domain (or an antigen-binding molecule containing the domain) whose antigen-binding activity in the first concentration is lower than that in the second concentration is selected.

In order to select an antigen-binding domain whose antigen-binding activity in the presence of a first concentration of MTA is lower than that in the presence of a second concentration of MTA, as long as the antigen-binding activity in the presence of the first concentration of MTA is lower than the antigen-binding activity in the presence of the second concentration of MTA, the difference between the antigen-binding activity in the presence of the first concentration of MTA and the antigen-binding activity in the presence of the second concentration of MTA is not particularly limited, but preferably, the antigen-binding activity in the presence of the second concentration of MTA as compared to the antigen-binding activity in the presence of the first concentration of MTA is 2 times or more, more preferably 10 times or more, and even more preferably 40 times or more. The upper limit of the difference in the antigen-binding activity is not particularly limited, and may be any value such as 400 times, 1000 times, 10000 times, etc. as long as it can be produced within the skill of a person skilled in the art. If no antigen-binding activity is observed in the presence of the first concentration of MTA, this upper limit is an infinite number.

Furthermore, in the present disclosure, the phrase "the antigen-binding activity in the presence of MTA is higher than the antigen-binding activity in the absence of MTA" can be alternatively expressed as "the antigen-binding activity of an antigen-binding domain (or an antigen-binding molecule containing the domain) in the absence of MTA is lower than the antigen-binding activity in the presence of MTA". Furthermore, in the present disclosure, "the antigen-binding activity of an antigen-binding domain (or an antigen-binding molecule containing the domain) in the absence of MTA is lower than the antigen-binding activity in the presence of MTA" may be alternatively described as "the antigen-binding activity of an antigen-binding domain (or an antigen-binding molecule containing the domain) in the absence of MTA is weaker than the antigen-binding activity in the presence of MTA".

Furthermore, in the present disclosure, the phrases "the antigen-binding activity in the presence of a first concentration of MTA is higher than the antigen-binding activity in the presence of the second concentration of MTA" and "the first concentration is higher than the second concentration" can be alternatively expressed as "the antigen-binding activity in the presence of a high concentration of MTA is higher than the antigen-binding activity in the presence of a low concentration of MTA" or "the antigen-binding activity of an antigen-binding domain (or an antigen-binding molecule containing the domain) in the presence of a low concentration of MTA is lower than the antigen-binding activity in the presence of a high concentration of MTA". In the present disclosure, "the antigen-binding activity of an antigen-binding domain (or an antigen-binding molecule containing the domain) in the presence of a low concentration of MTA is lower than the antigen-binding activity in the presence of a high concentration of MTA" may be alternatively described as "the antigen-binding activity of an antigen-binding domain (or an antigen-binding molecule containing the domain) in the presence of a low concentration of MTA is weaker than the antigen-binding activity in the presence of a high concentration of MTA".

Conditions when measuring antigen-binding activity other than the concentration of MTA are not particularly limited, and can be selected appropriately by those skilled in the art. For example, it is possible to measure under conditions of HEPES buffer and 37°C. For example, Biacore (GE Healthcare) or such can be used for measurement. When the antigen is a soluble molecule, the activity of an antigen-binding domain (or an antigen-binding molecule containing the domain) to bind to the soluble molecule can be determined by loading the antigen as an analyte onto a chip immobilized with the antigen-binding domain (or an antigen-binding molecule containing the domain). Alternatively, when the antigen is a membrane-type molecule, the binding activity towards the membrane-type molecule can be determined by loading the antigen-binding domain (or an antigen-binding molecule containing the domain) as an analyte onto a chip immobilized with the antigen.

Similar to the above MTA, it is possible to compare the antigen-binding activity of an antigen-binding domain (or antigen-binding molecule) in the presence of other small molecule compounds including MTA analogs at different small molecule compound concentrations. Specific examples of such small molecule compounds include, but are not limited to, metabolites of the polyamine biosynthetic pathway such as S-adenosylmethionine (SAM: S-adenoshylmethionine) and S-adenosylhomocysteine (SAH: S-adenosylhomocystein, S-(5'-adenosyl) -L-homocysteine) (Stevens et al. (J Chromatogr A. 2010 May 7; 1217 (19): 3282-8)), and AMP, ADP, ATP, adenosine, and the like, which are metabolites of the purine nucleotide metabolic pathway.

In one non-limiting embodiment, the present disclosure provides a method of screening for an antigen-binding molecule, wherein the method comprises the following steps (a) to (c):

(a) contacting an antigen-binding molecule comprising an antigen-binding domain with an antigen in the presence of a first concentration of MTA,
(b) placing the antigen-binding molecule comprising the antigen-binding domain bound in step (a) in a condition where a second concentration of MTA is present, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (a) to (d);

(a) contacting an antigen-binding molecule comprising an antigen-binding domain with an antigen in the presence of a first concentration of MTA,
(b) confirming the binding of the antigen-binding molecule comprising the antigen-binding domain to the antigen in step (a),
(c) placing the antigen-binding molecule comprising the antigen-binding domain that bound to the antigen in a condition where a second concentration of MTA is present, and
(d) isolating the antigen-binding molecule comprising the antigen-binding domain whose antigen-binding activity in step (c) is weaker than the standard used for confirming the binding to the antigen in step (b).

In one non-limiting embodiment, the present disclosure provides a method of screening for an antigen-binding molecule, wherein the method comprises the following steps (a) to (c):

(a) contacting an antigen-binding molecule comprising an antigen-binding domain capable of binding to MTA with an antigen in the presence of a first concentration of MTA,
(b) placing the antigen-binding molecule comprising the antigen-binding domain bound in step (a) in a condition where a second concentration of MTA is present, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (a) to (d):

(a) contacting an antigen-binding molecule comprising an antigen-binding domain capable of binding to MTA with an antigen in the presence of a first concentration of MTA,
(b) confirming the binding of the antigen-binding molecule comprising the antigen-binding domain to the antigen in step (a),

(c) placing the antigen-binding molecule comprising the antigen-binding domain bound to the antigen in a condition where a second concentration of MTA is present, and
(d) isolating the antigen-binding molecule comprising the antigen-binding domain whose antigen-binding activity in step (c) is weaker than the standard used for confirming the binding to the antigen in step (b).

In one non-limiting embodiment, the present disclosure provides a method of screening for an antigen-binding molecule, wherein the method comprises the following steps (a) to (d):

(a) contacting an antigen-binding molecule comprising an antigen-binding domain with an antigen in the presence of a first concentration of MTA,
(b) confirming the non-binding of the antigen-binding molecule comprising the antigen-binding domain to the antigen in step (a),
(c) allowing the antigen antigen-binding molecule comprising the antigen-binding domain not bound to the antigen to bind to the antigen in the presence of a second concentration of MTA, and
(d) isolating the antigen-binding molecule comprising the antigen-binding domain that bound to the antigen in step (c).

In one non-limiting embodiment, the present disclosure provides a method of screening for an antigen-binding molecule, wherein the method comprises the following steps (a) to (c):

(a) contacting a library displaying an antigen-binding molecule comprising an antigen-binding domain with an antigen in the presence of a first concentration of MTA.
(b) placing the antigen-binding molecule comprising the antigen-binding domain that bound in step (a) in a condition where a second concentration of MTA is present, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (a) to (d):

(a) contacting a library displaying an antigen-binding molecule comprising an antigen-binding domain with an antigen in the presence of a first concentration of MTA,
(b) selecting the antigen-binding molecule comprising the antigen-binding domain that bound to the antigen in step (a),
(c) placing the antigen-binding molecule comprising the antigen-binding domain selected in step (b) in a condition where a second concentration of MTA is present, and
(d) isolating the antigen-binding molecule comprising the antigen-binding domain whose antigen-binding activity in step (c) is weaker than the standard used for the selection in step (b).

In one non-limiting embodiment, the present disclosure provides a method of screening for an antigen-binding molecule, wherein the method comprises the following steps (1) to (c):

(1) contacting a library displaying an antigen-binding molecule comprising an antigen-binding domain with MTA, and
(2) selecting the antigen-binding molecule comprising the antigen-binding domain that bound to MTA in step (1),

(a) contacting the library displaying the antigen-binding molecule comprising the antigen-binding domain selected in steps (1) and (2) with an antigen in the presence of a first concentration of MTA.
(b) placing the antigen-binding molecule comprising the antigen-binding domain that bound in step (a) in a condition where a second concentration of MTA is present, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (1) to (d):

(1) contacting a library displaying an antigen-binding molecule comprising an antigen-binding domain with MTA, and
(2) selecting the antigen-binding molecule comprising the antigen-binding domain that bound to MTA in step (1),

(a) contacting the library displaying the antigen-binding molecule comprising the antigen-binding domain selected in steps (1) and (2) with an antigen in the presence of a first concentration of MTA,
(b) selecting the antigen-binding molecule comprising the antigen-binding domain that bound to the antigen in step (a),

(c) placing the antigen-binding molecule comprising the antigen-binding domain selected in step (b) in a condition where a second concentration of MTA is present, and
(d) isolating the antigen-binding molecule comprising the antigen-binding domain whose antigen-binding activity in step (c) is weaker than the standard used for the selection in step (b).

In one non-limiting embodiment, the present disclosure provides a method of screening for an antigen-binding molecule, wherein the method comprises the following steps (a) to (d):

(a) contacting a library displaying an antigen-binding molecule comprising an antigen-binding domain with an antigen in the presence of a first concentration of MTA,
(b) selecting the antigen-binding molecule comprising the antigen-binding domain that does not bind to the antigen in step (a),
(c) allowing the antigen-binding molecule comprising the antigen-binding domain selected in step (b) to bind to the antigen in the presence of a second concentration of MTA, and
(d) isolating the antigen-binding molecule comprising the antigen-binding domain that bound to the antigen in step (c).

In one non-limiting embodiment, the present disclosure provides a method of screening for an antigen-binding molecule, wherein the method comprises the following steps (a) to (c):

(a) contacting an antigen-binding molecule comprising an antigen-binding domain with an antigen in the presence of MTA,
(b) placing the antigen-binding molecule comprising the antigen-binding domain bound in step (a) in a condition where MTA is absent, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (a) to (d);

(a) contacting an antigen-binding molecule comprising an antigen-binding domain with an antigen in the presence of MTA,
(b) confirming the binding of the antigen-binding molecule comprising the antigen-binding domain to the antigen in step (a).
(c) placing the antigen-binding molecule comprising the antigen-binding domain that bound to the antigen in a condition where MTA is absent, and
(d) isolating the antigen-binding molecule comprising the antigen-binding domain whose antigen-binding activity in step (c) is weaker than the standard used for confirming the binding to the antigen in step (b).

In one non-limiting embodiment, the present disclosure provides a method of screening for an antigen-binding molecule, wherein the method comprises the following steps (a) to (c):

(a) contacting an antigen-binding molecule comprising an antigen-binding domain capable of binding to MTA with an antigen in the presence of MTA,
(b) placing the antigen-binding molecule comprising the antigen-binding domain bound in step (a) in a condition where MTA is absent, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (a) to (d);

(a) contacting an antigen-binding molecule comprising an antigen-binding domain capable of binding to MTA with an antigen in the presence of MTA,
(b) confirming the binding of the antigen-binding molecule comprising the antigen-binding domain to the antigen in step (a),
(c) placing the antigen-binding molecule comprising the antigen-binding domain bound to the antigen in a condition where MTA is absent, and
(d) isolating the antigen-binding molecule comprising the antigen-binding domain whose antigen-binding activity in step (c) is weaker than the standard used for confirming the binding to the antigen in step (b).

In one non-limiting embodiment, the present disclosure provides a method of screening for an antigen-binding molecule, wherein the method comprises the following steps (a) to (d):

(a) contacting an antigen-binding molecule comprising an antigen-binding domain with an antigen in the presence of MTA,
(b) confirming the non-binding of the antigen-binding molecule comprising the antigen-binding domain to the antigen in step (a).
(c) allowing the antigen-binding molecule comprising the antigen-binding domain that does not bind to the antigen to bind to the antigen in the absence of MTA, and
(d) isolating the antigen-binding molecule comprising the antigen-binding domain that bound to the antigen in step (c).

In one non-limiting embodiment, the present disclosure provides a method of screening for an antigen-binding molecule, wherein the method comprises the following steps (a) to (c):

(a) contacting a library displaying an antigen-binding molecule comprising an antigen-binding domain with an antigen in the presence of MTA,
(b) placing the antigen-binding molecule comprising the antigen-binding domain bound in step (a) in a condition where MTA is absent, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (a) to (d);

(a) contacting a library displaying an antigen-binding molecule comprising an antigen-binding domain with an antigen in the presence of MTA,
(b) selecting the antigen-binding molecule comprising the antigen-binding domain that bound to the antigen in step (a),
(c) placing the antigen-binding molecule comprising the antigen-binding domain selected in step (b) in a condition where MTA is absent, and
(d) isolating the antigen-binding molecule comprising the antigen-binding domain whose antigen-binding activity in step (c) is weaker than the standard used for the selection in step (b).

In one non-limiting embodiment, the present disclosure provides a method of screening for an antigen-binding molecule, wherein the method comprises the following steps (1) to (c):

(1) contacting a library displaying an antigen-binding molecule comprising an antigen-binding domain with MTA, and
(2) selecting the antigen-binding molecule comprising the antigen-binding domain that bound to MTA in step (1),

(a) contacting the library displaying the antigen-binding molecule comprising the antigen-binding domain selected in steps (1) and (2) with an antigen in the presence of MTA,
(b) placing the antigen-binding molecule comprising the antigen-binding domain bound in step (a) in a condition where MTA is absent, and
(c) isolating the antigen-binding molecule comprising the antigen-binding domain that dissociated in step (b).

In one non-limiting embodiment, the present disclosure provides a method of screening for an antigen-binding molecule, wherein the method comprises the following steps (1) to (d):

(1) contacting a library displaying an antigen-binding molecule comprising an antigen-binding domain with MTA, and
(2) selecting the antigen-binding molecule comprising the antigen-binding domain that bound to MTA in step (1),

(a) contacting the library displaying the antigen-binding molecule comprising the antigen-binding domain selected in steps (1) and (2) with an antigen in the presence of MTA,
(b) selecting the antigen-binding molecule comprising the antigen-binding domain that bound to the antigen in step (a),
(c) placing the antigen-binding molecule comprising the antigen-binding domain selected in step (b) in a condition where MTA is absent, and
(d) isolating the antigen-binding molecule comprising the antigen-binding domain whose antigen-binding activity in step (c) is weaker than the standard used for the selection in step (b).

In one non-limiting embodiment, the present disclosure provides a method of screening for an antigen-binding molecule, wherein the method comprises the following steps (a) to (d):

(a) contacting a library displaying an antigen-binding molecule comprising an antigen-binding domain with an antigen in the presence of MTA,
(b) selecting the antigen-binding molecule comprising the antigen-binding domain that does not bind to the antigen in step (a),
(c) allowing the antigen-binding molecule comprising the antigen-binding domain selected in step (b) to bind to the antigen in the absence of MTA, and
(d) isolating the antigen-binding molecule comprising the antigen-binding domain that bound to the antigen in step (c).

As a library displaying an antigen-binding molecule comprising an antigen-binding domain, a naive human antibody display library, a synthetic human antibody display library, or a library completed by the present disclosure described later can be used.
The antigen-binding domains (or antigen-binding molecules containing the domains) of the present disclosure which are to be screened by the aforementioned screening methods may be any antigen-binding domains (or antigen-binding molecules); and for example, the above-mentioned antigen-binding domains (or antigen-binding molecules) can be screened. For example, antigen-binding domains (or antigen-binding molecules) having naturally-occurring sequences can be screened, and antigen-binding domains (or antigen-binding molecules) with substituted amino acid sequences may be screened.

<u>A method of producing an antigen-binding molecule comprising an antigen-binding domain whose antigen-binding activity changes in an MTA-dependent manner</u>

As one aspect of the present disclosure, a method of producing an antigen-binding molecule comprising an antigen-binding domain whose antigen-binding activity changes in an MTA-dependent manner is provided.
An antigen-binding domain whose antigen-binding activity changes in an MTA-dependent manner (or an antigen-binding molecule comprising the domain) can be obtained in accordance with the teachings of the present disclosure. As non-limiting embodiments of the method of acquisition, some specific examples are illustrated below.
For example, in order to confirm that the antigen-binding activity of an antigen-binding domain (or an antigen-binding molecule containing the domain) in the presence of a first concentration of MTA is different from the antigen-binding activity of the antigen-binding domain (or an antigen-binding molecule containing the domain) in the presence of a second concentration of MTA, the antigen-binding activities of the antigen-binding domain (or the antigen-binding molecule containing the domain) in the presence of a first concentration of MTA and a second concentration of MTA are compared. To select an antigen-binding domain whose antigen-binding activity changes in an MTA-dependent manner, an antigen-binding domain (or an antigen-binding molecule containing the domain) whose antigen-binding activity in the presence of the first concentration of MTA is different from that in the presence of the second concentration of MTA is selected. The first concentration and the second concentration are different concentrations, and either one of these concentrations can be set to 0 (that is, MTA is absent).
When the first concentration is set to a concentration higher than the second concentration, the following acquisition method can be exemplified.
In order to obtain an antigen-binding domain (or an antigen-binding molecule containing the domain) whose antigen-binding activity in the presence of a high concentration of MTA is higher than that in the presence of a low concentration of MTA, an antigen-binding domain (or an antigen-binding molecule containing the domain) whose antigen-binding activity in a first concentration is higher than that in a second concentration is selected.
In order to obtain an antigen-binding domain (or an antigen-binding molecule containing the domain) whose antigen-binding activity in the presence of MTA is higher than that in the absence of MTA, the second concentration is set to 0, and an antigen-binding domain (or an antigen-binding molecule containing the domain) whose antigen-binding activity in the first concentration is higher than that in the second concentration is selected.
In order to select an antigen-binding domain whose antigen-binding activity in the presence of a first concentration of MTA is higher than that in the presence of a second concentration of MTA, as long as the antigen-binding activity in the presence of the first concentration of MTA is higher than the antigen-binding activity in presence of the second concentration of MTA, the difference between the antigen-binding activity in the presence of the first concentration of MTA and the antigen-binding activity in the presence of the second concentration of MTA is not particularly limited, but preferably, the antigen-binding activity in the presence of the first concentration of MTA as compared to that in the presence of the second concentration of MTA is 2 times or more, more preferably 10 times or more, and even more preferably 40 times or more. The upper limit of the difference in the antigen-binding activity is not particularly limited, and may be any value such as 400 times, 1000 times, 10000 times, etc. as long as it can be produced within the skill

of a person skilled in the art. If no antigen-binding activity is observed in the presence the a second concentration of MTA, this upper limit is an infinite number.

In order to obtain an antigen-binding domain (or an antigen-binding molecule containing the domain) whose antigen-binding activity in the presence of a high concentration of MTA is lower than that in the presence of a low concentration of MTA, an antigen-binding domain (or an antigen-binding molecule containing the domain) whose antigen-binding activity in the first concentration is lower than that in the second concentration is selected.

In order to obtain an antigen-binding domain (or an antigen-binding molecule containing the domain) whose antigen-binding activity in the presence of MTA is lower than that in the absence MTA, the second concentration is set to 0, and an antigen-binding domain (or an antigen-binding molecule containing the domain) whose antigen-binding activity in the first concentration is lower than that in the second concentration is selected.

In order to select an antigen-binding domain whose antigen-binding activity in the presence of a first concentration of MTA is lower than that in the presence of a second concentration of MTA, as long as the antigen-binding activity in the presence of the first concentration of MTA is lower than the antigen-binding activity in the presence of the second concentration of MTA, the difference between the antigen-binding activity in the presence of the first concentration of MTA and the antigen-binding activity in the presence of the second concentration of MTA is not particularly limited, but preferably, the antigen-binding activity in the presence of the second concentration of MTA as compared to the antigen-binding activity in the presence of the first concentration of MTA is 2 times or more, more preferably 10 times or more, and even more preferably 40 times or more. The upper limit of the difference in the antigen-binding activity is not particularly limited, and may be any value such as 400 times, 1000 times, 10000 times, etc. as long as it can be produced within the skill of a person skilled in the art. If no antigen-binding activity is observed in the presence of the first concentration of MTA, this upper limit is an infinite number.

An antigen-binding molecule comprising an antigen-binding domain whose antigen-binding activity changes in an MTA-dependent manner may be produced by culturing cells introduced with a vector in which a polynucleotide encoding the selected antigen-binding molecule comprising the antigen-binding domain is operably linked and recovering the antigen-binding molecule from the cell culture medium.

Furthermore, in the present disclosure, the phrase "the antigen-binding activity in the presence of MTA is higher than the antigen-binding activity in the absence of MTA" can be alternatively expressed as "the antigen-binding activity of an antigen-binding domain (or an antigen-binding molecule containing the domain) in the absence of MTA is lower than the antigen-binding activity in the presence of MTA". Furthermore, in the present disclosure, "the antigen-binding activity of an antigen-binding domain (or an antigen-binding molecule containing the domain) in the absence of MTA is lower than the antigen-binding activity in the presence of MTA" may be alternatively described as "the antigen-binding activity of an antigen-binding domain (or an antigen-binding molecule containing the domain) in the absence of MTA is weaker than the antigen-binding activity in the presence of MTA".

Furthermore, in the present disclosure, the phrases "the antigen-binding activity in the presence of a first concentration of MTA is higher than the antigen-binding activity in the presence of the second concentration of MTA" and "the first concentration is higher than the second concentration" can be alternatively expressed as "the antigen-binding activity in the presence of a high concentration of MTA is higher than the antigen-binding activity in the presence of a low concentration of MTA" or "the antigen-binding activity of an antigen-binding domain (or an antigen-binding molecule containing the domain) in the presence of a low concentration of MTA is lower than the antigen-binding activity in the presence of a high concentration of MTA". In the present disclosure, "the antigen-binding activity of an antigen-binding domain (or an antigen-binding molecule containing the domain) in the presence of a low concentration of MTA is lower than the antigen-binding activity in the presence of a high concentration of MTA" may be alternatively described as "the antigen-binding activity of an antigen-binding domain (or an antigen-binding molecule containing the domain) in the presence of a low concentration of MTA is weaker than the antigen-binding activity in the presence of a high concentration of MTA".

Conditions when measuring antigen-binding activity other than the concentration of MTA are not particularly limited, and can be selected appropriately by those skilled in the art. For example, it is possible to measure under conditions of HEPES buffer and 37°C. For example, Biacore (GE Healthcare) or such can be used for measurement. When the antigen is a soluble molecule, the activity of an antigen-binding domain (or an antigen-binding molecule containing the domain) to bind to the soluble molecule can be determined by loading the antigen as an analyte onto a chip immobilized with the antigen-binding domain (or an antigen-binding molecule containing the domain). Alternatively, when the antigen is a membrane-type molecule, the binding activity towards the membrane-type molecule can be determined by loading the antigen-binding domain (or an antigen-binding molecule containing the domain) as an analyte onto a chip immobilized with the antigen.

Similar to the above MTA, it is possible to compare the antigen-binding activity of an antigen-binding domain (or an antigen-binding molecule) in the presence of other small molecule compounds including MTA analogs at different small molecule compound concentrations. Specific examples of such small molecule compounds include, but are not limited to, metabolites of the polyamine biosynthesis pathway such as S-adenosylmethionine (SAM: S-adenosylmethionine) and S-adenosylhomocysteine (SAH: S-adenosylhomocysteine, S-(5'-adenosyl)-L-homocysteine) (Stevens et al. (J Chromatogr A. 2010 May 7; 1217 (19): 3282-8)), and AMP, ADP, ATP, adenosine and the like, which are metabolites in the purine nucleotide metabolic pathway.

In one non-limiting embodiment, the present disclosure provides a method of producing an antigen-binding molecule, wherein the method comprises the following steps (a) to (d):

(a) contacting an antigen-binding domain with an antigen in the presence of a first concentration of MTA,
(b) placing the antigen-binding domain bound in step (a) in a condition where a second concentration of MTA is present,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting an antigen-binding domain with an antigen in the presence of a first concentration of MTA,
(b) confirming the binding of the antigen-binding domain to the antigen in step (a),
(c) placing the antigen-binding domain bound to the antigen in a condition where a second concentration of MTA is present,
(d) isolating the antigen-binding domain whose antigen-binding activity in step (c) is weaker than the standard used for confirming the binding to the antigen in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (d):

(a) contacting an antigen-binding domain capable of binding to MTA with an antigen in the presence of a first concentration of MTA,
(b) placing the antigen-binding domain bound in step (a) in a condition where a second concentration of MTA is present,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting an antigen-binding domain capable of binding to MTA with an antigen in the presence of a first concentration of MTA,
(b) confirming the binding of the antigen-binding domain to the antigen in step (a),
(c) placing the antigen-binding domain bound to the antigen in a condition where a second concentration of MTA is present,
(d) isolating the antigen-binding domain whose antigen-binding activity in step (c) is weaker than the standard used for confirming the binding to the antigen in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting an antigen-binding domain with an antigen in the presence of a first concentration of MTA,
(b) confirming the non-binding of the antigen-binding domain to the antigen in step (a),

(c) allowing the antigen-binding domain that does not bind to the antigen to bind to the antigen in the presence of a second concentration of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (d):

(a) contacting a library displaying an antigen-binding domain with an antigen in the presence of a first concentration of MTA,
(b) placing the antigen-binding domain bound in step (a) in a condition where a second concentration of MTA is present,
(c) isolating an antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting a library displaying an antigen-binding domain with an antigen in the presence of a first concentration of MTA,
(b) selecting the antigen-binding domain that bound to the antigen in step (a),
(c) placing the antigen-binding domain selected in step (b) in a condition where a second concentration of MTA is present,
(d) isolating the antigen-binding domain whose antigen-binding activity in step (c) is weaker than the standard used for the selection in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (1) to (d):

(1) contacting a library displaying an antigen-binding domain with MTA, and
(2) selecting the antigen-binding domain bound to MTA in step (1),

(a) contacting the library displaying the antigen-binding domain selected in steps (1) and (2) with an antigen in the presence of a first concentration of MTA,
(b) placing the antigen-binding domain bound in step (a) in a condition where a second concentration of MTA is present,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (1) to (e):

(1) contacting a library displaying an antigen-binding domain with MTA, and
(2) selecting the antigen-binding domain that bound to MTA in step (1),

(a) contacting a library displaying the antigen-binding domain selected in steps (1) and (2) with an antigen in the presence of a first concentration of MTA,
(b) selecting the antigen-binding domain that bound to the antigen in step (a),
(c) placing the antigen-binding domain selected in step (b) in a condition where a second concentration of MTA

is present,
(d) isolating the antigen-binding domain whose antigen-binding activity in step (c) is weaker than the standard used for the selection in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting a library displaying an antigen-binding domain with an antigen in the presence of a first concentration of MTA,
(b) selecting the antigen-binding domain that does not bind to the antigen in step (a),
(c) allowing the antigen-binding domain selected in step (b) to bind to the antigen in the presence of a second concentration of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (d):

(a) contacting an antigen-binding domain with an antigen in the presence of MTA,
(b) placing the antigen-binding domain bound in step (a) in a condition where MTA is absent,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting an antigen-binding domain with an antigen in the presence of MTA,
(b) confirming the binding of the antigen-binding domain to the antigen in step (a),
(c) placing the antigen-binding domain bound to the antigen in a condition where MTA is absent,
(d) isolating the antigen-binding domain whose antigen-binding activity in step (c) is weaker than the standard used for confirming the binding to the antigen in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (d):

(a) contacting an antigen-binding domain capable of binding to MTA with an antigen in the presence of MTA,
(b) placing the antigen-binding domain that bound in step (a) in a condition where MTA is absent,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting an antigen-binding domain capable of binding to MTA with an antigen in the presence of MTA,
(b) confirming the binding of the antigen-binding domain to the antigen in step (a),
(c) placing the antigen-binding domain bound to the antigen in a condition where MTA is absent,

(d) isolating the antigen-binding domain whose antigen-binding activity in step (c) is weaker than the standard used for confirming the binding to the antigen in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting an antigen-binding domain with an antigen in the presence of MTA,
(b) confirming the non-binding of the antigen-binding domain to the antigen in step (a),
(c) allowing the antigen-binding domain that does not bind to the antigen to bind to the antigen in the absence of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (d):

(a) contacting a library displaying an antigen-binding domain with an antigen in the presence of MTA,
(b) placing the antigen-binding domain bound in step (a) in a condition where MTA is absent,
(c) isolating the antigen-binding domain that dissociated in step (b), and
(d) culturing a cell introduced with a vector in which a polynucleotide encoding an antigen-binding molecule comprising the antigen-binding domain isolated in step (c) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting a library displaying an antigen-binding domain with an antigen in the presence of MTA,
(b) selecting the antigen-binding domain that bound to the antigen in step (a),
(c) placing the antigen-binding domain selected in step (b) in a condition where MTA is absent,
(d) isolating the antigen-binding domain whose antigen-binding activity in step (c) is weaker than the standard used for the selection in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (1) to (d):

(1) contacting a library displaying an antigen-binding domain with MTA, and
(2) selecting the antigen-binding domain that bound to MTA in step (1),

> (a) contacting the library displaying the antigen-binding domain selected in steps (1) and (2) with an antigen in the presence of MTA.
> (b) placing the antigen-binding domain bound in step (a) in a condition where MTA is absent,
> (c) isolating the antigen-binding domain that dissociated in step (b), and
> (d) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (1) to (e):

(1) contacting a library displaying an antigen-binding domain with MTA, and
(2) selecting the antigen-binding domain that bound to MTA in step (1),

(a) contacting the library displaying the antigen-binding domain selected in steps (1) and (2) with an antigen in the presence of MTA.
(b) selecting the antigen-binding domain that bound to the antigen in step (a),
(c) placing the antigen-binding domain selected in step (b) in a condition where MTA is absent,
(d) isolating the antigen-binding domain whose antigen-binding activity in step (c) is weaker than the standard used for the selection in step (b), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

In one non-limiting embodiment, the present disclosure provides a method of producing an antigen-binding molecule, wherein the method comprises the following steps (a) to (e):

(a) contacting a library displaying an antigen-binding domain with an antigen in the presence of MTA,
(b) selecting the antigen-binding domain that does not bind to the antigen in step (a),
(c) allowing the antigen-binding domain selected in step (b) to bind to the antigen in the absence of MTA,
(d) isolating the antigen-binding domain that bound to the antigen in step (c), and
(e) culturing a cell introduced with a vector in which a polynucleotide encoding an antigen-binding molecule comprising the antigen-binding domain isolated in step (d) is operably linked, and recovering the antigen-binding molecule comprising the antigen-binding domain from the cell culture medium.

A naive human antibody display library, a synthetic human antibody display library, or a library completed by the present disclosure described later can be used as a library displaying an antigen-binding domain.

As noted in the Examples described later, when an antigen-binding domain isolated in a method of production of the present disclosure is selected from a library, a polynucleotide encoding the antigen-binding domain can be isolated from a virus such as a phage by ordinary gene amplification. When the antigen-binding domain or antibody thus isolated is selected from the culture medium of cells such as hybridomas, the antibody gene or the like is isolated from the cells as shown in the above section on antibodies, by ordinary gene amplification.

An antigen-binding molecule of the present disclosure produced by the above-mentioned production method may be any antigen-binding molecule, for example, an antigen-binding molecule having a native sequence may be produced, or an antigen-binding molecule in which the amino acid sequence is substituted may also be produced.

Libraries

As used herein, the term "library" refers to antigen-binding molecules comprising a plurality of antigen-binding domains having sequences different from each other, and/or to nucleic acids or polynucleotides encoding antigen-binding molecules comprising a plurality of antigen-binding domains having sequences different from each other. The antigen-binding molecules comprising the antigen-binding domains contained in the library, and/or nucleic acids encoding the antigen-binding molecules comprising the antigen-binding domains do not have uniform sequences, but are a plurality of antigen-binding molecules having sequences different from each other and/or nucleic acids encoding a plurality of antigen-binding molecules having sequences different from each other.

According to one embodiment, the antigen-binding domain of the present disclosure (or an antigen-binding molecule comprising the domain) can be obtained from a library consisting mainly of a plurality of antigen-binding molecules having sequences different from one another in which the antigen-binding domain comprises at least one amino acid residue that changes the antigen-binding activity in an MTA-dependent manner, and/or nucleic acids encoding antigen-binding molecules comprising a plurality of antigen-binding domains having sequences different from one another. The following are examples of libraries consisting mainly of a plurality of antigen-binding molecules having sequences different from one another in which the antigen-binding domain comprises at least one amino acid residue that changes the antigen-binding activity in an MTA-dependent manner, and/or nucleic acids encoding antigen-binding molecules comprising a plurality of antigen-binding domains having sequences different from one another.

An antigen-binding molecule library comprising antigen-binding domains having an amino acid residue that changes the antigen-binding activity in an MTA-dependent manner allows to efficiently yield an antigen-binding domain whose antigen-binding activity changes in an MTA-dependent manner and an antigen-binding molecule comprising such an antigen-binding domain. As an embodiment of a "library" of the present specification, the present disclosure not only provides: a library that allows efficient yield of antigen-binding molecules comprising an MTA switch antigen-binding domain, in other words an antigen-binding domain that binds to a target antigen in the presence of MTA, but that does not bind to the target antigen in the absence of MTA, or antigen binding molecules comprising an antigen binding domain that binds to the target antigen in the presence of a high concentration of MTA but that does not bind to the target antigen

in the presence of a low concentration of MTA; but also provides a library that allows efficient yield of antigen-binding molecules comprising an MTA reverse switch antigen-binding domain, in other words an antigen-binding domain that binds to a target antigen in the absence of MTA, but that does not bind to the target antigen in the presence of MTA, and antigen-binding molecules comprising an antigen-binding domain that binds to the target antigen in the presence of a low concentration of MTA, but that does not bind to the target antigen in the presence of a high concentration of MTA.

In one embodiment of the present disclosure, a fusion polypeptide of the antigen-binding molecule of the present disclosure and a heterologous polypeptide can be prepared. In a certain embodiment, the fusion polypeptide can be formed by fusion with at least a portion of a viral coat protein selected from the group consisting of, for example, viral coat proteins pIII, pVIII, pVII, pIX, Soc, Hoc, gpD, and pVI, and mutants thereof.

In one embodiment, the antigen-binding molecule of the present disclosure may be ScFv, a Fab fragment, $F(ab)_2$, or $F(ab')_2$. Therefore, in another embodiment, the present invention provides a library that comprises mainly a plurality of fusion polypeptides having different sequences from one another, in which the fusion polypeptides are formed by fusing these antigen-binding molecules with a heterologous polypeptide. Specifically, the present invention provides a library that comprises mainly a plurality of fusion polypeptides having different sequences from one another, in which the fusion polypeptides are formed by fusing these antigen-binding molecules with at least a portion of a viral coat protein selected from the group consisting of, for example, viral coat proteins pIII, pVIII, pVII, pIX, Soc, Hoc, gpD, and pVI, and mutants thereof. The antigen-binding molecule of the present disclosure may further comprise a dimerization domain. In one embodiment, the dimerization domain can be located between the heavy or light chain variable region of the antibody and at least a portion of the viral coat protein. This dimerization domain may comprise at least one dimerization sequence and/or one or more sequences comprising cysteine residue(s). This dimerization domain may be preferably linked to the C terminus of the heavy chain variable region or constant region. The dimerization domain can assume various structures, depending on whether the antibody variable region is prepared as a fusion polypeptide component with the viral coat protein component (an amber stop codon following the dimerization domain is absent) or depending on whether the antibody variable region is prepared predominantly without containing the viral coat protein component (e.g., an amber stop codon following the dimerization domain is present). When the antibody variable region is prepared predominantly as a fusion polypeptide with the viral coat protein component, bivalent display is achieved by one or more disulfide bonds and/or a single dimerization sequence.

A non-limiting example of an embodiment of a library of the present disclosure includes a library having a diversity of $1.2 \times 10^8$ or more, that is, a library comprising $1.2 \times 10^8$ or more antigen-binding molecules comprising a plurality of antigen-binding domains having sequences different from one another, or nucleic acids encoding antigen-binding molecules comprising a plurality of antigen-binding domains having sequences different from one another.

Herein, the phrase "sequences are different from one another" in the expression "a plurality of antigen-binding molecules comprising an antigen-binding domain whose sequences are different from one another" means that the sequences of antigen-binding molecules in a library are different from one another. Specifically, in a library, the number of sequences different from one another reflects the number of independent clones with different sequences, and may also be referred to as "library size". The library size of a conventional phage display library ranges from $10^6$ to $10^{12}$. The library size can be increased up to $10^{14}$ by the use of known techniques such as ribosome display. However, the actual number of phage particles used in panning selection of a phage library is in general 10 to 10,000 times greater than the library size. This excess multiplicity is also referred to as "the number of library equivalents", and means that there are 10 to 10,000 individual clones that have the same amino acid sequence. Thus, in the present disclosure, the phrase "sequences are different from one another" means that the sequences of independent antigen-binding molecules in a library, excluding library equivalents, are different from one another. More specifically, the above means that there are $10^6$ to $10^{14}$ antigen-binding molecules whose sequences are different from one another, preferably $10^7$ to $10^{12}$ molecules, more preferably $10^8$ to $10^{11}$ molecules, and particularly preferably $10^8$ to $10^{10}$ molecules whose sequences are different from one another.

Herein, the phrase "a plurality of' in the expression "a library mainly consisting of antigen-binding molecules comprising a plurality of antigen-binding domains having sequences different from one another, and/or nucleic acids encoding antigen-binding molecules comprising a plurality of antigen-binding domains having sequences different from one another" refers usually to a population of two or more types of substances of, for example, antigen-binding molecules, fusion polypeptides, polynucleotide molecules, vectors, or viruses of the present disclosure. For example, when two or more substances are different from one another with regard to a particular characteristic, this means that there are two or more types of substances. Examples may include mutants in which an amino acid mutation is observed at a specific amino acid site in an amino acid sequence. For example, when there are two or more antigen-binding molecules having substantially the same, preferably the same sequence, except for an amino acid at a specific amino acid site, there are a plurality of antigen-binding molecules. In another example, a plurality of polynucleotide molecules exist, if there are two or more polynucleotide molecules that are substantially the same, preferably the same sequence, except for a base coding for an amino acid at a particular amino acid site.

Furthermore, as used herein, the phrase "consisting mainly of' in the expression "a library mainly consisting of

antigen-binding molecules comprising a plurality of antigen-binding domains having sequences different from one another, and/or nucleic acids encoding antigen-binding molecules comprising a plurality of antigen-binding domains having sequences different from one another" reflects the number of antigen-binding molecules that differ in their antigen-binding activity in an MTA-dependent manner, out of the number of independent clones with different sequences in the library. Specifically, it is preferable that at least $10^4$ antigen-binding molecules having such binding activity are present in the library. More preferably, antigen-binding domains of the present disclosure can be obtained from a library containing at least $10^5$ antigen-binding molecules having such binding activity. Still more preferably, antigen-binding domains of the present disclosure can be obtained from a library containing at least $10^6$ antigen-binding molecules having such binding activity. Particularly preferably, antigen-binding domains of the present disclosure can be obtained from a library containing at least $10^7$ antigen-binding molecules having such binding activity. Yet more preferably, antigen-binding domains of the present disclosure can be obtained from a library containing at least $10^8$ antigen-binding molecules having such binding activity. Alternatively, this may also be preferably expressed as the ratio of the number of antigen-binding molecules in which antigen-binding activity of the antigen-binding domain varies in an MTA dependent manner with respect to the number of independent clones having different sequences in a library. Specifically, antigen-binding domains of the present disclosure can be obtained from a library in which antigen-binding molecules having such binding activity account for $10^{-6}$% to 80%, preferably $10^{-5}$% to 60%, more preferably $10^{-4}$% to 40% of independent clones with different sequences in the library. In the case of fusion polypeptides, polynucleotide molecules, or vectors, similar expressions may be possible using the number of molecules or the ratio to the total number of molecules. In the case of viruses, similar expressions may also be possible using the number of virions or the ratio to total number of virions. As a non-limiting embodiment of the present disclosure, when a plurality of antigen-binding molecules bind to a single type of antigen, preferably, at least 10, 100, 1000, $10^4$, $10^5$, $10^6$, $10^7$, or $10^8$ molecules are present in a library of antigen-binding molecules showing such binding activity. More preferably, antigen-binding domains of the present disclosure may be obtained from a library in which at least ten antigen-binding molecules showing such binding activity are present. More preferably, the antigen-binding domains of the present disclosure may be obtained from a library in which at least 100 antigen-binding molecules showing such binding activity are present. Particularly preferably, the antigen-binding domains of the present disclosure may be obtained from a library in which at least 1000 antigen-binding molecules showing such binding activity are present.

In one aspect, the present disclosure provides a library comprising a plurality of antibody variable regions and/or nucleic acids encoding the antibody variable regions.

In one non-limiting embodiment, the plurality of antibody variable regions contained in the library of the present disclosure, or encoded by the nucleic acids contained in the library of the present disclosure, are antibody variable regions comprising:

H-CDR1 comprising XAXWMC (SEQ ID NO: 65);
H-CDR2 comprising CIFAXXXYXXSGGSTYYASWAKG (SEQ ID NO: 66);
H-CDR3 comprising GXGXXXGXXDEL (SEQ ID NO: 67);
L-CDR1 comprising QSSEXVXXXXLS (SEQ ID NO: 68);
L-CDR2 comprising XAXTXPX (SEQ ID NO: 69); and
L-CDR3 comprising AGLYXGNIPA (SEQ ID NO: 70).

Here, X refers to an arbitrary amino acid, and X existing at different positions may or may not be an amino acid of the same type.

In one non-limiting embodiment, the plurality of antibody variable regions contained in the library of the present disclosure, or encoded by the nucleic acids contained in the library of the present disclosure, are antibody variable regions comprising:

H-CDR1 comprising XAXWMC (SEQ ID NO: 65);
H-CDR2 comprising CIFA$X_1$XXYXXSGGSTYYASWAKG (SEQ ID NO: 71);
H-CDR3 comprising GXGXXXGXXDEL (SEQ ID NO: 67);
L-CDR1 comprising QSSEXVXXX$X_1$XLS (SEQ ID NO: 72);
L-CDR2 comprising XAXTXPX (SEQ ID NO: 69); and
L-CDR3 comprising AGLYXGNIPA (SEQ ID NO: 70).

Here, X is an arbitrary amino acid, and $X_1$ is an amino acid selected from A, D, E, G, H, I, K, L, M, N, P, Q, R, S, T, and V. Further, X or $X_1$ existing at different positions may or may not be an amino acid of the same type.

In one non-limiting embodiment, the plurality of antibody variable regions contained in the library of the present disclosure, or encoded by the nucleic acids contained in the library of the present disclosure, are antibody variable regions comprising:

H-CDR1 comprising XXAXWMC (SEQ ID NO: 73);
H-CDR2 comprising CIFAX$_1$XXYXXSGGSTYYASWAKG (SEQ ID NO: 71);
H-CDR3 comprising GXGXXXGXXDEL (SEQ ID NO: 67);
L-CDR1 comprising QSSEXVXXX$_1$XLS (SEQ ID NO: 72);
L-CDR2 comprising XAXTXPX (SEQ ID NO: 69); and
L-CDR3 comprising AGLYXGNIPA (SEQ ID NO: 70).

Here, X is an arbitrary amino acid, and X$_1$ is an amino acid selected from A, D, E, G, H, I, K, L, M, N, P, Q, R, S, T, and V. Further, X or X$_1$ existing at different positions may or may not be the an amino acid of the same type.

In one non-limiting embodiment, the plurality of antibody variable regions contained in the library of the present disclosure, or encoded by the nucleic acids contained in the library of the present disclosure, are antibody variable regions comprising:

H-CDR1 comprising XXXWMC (SEQ ID NO: 74);
H-CDR2 comprising CIXSXXXXTXYASWVNG (SEQ ID NO: 75);
H-CDR3 comprising EXXXXSGALNL (SEQ ID NO: 76);
L-CDR1 comprising HSSKXVXXXXXLA (SEQ ID NO: 77);
L-CDR2 comprising XAXXLAS (SEQ ID NO: 78); and
L-CDR3 comprising QGTYXXXXFYFA (SEQ ID NO: 79).

Here, X refers to an arbitrary amino acid, and X existing at different positions may or may not be an amino acid of the same type.

In one non-limiting embodiment, the plurality of antibody variable regions contained in the library of the present disclosure, or encoded by the nucleic acids contained in the library of the present disclosure are, antibody variable regions comprising:

H-CDR1 comprising $X_2X_3X_4X_5$G (SEQ ID NO: 80);
H-CDR2 comprising $X_6$IG$X_7X_8X_9X_{10}X_{11}$W$X_{12}$P$X_{13}$WVK$X_{14}$ (SEQ ID NO: 81);
H-CDR3 comprising G$X_{15}X_{16}X_{17}X_{18}X_{19}X_{20}$NA$X_{21}$DP (SEQ ID NO: 82);
L-CDR1 comprising QSSQSV$X_{22}X_{23}$NN$X_{24}$LS (SEQ ID NO: 83);
L-CDR2 comprising DASTLAS (SEQ ID NO: 84); and
L-CDR3 comprising HG$X_{25}X_{26}X_{27}X_{28}X_{29}X_{30}X_{31}X_{32}$DN$X_{33}$ (SEQ ID NO: 85);

wherein,

$X_2$ is an amino acid selected from A, D, E, F, G, H, I, K, L, N, Q, R, S, T, V, W, and Y,
$X_3$ is an amino acid selected from D, E, F, H, K, N, P, R, and Y,
$X_4$ is an amino acid selected from A, I, P, T, and V,
$X_5$ is an amino acid selected from A, E, F, H, I, K, L, M, N, Q, S, T, V, W, and Y,
$X_6$ is an amino acid selected from D, I, and V,
$X_7$ is an amino acid selected from A, D, E, G, I, K, Q, and R,
$X_8$ is an amino acid selected from D, E, F, G, H, I, K, L, P, Q, R, S, T, V, W, and Y,
$X_9$ is an amino acid selected from A, D, E, F, G, H, and S,
$X_{10}$ is an amino acid selected from A, D, E, F, G, H, I, K, L, N, Q, R, S, T, V, W, and Y,
$X_{11}$ is an amino acid selected from A, D, E, G, H, I, K, L, N, P, Q, R, S, T, and V,
$X_{12}$ is an amino acid selected from A, D, E, F, G, H, I, K, L, Q, R, S, T, V, W, and Y,
$X_{13}$ is an amino acid selected from A, F, Q, R, S, T, V, W, and Y,
$X_{14}$ is an amino acid selected from A, F, and G,
$X_{15}$ is an amino acid selected from A, E, F, G, H, K, L, Q, R, S, T, W, and Y,
$X_{16}$ is an amino acid selected from F, H, K, N, W, and Y,
$X_{17}$ is an amino acid selected from A, D, E, F, G, H, I, K, L, N, P, Q, R, S, T, V, W, and Y,
$X_{18}$ is an amino acid selected from A, D, E, G, H, Q, and S,
$X_{19}$ is an amino acid selected from F and Y,
$X_{20}$ is an amino acid selected from N, T, and V,
$X_{21}$ is an amino acid selected from F and W,
$X_{22}$ is an amino acid selected from A, E, F, H, I, K, L, N, R, S, T, V, W, and Y,
$X_{23}$ is an amino acid selected from A, D, E, F, G, H, I, K, L, N, P, Q, R, S, T, V, W, and Y,
$X_{24}$ is an amino acid selected from A, E, F, G, H, S, and Y,

$X_{25}$ is an amino acid selected from A, S, and T,
$X_{26}$ is an amino acid selected from A, D, E, F, G, H, I, K, L, M, N, Q, R, S, T, V, W, and Y,
$X_{27}$ is an amino acid selected from A, D, E, F, G, H, L, N, Q, R, S, T, V, and Y,
$X_{28}$ is an amino acid selected from A, E, F, G, H, I, K, L, N, P, Q, R, S, T, V, W, and Y,
$X_{29}$ is an amino acid selected from A, D, E, F, G, H, I, K, L, N, P, Q, R, S, T, V, W, and Y,
$X_{30}$ is an amino acid selected from A, D, E, F, G, H, I, K, L, N, P, Q, R, V, W, and Y,
$X_{31}$ is an amino acid selected from A, D, E, F, G, H, I, K, L, N, P, Q, R, S, T, V, W, and Y,
$X_{32}$ is an amino acid selected from A, F, H, I, K, L, N, P, Q, R, S, T, V, W, and Y, and
$X_{33}$ is an amino acid selected from A and G.

Unmodified antibody variable regions (templates)

In one embodiment of the present disclosure, the antigen-binding domain in a library of antigen-binding molecules comprising antigen-binding domains having an amino acid residue that changes the antigen-binding activity in an MTA-dependent manner is an antibody variable region, and the antigen-binding domain contained in the library, or contained in the "plurality of antigen-binding molecules" encoded by nucleic acids contained in the library, includes a plurality of antibody variable region variants with sequences different from one another, and having amino acids different to the amino acids at one or more amino acid sites within an unmodified antibody variable region having MTA binding activity.

In one non-limiting embodiment, the unmodified antibody variable region is an antibody variable region having binding activity to MTA. In one detailed embodiment, the unmodified antibody variable region of the present disclosure may be an antibody variable region that does not substantially bind to adenosine and/or to S-(5'-adenosyl)-L-homocysteine (SAH). In another detailed embodiment, the unmodified antibody variable region of the present disclosure may be an antibody variable region having binding activity to adenosine as well. Further, the unmodified antibody variable region of the present disclosure may be an antibody variable region having binding activity to S-(5'-adenosyl)-L-homocysteine (SAH), AMP, ADP, and/or ATP as well.

The unmodified antibody variable region having binding activity to MTA can be obtained from antibodies having binding activity to MTA.

It can be referred to as a "template", since it can be used as a starting molecule when designing a library of antigen-binding molecules comprising antigen-binding domains having an amino acid residue that changes the antigen-binding activity in an MTA-dependent manner.

In one non-limiting embodiment, the unmodified antibody variable region of the present disclosure may be any of the following:

a) an antibody variable region comprising the heavy chain variable region set forth in SEQ ID NO: 46 and the light chain variable region set forth in SEQ ID NO: 47;
b) an antibody variable region comprising the heavy chain variable region set forth in SEQ ID NO: 50 and the light chain variable region set forth in SEQ ID NO: 51;
c) an antibody variable region comprising the heavy chain variable region set forth in SEQ ID NO: 48 and the light chain variable region set forth in SEQ ID NO: 49;
d) an antibody variable region comprising the heavy chain variable region set forth in SEQ ID NO: 52 and the light chain variable region set forth in SEQ ID NO: 53; or
e) an antibody variable region having the heavy chain variable region set forth in SEQ ID NO: 31 and the light chain variable region set forth in SEQ ID NO: 32.

With respect to methods for obtaining (methods of screening for) starting molecules (templates) to be used when producing a library of the present disclosure, these antigen-binding domains and such may be prepared in any manner. It is possible to use pre-existing antigen-binding domains or antibodies, and pre-existing libraries (phage libraries, etc.), antibodies or libraries prepared from hybridomas obtained by immunizing animals or from B cells of immunized animals, for example, antibodies or libraries prepared from immune cells such as B cells of animals immunized with a conjugate in which a small molecule compound comprising MTA is suitably linked to an adjuvant agent such as a highly immunogenic T cell epitope peptide, without being limited thereto. A non-limiting example of the T cell epitope peptide suitably includes a Tetanus toxin-derived p30 helper peptide (shown in SEQ ID NO: 4, and also referred to as Fragment C (FrC)).

Non-limiting embodiments of an antigen-binding domain of the present invention obtained by the aforementioned screening method include, for example, antigen-binding domains containing antibody heavy-chain and light-chain variable regions. Preferred examples of such antigen-binding domains include "single chain Fv (scFv)", "single chain antibody", "Fv", "single chain Fv 2 (scFv2)", "Fab", "F(ab')2", and IgG, and a library comprising thereof may also be used.

Furthermore, as a non-limiting embodiment of a method for preparing antigen-binding domains or antigen-binding molecules of the present invention obtained by the aforementioned screening method, it is possible to use a technique for preparing antigen-binding domains or antigen-binding molecules having binding activity to a small molecule compound by panning using an above-mentioned library. As a library, it is possible to use, for example, but without being limited thereto, a phage display library, a ribosome display library, an mRNA display library, a cDNA display library, a CIS display library, an *E. coli* display library, a Gram-positive bacterium display library, an yeast display library, a mammalian cell display library, a virus display library, and an *in vitro* virus display library.

In an embodiment of the aforementioned technique for preparing antigen-binding domains or antigen-binding molecules having binding activity to a small molecule compound by panning, small molecule compounds fixed onto a carrier such as beads can be used. The fixed small molecule compounds can be produced by, for example, without being limited thereto, a method of contacting small molecule compounds synthesized to be chemically linked to biotin *via* a linker with beads or a plate onto which streptavidin or NeutrAvidin has been fixed, or a method of adhering the small molecule compounds covalently linked to an adjuvant such as bovine serum albumin (BSA) to beads or plates by hydrophobic interaction. These methods are already publicly known (J. Immunol. Methods. 2003 Sep, 280 (1-2): 139-55; BMC Biotechnol. 2009 Jan 29; 9: 6. doi: 10.1186/1472-6750-9-6). Antigen-binding domains or antigen-binding molecules having binding activity to the small molecule compounds can be prepared by collecting antigen-binding domains or antigen-binding molecules that have binding activity to the fixed small molecule compounds.

Alternatively, in another embodiment of the aforementioned technique for preparing antigen-binding domains or antigen-binding molecules having binding activity to a small molecule compound by panning, a fluorescence-labeled small molecule compound, or a biotin-labeled small molecule compound and fluorescence-labeled streptavidin (or NeutrAvidin or avidin) may be used. Antigen-binding domains or antigen-binding molecules having binding activity to the small molecule compound can be prepared by contacting the fluorescence-labeled small molecule compound, or the biotin-labeled small molecule compound and fluorescence-labeled streptavidin (or NeutrAvidin or avidin), with a library presented on the cell surface or such, and then using the fluorescence-activated cell sorting (FACS) method. These methods are already publicly known (Proc Natl Acad Sci U S A. 2000 Sep 26; 97 (20): 10701-5).

Furthermore, in a non-limiting embodiment of a method for preparing antigen-binding domains or antigen-binding molecules of the present disclosure obtained by the aforementioned screening method, pre-existing antigen-binding domains having binding activity to the small molecule compound may be used. For example, when MTA is used as an example, without being limited thereto, antigen-binding domain having MTA-binding activity, antigen-binding domain whose antigen-binding activity varies in an MTA dependent manner, and antigen-binding domain having amino acid residues that interact with MTA may be used.

## Variants

The term "variant" in the present disclosure means a variant of a parent sequence in which at least one or more amino acids are substituted compared to an unmodified parent sequence.

In one embodiment, to prepare an antibody variable region variant of the present disclosure, a known method such as the site-specific mutagenesis method (Kunkel et al. (Proc. Natl. Acad. Sci. USA (1985) 82, 488-492)) or overlap extension PCR can be appropriately adopted to prepare an antigen-binding molecule contained in the heavy chain variable region or light chain variable region sequence, or the CDR sequence or framework sequence of the unmodified antibody variable region described above.

In one non-limiting embodiment, amino acid sites having amino acids different from an unmodified antibody variable region in the antibody variable region variants contained in the library of the present disclosure are located in the heavy and/or light chain CDR1, CDR2, and/or CDR3. In another non-limiting embodiment, amino acid sites of the antibody variable region variants having an amino acid different from that of the unmodified antibody variable region are located in the heavy and/or light chain FR1, FR2, FR3 and/or FR4.

Any framework sequence can be used as the framework sequence of the light-chain and/or heavy-chain variable regions of an antigen-binding molecule as long as the amino acids that interact with MTA are contained in the antigen-binding domain of the heavy chain and/or light chain. The origin of the framework sequences is not limited, and they may be obtained from human or any nonhuman organisms. Such organisms preferably include mice, rats, guinea pigs, hamsters, gerbils, cats, rabbits, dogs, goats, sheep, bovines, horses, camels and organisms selected from nonhuman primates. In a particularly preferred embodiment, the framework sequences of the light chain and/or heavy chain variable region of an antigen-binding molecule preferably have human germ-line framework sequences. Thus, in an embodiment of the present disclosure, if the entire framework sequences are human sequences, it is thought that an antigen-binding molecule of the present disclosure induces little or no immunogenic response when it is administered to humans (for example, to treat diseases). In the above sense, the phrase "containing a germ line sequence" in the present disclosure means that a part of the framework sequences of the present disclosure is identical to a part of any human germ line framework sequences. Specifically, the framework sequence of the present disclosure is at least 50% or more, 60% or

more, 70% or more, 80% or more, 90% or more, or 100% or more identical to the germ line sequence. For example, when the heavy chain FR2 sequence of an antigen-binding molecule of the present disclosure is a combination of heavy chain FR2 sequences of different human germ line framework sequences, such a molecule is also an antigen-binding molecule "containing a germ line sequence" in the present disclosure. Even when the framework sequences of antigen-binding molecules of the present disclosure are sequences with substitutions, they are antigen-binding molecules "containing a germ line sequence" of the present disclosure. Examples of such sequences with substitutions include, in particular, sequences in which amino acids of part of human germ line framework sequences have been substituted with amino acids that change the antigen-binding activity of the antigen-binding molecule depending on the presence or absence of MTA and/or adenosine.

Preferred examples of the frameworks include, for example, fully human framework region sequences currently known, which are included in the website of V-Base (http://vbase.mrc-cpe.cam.ac.uk/) or others. Those framework region sequences can be appropriately used as a germ line sequence contained in an antigen-binding molecule of the present disclosure. The germ line sequences may be categorized according to their similarity (Tomlinson et al. (J. Mol. Biol. (1992) 227, 776-798); Williams and Winter (Eur. J. Immunol. (1993) 23, 1456-1461); Cox et al. (Nat. Genetics (1994) 7, 162-168)). Appropriate germ line sequences can be selected from Vκ, which is grouped into seven subgroups; Vλ, which is grouped into ten subgroups; and VH, which is grouped into seven subgroups.

Fully human VH sequences preferably include, but are not limited to, for example, VH sequences of:

subgroup VH1 (for example, VH1-2, VH1-3, VH1-8, VH1-18, VH1-24, VH1-45, VH1-46, VH1-58, and VH1-69);
subgroup VH2 (for example, VH2-5, VH2-26, and VH2-70);
subgroup VH3 (VH3-7, VH3-9, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-72, VH3-73, and VH3-74);
subgroup VH4 (VH4-4, VH4-28, VH4-31, VH4-34, VH4-39, VH4-59, and VH4-61);
subgroup VH5 (VH5-51);
subgroup VH6 (VH6-1); and
subgroup VH7 (VH7-4 and VH7-81).

These are also described in known documents (Matsuda et al. (J. Exp. Med. (1998) 188, 1973-1975)) and such, and thus persons skilled in the art can appropriately design antigen-binding molecules of the present disclosure based on the information of these sequences. It is also preferable to use other fully human frameworks or framework sub-regions.

Fully human Vκ sequences preferably include, but are not limited to, for example:

A20, A30, L1, L4, L5, L8, L9, L11, L12, L14, L15, L18, L19, L22, L23, L24, O2, O4, O8, 012, 014, and 018 grouped into subgroup Vk1;
A1, A2, A3, A5, A7, A17, A18, A19, A23, O1, and O11, grouped into subgroup Vk2;
A11, A27, L2, L6, L10, L16, L20, and L25, grouped into subgroup Vk3;
B3, grouped into subgroup Vk4;
B2 (herein also referred to as Vk5-2), grouped into subgroup Vk5; and
A10, A14, and A26, grouped into subgroup Vk6
(Kawasaki et al. (Eur. J. Immunol. (2001) 31, 1017-1028); Schable and Zachau (Biol. Chem. Hoppe Seyler (1993) 374, 1001-1022); Brensing-Kuppers et al. (Gene (1997) 191, 173-181)).

Fully human Vλ sequences preferably include, but are not limited to, for example:

VI-2, V1-3, VI-4, V1-5, VI-7, V1-9, V1-11, V1-13, V1-16, V1-17, V1-18, V1-19, VI-20, and VI-22, grouped into subgroup VL1;
V2-1, V2-6, V2-7, V2-8, V2-11, V2-13, V2-14, V2-15, V2-17, and V2-19, grouped into subgroup VL1;
V3-2, V3-3, and V3-4, grouped into subgroup VL3;
V4-1, V4-2, V4-3, V4-4, and V4-6, grouped into subgroup VL4; and
V5-1, V5-2, V5-4, and V5-6, grouped into subgroup VL5 (Kawasaki et al. (Genome Res. (1997) 7, 250-261)).

Normally, these framework sequences are different from one another at one or more amino acid residues. Examples of the fully human frameworks used in the present disclosure include, but are not limited to, for example, KOL, NEWM, REI, EU, TUR, TEI, LAY, and POM (for example, Kabat *et al.* (1991) *supra;* Wu et al. (J. Exp. Med. (1970) 132, 211-250)).

Without being bound by a particular theory, one reason for the expectation that the use of germ line sequences precludes adverse immune responses in most individuals is believed to be as follows. As a result of the process of

affinity maturation during normal immune responses, somatic mutation occurs frequently in the variable regions of immunoglobulin. Such mutations mostly occur around CDRs whose sequences are hypervariable, but also affect residues of framework regions. Such framework mutations do not exist on the germ line genes, and also they are less likely to be immunogenic in patients. On the other hand, the normal human population is exposed to most of the framework sequences expressed from the germ line genes. As a result of immunotolerance, these germ line frameworks are expected to have low or no immunogenicity in patients. To maximize the possibility of immunotolerance, variable region-encoding genes may be selected from a group of commonly occurring functional germ line genes.

Furthermore, in a non-limiting embodiment of the present disclosure, amino acids of the variable region including the CDR region and/or the framework region may be altered appropriately to improve stability of the antigen-binding molecule. In a non-limiting embodiment, examples of such amino acids may include the amino acids of positions 1, 5, 10, 30, 48, and 58. More specifically, examples may include Gln at position 1, Gln at position 5, Asp at position 10, Asn at position 30, Leu at position 48, and Asn at position 58. For the improvement of antibody stability, these amino acids can be substituted with corresponding amino acids contained in a germ-line sequence. In a non-limiting embodiment, an example of such a germ line sequence may be the VH3-21 sequence. In this case, Gln of position 1 may be substituted with Glu, Gln of position 5 may be substituted with Val, Asp of position 10 may be substituted with Gly, Asn of position 30 may be substituted with Ser, Leu of position 48 may be substituted with Val, and Asn of position 58 may be substituted with Tyr.

Amino acid modification sites in the variants

In one non-limiting embodiment, amino acid sites of the antigen-binding domain variant having an amino acid different from that of the unmodified antigen-binding domain are one or more amino acid sites selected from the group of amino acid sites below:

1) an amino acid site corresponding to an amino acid site in the unmodified antigen-binding domain that is not involved in the binding to MTA,
2) an amino acid site that does not significantly weaken the binding of the antigen-binding domain variant to MTA as compared to the antigen-binding domain variant, and
3) an amino acid site that is likely to contribute to MTA-dependent binding of the antigen-binding domain variant to the antigen.

In one non-limiting embodiment, amino acid sites of the antigen-binding domain variant having an amino acid different from that of an unmodified antigen-binding domain are one or more amino acid sites selected from the group of amino acid sites below:

1) an amino acid site corresponding to an amino acid site in the unmodified antigen-binding domain that is not involved in the binding to MTA,
2) an amino acid site that does not significantly weaken the binding of the antigen-binding domain variant to MTA as compared to the antigen-binding domain variant.
3) an amino acid site corresponding to an amino acid site that is exposed on the surface of the unmodified antigen-binding domain, and
4) an amino acid site corresponding to an amino acid site located in a region where the rate of structural change is large at the time of MTA binding/non-binding in the unmodified antigen-binding domain.

Amino acid sites that can be diversified

Amino acid sites in the unmodified antigen-binding domain whose sequence has amino acids different from the sequence of the antigen-binding domain variant are sites that can be designed to contain diverse amino acids when designing a library, and in the present disclosure, they are also referred to as "diversifiable amino acid sites". Diversifiable amino acid sites can include the following:

(i) an amino acid site that is exposed on the surface of an antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs;

(vi) an amino acid site that is not important for the formation of a canonical structure; and
(vii) an amino acid site that is likely to contribute to the MTA-dependent binding of the antigen-binding domain variant to the antigen.

Amino acid sites not involved in the binding to MTA

"An amino acid site that is not involved in the binding to MTA" in the present disclosure can be identified by a method such as crystal structure analysis of a complex of MTA and antigen-binding molecule, three-dimensional structure analysis using NMR, or introduction of an amino acid mutation. As a non-limiting embodiment of the present disclosure, from the crystal structure analysis of the complex of MTA and antigen-binding molecule, amino acid residues of the antigen-binding molecule that are not involved in the binding to MTA, particularly amino acid residues in the antigen-binding domain that are not involved in the binding to MTA, can be identified. Here, "involved in the binding to MTA" means, when the antigen-binding domain is an antibody variable region, a condition where intermolecular interaction is formed between the atoms of MTA and the atoms of the main chain or side chains of the amino acids forming the H chain or L chain of an antibody variable region, at a distance that may have an effect on the binding activity; or a condition where certain amino acid residues are involved in the MTA binding, including an indirect effect of stabilizing the three-dimensional structure of the CDR loop and such to the conformation when bound to MTA; and a condition that satisfies both of these conditions.
In another embodiment, the amino acid site that is not involved in the binding to MTA can also be determined as an amino acid site other than any one or more amino acid sites that are selected from among amino acid sites that are involved in the binding to MTA.
When the antigen-binding domain is an antibody variable region, the "condition where intermolecular interaction is formed" herein can be determined, for example based on the interatomic distances between non-hydrogen atoms constituting the main chain or side chains of the amino acids that form the antibody variable region H and L chains and the non-hydrogen atoms constituting MTA, obtained from a crystal structure analysis of the complex formed by MTA and an antigen-binding molecule. For example, the above-mentioned interatomic distances are preferably 3.0 Å, 3.2 Å, 3.4 Å, 3.6 Å, 3.8 Å, 4.0 Å, 4.2 Å, 4.4 Å, 4.6 Å, 4.8 Å, or 5.0 Å or less, but are not limited thereto. More preferably, the interatomic distances are 3.6 Å, 3.8 Å, 4.0 Å, or 4.2 Å or less.
More specifically, the possibility of a direct interaction can be determined based on information on the interatomic distances in the three-dimensional structure and the types of intermolecular interactions formed, and information on the types of atoms. The determination can be done with more accuracy by, without restriction, observing the effect of introducing amino acid residue mutations, such as modifications to Ala or Gly, on the activity of MTA.
Also with respect to the "indirectly influencing condition" in the present specification, whether there is an indirect influence on MTA binding can be estimated, for example, by analyzing in detail the state of the conformation of amino acid residues and intermolecular interactions with surrounding residues from a three-dimensional structure of a complex formed between MTA and an antigen-binding molecule. The determination can be done more accurately by observing the effect of introducing amino acid residue mutations, such as modifications to Ala or Gly, on the activity of MTA.
As one embodiment of the present disclosure, it is possible to select an amino acid that can maintain the binding to MTA to an appropriate degree even if a residue identified as not being involved in the binding to MTA is replaced with another amino acid. In this way, a library can be designed so that the selected amino acid appears at a selected residue. In this case, it is possible to design a library consisting mainly of a plurality of antigen-binding molecules, so as to make a group of antigen-binding molecules in which the residues identified as not being involved in the binding to MTA are substituted with amino acids different from one another.

Amino acid sites that do not markedly reduce binding with MTA

In a non-limiting embodiment of the present disclosure, "amino acid sites that do not markedly reduce binding with MTA" can be identified by methods of introducing amino acid mutations. For example, amino acids of the variable region are comprehensively modified, and the binding of each variant to MTA is measured by known methods that use Biacore and such. The binding activity (affinity) of each variant to MTA is calculated as a KD value. This KD value is compared with the KD value of an unmodified antigen-binding domain/molecule which is the parent sequence, and the modified positions that show binding greater than a certain standard are determined as "amino acid sites that do not markedly reduce binding with MTA". For example, as a result of performing measurements using known methods such as Biacore, the binding activity (affinity) of the individual variants to MTA is calculated as a KD value; and sites of the heavy chain where alteration does not reduce the binding capacity to MTA to less than 1/100, 1/50, 1/10, 1/9, 1/8, 1/7, 1/6, 1/5, 1/4, 1/3, or 1/2 of the unmodified antigen-binding domain/molecule, and sites of the light chain where alteration does not reduce the binding capacity to MTA to less than 1/100, 1/50, 1/10, 1/9, 1/8, 1/7, 1/6, 1/5, 1/4, 1/3, or 1/2 of the unmodified antigen-binding domain/molecule are determined as amino acid sites that do not markedly reduce binding

with MTA, but the above-mentioned standards are non-limiting. Alternatively, instead of comparing with the KD value of the unmodified antigen-binding domain/molecule which is the parent sequence, the binding activity (affinity) of individual variants to MTA is calculated as a KD value, and heavy chain sites having binding capacity not lower than 10 mM, 1 mM, 100 uM, 10 uM, 1 uM, 100 nM, 10 nM, 1 nM, 100 pM, 10 pM, or 1 pM, and light chain sites having binding capacity not lower than 10 mM, 1 mM, 100 uM, 10 uM, 1 uM, 100 nM, 10 nM, 1 nM, 100 pM, 10 pM, or 1 pM are determined as amino acid sites that do not markedly reduce binding with MTA, but the above-mentioned standards are non-limiting. The binding activity of the unmodified antigen-binding domain/molecule and variants to MTA can be measured by appropriately selecting methods known to those skilled in the art (Biacore, ELISA, ECL, and such).

In another embodiment, amino acid sites that do not markedly reduce binding with MTA can be considered as amino acid sites other than any one or more amino acid sites selected from among the amino acid sites involved in binding to MTA.

Amino acid sites that are likely to contribute to MTA-dependent binding

"Amino acid sites that are likely to contribute to MTA-dependent binding" in the present disclosure can be identified or estimated by a method such as crystal structure analysis of a complex of MTA and an antigen-binding molecule, three-dimensional structure analysis using NMR, or introduction of an amino acid mutation. An example of a non-limiting method for estimating "amino acid sites that are likely to contribute to MTA-dependent binding" is a method of identifying amino acid sites in which amino acid residues exposed on the surface of antigen-binding molecule proteins are located, or amino acid sites located in a region whose structure changes when MTA is bound compared to when MTA is not bound.

Amino acid sites exposed on the surface of an antigen-binding domain

"Amino acid sites exposed on the surface of an antigen-binding domain" in the present disclosure refers to amino acid sites located on the surface of the three-dimensional structure of a protein. An amino acid residue and amino acid site located on the protein surface of an antigen-binding domain can be identified by a method such as crystal structure analysis or three-dimensional structure analysis using NMR of a single antigen-binding molecule comprising an antigen-binding domain or a complex of MTA and an antigen-binding molecule. An example of a non-limiting method for identifying an amino acid site exposed on the surface of an antigen-binding domain is the method of identifying an amino acid site exposed on the surface by calculating the accessible surface area.

Substitution of amino acids located at amino acid sites exposed on the surface of an antigen-binding domain with various amino acids is expected to confer binding activity to various antigen molecules. This makes it possible to design a library in which diverse amino acids appear at such amino acid sites. In this case, it is possible to design a library mainly consisting of a plurality of antigen-binding molecules so as to be an assembly of antigen-binding molecules having different amino acids at amino acid sites exposed on the surface of antigen-binding domains.

Amino acid sites located in a region of an antigen-binding domain where the rate of structural change is large when MTA is bound/not bound

In the present disclosure, "a region of an antigen-binding domain where the rate of structural change is large when MTA is bound/not bound" can be identified by identifying the region where the structural change occurs by using a technique such as crystal structure analysis or three-dimensional structure analysis using NMR to compare the structure of a single antigen-binding molecule comprising an antigen-binding domain and a complex formed by MTA and an antigen-binding molecule.

In addition, an "amino acid site located in a region of an antigen-binding domain where the rate of structural change is large when MTA is bound/not bound" can also be identified directly by comparing the structure of a single antigen-binding molecule comprising an antigen-binding domain and the structure of a complex formed by MTA and an antigen-binding molecule. When the antigen-binding domain is an antibody variable region, based on the distance between the Cα atoms of amino acid residues at the time of MTA binding/non-binding, which is calculated by superimposing the structure of the antibody H chain and L chain when bound to MTA and the structure of the antibody H chain and L chain when not bound to MTA, it is possible to determine whether an amino acid site where the amino acid residue is located is an "amino acid site located in a region of an antigen-binding domain where the rate of structural change is large when MTA is bound/not bound". For example, an amino acid site at which an amino acid residue is located can be determined to be "an amino acid site located in a region of an antigen-binding domain where the rate of structural change is large when MTA is bound/not bound" when the distance between Cα atoms at the time of MTA binding/non-binding is equal to or greater than the average value of the distance between Cα atoms at the time of MTA binding/non-bonding of all amino acid residues in the variable region, but the present invention is not limited thereto.

It is expected that MTA-dependent antigen-binding activity can be conferred by substituting various amino acids for such an amino acid at an amino acid site located in a region of an antigen-binding domain where the rate of structural change is large when MTA is bound/not bound. This makes it possible to design a library in which diverse amino acids appear at such amino acid sites. In this case, a library mainly consisting of a plurality of antigen-binding molecules can be designed so as to be an assembly of antigen-binding molecules having different amino acids at an amino acid site located in a region of the antigen-binding domain where the rate of structural change is large when MTA is bound/not bound.

Amino acid sites with diverse amino acid occurrence frequencies

"Amino acid sites with diverse amino acid occurrence frequencies" in the present disclosure refers to amino acid sites where two or more types of amino acids are found to be present at an occurrence frequency of 1% or higher in the antibody repertoire of the animal species to which the parent sequence belongs.

In the present disclosure, "an amino acid site with diverse amino acid occurrence frequencies in the animal species to which the antigen-binding domain belongs" refers to an amino acid site where diversity is recognized within the repertoire of antibody gene sequences found on the gene in the animal species from which the relevant unmodified body is derived. As an example, but not limited thereto, when the relevant unmodified body is derived from a human, the antibody repertoire of the animal species to which the unmodified body refers to the repertoire of antibody gene sequences found on the human gene. When the relevant unmodified body is derived from a rabbit, the antibody repertoire of the animal species to which the unmodified body belongs refers to the repertoire of antibody gene sequences found on the rabbit gene. However, even if present on the gene, sequences that are not actually expressed as antibodies due to the presence of stop codons or frameshifts are not included.

When the corresponding unmodified antigen-binding domain/molecule is derived from a non-human animal, it can be humanized according to conventional methods, and such techniques are widely known to those skilled in the art (for example, European patent publication EP239400, international publications WO1996/002576, WO1993/012227, WO1992/003918, WO1994/002602, WO1994/025585, WO1996/034096, WO1996/033735, WO1992/001047, WO1992/020791, WO1993/006213, WO1993/011236, WO1993/019172, WO1995/001438, and WO1995/015388, Cancer Res., (1993) 53, 851-856, and BBRC., (2013) 436(3):543-50). When a corresponding unmodified antigen-binding domain/molecule is humanized according to conventional methods and then made into a library, in the "antibody repertoire of the animal species to which the unmodified antigen-binding domain/molecule belongs" of the present disclosure, the antigen-binding domain prior to humanization and the humanized antigen-binding domain can be both treated as the antigen-binding domain. Accordingly, the human repertoire and the repertoire of the animal species from which the pre-humanization antigen-binding domains are derived can be both applied as a repertoire of the same animal species. Without being limited thereto, as an example, when the antigen-binding domains prior to humanization are derived from rabbits, the antibody repertoire of the animal species to which the unmodified antigen-binding domain/molecule belongs refers to the repertoire of antibody gene sequences found in the genes of humans and/or rabbits. However, it must be noted that sequences that are not actually expressed as antibodies due to frame shift or presence of termination/initiation codons are not included even if they are present in the genes.

As an example, the antibody repertoire of the animal species to which the unmodified antigen-binding domain/molecule belongs can be investigated by referring to a known database, without being limited thereto. The site where there is diversity of the amino acid occurrence frequency is generally in the CDR region. In one embodiment, when determining the hypervariable positions of known and/or naturally-occurring antibodies, the data provided by Kabat, Sequences of Proteins of Immunological Interest (National Institute of Health Bethesda Md., 1987 and 1991) are useful. Furthermore, multiple databases on the Internet (http://vbase.mrc-cpe.cam.ac.uk/ and http://www.bioinf.org.uk/abs/index.html) provide many collected sequences of human light chains and heavy chains, and their locations. Information on the sequences and their locations is useful for determining the hypervariable positions in the present disclosure.

In another embodiment, the antibody repertoire of the animal species to which the unmodified antigen-binding domain/molecule belongs can be examined by cloning antibody genes obtained from the corresponding animal species and analyzing their sequences. Without being limited thereto, as an example, a human antibody repertoire is constructed from antibody genes derived from lymphocytes of healthy individuals and may be examined by analyzing the sequences of a naive library comprising naive sequences which are unbiased antibody sequences in their repertoire (Gejima et al. (Human Antibodies (2002) 11, 121-129); Cardoso et al. (Scand. J. Immunol. (2000) 51, 337-344)). When examining a repertoire, it is desirable to analyze at least 100 types of sequences, preferably 200 types of sequences, and more preferably 400 types of sequences or more.

With respect to "the antibody repertoire of the animal species to which the unmodified antigen-binding domain/molecule belongs" in the present disclosure, more preferably it is desirable to examine subgroups of the germline to which the unmodified antigen-binding domain/molecule belongs, without being limited thereto. Examples of a framework include sequences of currently known completely human-type framework regions listed in a website such as V-Base (http://vbase.mrc-cpe.cam.ac.uk/). Any of the sequences of these framework regions may be appropriately used as a germline sequence contained in the antigen-binding molecule of the present disclosure. The germline sequences may be classified into subgroups based on their similarity (Tomlinson et al., J. Mol. Biol. (1992) 227, 776-798; Williams and Winter, Eur. J. Immunol. (1993) 23, 1456-1461; and Cox et al., Nat. Genetics (1994) 7, 162-168). In one example, seven subgroups for the heavy-chain variable region in human antibodies, seven subgroups for Vκ, and ten types of subgroups for Vλ have been reported; and without being particularly limited to this embodiment, each of the amino acid sites may be examined by analyzing the amino acid repertoire in the subgroup to which the unmodified antigen-binding domain/molecule belongs.

Amino acid sites that are not important for canonical structure formation

In the "amino acid sites that are not important for canonical structure formation" of the present disclosure, an antibody canonical structure shows clustering of the three-dimensional structures of mainly CDR1 and CDR2 of the antibody heavy chains and light chains, and the structures can be classified according to the antibody subgroups and the length or sequence of CDRs. In each canonical structure, residues important for maintaining the structure are already known, and by referring to the reports of Chothia et al. (J. Mol. Biol. (1992) 227, 799-817), Al-Lazikani et al. (J. Mol. Biol. (1997) 273, 927-948), Tomlinson et al. (J. Mol. Biol.(1992) 227, 776-798) and such, it is possible to identify the canonical structure that the corresponding parent antigen-binding molecule is classified to, and the residues important for that structure.

Furthermore, even in antigen-binding domains other than those of antibodies, it is known that there are residues important for maintaining the structure; and while not being limited thereto, amino acid sites not important for formation of the canonical structure in each antigen-binding domain can be identified by structural analysis and such of produced mutants.

Produced libraries

One embodiment of the present disclosure provides a library produced by a method comprising the following steps (a) and (b):

(a) identifying an amino acid site that satisfies at least one or more of the following (i) to (vi) in an antigen-binding domain having MTA-binding activity:

(i) an amino acid site exposed on the surface of the antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs; or
(vi) an amino acid site that is not important for the formation of a canonical structure; and,

(b) designing a library comprising a nucleic acid encoding an unmodified antigen-binding domain, and nucleic acids encoding a plurality of variants of the antigen-binding domain differing in sequence from one another and having amino acid modifications at one or more amino acid sites identified in step (a).

One embodiment of the present disclosure provides a library produced by the following steps (a) and (b):

(a) identifying an amino acid site that satisfies at least one or more of the following (i) to (vi) in an antigen-binding domain having MTA-binding activity:

(i) an amino acid site exposed on the surface of the antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs; or
(vi) an amino acid site that is not important for the formation of a canonical structure; and,

(b) designing a library comprising a nucleic acid encoding an unmodified antigen-binding domain, and nucleic acids encoding a plurality of variants of the antigen-binding domain differing in sequence from one another and having amino acid modifications at one or more amino acid sites identified in step (a),

wherein in the amino acid modifications in step (b) satisfy at least one or more of the following (1) to (3):

(1) when the structure is compared between when the antigen-binding domain variant having the amino acid modification is bound to MTA and when it is not bound to MTA, the rate of structural change of the amino acid site at which the modified amino acid is located is large;
(2) when the structure is compared between when the antigen-binding domain variant having the amino acid modification is bound to MTA and when it is not bound to MTA, the structural change of the antigen-binding domain variant is not inhibited by the presence of the modified amino acid; or
(3) the MTA-binding activity of the antigen-binding domain variant having the amino acid modification is not significantly weakened as compared to that of the unmodified antigen-binding domain.

"One or more amino acids" in the present disclosure does not particularly limit the number of amino acids, and may be two or more types of amino acids, five or more types of amino acids, ten or more types of amino acids, 15 or more types of amino acids, or 20 or more types of amino acids.
"Designing a library comprising nucleic acids that encode individually a plurality of variants of the aforementioned antigen-binding domains, which have different sequences from one another" in the present disclosure includes designing a library that comprises a plurality of variants of antigen-binding domains or antigen-binding molecules comprising an antigen-binding domain whose amino acids at specified sites have been modified to desired amino acids using known library techniques such as NNK and TRIM libraries (Gonzalez-Munoz A et al. MAbs 2012; Lee CV et al. J Mol Biol. 2004; Knappik A. et al. J Mol Biol. 2000; Tiller T et al. MAbs 2013), but is not particularly limited to this embodiment. In the present disclosure, "variant" refers to variants in which at least one or more amino acids are substituted with respect to its unmodified parent sequence.
In the "step of producing a plurality of variants of the aforementioned antigen-binding domains, which have different sequences from one another" of the present disclosure, among the identified amino acid sites, the sites in CDR1 and CDR2 can be substituted with amino acids having an occurrence frequency of 10% or more, 9% or more, 8% or more, 7% or more, 6% or more, 5% or more, 4% or more, 3% or more, 2% or more, or 1% or more in the germline, and the sites in CDR3 can be substituted with amino acids having an occurrence frequency of 10% or more, 9% or more, 8% or more, 7% or more, 6% or more, 5% or more, 4% or more, 3% or more, 2% or more, or 1% or more in the germline to produce the individual variants, but the production is not limited thereto.
In the step of producing "a plurality of variants of the antigen-binding domain differing in sequence from one another" in the present disclosure, when comparing the structure of a variant in which an amino acid at a specified amino acid site is substituted between when the variant is bound to MTA and when it is not bound to MTA, the variant can be prepared so that the rate of structural change of the amino acid site where the substituted amino acid is located is large, but is not limited thereto.
In the step of producing "a plurality of variants of the antigen-binding domain differing in sequence from one another" in the present disclosure, when comparing the structure of a variant in which an amino acid at a specified amino acid site is substituted between when the variant is bound to MTA and when it is not bound to MTA, the variant can be prepared in such a way that the presence of the substituted amino acid does not inhibit the structural change of the variant due to MTA binding, but is not limited thereto
In the step of producing "a plurality of variants of the antigen-binding domain differing in sequence from one another" in the present disclosure, the variant can be produced so that the MTA-binding activity of the variant in which an amino at the specified amino acid site is modified is not significantly weakened compared to the unmodified body, but is not limited thereto.

Concentrated libraries

In one non-limiting embodiment, the present disclosure provides a library in which nucleic acids encoding antigen-binding molecules comprising an antigen-binding domain that binds to MTA are concentrated.
"Concentrate" in the present disclosure means increasing the abundance ratio of nucleic acids encoding variants having the desired activity compared to the library before performing the concentration. The state in which the abundance ratio of the nucleic acids encoding variants having the desired activity is increased is referred to as "concentrated".
In a non-limiting example, the concentration of nucleic acids encoding antigen-binding molecules that bind to MTA can be achieved by increasing the abundance ratio of nucleic acids encoding antigen-binding molecules having MTA-binding activity by panning. In a more specific non-limiting example, it is possible to increase the abundance ratio

of nucleic acids encoding antigen-binding molecules having MTA-binding activity through panning by contacting MTA with phages presenting on the surface a library that comprises a plurality of antigen-binding molecules by the phage display method, removing phages displaying molecules that do not have binding activity and phages not displaying the molecules by washing, and then collecting only the phages that display antigen-binding molecules which maintain binding. Such a method is a method that comprises the following steps (1) to (2):

(1) contacting an antigen-binding domain displayed through the library with MTA, and
(2) selecting the antigen-binding domain that bound to MTA in step (1).

It is preferable that the abundance ratio of nucleic acids encoding antigen-binding molecules having the desired activity is increased by 1.1 times or more as compared with the library before the concentration. More preferably, it is possible to produce a library according to the present disclosure by increasing the abundance ratio of nucleic acids encoding antigen-binding molecules having the desired activity by 1.2 times or more, 1.5 times or more, 2 times or more, 4 times or more, 10 times or more, 25 times or more, or 100 times or more. In a non-limiting embodiment, the MTA-binding activity of antigen-binding molecules in a library concentrated in this way is also demonstrated in the absence of an antigen, which is a molecule of a type different to MTA.
The type of library to be concentrated is not limited as long as it contains an antigen binding domain whose antigen-binding activity changes in an MTA-dependent manner. Non-limiting examples include naive human antibody display libraries, synthetic human antibody display libraries, libraries designed according to the methods described hereinbelow, and libraries described in the Examples herein.
In another non-limiting example, the concentration of nucleic acids encoding antigen-binding molecules that bind to adenosine can be achieved by increasing the abundance ratio of nucleic acids encoding antigen-binding molecules having adenosine-binding activity by panning. In a more specific non-limiting example, it is possible to increase the abundance ratio of nucleic acids encoding antigen-binding molecules having adenosine-binding activity through panning by contacting adenosine with phages presenting on the surface a library that comprises a plurality of antigen-binding molecules by the phage display method, removing phages displaying molecules that do not have binding activity and phages not displaying the molecules by washing, and then collecting only the phages that display antigen-binding molecules which maintain binding.
Such a method is a method that comprises the following steps (1) to (2):

(1) contacting an antigen-binding domain displayed through the library with adenosine, and
(2) selecting the antigen-binding domain that bound to adenosine in the step (1).

It is preferable that the abundance ratio of nucleic acids encoding antigen-binding molecules having the desired activity is increased by 1.1 times or more as compared with the library before the concentration. More preferably, it is possible to produce a library according to the present disclosure by increasing the abundance ratio of nucleic acids encoding antigen-binding molecules having the desired activity by 1.2 times or more, 1.5 times or more, 2 times or more, 4 times or more, 10 times or more, 25 times or more, or 100 times or more. In a non-limiting embodiment, the adenosine-binding activity of antigen-binding molecules in a library concentrated in this way is also demonstrated in the absence of an antigen, which is a molecule of a type different to adenosine.
The type of library to be concentrated is not limited as long as it contains an antigen-binding domain whose antigen-binding activity changes in an adenosine-dependent manner. Non-limiting examples include naive human antibody display libraries, synthetic human antibody display libraries, libraries produced according to the library production methods described herein, and libraries described herein.

Library production method

The present disclosure also relates to methods for producing various embodiments of "libraries" included in the invention of this application described above.
The "library production method" of the present disclosure is not limited to any of the specific methods shown as examples below, and includes any method that can produce the above-described "libraries" of the present disclosure.
For example, "library production method" in the present disclosure includes the methods shown as examples below.
Each of the specific matters in the "library production method" shown as examples below has technical significance as described in detail above with regard to "concentrated from" the "library" in the invention of the present application.
As one embodiment of the present disclosure, a method of producing a library comprising the following steps (a) and (b) is provided:

(a) identifying an amino acid site that satisfies at least one or more of the following (i) to (vi) in an antigen-binding domain having MTA-binding activity:

(i) an amino acid site exposed on the surface of the antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs; or
(vi) an amino acid site that is not important for the formation of a canonical structure; and,

(b) designing a library comprising a nucleic acid encoding an unmodified antigen-binding domain, and nucleic acids encoding a plurality of variants of the antigen-binding domain differing in sequence from one another and having amino acid modifications at one or more amino acid sites identified in step (a).

As one embodiment of the present disclosure, a method of producing a library comprising the following steps (a) and (b) is provided:

(a) identifying an amino acid site that satisfies at least one or more of the following (i) to (vi) in an antigen-binding domain having MTA-binding activity:

(i) an amino acid site exposed on the surface of the antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs; or
(vi) an amino acid site that is not important for the formation of a canonical structure; and

(b) designing a library comprising a nucleic acid encoding an unmodified antigen-binding domain, and nucleic acids encoding a plurality of variants of the antigen-binding domain differing in sequence from one another and having amino acid modifications at one or more amino acid sites identified in step (a),

wherein the amino acid modifications in step (b) satisfy at least one or more of the following (1) to (3):

(1) when comparing the structure between when the antigen-binding domain variant having an amino acid modification is bound to MTA and when it is not bound to MTA, the rate of structural change of the amino acid site at which the modified amino acid is located is large;
(2) when comparing the structure between when the antigen-binding domain variant having an amino acid modification is bound to MTA and when it is not bound to MTA, the structural change of the antigen-binding domain variant is not inhibited by the presence of the modified amino acid; or
(3) the MTA-binding activity of the antigen-binding domain variant having an amino acid modification is not significantly weakened as compared with the unmodified antigen-binding domain.

In one aspect of the present disclosure, not only a method of producing a library encoding antigen-binding domains having an amino acid residue that interacts with MTA is provided, but also provided is a method of producing a library encoding antigen-binding domains having an amino acid residue that interacts with various small molecule compounds.

Each specific matter in the methods of producing a library illustrated below has the same technical significance as the method of producing the M library detailed above, and those skilled in the art can understand the meaning of various specific matters by reading "MTA" as "small molecule compound".

In one embodiment, a method of producing a library that comprises the following steps (a) and (b) is provided:

(a) identifying an amino acid site that satisfies at least one of the following (i) to (ii) in an antigen-binding domain having binding activity towards a small molecule compound:

(i) an amino acid site exposed on the surface of the antigen-binding domain;
(ii) an amino acid site located in a region where the rate of structural change is large when the structure is compared between when the antigen-binding domain is bound to the small molecule compound and when it is not bound to the small molecule compound; and,

(b) designing a library comprising a nucleic acid encoding an unmodified antigen-binding domain, and nucleic acids encoding a plurality of variants of the antigen-binding domain differing in sequence from one another and having amino acid modifications at one or more amino acid sites identified in step (a).

In one embodiment, a method of producing a library that comprises the following steps (a) and (b) is provided:

(a) identifying an amino acid site that satisfies at least one of the following (i) to (ii) in an antigen-binding domain having binding activity towards a small molecule compound:

(i) an amino acid site exposed on the surface of the antigen-binding domain;
(ii) an amino acid site located in a region where the rate of structural change is large when the structure is compared between when the antigen-binding domain is bound to the small molecule compound and when it is not bound to the small molecule compound;
and,

(b) designing a library comprising a nucleic acid encoding an unmodified antigen-binding domain, and nucleic acids encoding a plurality of variants of the antigen-binding domain differing in sequence from one another and having amino acid modifications at one or more amino acid sites identified in step (a),

wherein the amino acid modifications in step (b) satisfy at least one or more of the following (1) to (3):

(1) when comparing the structure between when the antigen-binding domain variant having an amino acid modification is bound to the small molecule compound and when it is not bound to the small molecule compound, the rate of structural change of the amino acid site at which the modified amino acid is located is large;
(2) when comparing the structure between when the antigen-binding domain variant having an amino acid modification is bound to the small molecule compound and when it is not bound to the small molecule compound, the structural change of the antigen-binding domain variant is not inhibited by the presence of the modified amino acid; or
(3) the binding activity to the small molecule compound of the antigen-binding domain variant having an amino acid modification is not significantly weakened as compared with the unmodified antigen-binding domain.

As one embodiment, a method of producing a library comprising the following steps (a) and (b) is provided:

(a) identifying an amino acid site that satisfies at least one of the following (i) to (iv) in an antigen-binding domain that interacts with a small molecule compound:

(i) an amino acid site exposed on the surface of the antigen-binding domain;
(ii) an amino acid site located in a region where the rate of structural change is large when the structure is compared between when the antigen-binding domain is bound to the small molecule compound and when it is not bound to the small molecule compound;
(iii) an amino acid site that is not involved in the binding to the small molecule compound;
(iv) an amino acid site that does not significantly weaken the binding to the small molecule compound;
(v) an amino acid site with diverse amino acid occurrence frequencies in the animal species to which the antigen-binding domain belongs; or
(vi) an amino acid site that is not important for canonical structure formation; and,

(b) designing a library comprising a nucleic acid encoding an unmodified antigen-binding domain, and nucleic acids encoding a plurality of variants of the antigen-binding domain differing in sequence from one another and having an amino acid modification satisfying at least one of the following (1) and (2) at one or more amino acid sites identified in step (a),

(1) when comparing the structure between when the antigen-binding domain variant having an amino acid modification is bound to the small molecule compound and when it is not bound to the small molecule compound, the rate of structural change of the amino acid site at which the modified amino acid is located is large; or

(2) when comparing the structure between when the antigen-binding domain variant having an amino acid modification is bound to the small molecule compound and when it is not bound to the small molecule compound, the structural change of the antigen-binding domain variant is not inhibited by the presence of the modified amino acid.

Antigen-binding molecules that specifically bind to MTA

The present disclosure also relates to antigen-binding molecules that specifically bind to MTA. A non-limiting example of an antigen-binding molecule that specifically binds to MTA is an antigen-binding molecule that does not substantially bind to one or more small molecule compounds selected from S-(5'-adenosyl)-L-homocysteine (SAH), SAM, AMP, ADP, and ATP.

Here, substantial non-binding is determined according to the method described in the section of binding activity herein, and is 85% or less, usually 50% or less, preferably 30% or less, particularly preferably 20% or less of the binding activity to MTA, for a small molecule compound other than MTA.

In one non-limiting embodiment, the antigen-binding molecule that specifically binds to MTA of the present disclosure does not substantially bind to adenosine.

In another non-limiting embodiment, the antigen-binding molecule that specifically binds to MTA of the present disclosure does not substantially bind to S-(5'-adenosyl)-L-homocysteine (SAH).

In another non-limiting embodiment, the antigen-binding molecule that specifically binds to MTA of the present disclosure does not substantially bind to SAM

In another non-limiting embodiment, the antigen-binding molecule that specifically binds to MTA of the present disclosure does not substantially bind to any of adenosine, AMP, ADP, or ATP.

In another non-limiting embodiment, the antigen-binding molecule that specifically binds to MTA of the present disclosure does not substantially bind to any of adenosine, S-(5'-adenosyl)-L-homocysteine (SAH).

In another non-limiting embodiment, the antigen-binding molecule that specifically binds to MTA of the present disclosure does not substantially bind to any of adenosine, S-(5'-adenosyl)-L-homocysteine (SAH), or SAM

In another non-limiting embodiment, the antigen-binding molecule that specifically binds to MTA of the present disclosure does not substantially bind to any of adenosine, S-(5'-adenosyl)-L-homocysteine (SAH), SAM, AMP, ADP, or ATP.

An example of a non-limiting embodiment of an antigen-binding molecule of the present disclosure that specifically binds to MTA is an antigen-binding molecule comprising any one of the following antibody variable regions:

a) an antibody variable region comprising the heavy chain variable region set forth in SEQ ID NO: 46 and the light chain variable region set forth in SEQ ID NO: 47;
b) an antibody variable region comprising the heavy chain variable region set forth in SEQ ID NO: 50 and the light chain variable region set forth in SEQ ID NO: 51;
c) an antibody variable region comprising the heavy chain variable region set forth in SEQ ID NO: 48 and the light chain variable region set forth in SEQ ID NO: 49; or
d) an antibody variable region comprising the heavy chain variable region set forth in SEQ ID NO: 52 and the light chain variable region set forth in SEQ ID NO: 53.

An example of a non-limiting embodiment of an antigen-binding molecule that specifically binds to MTA in the present disclosure is an isolated antibody that binds to MTA.

In any of the above embodiments, an antigen-binding molecule that specifically binds to MTA is humanized. In one embodiment, an antigen-binding molecule that specifically binds to MTA comprises HVRs as in any of the above embodiments, and further comprises an acceptor human framework, e.g. a human immunoglobulin framework or a human consensus framework. Further, the antigen-binding molecule that specifically binds to MTA may be introduced with amino acid modifications for the purpose of enhancing MTA-binding activity, or may be introduced with modifications for further increasing specificity to specific small molecule compounds. The purpose of introducing amino acid modifications are not limited to those mentioned above, and modifications for any purpose may be introduced.

In another aspect, an antigen-binding molecule that specifically binds to MTA comprises a heavy chain variable region sequence having at least 90%, 91%, 92%, 93%, 94%, 95%, 96%, 97%, 98%, 99%, or 100% sequence identity to the amino acid sequence of SEQ ID NOs: 46, 48, 50, and 52. In certain embodiments, a heavy-chain variable region sequence having at least 90%, 91%, 92%, 93%, 94%, 95%, 96%, 97%, 98%, or 99% identity contains substitutions (e.g., conservative substitutions), insertions, or deletions relative to the reference sequence, but an antigen-binding molecule that specifically binds to MTA comprising that sequence retains the ability to bind to MTA. In certain embodiments, a total of 1 to 10 amino acids have been substituted, inserted and/or deleted in SEQ ID NOs: 46, 48, 50, and 52.

In another aspect, an antigen-binding molecule that specifically binds to MTA comprises a light chain variable

region having at least 90%, 91%, 92%, 93%, 94%, 95%, 96%, 97%, 98%, 99%, or 100% sequence identity to the amino acid sequence of SEQ ID NOs: 47, 49, 51, and 53. In certain embodiments, a light-chain variable region sequence having at least 90%, 91%, 92%, 93%, 94%, 95%, 96%, 97%, 98%, or 99% identity contains substitutions (e.g., conservative substitutions), insertions, or deletions relative to the reference sequence, but an antigen-binding molecule that specifically binds to MTA comprising that sequence retains the ability to bind to MTA. In certain embodiments, a total of 1 to 10 amino acids have been substituted, inserted and/or deleted in SEQ ID NOs: 47, 49, 51, and 53.

In another aspect, an antigen-binding molecule that specifically binds to MTA is provided, wherein the antigen-binding molecule comprises a heavy-chain variable region as in any of the embodiments provided above, and a light-chain variable region as in any of the embodiments provided above. In one embodiment, the antibody comprises the heavy-chain variable region sequence and light-chain variable region sequence in SEQ ID NOs: 46, 48, 50, and 52 and SEQ ID NOs: 47, 49, 51, and 53, respectively, including post-translational modifications of those sequences. Post-translational modifications include but are not limited to a modification of glutamine or glutamate in N-terminal of heavy chain or light chain to pyroglutamic acid by pyroglutamylation.

## Methods and Compositions for Diagnostics and Detection

In certain embodiments, any of the antigen-binding molecule that specifically binds to MTA provided herein is useful for detecting the presence of MTA in a biological sample. The term "detecting" as used herein encompasses quantitative or qualitative detection. In certain embodiments, a biological sample comprises a cell or tissue, such as a cancer tissue.

In one embodiment, an antigen-binding molecule that specifically binds to MTA for use in a method of diagnosis or detection is provided. In a further aspect, a method of detecting the presence of MTA in a biological sample is provided. In certain embodiments, the method comprises contacting the biological sample with an antigen-binding molecule that specifically binds to MTA as described herein under conditions permissive for binding of the antigen-binding molecule that specifically binds to MTA to MTA, and detecting whether a complex is formed between the antigen-binding molecule that specifically binds to MTA and MTA. Such method may be an *in vitro* or *in vivo* method. In one embodiment, an antigen-binding molecule that specifically binds to MTA is used to select subjects eligible for therapy with an antigen-binding molecule that specifically binds to MTA, e.g. where MTA is a biomarker for selection of patients. In another embodiment, it may be used in a method of predicting the therapeutic effect for a patient.

An example of a non-limiting embodiment of the use for selecting a subject is the determination of whether it is a cancer in which MTA has accumulated or not. Since the present disclosure verified that MTA is accumulated in cancer tissues it possible to diagnose whether or not a subject has such a cancer by detecting MTA in a tissue and/or in the periphery.

Furthermore, as a non-limiting embodiment of the use for predicting a therapeutic effect, shrinkage or exacerbation of a cancerous tissue can be indirectly diagnosed by detecting MTA in a tissue and/or in the periphery.

In certain embodiments, a labeled antigen-binding molecule that specifically binds to MTA is provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}P$, $^{14}C$, $^{125}I$, $^{3}H$, and $^{131}I$, fluorophores such as rare earth chelates or fluorescein and its derivatives, rhodamine and its derivatives, dansyl, umbelliferone, luciferases, e.g., firefly luciferase and bacterial luciferase (U.S. Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, those coupled with an enzyme that employs hydrogen peroxide to oxidize a dye precursor such as HRP, lactoperoxidase, or microperoxidase, biotin/avidin, spin labels, bacteriophage labels, stable free radicals, and the like.

Amino acids contained in the amino acid sequences of the present disclosure may be post-translationally modified (for example, the modification of an N-terminal glutamine into a pyroglutamic acid by pyroglutamylation is well-known to those skilled in the art). Naturally, such post-translationally modified amino acids are included in the amino acid sequences in the present disclosure.

Those skilled in the art will naturally understand that any arbitrary combination of one or more of the embodiments described herein are included in the present disclosure, as long as it is not technically inconsistent with the common general knowledge of those skilled in the art.

All prior art documents cited in this specification are incorporated herein by reference.

[Examples]

Hereinafter, the present disclosure will be specifically described with reference to Examples, but is not limited

thereto.

[Example 1] Analysis of MTA accumulation in tumor tissue and normal tissue

The following cell lines were obtained to be used in the experiment: HCC827, HPAC, HT-1376, NCI-H2228, SK-LU-1, SK-MES-1, and U-87 MG from ATCC, Mia-PaCa2 from Dainippon, KM12 from NCI, KM12-Luc from JCRB, and PK-1 from RIKEN. For cell lines other than KM12-Luc, MTAP-deficient state was determined by referring to the SNPs array dataset of the cell lines published by Barretina J et al., Nature (2012) 483: 603-7. Although KM12-Luc was not included in this dataset, it was considered to have the same MTAP-deficient state as KM12 since it is a derivative of KM12.

(1-1) Measurement of intracellular MTA concentration of MTAP-deficient/non-deficient cells

$10^6$ cells of MTAP-deficient cells Mia-PaCa2, NCI-H2228, SK-LU-1, and U-87 MG, and MTAP-non-deficient cells HCC827, HPAC, and KM12-Luc were seeded into 75 $cm^2$ bottles and cultured for 48 hours. The cells were detached with trypsin and collected, $H_2O$ was added to the collected cells, and cell disruption by sonication was carried out to obtain a disrupted cell solution. To this disrupted cell solution, MeOH was added at three times the volume of the disrupted cell solution and vortexed, cell debris were removed by centrifugation, and the MTA concentration in the supernatant after centrifugation was measured using LC-MS (TSQ vantage, Thermo Fisher). Referring to the report by Reinoso RF et al., Drug Metab Dispos. 2001 Apr; 29 (4 Pt 1): 453-9, the cell volume was assumed to be 4.0 uL per $10^6$ cells, and the intracellular MTA concentration was calculated from the concentration of MTA in the supernatant after centrifugation. As shown in Fig. 1, it was found that all MTAP-deficient cells all had a higher intracellular MTA concentration than MTAP-non-deficient cells.

(1-2) Measurement of MTA concentration in the culture medium of MTAP-deficient/non-deficient cells

$10^6$ cells of MTAP-deficient cells Mia-PaCa2, NCI-H2228, SK-LU-1, and U-87 MG, and MTAP-non-deficient cells HCC827 and KM12-Luc were seeded into 75 $cm^2$ bottles, 14 mL of medium was added, and cultured. The culture solution was collected at each time point of 24 and 48 hours, MeOH was added at 3 times the volume of the collected culture solution, cell debris were removed by centrifugation, and the supernatant was obtained. The MTA concentration in the obtained culture supernatant was measured by LC-MS (LTQ Velos and TSQ vantage, Thermo Fisher). As a result, an increase in MTA concentration over time (accumulation of MTA) was observed in the culture solution of MTAP-deficient cells. On the other hand, in the culture solution of MTAP-non-deficient cell HCC827, MTA level was below the detection limit of 5 nM at any time point, and the MTA concentration in the culture solution of MTAP-non-deficient cell KM12-Luc was also only slightly higher than 5 nM (Fig. 2).

(1-3) Uptake of extracellular MTA by MTAP-non-deficient cells

Since the MTA concentration in the culture solution of MTAP-deficient cells increases over time, it is inferred that there is an intracellular to extracellular MTA transfer pathway. However, in MTAP-non-deficient cells, MTA concentration in the culture solution was very low (Fig. 2), despite the expected presence of ~ 1 uM MTA within the cells (Fig. 1). To get a clue as to why no MTA accumulation was observed in MTAP non-deficient cell culture solutions, 3 x $10^5$ of MTAP non-deficient cells HT-1376 and SK-MES-1 were seeded into 6-well plates and 3 mL of medium was added, and cells were adhered by incubating for 24 hours. Thereafter, an MTA solution was added to the culture solution so as to make the MTA concentration 100 nM, and the cells were cultured for a predetermined time (MTA: Sigma, Catalog No. D5011-100MG). The culture solution was collected at each time point of 0, 4, 24, 48, and 72 hours, and MTA concentration in the collected culture solution was measured by LC-MS, which showed that the MTA concentration in the culture solution decreased over time after the start of culture (Fig. 3). It was suggested that when cells can metabolize MTA using MTAP, the extracellular MTA is absorbed by the cells, making it rapidly disappear.

(1-4) Blood clearance of MTA

When culturing MTAP-non-deficient cells in MTA-added medium, MTA in the culture solution disappeared rapidly. Therefore, it was examined whether MTA was absorbed by cells and disappeared rapidly even in normal tissues.

Fresh blood was collected from mice. A stable isotope of MTA (SIL-MTA) was added to mouse blood that had been preheated at 37°C to a final concentration of 5 ng/mL, and an equal amount of acetonitrile was added 5 seconds later. Methanol and internal standard substances were added to the blood sample deproteinized with acetonitrile, the filtrate after centrifugation and filtration was analyzed by LC-MS/MS, and the SIL-MTA concentration in mouse blood 5

seconds after the addition of SIL-MTA was measured. The SIL-MTA concentration measured using a sample prepared by adding SIL-MTA to a final concentration of 5 ng/mL to a solution obtained by mixing equal amounts of mouse blood and acetonitrile was used as the initial value of SIL-MTA concentration. From the initial value of SIL-MTA concentration and SIL-MTA concentration in mouse blood 5 seconds after the addition of SIL-MTA, the SIL-MTA elimination rate constant from blood was calculated, and the total blood clearance calculated by multiplying the elimination rate constant and the blood volume per mouse unit body weight (85 mL/kg) was estimated to be 46685 mL/h/kg. This result showed that MTA added to mouse blood rapidly disappears from the blood.

(1-5) *In vivo* clearance of MTA in mice

Since the evaluation of Example (1-4) suggested that MTA blood clearance was large *in vitro,* an MTA pharmacokinetics test was conducted to examine whether MTA clearance was actually large even *in vivo.* SIL-MTA was used as the test substance to distinguish it from endogenous MTA. SIL-MTA was intravenously administered to NOD-scid mice at 0.1 to 100 μg/min/kg and 25 mL/kg (n = 3) at a constant rate, and blood was collected at 2, 5, 10, and 30 minutes after the start of administration. After collecting blood, cells were removed by centrifugation at 12000 rpm for 5 minutes at 4 °C to obtain plasma. Plasma SIL-MTA concentration was measured by LC-MS/MS. The systemic SIL-MTA clearance calculated from the plasma SIL-MTA concentration 30 minutes after the start of administration and the administration rate of SIL-MTA was 38961 mL/h/kg or more, which revealed that MTA disappears rapidly from the plasma even *in vivo.*

(1-6) Measurement of MTA concentration in a tumor of a tumor-bearing mouse model

MTAP-deficient cells Mia-PaCa2 and MTAP-non-deficient cells PK-1 were subcutaneously transplanted into NOD SCID mice to prepare tumor-bearing mice, and tumors were collected when the tumor volume was 188 to 347 $mm^3$ (n = 3). Methanol was added at three times the weight of the tumor, homogenized using stainless steel beads, and centrifuged at 12000 rpm for 10 minutes to obtain the supernatant. The obtained supernatant was diluted 10-fold with 75% methanol, an aqueous solution containing an internal standard substance was added, and the mixture was centrifuged at 10000 rpm for 10 minutes. The supernatant was filtered through a filter, the filtrate was analyzed by LC-MS/MS to measure MTA concentration, and MTA concentration (pmol/g tissue) in the tumor was calculated from the amount of MTA in the filtrate and the tumor weight. As a result, a high concentration of MTA in tumor was detected in NOD SCID mice transplanted with the MTAP-deficient cell Mia-PaCa2 (Fig. 4).

(1-7) Measurement of MTAP DNA amount and MTA concentration in human clinical samples

Whether MTA accumulation associated with MTAP deficiency was observed in fresh frozen clinical samples of 8 cases of bladder cancer and 10 cases of esophageal cancer purchased from Asterand Inc. and ILS Inc. was investigated.

(1-7-1) Analysis of MTAP DNA amount in human clinical samples

DNA was extracted from clinical samples using the QIAamp Fast DNA Tissue Kit [QIAGEN, cat.no. 51404]. Using the extracted DNA as a template, the MTAP gene region was amplified by the Real Time-PCR method, and the Ct value of MTAP gene was determined. The Ct value is the number of PCR cycles until the amplified DNA amount reaches the threshold value, and becomes a large value when the initial DNA amount is small. To correct the difference in amplification efficiency between experiments, the Ct value of ΨX4 of MTAP pseudogene on another chromosome was also calculated, following M'soka TJ et al. (Leukemia (2000) 14, 935-940). For each sample, ΔCt was calculated by subtracting the Ct value of the ΨX4 gene from the Ct value of MTAP gene.

(1-7-2) Analysis of MTA concentration in human clinical samples

Methanol was added in an amount three times the weight of the clinical sample, homogenized using stainless steel beads, and centrifuged at 12000 rpm for 10 minutes to obtain the supernatant. The obtained supernatant was diluted 10-fold with 75% methanol, an aqueous solution containing an internal standard substance was added, and the mixture was centrifuged at 10000 rpm for 10 minutes. The supernatant was filtered with a filter, the filtrate was analyzed by LC-MS/MS to measure MTA concentration, and MTA concentration (pmol/g tissue) in the tissue of each sample was calculated from the amount of MTA in the filtrate and the weight of the clinical sample. For samples below the detection limit (30 pmol/g tissue), the lower limit of detection was used as the measured value.

(1-7-3) Correlation analysis of MTAP DNA amount and MTA concentration in human clinical samples

The ΔCt value was plotted on the horizontal axis and the amount of MTA in tissue was plotted on the vertical axis, and the correlation between the amount of MTAP DNA and MTA concentration was analyzed (Fig. 5). In both the bladder cancer clinical samples and the esophageal cancer clinical samples, it was observed that MTA concentration increased as ΔCt increased, that is, as the amount of MTAP DNA in the tissue decreased.

(1-8) Measurement of MTA concentration in the tissue of tumor-bearing mouse model by microdialysis

MTAP-deficient cell Mia-PaCa2 and MTAP-non-deficient cell KM12 were transplanted subcutaneously into BALB/c nu/nu mice to prepare tumor-bearing mice (hereinafter, Mia-PaCa2 model and KM12 model). When the transplanted tumor volume reached 200 $mm^3$ or more, a microdialysis probe was inserted into the tumor and liver (the normal tissue used as a control) under isoflurane anesthesia, and tissue interstitial fluid was collected. MTA concentration in the tissue interstitial fluid collected 90 to 120 minutes after the probe was inserted was quantified by the LC-MS/MS method. A standard solution was prepared by adding MTA to a BSA solution (0, 1, 10, 100 mg/mL) at a known concentration, and MTA recovery rate was calculated from the ratio of MTA concentration collected from the microdialysis probe to MTA concentration of the standard solution. The extracellular MTA concentration in the tissue was calculated by correcting MTA concentration in the collected tissue interstitial fluid using the MTA recovery rate (16.3%).

The extracellular MTA concentration in the tumor was higher in the Mia-PaCa2 model than in the KM12 model, suggesting that MTA is excreted into the interstitial fluid from MTAP-deficient cells (Fig. 6). On the other hand, there was no difference in extracellular MTA concentration in the liver between the two tumor-bearing mouse models. From the above results, it was inferred that MTA was excreted from MTAP-deficient cells, but the extracellular MTA concentration in normal tissues was kept low because MTA derived from MTAP-deficient cells disappeared rapidly from the body.

[Example 2]

Obtaining antibodies that bind to antigens in an MTA-dependent manner from a naive library or synthetic library

(2-1) Panning to obtain antibodies that bind to an antigen in an MTA-dependent manner

Antibodies showing a binding activity to the human IL-6 receptor (hIL-6R) in the presence of MTA were screened from a library mimicking a human naive antibody repertoire.

A naive human antibody phage display library (called a naive library) consisting of a plurality of phages that present Fab domains of mutually different human antibody sequences was constructed according to a method known to those skilled in the art, using poly A RNA prepared from human PBMC or commercially available human poly A RNA as template.

In addition, a synthetic human antibody phage display library (called a synthetic library) consisting of a plurality of phages presenting Fab domains of mutually different human antibody sequences was constructed according to a method known to those skilled in the art based on an artificially planned design made referencing the human naive antibody repertoire.

Specifically, *E. coli* carrying the constructed phagemid vector were infected with M13KO7TC (WO2015046554A1) or M13KO7ΔpIII (called hyperphage) (PROGEN Biotechnik), and phages were collected from the supernatant of an overnight culture at 30°C. An antibody display phage library solution was prepared by diluting with TBS the phage population precipitated by adding 2.5 M NaCl/10% PEG to the *E. coli* culture solution in which the phages were produced.

Panning was carried out by the following method. BSA was added to the phage library solution to a final concentration of 4%. As necessary, to remove antibodies that bind to hIL-6R in the absence of MTA, magnetic beads immobilized with 0.1 nmol biotin-labeled hIL-6R and the prepared phage library solution were mixed and reacted at room temperature for 60 minutes. Then the phage solution was collected from the beads separated using a magnetic stand. To 0.8 mL of the collected phage library solution, 0.1 nmol of biotin-labeled hIL-6R and MTA having a final concentration of 100 μM were added, and the mixture was reacted at room temperature for 60 minutes. Magnetic beads (NeutrAvidin beads (TAMAGAWA SEIKI) or Dynabeads MyOne StreptAvidin T1 (Thermo Fisher Scientific)) blocked with BSA were added to this reaction solution, and the mixture was reacted at room temperature for 15 minutes. The beads were washed 2 or 3 times with 0.5 mL TBS/0.1% Tween 20, and 1 or 2 times with TBS. The beads to which TBS was added were then suspended at room temperature, and the phage solution was collected from the beads separated using a magnetic stand. After repeating this operation two or three times, the eluted phage solutions were combined. Trypsin at a final concentration of 1 mg/mL was added to the collected phage solution. The collected phages were added to 20 mL *of E. coli* strain ER2738 in the logarithmic growth phase (OD600 0.4-0.7). *E. coli* were infected with the phage by culturing

the above *E. coli* with stirring at 37°C for 1 hour. *E. coli* were seeded onto a 225 mm x 225 mm plate. This series of operations was repeated three more times.

(2-2) Evaluation of hIL-6R binding activity in the presence of MTA of the phage group acquired after panning by phage ELISA

From the single colonies *of E. coli* obtained by panning, the respective phage-containing culture supernatants were collected according to the conventional method (Methods mol. Biol. (2002) 178, 133-145). Hyperphages were used as helper phages, and antibody multivalent display phages were collected. The collected phage culture supernatants were ultrafiltrated using NucleoFast96 (MACHERY-NAGEL).

Phages diluted in TBS or TBS containing MTA with a final concentration of 100 μM were subjected to ELISA by the following procedure. 10 μl of TBS containing biotin-labeled hIL-6R was added to a streptavidin-coated 384-well microplate (Greiner) and allowed to stand for 1 hour or longer. After washing each well of the plate with TBST, each well was blocked with 80 uL of 0.2% skim milk-TBS for 1 hour or longer. After washing each well with TBST, the prepared phages were added to the wells and allowed to stand for 1 hour to bind the antibodies to be presented by the phages with biotin-labeled hIL-6R in the absence or presence of MTA. The wells were washed with TBST or TBST containing 100 μM MTA, and then TBS or HRP-conjugated anti-M13 antibody (GE Healthcare) diluted with TBS containing MTA at a final concentration of 100 μM was added to the wells, and was left to stand for 1 hour. After washing the wells with TBST or TBST containing MTA at a final concentration of 100 μM, TMB single solution (ZYMED) was added, and after a certain period of time, the coloring reaction of the solution was stopped by adding sulfuric acid, and then the absorbance at 450 nm wavelength was measured. As a result of the analysis, among the 384 clones evaluated, 4 clones of phages were confirmed that presented antibodies whose binding activity to hIL-6R changes in the presence of 100 μM MTA and in the absence of MTA.

(2-3) Expression and purification of an antibody that binds to an antigen in an MTA-dependent manner

The variable region sequences of the heavy and light chains of the antibodies whose binding activity to hIL-6R changes in the presence of 100 μM MTA and in the absence of MTA, which were confirmed in Example (2-2), were each inserted into a plasmid for expression in an animal having a heavy chain antibody constant region sequence (SEQ ID NO: 56) or a light chain kappa constant region sequence (SEQ ID NO: 39). The nucleotide sequence of the antibody gene amplified using the primers (SEQ ID NOs: 44 and 45) was analyzed, one type of antibody variable region sequence (heavy chain variable region: SEQ ID NO: 54, light chain variable region: SEQ ID NO: 55) was identified. The antibody (C03H-BH076N17/C03L-KT0) was expressed using the method of Reference Example 1.

(2-4) Evaluation of the antigen-binding activity in the presence of MTA using surface plasmon resonance

Using Biacore T200 (GE Healthcare), the effects of MTA and adenosine on the antigen-antibody reaction between C03H- BH076N17/C03L-KT0 and hIL-6R were evaluated. TBS, 0.02% (w/v) Tween20, pH 7.4 was used as the running buffer. ProA/G (Pierce) was immobilized onto Sensor Chip CM5 (GE Healthcare) by amine coupling, and after the antibody was immobilized on it, various antigens were allowed to interact as analytes, to observe changes in the antigen-antibody binding amount. The running buffer and a buffer in which either MTA or adenosine was added to the running buffer were used for antigen dilution, which were prepared in a series of stepwise concentrations. Biacore T200 Evaluation Software (GE Healthcare) was used to calculate the parameters.

The amount of C03H-BH076N17/C03L-KT0 bound to hIL-6R under different concentrations of MTA or adenosine is shown in Fig. 7. It was confirmed that C03H-BH076N17/C03L-KT0 showed stronger binding activity to hIL-6R as MTA concentration increased. Furthermore, in the presence of adenosine, no binding to hIL-6R was observed at any concentration. Therefore, it was shown that C03H-BH076N17/C03L-KT0 is an antibody that binds to hIL-6R in an MTA-specific and dependent manner.

[Example 3]

Designing a library using humanized SMB0002 as a template for obtaining antibodies that bind to antigens in an MTA-dependent manner

(3-1) Evaluation of humanized SMB0002 for the ability to bind MTA

Due to the structural similarity between MTA and adenosine, antibodies that bind to adenosine may cross-react to MTA, and adenosine antibodies that cross-react to MTA may be used as template antibodies when designing a library

for obtaining antibodies that bind to an antigen in an MTA-dependent manner. To examine the possibility of using humanized SMB0002 (heavy chain variable region: SEQ ID NO: 31; light chain variable region: SEQ ID NO: 32), which has been reported to bind to adenosine, as a template antibody to design a library for obtaining antibodies that bind to antigens in an MTA-dependent manner, Biacore T200 (GE Healthcare) was used to analyze the binding ability of humanized SMB0002 to MTA.

CaptureSelect™ Biotin Anti-IgG-CH1 Conjugate (Thermo fisher scientific), which is a biotinylated anti-human IgG CH1 molecule, was bound to Sensor Chip SA (GE Healthcare) onto which streptavidin was immobilized in advance, then humanized SMB0002 was allowed to be captured, and MTA (Sigma-Aldrich) or adenosine (Wako) was allowed to interact with humanized SMB0002. 50 mM Tris-HCl, 150 mM NaCl (Takara, T903), 0.02% (w/v) Tween20, 5% DMSO was used as the running buffer. MTA or adenosine was allowed to interact with humanized SMB0002 for 42 seconds at a flow rate of 100 μL/min and then dissociated from the antibody in the running buffer. The interaction was measured at 37°C, and the same buffer as the running buffer was used to dilute MTA or adenosine.

The dissociation constant $K_D$ (M) was calculated based on the binding rate constant ka (1/Ms) and the dissociation rate constant kd (1/s), which are kinetic parameters calculated from the sensorgrams obtained by the measurement. Alternatively, the dissociation constant $K_D$ (M) was calculated using steady state analysis. Biacore T200 Evaluation Software (GE Healthcare) was used to calculate each parameter.

The binding activity of humanized SMB0002 to MTA and adenosine was determined by this measurement as shown in Table 1, and it was confirmed that humanized SMB0002 has binding activity not only to adenosine, but also to MTA.

[Table 1]

| | Affinity $K_D$ [μmol/L] | |
|---|---|---|
| | MTA | Adenosine |
| Humanized SMB0002 | 3.8 | 0.064 |

(3-2) X-ray crystal structure analysis of humanized SMB0002

The crystal structures of the humanized SMB0002 Fab fragment (SMB0002hFab) alone and the SMB0002hFab-adenosine complex were analyzed.

(3-2-1) Preparation of humanized SMB0002 full-length antibody for crystallization

The humanized SMB0002 full-length antibody for crystallization was prepared and purified by a method known to those skilled in the art.

(3-2-2) Preparation of Fab fragments for crystal structure analysis of SMB0002hFab

SMB0002hFab was prepared by a conventional method of restriction digestion of humanized SMB0002 full-length antibody with Endoproteinase Lys-C (Roche, Catalog No. 11047825001), followed by loading onto a protein A column (MabSlect SuRe, GE Healthcare) for removing Fc fragments, a cation exchange column (HiTrap SP HP, GE Healthcare), and a gel filtration column (Superdex200 16/60, GE Healthcare). Fractions containing Fab fragments were pooled and stored at -80°C.

(3-2-3) Preparation of crystals of SMB0002hFab-adenosine complex

Crystallization was carried out using SMB0002hFab purified and concentrated to about 13 mg/mL by a method known to those skilled in the art, by the sitting drop vapor diffusion method at 5 °C. The reservoir solution consisted of 0.1 M disodium hydrogen phosphate-citric acid pH 4.2, 40% w/v polyethylene glycol 200.

(3-2-4) Collection of X-ray diffraction data from a crystal of the SMB0002hFab-adenosine complex and structure determination

The obtained crystal was frozen in liquid nitrogen, and X-ray diffraction data were measured at BL-17A of the Photon Factory, a radiation facility of the High Energy Accelerator Research Organization. During the measurement, the crystal was kept frozen by constantly keeping them under a nitrogen stream at -178°C. A total of 720 X-ray diffraction images were collected using a Pilatus 6M detector (DECTRIS) at the beamline, rotating the crystal by 0.25° at a time.

The obtained diffraction images were processed using Xia2 (J. Appl. Cryst. (2010). 43, 186-190), and diffraction intensity data up to 2.24 Å resolution was obtained. Crystallographic statistics are shown in Table 2.

Using the obtained X-ray diffraction intensity data, a molecular replacement method using Phaser (J. Appl. Cryst. (2007) 40, 658-674) was carried out with the known crystal structure of Fab as a search model, to determine the initial structure. After that, model building and refinement by Coot (Acta Cryst. D66: 486-501 (2010)) and Refmac5 (Acta Cryst. D67: 355-467 (2011)) were repeated, and the final refined coordinates were obtained. Crystallographic statistics are shown in Table 2.

(3-2-5) Preparation of SMB0002hFab crystals

SMB0002hFab purified by a method known to those skilled in the art was denatured with urea, refolded by the dialysis method, and subsequently purified by gel filtration chromatography (Superdex200 10/300 increase, GE Healthcare). Crystallization was performed at 20 °C by the sitting drop vapor diffusion method using SMB0002hFab concentrated to about 13 mg/mL. The reservoir solution consisted of 0.1 M Morpheus buffer 2 pH 7.5, 37.5% w/v M1K3350, 0.1 M Morpheus-Carboxylic acids (Morpheus, Molecular Dimensions).

(3-2-6) Collection of X-ray diffraction data from the SMB0002hFab crystal and structure determination

The obtained crystal was frozen in liquid nitrogen, and X-ray diffraction data were measured at BL-17A of the Photon Factory, a radiation facility of the High Energy Accelerator Research Organization. During the measurement, the crystal was kept frozen by constantly keeping them under a nitrogen stream at -178°C. A total of 4320 X-ray diffraction images were collected using a Pilatus 6M detector (DECTRIS) at the beamline, rotating the crystal by 0.25° at a time. The obtained diffraction images were processed using autoPROC (Acta Cryst. D67: 293-302 (2011)), and diffraction intensity data up to 1.83 Å resolution was obtained. Crystallographic statistics are shown in Table 2.

Using the obtained X-ray diffraction intensity data, the initial structure was determined by the molecular replacement method using Phaser (J. Appl. Cryst. (2007) 40, 658-674) and the known Fab crystal structure as a search model. After that, model building and refinement by Coot (Acta Cryst. D66: 486-501 (2010)) and Refmac5 (Acta Cryst. D67: 355-467 (2011)) were repeated, and the final refined coordinates were obtained. Crystallographic statistics are shown in Table 2.

[Table 2]

| X-ray Data Collection and Refinement Statistics | | | | |
|---|---|---|---|---|
| **Data collection** | | SMB0002hFab | | SMB0002hFab-ADO |
| **Space group** | | $C222_1$ | | $P2_12_12_1$ |
| **Unit cell** | | | | |
| *a, b, c* (Å) | | 119.91, 160.08, 118.05 | | 54.64, 113.41, 163.27 |
| $\alpha, \beta, \gamma$ (°) | | 90.00, 90.00, 90.00 | | 90.00, 90.00, 90.00 |
| Resolution (Å) | | 118.05-1.83 | | 66.26-2.24 |
| Total reflections | | 1887478 | | 330168 |
| Unique reflections | | 96083 | | 48900 |
| Completeness (highest resolution shell) (%) | | 96.0 (66.2) | | 98.6 (97.7) |
| $R_{merge}$[a](highest resolution shell) (%) | | 11.7 (121.9) | | 24.7 (142.5) |
| **Refinement** | | | | |
| Resolution (Å) | | 95.97-1.83 | | 66.26-2.24 |
| Reflections | | 91329 | | 46424 |
| *R*factor[b] ($R_{free}$[c]) (%) | | 20.36 (24.10) | | 19.47 (25.36) |
| rms deviation from ideal value | | | | |
| Bond length (Å) | | 0.013 | | 0.010 |

(continued)

| Refinement | | | | |
|---|---|---|---|---|
| Bond angle (°) | | 1.534 | | 1.344 |

a;

$$R_{merge} = \Sigma hkl \Sigma j | Ij(hkl) - \langle I(hkl) \rangle | / \Sigma hkl \Sigma j | Ij(hkl) |$$

Here *I,j* (*hkl*) and ⟨I(*hkl*)⟩ are the intensity of the measurent j having the index hkl and the average intensity of reflections, respectively.

b;

$$Rfactor = \Sigma hkl | F_{calc}(hkl) | - | F_{obs}(hkl) | / \Sigma hkl | F_{obs}(hkl) |,$$

Here, $F_{obs}$ and $F_{calc}$ are the observed and calculated amplitudes of the structural factors, **respectively.**

c; $R_{free}$ is calculated using 5% of the randomly excluded reflections.

(3-2-7) Identification of the interaction site between SMB0002hFab and adenosine

The asymmetric unit of the crystal structure of the complex of SMB0002hFab and adenosine (SMB0002hFab-adenosine complex) contained two complexes of SMB0002hFab and adenosine. Both structures can be well overlapped. Fig. 8 described below was made using only one of the molecules.

From the crystal structure of the SMB0002hFab-adenosine complex, it became clear that adenosine binds mainly to the pocket formed between the heavy chain and the light chain of the antibody Fab fragment, with the adenine ring portion bound pointing towards the back of the pocket.

As shown in Fig. 8, the adenine ring portion of adenosine is recognized by each side chain of light chains S91 and N96 and heavy chains A33, 150, and Y100, and each main chain of heavy chains T100a and G99 of humanized SMB0002. In particular, hydrogen bonds are formed between N at position 1 in the adenine ring and the side chain of the antibody light chain N96; between NH2 at position 6 of the adenine ring and the side chains of the antibody light chains N96 and S91 and the carbonyl oxygen of the main chain of the antibody heavy chain T100a; and between N at position 7 of the adenine ring and the amide NH of the main chain of the antibody heavy chain T100a. In addition, a weak hydrogen bond is formed between the CH at position 8 of the adenine ring and the carbonyl oxygen of the main chain of the antibody heavy chain G99. Furthermore, an interaction which uses the pi-electrons of the adenine ring portion, such as CH-π or π-π, is formed between the adenine ring and each side chain of the antibody heavy chains A33, 150, and Y100. In addition, the O at position 3' of the ribose moiety forms a hydrogen bond with each side chain of the antibody heavy chains D54 and S56. Furthermore, in addition to these interactions, adenosine is surrounded by antibody heavy chains T57, G52, W58, N100b, F100d, and antibody light chain Y95c, forming van der Waals interactions. It is inferred that adenosine is recognized by humanized SMB0002 due to these interactions. The amino acid residue numbering of Fab is based on the Kabat numbering scheme.

Moreover, as shown in Example (3-1), it has been revealed that humanized SMB0002 binds not only to adenosine but also to MTA. Since there are parts where the molecular structures of adenosine and MTA are similar, the binding mode of when MTA binds to humanized SMB0002 can be speculated from the crystal structure of humanized SMB0002 and adenosine. In the crystal structure of adenosine and humanized SMB0002, an intramolecular hydrogen bond is formed between OH of the ribose at position 5' of adenosine and N at position 3 of the adenine ring, but in MTA, its thiomethyl group cannot form this interaction. In order for MTA to bind to humanized SMB0002 while maintaining the interaction formed between the adenine ring moiety or ribose moiety and humanized SMB0002, the thiomethyl group of MTA must be located at a position away from the adenine ring moiety. As a result, it is presumed that the thiomethyl group is located closer to the heavy chain CDR2 and the light chain CDR3 than the ribose 5'position OH of adenosine and on the surface side of the antibody. Therefore, it is considered that MTA is more likely to form a direct interaction in antigen binding than adenosine.

(3-2-8) Comparison of the crystal structures of SMB0002hFab and SMB0002hFab-adenosine complex

The asymmetric unit of the crystal structure of SMB0002hFab contained two SMB0002hFab molecules (molecule 1, molecule 2). Fig. 9 shows the superimposition of variable regions extracted from molecule 1 (SMB0002hFab_1) and molecule 2 (SMB0002hFab_2). As shown in Fig. 9, with regard to the two structures, the torsion angles of the structure of heavy chain CDR1 and between the heavy chain and light chain were different.

From Fig. 10 showing the superimposition of the crystal structure of the SMB0002hFab-adenosine complex and SMB0002hFab_1, it was revealed that both structures can be superimposed well, and it was speculated that

SMB0002hFab_1 takes a similar structure as that when binding adenosine. On the other hand, the structure of SMB0002hFab_2 is different from the structure of the adenosine-bound complex, and it is inferred to be the structure when adenosine is not bound.

Fig. 11 shows the superimposition of the crystal structure of the SMB0002hFabadenosine complex and the crystal structure of SMB0002hFab_2. It is suggested that the structure of humanized SMB0002 may be fixed to a single structure such as the SMB0002hFabadenosine complex due to adenosine binding. Specifically, the structure of the heavy chain CDR1 and the torsion angle between the heavy chain and the light chain are changed by the binding of adenosine.

It is conceivable that the difference between the structure of humanized SMB0002 fixed by adenosine and the structure of humanized SMB0002 in other states is important for adenosine-dependent antigen binding in antibodies that bind to an antigen in an adenosine-dependent manner.

In other words, in an antibody that binds to an antigen in an adenosine-dependent manner, the antigen can bind to the antibody having a structure fixed by adenosine, while in the absence of adenosine, it is assumed that it is difficult for the antigen to bind to the antibody. Therefore, it is expected that the utilization of the region where the structural change occurs due to the binding of adenosine is effective in exerting the adenosine-dependent antigen-binding ability. Utilization of the antigen-binding interface of an antibody that is fixed by adenosine binding is considered to be important for exerting adenosine-dependent antigen-binding ability.

From the results of the above crystal structure analysis of humanized SMB0002, amino acid sites that are not significantly involved in adenosine binding or amino acid sites that are expected to be likely involved in adenosine-dependent binding to antigens (amino acid sites where the amino acid residues exposed on the surface of antibody proteins are located, or amino acid sites located in the antibody region whose structure changes when adenosine is bound) were selected.

(3-3) Comprehensive evaluation of monosubstituted variants for designing a library using humanized SMB0002 as a template

From the results of crystal structure analysis, amino acid sites in humanized SMB0002 that are not significantly involved in the binding of humanized SMB0002 to adenosine or MTA, or amino acid sites that are expected to be likely involved in adenosine-dependent or MTA-dependent binding to antigens were estimated. Even if the amino acids at these sites are modified to amino acids different from those of humanized SMB0002, it is highly likely that they will retain the interaction with MTA or adenosine, and therefore, they were thought to be diversifiable amino acid sites for library design. One of the amino acids at these diversifiable amino acid sites of humanized SMB0002 was modified to identify the types of amino acids at the diversifiable amino acid sites that do not affect the interaction of the antibody with adenosine or MTA, and thus humanized SMB0002 monosubstituted variants were comprehensively prepared.

Amino acid sites of heavy chains that can be diversified based on consideration of interaction with adenosine (sites represented by Kabat numbering and described as "Kabat" in the table), the amino acids before modification at these sites (amino acids appearing at the relevant sites of humanized SMB0002 and described as "native sequence" in the table), and the modified amino acids (amino acids described as "modified amino acid" in the table) are shown in Table 3.

[Table 3]

| | | HFR1 | | | | | | | | HCDR1 | | | | | HFR2 | HCDR2 | | | | | | | | | | | | |
|---|---|---|---|---|---|---|---|---|---|---|---|---|---|---|---|---|---|---|---|---|---|---|---|---|---|---|---|---|
| | Kabat | 23 | 24 | 25 | 26 | 27 | 28 | 29 | 30 | 31 | 32 | 33 | 34 | 35 | 49 | 50 | 51 | 52 | 53 | 54 | 55 | 56 | 57 | 58 | 59 | 61 | 62 | 65 |
| | Native sequence | K | V | S | G | I | D | L | T | N | Y | A | M | G | G | I | I | G | A | D | S | S | T | W | Y | S | W | G |
| Modified amino acid | A | A | A | A | A | A | A | A | A | A | A | | A | A | | A | A | A | | | A | A | A | A | A | A | A | A |
| | D | D | | D | D | | | | D | D | D | D | D | D | | D | D | D | D | | D | D | D | D | D | D | D | D |
| | E | E | | E | E | | E | | E | E | E | E | E | E | | E | E | E | E | E | E | E | E | E | E | | E | E |
| | F | F | | F | F | | F | | F | F | F | F | F | F | F | F | F | F | F | F | F | F | F | F | F | F | F | F |
| | G | G | | G | | | G | | G | G | G | G | G | | | G | G | | G | G | G | G | G | G | G | | G | |
| | H | H | | H | H | | H | | H | H | H | H | H | H | H | H | H | H | H | H | H | H | H | H | H | H | | |
| | I | I | | I | I | | I | | I | I | I | I | I | I | I | | | I | I | I | I | I | I | I | I | | | I |
| | K | | | K | K | | K | | K | K | K | K | K | K | | K | K | K | K | K | K | K | K | K | K | K | | K |
| | L | L | | L | L | | L | | L | L | L | L | L | L | L | L | L | L | L | L | L | L | L | L | L | L | | L |
| | M | M | | M | M | | M | | M | | | M | | M | M | M | M | M | | | | | | M | | | | |
| | N | N | | N | N | | N | | N | | N | N | N | N | N | N | N | N | | | | N | N | N | | N | | N |
| | P | P | | P | P | | P | | P | P | P | P | | P | | | P | P | P | P | P | P | P | P | P | P | | |
| | Q | Q | | Q | Q | | Q | | Q | Q | Q | Q | Q | Q | Q | Q | Q | Q | Q | Q | Q | Q | Q | Q | Q | Q | | Q |
| | R | R | | R | R | | R | | R | R | R | R | R | | | R | | R | R | R | R | R | R | R | R | R | | R |
| | S | S | | | S | | S | | S | S | S | S | S | S | S | S | | S | S | S | | | S | S | S | | | |
| | T | T | | T | T | | T | | | T | T | T | T | T | T | T | T | T | T | T | T | T | | T | T | T | | T |
| | V | V | | V | V | | V | | V | V | V | V | V | V | V | V | V | V | V | V | V | V | V | V | V | V | | V |
| | W | W | | W | W | | W | | W | W | W | W | W | W | W | W | W | W | W | W | W | W | W | | W | W | | W |
| | Y | Y | | Y | Y | | Y | | Y | Y | | Y | Y | Y | Y | Y | Y | Y | Y | Y | Y | Y | Y | Y | | Y | | |

| | | HFR3 | | | HCDR3 | | | | | | | | | |
|---|---|---|---|---|---|---|---|---|---|---|---|---|---|---|
| | Kabat | 73 | 78 | 94 | 96 | 97 | 98 | 99 | 100 | 100a | 100b | 100c | 100d | 101 | 102 |
| | Native sequence | T | V | R | R | F | V | G | Y | T | N | A | F | D | P |
| Modified amino acid | A | A | A | A | A | A | A | A | A | A | A | | A | A | A |
| | D | D | D | | D | D | D | D | D | D | D | | D | | D |
| | E | E | | | E | E | E | E | E | E | E | | E | E | E |
| | F | | F | | F | | F | F | F | F | F | | | F | F |
| | G | G | G | G | G | G | G | | G | G | G | G | G | G | G |
| | H | | | H | H | H | H | H | H | H | H | | H | H | H |
| | I | I | I | | I | I | I | I | I | I | I | | I | I | I |
| | K | K | K | K | K | K | K | K | K | K | K | | K | K | K |
| | L | | L | | L | L | L | L | L | L | L | | L | L | L |
| | M | M | M | | | | | | | | | | | | |
| | N | N | N | N | N | N | N | | N | N | | | N | N | N |
| | P | | | | P | P | P | P | P | | P | | | P | |
| | Q | Q | | | Q | Q | Q | Q | Q | Q | Q | | Q | Q | Q |
| | R | | | | | R | R | R | R | R | R | | R | R | R |
| | S | S | S | S | S | S | S | S | S | S | S | | S | S | S |
| | T | | T | T | T | T | T | T | T | | T | | T | T | T |
| | V | V | | V | V | V | | V | V | V | V | | V | V | V |
| | W | | | | W | W | W | W | W | W | W | | W | W | W |
| | Y | Y | Y | Y | Y | Y | Y | Y | | Y | Y | | Y | Y | Y |

Amino acid sites of light chains that can be diversified based on consideration of interaction with adenosine (sites represented by Kabat numbering and described as "Kabat" in the table), the amino acids before modification at these sites (amino acids appearing at the relevant sites of humanized SMB0002 and described as "native sequence" in the table), and the modified amino acids (amino acids described as "modified amino acid" in the table) are shown in Table 4.

[Table 4]

| | | LCDR1 | | | LCDR3 | | | | | | | | | | LFR4 |
|---|---|---|---|---|---|---|---|---|---|---|---|---|---|---|---|
| | Kabat | 28 | 29 | 32 | 91 | 92 | 93 | 94 | 95 | 95a | 95b | 95c | 95d | 96 | 97 | 98 |
| | Native sequence | W | N | Y | S | Y | A | N | S | G | W | Y | D | N | A | F |
| Modified amino acid | A | A | A | A | A | A | | A | A | A | A | A | | A | | A |
| | D | D | D | D | D | D | D | D | D | D | D | D | | D | | |
| | E | E | E | E | E | E | E | E | E | E | E | E | | E | | |
| | F | F | F | F | F | F | F | F | F | F | F | F | | F | | |
| | G | G | G | G | G | G | G | G | G | | G | G | G | G | G | G |
| | H | H | H | H | H | H | H | H | H | H | H | H | | H | | |
| | I | I | I | I | I | I | I | I | I | I | I | I | | I | | I |
| | K | K | K | K | K | K | | K | K | K | K | K | | K | | |
| | L | L | L | L | L | L | L | L | L | L | L | L | | L | | L |
| | M | | | | M | M | | | | | | | | M | | |
| | N | N | | | N | N | N | | N | N | N | N | | | | |
| | P | P | P | P | P | P | P | P | P | P | P | P | | P | | |
| | Q | Q | Q | Q | Q | Q | Q | Q | Q | Q | Q | Q | | Q | | |
| | R | R | R | R | R | R | R | R | R | R | R | R | | R | | |
| | S | S | S | S | | S | S | S | | | S | S | | S | | |
| | T | T | T | T | T | T | T | T | T | | T | T | | T | | |
| | V | V | V | V | V | V | V | V | V | V | V | V | | V | | V |
| | W | | W | W | W | W | | W | W | W | | W | | W | | |
| | Y | Y | Y | | Y | | Y | Y | Y | Y | Y | | | Y | | |

Amino acid sites of heavy chains that can be diversified based on consideration of interaction with MTA (sites represented by Kabat numbering and described as "Kabat" in the table), the amino acids before modification at these sites (amino acids appearing at the relevant sites of humanized SMB0002 and described as "native sequence" in the table), and the modified amino acids (amino acids described as "modified amino acid" in the table) are shown in Table 5.

[Table 5]

| | | HFR1 | | | | | | | | HCDR1 | | | HFR2 | HCDR2 | | | | | | | HFR3 | | | HCDR3 | | | | |
|---|---|---|---|---|---|---|---|---|---|---|---|---|---|---|---|---|---|---|---|---|---|---|---|---|---|---|---|---|
| | Kabat | 23 | 24 | 25 | 26 | 27 | 28 | 29 | 30 | 33 | 34 | 35 | 49 | 50 | 51 | 52 | 54 | 56 | 57 | 58 | 73 | 78 | 94 | 99 | 100 | 100a | 100b | 100d |
| | Native sequence | K | V | S | G | I | D | L | T | A | M | G | G | I | I | G | D | S | T | W | T | V | R | G | Y | T | N | F |
| Modified amino acid | A | A | A | A | A | A | A | A | A | | A | A | | A | A | A | | A | A | A | A | A | A | A | A | | | A |
| | D | D | | D | D | | | | D | D | D | D | | D | D | D | | D | D | D | D | D | | D | D | D | D | D |
| | E | E | | E | E | | E | | E | E | E | E | | E | E | E | E | E | E | E | E | | | E | E | E | E | E |
| | F | F | | F | F | | F | | F | F | F | F | F | F | F | F | F | F | F | F | | F | | F | F | F | F | |
| | G | G | | G | | | G | | G | G | G | | | G | G | | G | G | G | G | G | G | G | | G | | | |
| | H | H | | H | H | | H | | H | H | H | H | H | H | H | H | H | H | H | H | | | H | H | H | H | H | H |
| | I | I | | I | I | | I | | I | I | I | I | I | | | I | I | I | I | I | I | I | | I | I | I | I | I |
| | K | | | K | K | | K | | K | K | K | K | | K | K | K | K | K | K | K | K | K | K | K | K | K | K | K |
| | L | L | | L | L | | L | | L | L | L | L | L | L | L | L | L | L | L | L | | L | | L | L | L | L | L |
| | M | M | | M | M | | M | | M | M | | M | M | M | M | M | | | | M | M | M | | | | | | |
| | N | N | | N | N | | N | | N | N | N | N | N | N | N | N | | N | N | N | N | N | N | | N | N | | N |
| | P | P | | P | P | | P | | P | P | | P | | | P | P | P | P | P | P | | | | P | P | | P | |
| | Q | Q | | Q | Q | | Q | | Q | Q | Q | Q | Q | Q | Q | Q | Q | Q | Q | Q | Q | | | Q | Q | Q | Q | |
| | R | R | | R | R | | R | | R | R | R | | | R | | R | R | R | R | R | | | | R | R | R | R | R |
| | S | S | | | S | | S | | S | S | S | S | S | S | | S | S | | S | S | S | S | S | S | S | | | S |
| | T | T | | T | T | | T | | | T | T | T | T | T | T | T | T | T | | T | | T | T | T | T | | | T |
| | V | V | | V | V | | V | | V | V | V | V | V | V | V | V | V | V | V | V | V | | V | V | V | | V | V |
| | W | W | | W | W | | W | | W | W | W | W | W | W | W | W | W | W | W | | | | | W | W | W | W | W |
| | Y | Y | | Y | Y | | Y | | Y | Y | Y | Y | Y | Y | Y | Y | Y | Y | Y | Y | Y | Y | Y | Y | | Y | Y | Y |

Amino acid sites of light chains that can be diversified based on consideration of interaction with MTA (sites represented by Kabat numbering and described as "Kabat" in the table), the amino acids before modification at these sites (amino acids appearing at the relevant sites of humanized SMB0002 and described as "native sequence" in the table), and the modified amino acids (amino acids described as "modified amino acid" in the table) are shown in Table 6.

[Table 6]

| | | LCDR3 | | | | | | | | LFR4 |
|---|---|---|---|---|---|---|---|---|---|---|
| | Kabat | 91 | 92 | 93 | 95 | 95c | 95d | 96 | 97 | 98 |
| | Native sequence | S | Y | A | S | Y | D | N | A | F |
| Modified amino acid | A | A | A | | A | A | | A | | A |
| | D | D | D | | D | D | | D | | |
| | E | E | E | | E | E | | E | | |
| | F | F | F | | F | F | | F | | |
| | G | G | G | | G | G | G | G | G | G |
| | H | H | H | | H | H | | H | | |
| | I | I | I | | I | I | | I | | I |
| | K | K | K | | K | K | | K | | |
| | L | L | L | | | L | | L | | L |
| | M | M | M | | | | | M | | |
| | N | N | N | N | N | N | | | | |
| | P | P | P | | P | P | | P | | |
| | Q | Q | Q | | Q | Q | | Q | | |
| | R | R | R | | R | R | | R | | |
| | S | | S | | | S | | S | | |
| | T | T | T | | T | T | | T | | |
| | V | V | V | | V | V | | V | | V |
| | W | W | W | | W | W | | W | | |
| | Y | Y | | | Y | | | Y | | |

Binding of humanized SMB0002 or its monosubstituted variants to adenosine or MTA was measured and analyzed by a method using Biacore T200 (GE Healthcare). The antibody to be analyzed was captured on the sensor chip, and the interaction with adenosine or MTA was observed.

Diluted adenosine solution/diluted MTA solution and blank running buffer were added to the antibody captured on the sensor chip, and binding of adenosine/MTA to the antibody was observed. The running buffer was then flushed and dissociation of adenosine/MTA from the antibody was observed. Biacore T200 Evaluation Software (GE Healthcare) was used to calculate each parameter.

As a result of the measurement, the affinity of each variant for adenosine and MTA was calculated as the $K_D$ value. The ratio of the $K_D$ value of humanized SMB0002, which is the parent antibody, for adenosine and the $K_D$ value of each heavy chain-modified variant for adenosine ($K_D$ value of the parent antibody humanized SMB0002 for adenosine/$K_D$ value of each of the heavy chain-modified variants for adenosine) is shown in Table 7, and the ratio of the $K_D$ value of humanized SMB0002, which is the parent antibody, for adenosine and the $K_D$ value of each light chain-modified variant for adenosine ($K_D$ value of the parent antibody humanized SMB0002 for adenosine/the $K_D$ value of each of the light chain-modified variants for adenosine) is shown in Table 8. The ratio of the $K_D$ value of humanized SMB0002, which is the parent antibody, for MTA and the $K_D$ value of each heavy chain-modified variant for MTA ($K_D$ value of the parent antibody humanized SMB0002 for MTA/$K_D$ value of each of the heavy chain-modified variants for MTA) is shown in Table 9, and the ratio of the $K_D$ value of humanized SMB0002, which is the parent antibody, for MTA and the $K_D$ value of each light chain-modified variant for MTA($K_D$ value of the parent antibody humanized SMB0002 for MTA/$K_D$ value of each of the light chain-modified variants for MTA) is shown in Table 10.

[Table 7]

| | | HFR1 | | | | | | | | HCDR1 | | | | | HFR2 | HCDR2 | | | | | | | | | | | | |
|---|---|---|---|---|---|---|---|---|---|---|---|---|---|---|---|---|---|---|---|---|---|---|---|---|---|---|---|---|
| | Kabat | 23 | 24 | 25 | 26 | 27 | 28 | 29 | 30 | 31 | 32 | 33 | 34 | 35 | 49 | 50 | 51 | 52 | 53 | 54 | 55 | 56 | 57 | 58 | 59 | 61 | 62 | 65 |
| | Native sequence | K | V | S | G | I | D | L | T | N | Y | A | M | G | G | I | I | G | A | D | S | S | T | W | Y | S | W | G |
| Modified amino acid | A | 0.8 | 0.7 | 1.2 | 0.9 | 1.0 | 1.1 | 1.1 | 0.7 | 0.9 | 0.1 | | 0.5 | 0.4 | | 0.0 | 0.1 | 0.0 | | | 0.5 | 0.4 | 0.3 | 0.1 | 0.9 | 1.0 | 0.7 | 0.8 |
| | D | 0.7 | | 0.7 | 1.1 | | | | 0.6 | 0.6 | 0.1 | 0.0 | 0.4 | 0.4 | | 0.7 | 0.0 | 0.0 | 0.5 | | 0.5 | 0.4 | 0.2 | 0.2 | 0.7 | 0.0 | 1.0 | 0.7 |
| | E | 0.9 | | 0.6 | 0.8 | | 1.1 | | 0.7 | 0.7 | 0.1 | 0.0 | 0.6 | 0.9 | | 1.0 | 0.1 | 0.6 | 0.5 | 0.3 | 0.5 | 0.4 | 0.2 | 0.6 | 0.8 | | 0.9 | 0.7 |
| | F | 0.9 | | 0.7 | 0.9 | | 0.8 | | 1.1 | 0.7 | 0.7 | 0.2 | 0.7 | 0.0 | 0.2 | 0.2 | 0.1 | 0.5 | 0.2 | 0.5 | 0.5 | 0.2 | 0.2 | 0.0 | 0.6 | 1.1 | 0.8 | 0.9 |
| | G | 0.8 | | 1.0 | | | 0.9 | | 1.3 | 0.5 | 0.3 | 0.1 | 0.3 | | | 0.8 | 0.1 | | 0.8 | 0.3 | 0.6 | 0.3 | 0.2 | 0.1 | 0.7 | | 0.9 | |
| | H | 0.9 | | 0.9 | 0.8 | | 1.0 | | 1.1 | 0.4 | 0.7 | 0.0 | 0.9 | 0.3 | 0.2 | 0.4 | 0.1 | 0.3 | 0.2 | 0.3 | 0.6 | 0.3 | 0.2 | 0.1 | 0.9 | 0.6 | | |
| | I | 1.1 | | 0.9 | 0.6 | | 1.5 | | 0.8 | 1.1 | 0.3 | 0.0 | 0.9 | 0.0 | 0.3 | | | 0.1 | 0.4 | 0.2 | 0.4 | 0.2 | 0.2 | 0.1 | 0.7 | | | 0.7 |
| | K | | | 0.8 | 0.8 | | 1.2 | | 0.9 | 0.9 | 0.5 | 0.1 | 0.6 | 0.0 | | 1.1 | 0.1 | 0.6 | 0.8 | 0.1 | 0.3 | 0.4 | 0.2 | 0.2 | 0.6 | 0.5 | | 0.7 |
| | L | 0.9 | | 0.6 | 0.7 | | 1.3 | | 1.0 | 0.9 | 0.2 | 0.4 | 0.9 | 0.0 | 0.4 | 0.2 | 0.2 | 0.2 | 0.2 | 0.4 | 0.3 | 0.6 | 0.2 | 0.1 | 0.6 | 0.6 | | 0.7 |
| | M | 1.1 | | 0.7 | 0.8 | | 0.9 | | 0.8 | | | 0.1 | | 0.0 | 0.4 | 0.1 | 0.3 | 0.6 | | | | | | 0.2 | | | | |
| | N | 1.1 | | 0.8 | 0.8 | | 0.9 | | 0.9 | | 0.5 | 0.0 | 1.4 | 0.0 | 0.5 | 0.4 | 0.1 | 0.4 | | | | 0.3 | 0.2 | 0.1 | | 0.8 | | 0.7 |
| | P | 0.9 | | 1.0 | 1.4 | | 1.0 | | 1.0 | 0.3 | 1.6 | 0.7 | | 0.0 | | | 0.0 | 0.2 | 0.3 | 0.1 | 0.2 | 0.1 | 0.2 | 0.2 | 0.1 | 0.8 | | |
| | Q | 1.2 | | 1.0 | 1.3 | | 1.4 | | 1.2 | 1.2 | 0.3 | 0.1 | 1.0 | 0.2 | 0.4 | 0.5 | 0.3 | 0.2 | 0.5 | 0.4 | 0.3 | 0.3 | 0.5 | 0.1 | 0.7 | 1.1 | | 0.8 |
| | R | 1.0 | | 0.8 | 2.0 | | 1.0 | | 1.1 | 0.8 | 0.8 | 0.7 | 0.3 | | | 0.4 | | 0.7 | 0.5 | 0.7 | 0.4 | 0.5 | 0.3 | 0.0 | 0.6 | 0.9 | | 0.8 |
| | S | 1.0 | | | 1.2 | | 1.2 | | 1.1 | 1.0 | 0.2 | 0.2 | 0.7 | 0.1 | 0.6 | 0.2 | | 0.0 | 0.3 | 0.4 | | | 0.3 | 0.0 | 0.9 | | | |
| | T | 1.1 | | 0.8 | 1.3 | | 1.0 | | | 1.0 | 0.3 | 0.1 | 0.8 | 0.0 | 0.4 | 0.1 | 0.2 | 0.5 | 0.4 | 0.5 | 0.3 | 0.5 | | 0.0 | 0.6 | 1.1 | | 0.6 |
| | V | 0.9 | | 0.8 | 1.8 | | 1.2 | | 1.2 | 0.6 | 0.2 | 0.2 | 0.8 | 0.0 | 0.5 | 0.5 | 0.4 | 0.2 | 0.4 | 0.3 | 0.3 | 0.2 | 0.5 | 0.1 | 0.6 | 1.0 | | 0.6 |
| | W | 1.1 | | 0.7 | 1.3 | | 1.2 | | 1.3 | 0.6 | 0.4 | 0.3 | 0.7 | 0.1 | 0.0 | 0.3 | 0.0 | 0.7 | 0.0 | 0.2 | 0.1 | 0.1 | 0.2 | | 0.5 | 0.9 | | 0.7 |
| | Y | 1.2 | | 0.7 | 0.1 | | 1.1 | | 1.2 | 0.7 | | 0.9 | 0.8 | 0.0 | 0.2 | 0.4 | 0.0 | 1.2 | 0.2 | 0.2 | 0.3 | 0.2 | 0.2 | 0.0 | | 1.0 | | |

| | | HFR3 | | | HCDR3 | | | | | | | | | | |
|---|---|---|---|---|---|---|---|---|---|---|---|---|---|---|---|
| | Kabat | 73 | 78 | 94 | 96 | 97 | 98 | 99 | 100 | 100a | 100b | 100c | 100d | 101 | 102 |
| | Native sequence | T | V | R | R | F | V | G | Y | T | N | A | F | D | P |
| | Preference | | | | A | A | A | A | A | A | C | C | A | C | C |
| Modified amino acid | A | 0.9 | 0.5 | 0.8 | 0.5 | 0.3 | 0.7 | 0.4 | 0.0 | 0.2 | 0.1 | | 0.0 | 0.7 | 0.2 |
| | D | 1.0 | 0.6 | | 0.2 | 0.3 | 0.4 | 0.4 | 0.0 | 0.0 | 0.1 | | 0.1 | | 0.3 |
| | E | 0.9 | | | 1.0 | 0.3 | 0.5 | 0.2 | 0.0 | 0.1 | 0.1 | | 0.2 | 0.6 | 0.2 |
| | F | | 0.4 | | 1.6 | | 0.6 | 0.1 | 0.9 | 0.1 | 0.0 | | | 0.5 | 0.1 |
| | G | 0.8 | 0.3 | 1.0 | 0.7 | 0.2 | 0.4 | | 0.0 | 0.0 | 0.1 | 0.1 | 0.0 | 0.6 | 0.2 |
| | H | | | 0.6 | 0.7 | 0.5 | 0.6 | 0.2 | 0.2 | 0.0 | 0.3 | | 0.0 | 0.6 | 0.1 |
| | I | 1.0 | 1.0 | | 0.2 | 0.4 | 0.9 | 0.1 | 0.0 | 0.2 | 0.0 | | 0.2 | 0.5 | 0.2 |
| | K | 1.0 | 0.6 | 0.9 | 0.9 | 0.4 | 0.7 | 0.1 | 0.0 | 0.1 | 0.0 | | 0.1 | 0.6 | 0.2 |
| | L | | 1.2 | | 0.6 | 0.3 | 0.7 | 0.1 | 0.0 | 0.1 | 1.5 | | 0.1 | 0.5 | 0.1 |
| | M | 1.0 | 0.8 | | | | | | | | | | | | |
| | N | 1.0 | 0.8 | 0.6 | 0.4 | 0.4 | 0.6 | | 0.0 | 0.1 | | | 0.0 | 0.5 | 0.3 |
| | P | | | | 0.0 | 0.1 | 0.8 | 0.1 | 0.0 | | 0.1 | | | 0.0 | |
| | Q | 0.8 | | | 0.6 | 0.4 | 0.8 | 0.1 | 0.0 | 0.1 | 0.1 | | 0.1 | 0.5 | 0.2 |
| | R | | | | | 0.3 | 0.9 | 0.1 | 0.0 | 0.0 | 0.2 | | 0.2 | 0.4 | 0.2 |
| | S | 1.0 | 0.3 | 0.7 | 0.6 | 0.3 | 0.9 | 0.3 | 0.0 | 0.2 | 0.1 | | 2.1 | 0.7 | 0.2 |
| | T | | 0.9 | 0.7 | 0.9 | 0.3 | 0.6 | 0.1 | 0.0 | | 0.1 | | 1.4 | 0.4 | 0.1 |
| | V | 0.9 | | 0.6 | 0.4 | 0.4 | | 0.0 | 0.0 | 0.5 | 0.1 | | 1.1 | 0.4 | 0.1 |
| | W | | | | 0.9 | 0.5 | 0.5 | 0.0 | 0.1 | 0.0 | 0.3 | | 0.5 | 0.5 | 0.1 |
| | Y | 1.0 | 0.6 | 0.3 | 0.9 | 0.4 | 0.5 | 0.1 | | 0.1 | 0.4 | | 0.1 | 0.6 | 0.1 |

[Table 8]

| | LCDR1 | | | LCDR3 | | | | | | | | | | LFR4 |
|---|---|---|---|---|---|---|---|---|---|---|---|---|---|---|
| Kabat | 28 | 29 | 32 | 91 | 92 | 93 | 94 | 95 | 95a | 95b | 95c | 95d | 96 | 97 | 98 |
| Native sequence | w | N | Y | S | Y | A | N | S | G | W | Y | D | N | A | F |
| A | 0.3 | 0.7 | 0.2 | 0.2 | 0.7 | | 0.9 | 0.7 | 0.5 | 0.7 | 0.1 | | 0.9 | | 0.0 |
| D | 0.1 | 0.7 | 0.1 | 0.0 | 0.6 | 0.4 | 1.0 | 0.6 | 0.5 | 0.4 | 0.1 | | 0.0 | | |
| E | 0.1 | 0.7 | 0.2 | 0.7 | 0.7 | 0.4 | 0.7 | 0.7 | 0.5 | 0.6 | 0.1 | | 1.1 | | |
| F | 0.8 | 0.4 | 0.7 | 0.0 | 0.9 | 0.6 | 0.7 | 0.4 | 0.4 | 0.5 | 0.6 | | 1.0 | | |
| G | 0.1 | 0.7 | 0.2 | 0.0 | 0.2 | 0.3 | 1.1 | 0.9 | | 0.7 | 0.1 | 2.1 | 1.0 | 0.9 | 0.0 |
| H | 0.6 | 0.6 | 0.4 | 0.0 | 0.7 | 0.8 | 0.7 | 0.8 | 1.0 | 0.8 | 0.3 | | 1.0 | | |
| I | 0.1 | 0.4 | 0.0 | 0.1 | 1.0 | 0.1 | 0.6 | 0.8 | 0.5 | 0.7 | 0.2 | | 10.8 | | 0.3 |
| K | 0.3 | 0.6 | 0.1 | 1.4 | 0.5 | | 0.8 | 0.7 | 0.5 | 0.5 | 0.5 | | 9.3 | | |
| L | 0.2 | 0.6 | 0.1 | 0.0 | 0.6 | 0.7 | 0.6 | | 0.6 | 0.5 | 1.0 | | 0.1 | | 0.1 |
| M | | | | 0.1 | 0.8 | | | | | | | | 0.1 | | |
| N | 0.3 | | | 0.0 | 0.7 | 0.9 | | 0.5 | 1.0 | 0.5 | 0.6 | | | | |
| P | 0.1 | 0.3 | 0.1 | 0.6 | 0.0 | 0.1 | 0.3 | 0.6 | 0.3 | 0.4 | 0.1 | | 5.4 | | |
| Q | 0.1 | 0.8 | 0.1 | 0.4 | 0.8 | 0.8 | 0.6 | 0.6 | 0.7 | 0.8 | 0.0 | | 0.0 | | |
| R | 0.2 | 0.7 | 0.1 | 0.9 | 0.5 | 1.3 | 0.6 | 0.6 | 0.8 | 1.1 | 0.2 | | 2.5 | | |
| S | 0.6 | 0.7 | 0.2 | | 0.6 | 0.5 | 0.9 | | | 0.8 | 0.1 | | 0.1 | | |
| T | 0.5 | 0.4 | 0.1 | 0.4 | 0.8 | 0.7 | 0.7 | 0.7 | | 0.9 | 0.1 | | 3.4 | | |
| V | 0.6 | 0.4 | 0.0 | 0.0 | 0.9 | 0.5 | 0.4 | 0.7 | 0.6 | 0.7 | 0.2 | | 0.8 | | 0.1 |
| W | | 0.6 | 0.1 | 0.8 | 1.0 | | 0.7 | 0.5 | 0.5 | | 0.1 | | 1.2 | | |
| Y | 0.7 | 0.7 | | 0.1 | | 0.8 | 0.7 | 0.3 | 0.3 | 1.0 | | | 1.3 | | |

[Table 9]

| | HFR1 | | | | | | | | HCDR1 | | | HFR2 | HCDR2 | | | | | | | HFR3 | | | HCDR3 | | | | |
|---|---|---|---|---|---|---|---|---|---|---|---|---|---|---|---|---|---|---|---|---|---|---|---|---|---|---|---|
| Kabat | 23 | 24 | 25 | 26 | 27 | 28 29 | | 30 | 33 | 34 | 35 | 49 | 50 | 51 | 52 | 54 | 56 | 57 | 58 | 73 | 78 | 94 | 99 | 100 | 100a | 100b | 100d |
| Native sequence | K | V | s | G | I | D | L | T | A | M | G | G | I | I | G | D | S | T | W | T | V | R | G | Y | T | N | F |
| A | 1.0 | 0.9 | 1.1 | 1.3 | 0.7 | 0.8 | 0.8 | 1.3 | | 0.3 | 0.2 | | 0.1 | 0.3 | 0.3 | | 0.8 | 0.9 | 0.2 | 1.0 | 0.4 | 1.0 | 0.6 | 0.0 | | | 1.0 |
| D | 1.0 | | 1.0 | 1.1 | | | | 1.2 | 0.0 | 0.2 | 0.0 | | 1.8 | 0.1 | 0.1 | | 1.7 | 0.5 | 0.1 | 0.9 | 0.5 | | 0.8 | 0.1 | 0.1 | 0.2 | 0.1 |
| E | 1.2 | | 0.8 | 1.1 | | 1.1 | | 1.2 | 0.0 | 0.5 | 0.0 | | 0.2 | 0.3 | 0.1 | 0.5 | 1.0 | 0.7 | 0.1 | 1.0 | | | 0.7 | 0.0 | 0.1 | 0.2 | 0.2 |
| F | 1.1 | | 1.1 | 1.3 | | 1.1 | | 1.5 | 0.3 | 0.4 | 0.0 | 0.2 | 0.1 | 0.2 | 0.1 | 1.1 | 0.8 | 0.3 | 0.1 | | 0.4 | | 0.2 | 0.6 | 0.2 | 0.2 | |
| G | 1.0 | | 1.0 | | | 0.8 | | 1.2 | 0.2 | 0.2 | | | 0.2 | 0.3 | | 0.8 | 1.4 | 0.8 | 0.1 | 0.9 | 0.2 | 0.2 | | 0.1 | | | |
| H | 1.1 | | 0.8 | 1.4 | | 1.0 | | 1.3 | 0.1 | 0.6 | 0.0 | 0.1 | 0.1 | 0.2 | 0.2 | 0.8 | 1.0 | 0.5 | 0.1 | | | 0.1 | 0.4 | 0.2 | 0.1 | 0.3 | 0.1 |
| I | 1.1 | | 0.9 | 1.3 | | 1.4 | | 1.1 | 0.1 | 0.5 | 0.0 | 0.2 | | | 0.1 | 0.9 | 0.5 | 0.9 | 0.1 | 1.1 | 1.2 | | 0.1 | 0.0 | 0.2 | 0.1 | 0.0 |
| K | | | 0.9 | 1.5 | | 0.8 | | 1.2 | 1.8 | 0.3 | 0.0 | | 0.2 | 0.3 | 0.2 | 0.6 | 1.7 | 0.8 | 0.2 | 1.0 | 0.3 | 0.8 | 0.3 | 0.1 | 0.2 | 0.1 | 0.1 |
| L | 1.0 | | 0.7 | 1.1 | | 1.0 | | 1.3 | 0.2 | 0.6 | 0.0 | 0.4 | 0.4 | 0.4 | 0.2 | 0.5 | 0.9 | 0.7 | 0.1 | | 1.4 | | 0.2 | 0.1 | 0.1 | 0.2 | 0.1 |
| M | 1.2 | | 0.8 | 1.0 | | 0.9 | | 1.2 | 0.1 | | 0.0 | 0.3 | 0.2 | 0.6 | 0.1 | | | | 0.1 | 1.2 | 1.3 | | | | | | |
| N | 1.0 | | 0.9 | 1.2 | | 0.8 | | 1.1 | 0.0 | 0.8 | 0.0 | 0.4 | 0.3 | 0.3 | 0.1 | | 0.8 | 0.9 | 0.1 | 1.1 | 0.6 | 0.6 | | 0.0 | 0.4 | | 0.1 |
| P | 0.8 | | 1.0 | 1.3 | | 0.7 | | 0.8 | 0.7 | | 0.0 | | | 0.2 | 0.1 | 0.8 | 0.2 | 0.6 | 0.1 | | | | 0.2 | 0.1 | | 0.2 | |
| Q | 1.1 | | 1.0 | 1.3 | | 0.9 | | 1.2 | 1.8 | 0.7 | 0.0 | 0.2 | 0.4 | 0.8 | 0.1 | 0.7 | 1.0 | 1.4 | 0.1 | 1.0 | | | 0.4 | 0.0 | 0.2 | 0.1 | |
| R | 0.9 | | 1.0 | 1.6 | | 0.8 | | 1.0 | 0.0 | 0.2 | | | 0.3 | | 0.1 | 0.8 | 0.6 | 1.0 | 0.1 | | | | 0.3 | 0.0 | 0.1 | 0.1 | 0.1 |
| S | 0.9 | | | 1.5 | | 0.8 | | 1.1 | 0.2 | 0.4 | 0.1 | 0.5 | 0.2 | | 0.2 | 1.3 | | 0.8 | 0.1 | 0.2 | 0.3 | 0.2 | 0.6 | 0.1 | | | 0.1 |
| T | 1.1 | | 0.9 | 1.5 | | 1.0 | | | 0.1 | 0.6 | 0.0 | 0.3 | 0.2 | 0.6 | 0.2 | 1.1 | 1.1 | | 0.1 | | 1.0 | 0.6 | 0.2 | 0.0 | | | 0.2 |
| V | 1.0 | | 0.9 | 1.6 | | 1.1 | | 1.1 | 0.5 | 0.5 | 0.0 | 0.3 | 0.7 | 0.7 | 0.2 | 0.6 | 0.6 | 1.7 | 0.1 | 1.1 | | 0.5 | 0.1 | 0.0 | | 0.1 | 0.1 |
| w | 1.0 | | 0.9 | 1.5 | | 1.5 | | 1.0 | 0.3 | 0.3 | 0.0 | 0.0 | 0.3 | 0.1 | 0.3 | 1.0 | 0.6 | 0.3 | | | | | 0.2 | 0.1 | 0.1 | 0.1 | 0.4 |
| Y | 1.1 | | 0.8 | 1.4 | | 1.1 | | 1.2 | 0.1 | 0.6 | 0.0 | 0.1 | 0.3 | 0.1 | 0.3 | 0.8 | 1.1 | 0.2 | 0.1 | 1.2 | 0.9 | 0.2 | 0.2 | | 0.2 | 0.2 | 0.1 |

[Table 10]

| | LCDR3 | | | | | | | | LFR4 |
|---|---|---|---|---|---|---|---|---|---|
| Kabat | 91 | 92 | 93 | 95 | 95c | 95d | 96 | 97 | 98 |
| Native sequence | S | Y | A | S | Y | D | N | A | F |
| A | 0.3 | 0.5 | | 1.3 | 0.2 | | 0.0 | | 0.0 |
| D | 0.0 | 0.5 | | 1.0 | 0.1 | | 0.1 | | |
| E | 0.0 | 0.6 | | 0.8 | 0.1 | | 0.0 | | |
| F | 0.0 | 0.7 | | 0.7 | 0.8 | | 0.0 | | |
| G | 0.0 | 0.1 | | 1.0 | 0.0 | 0.0 | 0.1 | 1.1 | 0.1 |
| H | 0.0 | 0.5 | | 0.9 | 0.6 | | 0.0 | | |
| I | 0.0 | 0.7 | | 1.2 | 0.2 | | 0.0 | | 0.3 |
| K | 0.0 | 0.3 | | 1.0 | 0.4 | | 0.0 | | |
| L | 0.0 | 0.5 | | | 0.6 | | 0.0 | | 0.1 |
| M | 0.0 | 0.6 | | | | | 0.0 | | |
| N | 0.0 | 0.6 | 0.7 | 0.7 | 0.3 | | | | |
| P | 0.0 | 0.0 | | 1.5 | 0.2 | | 0.0 | | |
| Q | 0.0 | 0.6 | | 1.2 | 0.1 | | 0.0 | | |
| R | 0.0 | 0.4 | | 1.0 | 0.2 | | 0.0 | | |
| S | | 0.4 | | | 0.1 | | 0.0 | | |
| T | 0.3 | 0.5 | | 0.2 | 0.1 | | 0.0 | | |
| V | 0.1 | 0.7 | | 0.8 | 0.3 | | 0.0 | | 0.2 |
| W | 0.0 | 1.2 | | 1.0 | 0.5 | | 0.0 | | |
| Y | 0.0 | | | 0.4 | | | 0.0 | | |

(3-4) Designing a library using humanized SMB0002 as a template

In designing the library, amino acids that satisfy at least one of the following conditions were selected as amino acids that can be used for diversification, based on the information obtained from the comprehensive evaluation of monosubstituted variants.

Condition 1: Amino acids that are not significantly involved in the binding to MTA or adenosine; and
Condition 2: Amino acids that are expected to result in a large structural change ratio of humanized SMB0002 when adenosine is bound compared to when adenosine is not bound.

Modified amino acids whose ratio of the $K_D$ value of the parent antibody (humanized SMB0002) for MTA and the $K_D$ value of heavy chain-modified variants for MTA where the relevant sites have been modified to the relevant amino acids ($K_D$ value of the parent antibody for MTA/$K_D$ value of heavy chain-modified variants for MTA where the relevant sites have been modified to the relevant amino acids) is 0.4 or more, and modified amino acids whose ratio of the $K_D$ value to the light chain-modified variants for MTA ($K_D$ value of the parent antibody for MTA/$K_D$ value of light chain-modified variants for MTA where the relevant sites have been modified to the relevant amino acids) is 0.1 or more were determined to be amino acids of Condition 1 that are not significantly involved in the binding to MTA, and were selected as amino acids that can be used for diversification.

Modified amino acids whose ratio of the $K_D$ value of the parent antibody (humanized SMB0002) for adenosine and the $K_D$ value of heavy chain-modified variants for adenosine where the relevant sites have been modified to the relevant amino acids ($K_D$ value of the parent antibody for adenosine/$K_D$ value of heavy chain-modified variants for adenosine where the relevant sites have been modified to the relevant amino acids) is 0.4 or more, and modified amino

acids whose ratio of the $K_D$ value to the light chain-modified variants for adenosine ($K_D$ value of the parent antibody for adenosine/$K_D$ value of light chain-modified variants for adenosine where the relevant sites have been modified to the relevant amino acids) is 0.1 or more were determined to be amino acids of Condition 1 that are not significantly involved in the binding to adenosine, and were selected as amino acids that can be used for diversification.

If the amino acid at position 32 of the heavy chain according to Kabat numbering is D or E, or if the amino acid at position 33 is V, I, or T, the amino acid was determined to be an amino acid that satisfies Condition 2 and was selected as an amino acid that can be used for diversification.

A library for obtaining an antibody that binds to an antigen in an MTA or adenosine-dependent manner (hereinafter, S02 Library) was constructed by designing a library in which at least one or more of the selected diversifiable amino acids occur. The amino acid-diversified amino acid sites in the heavy chains of the S02 library, as well as the amino acid repertoire at those sites, are shown in Table 11. The amino acid-diversified amino acid sites in the light chains of the S02 library, as well as the amino acid repertoire at those sites, are shown in Table 12. In the tables, the sites represented by the Kabat numbering described as "Kabat" are the amino acid-diversified amino acid sites, the amino acids described as "native sequence" are the amino acids of the unmodified humanized SMB0002 at the sites, and the amino acids described as "amino acids that can be made into a library" indicate the amino acid repertoire at the sites. A library was designed in which at least one of the amino acids contained in the selected amino acid repertoire appears at each diversifiable amino acid site contained in the heavy chain. In addition, a library was designed in which at least one of the amino acids contained in the selected amino acid repertoire appears at each diversifiable amino acid site contained in the light chain.

[Table 11]

| | | HFR1 | | | | HCDR1 | | | | HCDR2 | | | | | | | | | HCDR3 | | | | | | |
|---|---|---|---|---|---|---|---|---|---|---|---|---|---|---|---|---|---|---|---|---|---|---|---|---|---|
| | Kabat | 26 | 28 | 29 | 30 | 31 | 32 | 33 | 34 | 50 | 53 | 54 | 55 | 56 | 57 | 59 | 61 | 65 | 96 | 97 | 98 | 99 | 100 | 100a | 100d |
| | Native sequence | G | D | L | T | N | Y | A | M | I | A | D | S | S | T | Y | S | G | R | F | V | G | Y | T | F |
| Amino acids that can be made into a library | A | A | A | A | A | A | | | A | | | | A | A | A | A | A | A | A | | A | A | | | |
| | D | D | | | D | D | D | | | D | D | | D | D | D | D | | | | | D | D | | | |
| | E | E | E | | E | E | E | | E | | E | E | E | E | E | E | | | E | | E | E | | | |
| | F | F | F | | F | F | F | | F | | | F | F | F | | F | F | F | F | | F | | F | | |
| | G | | G | | G | G | | | | | G | G | G | G | G | G | | | G | | G | | | | |
| | H | H | H | | H | H | H | | H | | | H | H | H | H | H | | | H | H | H | H | | | |
| | I | I | I | | I | I | | I | I | | I | I | | I | I | I | | | | | I | | | | |
| | K | K | K | | K | K | K | | K | | K | K | | K | K | K | | | K | K | K | | | | |
| | L | L | L | | L | L | | | L | | | L | | L | L | L | | | L | | L | | | | |
| | M | M | M | | M | | | | | | | | | | | | | | | | | | | | |
| | N | N | N | | N | | N | | N | | | | | N | N | | | | | N | N | | | N | |
| | P | P | P | | P | | P | P | | | | P | | | P | | | | | | P | | | | |
| | Q | Q | Q | | Q | Q | | | Q | | Q | Q | | Q | Q | Q | Q | | Q | | Q | Q | | | |
| | R | R | R | | R | R | R | | | | R | R | | R | R | R | R | | | | R | | | | |
| | S | S | S | | S | S | | | S | | | S | | | S | S | | | S | | S | S | | | |
| | T | T | T | | | T | | T | T | | | T | | T | | T | T | | T | | T | | | | |
| | V | V | V | | V | V | | V | V | V | | V | | V | V | V | V | | | | | | | V | |
| | W | W | W | | W | W | | | W | | | W | | W | | W | W | | W | W | W | | | | W |
| | Y | Y | Y | | Y | Y | | | Y | | | Y | | Y | | | Y | | Y | Y | Y | | | | |

[Table 12]

| | | LCDR1 | | | LCDR3 | | | | | | | | | LFR4 |
|---|---|---|---|---|---|---|---|---|---|---|---|---|---|---|
| | Kabat | 28 | 29 | 32 | 91 | 92 | 93 | 94 | 95 | 95a | 95b | 95c | 97 | 98 |
| | Native sequence | W | N | Y | S | Y | A | N | S | G | W | Y | A | F |
| Amino acids that can be made into a library | A | A | A | A | A | A | | A | A | A | A | A | | |
| | D | | D | | | D | D | | D | D | D | | | |
| | E | E | E | E | | E | E | E | E | E | E | | | |
| | F | F | F | F | | F | F | F | F | F | F | F | | |
| | G | | G | G | | G | G | G | G | | G | | G | |
| | H | H | H | H | | H | H | H | H | H | H | H | | |
| | I | I | I | | | I | | I | I | I | I | I | | I |
| | K | K | K | | | K | | K | K | K | K | K | | |
| | L | L | L | | | L | L | L | L | L | L | L | | L |
| | M | | | | | M | | | | | | | | |
| | N | N | | | | N | N | | N | N | N | N | | |
| | P | | P | | | | | P | P | P | P | P | | |
| | Q | | Q | | | Q | Q | Q | Q | Q | Q | Q | | |
| | R | R | R | | | R | R | R | R | R | R | R | | |
| | S | S | S | S | | S | S | S | | | S | S | | |
| | T | T | T | | T | T | T | T | T | | T | T | | |
| | V | V | V | | | V | V | V | V | V | V | V | | V |
| | W | | W | | | W | | W | W | W | | W | | |
| | Y | Y | Y | | | | Y | Y | Y | Y | Y | | | |

[Example 4]

Construction of a library for obtaining an antibody that binds to an antigen in an MTA-dependent manner and acquisition of an antibody that binds to an antigen in an MTA-dependent manner from the library

(4-1) Construction of a library for obtaining an antibody that binds to an antigen in an MTA-dependent manner

(4-1-1) Construction of a heavy chain or light chain variable region phage display library

Gene synthesis of the heavy chain variable region portion in the designed S02 library was done using TRIM technology (Tiller T et al. MAbs 2013) and combined with the light chain variable region sequence of humanized SMB0002 (SEQ ID NO: 32). This was introduced into a suitable phagemid vector having a human IgG-derived CH1 sequence and a human IgG-derived light chain constant region sequence. A heavy chain variable region phage display library for obtaining antibodies that bind to an antigen in an MTA or adenosine-dependent manner was constructed by introducing this phagemid vector into *E. coli* by electroporation.

Gene synthesis of the light chain variable region portion in the designed S02 library was done using TRIM technology (Tiller T et al. MAbs 2013) and combined with the heavy chain variable region sequence of humanized SMB0002 (SEQ ID NO: 64). This was introduced into a suitable phagemid vector having a human IgG-derived CH1 sequence and a human IgG-derived light chain constant region sequence. A light chain variable region phage display library for obtaining antibodies that bind to an antigen in an MTA or adenosine-dependent manner was constructed by introducing this phagemid vector into *E. coli* by electroporation.

(4-1-2) Concentration of antibody groups that bind to MTA using the heavy and light chain variable region phage display libraries

Panning using biotinylated MTA (Biotin-2'-MTA) was carried out to concentrate antibody groups that bind to MTA from each of the heavy chain variable region phage display library and light chain variable region phage display library constructed in Example (4-1-1).

Specifically, *E. coli* carrying the phagemid vector of the constructed heavy chain variable region phage display library or light chain variable region phage display library were infected with M13KO7ΔpIII (called hyperphage) (PROGEN Biotechnik), and phages were collected from the supernatant of an overnight culture at 25°C. An antibody multivalent

display phage library solution was prepared by diluting with TBS the phage population precipitated by adding 2.5 M NaCl/10% PEG to the *E. coli* culture solution in which the phages were produced.

Panning using biotin-2'-MTA immobilized on magnetic beads was carried out under two conditions. BSA was added to the antibody multivalent display phage library solution to a final concentration of 4%. NeutrAvidin beads (TAMAGAWA SEIKI) or Dynabeads MyOne Streptavidin T1 (Thermo Fisher Scientific) were used as the magnetic beads. Biotin-2'-MTA was immobilized on the magnetic beads by reacting BSA-blocked magnetic beads with Biotin-2'-MTA at a final concentration of 10 μM at room temperature for 30 minutes. The magnetic beads on which Biotin-2'-MTA was immobilized were washed 3 times with TBST, and under the first condition, 0.4 mL of the prepared antibody multivalent display phage library solution was added to the washed magnetic beads. Under the second condition, 0.4 mL of the prepared antibody multivalent display phage library solution was added to the washed magnetic beads in the presence of 1 mM adenosine, and reacted at room temperature for 60 minutes. The beads were washed 3 times with 400 μL TBST and 2 times with TBS. The beads to which 0.5 mL of TBS containing trypsin at a final concentration of 1 mg/mL was added were then suspended at room temperature for 15 minutes to elute phages. The beads were separated using a magnetic stand and the phage solution was collected. The collected phages were added to 20 mL of E. *coli* strain ER2738 during the logarithmic growth phase (OD600 0.4-0.7). Phages were allowed to infect the *E. coli* by culturing the above *E. coli* at 37°C with stirring for 1 hour. *E. coli* were seeded onto to a 225 mm x 225 mm plate. This series of operations was repeated three additional times, and antibody groups that bind to MTA were concentrated from each of the heavy chain variable region phage display library and the light chain variable region phage display library.

(4-1-3) Evaluation of binding activity to biotinylated MTA of clones acquired after panning by phage ELISA

From a single colony *of E. coli* infected with phages displaying antibodies of the antibody groups that bind to MTA concentrated in Example (4-1-2), a phage-containing culture supernatant was collected using the usual method (Methods Mol. Biol. (2002) 178, 133-145). Hyperphages were used as helper phages, and antibody multivalent display phages were collected and subjected to ELISA. 10 μL of TBS containing biotin-2'-MTA was added to a 384-well streptavidin-coated microplate (Greiner), and the mixture was allowed to stand for 1 hour or more. After washing each well of the plate with TBST, each well was blocked with 80 μL of 0.2% skim milk-TBS for 1 hour or more. Each well was washed with TBST, the prepared phages were added to each well and allowed to stand for 1 hour to bind the antibodies presented on the phages to biotin-2'-MTA. After washing each well with TBST, HRP-conjugated anti-M13 antibody (GE Healthcare) diluted with TBS was added to each well and allowed to stand for 1 hour. Each well was washed with TBST, TMB single solution (ZYMED) was then added, and after a certain period of time, the coloring reaction of the solution was stopped by adding sulfuric acid, and then the absorbance at 450 nm wavelength was measured. As a result of the analysis, a plurality of phages presenting antibodies that bind to biotin-2'-MTA were confirmed. The results of phage ELISA are shown in Fig. 12 (antibody groups concentrated from the heavy chain variable region phage display library), Fig. 13 (antibody groups concentrated from the light chain variable region phage display library), and Table 13. ELISA results showed that many MTA-binding antibodies were contained in the antibody groups concentrated by the panning of Example (4-1-2).

[Table 13]

| | MTA binding rate (the number of clones bound to biotin-2'-MTA within the 192 clones evaluated by ELISA) |
|---|---|
| Antibody groups concentrated from the heavy chain variable region phage display library | 93/192 |
| Antibody groups concentrated from the light chain variable region phage display library | 88/192 |

(4-1-4) Antibody sequence analysis of clones evaluated by phage ELISA

The nucleotide sequence of antibody genes amplified using primers (SEQ ID NOs: 33 and 34) were analyzed for the clones evaluated by phage ELISA. As a result of the analysis, the antibody sequence overlapped samples were 2 clones or less among the 192 clones evaluated by phage ELISA, showing that the library maintained high sequence diversity even after the concentration of Example (4-1-2).

(4-1-5) Construction of a library for obtaining antibodies that bind to an antibody in an MTA-dependent manner using panning against MTA

With regard to the MTA-binding antibody groups concentrated in Example (4-1-2), it was expected that Fab-displaying phage libraries prepared by combining the modified heavy chains and the modified light chains contained in the antibody groups would also contain many antibodies that maintain the binding to MTA, suggesting the possibility of constructing a library for obtaining antibodies that bind to an antigen in an MTA-dependent manner.

Antibody genes were extracted from *E. coli* infected with the phages displaying MTA-binding antibody groups concentrated in Example (4-1-2) by a method known to those skilled in the art. The heavy chain variable region genes were amplified using primers (SEQ ID NOs: 34 and 35) that allow amplification of the heavy chain variable regions against the antibody groups concentrated from the heavy chain variable region phage library. For the antibody groups concentrated from the light chain variable region phage library, the heavy chain variable region sequence of humanized SMB0002 was removed by restriction enzyme treatment, and a phagemid fragment containing the light chain variable region library gene, human IgG-derived light chain constant region sequence, and human IgG-derived CH1 sequence was prepared. By inserting the heavy chain variable region gene amplified from the heavy chain variable region library into the phagemid vector fragment, a phagemid vector was constructed into which the heavy chain/light chain variable region library genes were introduced. By introducing this vector into *E. coli* by electroporation, a library that displays a Fab domain consisting of a human antibody variable region and a constant region, which is to be used for obtaining antibodies that bind to an antigen in an MTA-dependent manner (hereinafter, MTA-concentrated S02 library) was constructed.

(4-2) Acquisition of antibodies that bind to an antigen in an MTA-dependent manner

Antibodies showing binding activity to each of human IL-6 receptor (hIL-6R), human IL-6 (hIL-6), and human IgA (hIgA) in the presence of MTA were screened from the MTA-concentrated S02 library constructed in Example (4-1-5).

Specifically, *E. coli* carrying the phagemid vector of the constructed MTA-concentrated S02 library were infected with M13KO7ΔpIII (called hyperphage) (PROGEN Biotechnik), cultured overnight at 25°C and phages were collected from the supernatant. An antibody multivalent display phage library solution was prepared by diluting with TBS the phage population precipitated by adding 2.5 M NaCl/10% PEG to the *E. coli* culture solution in which the phages were produced.

Respective panning was performed using hIL-6R, hIL-6, and hIgA immobilized on magnetic beads. BSA was added to the antibody multivalent display phage library solution to a final concentration of 4%. 0.1 nmol biotin-labeled antigen and MTA with a final concentration of 100 μM were added to 0.8 mL of antibody multivalent display phage library solution containing BSA, reacted at room temperature for 60 minutes, magnetic beads blocked by BSA were added, and reacted for 15 minutes at room temperature. NeutrAvidin beads (TAMAGAWA SEIKI) or Dynabeads MyOne Streptavidin T1 (Thermo Fisher Scientific) were used as the magnetic beads. The magnetic beads were washed 2 or 3 times with TBST containing 0.8 mL of MTA at a final concentration of 100 μM, and once or twice with TBS containing MTA at a final concentration of 100 μM. The beads to which 0.25 mL of TBS was added were then suspended at room temperature, the beads were separated using a magnetic stand, and the phage solution was eluted. 0.25 mL of TBS was added again to the separated beads, the beads were suspended at room temperature, the beads were separated using a magnetic stand, and the phage solution was eluted. The two eluted phage solutions were mixed. Trypsin at a final concentration of 1 mg/mL was added to the collected phage solution. The collected phages were added to 20 mL *of E. coli* strain ER2738 during the logarithmic growth phase (OD600 0.4-0.7). *E. coli* were infected with the phages by stirring and culturing the above E. *coli at 37°C* for 1 hour. *E. coli were* seeded onto a 225 mm x 225 mm plate. This series of operations was repeated three additional times.

(4-3) Evaluation of antigen-binding activity in the presence of MTA of clones acquired after panning by phage ELISA

From a single colony *of E. coli* obtained by the panning of Example (4-2), a phage-containing culture supernatant was collected according to a conventional method (Methods Mol. Biol. (2002) 178, 133-145). Hyperphages were used as helper phage to collect antibody multivalent display phages. The collected phage culture supernatant was ultrafiltered using NucleoFast96 (MACHERY-NAGEL). Specifically, 0.2 mL of each culture supernatant was added to each well of NucleoFast 96, and this was centrifuged at 6000 g for 40 minutes to remove the flow-through. After that, 0.2 mL of $H_2O$ was added to each well, and the flow-through was removed by centrifugation at 6000 g for 20 minutes. 0.2 mL of TBS was then added to each well, the mixture was allowed to stand at room temperature for 5 minutes, and then the antibody multivalent display phage solution was collected.

Phage ELISA in the absence of MTA was performed as follows. Phages diluted in TBS were subjected to ELISA by the following procedure. A 384-well streptavidin-coated microplate (Greiner) was added with 10 μl of TBS containing a biotin-labeled antigen and allowed to stand for 1 hour or longer. After washing each well of the plate with TBST, each

well was blocked with 80 μL of 0.02% skim milk-TBS for 1 hour or more. Each well was washed with TBST, the collected antibody multivalent-display phages were then added to each well and allowed to stand for 1 hour to bind the antibodies presented by the phages to the biotin-labeled antigen in the absence of MTA. After washing each well with TBST, HRP-conjugated anti-M13 antibody (GE Healthcare) diluted with TBS was added to each well and allowed to stand for 1 hour. After washing the wells with TBST, TMB single solution (ZYMED) was added, and after a certain period of time, the coloring reaction of the solution was stopped by adding sulfuric acid, and then the absorbance at 450 nm wavelength was measured.

Phage ELISA in the presence of MTA was performed as follows. Phage diluted in TBS containing MTA at a final concentration of 100 μM was subjected to ELISA by the following procedure. A 384-well streptavidin-coated microplate (Greiner) was added with 10 μl of TBS containing biotin-labeled antigen and allowed to stand for 1 hour or longer. After washing each well of the plate with TBST, each well was blocked with 80 μL 0.02% skim milk-TBS for 1 hour or longer. Each well was washed with TBST, the collected antibody multivalent display phages were added to each well and allowed to stand for 1 hour to bind the antibody presented on the phages to the biotin-labeled antigen in the presence of MTA. After washing each well with TBST containing a final concentration of 100 μM MTA, HRP-conjugated anti-M13 antibody (GE Healthcare) diluted with TBS containing a final concentration of 100 μM MTA was added to each well and allowed to stand for 1 hour. The wells were washed with TBST containing MTA at a final concentration of 100 μM, TMB single solution (Zymed) was added, and after a certain period, the coloring reaction of the solution was stopped by adding sulfuric acid, after which the absorbance at 450 nm wavelength was measured.

As a result of the analysis, multiple antibodies that bind to an antigen in an MTA-dependent manner were found from panning using each of biotin-labeled human IL-6 receptor (hIL-6R), human IL-6 (hIL-6), and human IgA (hIgA). The results of phage ELISA are shown in Table 14. Multiple clones were obtained that could bind to every antigen in the presence of MTA, but that could not bind to the antigen in the absence of MTA. Some of these clones had antibodies that bind to an antigen in both an adenosine- and MTA- dependent manner, and clones that did not show antigen binding in the presence of adenosine (antibodies that bound to an antigen in an MTA-specific and dependent manner) could also be obtained. Therefore, it was shown that antibodies having excellent MTA-dependent specificity which bind to the antigen in an MTA-specific and dependent manner but which do not bind to the antigen dependent on a small molecule compound having a structure similar to MTA could be obtained from the MTA-concentrated S02 library.

[Table 14]

| Target antigen | Panning rounds | Assessed antibody number | Antibodies binding to an antigen in an MTA-specific and dependent manner | Antibodies binding to an antigen in both an adenosineand MTA-dependent manner |
|---|---|---|---|---|
| hIL-6R | 3 | 96 | 11 | 20 |
| hIL-6R | 4 | 96 | 10 | 83 |
| hIL-6 | 3 | 96 | 22 | 8 |
| hIL-6 | 4 | 96 | 35 | 25 |
| hIgA | 3 | 96 | 11 | 1 |
| hIgA | 4 | 96 | 76 | 3 |

(4-4) Sequence analysis of antibodies that bind to an antigen in an MTA-dependent manner

The nucleotide sequences of antibody genes amplified using primers (SEQ ID NOs: 33 and 34) were analyzed for some of the antibody groups evaluated by phage ELISA.

(4-5) Expression and purification of antibodies that bind to an antigen in an MTA-dependent manner

The heavy chain variable region and light chain variable region sequences of the antibodies were respectively inserted into an animal expression plasmid having a heavy chain antibody constant region sequence (SEQ ID NO: 38) or a light chain kappa constant region sequence (SEQ ID NO: 39), respectively. The antibodies shown in Table 15 were expressed and purified using the method in Reference Example 1.

[Table 15]

| Antibody | Target antigen | H-chain variable region | H-chain constant region | L-chain variable region | L-chain constant region |
|---|---|---|---|---|---|
| 6RS2T007H-F760mnN17/6RS2T007L-KT0 | hIL-6R | SEQ ID NO: 57 | SEQ ID NO: 38 | SEQ ID NO: 58 | SEQ ID NO: 39 |
| 6LS2T001H-F760mnN17/6LS2T001L-KT0 | hIL-6 | SEQ ID NO: 59 | SEQ ID NO: 38 | SEQ ID NO: 60 | SEQ ID NO: 39 |
| IAS2T001H-F760mnN17/IAS2T001L-KT0 | hIgA | SEQ ID NO: 61 | SEQ ID NO: 38 | SEQ ID NO: 62 | SEQ ID NO: 39 |

(4-6) Evaluation of antibody-antigen binding activity in the presence of MTA using surface plasmon resonance

The effects of MTA and adenosine on the antigen-antibody reaction between the antibodies purified and produced in Example (4-5) and the target antigens were evaluated using Biacore T200 (GE Healthcare). 20 mM ACES, 150 mM NaCl, 0.05% (w/v) Tween20, pH 7.4 was used as the running buffer, and the measurement was performed at 25°C. Changes in the amount of binding between antigens and antibodies were observed by immobilizing ProA/G on the sensor chip CM4 by amine coupling, immobilizing the antibody on it, and then interacting with various antigens as analytes. The running buffer and a buffer prepared by adding either MTA or adenosine to the running buffer at a final concentration of 100 μM were used to dilute the antigens. In addition, the antigens were prepared in concentration series of several steps, and the dissociation constant $K_D$ of each clone was calculated by single cycle kinetics analysis from the sensorgram for each antigen concentration. Biacore T200 Evaluation Software (GE Healthcare) was used to calculate the parameters. The dissociation constant $K_D$ of each clone in the presence of 100 μM MTA is shown in Table 16. No binding to the antigen was observed in the presence of 100 μM adenosine.

[Table 16]

| | MTA | Adenosine | (-) |
|---|---|---|---|
| 6RS2T007H-F760mnN17 /6RS2T007L-KT0 | 1.01.E-06 | N.A. | N.A. |
| 6LS2T001H-F760mnN17 /6LS2T001L-KT0 | 1.26.E-07 | N.A. | N.A. |
| IAS2T001H-F760mnN17 /IAS2T001L-KT0 | 6.89.E-07 | N.A. | N.A. |

Fig. 14 shows the amount of binding of each clone to the antigen in the presence and absence of 100 μM MTA or 100 μM adenosine. As shown in Fig. 14, each clone bound to the antigen in the presence of 100 μM MTA, but did not bind to the antigen in the absence of MTA. This confirmed that each purified antibody has the property of binding to the antigen in an MTA-dependent manner.

(4-7) Evaluation of MTA-dependent T cell activation potency of MTA-dependently-binding antibodies

To confirm that MTA-dependently-binding antibodies function in an MTA-dependent manner, the T cell activation potency in the presence and absence of MTA was evaluated using the MTA-dependently-binding antibodies. For the evaluation, an anti-hIL-6R/hCD3 bispecific antibody consisting of the MTA-dependent anti-hIL-6R antibody cloned in Example (4-5) (6RS2T007H-F760mnN17/6RS2T007L-KT0) and the anti-CD3 antibody (heavy chain variable region: SEQ ID NO: 40, heavy chain constant region: SEQ ID NO: 42, light chain variable region: SEQ ID NO: 41, and light chain constant region: SEQ ID NO: 39) was used.

The above MTA-dependent anti-hIL-6R antibody and anti-CD3 antibody were each prepared according to the method in Reference Example 1. A bispecific antibody having the heavy and light chains of both antibodies and an antigen-binding domain that binds to hIL-6R in an MTA-dependent manner and an antigen-binding domain that binds to CD3 was prepared by mixing the obtained MTA-dependent anti-IL-6R antibody and anti-CD3 antibody and reacting in the presence of 25 mM 2-MEA (2-mercaptoethylamine) at 37°C for 90 minutes. The prepared bispecific antibody was solvent-substituted with PBS by dialysis, and then the absorbance at 280 nm was measured using a spectrophotometer. The concentration of the purified antibody was calculated from the obtained measured absorbance using the extinction coefficient calculated by the PACE method described in Reference Example 1. The antibody was diluted with RPMI1640 medium containing 10% FBS (assay buffer) or with the assay buffer containing MTA or adenosine and used for the evaluation of T cell activation potency.

NFAT-RE-luc2-Jurkat cells were used as the human T cell line to evaluate the T cell activation potency, and CT26/hIL-6R in which hIL-6R was forcibly expressed on CT26 was used as the hIL-6R-expressing cell line. NFAT-RE-luc2-Jurkat cells and CT26/hIL-6R cells were suspended in the assay buffer and used so that the cell densities were 3 $\times$ $10^6$ cells/mL and 1 $\times$ $10^6$ cells/mL, respectively. To each well of the 384-well plate, 10 μl of each of the prepared human T cell line and hIL-6R-expressing cell line suspensions were added, and 10 μl of the prepared antibody solution was further added. This was left to stand at 37°C for 6 hours in a 5% $CO_2$ incubator. After allowing to stand, the luciferase activity of the sample was measured to evaluate the ability to activate human T cell lines. Luciferase activity was measured using a commercially available luciferase activity measuring reagent (Bio-Glo luciferase assay system, Promega). 30 μl of the luciferase luminescent substrate was added to the wells of the plate after standing, and after allowing the plate to stand for another 10 minutes, the luminescence amount of the wells was measured by Envision (PerkinElmer). The value obtained by dividing the measured value of each well by the measured value of the control well to which no antibody was added was used as the Luminescence fold as an index for evaluating the T cell activation potency.

Fig. 28 shows the results of evaluating the T cell activation potency of the produced bispecific antibody in the presence or absence of MTA or adenosine. As shown in Fig. 28, it was confirmed that the prepared bispecific antibody strongly activated T cells in the presence of 300 μM MTA as compared to when MTA was absent. Furthermore, since it was confirmed that T cells were strongly activated in the presence of 300 μM MTA compared to when 300 μM adenosine was present, it was confirmed that the prepared bispecific antibody having an antigen-binding domain that binds to hIL-6R in an MTA-dependent manner and an antigen-binding domain that binds to CD3 activates T cells in an MTA-dependent and MTA-specific manner.

(4-8) Verification of MTA-dependent binding of an antibody that binds in an MTA-dependent manner

For the MTA-dependently binding anti-hIL-6R antibody cloned in Example (4-5) (6RS2T007H-F760mnN17/6RS2T007L-KT0), the change in the amount of antigen binding dependent on MTA concentration was assessed using Biacore T200 (GE Healthcare). The measurement was done at 25°C, using 20 mM ACES added with MTA at final concentrations of 0.1, 1, 10, and 100 μM, 150 mM NaCl, 0.05% (w/v) Tween20, pH 7.4 as the running buffer . Series S Sensor Chip CM4 was used as the sensor chip, ProA/G was immobilized by amine coupling with the antibody mobilised on the top of that, after which hIL-6R was allowed to interact as an analyte to measure the change in binding amount between antigen-antibody. The running buffer was used to dilute the antigen. The change in the amount of antigen binding of the antibody at each MTA concentration is shown in Fig. 29. This result confirmed that the amount of the antigen binding of the antibody that binds in an MTA-dependent manner changes stepwise according to the MTA concentration, and that the antibody binds in an MTA concentration-dependent manner.

In addition, it was verified using the Octet RED384 system (Forte Bio) that the MTA-dependently-bound antibody reversibly dissociates from the antigen as MTA concentration decreases. After immobilizing the antibody on a Protein A biosensor (Forte Bio), hIL-6R was allowed to interact as an analyte in the binding phase, and the change in the amount of binding between the antigen-antibody was measured. A buffer containing 3000 nM of an analyte diluted with 20 mM ACES added with MTA at a final concentration of 10, 100 μM, 150 mM NaCl, 0.05% (w/v) Tween 20, pH 7.4 was used in the binding phase. In the dissociation phase, a solution added with MTA at the same concentration as in the binding phase and containing an analyte at the same concentration as in the binding phase, or a buffer having no MTA added and containing an analyte at the same concentration as in the binding phase, were used. A sensorgram showing the change over time in the amount of binding to the antigen from the binding phase to the dissociation phase is shown in Fig. 30. In the figure, w/MTA shows that the dissociation phase contains the same concentration of MTA as in the binding phase, and w/o MTA shows that the dissociation phase does not contain MTA. From this result, it was confirmed that the bond with the analyte was maintained in the dissociation phase in the solution containing MTA, whereas the dissociation rate with the analyte was increased in the solution without MTA, confirming that the binding between an antibody that binds in an MTA-dependent manner and the antigen can reversibly change the binding activity in the presence and absence of MTA.

[Example 5] Acquisition and evaluation of an anti-MTA antibody by rabbit B cell cloning

(5-1) Acquisition of an anti-MTA antibody by rabbit B cell cloning

(5-1-1) Designing an immunogen for obtaining an anti-MTA antibody

6'-MTA- Keyhole Limpet Hemocyanin (6'-MTA-KLH) was used as the immunogen to immunize rabbits. Mariculture Keyhole Limpet Hemocyanin (KLH) is a highly antigenic protein that can be recognized by T cell receptors expressed on helper T cells and is known to activate antibody production. Therefore, by linking with MTA, it is expected to enhance the production of antibodies against MTA.

Also, 6'-MTA-biotin was prepared by linking biotin to MTA instead of KLH.

(5-1-2) Synthesis of an immunogen for obtaining an anti-MTA antibody (2R,3R,4S,5S)-2-(6-Amino-9H-purin-9-yl)-5-(chloromethyl)oxolane-3,4-diol

[Compound 13]

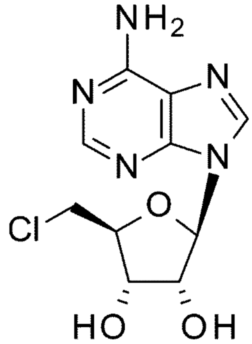

To an acetonitrile solution (210 mL) of (2R,3R,4S,5R)-2-(6-amino-9H-purin-9-yl)-5-(hydroxymethyl)oxolane-3,4-diol (16.08 g, 60.17 mmol, 1 eq), pyridine (9.78 mL, 2.0 eq) was added under a nitrogen atmosphere. Thionyl chloride (22.2 mL, 5.0 eq) was slowly added dropwise to this solution with stirring at 0°C, followed by stirring for 6 hours at 5°C, then overnight at 25°C. The solution was diluted with methanol:water = 5:1 (v/v, 360 mL) and further neutralized with 28% aqueous ammonia (30 mL). The precipitated solid was collected by filtration and dried to obtain (2R,3R,4S,5S)-2-(6-amino-9H-purin-9-yl)-5-(chloromethyl)oxolane-3,4-diol (12.0 g, yield 70%) as a white solid.

$^1$H-NMR (400MHz, DMSO-$d_6$, ppm) $\delta$ 8.35 (s, 1H), 8.17 (s, 1H), 7.30 (s, 2H), 5.94 (d, J = 5.6Hz, 1H), 5.60 (d, J = 6.0Hz, 1H), 5.46 (d, J = 5.2Hz, 1H), 4.76 (q, J = 5.6Hz, 1H), 4.23 (q, J = 4.8Hz, 1H), 4.14-4.06 (m, 1H), 3.96 (dd, J = 11.6, 5.1Hz, 1H), 3.85 (dd, J = 11.6, 6.4 Hz, 1H).

(2R,3R,4S,5S)-2-(6-Amino-9H-purin-9-yl)-5-[(methylsulfanyl)methyl]oxolane-3,4-diol

[Compound 14]

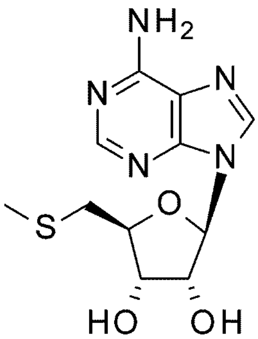

Sodium thiomethoxide (14.73 g, 210 mmol, 3.0 eq) was added to a (2R,3R,4S,5S)-2-(6-amino-9H-purin-9-yl)-5-(chloromethyl)oxolane-3,4-diol (10.0 g, 35.0 mmol, 1 eq) N,N-dimethylformamide solution (300 mL), and the mixture was stirred at 50°C for 2 hours under a nitrogen atmosphere. The reaction solution was concentrated under reduced pressure, and the obtained residue was diluted with water (500 mL). The pH of the solution was adjusted to 7 with 4N hydrochloric acid, and the precipitated solid was collected by filtration to obtain (2R,3R,4S,5S)-2-(6-amino-9H-purin-9-yl)-5-[(methylsulfanyl)methyl]oxolane-3,4-diol (7.0 g, yield 67%) as a white solid.

LC-MS (ES, m/z): 298 $[M + H]^+$

$^1$H-NMR (400MHz, DMSO-$d_6$, ppm) δ 8.36 (s, 1H), 8.16 (s, 1H), 7.28 (s, 2H), 5.90 (d, J = 5.7Hz, 1H), 5.52 (d, J = 5.7Hz, 1H), 5.37-5.31 (m, 1H), 4.76 (q, J = 5.0Hz, 1H), 4.16 (q, J = 3.6Hz, 1H), 4.04 (td, J = 6.3, 3.6Hz, 1H), 2.94-2.71 (m, 2H), 2.06 (S, 3H).

9-[(3aR,4R,6S,6aS)-2,2-Dimethyl-6-[(methylsulfanyl)methyl]-tetrahydro-2H-furo[3,4-d][1,3]dioxol-4-yl]-9H-purine-6-amine

[Compound 15]

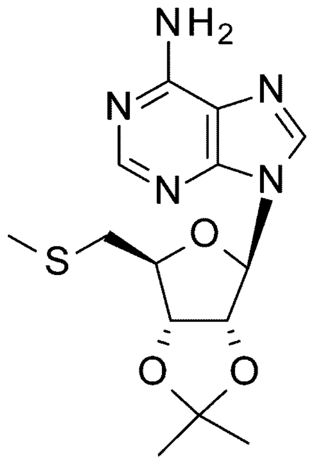

To an acetone solution (500 mL) of (2R,3R,4S,5S)-2-(6-amino-9H-purin-9-yl)-5-[(methylsulfanyl)methyl]oxolane-3,4-diol (7.0 g, 23.54 mmol, 1.0 eq), 2,2-dimethoxypropane (19.55 g, 187.71 mmol, 3.0 eq) and p-toluenesulfonic acid (7.91 g, 45.93 mmol, 2.0 eq) were added at 0°C and stirred at 25°C for 4 hours. After adjusting the pH of the solution to 7 with 1N aqueous ammonia, the solution was concentrated under reduced pressure. The residue was dissolved in ethyl acetate (200 mL), washed twice with distilled water (100 mL), and the organic layer was concentrated under reduced pressure. The residue was purified on a silica gel column (dichloromethane:methanol = 10:1) and 9-[(3aR,4R,6S,6aS)-2,2-dimethyl-6-[(methylsulfanyl)methyl]-tetrahydro-2H-furo[3,4-d][1,3]dioxol-4-yl]-9H-purine-6-amine was obtained as a white solid (6.0 g, yield 76%).

LC-MS (ES, m/z): 338 $[M + H]^+$

$^1$H-NMR (300MHz, Chloroform-d, ppm) δ 8.32 (s, 1H), 7.91 (s, 1H), 6.06 (d, J = 2.2Hz, 1H), 5.89 (s, 2H), 5.49 (dd, J = 6.4, 2.2Hz, 1H), 5.04 (dd, J = 6.4, 3.2 Hz, 1H), 4.45-4.33 (m, 1H), 2.75 (qt, J = 18.4, 9.8Hz, 2H), 2.08 (s, 3H), 1.59 (s, 3H), 1.38 (s, 3H).

6-([9-[(3aR,4R,6S,6aS)-2,2-Dimethyl-6-[(methylsulfanyl)methyl]-tetrahydro-2H-furo[3,4-d][1,3]dioxol-4-yl]-9H-purin-6-yl]amino)hexanoic acid ethyl ester

[Compound 16]

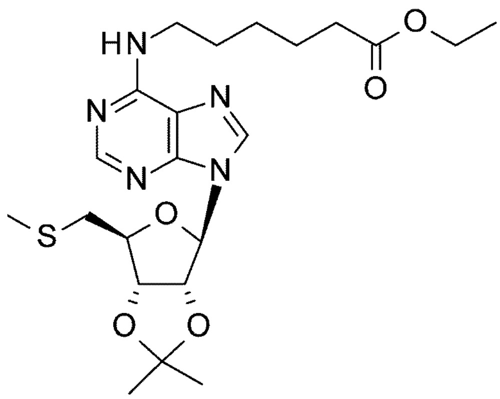

To an N,N-dimethylformamide solution (17 mL) of 9-[(3aR,4R,6S,6aS)-2,2-dimethyl-6-[(methylsulfanyl)methyl]-tetrahydro-2H-furo[3,4-d][1,3]dioxol-4-yl]-9H-purine-6-amine (2.0 g, 5.93 mmol, 1 eq), sodium hydride (60%, 248 mg, 6.2 mmol, 1.05 eq) was added at 25°C and stirred in a nitrogen atmosphere for 2 hours. An N,N-dimethylformamide solution (4 mL) of ethyl 6-bromohexanoate (1.38 g, 6.19 mmol, 1.05 eq) was slowly added dropwise and stirred at 60°C for 2 hours. The reaction mixture was poured into 100 mL of ice water and extracted with ethyl acetate (100 mL). The organic layer was concentrated under reduced pressure, and the obtained residue was purified by a silica gel column (ethyl acetate:petroleum ether = 1:1) to obtain 6-([9-[(3aR,4R,6S,6aS)-2,2-dimethyl-6-[(methylsulfanyl)methyl]-tetrahydro-2H-furo[3,4-d][1,3]dioxol-4-yl]-9H-purin-6-yl]amino)hexanoic acid ethyl ester (1.4 g, yield 49%).
LC-MS (ES, m/z): 480 $[M + H]^+$

6-([9-[(2R,3R,4S,5S)-3,4-dihydroxy-5-[(methylsulfanyl)methyl]oxolan-2-yl]-9H-purin-6-yl]amino)hexanoic acid

[Compound 17]

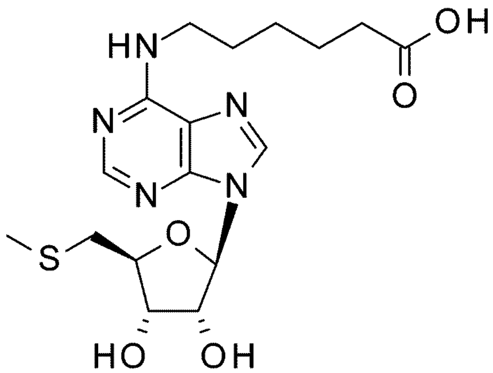

To a tetrahydrofuran solution (7 mL) of 6-([9-[(3aR,4R,6S,6aS)-2,2-dimethyl-6-[(methylsulfanyl)methyl]-tetrahydro-2H-furo[3,4-d][1,3]dioxol-4-yl]-9H-purin-6-yl]amino)hexanoic acid ethyl ester (450 mg, 0.94 mmol, 1 eq), 1N hydrochloric acid (3.5 mL) was added and stirred for 6 hours at 50°C. The residue obtained by concentrating the reaction

solution under reduced pressure was diluted with distilled water (8 mL), and the precipitated solid was collected by filtration. The obtained crude product was purified by preparative HPLC (Shimadzu HPLC-10, XBridge Shield RP18 OBD column, 5 μm, 19 mm x 150 mm, mobile phase water (containing 0.05% ammonia water) and acetonitrile, gradient 5.0-20.0%, detection UV 254 nm) and 6-([9-[(2R,3R,4S,5S)-3,4-dihydroxy-5-[(methylsulfanyl)methyl]oxolan-2-yl]-9H-purin-6-yl]amino)hexanoic acid was obtained as a white solid (159.5 mg, 41%).

LC-MS (ES, m/z): 412 $[M + H]^+$

$^1$H-NMR (400MHz, DMSO-$d_6$, ppm) δ 11.81 (s, 1H), 8.35 (s, 1H), 8.22 (s, 1H), 7.82 (s, 1H), 5.90 (d, J = 5.7Hz, 1H), 5.49 (s, 1H), 5.31 (s, 1H), 4.75 (t, J = 5.3Hz, 1H), 4.16 (t, J = 4.3Hz, 1H), 4.04 (ddd, J = 6.9, 5.8, 3.7Hz, 1H), 3.47 (s, 2H), 2.94-2.74 (m, 2H), 2.20 (t, J = 7.3Hz, 2H), 2.07 (s, 3H), 1.56 (dp, J = 25.6, 7.4Hz, 4H), 1.33 (qd, J = 8.4, 6.0Hz, 2H).

6-([9-[(2R,3R,4S,5S)-3,4-Dihydroxy-5-[(methylsulfanyl)methyl]oxolan-2-yl]-9H-purin-6-yl]amino)-N-(prop-2-yn-1-yl)hexanamide

[Compound 18]

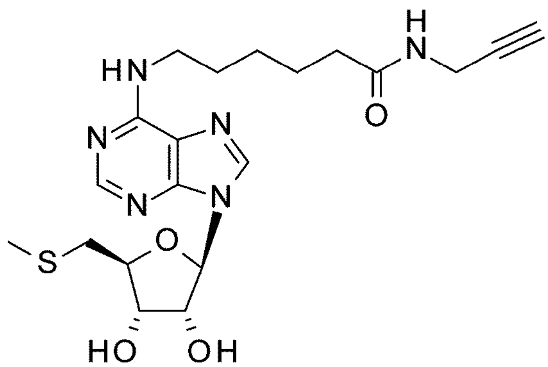

To a dichloromethane solution (20 mL) of 6-([9-[(2R,3R,4S,5S)-3,4-dihydroxy-5-[(methylsulfanyl)methyl]oxolan2-yl]-9H-purin-6-yl]amino)hexanoic acid (580 mg, 1.41 mmol, 1 eq), 1-ethyl-3-(3-dimethylaminopropyl)carbodiimide hydrochloride (542 mg, 2.83 mmol, 2.0 eq), diisopropylethylamine (910 mg, 7.04 mmol, 5.0 eq), and prop-2-yn-1-amine (388 mg, 7.04 mmol, 5.0 eq) were added and stirred at 25°C for 3 days. The solution was diluted with 100 mL of distilled water, and the precipitated solid was collected by filtration and dried to obtain 6-([9-[(2R,3R,4S,5S)-3,4-dihydroxy-5-[(methylsulfanyl)methyl]oxolan-2-yl]-9H-purin-6-yl]amino)-N-(prop-2-yn-1-yl)hexanamide as a white solid, which was then used in the next step without further purification (420 mg, 66% yield).

N-[[1-(2-[2-[2-(2-[5-[(3aS,4S,6aR)-2-oxo-hexahydro-1H-thieno[3,4-d]imidazolidin-4-yl]pentanamide]ethoxy)ethoxy]ethoxy]ethyl)-1H-1,2,3-triazol-4-yl]methyl]-6-([9-[(2R,3R,4S,5S)-3,4-dihydroxy-5-[(methylsulfanyl)methyl]oxolan-2-yl]-9H-purin-6-yl]amino)hexanamide

[Compound 19]

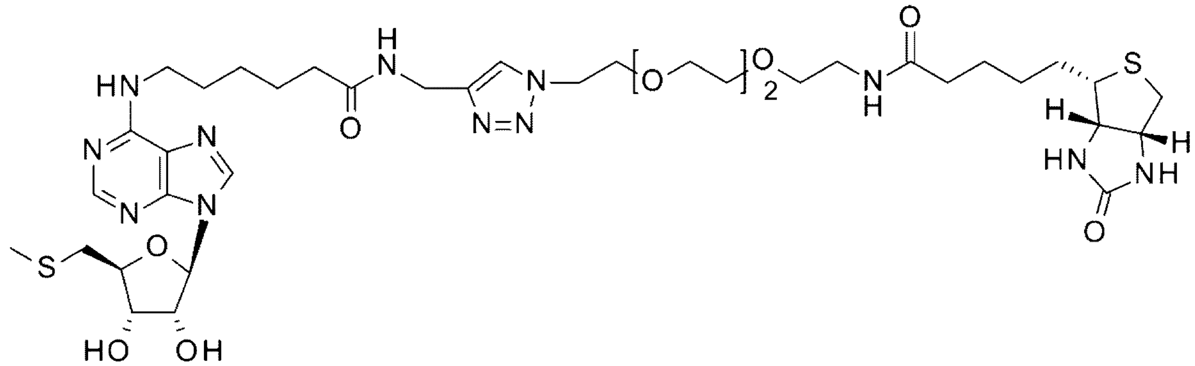

To a tetrahydrofuran (3 mL) and tert-butanol (3 mL) solution of 6-([9-[(2R,3R,4S,5S)-3,4-dihydroxy-5-[(methylsulfanyl)methyl]oxolan-2-yl]-9H-purin-6-yl]amino)-N-(prop-2-yn-1-yl)hexanamide (100 mg, 0.22 mmol, 1 eq), N-(2-(2-(2-(2-azidoethoxy)ethoxy)ethoxy)ethyl)-5-((3aS,4S,6aR)-2-oxo-hexahydro-1H-thieno[3,4-d]imidazol-4-yl)pentanamide (99.1 mg, 0.22 mmol, 1.0 eq), $CuSO_4$ $5H_2O$ (55.5 mg, 0.22 mmol, 1.0 eq), and sodium ascorbate (1 M aqueous solution, 7 drops) were added and the mixture was stirred for 16 hours at 20°C. The solution was diluted with 20 mL of ethyl acetate and partitioned with 15 mL of distilled water. The crude product obtained by concentrating the organic layer under reduced pressure was purified by preparative HPLC (Shimadzu HPLC-10, XBridge Shield RP18 OBD column, 5 um, 19 mm x 150 mm, mobile phase water (containing 10 mmol/L $NH_4HCO_3$ + 0.1% ammonia water) and acetonitrile, gradient 16.0-27.0%, detection UV 220 nm), to obtain N-[[1-(2-[2-[2-(2-[5-[(3aS,4S,6aR)-2-oxo-hexahydro-1H-thieno[3,4-d]imidazolidin-4-yl]pentanamide]ethoxy)ethoxy]ethoxy]ethyl)-1H-1,2,3-triazol-4-yl]methyl]-6-([9-[(2R,3R,4S,5S)-3,4-dihydroxy-5-[(methylsulfanyl)methyl]oxolan-2-yl]-9H-purin-6-yl]amino)hexanamide as a white solid (57.4 mg, yield 29%).

LC-MS (ES, m/z): 893 $[M + H]^+$

$^1$H-NMR (400MHz, DMSO-$d_6$, ppm) $\delta$ 8.35 (s, 1H), 8.25 (dd, J = 13.4, 7.7Hz, 2H), 7.89-7.78 (m, 3H), 6.41 (s, 1H), 6.35 (s, 1H), 5.90 (d, J = 5.7Hz, 1H), 5.48 (d, J = 6.0Hz, 1H), 5.31 (d, J = 5.0Hz, 1H), 4.75 (q, J = 5.6Hz, 1H), 4.49 (t, J = 5.3Hz, 2H), 4.29 (dd, J = 14.3, 6.7Hz, 3H), 4.20-4.08 (m, 2H), 4.04 (td, J = 6.3, 3.7Hz, 1H), 3.80 (t, J = 5.3Hz, 2H), 3.55-3.27 (m, 14H), 3.23-3.04 (m, 3H), 2.94-2.74 (m, 3H), 2.58 (d, J = 12.4Hz, 1H), 2.14-2.02 (m, 7H), 1.53 (ddtd, J = 35.3, 28.2, 13.8, 7.6Hz, 8H), 1.38-1.22 (m, 4H)

Keyhole Limpet Hemocyanin (KLH) conjugate (6'-MTA-KLH) of 6-([9-[(2R,3R,4S,5S)-3,4-dihydroxy-5-[(methylsulfanyl)methyl]oxolan-2-yl]-9H-purin-6-yl]amino)hexanoic acid

To a DMSO solution (200 μl) of 6-([9-[(2R,3R,4S,5S)-3,4-dihydroxy-5-[(methylsulfanyl)methyl]oxolan-2-yl]-9H-purin-6-yl]amino)hexanoic acid (4.94 mg, 0.012 mmol, 1 eq), 1-ethyl-3-(3-dimethylaminopropyl)carbodiimide hydrochloride (2.30 mg, 0.012 mmol, 1.0 eq) and hydroxy-2,5-dioxopyrrolidine-3-sulfonic acid sodium salt (2.61 mg, 0.012 mmol, 1.0 eq) were added, and the mixture was stirred for 2 hours at 20°C. 50 μl of this solution was taken, a phosphate buffer solution (pH 8.4, 0.95 μL) of KLH (10 mg, Thermo Fisher Scientific, 77600) was added at 4°C, the mixture was gently stirred and allowed to stand at 4°C for 24 hours. The obtained solution containing the conjugate was used as is for animal immunization.

(5-1-3) Screening of anti-MTA antibodies by rabbit B cell cloning

Rabbits were immunized by methods known to those skilled in the art using the 6'-MTA-KLH synthesized in Example (5-1-2). From suspensions of cells collected from immunized rabbit blood or spleen, cell candidates with MTA-binding activity even in the presence of unlabeled adenosine were selected using autoMACS Pro Separator and FACSAria (BD). Cells were then screened using the antibody secreted in the culture supernatant of the selected cells. Specifically, the presence or absence of binding activity to 6'-MTA-biotin and the presence or absence of binding activity to 6'-MTA-biotin in the presence of unlabeled MTA were evaluated by the ELISA method. Using PCR, the heavy and light chain variable region genes were obtained from cells selected using as index the secretion of an antibody that binds to 6'-MTA-biotin and suppresses binding to 6'-MTA-biotin in the presence of unlabeled MTA regardless of the presence or absence of adenosine. Antibodies were expressed from the obtained variable region genes in combination with the

human IgG1 heavy chain constant region and the human light chain constant region.

(5-1-4) Sequence analysis of clones obtained from rabbit B cell cloning

The nucleotide sequences of the antibody genes amplified using primers (SEQ ID NOs: 44 and 45) were analyzed for the antibodies obtained in Example (5-1-3). The sequences are shown in Table 17 below.

[Table 17]

| | H-chain variable region amino acid sequence | L-chain variable region amino acid sequence |
|---|---|---|
| MTA0303 | SEQ ID NO : 46 | SEQ ID NO : 47 |
| MTA0330 | SEQ ID NO : 48 | SEQ ID NO : 49 |

(5-2) Evaluation of the binding activity of clones obtained from rabbit B cell cloning to MTA and its analogs

Biacore T200 (GE Healthcare) was used to analyze the binding properties to MTA and its analogs to verify whether the clones obtained from rabbit B cell cloning bind specifically to MTA.

The antibody of interest was captured on Sensor Chip Protein A (GE healthcare) with protein A pre-immobilized, or on an immobilized surface prepared by binding onto Streptavidinimmobilized Sensor chip SA (GE Healthcare) the Capture Select Biotin Anti-IgG-Fc (Hu) (Thermo fisher scientific), which is a biotinylated anti-human IgG CH1 molecule, and interacted with MTA (Sigma-Aldrich) or MTA analogs. As MTA analogs, adenosine (Wako) (measured concentration in tumor tissue: 182 nM) and S-(5'-adenosyl)-L-homocysteine, (SAH) (Sigma-Aldrich) (measured concentration in tumor tissue: 30 nM) were selected based on the structural similarity to MTA and the concentration in tumor tissue of tumor-bearing mice of an MTAPdeficient cell line. 20 mM ACES-NaOH, 150 mM NaCl, 0.05% Tween20, 0.05% DMSO was used as the running buffer. MTA or adenosine or SAH was allowed to interact at a flow rate of 30 μL/min for 120 seconds and then dissociated in the running buffer. The interaction was measured at 25°C and the same buffer as the running buffer was used to dilute MTA and its analogs.

The dissociation constant $K_D$ (M) was calculated based on the binding rate constant ka (1/Ms) and the dissociation rate constant kd (1/s), which are kinetic parameters calculated from the sensorgrams obtained by the measurement. Alternatively, the dissociation constant $K_D$ (M) was calculated using steady state analysis. Biacore T200 Evaluation Software (GE Healthcare) was used to calculate each parameter.

By this measurement, the binding activity of each antibody to MTA and its analogs was determined as shown in Table 18, and it was confirmed that all the selected antibodies had a high selectivity for MTA.

[Table 18]

| | Affinity $K_D$ [μmol/L] | | |
|---|---|---|---|
| | MTA | Adenosine | SAH |
| MTA0303 | 0.00246 | 11.4 | 119 |
| MTA0330 | 0.0144 | 63.5 | 187 |

(5-3) X-ray crystal structure analysis of anti-MTA antibody MTA0303

The crystal structure of the complex (MTA0303Fab-MTA) between the MTA0303 Fab fragment (MTA0303Fab) and methylthioadenosine (MTA) was analyzed.

(5-3-1) Preparation of MTA0303 full-length antibody for crystallization

Preparation and purification of MTA0303 full-length antibody for crystallization were carried out by methods known to those skilled in the art.

(5-3-2) Preparation of Fab fragments for crystal structure analysis of MTA0303Fab

MTA0303Fab was prepared by the conventional method of restriction digestion of MTA0303 full-length antibody with Endoproteinase Lys-C (Roche, Catalog No. 11047825001), followed by loading on a protein A column (Mab Slect

SuRe, GE Healthcare) for removing Fc fragments, a cation exchange column (HiTrap SP HP, GE Healthcare), and gel filtration column (Superdex75 16/60, GE Healthcare). Fractions containing Fab fragments were pooled and stored at -80°C.

(5-3-3) Preparation of crystals of a complex of MTA0303Fab and MTA (MTA0303Fab-MTA complex)

Crystallization was carried out at 5°C using the sitting drop vapor diffusion method that combines the seeding method and femtosecond laser irradiation (J. Appl. Phys. 42: L798-L800 (2003)) performed at SOSHO, Inc. using a solution prepared by mixing MAT0303Fab purified by a method known to those skilled in the art and concentrated to about 16 mg/mL with MTA to a final concentration of 2 mM. The reservoir solution consisted of 0.1 M HEPES pH 7.5, 70% v/v(+/-)-2-methyl-2,4-pentanediol.

(5-3-4) Collection of X-ray diffraction data from a MTA0303Fab-MTA complex crystal and structure determination

The obtained crystal was frozen in liquid nitrogen, and X-ray diffraction data were measured at the Swiss Light Source X10SA, a radiation light facility at Paul Scherrer Institute. During the measurement, the crystal was kept frozen by keeping them under constant nitrogen stream at -178°C. A total of 720 X-ray diffraction images were collected using a Pilatus 6M detector (DECTRIS) at the beamline, rotating the crystal by 0.25° at a time. The obtained diffraction images were processed using autoPROC (Acta Cryst. D67: 293-302 (2011)), and diffraction intensity data up to 1.95 Å resolution was obtained. Crystallographic statistics are shown in Table 19.

Using the obtained X-ray diffraction intensity data, a molecular replacement method using Phaser (J. Appl. Cryst. (2007) 40, 658-674) was carried out with the known crystal structure of Fab as a search model to determine the initial structure. Model building and refinement by Coot (Acta Cryst. D66: 486-501 (2010)) and Refmac5 (Acta Cryst. D67: 355-467 (2011)) were repeated thereafter, and the final refined coordinates were obtained. Crystallographic statistics are shown in Table 19.

(5-3-5) Identification of the interaction site between MTA0303Fab and MTA

From the crystal structure of MTA0303Fab-MTA complex shown in Fig. 20, it was revealed that MTA binds to the pocket formed between the heavy chain and the light chain of MTA0303Fab so that the thiomethyl group portion faces toward the back of the pocket. Further, as shown in Fig. 15, MTA was bound in a way that it is buried deeply and covered by the heavy chain CDR2.

As shown in Fig. 16, the adenine ring portion of MTA is recognized by each side chain of the light chains R32, S50, and L91, and heavy chains W34 and Y52e of the antibody. In particular, hydrogen bonds are formed between the $7^{th}$ position N of the adenine ring and the side chain of the heavy chain Y52e, and between the $2^{nd}$ position CH of the adenine ring and the side chain of the light chain S50. Furthermore, an interaction is formed between the adenine ring and each side chain of the heavy chain W34 and the light chains R32 and L91 using pi-electrons of the adenine ring portion, such as CH-$\pi$ and $\pi$-$\pi$. In addition, hydrogen bonds are formed between the 3'position O of the ribose moiety and the side chain of the light chain Y36, and between the 2'position O of the ribose moiety and each side chain of the light chain S34 and the heavy chain E101. The thiomethyl group moiety is recognized by utilizing the CH-$\pi$ interaction between each side chain of the heavy chains W34, W47, and F52 and the interaction formed between the sulfur atom and the pi electron. Furthermore, in addition to these interactions, MTA is surrounded by heavy chain C35a and light chains L46, Y49, A89, G90, and P96, forming van der Waals interactions. It is inferred that MTA is strongly recognized by the antibody due to these interactions.

In addition, the heavy chain CDR2 of the antibody forms a characteristic helix structure as shown in Fig. 17. It has been revealed from the crystal structure that the side chain of the heavy chain Y52e contained in this secondary structure forms a hydrogen bond with the adenine ring moiety of MTA. It is thought that the formation of this interaction is the trigger that forms the helix structure. Therefore, it is thought that the structure of the antibody may change with the binding of MTA.

The difference between the structure of the antibody in the absence of MTA and the structure of the antibody in the presence of MTA is considered to be important for MTA-dependent binding to an antigen. That is, the protein antigen can bind only to an antibody whose structure has changed due to the binding of MTA, while in the absence of MTA, as the structure of the antibody is different from the structure in the presence of MTA, it is assumed that the protein antigen cannot bind to the antibody in the absence of MTA. Therefore, it is expected that the utilization of the region where the structural change occurs due to the binding of MTA is effective for the exertion of MTA-dependent antigen-binding ability. Since a structural change of the heavy chain CDR2 is considered to accompany the binding of MTA in the antibody, it is expected that antibodies that bind to the antigen in an MTA-dependent manner can be obtained by using the heavy chain CDR2. The amino acid residue numbering of Fab is based on the Kabat numbering scheme.

(5-4) X-ray crystal structure analysis of anti-MTA antibody MTA0330

The crystal structure of a complex (MTA0330Fab-MTA) of the Fab fragment of MTA0330 (MTA0330Fab) and MTA was analyzed.

(5-4-1) Preparation of MTA0330 full-length antibody for crystallization

Preparation and purification of MTA0330 full-length antibody for crystallization were carried out by methods known to those skilled in the art.

(5-4-2) Preparation of Fab fragments for crystal structure analysis of MTA0330Fab

MTA0330Fab was prepared by the conventional method of restriction digestion of MTA0330 full-length antibody with endoproteinase Lys-C (Roche, Catalog No. 11047825001), followed by loading on a protein A column (Mab Slect SuRe, GE Healthcare) for removing Fc fragments, a cation exchange column (HiTrap SP HP, GE Healthcare), and gel filtration column (Superdex75 16/60, GE Healthcare). Fractions containing Fab fragments were pooled and stored at -80°C.

(5-4-3) Preparation of crystals of a complex of MTA0330Fab and MTA (MTA0330Fab-MTA complex)

Crystallization was carried out at 20°C by the sitting drop vapor diffusion method using a solution prepared by mixing MAT0330 Fab purified by a method known to those skilled in the art and concentrated to about 13.3 mg/mL with MTA to a final concentration of 2 mM. The reservoir solution consisted of 30.0 % w/v P550MME_P20K, 0.1 M Morpheus buffer 1 pH 6.5, 60 mM Morpheus Divalents (Morpheus, Molecular Dimensions).

(5-4-4) Collection of X-ray diffraction data from a MTA0330Fab-MTA complex crystal and structure determination

The obtained crystal was frozen in liquid nitrogen, and X-ray diffraction data were measured at the Swiss Light Source X10SA, a radiation light facility at Paul Scherrer Institute. During the measurement, the crystal was kept frozen by keeping them under constant nitrogen stream at -178°C. A total of 1440 X-ray diffraction images were collected using Pilatus 6M detector (DECTRIS) at the beamline, rotating the crystal by 0.25° at a time. The obtained diffraction images were processed using autoPROC (Acta Cryst. D67: 293-302 (2011)), and diffraction intensity data up to 1.46 Å resolution was obtained. Crystallographic statistics are shown in Table 19.

Using the obtained X-ray diffraction intensity data, the initial structure was determined by the molecular replacement method using Phaser (J. Appl. Cryst. (2007) 40, 658-674) with the known Fab crystal structure as a search model. Thereafter, model building and refinement by Coot (Acta Cryst. D66: 486-501 (2010)) and Refmac5 (Acta Cryst. D67: 355-467 (2011)) were repeated, and the final refined coordinates were obtained. Crystallographic statistics are shown in Table 19.

[Table 19]

| X-ray Data Collection and Refinement Statistics | | | | |
|---|---|---|---|---|
| **Data collection** | | MTA0303Fab-MTA | | MTA0330Fab-MTA |
| **Space group** | | C2 | | $P2_12_12$ |
| **Unit cell** | | | | |
| *a, b, c* (Å) | | 145.68, 51.13, 63.29 | | 92.19, 59.01, 84.71 |
| $\alpha, \beta, \gamma$ (°) | | 90.00, 96.67, 90.00 | | 90.00, 90.00, 90.00 |
| Resolution (Å) | | 72.35-2.37 | | 84.72-1.59 |
| Total reflections | | 64024 | | 832722 |
| Unique reflections | | 19092 | | 63155 |
| Completeness (highest resolution shell) (%) | | 99.8 (100.0) | | 99.7 (99.0) |
| $R_{merge}$[a](highest resolution shell) (%) | | 8.6 (57.2) | | 8.6 (119.4) |

(continued)

| **Refinement** | | | | |
|---|---|---|---|---|
| Resolution (Å) | | 72.35-2.37 | | 84.71-1.59 |
| Reflections | | 18148 | | 60089 |
| $R$factor[b] ($R_{free}$[c]) (%) | | 17.95 (22.69) | | 16.28 (19.42) |
| rms deviation from ideal value | | | | |
| Bond length (Å) | | 0.008 | | 0.014 |
| Bond angle (°) | | 1.530 | | 1.696 |

a;

$$R_{merge} = \Sigma hkl \Sigma j | Ij(hkl) - \langle I(hkl) \rangle | / \Sigma hkl \Sigma j | Ij(hkl) |$$

Here $I.i(hkl)$and $\langle I(hkl) \rangle$are the intensity of the measurent j having the index hkl and the average intensity of reflections, respectively.

b;

$$R\text{factor} = \Sigma hkl | F_{calc}(hkl) | - | F_{obs}(hkl) | / \Sigma hkl | F_{obs}(hkl) | ,$$

Here, $F_{obs}$ and $F_{calc}$ are the observed and calculated amplitudes of the structural factors, respectively.

c; $R_{free}$ is calculated using 5% of the randomly excluded reflections.

(5-4-5) Identification of the interaction site between MTA0330Fab and MTA

From the crystal structure of MTA0330Fab-MTA complex shown in Fig. 21, it was revealed that MTA binds in such a way that the thiomethyl group portion binds to the pocket formed between the heavy chain and the light chain of MTA0330Fab facing towards the back of the pocket, and as is shown in Fig. 18, MTA binds in such a way that the adenine ring portion is exposed from the surface of the antibody.

As shown in Fig. 19, the adenine ring portion of MTA is recognized by the formation of a π-π interaction between each side chain of the light chain F95b and heavy chain F98 of the antibody. The ribose moiety is recognized by the formation of a hydrogen bond of the 2' position O, the 3' position O, and the side chain of the heavy chain E95. The thiomethyl group moiety forms a CH-π interaction with the side chain of the light chain F96. Furthermore, in addition to these interactions, MTA is surrounded by heavy chains W34, W47, C50, Y58, G99, and G100a and light chains Y28, T91, and Y95c, forming van der Waals interactions. From these interactions, it is inferred that MTA is more strongly recognized by the antibody. The amino acid residue numbering of Fab is based on the Kabat numbering scheme.

(5-5) NMR analysis of anti-MTA antibodies MTA0303 and MTA0330

(5-5-1) Preparation of MTA0303 and MTA0330 full-length antibodies for NMR

Preparation and purification of MTA0303 and MTA0330 full-length antibodies for NMR were carried out by methods known to those skilled in the art. However, to carry out stable isotope labeling of amino acids, 200 mg/L [d-$^{13}CH_3$, $^{15}N$, $^{2}H$]Isoleucine, 300 mg/L [d2-$^{13}CH_3$, $^{2}H$, $^{15}N$]Leucine, 190 mg/L [g1-$^{13}CH_3$, $^{2}H$, $^{15}N$]Valine, 360 mg/L [b-$^{13}CH_3$, $^{2}H$, $^{15}N$]Alanine, 60 mg/L β-chloro-L-Alanine were added to the medium.

(5-5-2) Preparation of Fab fragments for NMR analysis of MTA0303Fab and MTA0330Fab

Fab fragments of MTA0303 (MTA0303Fab) and MTA0330 (MTA0330Fab) were prepared by the conventional method of restriction digestion with Lys-C (Roche, Catalog No. 11 047 825 001), followed by the use of a protein A column (Mab Slect SuRe, GE Healthcare) to remove Fc fragments. Fractions containing Fab fragments were pooled, replaced with 5 mM d-citrate, pH 6.5, 20 mM NaCl, 5% $D_2O$ and concentrated to 0.15 mM to 0.3 mM.

(5-5-3) NMR spectrum measurement of MTA0303Fab and MTA0330Fab

Using the samples prepared in Example (5-5-2), the NMR spectrum was measured with Avance III 600 (Bruker). The measurement temperature was 305K. The trosyetf3gpiasi and sfhmqcf2gpph pulse programs (Bruker standard pulse programs) were used to acquire the $^{1}H$-$^{15}N$ TROSY spectrum and the $^{1}H$-$^{13}C$ SOFAST-HMQC spectrum, respectively. TopSpin (Bruker) was used for the Fourier transformation of the spectra.

(5-5-4) NMR spectra measurement of MTA0303Fab and MTA complex (MTA0303Fab-MTA complex) and MTA0330Fab and MTA complex (MTA0330Fab-MTA complex)

MTA was added to the samples prepared in Example (5-5-2) so that the final concentration was 0.4 mM, and the NMR spectrum was measured with Avance III 600. The measurement temperature was 305K. The trosyetf3gpiasi and sfhmqcf2gpph pulse programs (Bruker standard pulse programs) were used to acquire the $^{1}H$-$^{15}N$ TROSY spectrum and the $^{1}H$-$^{13}C$ SOFAST-HMQC spectrum, respectively. TopSpin (Bruker) was used for the Fourier transformation of the spectra.

(5-5-5) $^{1}H$-$^{15}N$ TROSY spectra comparison of MTA0303Fab and MTA0303Fab-MTA complex

Using Sparky (UCSF), which is an NMR spectrum display software, the NMR spectrum of MTA0303Fab and the NMR spectrum of MTA0303Fab-MTA complex were superimposed and the spectra were compared. The results are shown in Fig. 22. There were 17 signals where the chemical shift was changed by one peak or more in the MTA-bound and free states.

(5-5-6) $^{1}H$-$^{13}C$ SOFAST-HMQC spectra comparison of MTA0303Fab and MTA0303Fab-MTA complex

Using Sparky (UCSF), which is an NMR spectrum display software, the NMR spectrum of MTA0303Fab and the NMR spectrum of MTA0303Fab-MTA complex were superimposed and the spectra were compared. The results are shown in Fig. 23. There were 20 signals where the chemical shift was changed by one peak or more in the MTA-bound and free states.

(5-5-7) $^{1}H$-$^{15}N$ TROSY spectra comparison of MTA0330Fab and MTA0330Fab-MTA complex

Using Sparky (UCSF), which is an NMR spectrum display software, the NMR spectrum of MTA0330Fab and the NMR spectrum of MTA0330Fab-MTA complex were superimposed and the spectra were compared. The results are shown in Fig. 24. There were 7 signals where the chemical shift was changed by one peak or more in the MTA-bound and free states.

(5-5-8) $^{1}H$-$^{13}C$ SOFAST-HMQC spectra comparison of MTA0330Fab and MTA0330Fab-MTA complex

Using Sparky (UCSF), which is an NMR spectrum display software, the NMR spectrum of MTA0330Fab and the NMR spectrum of MTA0330Fab-MTA complex were superimposed and the spectra were compared. The results are shown in Fig. 25. There were 7 signals where the chemical shift was changed by one peak or more in the MTA-bound and free states.

[Example 6] Designing a library using anti-MTA antibodies obtained from rabbit B cell cloning as templates

Using the anti-MTA antibodies MTA0303 and MTA0330 obtained in Example 5 as templates, a library for obtaining antibodies that bind to an antigen in an MTA-dependent manner was designed.

(6-1) Humanization of antibodies MTA0303 and MTA0330 obtained from rabbit B cell cloning

Humanization of MTA0303 and MTA0330 was carried out by methods known to those skilled in the art (European Patent Publication EP239400, International Publication WO1996/00257, WO1993/012227, WO1992/003918, WO1994/002602, WO1994/025585, WO1996/034096, WO1996/033735, WO1992/001047, WO1992/020791, WO1993/006213, WO1993/011236, WO1993/019172, WO1995/001438, WO1995/015388, Cancer Res., (1993) 53, 851-856, BBRC., (2013) 436 (3): 543-50 etc.). The sequences of the humanized antibodies are shown in Table 20 below.

[Table 20]

| | H-chain variable region amino acid sequence | L-chain variable region amino acid sequence |
|---|---|---|
| Humanized MTA0303 | SEQ ID NO: 50 | SEQ ID NO: 51 |
| Humanized MTA0330 | SEQ ID NO: 52 | SEQ ID NO: 53 |

Humanized MTA0303 and humanized MTA0330 were expressed and purified by the method shown in Reference Example 1.

(6-2) Evaluation of MTA binding activity of humanized MTA0303 and humanized MTA0330 using surface plasmon resonance

Biacore T200 (GE Healthcare) was used to analyze binding to MTA and MTA analogs (adenosine, SAH) to verify whether humanized MTA0303 and humanized MTA0330 specifically bind to MTA.

The antibody of interest was captured on Sensor Chip Protein A (GE healthcare) with protein A pre-immobilized, or on an immobilized surface prepared by binding onto Streptavidinimmobilized Sensor chip SA (GE Healthcare) the Capture Select Biotin Anti-IgG-Fc (Hu) (Thermo fisher scientific), which is a biotinylated anti-human IgG CH1 molecule, and interacted with MTA (Sigma-Aldrich), or adenosine (Wako), or S-(5'-adenosyl)-L-homocysteine, (SAH) (Sigma-Aldrich). 20 mM ACES-NaOH, 150 mM NaCl, 0.05% Tween20, 0.05% DMSO was used as the running buffer. MTA or adenosine or SAH was allowed to interact at a flow rate of 30 μL/min for 120 seconds and then dissociated in the running buffer. The interaction was measured at 25°C and the same buffer as the running buffer was used to dilute MTA, or adenosine, or SAH.

The dissociation constant $K_D$ (M) was calculated based on the binding rate constant ka (1/Ms) and the dissociation rate constant kd (1/s), which are kinetic parameters calculated from the sensorgrams obtained by the measurement. Alternatively, the dissociation constant $K_D$ (M) was calculated using steady state analysis. Biacore T200 Evaluation Software (GE Healthcare) was used to calculate each parameter.

The binding activity of each antibody to MTA and MTA analogs was determined by this measurement as shown in Table 21, and it was confirmed that all of the humanized antibodies have high selectivity for MTA.

[Table 21]

| | Affinity $K_D$ [μmol/L] | | |
|---|---|---|---|
| | MTA | Adenosine | SAH |
| HumafiizedMTA0303 | 0.0108 | 41.4 | 211 |
| HumanizedMTA0330 | 0.00888 | 44.8 | 84.7 |

(6-3) Comprehensive evaluation of humanized MTA0303 variants

From the results of MTA0303 crystal structure analysis of Example (5-3), amino acid sites that are not significantly involved in MTA binding or amino acid sites of the antibody variable region that are expected to be likely involved in MTA-dependent binding to antigens were estimated for humanized MTA0303. Since these sites are thought to highly likely retain MTA-binding property even after diversification, they are considered to be candidates for diversification sites for making a library. Ala/Val substitution variants for amino acids at each site were prepared to identify sites that were not actually significantly involved in MTA binding among the estimated sites.

Modified sites of each MTA0303 heavy chain variant (sites represented by Kabat numbering and described as "Kabat" in the table) and the amino acids before modification at the sites (amino acids described as "native sequence" in the table) and modified amino acids (amino acids described as "modified amino acids" in the table) are shown in Table 22.

[Table 22]

| | HFR1 | HCDR1 | | | HCDR2 | | | | | | | | | | | HCDR3 | | | | | | | | |
|---|---|---|---|---|---|---|---|---|---|---|---|---|---|---|---|---|---|---|---|---|---|---|---|---|
| Kabat | 30 | 31 | 32 | 33 | 52a | 52b | 52c | 52d | 52e | 52f | 52g | 53 | 54 | 55 | 56 | 96 | 97 | 98 | 99 | 100 | 100a | 100b | 100c | 101 |
| Native sequence | S | S | A | Y | A | S | A | I | Y | A | G | S | G | G | S | Y | G | S | S | G | G | G | F | E |
| Modified sequence | A | A | V | A | V | A | V | A | A | V | A | A | A | A | A | A | A | A | A | A | A | A | A | A |
| KD ratio | 2.9 | 2.0 | 0.6 | 1.2 | - | 9.4 | 0.5 | 2.9 | 2.1 | 7.5 | 3.5 | - | - | - | - | 0.9 | - | 2.7 | 2.4 | 5.2 | - | 1.4 | 0.6 | 2.0 |

Modified sites of each MTA0303 light chain variant (sites represented by Kabat numbering and described as "Kabat" in the table), the amino acids before modification at the sites (amino acids described as "native sequence" in the table), and the modified amino acids (amino acids described as "modified amino acid" in the table) are shown in Table 23.

[Table 23]

| | LCDR1 | | | | | LFR2 | LCDR2 | | | | | | LCDR3 | | |
|---|---|---|---|---|---|---|---|---|---|---|---|---|---|---|---|
| Kabat | 27a | 29 | 30 | 31 | 32 | 49 | 50 | 51 | 52 | 53 | 54 | 56 | 93 | 94 | 95 |
| Native sequence | S | Y | A | N | R | Y | S | A | S | T | L | S | S | G | N |
| Modified sequence | A | A | V | A | A | A | A | V | A | A | A | A | A | A | A |
| KD ratio | 0.7 | 12.0 | 7.8 | 2.7 | 1.0 | 1.5 | 0.9 | - | 1.2 | - | 1.0 | 1.9 | 2.0 | - | - |

Using Biacore T200 (GE Healthcare), the binding activity between the prepared variants and MTA was evaluated. 20 mM ACES, 150 mM NaCl, 0.05% (w/v) Tween20, pH 7.4 was used as the running buffer, and the measurement was done at 25°C. By immobilizing ProA/G on Sensor Chip CM4 by amine coupling, immobilizing the antibody on it, and then interacting with MTA as an analyte, changes in the amount of binding between MTA and antibody were observed. The running buffer was used to dilute MTA, prepared in a concentration series of several steps, and the dissociation constant $K_D$ of each clone was calculated by single-cycle kinetics analysis from the sensorgram for the MTA concentrations. Biacore T200 Evaluation Software (GE Healthcare) was used to calculate the parameters. The ratio of the dissociation constant $K_D$ of humanized MTA0303 for MTA to the dissociation constant $K_D$ of each variant for MTA ($K_D$ of the humanized MTA0303 for MTA/$K_D$ of each variant for MTA) is shown in Table 22 (MTA0303 heavy chain variant) and Table 23 (MTA0303 light chain variant).

(6-4) Designing a library using humanized MTA0303 as a template

Among the variants evaluated for binding, sites that satisfy the following condition were selected as amino acid sites that can be diversified in designing the library based on the obtained information:
From the results of the comprehensive evaluation of the variants, amino acid sites that are not significantly involved in the binding to MTA.

Among the amino acid sites located in the heavy chain, the following amino acid sites were determined as amino acid sites that are diversifiable and satisfy Condition 1:

- amino acid sites where the amino acid residue is present on the surface side of the antibody, with side chain(s) exposed on the antibody surface, and wherein the amino acid residue is located on the assumed antigen binding surface,
- amino acid sites where the amino acid residue is presumed not to be significantly involved in binding to small molecules, and wherein the $K_D$ value of the parent antibody (humanized MTA0303) for MTA is 50% or more of the $K_D$ value for MTA of an MTA0303 variant in which the amino acid at the site is modified, or
- amino acid sites in which the $K_D$ value of the parent antibody (humanized MTA0303) for MTA is 70% or more of the $K_D$ value for MTA of the MTA0303 variant in which the amino acid at the site is modified,.

A library for obtaining antibodies that bind to an antigen in an MTA-dependent manner was constructed by designing a library in which at least one or more amino acids that can be made into a library appear at the determined diversifiable amino acid sites. All types of amino acids were used as amino acids that can be made into a library, and from the results of X-ray crystal structure analysis, amino acids that are expected to suppress the structural change of the antibody when MTA binds were excluded, only regarding the heavy chain S52b and light chain N31.

Table 24 shows the diversifiable amino acid sites in the heavy chain of humanized MTA0303 and the amino acid repertoire at the sites. Table 25 shows the diversifiable amino acid positions in the light chain of humanized MTA0303 and the amino acid repertoire at the sites. In the table, the sites represented by the Kabat numbering and described as "Kabat" show the diversifiable amino acid sites, and the amino acids described as "native sequence" show amino acids of humanized MTA0303 at the sites, and amino acids described as "amino acids that can be made into a library" show diversifiable amino acids at the sites. A library was designed in which at least one of the amino acids contained in the amino acid repertoire in the table appears at each diversifiable amino acid site in the heavy chain. A library was constructed in which at least one of the amino acids contained in the amino acid repertoire in the table appears at each diversifiable amino acid site of the light chain.

[Table 24]

| | | HFR1 | HCDR1 | | HCDR2 | | | | | HCDR3 | | | | | |
|---|---|---|---|---|---|---|---|---|---|---|---|---|---|---|---|
| | Kabat | 30 | 31 | 33 | 52b | 52c | 52d | 52f | 52g | 96 | 98 | 99 | 100 | 100b | 100c |
| | Native sequence | S | S | Y | S | A | I | A | G | Y | S | S | G | G | F |
| Amino acids that can be made into a library | A | A | A | A | A | A | A | A | A | A | A | A | A | A | A |
| | C | C | C | C | | C | C | C | C | C | C | C | C | C | C |
| | D | D | D | D | D | D | D | D | D | D | D | D | D | D | D |
| | E | E | E | E | E | E | E | E | E | E | E | E | E | E | E |
| | F | F | F | F | | F | F | F | F | F | F | F | F | F | F |
| | G | G | G | G | G | G | G | G | G | G | G | G | G | G | G |
| | H | H | H | H | H | H | H | H | H | H | H | H | H | H | H |
| | I | I | I | I | I | I | I | I | I | I | I | I | I | I | I |
| | K | K | K | K | K | K | K | K | K | K | K | K | K | K | K |
| | L | L | L | L | L | L | L | L | L | L | L | L | L | L | L |
| | M | M | M | M | M | M | M | M | M | M | M | M | M | M | M |
| | N | N | N | N | N | N | N | N | N | N | N | N | N | N | N |
| | P | P | P | P | P | P | P | P | P | P | P | P | P | P | P |
| | Q | Q | Q | Q | Q | Q | Q | Q | Q | Q | Q | Q | Q | Q | Q |
| | R | R | R | R | R | R | R | R | R | R | R | R | R | R | R |
| | S | S | S | S | S | S | S | S | S | S | S | S | S | S | S |
| | T | T | T | T | T | T | T | T | T | T | T | T | T | T | T |
| | V | V | V | V | V | V | V | V | V | V | V | V | V | V | V |
| | W | W | W | W | | W | W | W | W | W | W | W | W | W | W |
| | Y | Y | Y | Y | | Y | Y | Y | Y | Y | Y | Y | Y | Y | Y |

[Table 25]

| | | LCDR1 | | | | | LFR2 | LCDR2 | | | | LCDR3 |
|---|---|---|---|---|---|---|---|---|---|---|---|---|
| | Kabat | 27a | 29 | 30 | 31 | 32 | 49 | 50 | 52 | 54 | 56 | 93 |
| | Native sequence | S | Y | A | N | R | Y | S | S | L | S | S |
| Amino acids that can be made into a library | A | A | A | A | A | A | A | A | A | A | A | A |
| | C | C | C | C | | C | C | C | C | C | C | C |
| | D | D | D | D | D | D | D | D | D | D | D | D |
| | E | E | E | E | E | E | E | E | E | E | E | E |
| | F | F | F | F | | F | F | F | F | F | F | F |
| | G | G | G | G | G | G | G | G | G | G | G | G |
| | H | H | H | H | H | H | H | H | H | H | H | H |
| | I | I | I | I | I | I | I | I | I | I | I | I |
| | K | K | K | K | K | K | K | K | K | K | K | K |
| | L | L | L | L | L | L | L | L | L | L | L | L |
| | M | M | M | M | M | M | M | M | M | M | M | M |
| | N | N | N | N | N | N | N | N | N | N | N | N |
| | P | P | P | P | P | P | P | P | P | P | P | P |
| | Q | Q | Q | Q | Q | Q | Q | Q | Q | Q | Q | Q |
| | R | R | R | R | R | R | R | R | R | R | R | R |
| | S | S | S | S | S | S | S | S | S | S | S | S |
| | T | T | T | T | T | T | T | T | T | T | T | T |
| | V | V | V | V | V | V | V | V | V | V | V | V |
| | W | W | W | W | | W | W | W | W | W | W | W |
| | Y | Y | Y | Y | | Y | Y | Y | Y | Y | Y | Y |

(6-5) Comprehensive evaluation of humanized MTA0330 variants

From the results of crystal structure analysis of MTA0330 in Example (5-4), amino acid sites in humanized MTA0330 that are not significantly involved in MTA binding or amino acid sites of the antibody variable region that are expected to be likely involved in the MTA-dependent binding with an antigen were estimated. Since these sites are thought to highly likely retain MTA binding even after diversification, they are considered to be candidates for diversification sites for making a library. Ala-substitution variants for amino acids at each site were prepared to identify sites that were not actually significantly involved in MTA binding among the estimated sites.

The modified sites of each MTA0330 heavy chain variant (sites represented by Kabat numbering and described as "Kabat" in the table), the amino acids before modification at these sites (amino acids described as "native sequence" in the table), and the modified amino acids (amino acids described as "modified amino acid" in the table) are shown in Table 26.

[Table 26]

| | HCDR1 | | | | HCDR2 | | | | | | HCDR3 | | | |
|---|---|---|---|---|---|---|---|---|---|---|---|---|---|---|
| Kabat | 31 | 32 | 33 | 34 | 52 | 53 | 54 | 55 | 56 | 58 | 96 | 97 | 98 | 99 |
| Native sequence | S | S | Y | W | Y | G | D | G | S | Y | N | I | F | G |
| Modified sequence | A | A | A | A | A | A | A | A | A | A | A | A | A | A |
| KD ratio | 1.0 | 0.9 | 0.6 | - | 0.4 | 1.7 | 0.9 | 1.3 | 1.1 | 0.5 | 0.5 | 0.9 | 0.7 | 0.9 |

The modified sites of each MTA0330 light chain variant (sites represented by Kabat numbering and described as "Kabat" in the table), the amino acids before modification at these sites (amino acids described as "native sequence" in the table), and the modified amino acids (amino acids described as "modified amino acid" in the table) are shown in Table 27.

[Table 27]

| | LCDR1 | | | | | | LFR2 | LCDR2 | | | LCDR3 | | | | |
|---|---|---|---|---|---|---|---|---|---|---|---|---|---|---|---|
| Kabat | 27a | 28 | 29 | 30 | 31 | 32 | 49 | 50 | 52 | 53 | 93 | 94 | 95 | 95a | 95b |
| Native sequence | S | Y | S | N | N | R | Y | Q | S | T | Y | S | S | G | F |
| Modified sequence | A | A | A | A | A | A | A | A | A | A | A | A | A | A | A |
| KD ratio | 1.2 | 2.3 | 1.0 | 1.8 | 1.0 | 1.4 | 0.7 | 1.3 | 1.1 | 1.0 | 1.5 | 1.0 | 1.1 | 0.9 | - |

Biacore T200 (GE Healthcare) was used to evaluate the binding activity between the prepared variants and MTA. 20 mM ACES, 150 mM NaCl, 0.05% (w/v) Tween20, pH 7.4 was used as the running buffer, and the measurement was performed at 25°C. By immobilizing ProA/G on Sensor Chip CM4 by amine coupling, immobilizing the antibody on it, and then interacting with MTA as an analyte, changes in the amount of binding between MTA and antibody were observed. The running buffer was used to dilute MTA, prepared in a concentration series of several steps, and the dissociation constant $K_D$ of each clone was calculated by single-cycle kinetics analysis from the sensorgram for the MTA concentrations. Biacore T200 Evaluation Software (GE Healthcare) was used to calculate the parameters. The ratio of the dissociation constant $K_D$ of humanized MTA0330 to MTA and the dissociation constant $K_D$ of each variant to MTA ($K_D$ of humanized MTA0330 for MTA/$K_D$ of each variant for MTA) is shown in Table 26 (MTA0330 heavy chain variant) and Table 27 (MTA0330 light chain variant).

(6-6) Designing a library using humanized MTA0303 and humanized MTA0330 as templates

Among the variants evaluated for binding, sites that satisfy the following condition were selected as amino acid sites that can be diversified in designing the library based on the obtained information:
From the results of the comprehensive evaluation of the variants, amino acid sites that are not significantly involved in the binding to MTA.

Amino acid sites located in the heavy chain and where the $K_D$ value of the parent antibody (humanized MTA0330) for MTA is 40% or more of the $K_D$ value for MTA of an MTA0330 variant in which the amino acid at the site is modified, or amino acid sites located in the light chain and where the $K_D$ value of the parent antibody (humanized MTA0330) for MTA is 70% or more of the $K_D$ value for MTA of an MTA0330 variant in which the amino acid at the site is modified, were determined as amino acid sites that are diversifiable and satisfy Condition 1.

A library for obtaining antibodies that bind to an antigen in an MTA-dependent manner was constructed by designing a library in which at least one or more amino acids that can be made into a library appear at the determined diversifiable amino acid sites. All kinds of amino acids were adopted as amino acids that can be made into a library.

Table 28 shows the diversifiable amino acid sites in the heavy chain of humanized MTA0330 and the amino acid repertoire at the sites. Table 29 shows the diversifiable amino acid positions in the light chain of humanized MTA0330 and the amino acid repertoire at the sites. In the table, the sites represented by Kabat numbering and described as "Kabat" show diversifiable amino acid sites, the amino acids described as "native sequence" show amino acids of humanized MTA0330 at the sites, and the amino acids described as "amino acids that can be made into a library" show diversifiable amino acids at the sites, respectively. A library was designed in which at least one of the amino acids contained in the amino acid repertoire in the table appears at each diversifiable amino acid site in the heavy chain. A library was constructed in which at least one of the amino acids contained in the amino acid repertoire in the table appears at each diversifiable amino acid site of the light chain.

[Table 28]

| | | HCDR1 | | | HCDR2 | | | | | | HCDR3 | | | |
|---|---|---|---|---|---|---|---|---|---|---|---|---|---|---|
| | Kabat | 31 | 32 | 33 | 52 | 53 | 54 | 55 | 56 | 58 | 96 | 97 | 98 | 99 |
| | Native sequence | S | S | Y | Y | G | D | G | S | Y | N | I | F | G |
| Amino acids that can be made into a library | A | A | A | A | A | A | A | A | A | A | A | A | A | A |
| | C | C | C | C | C | C | C | C | C | C | C | C | C | C |
| | D | D | D | D | D | D | D | D | D | D | D | D | D | D |
| | E | E | E | E | E | E | E | E | E | E | E | E | E | E |
| | F | F | F | F | F | F | F | F | F | F | F | F | F | F |
| | G | G | G | G | G | G | G | G | G | G | G | G | G | G |
| | H | H | H | H | H | H | H | H | H | H | H | H | H | H |
| | I | I | I | I | I | I | I | I | I | I | I | I | I | I |
| | K | K | K | K | K | K | K | K | K | K | K | K | K | K |
| | L | L | L | L | L | L | L | L | L | L | L | L | L | L |
| | M | M | M | M | M | M | M | M | M | M | M | M | M | M |
| | N | N | N | N | N | N | N | N | N | N | N | N | N | N |
| | P | P | P | P | P | P | P | P | P | P | P | P | P | P |
| | Q | Q | Q | Q | Q | Q | Q | Q | Q | Q | Q | Q | Q | Q |
| | R | R | R | R | R | R | R | R | R | R | R | R | R | R |
| | S | S | S | S | S | S | S | S | S | S | S | S | S | S |
| | T | T | T | T | T | T | T | T | T | T | T | T | T | T |
| | V | V | V | V | V | V | V | V | V | V | V | V | V | V |
| | W | W | W | W | W | W | W | W | W | W | W | W | W | W |
| | Y | Y | Y | Y | Y | Y | Y | Y | Y | Y | Y | Y | Y | Y |

[Table 29]

| | | LCDR1 | | | | | | LFR2 | LCDR2 | | | LCDR3 | | | |
|---|---|---|---|---|---|---|---|---|---|---|---|---|---|---|---|
| | Kabat | 27a | 28 | 29 | 30 | 31 | 32 | 49 | 50 | 52 | 53 | 93 | 94 | 95 | 95a |
| | Native sequence | S | Y | S | N | N | R | Y | Q | S | T | Y | S | S | G |
| Amino acids that can be made into a library | A | A | A | A | A | A | A | A | A | A | A | A | A | A | A |
| | C | C | C | C | C | C | C | C | C | C | C | C | C | C | C |
| | D | D | D | D | D | D | D | D | D | D | D | D | D | D | D |
| | E | E | E | E | E | E | E | E | E | E | E | E | E | E | E |
| | F | F | F | F | F | F | F | F | F | F | F | F | F | F | F |
| | G | G | G | G | G | G | G | G | G | G | G | G | G | G | G |
| | H | H | H | H | H | H | H | H | H | H | H | H | H | H | H |
| | I | I | I | I | I | I | I | I | I | I | I | I | I | I | I |
| | K | K | K | K | K | K | K | K | K | K | K | K | K | K | K |
| | L | L | L | L | L | L | L | L | L | L | L | L | L | L | L |
| | M | M | M | M | M | M | M | M | M | M | M | M | M | M | M |
| | N | N | N | N | N | N | N | N | N | N | N | N | N | N | N |
| | P | P | P | P | P | P | P | P | P | P | P | P | P | P | P |
| | Q | Q | Q | Q | Q | Q | Q | Q | Q | Q | Q | Q | Q | Q | Q |
| | R | R | R | R | R | R | R | R | R | R | R | R | R | R | R |
| | S | S | S | S | S | S | S | S | S | S | S | S | S | S | S |
| | T | T | T | T | T | T | T | T | T | T | T | T | T | T | T |
| | V | V | V | V | V | V | V | V | V | V | V | V | V | V | V |
| | W | W | W | W | W | W | W | W | W | W | W | W | W | W | W |
| | Y | Y | Y | Y | Y | Y | Y | Y | Y | Y | Y | Y | Y | Y | Y |

(6-7) Acquisition of MTA-dependent antibodies from a library using humanized MTA0330 as a template

(6-7-1) Construction of a humanized MTA0330 heavy chain or light chain variable region phage display library

The heavy chain variable region library designed using the humanized MTA0330 constructed in Example (6-6) as a template was genetically synthesized, and the synthesized heavy chain variable region of humanized MTA0330 was introduced into a phagemid vector containing the light chain variable region of humanized MTA0330 and human IgG-derived heavy chain CH1 sequence and human IgG-derived light chain constant region sequence. The light chain variable region library designed using humanized MTA0330 as a template was genetically synthesized using a similar method, and the synthesized humanized MTA0330 light chain variable region was introduced into a phagemid vector containing the heavy chain variable region of humanized MTA0330 and the human IgG-derived heavy chain CH1 sequence and the human IgG-derived light chain constant region sequence. By introducing the prepared phagemid vector into *E. coli* by electroporation, a phage display library that allows obtainment of heavy chain/light chain variable regions capable of binding to an antigen in an MTA-dependent manner was constructed.

(6-7-2) Acquisition of antibodies that bind to MTA by panning using the heavy chain and light chain variable region phage display library

From each of the phage display libraries of the heavy chain variable region and the light chain variable region constructed in Example (6-7-1), panning was carried out using biotinylated MTA, to obtain a population of phages having antibody variable regions that bind to MTA. Specifically, *E. coli* carrying the constructed phagemid vector were infected with M13KO7ΔpIII (called hyperphage) (PROGEN Biotechnik), and the phages were collected from the supernatant of an overnight culture at 25°C. The culture solution *of E. coli* in which phages were produced was replaced with TBS as a solvent to prepare an antibody multivalent display phage library solution.

Panning was done using the antigen immobilized on magnetic beads. 12% BSA was added to the phage library solution. NeutrAvidin beads (TAMAGAWA SEIKI) or Dynabeads MyOne Streptavidin T1 (Thermo Fisher Scientific) was used as the magnetic beads. BSA-blocked magnetic beads, biotin-6'-MTA at a final concentration of 5.7 μM and 0.91 mM adenosine were added to 0.3 mL of the prepared phage library solution and reacted at room temperature for 60 minutes. The beads were washed twice with 400 μL TBST and once with TBS. The beads added with 0.5 mL of TBS containing trypsin at a final concentration of 1 mg/mL were then suspended at room temperature for 15 minutes. The phage solution was collected from the beads separated using a magnetic stand. The collected phages were added to *E. coli* strain ER2738 during the logarithmic growth phase (OD600 0.4-0.7). *E. coli* were infected with the phages by stirring and culturing the above *E. coli* at 37°C for 1 hour. This series of operations was repeated once more.

(6-7-3) Construction of a library for obtaining MTA-dependent antibodies by panning against MTA

With regard to the population of heavy and light chains that bind to MTA obtained from the heavy and light chain variable region phage display libraries, respectively, a library of Fab-presenting phages constructed by combining the heavy and light chains of the population was also expected to contain many clones that maintain the binding to MTA, and thus, it was expected that a library that allows more efficient obtainment of MTA-dependent antibodies could be constructed..

Genes were extracted by a method known to those skilled in the art from *E. coli* infected with each of the phage libraries of heavy and light chains obtained by panning against MTA. In the post-panning heavy chain variable region phage library, the heavy chain variable region gene was amplified using primers (SEQ ID NOs: 88, 89) that allow amplification of amplifying the heavy chain variable region.

In the post-panning light chain variable region phage library, the heavy chain variable region sequence of humanized MTA0330 was extracted by restriction enzyme treatment, and a phagemid fragment containing the light chain variable region library gene, human IgG-derived light chain constant region sequence, and human IgG-derived CH1 sequence was prepared. A phagemid vector into which the heavy chain/light chain variable region library gene was introduced was constructed by inserting the heavy chain variable region library gene into the prepared phagemid vector fragment containing the light chain variable region library gene. By introducing this vector into *E. coli* by electroporation, a library that displays a Fab domain consisting of the human antibody variable region and constant region, and which enables isolation of antibodies that can bind to an antigen in an MTA-dependent manner was constructed.

(6-7-4) Panning for obtaining MTA-dependent antibodies from an M30 library

The constructed phage display library for obtaining MTA-dependent antibodies (called M30 library) was screened for antibodies showing binding activity to each of human IL-6 receptor (hIL-6R), human IL-6 (hIL-6), and human

IgA (hIgA) in the presence of MTA.

Specifically, *E. coli* carrying the constructed phagemid vector were infected with M13KO7ΔpIII (called hyperphage) (PROGEN Biotechnik), and phages were collected from the supernatant of an overnight culture at 25°C. The culture solution *of E. coli* in which phages were produced was replaced with TBS as a solvent to prepare an antibody multivalent display phage library solution.

Panning was performed using each antigen immobilized on magnetic beads as a different condition. 10% BSA was added to the phage library solution. To 0.8 mL of the prepared phage library solution, 0.1 nmol of biotin-labeled antigen and MTA having a final concentration of 100 μM were added, and the mixture was reacted at room temperature for 60 minutes. NeutrAvidin beads (TAMAGAWA SEIKI) or Dynabeads MyOne Streptavidin T1 (Thermo Fisher Scientific) was used as the magnetic beads. Magnetic beads blocked with BSA were added to the reaction solution of phages and antigen and reacted at room temperature for 15 minutes. The beads were washed 2 or 3 times with 0.8 mL TBST and 1 or 2 times with TBS. The beads to which 0.25 mL of TBS was added were then suspended at room temperature, and the phage solution was collected from the beads separated using a magnetic stand. After repeating this process again, the two separate elution of phage solutions were combined. A final concentration of 1 mg/mL trypsin was added to the collected phage solution. The collected phages were added to *E. coli* strain ER2738 during the logarithmic growth phase (OD600 0.4-0.7). *E. coli* were infected with the phages by stirring and culturing the above E. *coli* at 37°C for 1 hour. *E. coli* were seeded onto a 225 mm x 225 mm plate. This series of operations was repeated for two additional rounds.

(6-7-5) Evaluation of antigen-binding activity in the presence of MTA by flow cytometry

The variable region sequences of the heavy and light chains obtained by panning were inserted into an animal expression plasmid having a heavy chain antibody constant region sequence (SEQ ID NO: 86) or a light chain kappa constant region sequence (SEQ ID NO: 87). The prepared plasmid was introduced into the seeded Expi293 strain (Thermo Fisher Scientific) by the lipofection method. Supernatant of a culture incubated in a $CO_2$ incubator (37°C, 8% $CO_2$, 1000 rpm) for 4 days was collected.

The culture supernatant was subjected to antigen binding evaluation using flow cytometry according to the following procedure. To a 384-well microplate (Greiner), magnetic beads onto which each biotin-labeled antigen was immobilized, culture supernatant, 0.1% BSA/5 mM $Mg_2CL_2$/PBS or 0.1% BSA/5 mM $Mg_2CL_2$/PBS containing MTA at a final concentration of 100 μM were added, and this was left to stand for 30 minutes or more. The beads on the plate were washed with 0.1% BSA/5 mM $Mg_2CL_2$/PBS or 0.1% BSA/5 mM $Mg_2CL_2$/PBS containing 100 μM MTA, and then to the wells were added PBS or DyLight488-labeled anti-human Fc antibody (Invitrogen) diluted with 0.1% BSA/5 mM $Mg_2CL_2$/PBS containing MTA at a final concentration of 100 μM, and this was allowed to stand for 30 minutes or longer. The wells were washed with 0.1% BSA/5 mM $Mg_2CL_2$/PBS or 0.1% BSA/5 mM $Mg_2CL_2$/PBS containing MTA at a final concentration of 100 μM, and then the fluorescence intensity of the samples was measured with iQue screener (Intellicyt). As a result of the analysis, multiple antibodies that bind to the antigen in an MTA-dependent manner were confirmed under each condition of panning using each of biotin-labeled hIL-6R, hIL-6, and hIgA. The results are shown in Table 31.

[Table 31]

| Target antigen | Panning rounds | Number of assessed antibodies | Antibodies binding to an antigen in an MTA-specific and MTA-dependent manner | Antibodies binding to an antigen in both an adenosineand MTA-dependent manner |
|---|---|---|---|---|
| hIL-6R | 3 | 96 | 43 | 43 |
| HIL-6 | 3 | 96 | 5 | 5 |
| hIgA | 3 | 96 | 5 | 5 |

From this result, multiple clones were obtained that could bind to multiple antigens with different sequences and structures in the presence of MTA, but that could not bind to the antigens in the absence of MTA. In addition, all of the obtained clones did not show binding to the antigens in the presence of adenosine, indicating that antibodies that bind to an antigen in an MTA-specific and MTA concentration-dependent manner can be obtained.

[Example 7] Construction of a library for obtaining antibodies that bind to an antigen in an adenosine-dependent manner

(7-1) Concentration of a group of antibodies that bind to adenosine using heavy chain and light chain variable region phage display libraries

Panning was performed using a mixture of biotin-2'-adenosine and biotin-5'-adenosine (biotin-adenosine mixture) to concentrate a group of antibodies that bind to adenosine from each of the heavy chain variable region phage display library and the light chain variable region phage display library constructed in Example (4-1-1).

Specifically, *E. coli* carrying the phagemid vector of the constructed heavy chain variable region phage display library or light chain variable region phage display library were infected with M13KO7ΔpIII (called hyperphage) (PROGEN Biotechnik), and phages were collected from the supernatant of an overnight culture at 25°C. An antibody multivalent display phage library solution was prepared by diluting with TBS the phage population precipitated by adding 2.5 M NaCl/10% PEG to the *E. coli* culture solution in which the phages were produced.

Panning was done using a biotin-adenosine mixture immobilized on magnetic beads. BSA was added to the antibody multivalent display phage library solution to a final concentration of 4%. NeutrAvidin beads (TAMAGAWA SEIKI) or Dynabeads MyOne Streptavidin T1 (Thermo Fisher Scientific) was used as the magnetic beads. The biotin-adenosine mixture was immobilized on the magnetic beads by reacting BSA-blocked magnetic beads with the biotin-adenosine mixture at a final concentration of 10 μM at room temperature for 30 minutes. Magnetic beads onto which the biotin-adenosine mixture was immobilized were washed three times with TBST, and 0.8 mL of the prepared antibody multivalent display phage library solution was added, and the mixture was reacted at room temperature for 60 minutes. The beads were washed twice with 400 μL TBST and once with TBS. The beads added with 0.5 mL of TBS containing trypsin at a final concentration of 1 mg/mL were then suspended at room temperature for 15 minutes, and the phages were eluted. The beads were separated using a magnetic stand and the phage solution was collected. The collected phages were added to 20 mL *of E. coli* strain ER2738 during the logarithmic growth phase (OD600 0.4-0.7). *E. coli* were infected with the phages by stirring and culturing the above *E. coli* at 37°C for 1 hour. *E. coli were* seeded onto a 225 mm x 225 mm plate. This series of operations was repeated one additional time, and a group of antibodies that bind to adenosine was concentrated from each of the heavy chain variable region phage display library and the light chain variable region phage display library.

(7-2) Evaluation of the binding activity to biotinylated adenosine of clones obtained after panning by phage ELISA

From a single colony *of E. coli* infected with phages displaying antibodies of the group of antibodies that bind to adenosine concentrated in Example (7-1), a phage-containing culture supernatant was collected according to a conventional method (Methods Mol. Biol. (2002) 178, 133-145). Hyperphages were used as helper phage, and antibody multivalent display phages were collected and subjected to ELISA. To a 384-well streptavidin-coated microplate (Greiner), 10 μL of TBS containing a biotin-adenosine mixture was added, and the mixture was allowed to stand for 1 hour or more. After washing each well of the plate with TBST, each well was blocked with 80 uL of 0.2% skim milk-TBS for 1 hour or more. Each well was washed with TBST, and then the prepared phages were added to each well and allowed to stand for 1 hour to bind the phage-presented antibodies to the biotin-adenosine mixture. Each well was washed with TBST, HRP-conjugated anti-M13 antibody (GE Healthcare) diluted with TBS was then added to each well and allowed to stand for 1 hour. After washing each well with TBST, TMB single solution (ZYMED) was added, and after a certain period, the coloring reaction of the solution was stopped by adding sulfuric acid, and then the absorbance at 450 nm wavelength was measured. As a result of the analysis, a plurality of phages displaying antibodies that bind to biotin-2'-adenosine were confirmed. The results of phage ELISA are shown in Table 30. From the results of ELISA, it was shown that many adenosine-binding antibodies were contained in the group of antibodies concentrated by the panning of Example (7-1).

[Table 30]

| | Adenosine binding rate (the number of clones that bound to biotin-2'-adenosine among the 192 clones evaluated by ELISA) |
|---|---|
| Group of antibodies concentrated from the heavy chain variable region phage display library | 22/96 |
| Group of antibodies concentrated from the light chain variable region phage display library | 90/96 |

(7-3) Construction of a library for obtaining antibodies that bind to an antibody in an adenosine-dependent manner using panning against adenosine

With regard to the group of adenosine-binding antibodies concentrated in Example (7-2), the Fab-presenting phage library constructed by combining the modified heavy chains and modified light chains contained in the group of antibodies was also expected to contain many antibodies that maintain the binding to adenosine, and it was thought that a library for obtaining antibodies that bind to an antigen in an adenosine-dependent manner could be constructed.

Antibody genes were extracted by a method known to those skilled in the art from E. *coli* infected with phages presenting the group of adenosine-binding antibodies concentrated in Example (7-2). The heavy chain variable region gene was amplified using primers (SEQ ID NOs: 34, 35) that allow amplification of amplifying the heavy chain variable region for the group of antibodies concentrated from the heavy chain variable region phage library. For the light chain variable region phage library and the group of antibodies concentrated from the library, the heavy chain variable region sequence of humanized SMB0002 was removed by restriction enzyme treatment, and a phagemid fragment containing the light chain variable region library gene and the human IgG-derived light chain constant region sequence and the human IgG-derived CH1 sequence was prepared. A phagemid vector into which the heavy chain/light chain variable region library gene was introduced was constructed by inserting the heavy chain variable region gene amplified from the heavy chain variable region library into the phagemid vector fragment. By introducing this vector into *E. coli* by electroporation, a library that displays a Fab domain consisting of the human antibody variable region and constant region, and that enables isolation of antibodies that can bind to an antigen in an adenosine-dependent manner (adenosine-concentrated S02 library) was constructed.

[Reference Example 11 Expression and purification of antibodies

The antibody proteins were expressed using the following method. The prepared plasmid was introduced into a seeded human fetal kidney cell-derived FreeStyle 293 strain or Expi 293 strain (Thermo Fisher Scientific) by the lipofection method. Antibodies were purified by a method known to those skilled in the art using rProtein A Sepharose Fast Flow (Amersham Biosciences) or MonoSpin ProA (GL Sciences) from supernatants of cultures incubated in a $CO_2$ incubator (37°C, 8% $CO_2$, 1000 rpm) for 4 days. The absorbance of the purified antibody solution at 280 nm was measured using a . The concentration of the purified antibodies was calculated from the obtained measured values using the extinction coefficient calculated by the PACE method (Protein Science (1995) 4, 2411-2423).

[Industrial applicability]

The antigen-binding domains of the present disclosure whose antigen-binding activity changes in an MTA-dependent manner and antigen-binding molecules comprising the antigen-binding domains, and pharmaceutical compositions comprising them do not act systemically in normal tissues or in blood, and by binding to and acting on a target antigen in a cancer, they can exert a drug effect while avoiding side effects and treat the cancer.

Furthermore, by using a library of the present disclosure that contains a plurality of antigen-binding domains having mutually different sequences whose antigen-binding activity changes in an MTA-dependent manner, or antigen-binding molecules containing the antigen-binding domains, it is possible to efficiently obtain antigen-binding domains whose antigen-binding activity changes in an MTA-dependent manner and antigen-binding molecules comprising the antigen-binding domains in a short time.

## Claims

1. An antigen-binding molecule comprising an antigen-binding domain whose antigen-binding activity changes in a 5'-methylthioadenosine (MTA)-dependent manner.

2. The antigen-binding molecule according to claim 1, wherein the antigen-binding activity of the antigen-binding domain in the presence of MTA is different from the antigen-binding activity in the absence of MTA.

3. The antigen-binding molecule according to claim 1 or 2, wherein the antigen-binding domain comprises an antibody variable region and/or a single-domain antibody.

4. The antigen-binding molecule according to any one of claims 1 to 3, wherein the antigen-binding activity of the antigen-binding domain in the presence of MTA is stronger than the antigen-binding activity of the antigen-binding domain in the absence of MTA.

5. The antigen-binding molecule according to any one of claims 1 to 4, wherein the antigen is a membrane-type molecule and which is expressed in a cancer tissue.

6. A pharmaceutical composition comprising the antigen-binding molecule according to any one of claims 1 to 5.

7. A library mainly consisting of a plurality of antigen-binding molecules comprising antigen-binding domains differing in sequence from one another and/or nucleic acids encoding a plurality of antigen-binding molecules comprising antigen-binding domains differing in sequence from one another, wherein the antigen-binding domains are antigen-binding domains that interact with MTA.

8. The library according to claim 7, wherein the antigen-binding domains are antibody variable regions.

9. The library according to claim 8, wherein the library comprises a plurality of antibody variable region variants differing in sequence from one another and having amino acids different to amino acids located at one or more amino acid sites in an unmodified antibody variable region having a binding activity towards MTA, and/or nucleic acids encoding each of a plurality of antibody variable region variants differing in sequence from one another and having amino acids different to amino acids located in one or more amino acid sites in an unmodified antibody variable region having a binding activity towards MTA.

10. A method of producing a library comprising the following steps (a) and (b):

(a) identifying an amino acid site that satisfies at least one or more of the following (i) to (vi) in an antigen-binding domain having MTA-binding activity:

(i) an amino acid site exposed on the surface of the antigen-binding domain;
(ii) an amino acid site located in a region where the rate of structural change is large when the structure is compared between when the antigen-binding domain is bound to MTA and when it is not bound to MTA;
(iii) an amino acid site that is not involved in the binding to MTA;
(iv) an amino acid site that does not significantly weaken the binding to MTA;
(v) an amino acid site with diverse amino acid occurrence frequencies in the animal species to which the antigen-binding domain belongs; or
(vi) an amino acid site which is not important for canonical structure formation; and

(b) designing a library comprising a nucleic acid encoding an unmodified antigen binding domain, and nucleic acids encoding a plurality of variants of the antigen-binding domain differing in sequence from one another and having an amino acid modification at one or more amino acid sites identified in step (a).

11. A method of screening for an antigen-binding molecule comprising an antigen-binding domain whose antigen-binding activity changes in an MTA-dependent manner.

12. The method according to claim 11, wherein the method further comprises the step of selecting an antigen-binding domain whose antigen-binding activity in the presence of a first concentration of MTA and the antigen-binding activity in the presence of a second concentration of MTA are different.

13. A method of producing an antigen-binding molecule comprising an antigen-binding domain whose antigen-binding activity changes in an MTA-dependent manner.

14. An antigen-binding molecule that specifically binds to MTA.

15. A kit for diagnosing a disease, wherein the kit comprises the antigen-binding molecule according to claim 14.

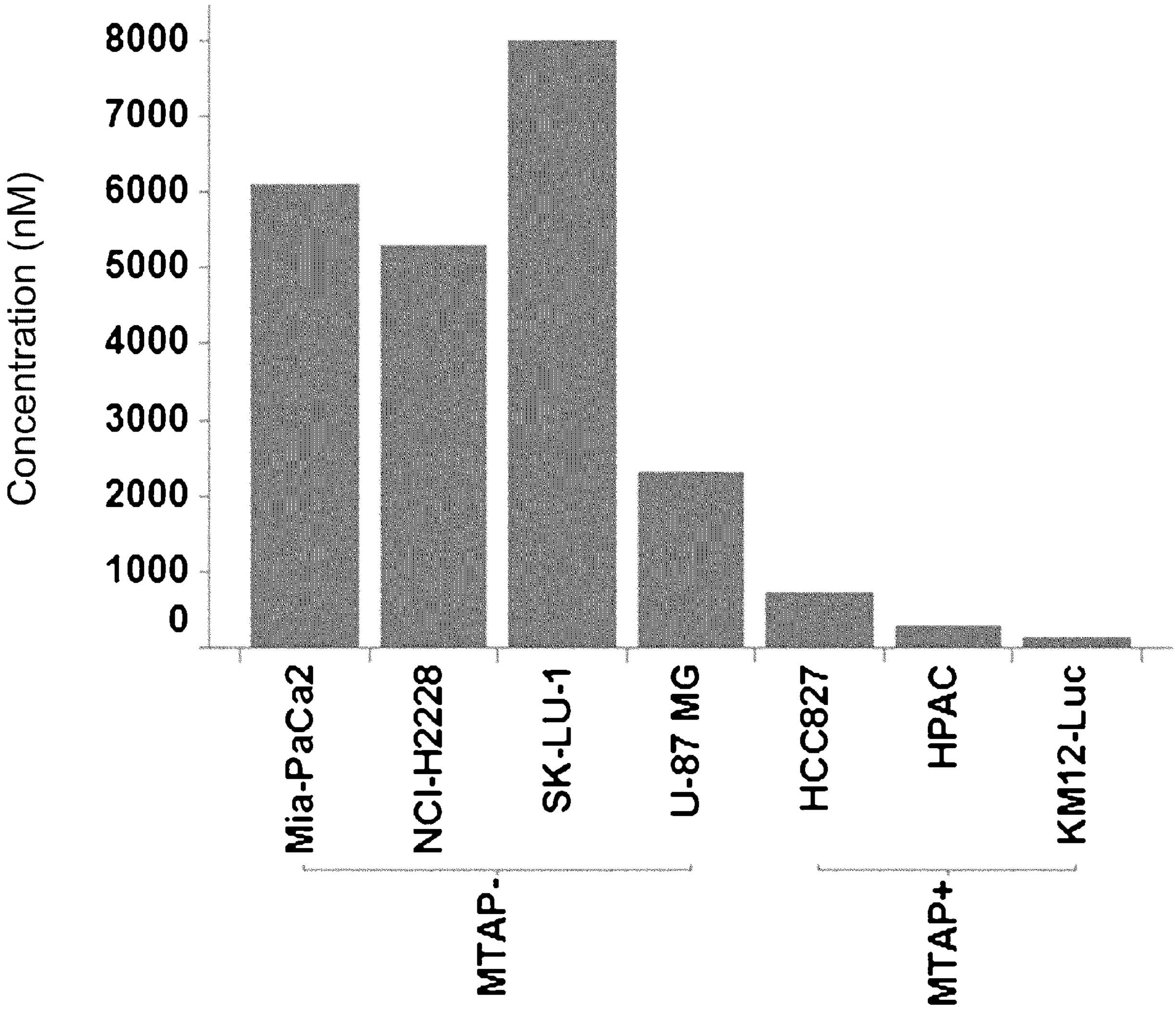
Concentration (nM)
8000
7000
6000
5000
4000
3000
2000
1000
0
Mia-PaCa2
NCI-H2228
SK-LU-1
U-87 MG
HCC827
HPAC
KM12-Luc
MTAP-
MTAP+

FIG. 1

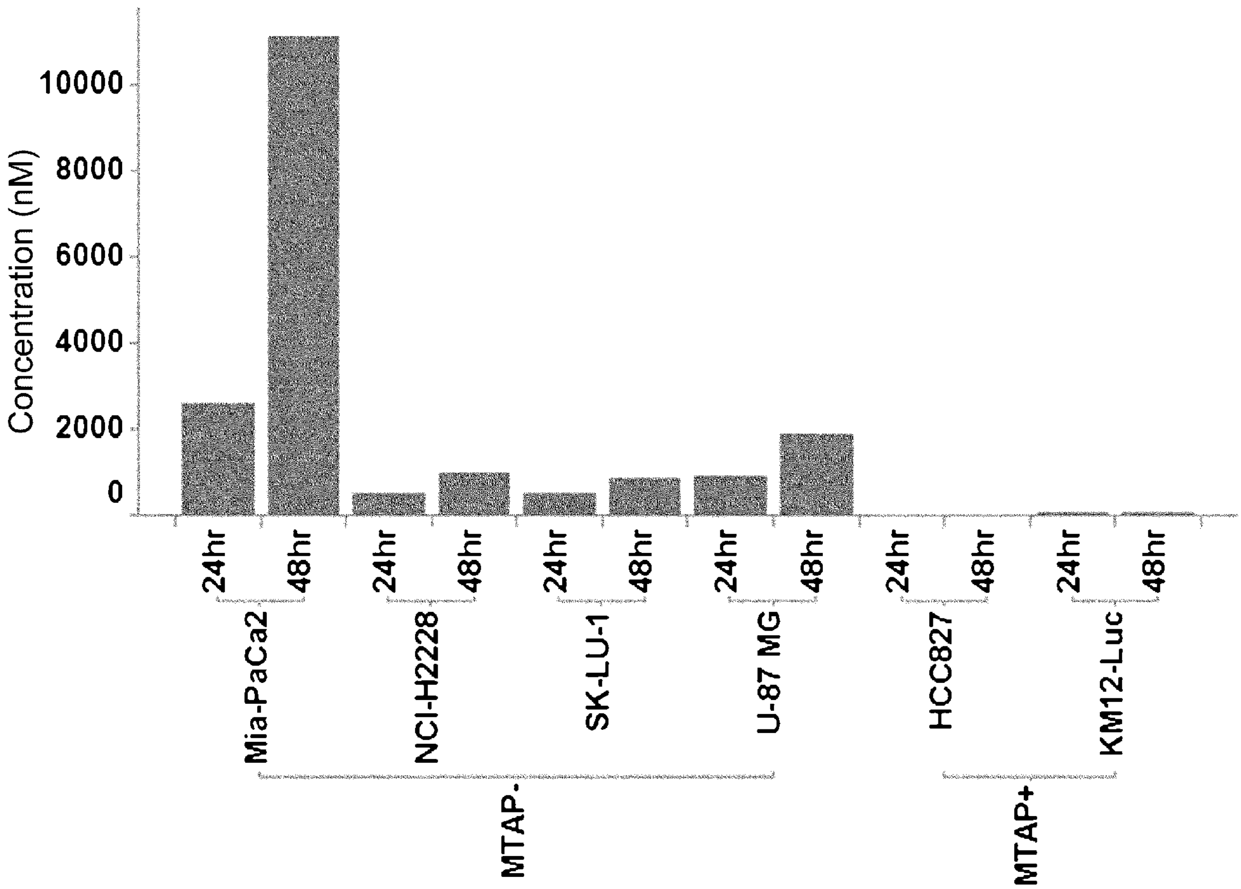
Concentration (nM)
10000
8000
6000
4000
2000
0
24hr
48hr
Mia-PaCa2
NCI-H2228
SK-LU-1
U-87 MG
HCC827
KM12-Luc
MTAP-
MTAP+

FIG. 2

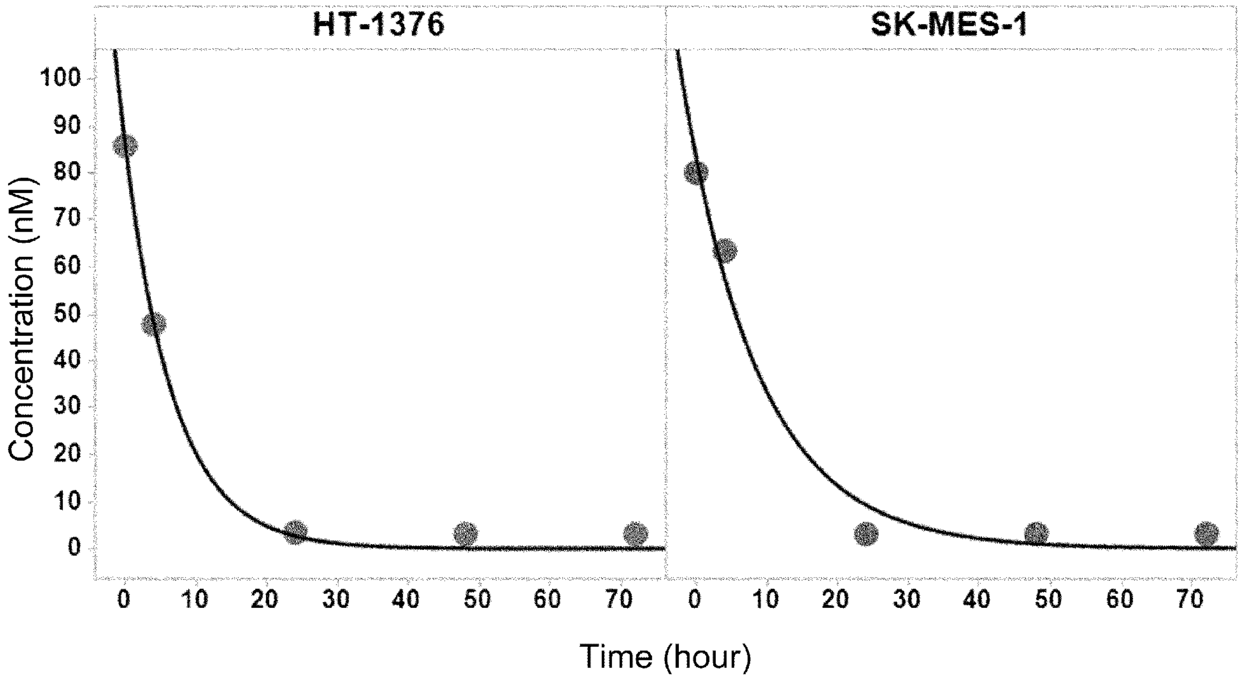
HT-1376
SK-MES-1
Concentration (nM)
100
90
80
70
60
50
40
30
20
10
0
0
10
20
30
40
50
60
70
Time (hour)

FIG. 3

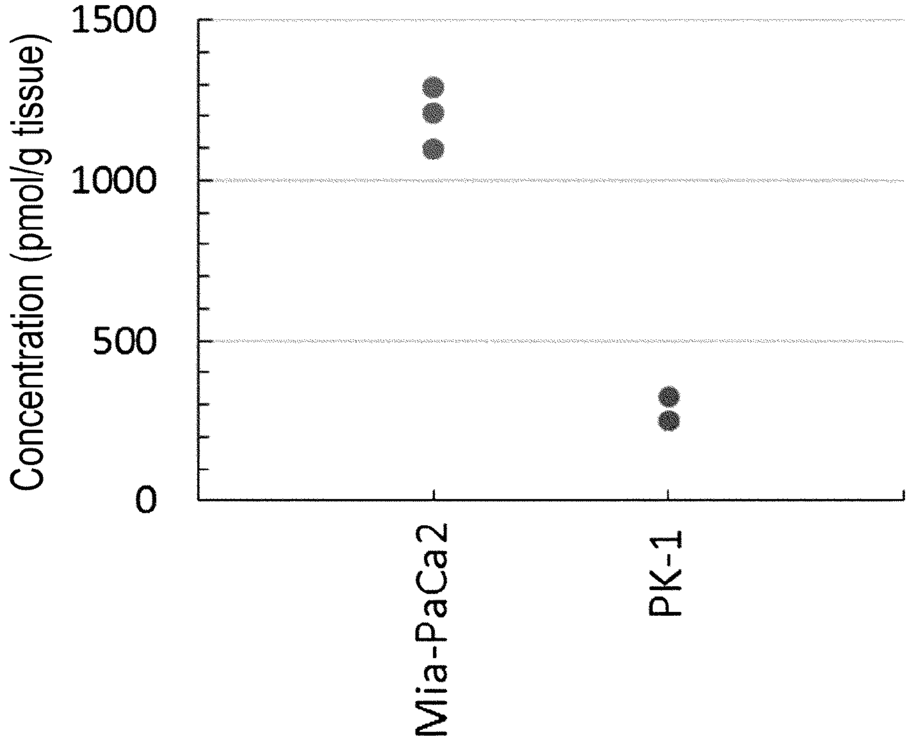


FIG. 4

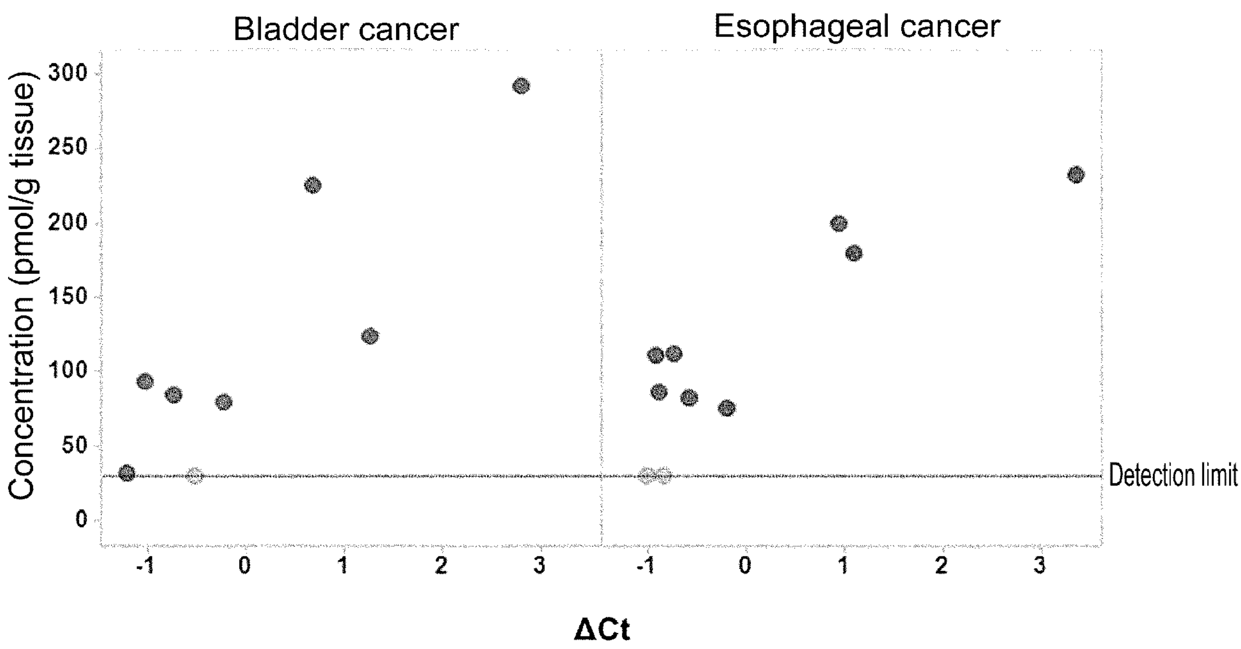
Bladder cancer
Esophageal cancer
Concentration (pmol/g tissue)
300
250
200
150
100
50
0
Detection limit
-1
0
1
2
3
-1
0
1
2
3
ΔCt

FIG. 5

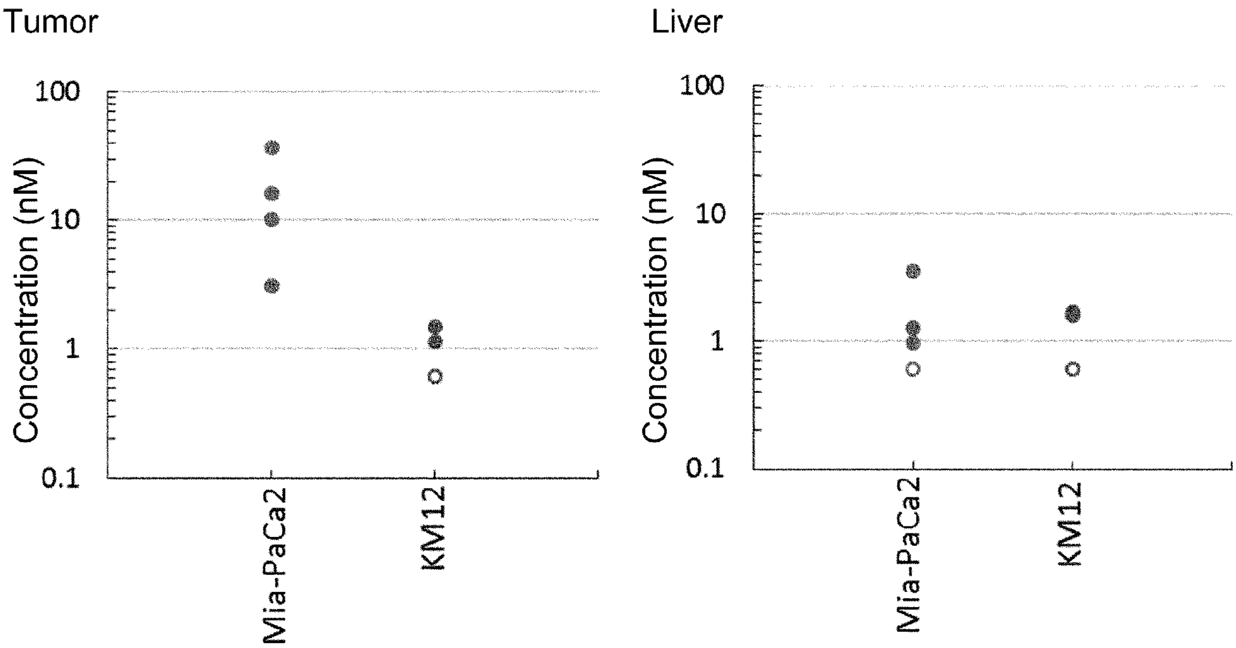


FIG. 6

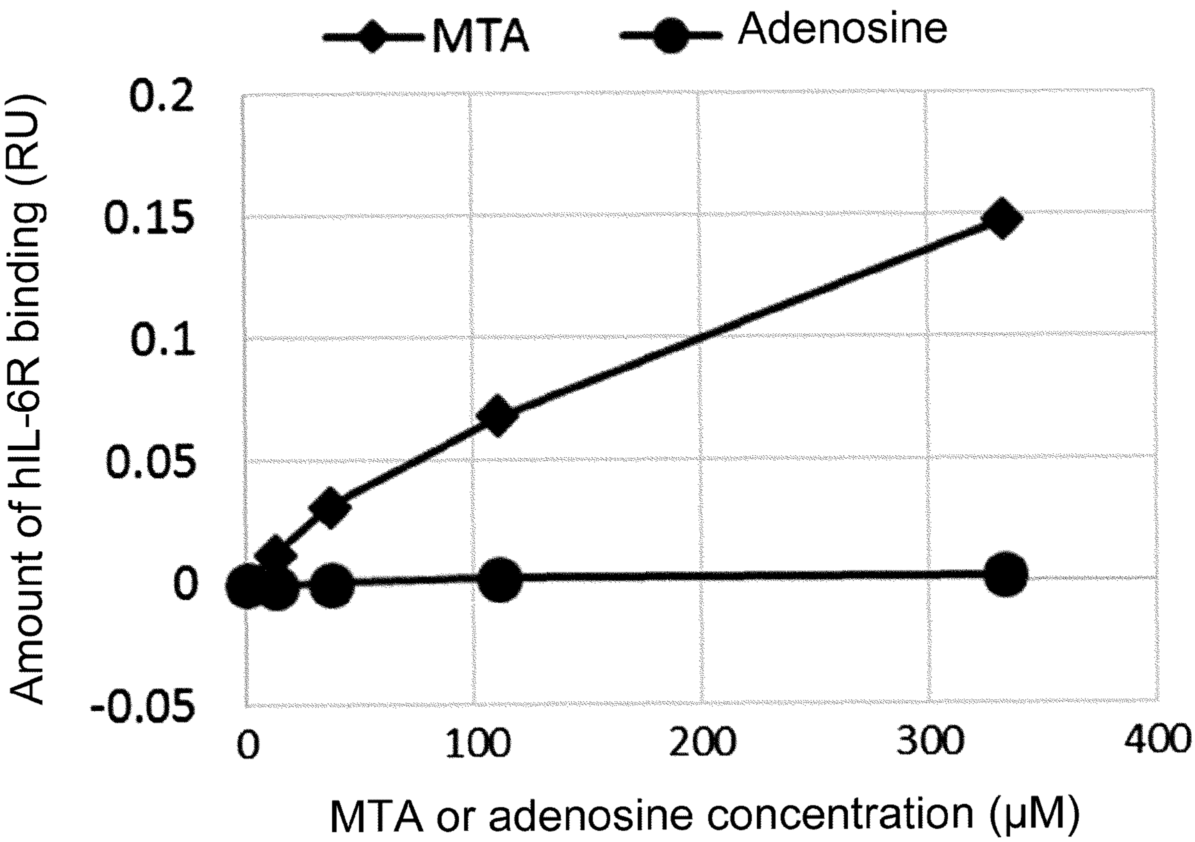
MTA
Adenosine
Amount of hIL-6R binding (RU)
0.2
0.15
0.1
0.05
0
-0.05
0
100
200
300
400
MTA or adenosine concentration (μM)

FIG. 7

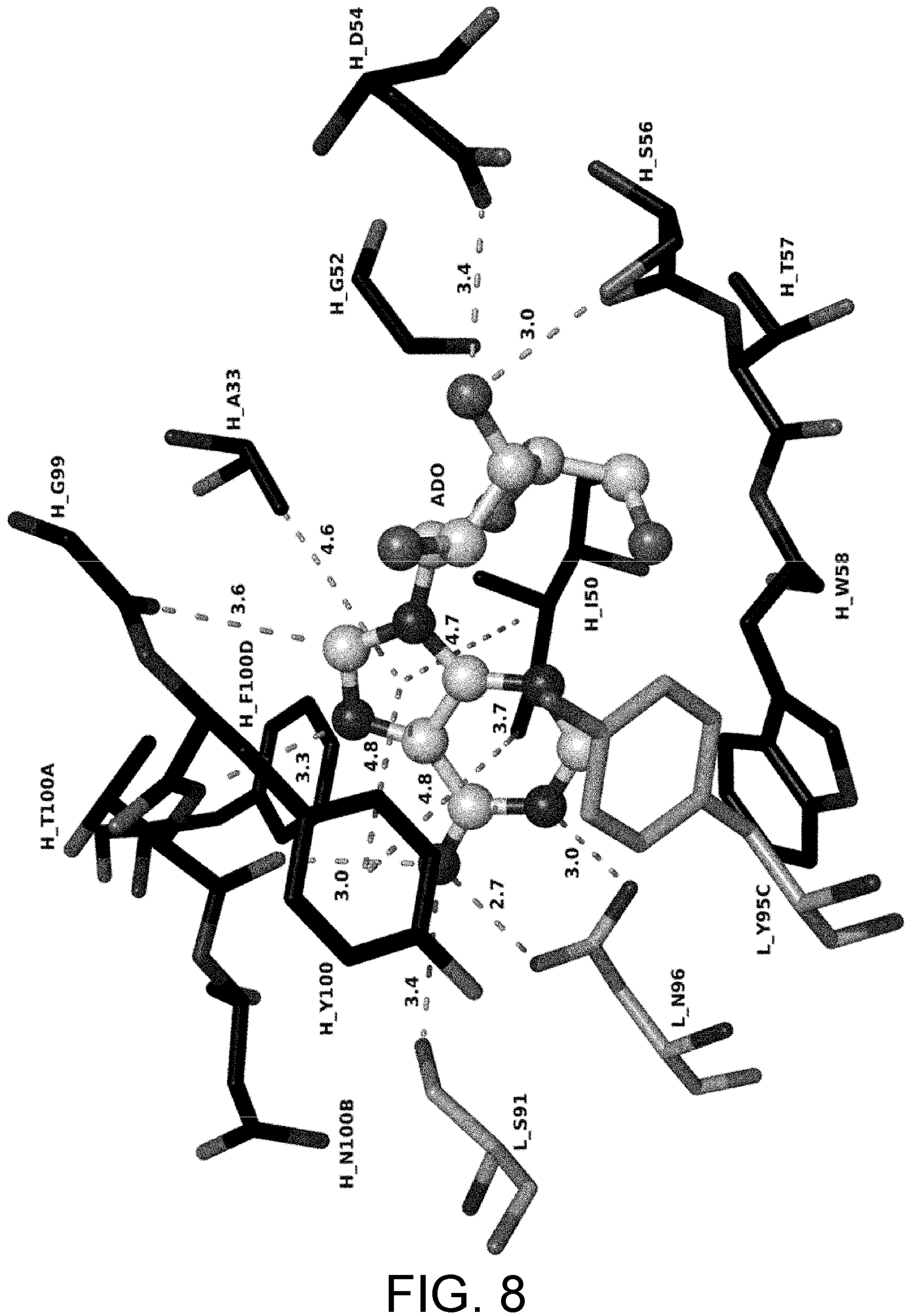
H_D54
H_S56
H_G52
3.4
3.0
H_T57
H_A33
ADO
H_G99
4.6
3.6
4.7
H_I50
H_W58
H_F100D
3.7
3.3
4.8
4.8
H_T100A
3.0
3.0
2.7
L_Y95C
H_Y100
3.4
L_N96
H_N100B
L_S91

FIG. 8

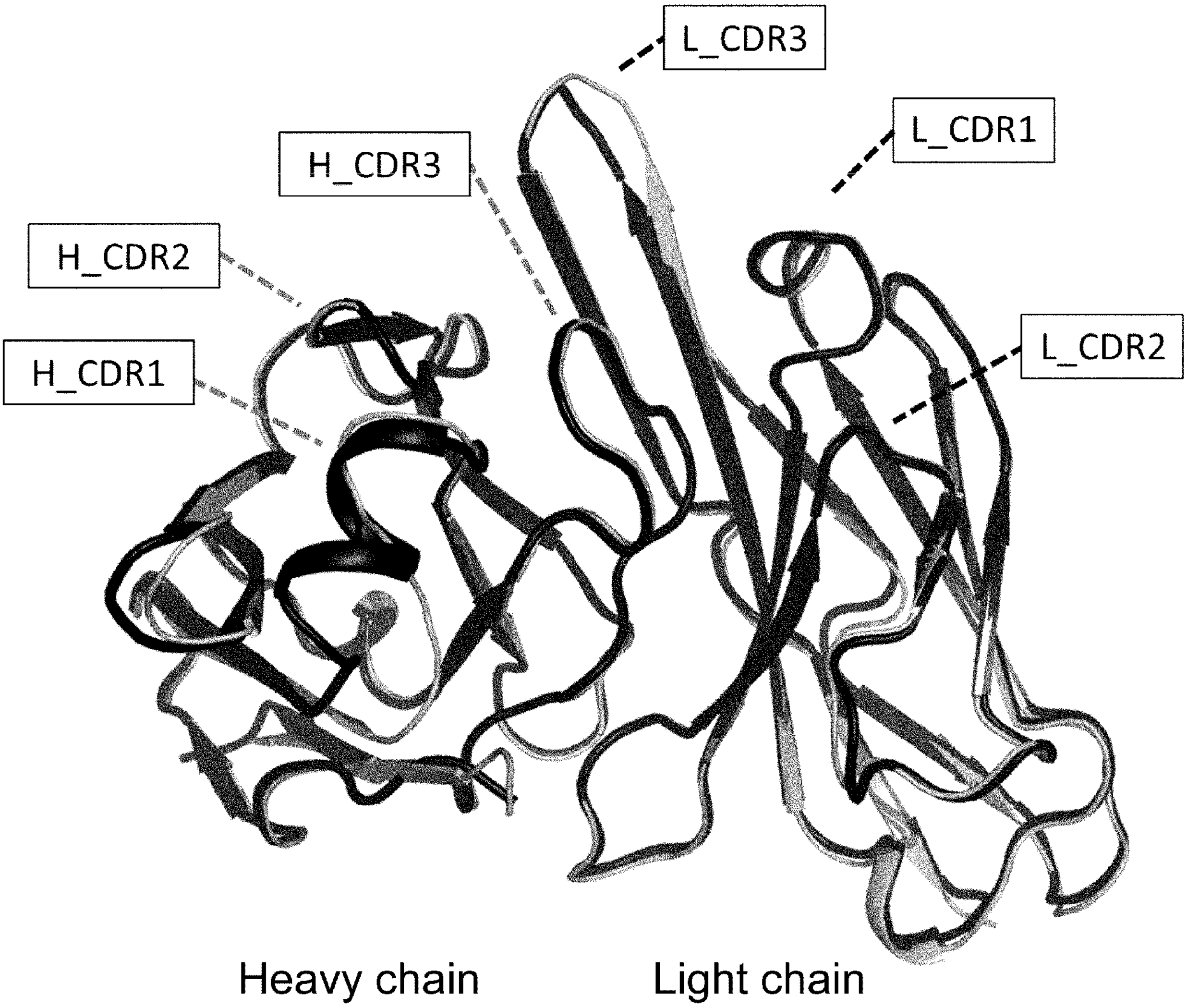
L_CDR3
L_CDR1
H_CDR3
H_CDR2
L_CDR2
H_CDR1
Heavy chain
Light chain

FIG. 9

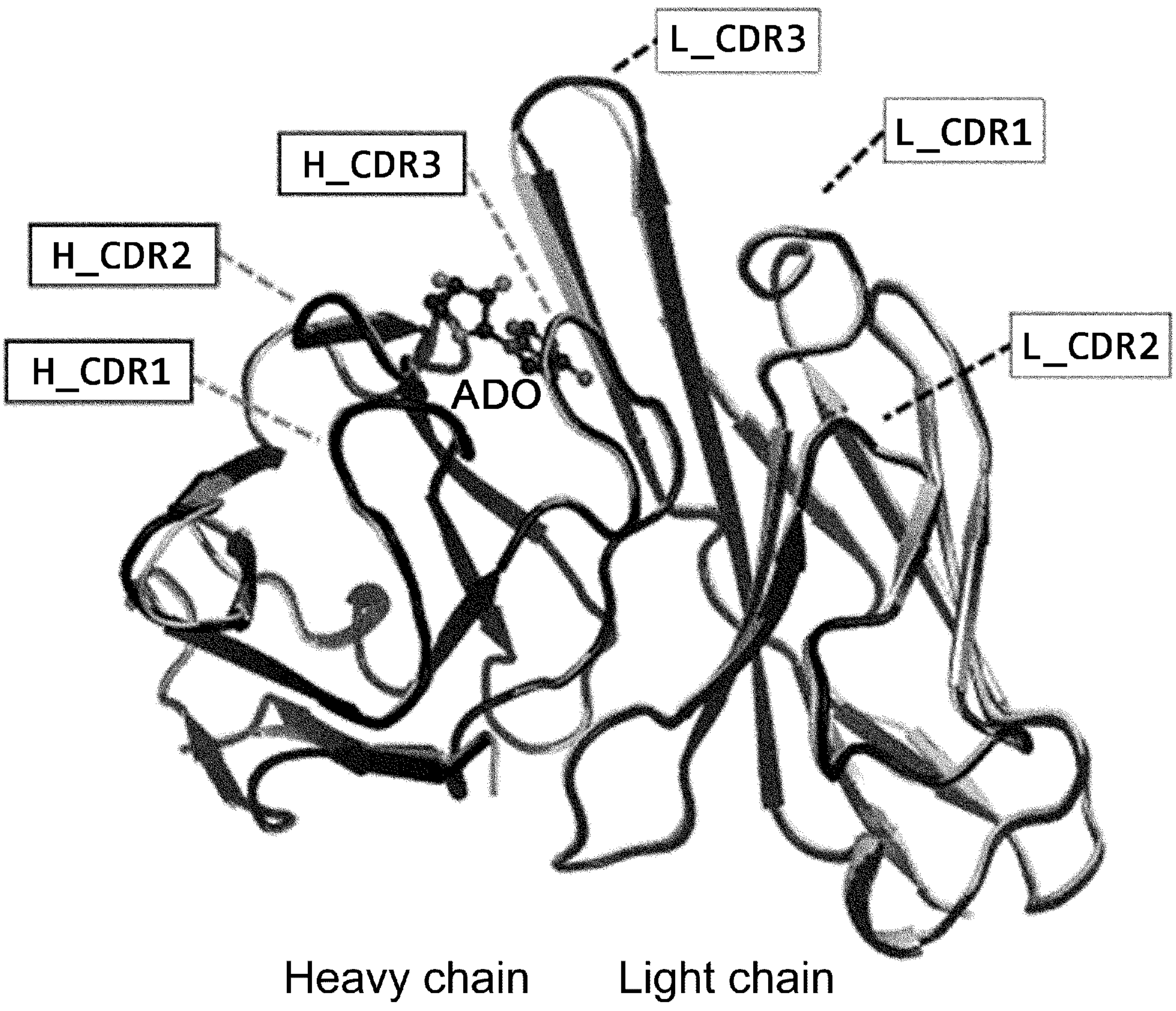
L_CDR3
L_CDR1
H_CDR3
H_CDR2
L_CDR2
H_CDR1
ADO
Heavy chain
Light chain

FIG. 10

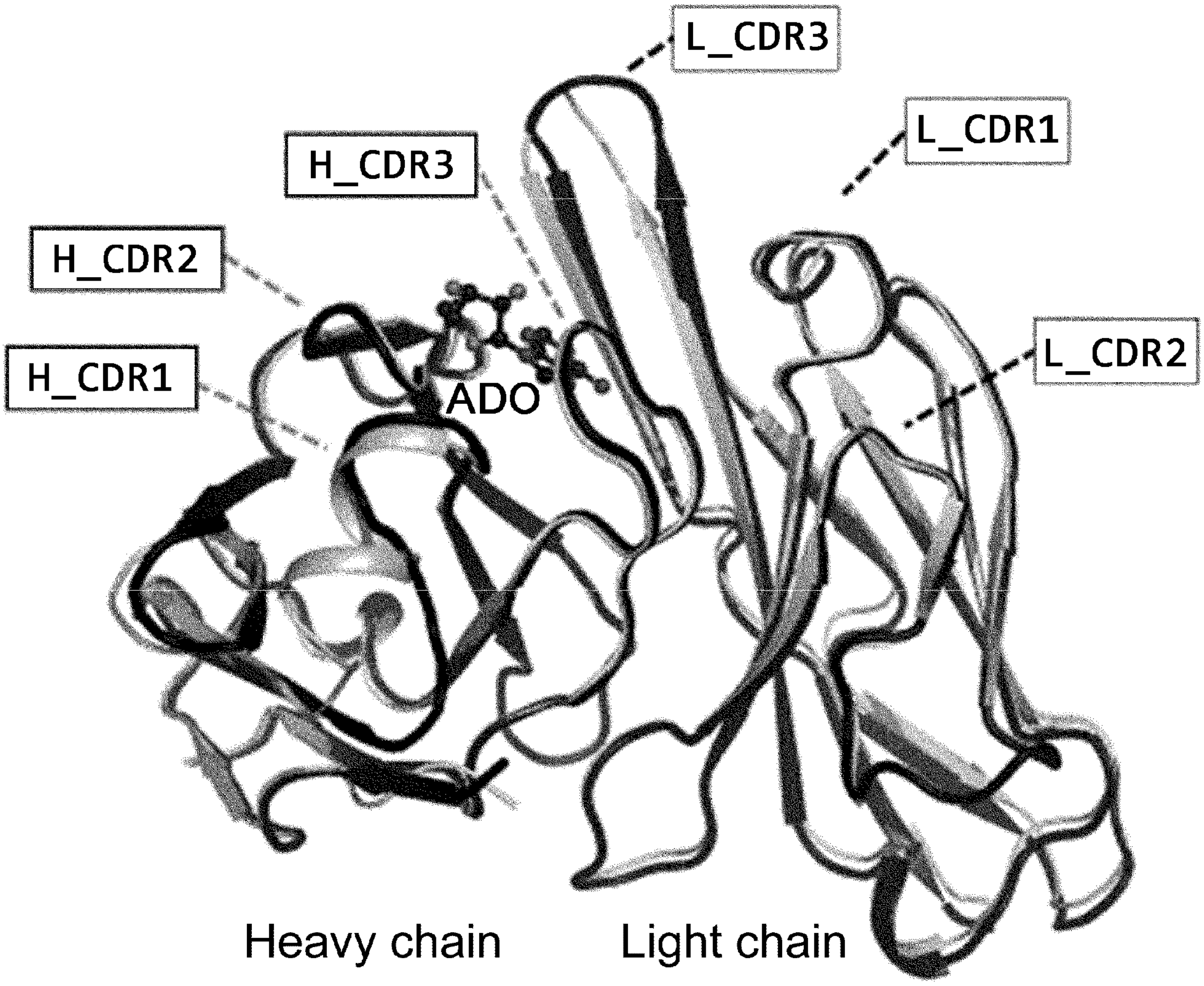
L_CDR3
L_CDR1
H_CDR3
H_CDR2
L_CDR2
H_CDR1
ADO
Heavy chain
Light chain

FIG. 11

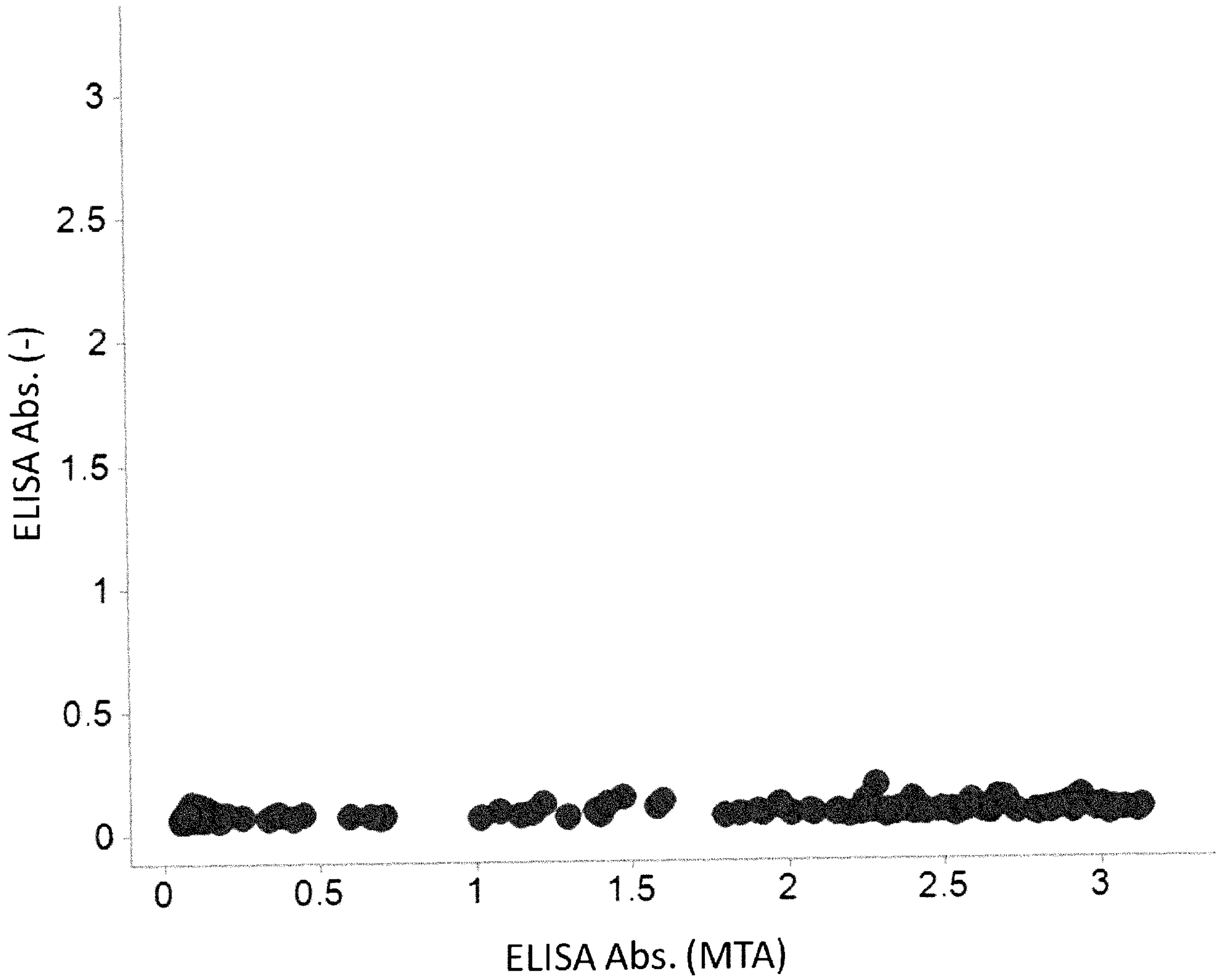


FIG. 12

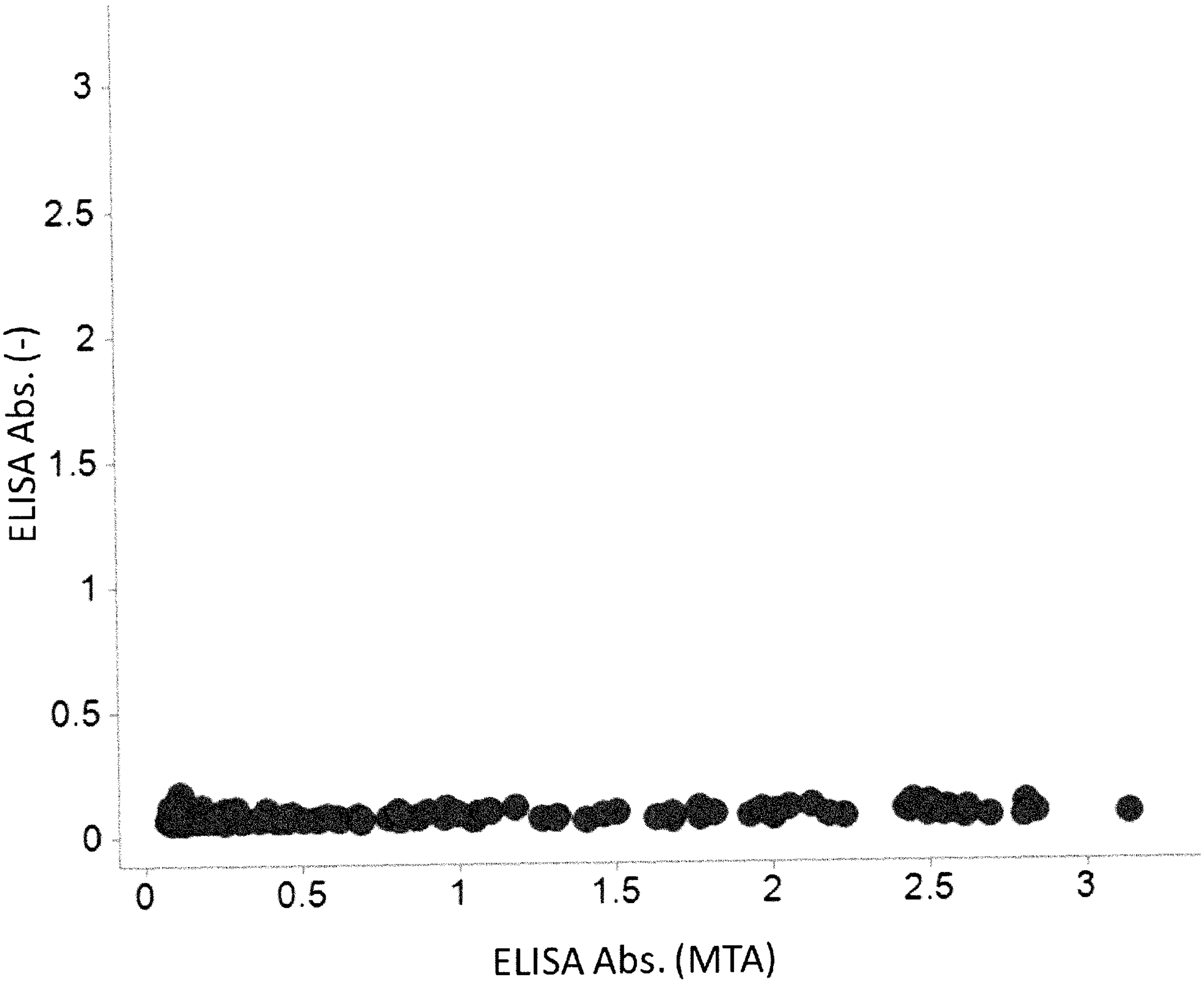


FIG. 13

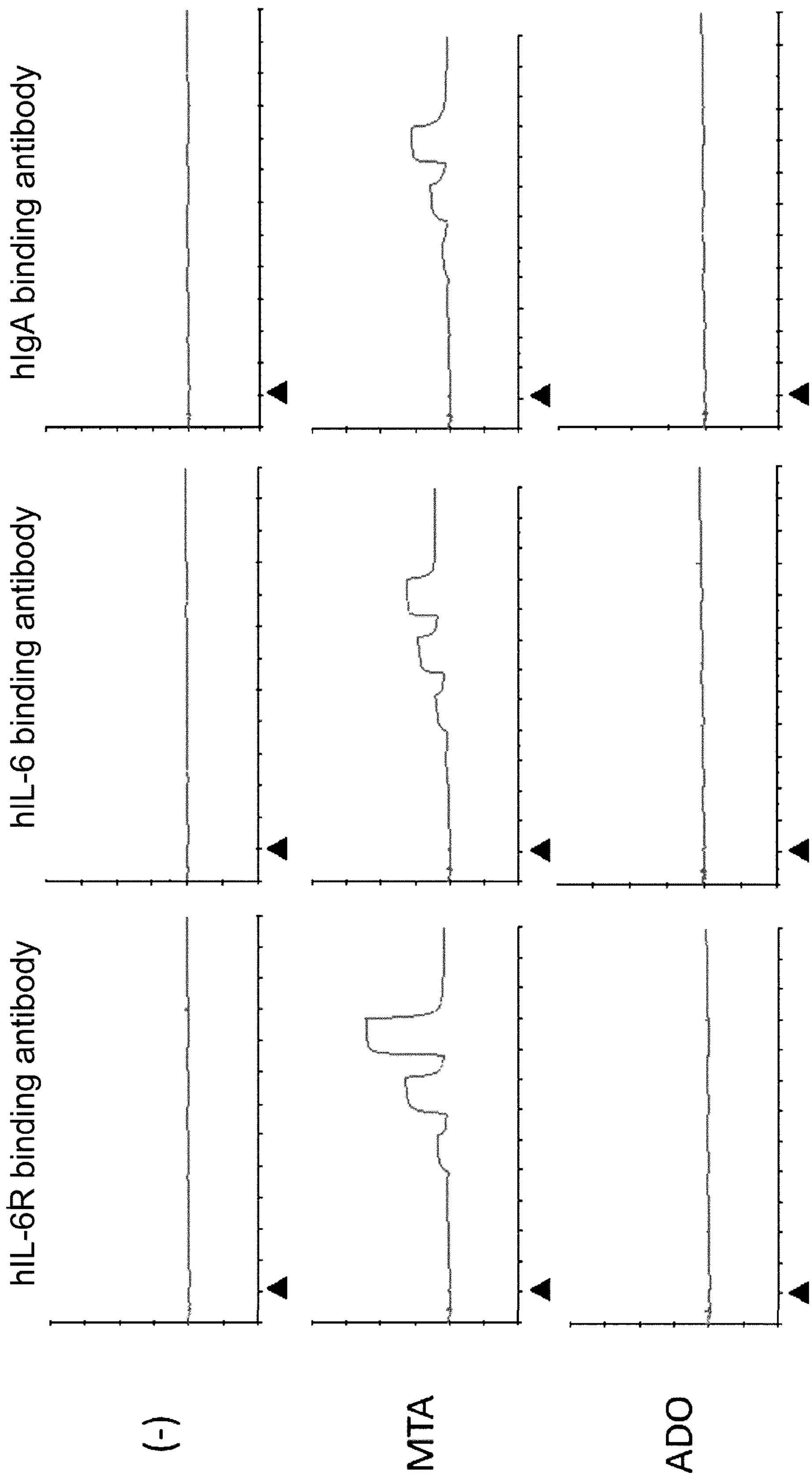
hIL-6R binding antibody
hIL-6 binding antibody
hIgA binding antibody
(-)
MTA
ADO

FIG. 14

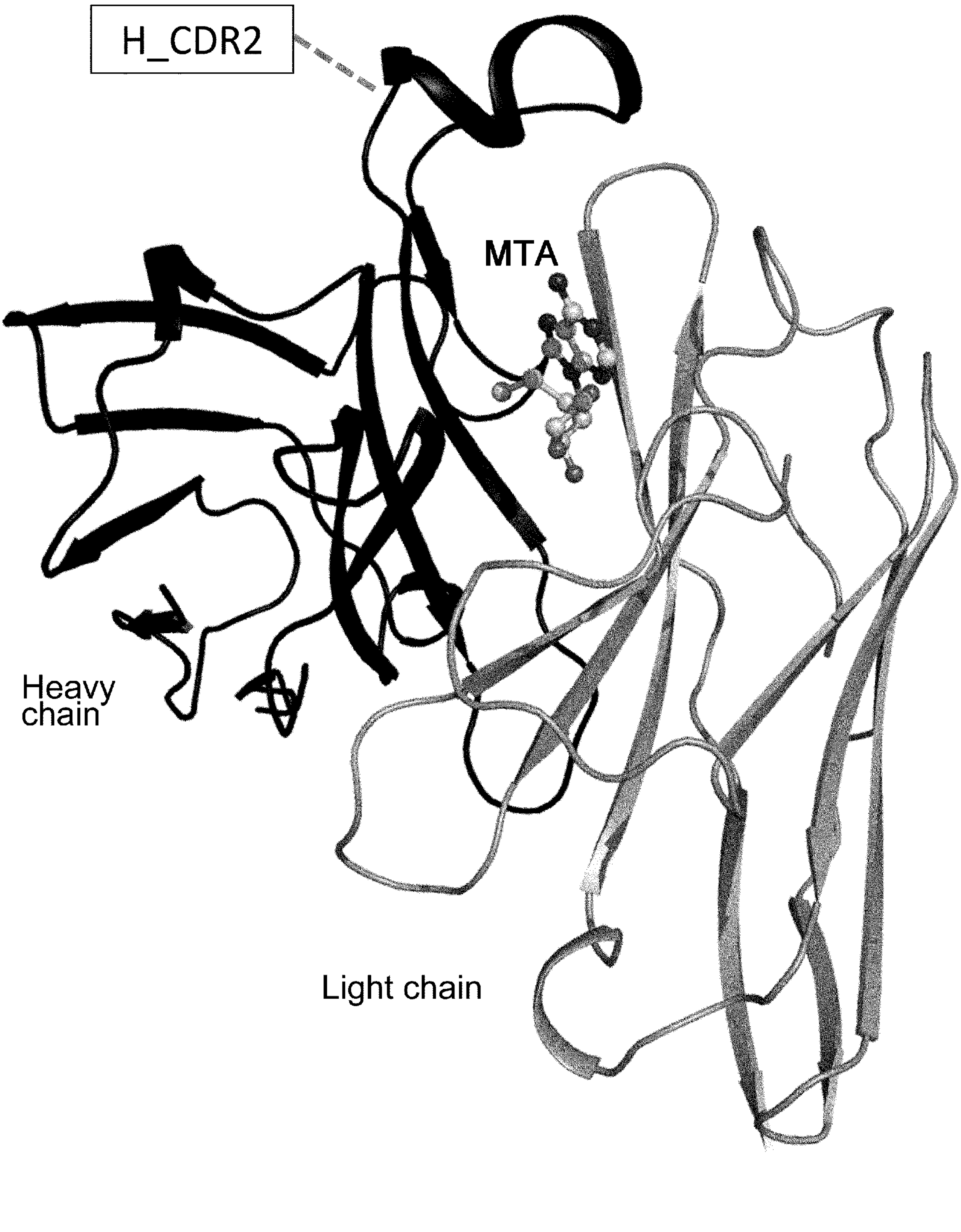


FIG. 15

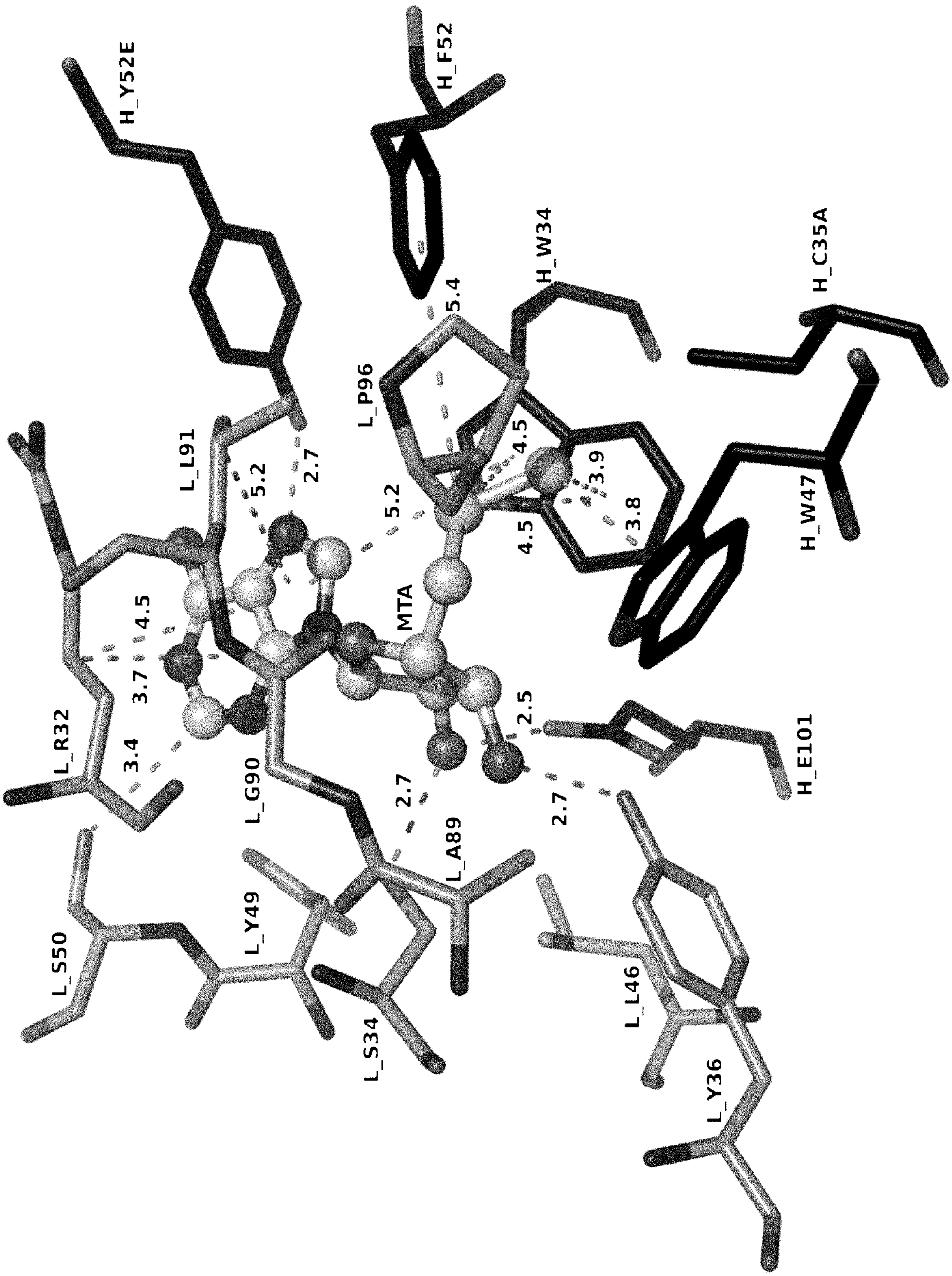
H_Y52E
H_F52
H_W34
H_C35A
L_P96
L_L91
MTA
H_W47
L_R32
L_G90
L_A89
H_E101
L_Y49
L_S50
L_S34
L_L46
L_Y36
5.4
5.2
2.7
5.2
4.5
3.9
3.8
4.5
4.5
3.7
3.4
2.5
2.7
2.7

FIG. 16

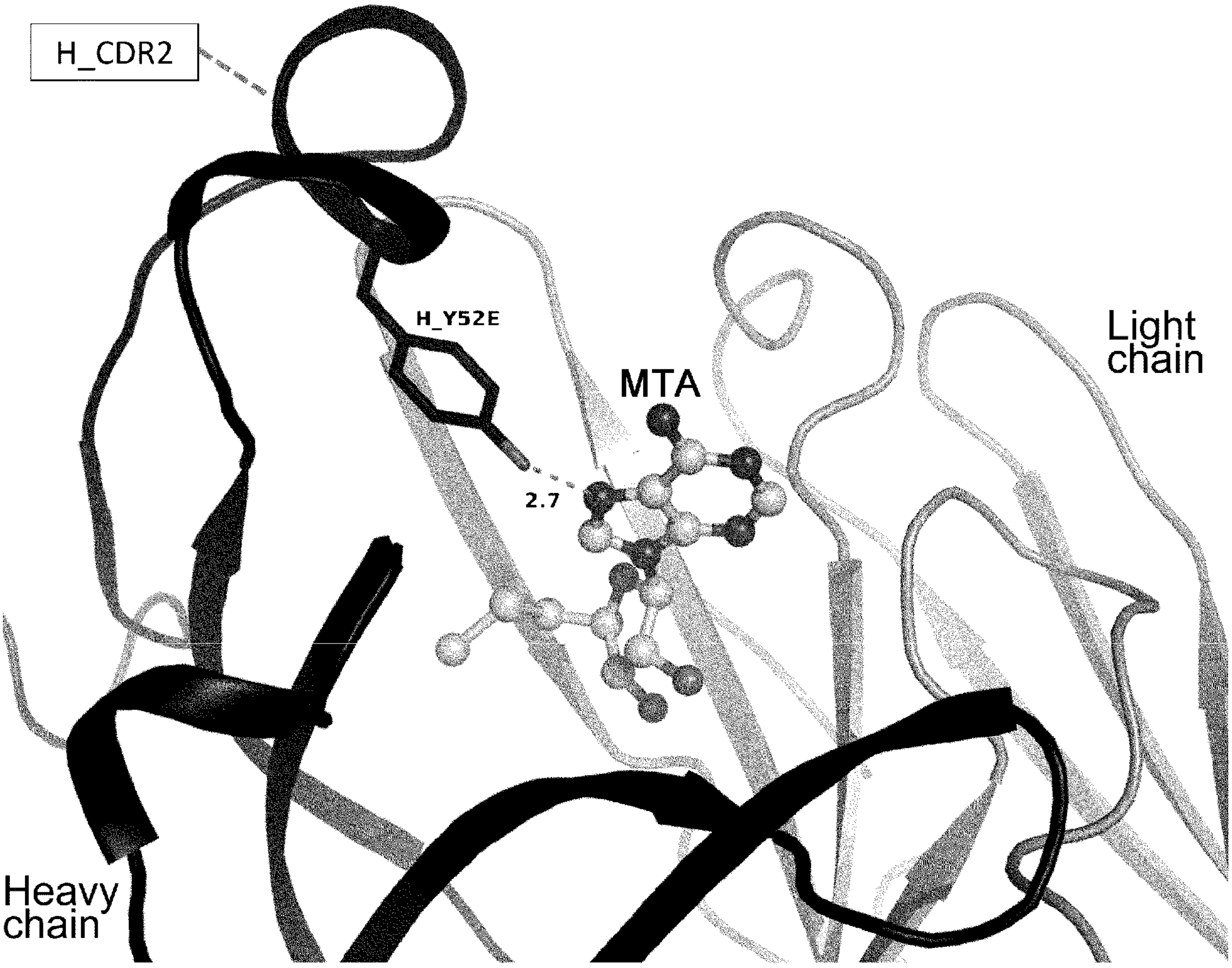


FIG. 17

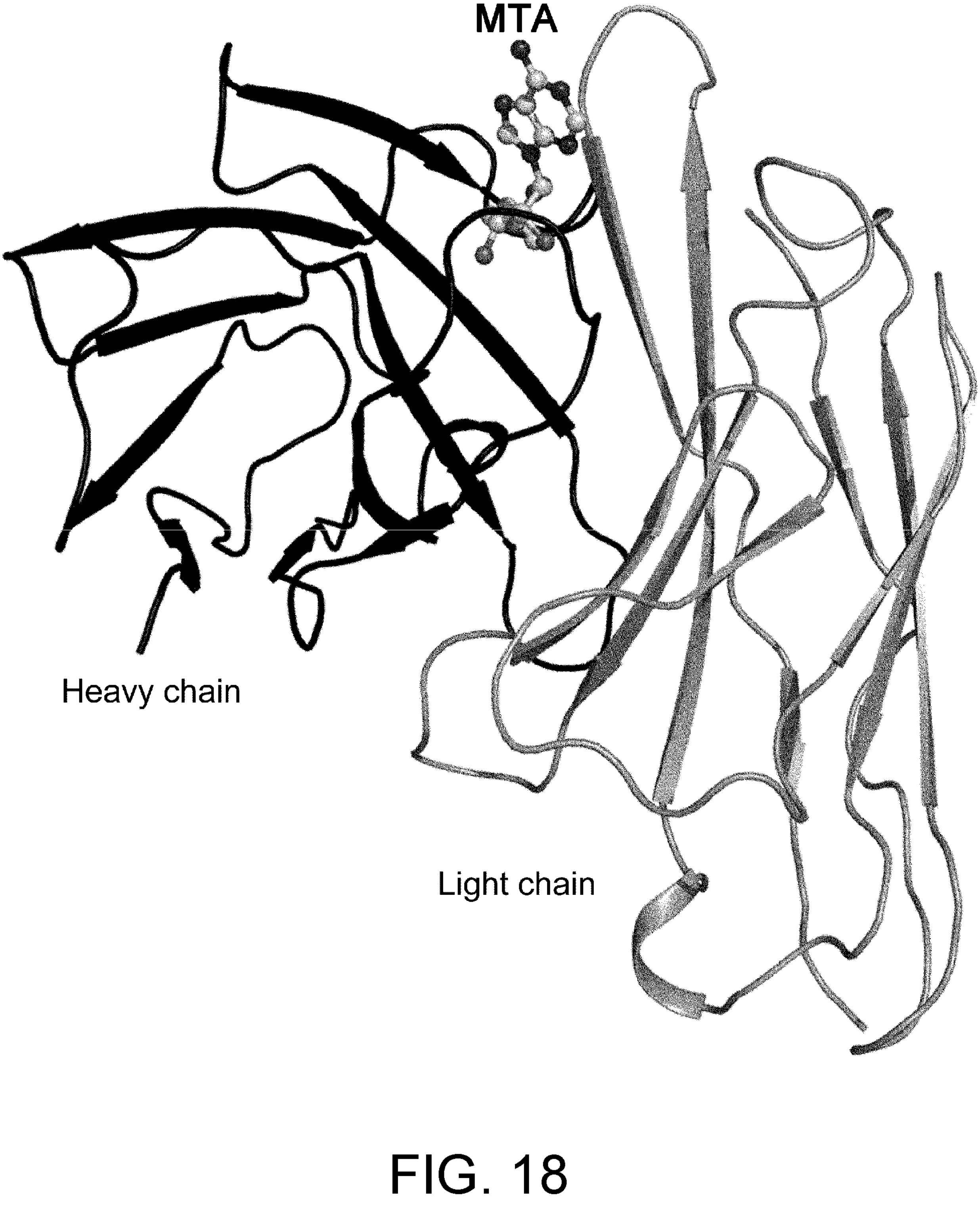


FIG. 18

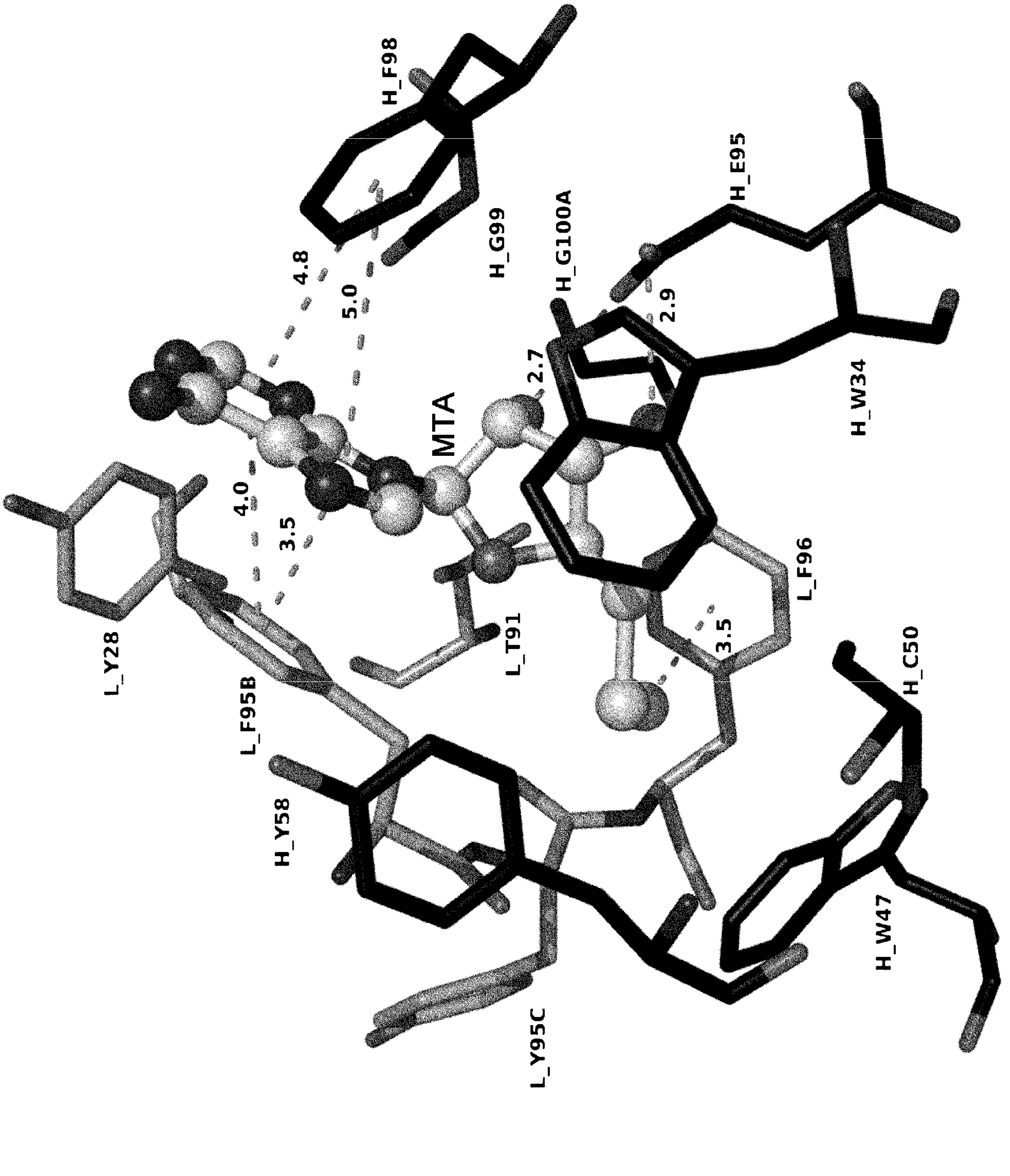
H_F98
H_E95
H_G99
H_G100A
4.8
5.0
2.9
2.7
MTA
H_W34
4.0
3.5
L_F96
3.5
L_Y28
L_F95B
L_T91
H_C50
H_Y58
H_W47
L_Y95C

FIG. 19

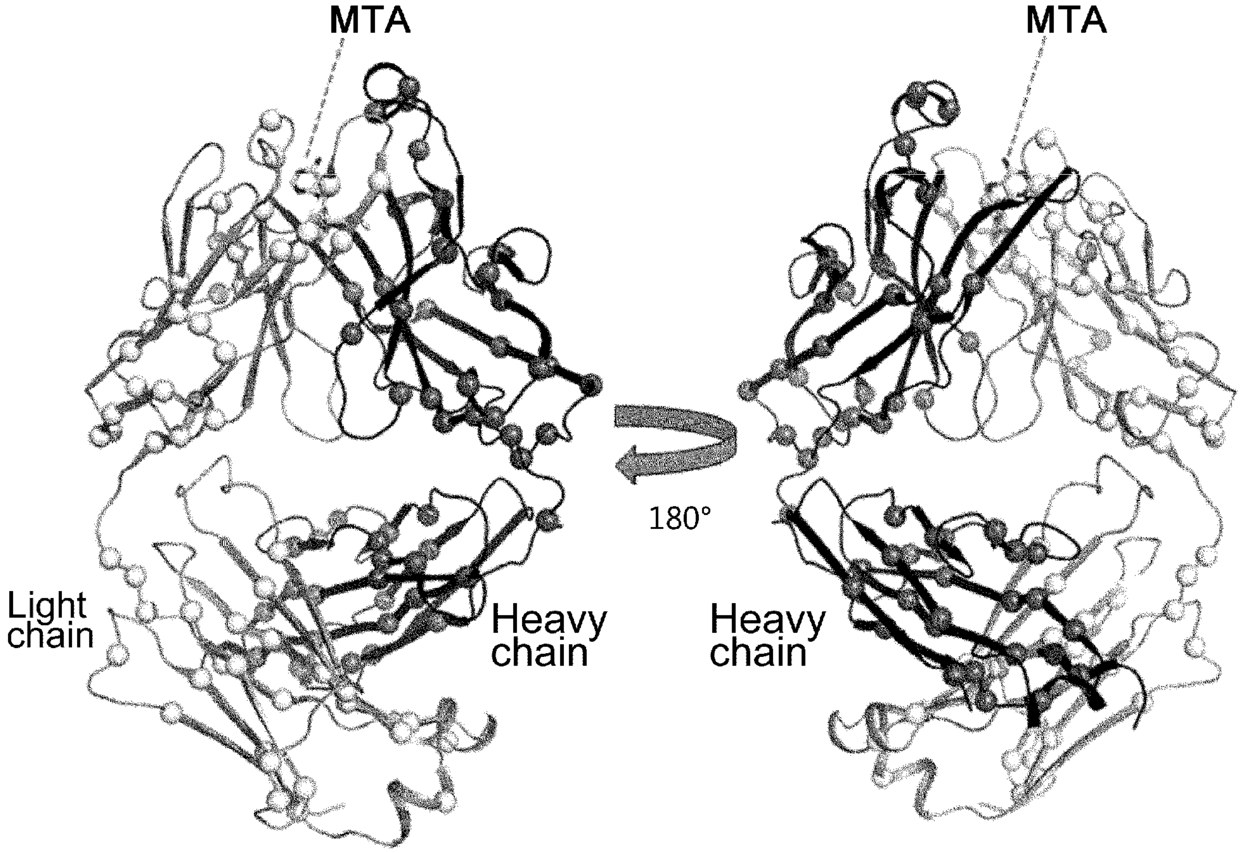


FIG. 20

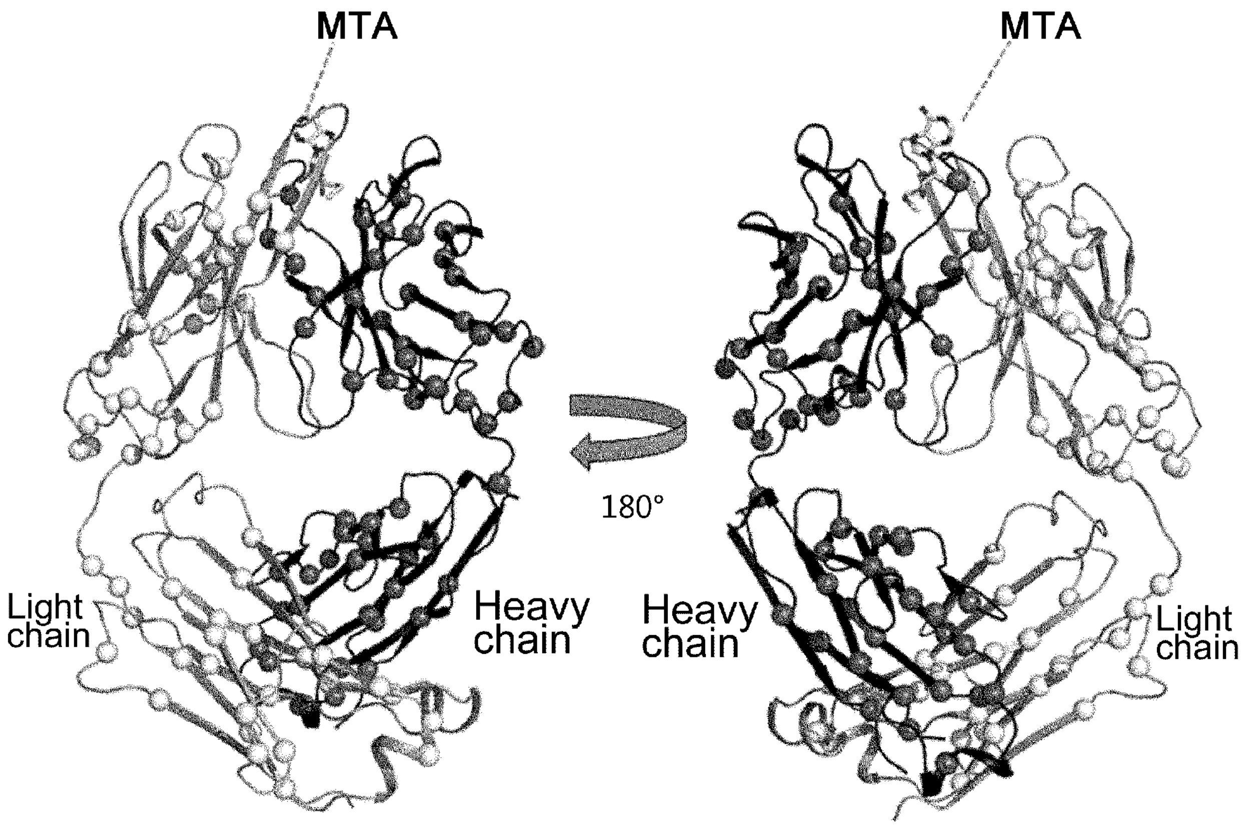


FIG. 21

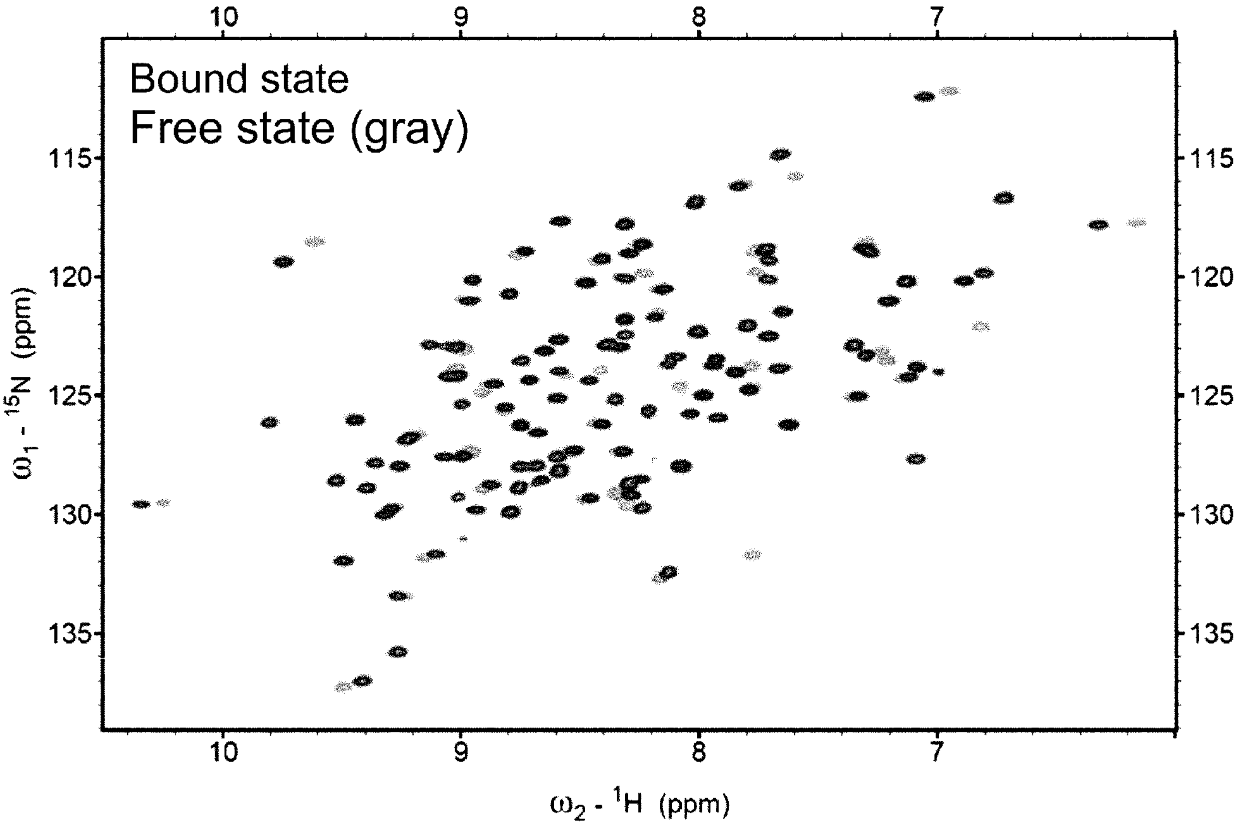
Bound state
Free state (gray)
10
9
8
7
115
120
125
130
135
ω1 - 15N (ppm)
ω2 - 1H (ppm)

FIG. 22

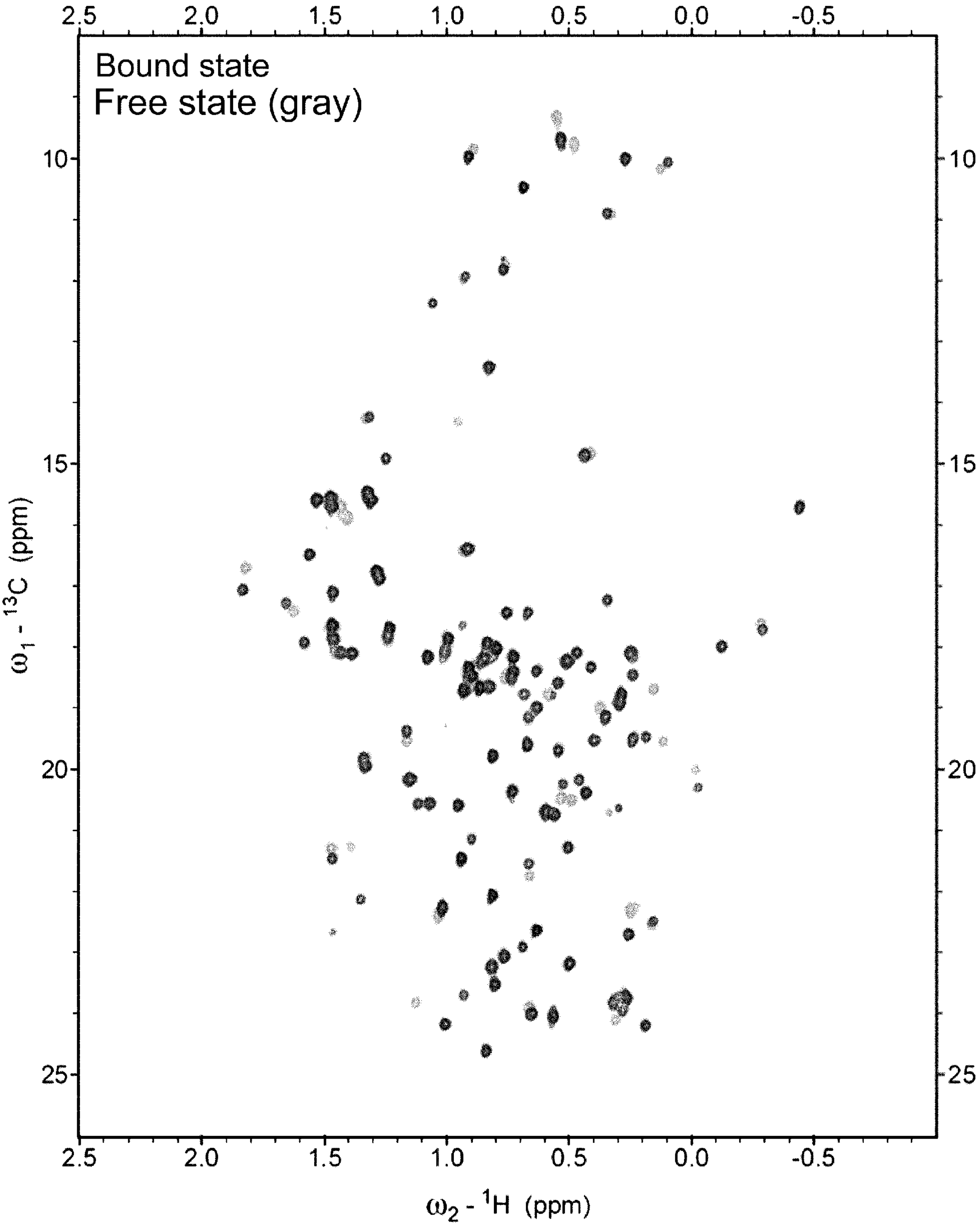
Bound state
Free state (gray)
2.5
2.0
1.5
1.0
0.5
0.0
-0.5
10
15
20
25
ω1 - 13C (ppm)
ω2 - 1H (ppm)

FIG. 23

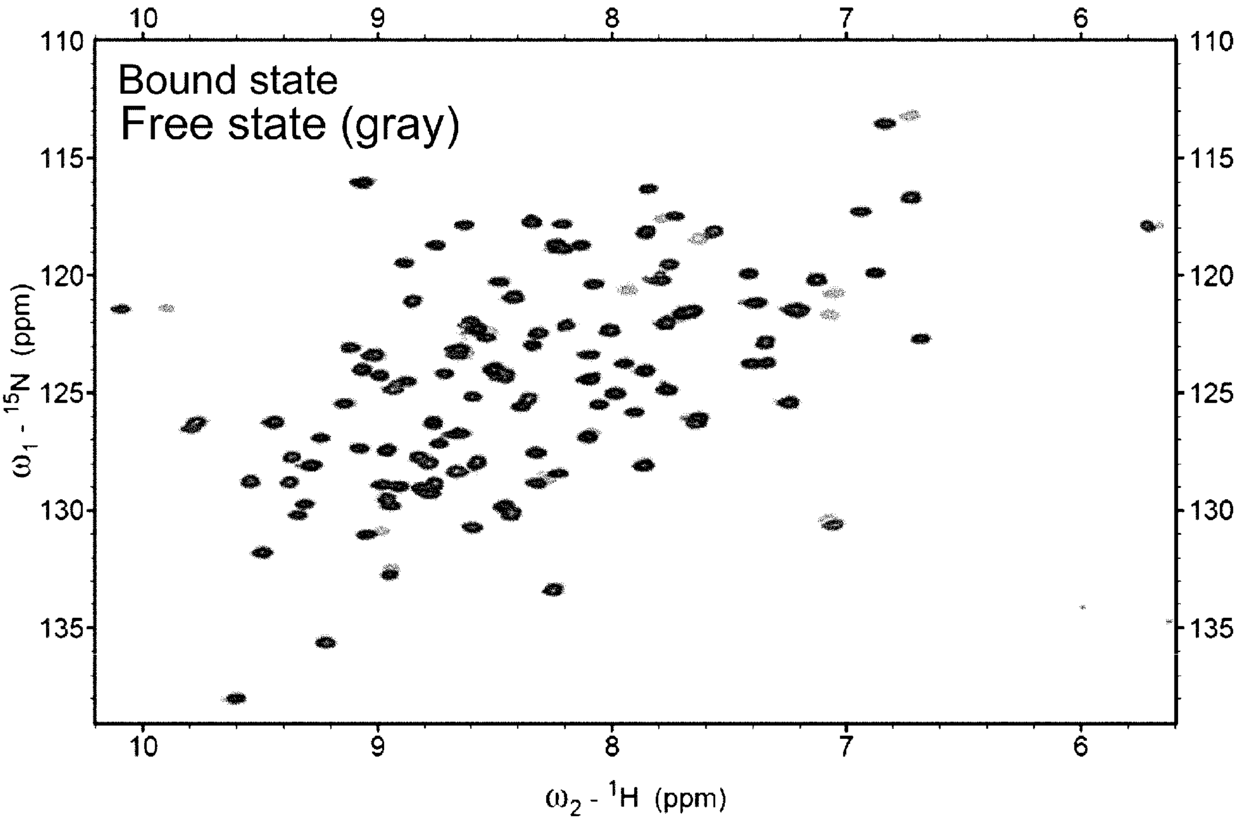
Bound state
Free state (gray)
10
9
8
7
6
110
115
120
125
130
135
ω1 - 15N (ppm)
ω2 - 1H (ppm)

FIG. 24

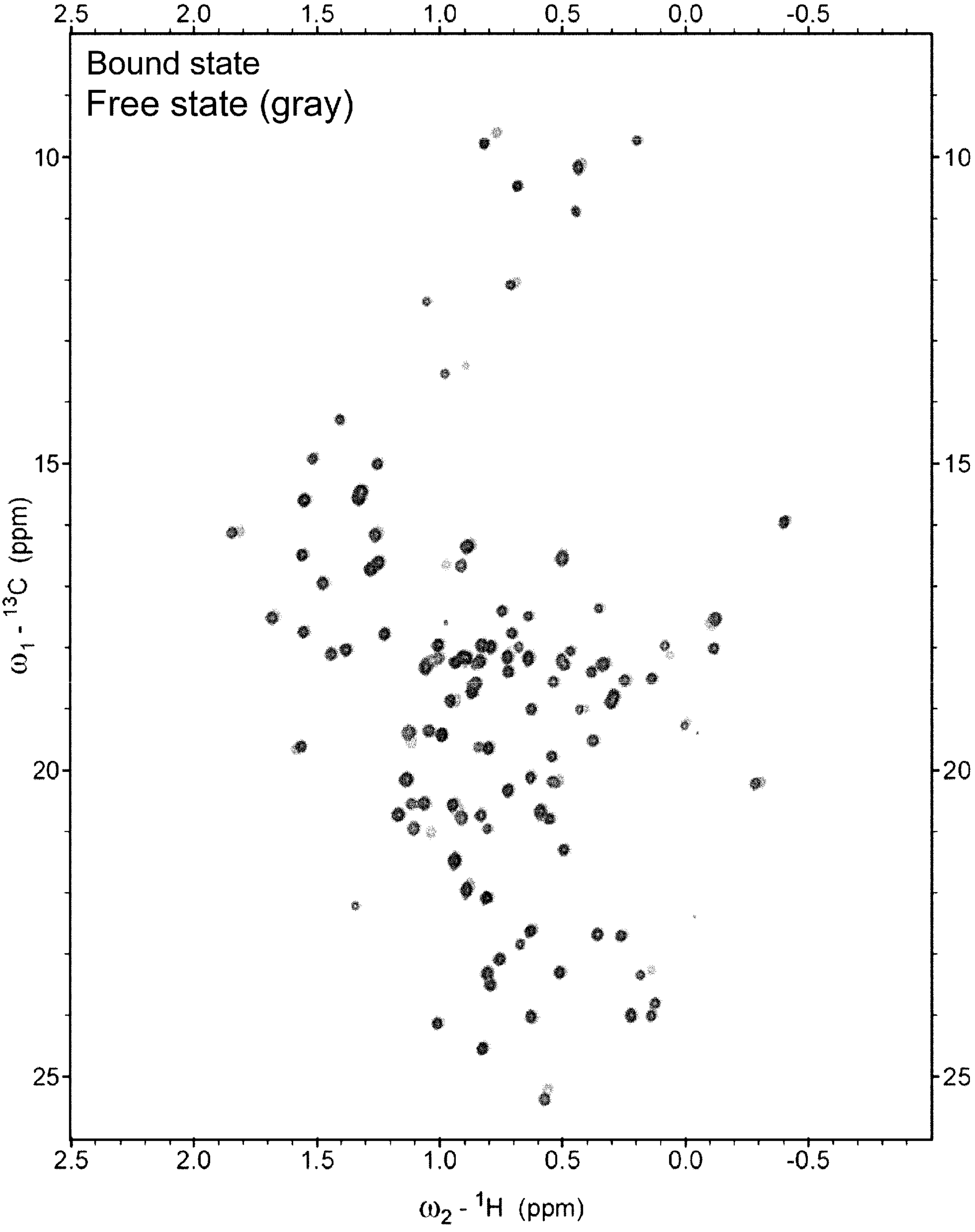
Bound state
Free state (gray)
2.5
2.0
1.5
1.0
0.5
0.0
-0.5
10
15
20
25
$\omega_1$ - $^{13}$C (ppm)
$\omega_2$ - $^{1}$H (ppm)

FIG. 25

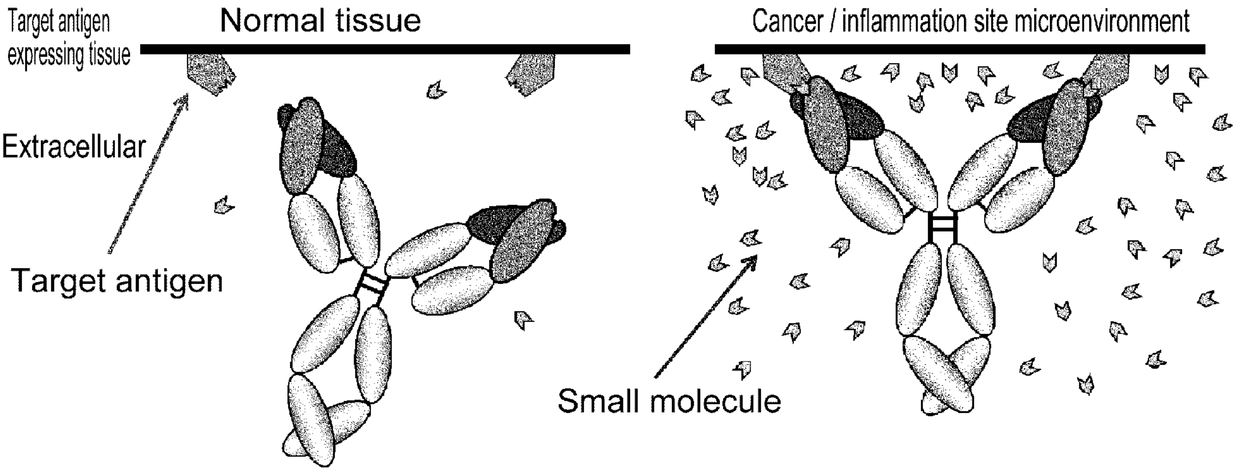
Target antigen expressing tissue
Normal tissue
Extracellular
Target antigen
Cancer / inflammation site microenvironment
Small molecule

FIG. 26

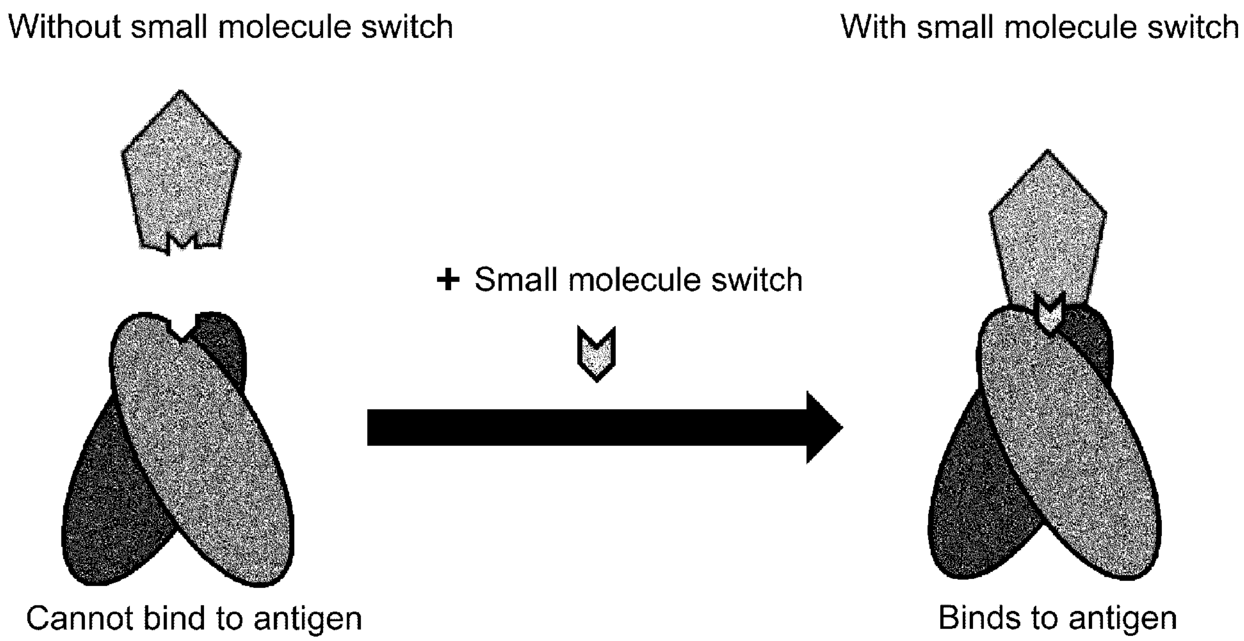
Without small molecule switch
With small molecule switch
+ Small molecule switch
Cannot bind to antigen
Binds to antigen

FIG. 27

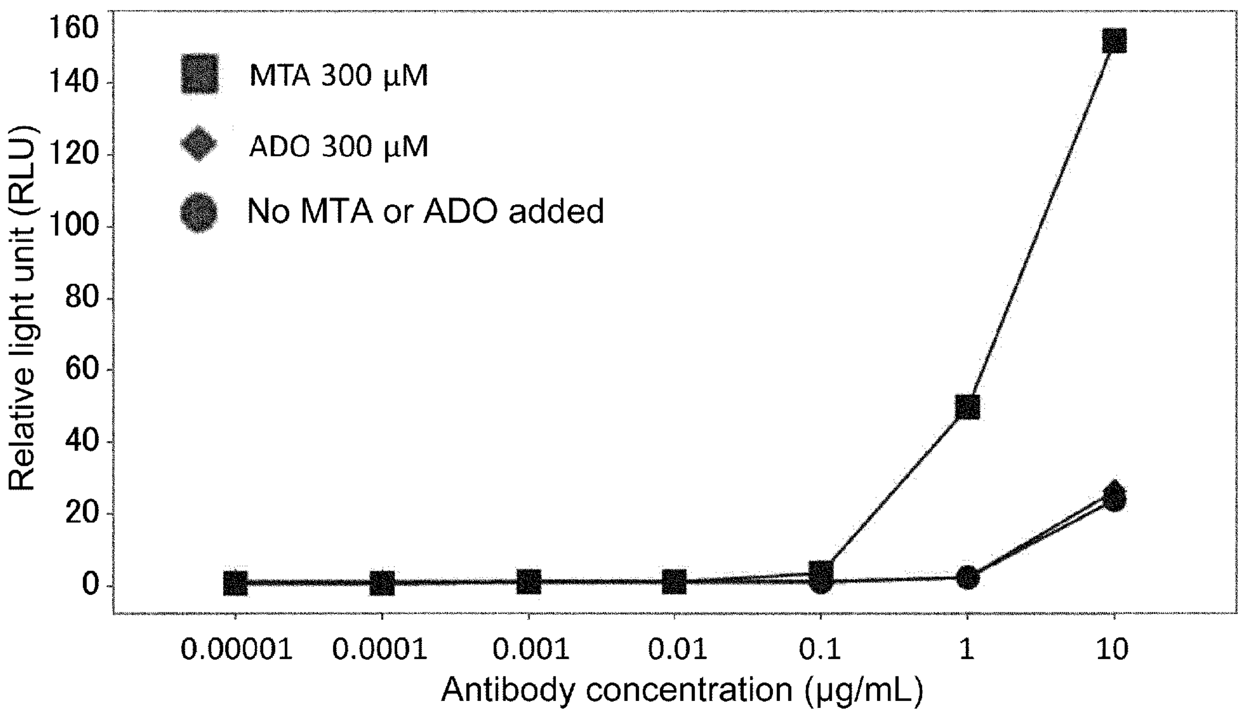
160
140
120
100
80
60
40
20
0
Relative light unit (RLU)
MTA 300 μM
ADO 300 μM
No MTA or ADO added
0.00001
0.0001
0.001
0.01
0.1
1
10
Antibody concentration (μg/mL)

FIG. 28

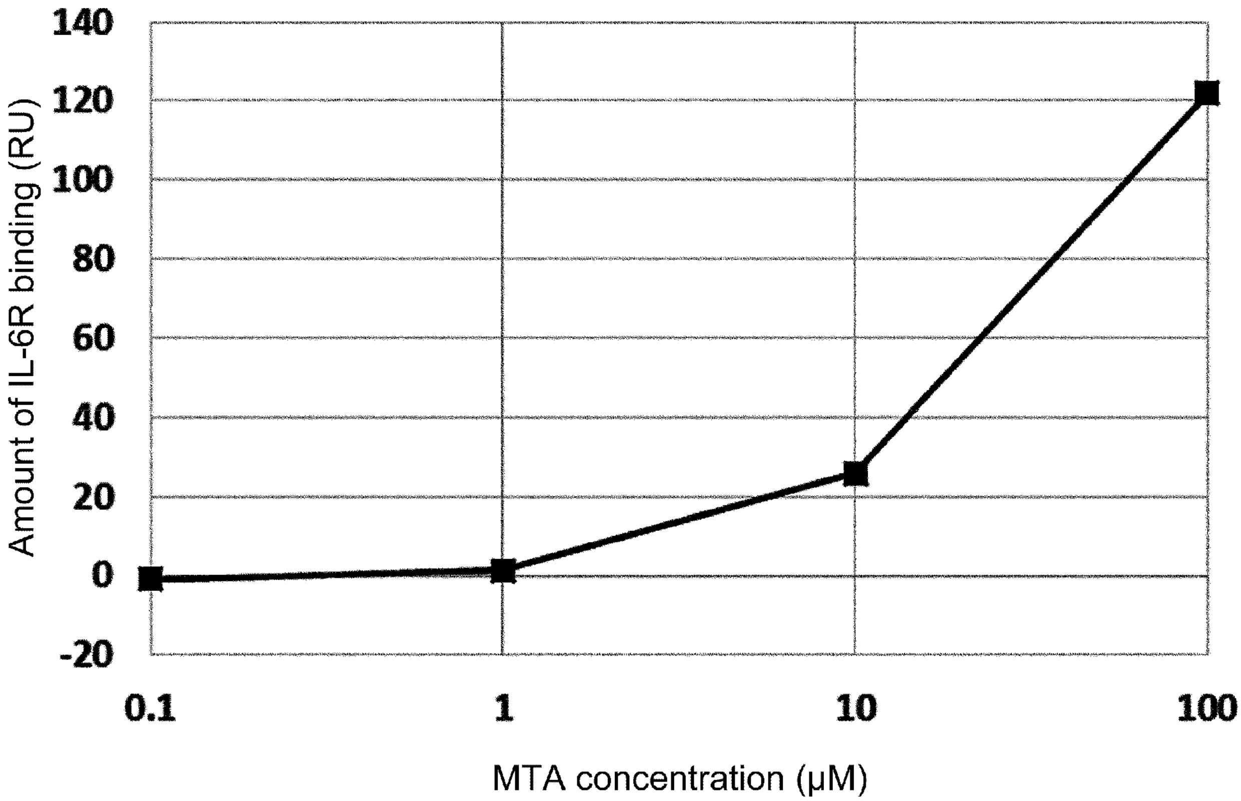
140
120
100
80
60
40
20
0
-20
Amount of IL-6R binding (RU)
0.1
1
10
100
MTA concentration (μM)

FIG. 29

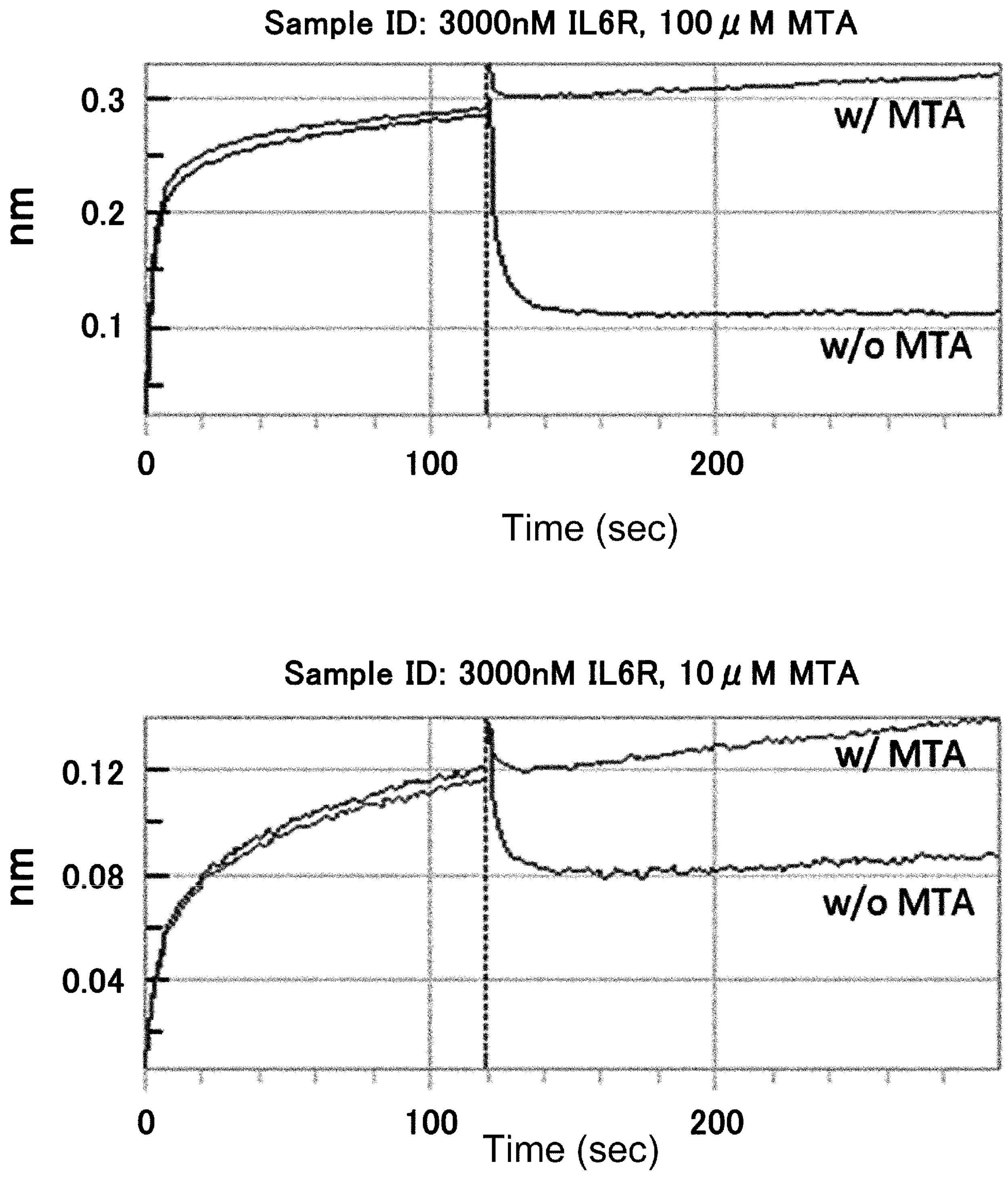
Sample ID: 3000nM IL6R, 100 μ M MTA
0.3
0.2
0.1
nm
w/ MTA
w/o MTA
0
100
200
Time (sec)
Sample ID: 3000nM IL6R, 10 μ M MTA
0.12
0.08
0.04
nm
w/ MTA
w/o MTA
0
100
200
Time (sec)

FIG. 30

## INTERNATIONAL SEARCH REPORT

International application No.
PCT/JP2020/012189

A. CLASSIFICATION OF SUBJECT MATTER
A61K 39/395(2006.01)i; A61P 35/00(2006.01)i; C07K 16/28(2006.01)i; C07K 16/44(2006.01)i; C40B 40/08(2006.01)i; C40B 40/10(2006.01)i; C12N 15/13(2006.01)i; G01N 33/15(2006.01)i; G01N 33/53(2006.01)i; C12P 21/08(2006.01)i
FI: C07K16/28 ZNA; A61K39/395 D; A61K39/395 N; A61P35/00; C07K16/44; C12N15/13; C12P21/08; C40B40/08; C40B40/10; G01N33/15 Z; G01N33/53 D
According to International Patent Classification (IPC) or to both national classification and IPC

B. FIELDS SEARCHED
Minimum documentation searched (classification system followed by classification symbols)
A61K39/395; A61P35/00; C07K16/28; C07K16/44; C40B40/08; C40B40/10; C12N15/13; G01N33/15; G01N33/53; C12P21/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | JP 2007-531724 A (QUARK PHARMACEUTICALS INC.) 08.11.2007 (2007-11-08) claims 1, 21-25, paragraph [0066], examples 4, 6, etc. | 7-9, 14, 15 |
| X | ENOMOTO, K. et al., "Development of high-throughput spermidine synthase activity assay using homogeneous time-resolved fluorescence", Analytical Biochemistry, 2006, vol. 351, pp. 229-240 abstract, page 233, right column to page 234, left column, section "Generation of anti-MTA monoclonal antibody", etc. | 14 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 May 2020 (29.05.2020) | 09 June 2020 (09.06.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## INTERNATIONAL SEARCH REPORT

International application No.
PCT/JP2020/012189

C (Continuation). DOCUMENTS CONSIDERED TO BE RELEVANT

| Category* | Citation of document, with indication, where appropriate, of the relevant passages | Relevant to claim No. |
|---|---|---|
| X | WO 2015/08:3764 A1 (CHUGAI PHARMACEUTICAL CO., LTD.) 11.06.2015 (2015-06-11) claims 1-3, 10, 12, 14, examples 4-7, etc. | 7-9 |
| Y | claims 1-3, 10, 12, 14, examples 4-7, etc. | 1-15 |
| X | WO 2013/180200 A1 (CHUGAI PHARMACEUTICAL CO., LTD.) 05.12.2013 (2013-12-05) claims 1, 2, 7, 8, 44, 45, paragraphs [0154], [0155], examples 7-10, etc. | 1-15 |
| Y | claims 1, 2, 7, 8, 44, 45, paragraphs [0154], [0155], examples 7-10, etc. | |
| Y | JP 2018-537473 A (AGIOS PHARMACEUTICALS, INC.) 20.12.2018 (2018-12-20) paragraph [0023], fig. 3D | 1-15 |
| Y | STEVENS, A. P. et al., "Quantitative analysis of 5'-deoxy-5'-methylthioadenosine in melanoma cells by liquid chromatography-stable isotope ratio tandem mass spectrometry", Journal of Chromatography B, 2008, vol. 876, pp. 123-128 abstract, etc. | |
| Y | LIMM, K. et al., "The metabolite 5'-methylthioadenosine signals through the adenosine receptor A2B in melanoma", European Journal of Cancer, 2014, vol. 50, pp. 2714-2724 abstract, etc. | 1-15 |

Form PCT/ISA/210 (continuation of second sheet) (January 2015)

**INTERNATIONAL SEARCH REPORT**
Information on patent family members

International application No.
PCT/JP2020/012189

| Patent Documents referred in the Report | Publication Date | Patent Family | Publication Date |
|---|---|---|---|
| JP 2007-531724 A | 08 Nov. 2007 | US 2007/0280946 A1<br>claims 1, 21-25,<br>examples 4, 6<br>JP 2005-292087 A | |
| WO 2015/083764 A1 | 11 Jun. 2015 | US 2016/0304862 A<br>claims 1-3, 10, 12,<br>14, examples 4-7<br>EP 3078744 A1<br>KR 10-2016-0093666 A<br>CN 105980557 A | |
| WO 2013/180200 A1 | 05 Dec. 2013 | US 2015/0166654 A1<br>claims 1, 2, 7, 8,<br>44, 45, examples 7-10<br>EP 2857420 A1<br>KR 10-2015-0016579 A<br>CN 104487457 A | |
| JP 2018-537473 A | 20 Dec. 2018 | US 2018/0371551 A<br>WO 2017/096165 A1<br>fig. 3D<br>EP 3383375 A<br>KR 10-2018-0100125 A<br>CN 108601752 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003105757 A
- WO 2012033953 A
- WO 2010081173 A
- WO 2013180200 A
- WO 2015083764 A
- WO 2004044011 A
- WO 2005040229 A
- WO 2002032925 A
- WO 1995001937 A
- WO 2002020565 A
- WO 2003029462 A
- WO 2008016854 A
- EP 404097 A
- WO 199311161 A
- US 6248516 B1
- WO 2015143414 A
- US 20110123527 A1
- WO 2003000883 A
- WO 2004022754 A
- WO 2006006693 A
- WO 2003104453 A
- WO 1994011523 A
- WO 1996034103 A
- WO 1999023221 A
- WO 1996027011 A
- WO 2006106905 A
- WO 2009086320 A
- WO 2008092117 A
- WO 2007041635 A
- WO 2006105338 A
- WO 2007024249 A
- WO 2007021841 A
- WO 2006031370 A
- WO 2000042072 A
- WO 2004029207 A
- WO 2004099249 A
- WO 2005070963 A
- WO 2006020114 A
- WO 2006116260 A
- WO 2006023403 A
- US 20090136485 A
- JP 2012054624 W
- WO 1999054342 A
- WO 2000061739 A
- WO 2002031140 A
- WO 2006067913 A
- WO 2002079255 A
- WO 2004065540 A
- JP 2012058603 W
- WO 2002060919 A
- WO 2004035752 A
- WO 2011122011 A
- WO 2012133782 A
- WO 2012115241 A
- WO 2013125667 A
- WO 2013002362 A
- WO 2013046704 A
- WO 2009125825 A
- WO 2012073992 A
- WO 2009140242 A
- EP 239400 A
- WO 1996002576 A
- US 4816567 A
- US 5648237 A
- US 5789199 A
- US 5840523 A
- US 5959177 A
- US 6040498 A
- US 6420548 B
- US 7125978 B
- US 6417429 B
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- US 4737456 A
- WO 2015046554 A1
- WO 199600257 A

**Non-patent literature cited in the description**


- **JANICE M REICHERT ; CLARK J ROSENSWEIG ; LAURA B FADEN ; MATTHEW C DEWITZ.** Monoclonal antibody successes in the clinic. *Nat. Biotechnol.,* 2005, vol. 23, 1073-1078
- **PAVLOU AK ; BELSEY MJ.** The therapeutic antibodies market to 2008. *Eur. J. Pharm. Biopharm.,* 2005, vol. 59 (3), 389-396
- **WEINER LM ; SURANA R ; WANG S.** Monoclonal antibodies: versatile platforms for cancer immunotherapy. *Nat. Rev. Immunol.,* 2010, vol. 10 (5), 317-327
- **LEWIS GD ; FIGARI I ; FENDLY B ; WONG WL ; CARTER P ; GORMAN C ; SHEPARD HM.** Differential responses of human tumor cell lines to anti-p185HER2 monoclonal antibodies. *Cancer Immunol. Immunotherapy,* 1993, vol. 37, 255-263
- **SATOH M ; IIDA S ; SHITARA K.** Non-fucosylated therapeutic antibodies as next-generation therapeutic antibodies. *Expert Opin. Biol. Ther.,* 2006, vol. 6 (11), 1161-1173
- **DESJARLAIS JR ; LAZAR GA ; ZHUKOVSKY EA ; CHU SY.** Optimizing engagement of the immune system by anti-tumor antibodies: an engineer's perspective. *Drug Discov. Today,* 2007, vol. 12 (21-22), 898-910
- **ALLEY SC ; OKELEY NM ; SENTER PD.** Antibody-drug conjugates: targeted drug delivery for cancer. *Curr. Opin. Chem. Biol.,* 2010, vol. 14 (4), 529-537
- **BAEUERLE PA ; KUFER P ; BARGOU R.** BiTE: Teaching antibodies to engage T-cells for cancer therapy. *Curr. Opin. Mol. Ther.,* 2009, vol. 11 (1), 22-30
- **LUTTERBUESE R ; RAUM T ; KISCHEL R ; HOFFMANN P ; MANGOLD S ; RATTEL B ; FRIEDRICH M ; THOMAS O ; LORENCZEWSKI G ; RAU D.** T cell-engaging BiTE antibodies specific for EGFR potently eliminate KRAS- and BRAF-mutated colorectal cancer cells. *Proc. Natl. Acad. Sci. U.S.A.,* 2010, vol. 107 (28), 12605-12610
- **RIECHELMANN H ; SAUTER A ; GOLZE W ; HANFT G ; SCHROEN C ; HOERMANN K ; ERHARDT T ; GRONAU S.** Phase I trial with the CD44v6-targeting immunoconjugate bivatuzumab mertansine in head and neck squamous cell carcinoma. *Oral Oncol.,* 2008, vol. 44 (9), 823-829
- **TRINH VA ; HWU WJ.** Ipilimumab in the treatment of melanoma. *Expert Opin. Biol. Ther.,* 14 April 2012
- **JUSZCZAK A ; GUPTA A ; KARAVITAKI N ; MIDDLETON MR ; GROSSMAN A.** IPILIMUMAB - A NOVEL IMMUNOMODULATING THERAPY CAUSING AUTOIMMUNE HYPOPHYSITIS: A CASE REPORT AND REVIEW. *Eur. J. Endocrinol.,* 10 April 2012
- **KIM SJ ; PARK Y ; HONG HJ.** Antibody engineering for the development of therapeutic antibodies. *Mol. Cells.,* 2005, vol. 20 (1), 17-29
- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- **MULLBERG et al.** *J. Immunol.,* 1994, vol. 152 (10), 4958-4968
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **KABAT et al.** *Sequences of Proteins of Immunological Interest,* 1991
- **MACCALLUM et al.** *J. Mol. Biol.,* 1996, vol. 262, 732-745
- **CO et al.** *J. Immunol.,* 1994, vol. 152, 2968-2976
- **BETTER ; HORWITZ.** *Methods in Enzymology,* 1989, vol. 178, 476-496
- **PLUECKTHUN ; SKERRA.** *Methods in Enzymology,* 1989, vol. 178, 476-496
- **LAMOYI.** *Methods in Enzymology,* 1989, vol. 121, 652-663
- **ROUSSEAUX.** *Methods in Enzymology,* 1989, vol. 121, 663-669
- **BIRD et al.** *TIBTECH,* 1991, vol. 9, 132-137
- **HOLLINGER et al.** *Proc. Natl. Acad. Sci. USA,* 1993, vol. 90, 6444-6448
- **HUSTON et al.** *Proc. Natl. Acad. Sci. U.S.A.,* 1988, vol. 85, 5879-5883
- **HUDSON et al.** *J. Immunol. Methods,* 1999, vol. 231, 177-189
- **CO, M. S. et al.** *J. Immunol.,* 1994, vol. 152, 2968-2976
- **BETTER, M. ; HORWITZ, A. H.** *Methods Enzymol.,* 1989, vol. 178, 476-496
- **PLUCKTHUN, A. ; SKERRA, A.** *Methods Enzymol.,* 1989, vol. 178, 497-515
- **LAMOYI, E.** *Methods Enzymol.,* 1986, vol. 121, 652-663
- **ROUSSEAUX, J. et al.** *Methods Enzymol.,* 1986, vol. 121, 663-669
- **BIRD, R. E. ; WALKER, B. W.** *Trends Biotechnol.,* 1991, vol. 9, 132-137
- **C VINCKE et al.** *The Journal of Biological Chemistry,* vol. 284, 3273-3284
- *Methods in Molecular Biology,* 2012, vol. 911, 65-78

- *Biochimica et Biophysica Acta-Proteins and Proteomics,* 2006, vol. 1764 (8), 1307-1319
- *Journal of Applied Microbiology,* 2014, vol. 117 (2), 528-536
- *Journal of Biomolecular Screening,* 2016, vol. 21 (1), 35-43
- *Journal of Biological Chemistry,* 2016, vol. 291 (24), 12641-12657
- *AIDS,* 2016, vol. 30 (11), 1691-1701
- **SAMBROOK, J et al.** Molecular Cloning. Cold Spring Harbor Lab. Press, 1989, 9.47-9.58
- *J. Immunol.,* 1979, vol. 123 (4), 1548-1550
- *Current Topics in Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- **KOHLER ; MILSTEIN et al.** *Methods Enzymol.,* 1981, vol. 73, 3-46
- **VANDAMME et al.** *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. U.S.A.,* 1988, vol. 85 (23), 8998-9002
- *Nucleic Acids Res.,* 1989, vol. 17 (8), 2919-2932
- *Current Protocols in Protein Science,* May 2001
- *Bio/Technology,* 1994, vol. 12 (7), 699-702
- **CONRATH et al.** *J. Biol. Chem.,* 2001, vol. 276 (10), 7346-7350
- **MUYLDERMANS.** *Rev. Mol. Biotech.,* 2001, vol. 74, 277-302
- **KONTERMANN R.E.** *Bispecific Antibodies,* 2011
- **MILSTEIN et al.** *Nature,* 1983, vol. 305, 537-540
- **RIDGWAY et al.** *Protein Engineering,* 1996, vol. 9, 617-621
- **MERCHANT et al.** *Nature Biotechnology,* 1998, vol. 16, 677-681
- **STEVENS et al.** *J Chromatogr A.,* 07 May 2010, vol. 1217 (19), 3282-8
- **EVA GOTTFRIED ; KATRIN PETER ; MARINA P. KREUTZ.** *From Molecular to Modular Tumor Therapy,* 2010, vol. 3 (2), 111-132
- **DHANASEKARAN et al.** *Nature,* 2001, vol. 412, 822-826
- **LAPOINTE et al.** *Proc. Natl. Acad. Sci. U.S.A.,* 2004, vol. 101, 811-816
- **PEROU et al.** *Nature,* 2000, vol. 406, 747-752
- **AHRAM et al.** *Mol. Carcinog.,* 2002, vol. 33, 9-15
- **HOOD et al.** *Mol. Cell. Proteomics,* 2005, vol. 4, 1741-1753
- **BRINDLE et al.** *J. Mol. Recognit.,* 1997, vol. 10, 182-187
- **GATES ; SWEELEY.** *Clin. Chem.,* 1978, vol. 24, 1663-1673
- **DANG ; SEMENZA.** *Trends Biochem. Sci.,* 1999, vol. 24, 68-72
- **VIZAN et al.** *Cancer Res.,* 2005, vol. 65, 5512-5515
- **LAWTON et al.** *Pharmacogenomics,* 2008, vol. 9, 383
- **DESMOULIN F. et al.** *Drug Metab Dispos.,* 2002
- **DUAN JX et al.** *J Med Chem.,* 2008
- **CHRISTOFK et al.** *Nature,* 2008, vol. 452, 230-233
- **FANTIN et al.** *Cancer Cell,* 2006, vol. 9, 425-434
- **TERESA et al.** *Mol. Cancer,* 2009, vol. 8, 41-59
- *Nature Immunology,* 2008, vol. 9, 1261-1269
- **MAZUREK ; EIGENBRODT.** *Anticancer Res.,* 2003, vol. 23, 1149-1154
- **MAZUREK et al.** *J. Cell. Physiol.,* 1999, vol. 181, 136-146
- **DROGE et al.** *Immunobiology,* 1987, vol. 174, 473-479
- **UYTTENHOVE et al.** *Nat. Med.,* 2003, vol. 9, 1269-127
- **LOB et al.** *Cancer Immunol. Immunother.,* 2009, vol. 58, 153-157
- **OPITZ et al.** *Nature,* 2011, vol. 478 (7368), 197-203
- *J. Immunol.,* 2008, vol. 181, 5396-5404
- **YU et al.** *J. Immunol.,* 2013, vol. 190, 3783-3797
- **FRUMENTO et al.** *J. Exp. Med.,* 2002, vol. 196, 459-468
- *FASEB J.,* 2004, vol. 18, 790-804
- **SHENG et al.** *Cancer Res.,* 1998, vol. 58, 362-366
- **DENKERT et al.** *Clin. Breast Cancer,* 2004, vol. 4, 428-433
- **DENKER et al.** *Mod. Pathol.,* 2006, vol. 19, 1261-1269
- *Curr. Med. Chem.,* 2011, vol. 18, 5217-5223
- *Nat. Rev. Cancer,* 2012, vol. 12 (11), 782-792
- *J. Lab. Clin. Med.,* 1993, vol. 122, 518-523
- *Eur. J. Clin. Pharmacol.,* 1994, vol. 46, 3-7
- *Clin. Exp. Rheumatol.,* 1999, vol. 17, 151-160
- *Am. J. Vet. Res.,* 2004, vol. 65, 1269-1275
- *PLoS One.,* 2008, vol. 3, e2599
- **RESTA ; THOMPSON.** *Immunol. Rev.,* 1998, vol. 161, 95-109
- **SADEJ et al.** *Melanoma Res.,* 2006, vol. 16, 213-222

- **BLAY ; HOSKIN.** *Cancer Res.,* 1997, vol. 57, 2602-2605
- **KOBIE et al.** *J. Immunol.,* 2006, vol. 177, 6780-6786
- **CANBOLAT et al.** *Breast Cancer Res. Treat.,* 1996, vol. 37, 189-193
- **DURAK et al.** *Cancer Lett.,* 1994, vol. 84, 199-202
- **FLOCKE ; MANNHERZ.** *Biochim. Biophys. Acta,* 1991, vol. 1076, 273-281
- **BARDOT et al.** *Br. J. Cancer,* 1994, vol. 70, 212-218
- **HEADRICK ; WILLIS.** *Biochem. J.,* 1989, vol. 261, 541-550
- *Proc. Natl. Acad. Sci.,* 2006, vol. 103 (35), 13132-13137
- *Curr. Med. Chem.,* 2011, vol. 18, 5217-23
- *Nat. Med.,* 2007, vol. 13, 913-919
- *Am. J. Respir. Crit. Care Med.,* 2010, vol. 181, 928-934
- *Journal of Pharmaceutical and Biomedical Analysis,* 2004, vol. 36, 877-882
- *Nat. Med.,* 2010, vol. 16, 1434-1438
- *FASEB J.,* 2008, vol. 22, 2263-2272
- *J. Immunol.,* 2006, vol. 176, 4449-4458
- *J. Am. Soc. Nephrol.,* 2011, vol. 22 (5), 890-901
- *PLoS ONE J.,* 2010, vol. 5 (2), e9242
- *Am. J. Respir. Crit. Care Med.,* 2010, vol. 182, 774-783
- *Nat. Med.,* 2007, vol. 13, 851-856
- *J. Clin. Invest.,* 2010, vol. 120 (6), 1939-1949
- **ULANOVSKAYA et al.** *Nat. Chem. Biol.,* 2013, vol. 9 (5), 300-306
- **YAMADA et al.** *J. Nutr. Sci. Vitaminol.,* 2010, vol. 56, 83-86
- Epitope Mapping Protocols in Methods in Molecular Biology. 1996, vol. 66
- Antibodies: A Laboratory Manual. 1988, 359-420
- **HOLLIGER et al.** *Protein Engineering,* 1996, vol. 9 (3), 299-305
- *Proc. Natl. Acad. Sci. USA,* 2006, vol. 103 (11), 4005-4010
- *Proc. Natl. Acad. Sci. U.S.A.,* 2006, vol. 103 (11), 4005-4010
- *Curr. Opin. Biotechnol.,* 2009, vol. 20 (6), 685-91
- *Curr. Opin. Immunol.,* 2008, vol. 20 (4), 460-470
- *Protein Eng. Des. Sel.,* 2010, vol. 23 (4), 195-202
- **VAUPEL et al.** *Cancer Res.,* 1989, vol. 49, 6449-6665
- **SATOH M ; IIDA S ; SHITARA K.** *Expert Opin. Biol. Ther.,* 2006, vol. 6 (11), 1161-1173
- **WEITZHANDLER et al.** *J. Pharma. Sciences,* 1994, vol. 83 (12), 1670-1675
- **SCHENK et al.** *J. Clin. Investigation,* 2001, vol. 108 (11), 1687-1695
- **BIGGE et al.** *Anal. Biochem.,* 1995, vol. 230 (2), 229-238
- **BURMEISTER et al.** *Nature,* 1994, vol. 372, 336-343
- *Cur. Opin. in Biotech.,* 2009, vol. 20 (6), 685-691
- *Immunity,* 2005, vol. 23, 503-514
- **GHETIE et al.** *Immunol. Today,* 1997, vol. 18 (12), 592-598
- **RAGHAVAN et al.** *Immunity,* 1994, vol. 1, 303-315
- *Curr Opin Chem Biol,* 2010, vol. 14, 529-37
- **LANGONE et al.** *Methods in Enzymology,* 1983, vol. 93, 307-308
- *Nature Medicine,* 1996, vol. 2, 350-353
- **FULTON et al.** *J. Biol. Chem.,* 1986, vol. 261, 5314-5319
- **SIVAM et al.** *Cancer Res.,* 1987, vol. 47, 3169-3173
- **CUMBER et al.** *J. Immunol. Methods,* 1990, vol. 135, 15-24
- **WAWRZYNCZAK et al.** *Cancer Res.,* 1990, vol. 50, 7519-7562
- **GHEEITE et al.** *J. Immunol. Methods,* 1991, vol. 142, 223-230
- **THORPE et al.** *Cancer Res.,* 1987, vol. 47, 5924-5931
- **WAWRZYNCZAK et al.** *Br. J. Cancer,* 1992, vol. 66, 361-366
- **BOLOGNESI et al.** *Clin. exp. Immunol.,* 1992, vol. 89, 341-346
- **STIRPE F. ; BARBIERI L.** *FEBS letter,* 1986, vol. 195, 1-8
- **CASELLAS et al.** *Eur. J. Biochem.,* 1988, vol. 176, 581-588
- **HEINRICH et al.** *Biochem. J.,* 1998, vol. 334, 297-314
- **ISHIKAWA et al.** *J. Clin. Exp. Hematopathol.,* 2006, vol. 46 (2), 55-66
- *Cancer Res.,* 1993, vol. 53, 851-856
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268

- **STEVENS et al.** *J Chromatogr A,* 07 May 2010, vol. 1217 (19), 3282-8
- *J. Immunol. Methods.,* September 2003, vol. 280 (1-2), 139-55
- *BMC Biotechnol,* 29 January 2009, vol. 9 (6
- *Proc Natl Acad Sci U S A.,* 26 September 2000, vol. 97 (20), 10701-5
- **TOMLINSON et al.** *J. Mol. Biol.,* 1992, vol. 227, 776-798
- **WILLIAMS ; WINTER.** *Eur. J. Immunol.,* 1993, vol. 23, 1456-1461
- **COX et al.** *Nat. Genetics,* 1994, vol. 7, 162-168
- **MATSUDA et al.** *J. Exp. Med.,* 1998, vol. 188, 1973-1975
- **KAWASAKI et al.** *Eur. J. Immunol.,* 2001, vol. 31, 1017-1028
- **SCHABLE ; ZACHAU.** *Biol. Chem. Hoppe Seyler,* 1993, vol. 374, 1001-1022
- **BRENSING-KUPPERS et al.** *Gene,* 1997, vol. 191, 173-181
- **KAWASAKI et al.** *Genome Res.,* 1997, vol. 7, 250-261
- **WU et al.** *J. Exp. Med.,* 1970, vol. 132, 211-250
- *BBRC.,* 2013, vol. 436 (3), 543-50
- **GEJIMA et al.** *Human Antibodies,* 2002, vol. 11, 121-129
- **CARDOSO et al.** *Scand. J. Immunol.,* 2000, vol. 51, 337-344
- **CHOTHIA et al.** *J. Mol. Biol.,* 1992, vol. 227, 799-817
- **AL-LAZIKANI et al.** *J. Mol. Biol.,* 1997, vol. 273, 927-948
- **GONZALEZ-MUNOZ A et al.** *MAbs,* 2012
- **LEE CV et al.** *J Mol Biol.,* 2004
- **KNAPPIK A. et al.** *J Mol Biol.,* 2000
- **TILLER T et al.** *MAbs,* 2013
- **BARRETINA J et al.** *Nature,* 2012, vol. 483, 603-7
- **REINOSO RF et al.** *Drug Metab Dispos.,* April 2001, vol. 29 (4), 453-9
- **M'SOKA TJ et al.** *Leukemia,* 2000, vol. 14, 935-940
- *Methods mol. Biol.,* 2002, vol. 178, 133-145
- *J. Appl. Cryst.,* 2010, vol. 43, 186-190
- *J. Appl. Cryst.,* 2007, vol. 40, 658-674
- *Acta Cryst.,* 2010, vol. D66, 486-501
- *Acta Cryst.,* 2011, vol. D67, 355-467
- *Acta Cryst.,* 2011, vol. D67, 293-302
- *Methods Mol. Biol.,* 2002, vol. 178, 133-145
- *J. Appl. Phys.,* 2003, vol. 42, L798-L800
- *Protein Science,* 1995, vol. 4, 2411-2423